# EUROPEAN PATENT SPECIFICATION

(11) **EP 2 710 114 B1**
(45) Date of publication and mention of the grant of the patent: **03.11.2021**
(21) Application number: 12789187.7
(22) Date of filing: 08.05.2012
(51) Int. Cl.: C12N 1/19, C12P 21/08, C12N 1/38, C12N 15/12, C07K 16/00, C12P 21/00

(54) **HIGH-PURITY PRODUCTION OF MULTI-SUBUNIT PROTEINS SUCH AS ANTIBODIES IN TRANSFORMED MICROBES SUCH AS PICHIA PASTORIS**
HERSTELLUNG HOCHREINER PROTEINE MIT MEHREREN UNTEREINHEITEN, Z. B. ANTIKÖRPERN IN TRANSFORMIERTEN MIKROBEN WIE PICHIA PASTORIS
PRODUCTION DE PROTÉINES À SOUS-UNITÉS MULTIPLES DE PURETÉ ÉLEVÉE TELLES QUE DES ANTICORPS DANS DES MICROBES TRANSFORMÉS TELS QUE PICHIA PASTORIS

(30) Priority: 20.05.2011 US 201161488660 P; 14.06.2011 US 201161496873 P; 14.06.2011 US 201161496860 P; 19.08.2011 US 201161525307 P
(43) Date of publication of application: 26.03.2014
(73) Proprietor: H. Lundbeck A/S, 2500 Valby (DK)
(72) Inventor: MCNEILL, Patricia, Dianne, Federal Way, WA 98003 (US); JANSON, Nicole, Edmonds, WA 98026 (US); LESNICKI, Gary, L., Woodinville, WA 98072 (US); QI, Pei, Bothell, WA 98021 (US); LATHAM, John, A., Seattle, WA 98119 (US); GARCIA-MARTINEZ, Leon, F., Woodinville, WA 98072 (US)
(74) Representative: Stephens, Clare Jean
(86) International application number: PCT/US2012/036953
(87) International publication number: WO 2012/161956

(56) References cited:
- WO-A2-00/56903
- WO-A2-02/48382
- WO-A2-2008/063302
- WO-A2-2012/075340
- US-A1- 2004 077 069
- US-B2- 7 927 863
- TAPANI E ET AL: "Toxicity of ethanol in low concentrations. Experimental evaluation in cell culture.", ACTA RADIOLOGICA (STOCKHOLM, SWEDEN : 1987) NOV 1996, vol. 37, no. 6, November 1996 (1996-11), pages 923-926, XP009180529, ISSN: 0284-1851
- MUHLBAUER E ET AL: "Impaired immunoglobulin M production by incubation of hybridoma cells with ethanol", ALCOHOL, PERGAMON PRESS, LONDON, GB, vol. 24, no. 3, 1 July 2001 (2001-07-01), pages 179-187, XP027230584, ISSN: 0741-8329 [retrieved on 2001-07-01]
- KRISTIN BAUMANN ET AL: "Hypoxic fed-batch cultivation ofPichia pastoris increases specific and volumetric productivity of recombinant proteins", BIOTECHNOLOGY AND BIOENGINEERING, vol. 100, no. 1, 1 May 2008 (2008-05-01), pages 177-183, XP55026399, ISSN: 0006-3592, DOI: 10.1002/bit.21763
- E Thalia David ET AL: "E THE JOURNAL OF BIOLOGICAL CHEMISTRY Studies on the Effect of Ethanol on Eukaryotic Protein Synthesis in Vitro*", J. Biol. Chem. at EUROPEAN PATENT OFFICE on October, 25 June 1983 (1983-06-25), pages 7702-7706, XP055144164, Retrieved from the Internet: URL:http://www.jbc.org/content/258/12/7702 .full.pdf [retrieved on 2014-10-02]
- TEUN VAN DE LAAR ET AL: "Increased Heterologous Protein Production by Saccharomyces cerevisiae Growing on Ethanol as Sole Carbon Source", BIOTECHNOLOGY AND BIOENGINEERING, WILEY & SONS, HOBOKEN, NJ, US, vol. 96, no. 3, 15 February 2007 (2007-02-15), pages 483-494, XP007914112, ISSN: 0006-3592 [retrieved on 2006-08-31]
- TEUN VAN DE LAAR ET AL.: 'Increased heterologous protein production by Saccharomyces cerevisiae growing on ethanol as sole carbon source' BIOTECHNOLOGY AND BIOENGINEERING. vol. 96, no. 3, 15 February 2007, pages 483 - 494, XP007914112
- STEPHAN HELLWIG ET AL.: 'Analysis of single-chain antibody production in Pichia pastoris using on-line methanol control in fed-batch and mixed-feed fermentations' BIOTECHNOLOGY AND BIOENGINEERING. vol. 74, no. 4, 20 August 2001, pages 344 - 352, XP055134090
- MICHELE SALIOLA ET AL.: 'Use of the KIADH4 promoter for ethanol-dependent production of recombinant human serum albumin in Kluyveromyces lactis' APPLIED AND ENVIRONMENTAL MICROBIOLOGY. vol. 65, no. 1, January 1999, pages 53 - 60, XP002172958
- None

### Sequence Listing

<110> Patti McNeill Leon F. Garcia-Martinez Nicole Janson Gary Lesnicki Pei Qi John Latham
<120> HIGH-PURITY PRODUCTION OF MULTI-SUBUNIT PROTEINS SUCH AS ANTIBODIES IN TRANSFORMED MICROBES SUCH AS PICHIA PASTORIS
<130> 67858.711002
<150> 61/525,307
   <151> 2011-08-19
<150> 61/496,860
   <151> 2011-06-14
<150> 61/496,873
   <151> 2011-06-14
<150> 61/418,832
   <151> 2010-12-01
<160> 441
<170> PatentIn version 3.5
<210> 1
   <211> 111
   <212> PRT
   <213> Oryctolagus cuniculus
<400> 1
   Ala Leu Val Met Thr Gln Thr Pro Ser Ser Val Ser Ala Ala Val Gly
   1               5                   10                  15
   Gly Thr Val Thr Ile Asn Cys Gln Ala Ser Gln Asn Ile Tyr Ser Asn
               20                  25                  30
   Leu Ala Trp Tyr Gln Gln Arg Pro Gly Gln Arg Pro Lys Leu Leu Ile
           35                  40                  45
   Tyr Gly Ala Ser Asn Leu Asp Ala Gly Val Pro Ser Arg Phe Arg Gly
       50                  55                  60
   Ser Gly Ser Gly Thr Glu Tyr Thr Leu Thr Ile Ser Asp Leu Glu Cys
   65                  70                  75                  80
   Asp Asp Val Gly Thr Tyr Tyr Cys Gln Ser Ala Phe Asp Ser Asp Ser
                   85                  90                  95
   Thr Glu Asn Thr Phe Gly Gly Gly Thr Glu Val Val Val Lys Arg
               100                 105                 110
<210> 2
   <211> 217
   <212> PRT
   <213> Synthetic
<400> 2
   Ala Leu Val Met Thr Gln Thr Pro Ser Ser Val Ser Ala Ala Val Gly
   1               5                   10                  15
   Gly Thr Val Thr Ile Asn Cys Gln Ala Ser Gln Asn Ile Tyr Ser Asn
               20                  25                  30
   Leu Ala Trp Tyr Gln Gln Arg Pro Gly Gln Arg Pro Lys Leu Leu Ile
           35                  40                  45
   Tyr Gly Ala Ser Asn Leu Asp Ala Gly Val Pro Ser Arg Phe Arg Gly
       50                  55                  60
   Ser Gly Ser Gly Thr Glu Tyr Thr Leu Thr Ile Ser Asp Leu Glu Cys
   65                  70                  75                  80
   Asp Asp Val Gly Thr Tyr Tyr Cys Gln Ser Ala Phe Asp Ser Asp Ser
                   85                  90                  95
   Thr Glu Asn Thr Phe Gly Gly Gly Thr Glu Val Val Val Lys Arg Thr
               100                 105                 110
   Val Ala Ala Pro Ser Val Phe Ile Phe Pro Pro Ser Asp Glu Gln Leu
           115                 120                 125
   Lys Ser Gly Thr Ala Ser Val Val Cys Leu Leu Asn Asn Phe Tyr Pro
       130                 135                 140
   Arg Glu Ala Lys Val Gln Trp Lys Val Asp Asn Ala Leu Gln Ser Gly
   145                 150                 155                 160
   Asn Ser Gln Glu Ser Val Thr Glu Gln Asp Ser Lys Asp Ser Thr Tyr
                   165                 170                 175
   Ser Leu Ser Ser Thr Leu Thr Leu Ser Lys Ala Asp Tyr Glu Lys His
               180                 185                 190
   Lys Val Tyr Ala Cys Glu Val Thr His Gln Gly Leu Ser Ser Pro Val
           195                 200                 205
   Thr Lys Ser Phe Asn Arg Gly Glu Cys
       210                 215
<210> 3
   <211> 118
   <212> PRT
   <213> Oryctolagus cuniculus
<400> 3
   Gln Ser Leu Glu Glu Ser Gly Gly Arg Leu Val Thr Pro Gly Thr Pro
   1               5                   10                  15
   Leu Thr Leu Thr Cys Thr Val Ser Gly Phe Ser Leu Ser Ser Tyr Ala
               20                  25                  30
   Met Ser Trp Val Arg Gln Ala Pro Gly Lys Gly Leu Glu Trp Ile Gly
           35                  40                  45
   Val Ile Thr Ser Ile Gly Ser Thr Val Tyr Ala Ser Trp Ala Lys Gly
       50                  55                  60
   Arg Phe Thr Ile Ser Lys Thr Ser Thr Thr Val Asp Leu Lys Ile Thr
   65                  70                  75                  80
   Ser Pro Thr Thr Glu Asp Thr Ala Thr Tyr Phe Cys Ala Arg Gly Tyr
                   85                  90                  95
   Asp Asp Tyr Asp Glu Met Thr Tyr Phe Asn Ile Trp Gly Gln Gly Thr
               100                 105                 110
   Leu Val Thr Val Ser Ser
           115
<210> 4
   <211> 448
   <212> PRT
   <213> Synthetic
<400> 4
   Gln Ser Leu Glu Glu Ser Gly Gly Arg Leu Val Thr Pro Gly Thr Pro
   1               5                   10                  15
   Leu Thr Leu Thr Cys Thr Val Ser Gly Phe Ser Leu Ser Ser Tyr Ala
               20                  25                  30
   Met Ser Trp Val Arg Gln Ala Pro Gly Lys Gly Leu Glu Trp Ile Gly
           35                  40                  45
   Val Ile Thr Ser Ile Gly Ser Thr Val Tyr Ala Ser Trp Ala Lys Gly
       50                  55                  60
   Arg Phe Thr Ile Ser Lys Thr Ser Thr Thr Val Asp Leu Lys Ile Thr
   65                  70                  75                  80
   Ser Pro Thr Thr Glu Asp Thr Ala Thr Tyr Phe Cys Ala Arg Gly Tyr
                   85                  90                  95
   Asp Asp Tyr Asp Glu Met Thr Tyr Phe Asn Ile Trp Gly Gln Gly Thr
               100                 105                 110
   Leu Val Thr Val Ser Ser Ala Ser Thr Lys Gly Pro Ser Val Phe Pro
           115                 120                 125
   Leu Ala Pro Ser Ser Lys Ser Thr Ser Gly Gly Thr Ala Ala Leu Gly
       130                 135                 140
   Cys Leu Val Lys Asp Tyr Phe Pro Glu Pro Val Thr Val Ser Trp Asn
   145                 150                 155                 160
   Ser Gly Ala Leu Thr Ser Gly Val His Thr Phe Pro Ala Val Leu Gln
                   165                 170                 175
   Ser Ser Gly Leu Tyr Ser Leu Ser Ser Val Val Thr Val Pro Ser Ser
               180                 185                 190
   Ser Leu Gly Thr Gln Thr Tyr Ile Cys Asn Val Asn His Lys Pro Ser
           195                 200                 205
   Asn Thr Lys Val Asp Lys Arg Val Glu Pro Lys Ser Cys Asp Lys Thr
       210                 215                 220
   His Thr Cys Pro Pro Cys Pro Ala Pro Glu Leu Leu Gly Gly Pro Ser
   225                 230                 235                 240
   Val Phe Leu Phe Pro Pro Lys Pro Lys Asp Thr Leu Met Ile Ser Arg
                   245                 250                 255
   Thr Pro Glu Val Thr Cys Val Val Val Asp Val Ser His Glu Asp Pro
               260                 265                 270
   Glu Val Lys Phe Asn Trp Tyr Val Asp Gly Val Glu Val His Asn Ala
           275                 280                 285
   Lys Thr Lys Pro Arg Glu Glu Gln Tyr Ala Ser Thr Tyr Arg Val Val
       290                 295                 300
   Ser Val Leu Thr Val Leu His Gln Asp Trp Leu Asn Gly Lys Glu Tyr
   305                 310                 315                 320
   Lys Cys Lys Val Ser Asn Lys Ala Leu Pro Ala Pro Ile Glu Lys Thr
                   325                 330                 335
   Ile Ser Lys Ala Lys Gly Gln Pro Arg Glu Pro Gln Val Tyr Thr Leu
               340                 345                 350
   Pro Pro Ser Arg Glu Glu Met Thr Lys Asn Gln Val Ser Leu Thr Cys
           355                 360                 365
   Leu Val Lys Gly Phe Tyr Pro Ser Asp Ile Ala Val Glu Trp Glu Ser
       370                 375                 380
   Asn Gly Gln Pro Glu Asn Asn Tyr Lys Thr Thr Pro Pro Val Leu Asp
   385                 390                 395                 400
   Ser Asp Gly Ser Phe Phe Leu Tyr Ser Lys Leu Thr Val Asp Lys Ser
                   405                 410                 415
   Arg Trp Gln Gln Gly Asn Val Phe Ser Cys Ser Val Met His Glu Ala
               420                 425                 430
   Leu His Asn His Tyr Thr Gln Lys Ser Leu Ser Leu Ser Pro Gly Lys
           435                 440                 445
<210> 5
   <211> 11
   <212> PRT
   <213> Oryctolagus cuniculus
<400> 5
   Gln Ala Ser Gln Asn Ile Tyr Ser Asn Leu Ala
   1               5                   10
<210> 6
   <211> 7
   <212> PRT
   <213> Oryctolagus cuniculus
<400> 6
   Gly Ala Ser Asn Leu Asp Ala
   1               5
<210> 7
   <211> 12
   <212> PRT
   <213> Oryctolagus cuniculus
<400> 7
   Gln Ser Ala Phe Asp Ser Asp Ser Thr Glu Asn Thr
   1               5                   10
<210> 8
   <211> 5
   <212> PRT
   <213> Oryctolagus cuniculus
<400> 8
   Ser Tyr Ala Met Ser
   1               5
<210> 9
   <211> 16
   <212> PRT
   <213> Oryctolagus cuniculus
<400> 9
   Val Ile Thr Ser Ile Gly Ser Thr Val Tyr Ala Ser Trp Ala Lys Gly
   1               5                   10                  15
<210> 10
   <211> 13
   <212> PRT
   <213> Oryctolagus cuniculus
<400> 10
   Gly Tyr Asp Asp Tyr Asp Glu Met Thr Tyr Phe Asn Ile
   1               5                   10
<210> 11
   <211> 111
   <212> PRT
   <213> Synthetic
<400> 11
   Asp Ile Gln Met Thr Gln Ser Pro Ser Thr Leu Ser Ala Ser Val Gly
   1               5                   10                  15
   Asp Arg Val Thr Ile Thr Cys Gln Ala Ser Gln Asn Ile Tyr Ser Asn
               20                  25                  30
   Leu Ala Trp Tyr Gln Gln Lys Pro Gly Lys Ala Pro Lys Leu Leu Ile
           35                  40                  45
   Tyr Gly Ala Ser Asn Leu Asp Ala Gly Val Pro Ser Arg Phe Ser Gly
       50                  55                  60
   Ser Gly Ser Gly Thr Glu Tyr Thr Leu Thr Ile Ser Ser Leu Gln Pro
   65 70 75 80
   Asp Asp Phe Ala Thr Tyr Tyr Cys Gln Ser Ala Phe Asp Ser Asp Ser
   85 90 95
   Thr Glu Asn Thr Phe Gly Gly Gly Thr Lys Val Glu Ile Lys Arg
   100 105 110
<210> 12
   <211> 217
   <212> PRT
   <213> Synthetic
<400> 12
   Asp Ile Gln Met Thr Gln Ser Pro Ser Thr Leu Ser Ala Ser Val Gly
   1 5 10 15
   Asp Arg Val Thr Ile Thr Cys Gln Ala Ser Gln Asn Ile Tyr Ser Asn
   20 25 30
   Leu Ala Trp Tyr Gln Gln Lys Pro Gly Lys Ala Pro Lys Leu Leu Ile
   35 40 45
   Tyr Gly Ala Ser Asn Leu Asp Ala Gly Val Pro Ser Arg Phe Ser Gly
   50 55 60
   Ser Gly Ser Gly Thr Glu Tyr Thr Leu Thr Ile Ser Ser Leu Gln Pro
   65 70 75 80
   Asp Asp Phe Ala Thr Tyr Tyr Cys Gln Ser Ala Phe Asp Ser Asp Ser
   85 90 95
   Thr Glu Asn Thr Phe Gly Gly Gly Thr Lys Val Glu Ile Lys Arg Thr
   100 105 110
   Val Ala Ala Pro Ser Val Phe Ile Phe Pro Pro Ser Asp Glu Gln Leu
   115 120 125
   Lys Ser Gly Thr Ala Ser Val Val Cys Leu Leu Asn Asn Phe Tyr Pro
   130 135 140
   Arg Glu Ala Lys Val Gln Trp Lys Val Asp Asn Ala Leu Gln Ser Gly
   145 150 155 160
   Asn Ser Gln Glu Ser Val Thr Glu Gln Asp Ser Lys Asp Ser Thr Tyr
   165 170 175
   Ser Leu Ser Ser Thr Leu Thr Leu Ser Lys Ala Asp Tyr Glu Lys His
               180                 185                 190
   Lys Val Tyr Ala Cys Glu Val Thr His Gln Gly Leu Ser Ser Pro Val
           195                 200                 205
   Thr Lys Ser Phe Asn Arg Gly Glu Cys
       210                 215
<210> 13
   <211> 121
   <212> PRT
   <213> Synthetic
<400> 13
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Val Ser Ser Tyr
               20                  25                  30
   Ala Met Ser Trp Val Arg Gln Ala Pro Gly Lys Gly Leu Glu Trp Val
           35                  40                  45
   Gly Val Ile Thr Ser Ile Gly Ser Thr Val Tyr Ala Ser Ser Ala Lys
       50                  55                  60
   Gly Arg Phe Thr Ile Ser Arg Asp Asn Ser Lys Asn Thr Leu Tyr Leu
   65                  70                  75                  80
   Gln Met Asn Ser Leu Arg Ala Glu Asp Thr Ala Val Tyr Tyr Cys Ala
                   85                  90                  95
   Arg Gly Tyr Asp Asp Tyr Asp Glu Met Thr Tyr Phe Asn Ile Trp Gly
               100                 105                 110
   Gln Gly Thr Leu Val Thr Val Ser Ser
           115                 120
<210> 14
   <211> 451
   <212> PRT
   <213> Synthetic
<400> 14
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Val Ser Ser Tyr
   20 25 30
   Ala Met Ser Trp Val Arg Gln Ala Pro Gly Lys Gly Leu Glu Trp Val
   35 40 45
   Gly Val Ile Thr Ser Ile Gly Ser Thr Val Tyr Ala Ser Ser Ala Lys
   50 55 60
   Gly Arg Phe Thr Ile Ser Arg Asp Asn Ser Lys Asn Thr Leu Tyr Leu
   65 70 75 80
   Gln Met Asn Ser Leu Arg Ala Glu Asp Thr Ala Val Tyr Tyr Cys Ala
   85 90 95
   Arg Gly Tyr Asp Asp Tyr Asp Glu Met Thr Tyr Phe Asn Ile Trp Gly
   100 105 110
   Gln Gly Thr Leu Val Thr Val Ser Ser Ala Ser Thr Lys Gly Pro Ser
   115 120 125
   Val Phe Pro Leu Ala Pro Ser Ser Lys Ser Thr Ser Gly Gly Thr Ala
   130 135 140
   Ala Leu Gly Cys Leu Val Lys Asp Tyr Phe Pro Glu Pro Val Thr Val
   145 150 155 160
   Ser Trp Asn Ser Gly Ala Leu Thr Ser Gly Val His Thr Phe Pro Ala
   165 170 175
   Val Leu Gln Ser Ser Gly Leu Tyr Ser Leu Ser Ser Val Val Thr Val
   180 185 190
   Pro Ser Ser Ser Leu Gly Thr Gln Thr Tyr Ile Cys Asn Val Asn His
   195 200 205
   Lys Pro Ser Asn Thr Lys Val Asp Lys Arg Val Glu Pro Lys Ser Cys
   210 215 220
   Asp Lys Thr His Thr Cys Pro Pro Cys Pro Ala Pro Glu Leu Leu Gly
   225 230 235 240
   Gly Pro Ser Val Phe Leu Phe Pro Pro Lys Pro Lys Asp Thr Leu Met
   245 250 255
   Ile Ser Arg Thr Pro Glu Val Thr Cys Val Val Val Asp Val Ser His
   260 265 270
   Glu Asp Pro Glu Val Lys Phe Asn Trp Tyr Val Asp Gly Val Glu Val
           275                 280                 285
   His Asn Ala Lys Thr Lys Pro Arg Glu Glu Gln Tyr Ala Ser Thr Tyr
       290                 295                 300
   Arg Val Val Ser Val Leu Thr Val Leu His Gln Asp Trp Leu Asn Gly
   305                 310                 315                 320
   Lys Glu Tyr Lys Cys Lys Val Ser Asn Lys Ala Leu Pro Ala Pro Ile
                   325                 330                 335
   Glu Lys Thr Ile Ser Lys Ala Lys Gly Gln Pro Arg Glu Pro Gln Val
               340                 345                 350
   Tyr Thr Leu Pro Pro Ser Arg Glu Glu Met Thr Lys Asn Gln Val Ser
           355                 360                 365
   Leu Thr Cys Leu Val Lys Gly Phe Tyr Pro Ser Asp Ile Ala Val Glu
       370                 375                 380
   Trp Glu Ser Asn Gly Gln Pro Glu Asn Asn Tyr Lys Thr Thr Pro Pro
   385                 390                 395                 400
   Val Leu Asp Ser Asp Gly Ser Phe Phe Leu Tyr Ser Lys Leu Thr Val
                   405                 410                 415
   Asp Lys Ser Arg Trp Gln Gln Gly Asn Val Phe Ser Cys Ser Val Met
               420                 425                 430
   His Glu Ala Leu His Asn His Tyr Thr Gln Lys Ser Leu Ser Leu Ser
           435                 440                 445
   Pro Gly Lys
       450
<210> 15
   <211> 11
   <212> PRT
   <213> Oryctolagus cuniculus
<400> 15
   Gln Ala Ser Gln Asn Ile Tyr Ser Asn Leu Ala
   1               5                   10
<210> 16
   <211> 7
   <212> PRT
   <213> Oryctolagus cuniculus
<400> 16
   Gly Ala Ser Asn Leu Asp Ala
   1 5
<210> 17
   <211> 12
   <212> PRT
   <213> Oryctolagus cuniculus
<400> 17
   Gln Ser Ala Phe Asp Ser Asp Ser Thr Glu Asn Thr
   1 5 10
<210> 18
   <211> 5
   <212> PRT
   <213> Oryctolagus cuniculus
<400> 18
   Ser Tyr Ala Met Ser
   1 5
<210> 19
   <211> 16
   <212> PRT
   <213> Synthetic
<400> 19
   Val Ile Thr Ser Ile Gly Ser Thr Val Tyr Ala Ser Ser Ala Lys Gly
   1 5 10 15
<210> 20
   <211> 13
   <212> PRT
   <213> Oryctolagus cuniculus
<400> 20
   Gly Tyr Asp Asp Tyr Asp Glu Met Thr Tyr Phe Asn Ile
   1 5 10
<210> 21
   <211> 111
   <212> PRT
   <213> Oryctolagus cuniculus
<400> 21
   Ala Val Leu Thr Gln Thr Pro Ser Pro Val Ser Ala Ala Met Gly Asp
   1 5 10 15
   Thr Val Thr Ile Lys Cys Gln Ser Ser Gln Ser Val Tyr Lys Asn Asn
   20 25 30
   Tyr Leu Ser Trp Tyr Gln Gln Lys Pro Gly Gln Pro Pro Arg Leu Leu
   35 40 45
   Ile Tyr Asp Ala Ser Asn Leu Pro Ser Gly Val Pro Ser Arg Phe Ser
   50 55 60
   Gly Ser Gly Ser Gly Thr Gln Phe Thr Leu Thr Ile Ser Gly Val Gln
   65 70 75 80
   Cys Asp Asp Ala Ala Thr Tyr Tyr Cys Leu Gly Asp Tyr Asp Asp Asp
   85 90 95
   Ala Asp Asn Ala Phe Gly Gly Gly Thr Glu Val Val Val Lys Arg
   100 105 110
<210> 22
   <211> 217
   <212> PRT
   <213> Synthetic
<400> 22
   Ala Val Leu Thr Gln Thr Pro Ser Pro Val Ser Ala Ala Met Gly Asp
   1               5                   10                  15
   Thr Val Thr Ile Lys Cys Gln Ser Ser Gln Ser Val Tyr Lys Asn Asn
               20                  25                  30
   Tyr Leu Ser Trp Tyr Gln Gln Lys Pro Gly Gln Pro Pro Arg Leu Leu
           35                  40                  45
   Ile Tyr Asp Ala Ser Asn Leu Pro Ser Gly Val Pro Ser Arg Phe Ser
       50                  55                  60
   Gly Ser Gly Ser Gly Thr Gln Phe Thr Leu Thr Ile Ser Gly Val Gln
   65                  70                  75                  80
   Cys Asp Asp Ala Ala Thr Tyr Tyr Cys Leu Gly Asp Tyr Asp Asp Asp
                   85                  90                  95
   Ala Asp Asn Ala Phe Gly Gly Gly Thr Glu Val Val Val Lys Arg Thr
               100                 105                 110
   Val Ala Ala Pro Ser Val Phe Ile Phe Pro Pro Ser Asp Glu Gln Leu
           115                 120                 125
   Lys Ser Gly Thr Ala Ser Val Val Cys Leu Leu Asn Asn Phe Tyr Pro
       130                 135                 140
   Arg Glu Ala Lys Val Gln Trp Lys Val Asp Asn Ala Leu Gln Ser Gly
   145                 150                 155                 160
   Asn Ser Gln Glu Ser Val Thr Glu Gln Asp Ser Lys Asp Ser Thr Tyr
                   165                 170                 175
   Ser Leu Ser Ser Thr Leu Thr Leu Ser Lys Ala Asp Tyr Glu Lys His
               180                 185                 190
   Lys Val Tyr Ala Cys Glu Val Thr His Gln Gly Leu Ser Ser Pro Val
           195                 200                 205
   Thr Lys Ser Phe Asn Arg Gly Glu Cys
       210                 215
<210> 23
   <211> 113
   <212> PRT
   <213> Oryctolagus cuniculus
<400> 23
   Gln Ser Val Glu Glu Ser Gly Gly Arg Leu Val Thr Pro Gly Thr Pro
   1               5                   10                  15
   Leu Thr Leu Thr Cys Thr Val Ser Gly Phe Ser Leu Ser Ser Tyr Val
               20                  25                  30
   Met Ile Trp Val Arg Gln Ala Pro Gly Lys Gly Leu Glu Tyr Ile Gly
           35                  40                  45
   Ile Thr Trp Ser Ala Gly Thr Tyr Tyr Ala Ser Trp Ala Lys Gly Arg
       50                  55                  60
   Phe Thr Ile Ser Lys Thr Ser Ser Thr Thr Val Asp Leu Lys Ile Thr
   65                  70                  75                  80
   Ser Pro Thr Thr Glu Asp Thr Ala Thr Tyr Phe Cys Ala Gly Gly Gly
                   85                  90                  95
   Gly Ser Ile Tyr Asp Ile Trp Gly Pro Gly Thr Leu Val Thr Val Ser
               100                 105                 110
   Ser
<210> 24
   <211> 443
   <212> PRT
   <213> Synthetic
<400> 24
   Gln Ser Val Glu Glu Ser Gly Gly Arg Leu Val Thr Pro Gly Thr Pro
   1               5                   10                  15
   Leu Thr Leu Thr Cys Thr Val Ser Gly Phe Ser Leu Ser Ser Tyr Val
               20                  25                  30
   Met Ile Trp Val Arg Gln Ala Pro Gly Lys Gly Leu Glu Tyr Ile Gly
           35                  40                  45
   Ile Thr Trp Ser Ala Gly Thr Tyr Tyr Ala Ser Trp Ala Lys Gly Arg
       50                  55                  60
   Phe Thr Ile Ser Lys Thr Ser Ser Thr Thr Val Asp Leu Lys Ile Thr
   65                  70                  75                  80
   Ser Pro Thr Thr Glu Asp Thr Ala Thr Tyr Phe Cys Ala Gly Gly Gly
                   85                  90                  95
   Gly Ser Ile Tyr Asp Ile Trp Gly Pro Gly Thr Leu Val Thr Val Ser
               100                 105                 110
   Ser Ala Ser Thr Lys Gly Pro Ser Val Phe Pro Leu Ala Pro Ser Ser
           115                 120                 125
   Lys Ser Thr Ser Gly Gly Thr Ala Ala Leu Gly Cys Leu Val Lys Asp
       130                 135                 140
   Tyr Phe Pro Glu Pro Val Thr Val Ser Trp Asn Ser Gly Ala Leu Thr
   145                 150                 155                 160
   Ser Gly Val His Thr Phe Pro Ala Val Leu Gln Ser Ser Gly Leu Tyr
                   165                 170                 175
   Ser Leu Ser Ser Val Val Thr Val Pro Ser Ser Ser Leu Gly Thr Gln
               180                 185                 190
   Thr Tyr Ile Cys Asn Val Asn His Lys Pro Ser Asn Thr Lys Val Asp
           195                 200                 205
   Lys Arg Val Glu Pro Lys Ser Cys Asp Lys Thr His Thr Cys Pro Pro
       210                 215                 220
   Cys Pro Ala Pro Glu Leu Leu Gly Gly Pro Ser Val Phe Leu Phe Pro
   225                 230                 235                 240
   Pro Lys Pro Lys Asp Thr Leu Met Ile Ser Arg Thr Pro Glu Val Thr
                   245                 250                 255
   Cys Val Val Val Asp Val Ser His Glu Asp Pro Glu Val Lys Phe Asn
               260                 265                 270
   Trp Tyr Val Asp Gly Val Glu Val His Asn Ala Lys Thr Lys Pro Arg
           275                 280                 285
   Glu Glu Gln Tyr Ala Ser Thr Tyr Arg Val Val Ser Val Leu Thr Val
       290                 295                 300
   Leu His Gln Asp Trp Leu Asn Gly Lys Glu Tyr Lys Cys Lys Val Ser
   305                 310                 315                 320
   Asn Lys Ala Leu Pro Ala Pro Ile Glu Lys Thr Ile Ser Lys Ala Lys
                   325                 330                 335
   Gly Gln Pro Arg Glu Pro Gln Val Tyr Thr Leu Pro Pro Ser Arg Glu
               340                 345                 350
   Glu Met Thr Lys Asn Gln Val Ser Leu Thr Cys Leu Val Lys Gly Phe
           355                 360                 365
   Tyr Pro Ser Asp Ile Ala Val Glu Trp Glu Ser Asn Gly Gln Pro Glu
       370                 375                 380
   Asn Asn Tyr Lys Thr Thr Pro Pro Val Leu Asp Ser Asp Gly Ser Phe
   385                 390                 395                 400
   Phe Leu Tyr Ser Lys Leu Thr Val Asp Lys Ser Arg Trp Gln Gln Gly
                   405                 410                 415
   Asn Val Phe Ser Cys Ser Val Met His Glu Ala Leu His Asn His Tyr
               420                 425                 430
   Thr Gln Lys Ser Leu Ser Leu Ser Pro Gly Lys
           435                 440
<210> 25
   <211> 13
   <212> PRT
   <213> Oryctolagus cuniculus
<400> 25
   Gln Ser Ser Gln Ser Val Tyr Lys Asn Asn Tyr Leu Ser
   1 5 10
<210> 26
   <211> 7
   <212> PRT
   <213> Oryctolagus cuniculus
<400> 26
   Asp Ala Ser Asn Leu Pro Ser
   1 5
<210> 27
   <211> 11
   <212> PRT
   <213> Oryctolagus cuniculus
<400> 27
   Leu Gly Asp Tyr Asp Asp Asp Ala Asp Asn Ala
   1 5 10
<210> 28
   <211> 5
   <212> PRT
   <213> Oryctolagus cuniculus
<400> 28
   Ser Tyr Val Met Ile
   1 5
<210> 29
   <211> 15
   <212> PRT
   <213> Oryctolagus cuniculus
<400> 29
   Ile Thr Trp Ser Ala Gly Thr Tyr Tyr Ala Ser Trp Ala Lys Gly
   1 5 10 15
<210> 30
   <211> 8
   <212> PRT
   <213> Oryctolagus cuniculus
<400> 30
   Gly Gly Gly Ser Ile Tyr Asp Ile
   1 5
<210> 31
   <211> 112
   <212> PRT
   <213> Synthetic
<400> 31
   Asp Ile Gln Met Thr Gln Ser Pro Ser Thr Leu Ser Ala Ser Val Gly
   1               5                   10                  15
   Asp Arg Val Thr Ile Thr Cys Gln Ser Ser Gln Ser Val Tyr Lys Asn
               20                  25                  30
   Asn Tyr Leu Ser Trp Tyr Gln Gln Lys Pro Gly Lys Ala Pro Lys Leu
           35                  40                  45
   Leu Ile Tyr Asp Ala Ser Asn Leu Pro Ser Gly Val Pro Ser Arg Phe
       50                  55                  60
   Ser Gly Ser Gly Ser Gly Thr Glu Phe Thr Leu Thr Ile Ser Ser Leu
   65                  70                  75                  80
   Gln Pro Asp Asp Phe Ala Thr Tyr Tyr Cys Leu Gly Asp Tyr Asp Asp
                   85                  90                  95
   Asp Ala Asp Asn Ala Phe Gly Gly Gly Thr Lys Val Glu Ile Lys Arg
               100                 105                 110
<210> 32
   <211> 218
   <212> PRT
   <213> Synthetic
<400> 32
   Asp Ile Gln Met Thr Gln Ser Pro Ser Thr Leu Ser Ala Ser Val Gly
   1               5                   10                  15
   Asp Arg Val Thr Ile Thr Cys Gln Ser Ser Gln Ser Val Tyr Lys Asn
               20                  25                  30
   Asn Tyr Leu Ser Trp Tyr Gln Gln Lys Pro Gly Lys Ala Pro Lys Leu
           35                  40                  45
   Leu Ile Tyr Asp Ala Ser Asn Leu Pro Ser Gly Val Pro Ser Arg Phe
       50                  55                  60
   Ser Gly Ser Gly Ser Gly Thr Glu Phe Thr Leu Thr Ile Ser Ser Leu
   65                  70                  75                  80
   Gln Pro Asp Asp Phe Ala Thr Tyr Tyr Cys Leu Gly Asp Tyr Asp Asp
                   85                  90                  95
   Asp Ala Asp Asn Ala Phe Gly Gly Gly Thr Lys Val Glu Ile Lys Arg
               100                 105                 110
   Thr Val Ala Ala Pro Ser Val Phe Ile Phe Pro Pro Ser Asp Glu Gln
           115                 120                 125
   Leu Lys Ser Gly Thr Ala Ser Val Val Cys Leu Leu Asn Asn Phe Tyr
       130                 135                 140
   Pro Arg Glu Ala Lys Val Gln Trp Lys Val Asp Asn Ala Leu Gln Ser
   145                 150                 155                 160
   Gly Asn Ser Gln Glu Ser Val Thr Glu Gln Asp Ser Lys Asp Ser Thr
                   165                 170                 175
   Tyr Ser Leu Ser Ser Thr Leu Thr Leu Ser Lys Ala Asp Tyr Glu Lys
               180                 185                 190
   His Lys Val Tyr Ala Cys Glu Val Thr His Gln Gly Leu Ser Ser Pro
           195                 200                 205
   Val Thr Lys Ser Phe Asn Arg Gly Glu Cys
       210                 215
<210> 33
   <211> 115
   <212> PRT
   <213> Synthetic
<400> 33
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Val Ser Ser Tyr
               20                  25                  30
   Val Met Ile Trp Val Arg Gln Ala Pro Gly Lys Gly Leu Glu Tyr Ile
           35                  40                  45
   Gly Ile Thr Trp Ser Ala Gly Thr Tyr Tyr Ala Ser Ser Ala Lys Gly
       50                  55                  60
   Arg Phe Thr Ile Ser Arg Asp Asn Ser Lys Asn Thr Leu Tyr Leu Gln
   65                  70                  75                  80
   Met Asn Ser Leu Arg Ala Glu Asp Thr Ala Val Tyr Tyr Cys Ala Gly
                   85                  90                  95
   Gly Gly Gly Ser Ile Tyr Asp Ile Trp Gly Gln Gly Thr Leu Val Thr
               100                 105                 110
   Val Ser Ser
           115
<210> 34
   <211> 445
   <212> PRT
   <213> Synthetic
<400> 34
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Val Ser Ser Tyr
               20                  25                  30
   Val Met Ile Trp Val Arg Gln Ala Pro Gly Lys Gly Leu Glu Tyr Ile
           35                  40                  45
   Gly Ile Thr Trp Ser Ala Gly Thr Tyr Tyr Ala Ser Ser Ala Lys Gly
       50                  55                  60
   Arg Phe Thr Ile Ser Arg Asp Asn Ser Lys Asn Thr Leu Tyr Leu Gln
   65                  70                  75                  80
   Met Asn Ser Leu Arg Ala Glu Asp Thr Ala Val Tyr Tyr Cys Ala Gly
                   85                  90                  95
   Gly Gly Gly Ser Ile Tyr Asp Ile Trp Gly Gln Gly Thr Leu Val Thr
               100                 105                 110
   Val Ser Ser Ala Ser Thr Lys Gly Pro Ser Val Phe Pro Leu Ala Pro
           115                 120                 125
   Ser Ser Lys Ser Thr Ser Gly Gly Thr Ala Ala Leu Gly Cys Leu Val
       130                 135                 140
   Lys Asp Tyr Phe Pro Glu Pro Val Thr Val Ser Trp Asn Ser Gly Ala
   145                 150                 155                 160
   Leu Thr Ser Gly Val His Thr Phe Pro Ala Val Leu Gln Ser Ser Gly
                   165                 170                 175
   Leu Tyr Ser Leu Ser Ser Val Val Thr Val Pro Ser Ser Ser Leu Gly
               180                 185                 190
   Thr Gln Thr Tyr Ile Cys Asn Val Asn His Lys Pro Ser Asn Thr Lys
           195                 200                 205
   Val Asp Lys Arg Val Glu Pro Lys Ser Cys Asp Lys Thr His Thr Cys
       210                 215                 220
   Pro Pro Cys Pro Ala Pro Glu Leu Leu Gly Gly Pro Ser Val Phe Leu
   225                 230                 235                 240
   Phe Pro Pro Lys Pro Lys Asp Thr Leu Met Ile Ser Arg Thr Pro Glu
                   245                 250                 255
   Val Thr Cys Val Val Val Asp Val Ser His Glu Asp Pro Glu Val Lys
               260                 265                 270
   Phe Asn Trp Tyr Val Asp Gly Val Glu Val His Asn Ala Lys Thr Lys
           275                 280                 285
   Pro Arg Glu Glu Gln Tyr Ala Ser Thr Tyr Arg Val Val Ser Val Leu
       290                 295                 300
   Thr Val Leu His Gln Asp Trp Leu Asn Gly Lys Glu Tyr Lys Cys Lys
   305                 310                 315                 320
   Val Ser Asn Lys Ala Leu Pro Ala Pro Ile Glu Lys Thr Ile Ser Lys
                   325                 330                 335
   Ala Lys Gly Gln Pro Arg Glu Pro Gln Val Tyr Thr Leu Pro Pro Ser
               340                 345                 350
   Arg Glu Glu Met Thr Lys Asn Gln Val Ser Leu Thr Cys Leu Val Lys
           355                 360                 365
   Gly Phe Tyr Pro Ser Asp Ile Ala Val Glu Trp Glu Ser Asn Gly Gln
       370                 375                 380
   Pro Glu Asn Asn Tyr Lys Thr Thr Pro Pro Val Leu Asp Ser Asp Gly
   385                 390                 395                 400
   Ser Phe Phe Leu Tyr Ser Lys Leu Thr Val Asp Lys Ser Arg Trp Gln
                   405                 410                 415
   Gln Gly Asn Val Phe Ser Cys Ser Val Met His Glu Ala Leu His Asn
               420                 425                 430
   His Tyr Thr Gln Lys Ser Leu Ser Leu Ser Pro Gly Lys
           435                 440                 445
<210> 35
   <211> 13
   <212> PRT
   <213> Oryctolagus cuniculus
<400> 35
   Gln Ser Ser Gln Ser Val Tyr Lys Asn Asn Tyr Leu Ser
   1 5 10
<210> 36
   <211> 7
   <212> PRT
   <213> Oryctolagus cuniculus
<400> 36
   Asp Ala Ser Asn Leu Pro Ser
   1 5
<210> 37
   <211> 11
   <212> PRT
   <213> Oryctolagus cuniculus
<400> 37
   Leu Gly Asp Tyr Asp Asp Asp Ala Asp Asn Ala
   1 5 10
<210> 38
   <211> 5
   <212> PRT
   <213> Oryctolagus cuniculus
<400> 38
   Ser Tyr Val Met Ile
   1 5
<210> 39
   <211> 15
   <212> PRT
   <213> Synthetic
<400> 39
   Ile Thr Trp Ser Ala Gly Thr Tyr Tyr Ala Ser Ser Ala Lys Gly
   1 5 10 15
<210> 40
   <211> 8
   <212> PRT
   <213> Oryctolagus cuniculus
<400> 40
   Gly Gly Gly Ser Ile Tyr Asp Ile
   1 5
<210> 41
   <211> 111
   <212> PRT
   <213> Oryctolagus cuniculus
<400> 41
   Ala Tyr Asp Met Thr Gln Thr Pro Ala Ser Val Glu Val Ala Val Gly
   1               5                   10                  15
   Gly Thr Val Thr Ile Lys Cys Gln Ala Ser Gln Ser Ile Tyr Ser Asn
               20                  25                  30
   Leu Ala Trp Tyr Gln Gln Arg Pro Gly Gln Pro Pro Lys Leu Leu Ile
           35                  40                  45
   Tyr Asp Ala Ser Thr Leu Glu Ser Gly Val Pro Ser Arg Phe Lys Gly
       50                  55                  60
   Ser Gly Ser Gly Thr Glu Tyr Thr Leu Thr Ile Ser Gly Val Glu Cys
   65                  70                  75                  80
   Ala Asp Ala Ala Ser Tyr Tyr Cys Gln Gln Gly Phe Thr Val Ser Asp
                   85                  90                  95
   Ile Asp Asn Ala Phe Gly Gly Gly Thr Glu Val Val Val Lys Arg
               100                 105                 110
<210> 42
   <211> 217
   <212> PRT
   <213> Synthetic
<400> 42
   Ala Tyr Asp Met Thr Gln Thr Pro Ala Ser Val Glu Val Ala Val Gly
   1               5                   10                  15
   Gly Thr Val Thr Ile Lys Cys Gln Ala Ser Gln Ser Ile Tyr Ser Asn
               20                  25                  30
   Leu Ala Trp Tyr Gln Gln Arg Pro Gly Gln Pro Pro Lys Leu Leu Ile
           35                  40                  45
   Tyr Asp Ala Ser Thr Leu Glu Ser Gly Val Pro Ser Arg Phe Lys Gly
       50                  55                  60
   Ser Gly Ser Gly Thr Glu Tyr Thr Leu Thr Ile Ser Gly Val Glu Cys
   65                  70                  75                  80
   Ala Asp Ala Ala Ser Tyr Tyr Cys Gln Gln Gly Phe Thr Val Ser Asp
   85 90 95
   Ile Asp Asn Ala Phe Gly Gly Gly Thr Glu Val Val Val Lys Arg Thr
   100 105 110
   Val Ala Ala Pro Ser Val Phe Ile Phe Pro Pro Ser Asp Glu Gln Leu
   115 120 125
   Lys Ser Gly Thr Ala Ser Val Val Cys Leu Leu Asn Asn Phe Tyr Pro
   130 135 140
   Arg Glu Ala Lys Val Gln Trp Lys Val Asp Asn Ala Leu Gln Ser Gly
   145 150 155 160
   Asn Ser Gln Glu Ser Val Thr Glu Gln Asp Ser Lys Asp Ser Thr Tyr
   165 170 175
   Ser Leu Ser Ser Thr Leu Thr Leu Ser Lys Ala Asp Tyr Glu Lys His
   180 185 190
   Lys Val Tyr Ala Cys Glu Val Thr His Gln Gly Leu Ser Ser Pro Val
   195 200 205
   Thr Lys Ser Phe Asn Arg Gly Glu Cys
   210 215
<210> 43
   <211> 118
   <212> PRT
   <213> Oryctolagus cuniculus
<400> 43
   Gln Ser Val Glu Glu Ser Gly Gly Arg Leu Val Thr Pro Gly Thr Pro
   1 5 10 15
   Leu Thr Leu Thr Cys Thr Val Ser Gly Phe Ser Leu Ser Asn Tyr Ala
   20 25 30
   Val Gly Trp Val Arg Gln Ala Pro Gly Lys Gly Leu Glu Trp Ile Gly
   35 40 45
   Ile Ile Gly Arg Asn Gly Asn Thr Trp Tyr Ala Ser Trp Ala Arg Gly
   50 55 60
   Arg Phe Thr Ile Ser Lys Thr Ser Thr Thr Val Asp Leu Lys Ile Thr
   65 70 75 80
   Ser Pro Thr Ser Glu Asp Thr Ala Thr Tyr Phe Cys Ala Arg Gly Tyr
   85 90 95
   Gly Arg Ser Val Ala Tyr Tyr Val Phe Asn Ile Trp Gly Pro Gly Thr
   100 105 110
   Leu Val Thr Val Ser Ser
   115
<210> 44
   <211> 448
   <212> PRT
   <213> Synthetic
<400> 44
   Gln Ser Val Glu Glu Ser Gly Gly Arg Leu Val Thr Pro Gly Thr Pro
   1 5 10 15
   Leu Thr Leu Thr Cys Thr Val Ser Gly Phe Ser Leu Ser Asn Tyr Ala
   20 25 30
   Val Gly Trp Val Arg Gln Ala Pro Gly Lys Gly Leu Glu Trp Ile Gly
   35 40 45
   Ile Ile Gly Arg Asn Gly Asn Thr Trp Tyr Ala Ser Trp Ala Arg Gly
   50 55 60
   Arg Phe Thr Ile Ser Lys Thr Ser Thr Thr Val Asp Leu Lys Ile Thr
   65 70 75 80
   Ser Pro Thr Ser Glu Asp Thr Ala Thr Tyr Phe Cys Ala Arg Gly Tyr
   85 90 95
   Gly Arg Ser Val Ala Tyr Tyr Val Phe Asn Ile Trp Gly Pro Gly Thr
   100 105 110
   Leu Val Thr Val Ser Ser Ala Ser Thr Lys Gly Pro Ser Val Phe Pro
   115 120 125
   Leu Ala Pro Ser Ser Lys Ser Thr Ser Gly Gly Thr Ala Ala Leu Gly
   130 135 140
   Cys Leu Val Lys Asp Tyr Phe Pro Glu Pro Val Thr Val Ser Trp Asn
   145 150 155 160
   Ser Gly Ala Leu Thr Ser Gly Val His Thr Phe Pro Ala Val Leu Gln
   165 170 175
   Ser Ser Gly Leu Tyr Ser Leu Ser Ser Val Val Thr Val Pro Ser Ser
               180                 185                 190
   Ser Leu Gly Thr Gln Thr Tyr Ile Cys Asn Val Asn His Lys Pro Ser
           195                 200                 205
   Asn Thr Lys Val Asp Lys Arg Val Glu Pro Lys Ser Cys Asp Lys Thr
       210                 215                 220
   His Thr Cys Pro Pro Cys Pro Ala Pro Glu Leu Leu Gly Gly Pro Ser
   225                 230                 235                 240
   Val Phe Leu Phe Pro Pro Lys Pro Lys Asp Thr Leu Met Ile Ser Arg
                   245                 250                 255
   Thr Pro Glu Val Thr Cys Val Val Val Asp Val Ser His Glu Asp Pro
               260                 265                 270
   Glu Val Lys Phe Asn Trp Tyr Val Asp Gly Val Glu Val His Asn Ala
           275                 280                 285
   Lys Thr Lys Pro Arg Glu Glu Gln Tyr Ala Ser Thr Tyr Arg Val Val
       290                 295                 300
   Ser Val Leu Thr Val Leu His Gln Asp Trp Leu Asn Gly Lys Glu Tyr
   305                 310                 315                 320
   Lys Cys Lys Val Ser Asn Lys Ala Leu Pro Ala Pro Ile Glu Lys Thr
                   325                 330                 335
   Ile Ser Lys Ala Lys Gly Gln Pro Arg Glu Pro Gln Val Tyr Thr Leu
               340                 345                 350
   Pro Pro Ser Arg Glu Glu Met Thr Lys Asn Gln Val Ser Leu Thr Cys
           355                 360                 365
   Leu Val Lys Gly Phe Tyr Pro Ser Asp Ile Ala Val Glu Trp Glu Ser
       370                 375                 380
   Asn Gly Gln Pro Glu Asn Asn Tyr Lys Thr Thr Pro Pro Val Leu Asp
   385                 390                 395                 400
   Ser Asp Gly Ser Phe Phe Leu Tyr Ser Lys Leu Thr Val Asp Lys Ser
                   405                 410                 415
   Arg Trp Gln Gln Gly Asn Val Phe Ser Cys Ser Val Met His Glu Ala
               420                 425                 430
   Leu His Asn His Tyr Thr Gln Lys Ser Leu Ser Leu Ser Pro Gly Lys
   435 440 445
<210> 45
   <211> 11
   <212> PRT
   <213> Oryctolagus cuniculus
<400> 45
   Gln Ala Ser Gln Ser Ile Tyr Ser Asn Leu Ala
   1 5 10
<210> 46
   <211> 7
   <212> PRT
   <213> Oryctolagus cuniculus
<400> 46
   Asp Ala Ser Thr Leu Glu Ser
   1 5
<210> 47
   <211> 12
   <212> PRT
   <213> Oryctolagus cuniculus
<400> 47
   Gln Gln Gly Phe Thr Val Ser Asp Ile Asp Asn Ala
   1 5 10
<210> 48
   <211> 5
   <212> PRT
   <213> Oryctolagus cuniculus
<400> 48
   Asn Tyr Ala Val Gly
   1 5
<210> 49
   <211> 16
   <212> PRT
   <213> Oryctolagus cuniculus
<400> 49
   Ile Ile Gly Arg Asn Gly Asn Thr Trp Tyr Ala Ser Trp Ala Arg Gly
   1 5 10 15
<210> 50
   <211> 13
   <212> PRT
   <213> Oryctolagus cuniculus
<400> 50
   Gly Tyr Gly Arg Ser Val Ala Tyr Tyr Val Phe Asn Ile
   1               5                   10
<210> 51
   <211> 111
   <212> PRT
   <213> Synthetic
<400> 51
   Asp Ile Gln Met Thr Gln Ser Pro Ser Thr Leu Ser Ala Ser Val Gly
   1               5                   10                  15
   Asp Arg Val Thr Ile Thr Cys Gln Ala Ser Gln Ser Ile Tyr Ser Asn
               20                  25                  30
   Leu Ala Trp Tyr Gln Gln Lys Pro Gly Lys Ala Pro Lys Leu Leu Ile
           35                  40                  45
   Tyr Asp Ala Ser Thr Leu Glu Ser Gly Val Pro Ser Arg Phe Ser Gly
       50                  55                  60
   Ser Gly Ser Gly Thr Glu Tyr Thr Leu Thr Ile Ser Ser Leu Gln Pro
   65                  70                  75                  80
   Asp Asp Phe Ala Thr Tyr Tyr Cys Gln Gln Gly Phe Thr Val Ser Asp
                   85                  90                  95
   Ile Asp Asn Ala Phe Gly Gly Gly Thr Lys Val Glu Ile Lys Arg
               100                 105                 110
<210> 52
   <211> 217
   <212> PRT
   <213> Synthetic
<400> 52
   Asp Ile Gln Met Thr Gln Ser Pro Ser Thr Leu Ser Ala Ser Val Gly
   1               5                   10                  15
   Asp Arg Val Thr Ile Thr Cys Gln Ala Ser Gln Ser Ile Tyr Ser Asn
               20                  25                  30
   Leu Ala Trp Tyr Gln Gln Lys Pro Gly Lys Ala Pro Lys Leu Leu Ile
           35                  40                  45
   Tyr Asp Ala Ser Thr Leu Glu Ser Gly Val Pro Ser Arg Phe Ser Gly
       50                  55                  60
   Ser Gly Ser Gly Thr Glu Tyr Thr Leu Thr Ile Ser Ser Leu Gln Pro
   65                  70                  75                  80
   Asp Asp Phe Ala Thr Tyr Tyr Cys Gln Gln Gly Phe Thr Val Ser Asp
                   85                  90                  95
   Ile Asp Asn Ala Phe Gly Gly Gly Thr Lys Val Glu Ile Lys Arg Thr
               100                 105                 110
   Val Ala Ala Pro Ser Val Phe Ile Phe Pro Pro Ser Asp Glu Gln Leu
           115                 120                 125
   Lys Ser Gly Thr Ala Ser Val Val Cys Leu Leu Asn Asn Phe Tyr Pro
       130                 135                 140
   Arg Glu Ala Lys Val Gln Trp Lys Val Asp Asn Ala Leu Gln Ser Gly
   145                 150                 155                 160
   Asn Ser Gln Glu Ser Val Thr Glu Gln Asp Ser Lys Asp Ser Thr Tyr
                   165                 170                 175
   Ser Leu Ser Ser Thr Leu Thr Leu Ser Lys Ala Asp Tyr Glu Lys His
               180                 185                 190
   Lys Val Tyr Ala Cys Glu Val Thr His Gln Gly Leu Ser Ser Pro Val
           195                 200                 205
   Thr Lys Ser Phe Asn Arg Gly Glu Cys
       210                 215
<210> 53
   <211> 121
   <212> PRT
   <213> Synthetic
<400> 53
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Val Ser Asn Tyr
               20                  25                  30
   Ala Val Gly Trp Val Arg Gln Ala Pro Gly Lys Gly Leu Glu Trp Val
           35                  40                  45
   Gly Ile Ile Gly Arg Asn Gly Asn Thr Trp Tyr Ala Ser Ser Ala Arg
       50                  55                  60
   Gly Arg Phe Thr Ile Ser Arg Asp Asn Ser Lys Asn Thr Leu Tyr Leu
   65                  70                  75                  80
   Gln Met Asn Ser Leu Arg Ala Glu Asp Thr Ala Val Tyr Tyr Cys Ala
                   85                  90                  95
   Arg Gly Tyr Gly Arg Ser Val Ala Tyr Tyr Val Phe Asn Ile Trp Gly
               100                 105                 110
   Pro Gly Thr Leu Val Thr Val Ser Ser
           115                 120
<210> 54
   <211> 451
   <212> PRT
   <213> Synthetic
<400> 54
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Val Ser Asn Tyr
               20                  25                  30
   Ala Val Gly Trp Val Arg Gln Ala Pro Gly Lys Gly Leu Glu Trp Val
           35                  40                  45
   Gly Ile Ile Gly Arg Asn Gly Asn Thr Trp Tyr Ala Ser Ser Ala Arg
       50                  55                  60
   Gly Arg Phe Thr Ile Ser Arg Asp Asn Ser Lys Asn Thr Leu Tyr Leu
   65                  70                  75                  80
   Gln Met Asn Ser Leu Arg Ala Glu Asp Thr Ala Val Tyr Tyr Cys Ala
                   85                  90                  95
   Arg Gly Tyr Gly Arg Ser Val Ala Tyr Tyr Val Phe Asn Ile Trp Gly
               100                 105                 110
   Pro Gly Thr Leu Val Thr Val Ser Ser Ala Ser Thr Lys Gly Pro Ser
           115                 120                 125
   Val Phe Pro Leu Ala Pro Ser Ser Lys Ser Thr Ser Gly Gly Thr Ala
       130                 135                 140
   Ala Leu Gly Cys Leu Val Lys Asp Tyr Phe Pro Glu Pro Val Thr Val
   145                 150                 155                 160
   Ser Trp Asn Ser Gly Ala Leu Thr Ser Gly Val His Thr Phe Pro Ala
                   165                 170                 175
   Val Leu Gln Ser Ser Gly Leu Tyr Ser Leu Ser Ser Val Val Thr Val
               180                 185                 190
   Pro Ser Ser Ser Leu Gly Thr Gln Thr Tyr Ile Cys Asn Val Asn His
           195                 200                 205
   Lys Pro Ser Asn Thr Lys Val Asp Lys Arg Val Glu Pro Lys Ser Cys
       210                 215                 220
   Asp Lys Thr His Thr Cys Pro Pro Cys Pro Ala Pro Glu Leu Leu Gly
   225                 230                 235                 240
   Gly Pro Ser Val Phe Leu Phe Pro Pro Lys Pro Lys Asp Thr Leu Met
                   245                 250                 255
   Ile Ser Arg Thr Pro Glu Val Thr Cys Val Val Val Asp Val Ser His
               260                 265                 270
   Glu Asp Pro Glu Val Lys Phe Asn Trp Tyr Val Asp Gly Val Glu Val
           275                 280                 285
   His Asn Ala Lys Thr Lys Pro Arg Glu Glu Gln Tyr Ala Ser Thr Tyr
       290                 295                 300
   Arg Val Val Ser Val Leu Thr Val Leu His Gln Asp Trp Leu Asn Gly
   305                 310                 315                 320
   Lys Glu Tyr Lys Cys Lys Val Ser Asn Lys Ala Leu Pro Ala Pro Ile
                   325                 330                 335
   Glu Lys Thr Ile Ser Lys Ala Lys Gly Gln Pro Arg Glu Pro Gln Val
               340                 345                 350
   Tyr Thr Leu Pro Pro Ser Arg Glu Glu Met Thr Lys Asn Gln Val Ser
           355                 360                 365
   Leu Thr Cys Leu Val Lys Gly Phe Tyr Pro Ser Asp Ile Ala Val Glu
       370                 375                 380
   Trp Glu Ser Asn Gly Gln Pro Glu Asn Asn Tyr Lys Thr Thr Pro Pro
   385                 390                 395                 400
   Val Leu Asp Ser Asp Gly Ser Phe Phe Leu Tyr Ser Lys Leu Thr Val
   405 410 415
   Asp Lys Ser Arg Trp Gln Gln Gly Asn Val Phe Ser Cys Ser Val Met
   420 425 430
   His Glu Ala Leu His Asn His Tyr Thr Gln Lys Ser Leu Ser Leu Ser
   435 440 445
   Pro Gly Lys
   450
<210> 55
   <211> 11
   <212> PRT
   <213> Oryctolagus cuniculus
<400> 55
   Gln Ala Ser Gln Ser Ile Tyr Ser Asn Leu Ala
   1 5 10
<210> 56
   <211> 7
   <212> PRT
   <213> Oryctolagus cuniculus
<400> 56
   Asp Ala Ser Thr Leu Glu Ser
   1 5
<210> 57
   <211> 12
   <212> PRT
   <213> Oryctolagus cuniculus
<400> 57
   Gln Gln Gly Phe Thr Val Ser Asp Ile Asp Asn Ala
   1 5 10
<210> 58
   <211> 5
   <212> PRT
   <213> Oryctolagus cuniculus
<400> 58
   Asn Tyr Ala Val Gly
   1 5
<210> 59
   <211> 16
   <212> PRT
   <213> Synthetic
<400> 59
   Ile Ile Gly Arg Asn Gly Asn Thr Trp Tyr Ala Ser Ser Ala Arg Gly
   1               5                   10                  15
<210> 60
   <211> 13
   <212> PRT
   <213> Oryctolagus cuniculus
<400> 60
   Gly Tyr Gly Arg Ser Val Ala Tyr Tyr Val Phe Asn Ile
   1               5                   10
<210> 61
   <211> 112
   <212> PRT
   <213> Oryctolagus cuniculus
<400> 61
   Ala Asp Val Val Met Thr Gln Thr Pro Ala Ser Val Ser Gln Pro Val
   1               5                   10                  15
   Gly Gly Thr Val Thr Ile Lys Cys Gln Ala Ser Glu Asp Ile Tyr Asn
               20                  25                  30
   Leu Leu Ala Trp Tyr Gln Gln Lys Pro Gly Gln Pro Pro Lys Leu Leu
           35                  40                  45
   Ile Tyr Ser Ala Ser Thr Leu Ala Ser Gly Val Pro Ser Arg Phe Lys
       50                  55                  60
   Gly Ser Gly Ser Gly Thr Glu Tyr Thr Leu Thr Ile Ser Gly Leu Glu
   65                  70                  75                  80
   Cys Ala Asp Ala Ala Thr Tyr Tyr Cys Gln Asn Asn Tyr Leu Val Thr
                   85                  90                  95
   Thr Tyr Gly Val Ala Phe Gly Gly Gly Thr Glu Val Val Val Lys Arg
               100                 105                 110
<210> 62
   <211> 218
   <212> PRT
   <213> Synthetic
<400> 62
   Ala Asp Val Val Met Thr Gln Thr Pro Ala Ser Val Ser Gln Pro Val
   1 5 10 15
   Gly Gly Thr Val Thr Ile Lys Cys Gln Ala Ser Glu Asp Ile Tyr Asn
   20 25 30
   Leu Leu Ala Trp Tyr Gln Gln Lys Pro Gly Gln Pro Pro Lys Leu Leu
   35 40 45
   Ile Tyr Ser Ala Ser Thr Leu Ala Ser Gly Val Pro Ser Arg Phe Lys
   50 55 60
   Gly Ser Gly Ser Gly Thr Glu Tyr Thr Leu Thr Ile Ser Gly Leu Glu
   65 70 75 80
   Cys Ala Asp Ala Ala Thr Tyr Tyr Cys Gln Asn Asn Tyr Leu Val Thr
   85 90 95
   Thr Tyr Gly Val Ala Phe Gly Gly Gly Thr Glu Val Val Val Lys Arg
   100 105 110
   Thr Val Ala Ala Pro Ser Val Phe Ile Phe Pro Pro Ser Asp Glu Gln
   115 120 125
   Leu Lys Ser Gly Thr Ala Ser Val Val Cys Leu Leu Asn Asn Phe Tyr
   130 135 140
   Pro Arg Glu Ala Lys Val Gln Trp Lys Val Asp Asn Ala Leu Gln Ser
   145 150 155 160
   Gly Asn Ser Gln Glu Ser Val Thr Glu Gln Asp Ser Lys Asp Ser Thr
   165 170 175
   Tyr Ser Leu Ser Ser Thr Leu Thr Leu Ser Lys Ala Asp Tyr Glu Lys
   180 185 190
   His Lys Val Tyr Ala Cys Glu Val Thr His Gln Gly Leu Ser Ser Pro
   195 200 205
   Val Thr Lys Ser Phe Asn Arg Gly Glu Cys
   210 215
<210> 63
   <211> 120
   <212> PRT
   <213> Oryctolagus cuniculus
<400> 63
   Gln Glu Gln Leu Lys Glu Ser Gly Gly Arg Leu Val Thr Pro Gly Thr
   1               5                   10                  15
   Pro Leu Thr Leu Thr Cys Thr Val Ser Gly Phe Ser Leu Ser Ser Tyr
               20                  25                  30
   Ala Met Ile Trp Val Arg Gln Ala Pro Gly Lys Gly Leu Glu Tyr Ile
           35                  40                  45
   Gly Tyr Ile Asp Thr Asp Thr Ser Ala Tyr Tyr Ala Ser Trp Val Lys
       50                  55                  60
   Gly Arg Phe Thr Ile Ser Arg Thr Ser Thr Thr Val Asp Leu Lys Ile
   65                  70                  75                  80
   Thr Ser Pro Thr Thr Glu Asp Thr Ala Thr Tyr Phe Cys Ala Arg Ser
                   85                  90                  95
   Tyr Ala Ala Tyr Gly Gly Tyr Pro Ala Thr Phe Asp Pro Trp Gly Pro
               100                 105                 110
   Gly Thr Leu Val Thr Val Ser Ser
           115                 120
<210> 64
   <211> 450
   <212> PRT
   <213> Synthetic
<400> 64
<210> 65
   <211> 11
   <212> PRT
   <213> Oryctolagus cuniculus
<400> 65
   Gln Ala Ser Glu Asp Ile Tyr Asn Leu Leu Ala
   1               5                   10
<210> 66
   <211> 7
   <212> PRT
   <213> Oryctolagus cuniculus
<400> 66
   Ser Ala Ser Thr Leu Ala Ser
   1               5
<210> 67
   <211> 12
   <212> PRT
   <213> Oryctolagus cuniculus
<400> 67
   Gln Asn Asn Tyr Leu Val Thr Thr Tyr Gly Val Ala
   1               5                   10
<210> 68
   <211> 5
   <212> PRT
   <213> Oryctolagus cuniculus
<400> 68
   Ser Tyr Ala Met Ile
   1               5
<210> 69
   <211> 16
   <212> PRT
   <213> Oryctolagus cuniculus
<400> 69
   Tyr Ile Asp Thr Asp Thr Ser Ala Tyr Tyr Ala Ser Trp Val Lys Gly
   1               5                   10                  15
<210> 70
   <211> 14
   <212> PRT
   <213> Oryctolagus cuniculus
<400> 70
   Ser Tyr Ala Ala Tyr Gly Gly Tyr Pro Ala Thr Phe Asp Pro
   1               5                   10
<210> 71
   <211> 111
   <212> PRT
   <213> Synthetic
<400> 71
   Asp Ile Gln Met Thr Gln Ser Pro Ser Ser Leu Ser Ala Ser Val Gly
   1               5                   10                  15
   Asp Arg Val Thr Ile Thr Cys Gln Ala Ser Glu Asp Ile Tyr Asn Leu
               20                  25                  30
   Leu Ala Trp Tyr Gln Gln Lys Pro Gly Lys Val Pro Lys Leu Leu Ile
           35                  40                  45
   Tyr Ser Ala Ser Thr Leu Ala Ser Gly Val Pro Ser Arg Phe Ser Gly
       50                  55                  60
   Ser Gly Ser Gly Thr Asp Tyr Thr Leu Thr Ile Ser Ser Leu Gln Pro
   65                  70                  75                  80
   Glu Asp Val Ala Thr Tyr Tyr Cys Gln Asn Asn Tyr Leu Val Thr Thr
                   85                  90                  95
   Tyr Gly Val Ala Phe Gly Gly Gly Thr Lys Val Glu Ile Lys Arg
             100                 105                 110
<210> 72
   <211> 217
   <212> PRT
   <213> Synthetic
<400> 72
   Asp Ile Gln Met Thr Gln Ser Pro Ser Ser Leu Ser Ala Ser Val Gly
   1               5                   10                  15
   Asp Arg Val Thr Ile Thr Cys Gln Ala Ser Glu Asp Ile Tyr Asn Leu
               20                  25                  30
   Leu Ala Trp Tyr Gln Gln Lys Pro Gly Lys Val Pro Lys Leu Leu Ile
           35                  40                  45
   Tyr Ser Ala Ser Thr Leu Ala Ser Gly Val Pro Ser Arg Phe Ser Gly
       50                  55                  60
   Ser Gly Ser Gly Thr Asp Tyr Thr Leu Thr Ile Ser Ser Leu Gln Pro
   65                  70                  75                  80
   Glu Asp Val Ala Thr Tyr Tyr Cys Gln Asn Asn Tyr Leu Val Thr Thr
                   85                  90                  95
   Tyr Gly Val Ala Phe Gly Gly Gly Thr Lys Val Glu Ile Lys Arg Thr
               100                 105                 110
   Val Ala Ala Pro Ser Val Phe Ile Phe Pro Pro Ser Asp Glu Gln Leu
           115                 120                 125
   Lys Ser Gly Thr Ala Ser Val Val Cys Leu Leu Asn Asn Phe Tyr Pro
       130                 135                 140
   Arg Glu Ala Lys Val Gln Trp Lys Val Asp Asn Ala Leu Gln Ser Gly
   145                 150                 155                 160
   Asn Ser Gln Glu Ser Val Thr Glu Gln Asp Ser Lys Asp Ser Thr Tyr
                   165                 170                 175
   Ser Leu Ser Ser Thr Leu Thr Leu Ser Lys Ala Asp Tyr Glu Lys His
               180                 185                 190
   Lys Val Tyr Ala Cys Glu Val Thr His Gln Gly Leu Ser Ser Pro Val
           195                 200                 205
   Thr Lys Ser Phe Asn Arg Gly Glu Cys
                       210                 215
<210> 73
   <211> 122
   <212> PRT
   <213> Synthetic
<400> 73
   Gln Val Gln Leu Val Glu Ser Gly Gly Gly Val Val Gln Pro Gly Arg
   1               5                   10                  15
   Ser Leu Arg Leu Ser Cys Ala Ala Ser Gly Phe Thr Phe Ser Ser Tyr
               20                  25                  30
   Ala Met Ile Trp Val Arg Gln Ala Pro Gly Lys Gly Leu Glu Tyr Ile
           35                  40                  45
   Gly Tyr Ile Asp Thr Asp Thr Ser Ala Tyr Tyr Ala Ser Ser Val Lys
       50                  55                  60
   Gly Arg Phe Thr Ile Ser Arg Asp Asn Ser Lys Asn Thr Leu Tyr Leu
   65                  70                  75                  80
   Gln Met Ser Ser Leu Arg Ala Glu Asp Thr Ala Val Tyr Tyr Cys Ala
                   85                  90                  95
   Arg Ser Tyr Ala Ala Tyr Gly Gly Tyr Pro Ala Thr Phe Asp Pro Trp
               100                 105                 110
   Gly Gln Gly Thr Leu Val Thr Val Ser Ser
           115                 120
<210> 74
   <211> 452
   <212> PRT
   <213> Synthetic
<400> 74
   Gln Val Gln Leu Val Glu Ser Gly Gly Gly Val Val Gln Pro Gly Arg
   1               5                   10                  15
   Ser Leu Arg Leu Ser Cys Ala Ala Ser Gly Phe Thr Phe Ser Ser Tyr
               20                  25                  30
   Ala Met Ile Trp Val Arg Gln Ala Pro Gly Lys Gly Leu Glu Tyr Ile
           35                  40                  45
   Gly Tyr Ile Asp Thr Asp Thr Ser Ala Tyr Tyr Ala Ser Ser Val Lys
       50                  55                  60
   Gly Arg Phe Thr Ile Ser Arg Asp Asn Ser Lys Asn Thr Leu Tyr Leu
   65 70 75 80
   Gln Met Ser Ser Leu Arg Ala Glu Asp Thr Ala Val Tyr Tyr Cys Ala
   85 90 95
   Arg Ser Tyr Ala Ala Tyr Gly Gly Tyr Pro Ala Thr Phe Asp Pro Trp
   100 105 110
   Gly Gln Gly Thr Leu Val Thr Val Ser Ser Ala Ser Thr Lys Gly Pro
   115 120 125
   Ser Val Phe Pro Leu Ala Pro Ser Ser Lys Ser Thr Ser Gly Gly Thr
   130 135 140
   Ala Ala Leu Gly Cys Leu Val Lys Asp Tyr Phe Pro Glu Pro Val Thr
   145 150 155 160
   Val Ser Trp Asn Ser Gly Ala Leu Thr Ser Gly Val His Thr Phe Pro
   165 170 175
   Ala Val Leu Gln Ser Ser Gly Leu Tyr Ser Leu Ser Ser Val Val Thr
   180 185 190
   Val Pro Ser Ser Ser Leu Gly Thr Gln Thr Tyr Ile Cys Asn Val Asn
   195 200 205
   His Lys Pro Ser Asn Thr Lys Val Asp Lys Arg Val Glu Pro Lys Ser
   210 215 220
   Cys Asp Lys Thr His Thr Cys Pro Pro Cys Pro Ala Pro Glu Leu Leu
   225 230 235 240
   Gly Gly Pro Ser Val Phe Leu Phe Pro Pro Lys Pro Lys Asp Thr Leu
   245 250 255
   Met Ile Ser Arg Thr Pro Glu Val Thr Cys Val Val Val Asp Val Ser
   260 265 270
   His Glu Asp Pro Glu Val Lys Phe Asn Trp Tyr Val Asp Gly Val Glu
   275 280 285
   Val His Asn Ala Lys Thr Lys Pro Arg Glu Glu Gln Tyr Ala Ser Thr
   290 295 300
   Tyr Arg Val Val Ser Val Leu Thr Val Leu His Gln Asp Trp Leu Asn
   305 310 315 320
   Gly Lys Glu Tyr Lys Cys Lys Val Ser Asn Lys Ala Leu Pro Ala Pro
   325 330 335
   Ile Glu Lys Thr Ile Ser Lys Ala Lys Gly Gln Pro Arg Glu Pro Gln
   340 345 350
   Val Tyr Thr Leu Pro Pro Ser Arg Glu Glu Met Thr Lys Asn Gln Val
   355 360 365
   Ser Leu Thr Cys Leu Val Lys Gly Phe Tyr Pro Ser Asp Ile Ala Val
   370 375 380
   Glu Trp Glu Ser Asn Gly Gln Pro Glu Asn Asn Tyr Lys Thr Thr Pro
   385 390 395 400
   Pro Val Leu Asp Ser Asp Gly Ser Phe Phe Leu Tyr Ser Lys Leu Thr
   405 410 415
   Val Asp Lys Ser Arg Trp Gln Gln Gly Asn Val Phe Ser Cys Ser Val
   420 425 430
   Met His Glu Ala Leu His Asn His Tyr Thr Gln Lys Ser Leu Ser Leu
   435 440 445
   Ser Pro Gly Lys
   450
<210> 75
   <211> 11
   <212> PRT
   <213> Oryctolagus cuniculus
<400> 75
   Gln Ala Ser Glu Asp Ile Tyr Asn Leu Leu Ala
   1 5 10
<210> 76
   <211> 7
   <212> PRT
   <213> Oryctolagus cuniculus
<400> 76
   Ser Ala Ser Thr Leu Ala Ser
   1 5
<210> 77
   <211> 12
   <212> PRT
   <213> Oryctolagus cuniculus
<400> 77
   Gln Asn Asn Tyr Leu Val Thr Thr Tyr Gly Val Ala
   1 5 10
<210> 78
   <211> 5
   <212> PRT
   <213> Oryctolagus cuniculus
<400> 78
   Ser Tyr Ala Met Ile
   1 5
<210> 79
   <211> 16
   <212> PRT
   <213> Synthetic
<400> 79
   Tyr Ile Asp Thr Asp Thr Ser Ala Tyr Tyr Ala Ser Ser Val Lys Gly
   1 5 10 15
<210> 80
   <211> 14
   <212> PRT
   <213> Oryctolagus cuniculus
<400> 80
   Ser Tyr Ala Ala Tyr Gly Gly Tyr Pro Ala Thr Phe Asp Pro
   1 5 10
<210> 81
   <211> 111
   <212> PRT
   <213> Oryctolagus cuniculus
<400> 81
   Ala Tyr Asp Met Thr Gln Thr Pro Ala Ser Val Ser Ala Ala Val Gly
   1               5                   10                  15
   Gly Thr Val Thr Ile Lys Cys Gln Ala Ser Glu Asn Ile Gly Ser Tyr
               20                  25                  30
   Leu Ala Trp Tyr Gln Gln Lys Pro Gly Gln Pro Pro Glu Leu Leu Ile
           35                  40                  45
   Tyr Arg Ala Ser Thr Leu Ala Ser Gly Val Pro Ser Arg Phe Lys Gly
       50                  55                  60
   Ser Gly Ser Gly Thr Gln Phe Thr Leu Thr Ile Ser Gly Val Glu Cys
   65                  70                  75                  80
   Ala Asp Ala Ala Thr Tyr Tyr Cys Gln Gln Gly Tyr Asn Ser Glu Asn
                   85                  90                  95
   Leu Asp Asn Ala Phe Gly Gly Gly Thr Glu Val Val Val Lys Arg
               100                 105                 110
<210> 82
   <211> 217
   <212> PRT
   <213> Synthetic
<400> 82
   Ala Tyr Asp Met Thr Gln Thr Pro Ala Ser Val Ser Ala Ala Val Gly
   1               5                   10                  15
   Gly Thr Val Thr Ile Lys Cys Gln Ala Ser Glu Asn Ile Gly Ser Tyr
               20                  25                  30
   Leu Ala Trp Tyr Gln Gln Lys Pro Gly Gln Pro Pro Glu Leu Leu Ile
           35                  40                  45
   Tyr Arg Ala Ser Thr Leu Ala Ser Gly Val Pro Ser Arg Phe Lys Gly
       50                  55                  60
   Ser Gly Ser Gly Thr Gln Phe Thr Leu Thr Ile Ser Gly Val Glu Cys
   65                  70                  75                  80
   Ala Asp Ala Ala Thr Tyr Tyr Cys Gln Gln Gly Tyr Asn Ser Glu Asn
                   85                  90                  95
   Leu Asp Asn Ala Phe Gly Gly Gly Thr Glu Val Val Val Lys Arg Thr
               100                 105                 110
   Val Ala Ala Pro Ser Val Phe Ile Phe Pro Pro Ser Asp Glu Gln Leu
           115                 120                 125
   Lys Ser Gly Thr Ala Ser Val Val Cys Leu Leu Asn Asn Phe Tyr Pro
       130                 135                 140
   Arg Glu Ala Lys Val Gln Trp Lys Val Asp Asn Ala Leu Gln Ser Gly
   145                 150                 155                 160
   Asn Ser Gln Glu Ser Val Thr Glu Gln Asp Ser Lys Asp Ser Thr Tyr
                   165                 170                 175
   Ser Leu Ser Ser Thr Leu Thr Leu Ser Lys Ala Asp Tyr Glu Lys His
               180                 185                 190
   Lys Val Tyr Ala Cys Glu Val Thr His Gln Gly Leu Ser Ser Pro Val
           195                 200                 205
   Thr Lys Ser Phe Asn Arg Gly Glu Cys
       210                 215
<210> 83
   <211> 115
   <212> PRT
   <213> Oryctolagus cuniculus
<400> 83
   Gln Ser Val Glu Glu Ser Gly Gly Arg Leu Val Thr Pro Gly Thr Pro
   1               5                   10                  15
   Leu Thr Leu Thr Cys Thr Val Ser Gly Ile Asp Leu Ser Met Tyr Ser
               20                  25                  30
   Met Gly Trp Val Arg Gln Ala Pro Gly Lys Gly Leu Glu Tyr Ile Gly
           35                  40                  45
   Trp Ile Ser Tyr Gly Gly Thr Ala Tyr Tyr Ala Ser Trp Ala Lys Gly
       50                  55                  60
   Arg Phe Thr Ile Ser Lys Thr Ser Thr Thr Val Glu Leu Lys Ile Thr
   65                  70                  75                  80
   Ser Pro Thr Ile Glu Asp Thr Ala Thr Tyr Phe Cys Ala Arg Glu Thr
                   85                  90                  95
   Pro Val Asn Tyr Tyr Leu Asp Ile Trp Gly Gln Gly Thr Leu Val Thr
               100                 105                 110
   Val Ser Ser
           115
<210> 84
   <211> 445
   <212> PRT
   <213> Synthetic
<400> 84
   Gln Ser Val Glu Glu Ser Gly Gly Arg Leu Val Thr Pro Gly Thr Pro
   1               5                   10                  15
   Leu Thr Leu Thr Cys Thr Val Ser Gly Ile Asp Leu Ser Met Tyr Ser
               20                  25                  30
   Met Gly Trp Val Arg Gln Ala Pro Gly Lys Gly Leu Glu Tyr Ile Gly
           35                  40                  45
   Trp Ile Ser Tyr Gly Gly Thr Ala Tyr Tyr Ala Ser Trp Ala Lys Gly
       50                  55                  60
   Arg Phe Thr Ile Ser Lys Thr Ser Thr Thr Val Glu Leu Lys Ile Thr
   65                  70                  75                  80
   Ser Pro Thr Ile Glu Asp Thr Ala Thr Tyr Phe Cys Ala Arg Glu Thr
                   85                  90                  95
   Pro Val Asn Tyr Tyr Leu Asp Ile Trp Gly Gln Gly Thr Leu Val Thr
               100                 105                 110
   Val Ser Ser Ala Ser Thr Lys Gly Pro Ser Val Phe Pro Leu Ala Pro
           115                 120                 125
   Ser Ser Lys Ser Thr Ser Gly Gly Thr Ala Ala Leu Gly Cys Leu Val
       130                 135                 140
   Lys Asp Tyr Phe Pro Glu Pro Val Thr Val Ser Trp Asn Ser Gly Ala
   145                 150                 155                 160
   Leu Thr Ser Gly Val His Thr Phe Pro Ala Val Leu Gln Ser Ser Gly
                   165                 170                 175
   Leu Tyr Ser Leu Ser Ser Val Val Thr Val Pro Ser Ser Ser Leu Gly
               180                 185                 190
   Thr Gln Thr Tyr Ile Cys Asn Val Asn His Lys Pro Ser Asn Thr Lys
           195                 200                 205
   Val Asp Lys Arg Val Glu Pro Lys Ser Cys Asp Lys Thr His Thr Cys
       210                 215                 220
   Pro Pro Cys Pro Ala Pro Glu Leu Leu Gly Gly Pro Ser Val Phe Leu
   225                 230                 235                 240
   Phe Pro Pro Lys Pro Lys Asp Thr Leu Met Ile Ser Arg Thr Pro Glu
                   245                 250                 255
   Val Thr Cys Val Val Val Asp Val Ser His Glu Asp Pro Glu Val Lys
               260                 265                 270
   Phe Asn Trp Tyr Val Asp Gly Val Glu Val His Asn Ala Lys Thr Lys
           275                 280                 285
   Pro Arg Glu Glu Gln Tyr Ala Ser Thr Tyr Arg Val Val Ser Val Leu
       290                 295                 300
   Thr Val Leu His Gln Asp Trp Leu Asn Gly Lys Glu Tyr Lys Cys Lys
   305                 310                 315                 320
   Val Ser Asn Lys Ala Leu Pro Ala Pro Ile Glu Lys Thr Ile Ser Lys
                   325                 330                 335
   Ala Lys Gly Gln Pro Arg Glu Pro Gln Val Tyr Thr Leu Pro Pro Ser
               340                 345                 350
   Arg Glu Glu Met Thr Lys Asn Gln Val Ser Leu Thr Cys Leu Val Lys
           355                 360                 365
   Gly Phe Tyr Pro Ser Asp Ile Ala Val Glu Trp Glu Ser Asn Gly Gln
       370                 375                 380
   Pro Glu Asn Asn Tyr Lys Thr Thr Pro Pro Val Leu Asp Ser Asp Gly
   385                 390                 395                 400
   Ser Phe Phe Leu Tyr Ser Lys Leu Thr Val Asp Lys Ser Arg Trp Gln
                   405                 410                 415
   Gln Gly Asn Val Phe Ser Cys Ser Val Met His Glu Ala Leu His Asn
               420                 425                 430
   His Tyr Thr Gln Lys Ser Leu Ser Leu Ser Pro Gly Lys
           435                 440                 445
<210> 85
   <211> 11
   <212> PRT
   <213> Oryctolagus cuniculus
<400> 85
   Gln Ala Ser Glu Asn Ile Gly Ser Tyr Leu Ala
   1               5                   10
<210> 86
   <211> 7
   <212> PRT
   <213> Oryctolagus cuniculus
<400> 86
   Arg Ala Ser Thr Leu Ala Ser
   1 5
<210> 87
   <211> 12
   <212> PRT
   <213> Oryctolagus cuniculus
<400> 87
   Gln Gln Gly Tyr Asn Ser Glu Asn Leu Asp Asn Ala
   1 5 10
<210> 88
   <211> 5
   <212> PRT
   <213> Oryctolagus cuniculus
<400> 88
   Met Tyr Ser Met Gly
   1 5
<210> 89
   <211> 16
   <212> PRT
   <213> Oryctolagus cuniculus
<400> 89
   Trp Ile Ser Tyr Gly Gly Thr Ala Tyr Tyr Ala Ser Trp Ala Lys Gly
   1 5 10 15
<210> 90
   <211> 10
   <212> PRT
   <213> Oryctolagus cuniculus
<400> 90
   Glu Thr Pro Val Asn Tyr Tyr Leu Asp Ile
   1 5 10
<210> 91
   <211> 111
   <212> PRT
   <213> Synthetic
<400> 91
   Ala Tyr Asp Met Thr Gln Ser Pro Ser Ser Leu Ser Ala Ser Val Gly
   1 5 10 15
   Asp Arg Val Thr Ile Thr Cys Gln Ala Ser Glu Asn Ile Gly Ser Tyr
   20 25 30
   Leu Ala Trp Tyr Gln Gln Lys Pro Gly Lys Val Pro Lys Leu Leu Ile
           35                  40                  45
   Tyr Arg Ala Ser Thr Leu Ala Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Val Ala Thr Tyr Tyr Cys Gln Gln Gly Tyr Asn Ser Glu Asn
                   85                  90                  95
   Leu Asp Asn Ala Phe Gly Gly Gly Thr Lys Val Glu Ile Lys Arg
               100                 105                 110
<210> 92
   <211> 217
   <212> PRT
   <213> Synthetic
<400> 92
   Ala Tyr Asp Met Thr Gln Ser Pro Ser Ser Leu Ser Ala Ser Val Gly
   1               5                   10                  15
   Asp Arg Val Thr Ile Thr Cys Gln Ala Ser Glu Asn Ile Gly Ser Tyr
               20                  25                  30
   Leu Ala Trp Tyr Gln Gln Lys Pro Gly Lys Val Pro Lys Leu Leu Ile
           35                  40                  45
   Tyr Arg Ala Ser Thr Leu Ala Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Val Ala Thr Tyr Tyr Cys Gln Gln Gly Tyr Asn Ser Glu Asn
                   85                  90                  95
   Leu Asp Asn Ala Phe Gly Gly Gly Thr Lys Val Glu Ile Lys Arg Thr
               100                 105                 110
   Val Ala Ala Pro Ser Val Phe Ile Phe Pro Pro Ser Asp Glu Gln Leu
           115                 120                 125
   Lys Ser Gly Thr Ala Ser Val Val Cys Leu Leu Asn Asn Phe Tyr Pro
       130                 135                 140
   Arg Glu Ala Lys Val Gln Trp Lys Val Asp Asn Ala Leu Gln Ser Gly
   145                 150                 155                 160
   Asn Ser Gln Glu Ser Val Thr Glu Gln Asp Ser Lys Asp Ser Thr Tyr
                   165                 170                 175
   Ser Leu Ser Ser Thr Leu Thr Leu Ser Lys Ala Asp Tyr Glu Lys His
               180                 185                 190
   Lys Val Tyr Ala Cys Glu Val Thr His Gln Gly Leu Ser Ser Pro Val
           195                 200                 205
   Thr Lys Ser Phe Asn Arg Gly Glu Cys
       210                 215
<210> 93
   <211> 118
   <212> PRT
   <213> Synthetic
<400> 93
   Gln Val Gln Leu Val Glu Ser Gly Gly Gly Val Val Gln Pro Gly Arg
   1               5                   10                  15
   Ser Leu Arg Leu Ser Cys Ala Ala Ser Gly Phe Thr Phe Ser Met Tyr
               20                  25                  30
   Ser Met Gly Trp Val Arg Gln Ala Pro Gly Lys Gly Leu Glu Tyr Ile
           35                  40                  45
   Gly Trp Ile Ser Tyr Gly Gly Thr Ala Tyr Tyr Ala Ser Ser Ala Lys
       50                  55                  60
   Gly Arg Phe Thr Ile Ser Arg Asp Asn Ser Lys Asn Thr Leu Tyr Leu
   65                  70                  75                  80
   Gln Met Ser Ser Leu Arg Ala Glu Asp Thr Ala Val Tyr Tyr Cys Ala
                   85                  90                  95
   Arg Glu Thr Pro Val Asn Tyr Tyr Leu Asp Ile Trp Gly Gln Gly Thr
               100                 105                 110
   Leu Val Thr Val Ser Ser
           115
<210> 94
   <211> 448
   <212> PRT
   <213> Synthetic
<400> 94
   Gln Val Gln Leu Val Glu Ser Gly Gly Gly Val Val Gln Pro Gly Arg
   1               5                   10                  15
   Ser Leu Arg Leu Ser Cys Ala Ala Ser Gly Phe Thr Phe Ser Met Tyr
               20                  25                  30
   Ser Met Gly Trp Val Arg Gln Ala Pro Gly Lys Gly Leu Glu Tyr Ile
           35                  40                  45
   Gly Trp Ile Ser Tyr Gly Gly Thr Ala Tyr Tyr Ala Ser Ser Ala Lys
       50                  55                  60
   Gly Arg Phe Thr Ile Ser Arg Asp Asn Ser Lys Asn Thr Leu Tyr Leu
   65                  70                  75                  80
   Gln Met Ser Ser Leu Arg Ala Glu Asp Thr Ala Val Tyr Tyr Cys Ala
                   85                  90                  95
   Arg Glu Thr Pro Val Asn Tyr Tyr Leu Asp Ile Trp Gly Gln Gly Thr
               100                 105                 110
   Leu Val Thr Val Ser Ser Ala Ser Thr Lys Gly Pro Ser Val Phe Pro
           115                 120                 125
   Leu Ala Pro Ser Ser Lys Ser Thr Ser Gly Gly Thr Ala Ala Leu Gly
       130                 135                 140
   Cys Leu Val Lys Asp Tyr Phe Pro Glu Pro Val Thr Val Ser Trp Asn
   145                 150                 155                 160
   Ser Gly Ala Leu Thr Ser Gly Val His Thr Phe Pro Ala Val Leu Gln
                   165                 170                 175
   Ser Ser Gly Leu Tyr Ser Leu Ser Ser Val Val Thr Val Pro Ser Ser
               180                 185                 190
   Ser Leu Gly Thr Gln Thr Tyr Ile Cys Asn Val Asn His Lys Pro Ser
           195                 200                 205
   Asn Thr Lys Val Asp Lys Arg Val Glu Pro Lys Ser Cys Asp Lys Thr
       210                 215                 220
   His Thr Cys Pro Pro Cys Pro Ala Pro Glu Leu Leu Gly Gly Pro Ser
   225                 230                 235                 240
   Val Phe Leu Phe Pro Pro Lys Pro Lys Asp Thr Leu Met Ile Ser Arg
   245 250 255
   Thr Pro Glu Val Thr Cys Val Val Val Asp Val Ser His Glu Asp Pro
   260 265 270
   Glu Val Lys Phe Asn Trp Tyr Val Asp Gly Val Glu Val His Asn Ala
   275 280 285
   Lys Thr Lys Pro Arg Glu Glu Gln Tyr Ala Ser Thr Tyr Arg Val Val
   290 295 300
   Ser Val Leu Thr Val Leu His Gln Asp Trp Leu Asn Gly Lys Glu Tyr
   305 310 315 320
   Lys Cys Lys Val Ser Asn Lys Ala Leu Pro Ala Pro Ile Glu Lys Thr
   325 330 335
   Ile Ser Lys Ala Lys Gly Gln Pro Arg Glu Pro Gln Val Tyr Thr Leu
   340 345 350
   Pro Pro Ser Arg Glu Glu Met Thr Lys Asn Gln Val Ser Leu Thr Cys
   355 360 365
   Leu Val Lys Gly Phe Tyr Pro Ser Asp Ile Ala Val Glu Trp Glu Ser
   370 375 380
   Asn Gly Gln Pro Glu Asn Asn Tyr Lys Thr Thr Pro Pro Val Leu Asp
   385 390 395 400
   Ser Asp Gly Ser Phe Phe Leu Tyr Ser Lys Leu Thr Val Asp Lys Ser
   405 410 415
   Arg Trp Gln Gln Gly Asn Val Phe Ser Cys Ser Val Met His Glu Ala
   420 425 430
   Leu His Asn His Tyr Thr Gln Lys Ser Leu Ser Leu Ser Pro Gly Lys
   435 440 445
<210> 95
   <211> 11
   <212> PRT
   <213> Oryctolagus cuniculus
<400> 95
   Gln Ala Ser Glu Asn Ile Gly Ser Tyr Leu Ala
   1 5 10
<210> 96
   <211> 7
   <212> PRT
   <213> Oryctolagus cuniculus
<400> 96
   Arg Ala Ser Thr Leu Ala Ser
   1               5
<210> 97
   <211> 12
   <212> PRT
   <213> Oryctolagus cuniculus
<400> 97
   Gln Gln Gly Tyr Asn Ser Glu Asn Leu Asp Asn Ala
   1               5                   10
<210> 98
   <211> 5
   <212> PRT
   <213> Oryctolagus cuniculus
<400> 98
   Met Tyr Ser Met Gly
   1               5
<210> 99
   <211> 16
   <212> PRT
   <213> Synthetic
<400> 99
   Trp Ile Ser Tyr Gly Gly Thr Ala Tyr Tyr Ala Ser Ser Ala Lys Gly
   1               5                   10                  15
<210> 100
   <211> 10
   <212> PRT
   <213> Oryctolagus cuniculus
<400> 100
   Glu Thr Pro Val Asn Tyr Tyr Leu Asp Ile
   1               5                   10
<210> 101
   <211> 109
   <212> PRT
   <213> Oryctolagus cuniculus
<400> 101
   Ala Phe Glu Leu Thr Gln Thr Pro Ser Ser Val Glu Ala Ala Val Gly
   1               5                   10                  15
   Gly Thr Val Thr Ile Lys Cys Gln Ala Ser Gln Asn Ile Val Thr Asn
               20                  25                  30
   Leu Ala Trp Tyr Gln Gln Lys Pro Gly Gln Pro Pro Lys Leu Leu Ile
           35                  40                  45
   Tyr Gly Ala Ser Thr Leu Ala Ser Gly Val Ser Ser Arg Phe Lys Gly
       50                  55                  60
   Ser Gly Ser Gly Thr Gln Phe Thr Leu Thr Ile Ser Asp Leu Glu Cys
   65                  70                  75                  80
   Ala Asp Ala Ala Thr Tyr Phe Cys Gln Ser Tyr Asp Gly Phe Asn Ser
                   85                  90                  95
   Ala Gly Phe Gly Gly Gly Thr Glu Val Val Val Lys Arg
               100                 105
<210> 102
   <211> 215
   <212> PRT
   <213> Synthetic
<400> 102
   Ala Phe Glu Leu Thr Gln Thr Pro Ser Ser Val Glu Ala Ala Val Gly
   1               5                   10                  15
   Gly Thr Val Thr Ile Lys Cys Gln Ala Ser Gln Asn Ile Val Thr Asn
               20                  25                  30
   Leu Ala Trp Tyr Gln Gln Lys Pro Gly Gln Pro Pro Lys Leu Leu Ile
           35                  40                  45
   Tyr Gly Ala Ser Thr Leu Ala Ser Gly Val Ser Ser Arg Phe Lys Gly
       50                  55                  60
   Ser Gly Ser Gly Thr Gln Phe Thr Leu Thr Ile Ser Asp Leu Glu Cys
   65                  70                  75                  80
   Ala Asp Ala Ala Thr Tyr Phe Cys Gln Ser Tyr Asp Gly Phe Asn Ser
                   85                  90                  95
   Ala Gly Phe Gly Gly Gly Thr Glu Val Val Val Lys Arg Thr Val Ala
               100                 105                 110
   Ala Pro Ser Val Phe Ile Phe Pro Pro Ser Asp Glu Gln Leu Lys Ser
   115 120 125
   Gly Thr Ala Ser Val Val Cys Leu Leu Asn Asn Phe Tyr Pro Arg Glu
   130 135 140
   Ala Lys Val Gln Trp Lys Val Asp Asn Ala Leu Gln Ser Gly Asn Ser
   145 150 155 160
   Gln Glu Ser Val Thr Glu Gln Asp Ser Lys Asp Ser Thr Tyr Ser Leu
   165 170 175
   Ser Ser Thr Leu Thr Leu Ser Lys Ala Asp Tyr Glu Lys His Lys Val
   180 185 190
   Tyr Ala Cys Glu Val Thr His Gln Gly Leu Ser Ser Pro Val Thr Lys
   195 200 205
   Ser Phe Asn Arg Gly Glu Cys
   210 215
<210> 103
   <211> 120
   <212> PRT
   <213> Oryctolagus cuniculus
<400> 103
   Gln Ser Leu Glu Glu Ser Gly Gly Arg Leu Val Thr Pro Gly Thr Pro
   1 5 10 15
   Leu Thr Leu Thr Cys Thr Ala Ser Gly Phe Ser Leu Ser Gly Tyr Asp
   20 25 30
   Met Ser Trp Val Arg Gln Ala Pro Gly Lys Gly Leu Glu Tyr Ile Gly
   35 40 45
   Leu Ile Ser Tyr Asp Gly Asn Thr Tyr Tyr Ala Thr Trp Ala Lys Gly
   50 55 60
   Arg Phe Thr Ile Ser Lys Thr Ser Thr Thr Val Asp Leu Lys Ile Thr
   65 70 75 80
   Ser Pro Thr Thr Glu Asp Thr Ala Thr Tyr Phe Cys Ala Arg Ser Leu
   85 90 95
   Tyr Ala Gly Pro Asn Ala Gly Ile Gly Pro Phe Asn Ile Trp Gly Gln
   100 105 110
   Gly Thr Leu Val Thr Val Ser Ser
   115 120
<210> 104
   <211> 450
   <212> PRT
   <213> Synthetic
<400> 104
   Gln Ser Leu Glu Glu Ser Gly Gly Arg Leu Val Thr Pro Gly Thr Pro
   1 5 10 15
   Leu Thr Leu Thr Cys Thr Ala Ser Gly Phe Ser Leu Ser Gly Tyr Asp
   20 25 30
   Met Ser Trp Val Arg Gln Ala Pro Gly Lys Gly Leu Glu Tyr Ile Gly
   35 40 45
   Leu Ile Ser Tyr Asp Gly Asn Thr Tyr Tyr Ala Thr Trp Ala Lys Gly
   50 55 60
   Arg Phe Thr Ile Ser Lys Thr Ser Thr Thr Val Asp Leu Lys Ile Thr
   65 70 75 80
   Ser Pro Thr Thr Glu Asp Thr Ala Thr Tyr Phe Cys Ala Arg Ser Leu
   85 90 95
   Tyr Ala Gly Pro Asn Ala Gly Ile Gly Pro Phe Asn Ile Trp Gly Gln
   100 105 110
   Gly Thr Leu Val Thr Val Ser Ser Ala Ser Thr Lys Gly Pro Ser Val
   115 120 125
   Phe Pro Leu Ala Pro Ser Ser Lys Ser Thr Ser Gly Gly Thr Ala Ala
   130 135 140
   Leu Gly Cys Leu Val Lys Asp Tyr Phe Pro Glu Pro Val Thr Val Ser
   145 150 155 160
   Trp Asn Ser Gly Ala Leu Thr Ser Gly Val His Thr Phe Pro Ala Val
   165 170 175
   Leu Gln Ser Ser Gly Leu Tyr Ser Leu Ser Ser Val Val Thr Val Pro
   180 185 190
   Ser Ser Ser Leu Gly Thr Gln Thr Tyr Ile Cys Asn Val Asn His Lys
   195 200 205
   Pro Ser Asn Thr Lys Val Asp Lys Arg Val Glu Pro Lys Ser Cys Asp
       210                 215                 220
   Lys Thr His Thr Cys Pro Pro Cys Pro Ala Pro Glu Leu Leu Gly Gly
   225                 230                 235                 240
   Pro Ser Val Phe Leu Phe Pro Pro Lys Pro Lys Asp Thr Leu Met Ile
                   245                 250                 255
   Ser Arg Thr Pro Glu Val Thr Cys Val Val Val Asp Val Ser His Glu
               260                 265                 270
   Asp Pro Glu Val Lys Phe Asn Trp Tyr Val Asp Gly Val Glu Val His
           275                 280                 285
   Asn Ala Lys Thr Lys Pro Arg Glu Glu Gln Tyr Ala Ser Thr Tyr Arg
       290                 295                 300
   Val Val Ser Val Leu Thr Val Leu His Gln Asp Trp Leu Asn Gly Lys
   305                 310                 315                 320
   Glu Tyr Lys Cys Lys Val Ser Asn Lys Ala Leu Pro Ala Pro Ile Glu
                   325                 330                 335
   Lys Thr Ile Ser Lys Ala Lys Gly Gln Pro Arg Glu Pro Gln Val Tyr
               340                 345                 350
   Thr Leu Pro Pro Ser Arg Glu Glu Met Thr Lys Asn Gln Val Ser Leu
           355                 360                 365
   Thr Cys Leu Val Lys Gly Phe Tyr Pro Ser Asp Ile Ala Val Glu Trp
       370                 375                 380
   Glu Ser Asn Gly Gln Pro Glu Asn Asn Tyr Lys Thr Thr Pro Pro Val
   385                 390                 395                 400
   Leu Asp Ser Asp Gly Ser Phe Phe Leu Tyr Ser Lys Leu Thr Val Asp
                   405                 410                 415
   Lys Ser Arg Trp Gln Gln Gly Asn Val Phe Ser Cys Ser Val Met His
               420                 425                 430
   Glu Ala Leu His Asn His Tyr Thr Gln Lys Ser Leu Ser Leu Ser Pro
           435                 440                 445
   Gly Lys
       450
<210> 105
   <211> 11
   <212> PRT
   <213> Oryctolagus cuniculus
<400> 105
   Gln Ala Ser Gln Asn Ile Val Thr Asn Leu Ala
   1               5                   10
<210> 106
   <211> 7
   <212> PRT
   <213> Oryctolagus cuniculus
<400> 106
   Gly Ala Ser Thr Leu Ala Ser
   1               5
<210> 107
   <211> 10
   <212> PRT
   <213> Oryctolagus cuniculus
<400> 107
   Gln Ser Tyr Asp Gly Phe Asn Ser Ala Gly
   1               5                   10
<210> 108
   <211> 5
   <212> PRT
   <213> Oryctolagus cuniculus
<400> 108
   Gly Tyr Asp Met Ser
   1               5
<210> 109
   <211> 16
   <212> PRT
   <213> Oryctolagus cuniculus
<400> 109
   Leu Ile Ser Tyr Asp Gly Asn Thr Tyr Tyr Ala Thr Trp Ala Lys Gly
   1               5                   10                  15
<210> 110
   <211> 15
   <212> PRT
   <213> Oryctolagus cuniculus
<400> 110
   Ser Leu Tyr Ala Gly Pro Asn Ala Gly Ile Gly Pro Phe Asn Ile
   1 5 10 15
<210> 111
   <211> 109
   <212> PRT
   <213> Synthetic
<400> 111
   Ala Phe Gln Met Thr Gln Ser Pro Ser Ser Leu Ser Ala Ser Val Gly
   1 5 10 15
   Asp Arg Val Thr Ile Thr Cys Gln Ala Ser Gln Asn Ile Val Thr Asn
   20 25 30
   Leu Ala Trp Tyr Gln Gln Lys Pro Gly Lys Val Pro Lys Leu Leu Ile
   35 40 45
   Tyr Gly Ala Ser Thr Leu Ala Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Val Ala Thr Tyr Tyr Cys Gln Ser Tyr Asp Gly Phe Asn Ser
   85 90 95
   Ala Gly Phe Gly Gly Gly Thr Lys Val Glu Ile Lys Arg
   100 105
<210> 112
   <211> 215
   <212> PRT
   <213> Synthetic
<400> 112
   Ala Phe Gln Met Thr Gln Ser Pro Ser Ser Leu Ser Ala Ser Val Gly
   1               5                   10                  15
   Asp Arg Val Thr Ile Thr Cys Gln Ala Ser Gln Asn Ile Val Thr Asn
               20                  25                  30
   Leu Ala Trp Tyr Gln Gln Lys Pro Gly Lys Val Pro Lys Leu Leu Ile
           35                  40                  45
   Tyr Gly Ala Ser Thr Leu Ala Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Val Ala Thr Tyr Tyr Cys Gln Ser Tyr Asp Gly Phe Asn Ser
                   85                  90                  95
   Ala Gly Phe Gly Gly Gly Thr Lys Val Glu Ile Lys Arg Thr Val Ala
               100                 105                 110
   Ala Pro Ser Val Phe Ile Phe Pro Pro Ser Asp Glu Gln Leu Lys Ser
           115                 120                 125
   Gly Thr Ala Ser Val Val Cys Leu Leu Asn Asn Phe Tyr Pro Arg Glu
       130                 135                 140
   Ala Lys Val Gln Trp Lys Val Asp Asn Ala Leu Gln Ser Gly Asn Ser
   145                 150                 155                 160
   Gln Glu Ser Val Thr Glu Gln Asp Ser Lys Asp Ser Thr Tyr Ser Leu
                   165                 170                 175
   Ser Ser Thr Leu Thr Leu Ser Lys Ala Asp Tyr Glu Lys His Lys Val
               180                 185                 190
   Tyr Ala Cys Glu Val Thr His Gln Gly Leu Ser Ser Pro Val Thr Lys
           195                 200                 205
   Ser Phe Asn Arg Gly Glu Cys
       210                 215
<210> 113
   <211> 123
   <212> PRT
   <213> Synthetic
<400> 113
   Gln Val Gln Leu Val Glu Ser Gly Gly Gly Val Val Gln Pro Gly Arg
   1               5                   10                  15
   Ser Leu Arg Leu Ser Cys Ala Ala Ser Gly Phe Ser Leu Ser Gly Tyr
               20                  25                  30
   Asp Met Ser Trp Val Arg Gln Ala Pro Gly Lys Gly Leu Glu Trp Val
           35                  40                  45
   Gly Leu Ile Ser Tyr Asp Gly Asn Thr Tyr Tyr Ala Thr Ser Ala Lys
       50                  55                  60
   Gly Arg Phe Thr Ile Ser Arg Asp Asn Ser Lys Asn Thr Leu Tyr Leu
   65                  70                  75                  80
   Gln Met Ser Ser Leu Arg Ala Glu Asp Thr Ala Val Tyr Tyr Cys Ala
                   85                  90                  95
   Arg Ser Leu Tyr Ala Gly Pro Asn Ala Gly Ile Gly Pro Phe Asn Ile
               100                 105                 110
   Trp Gly Gln Gly Thr Leu Val Thr Val Ser Ser
           115                 120
<210> 114
   <211> 453
   <212> PRT
   <213> Synthetic
<400> 114
   Gln Val Gln Leu Val Glu Ser Gly Gly Gly Val Val Gln Pro Gly Arg
   1               5                   10                  15
   Ser Leu Arg Leu Ser Cys Ala Ala Ser Gly Phe Ser Leu Ser Gly Tyr
               20                  25                  30
   Asp Met Ser Trp Val Arg Gln Ala Pro Gly Lys Gly Leu Glu Trp Val
           35                  40                  45
   Gly Leu Ile Ser Tyr Asp Gly Asn Thr Tyr Tyr Ala Thr Ser Ala Lys
       50                  55                  60
   Gly Arg Phe Thr Ile Ser Arg Asp Asn Ser Lys Asn Thr Leu Tyr Leu
   65                  70                  75                  80
   Gln Met Ser Ser Leu Arg Ala Glu Asp Thr Ala Val Tyr Tyr Cys Ala
                   85                  90                  95
   Arg Ser Leu Tyr Ala Gly Pro Asn Ala Gly Ile Gly Pro Phe Asn Ile
               100                 105                 110
   Trp Gly Gln Gly Thr Leu Val Thr Val Ser Ser Ala Ser Thr Lys Gly
           115                 120                 125
   Pro Ser Val Phe Pro Leu Ala Pro Ser Ser Lys Ser Thr Ser Gly Gly
       130                 135                 140
   Thr Ala Ala Leu Gly Cys Leu Val Lys Asp Tyr Phe Pro Glu Pro Val
   145                 150                 155                 160
   Thr Val Ser Trp Asn Ser Gly Ala Leu Thr Ser Gly Val His Thr Phe
                   165                 170                 175
   Pro Ala Val Leu Gln Ser Ser Gly Leu Tyr Ser Leu Ser Ser Val Val
               180                 185                 190
   Thr Val Pro Ser Ser Ser Leu Gly Thr Gln Thr Tyr Ile Cys Asn Val
           195                 200                 205
   Asn His Lys Pro Ser Asn Thr Lys Val Asp Lys Arg Val Glu Pro Lys
       210                 215                 220
   Ser Cys Asp Lys Thr His Thr Cys Pro Pro Cys Pro Ala Pro Glu Leu
   225                 230                 235                 240
   Leu Gly Gly Pro Ser Val Phe Leu Phe Pro Pro Lys Pro Lys Asp Thr
                   245                 250                 255
   Leu Met Ile Ser Arg Thr Pro Glu Val Thr Cys Val Val Val Asp Val
               260                 265                 270
   Ser His Glu Asp Pro Glu Val Lys Phe Asn Trp Tyr Val Asp Gly Val
           275                 280                 285
   Glu Val His Asn Ala Lys Thr Lys Pro Arg Glu Glu Gln Tyr Ala Ser
       290                 295                 300
   Thr Tyr Arg Val Val Ser Val Leu Thr Val Leu His Gln Asp Trp Leu
   305                 310                 315                 320
   Asn Gly Lys Glu Tyr Lys Cys Lys Val Ser Asn Lys Ala Leu Pro Ala
                   325                 330                 335
   Pro Ile Glu Lys Thr Ile Ser Lys Ala Lys Gly Gln Pro Arg Glu Pro
               340                 345                 350
   Gln Val Tyr Thr Leu Pro Pro Ser Arg Glu Glu Met Thr Lys Asn Gln
           355                 360                 365
   Val Ser Leu Thr Cys Leu Val Lys Gly Phe Tyr Pro Ser Asp Ile Ala
       370                 375                 380
   Val Glu Trp Glu Ser Asn Gly Gln Pro Glu Asn Asn Tyr Lys Thr Thr
   385                 390                 395                 400
   Pro Pro Val Leu Asp Ser Asp Gly Ser Phe Phe Leu Tyr Ser Lys Leu
                   405                 410                 415
   Thr Val Asp Lys Ser Arg Trp Gln Gln Gly Asn Val Phe Ser Cys Ser
   420 425 430
   Val Met His Glu Ala Leu His Asn His Tyr Thr Gln Lys Ser Leu Ser
   435 440 445
   Leu Ser Pro Gly Lys
   450
<210> 115
   <211> 11
   <212> PRT
   <213> Oryctolagus cuniculus
<400> 115
   Gln Ala Ser Gln Asn Ile Val Thr Asn Leu Ala
   1 5 10
<210> 116
   <211> 7
   <212> PRT
   <213> Oryctolagus cuniculus
<400> 116
   Gly Ala Ser Thr Leu Ala Ser
   1 5
<210> 117
   <211> 10
   <212> PRT
   <213> Oryctolagus cuniculus
<400> 117
   Gln Ser Tyr Asp Gly Phe Asn Ser Ala Gly
   1 5 10
<210> 118
   <211> 5
   <212> PRT
   <213> Oryctolagus cuniculus
<400> 118
   Gly Tyr Asp Met Ser
   1 5
<210> 119
   <211> 16
   <212> PRT
   <213> Synthetic
<400> 119
   Leu Ile Ser Tyr Asp Gly Asn Thr Tyr Tyr Ala Thr Ser Ala Lys Gly
   1               5                   10                  15
<210> 120
   <211> 15
   <212> PRT
   <213> Oryctolagus cuniculus
<400> 120
   Ser Leu Tyr Ala Gly Pro Asn Ala Gly Ile Gly Pro Phe Asn Ile
   1               5                   10                  15
<210> 121
   <211> 112
   <212> PRT
   <213> Oryctolagus cuniculus
<400> 121
   Ala Ala Val Leu Thr Gln Thr Pro Ser Pro Val Ser Ala Ala Val Gly
   1               5                   10                  15
   Gly Thr Val Ser Ile Ser Cys Gln Ser Ser Gln Asn Val Tyr Lys Asn
               20                  25                  30
   Asn Tyr Leu Ser Trp Tyr Gln Gln Lys Pro Gly Gln Pro Pro Lys Leu
           35                  40                  45
   Leu Ile Tyr Lys Ala Ser Thr Leu Ala Ser Gly Val Pro Ser Arg Phe
       50                  55                  60
   Lys Gly Gly Gly Ser Gly Thr Asp Phe Thr Leu Thr Ile Ser Asp Val
   65                  70                  75                  80
   Gln Cys Asp Ala Ala Ala Thr Tyr Tyr Cys Ala Gly Gly Tyr Thr Ser
                   85                  90                  95
   Ser Ser Asp Asn Ala Phe Gly Gly Gly Thr Glu Val Val Val Lys Arg
               100                 105                 110
<210> 122
   <211> 218
   <212> PRT
   <213> Synthetic
<400> 122
   Ala Ala Val Leu Thr Gln Thr Pro Ser Pro Val Ser Ala Ala Val Gly
   1               5                   10                  15
   Gly Thr Val Ser Ile Ser Cys Gln Ser Ser Gln Asn Val Tyr Lys Asn
               20                  25                  30
   Asn Tyr Leu Ser Trp Tyr Gln Gln Lys Pro Gly Gln Pro Pro Lys Leu
           35                  40                  45
   Leu Ile Tyr Lys Ala Ser Thr Leu Ala Ser Gly Val Pro Ser Arg Phe
       50                  55                  60
   Lys Gly Gly Gly Ser Gly Thr Asp Phe Thr Leu Thr Ile Ser Asp Val
   65                  70                  75                  80
   Gln Cys Asp Ala Ala Ala Thr Tyr Tyr Cys Ala Gly Gly Tyr Thr Ser
                   85                  90                  95
   Ser Ser Asp Asn Ala Phe Gly Gly Gly Thr Glu Val Val Val Lys Arg
               100                 105                 110
   Thr Val Ala Ala Pro Ser Val Phe Ile Phe Pro Pro Ser Asp Glu Gln
           115                 120                 125
   Leu Lys Ser Gly Thr Ala Ser Val Val Cys Leu Leu Asn Asn Phe Tyr
       130                 135                 140
   Pro Arg Glu Ala Lys Val Gln Trp Lys Val Asp Asn Ala Leu Gln Ser
   145                 150                 155                 160
   Gly Asn Ser Gln Glu Ser Val Thr Glu Gln Asp Ser Lys Asp Ser Thr
                   165                 170                 175
   Tyr Ser Leu Ser Ser Thr Leu Thr Leu Ser Lys Ala Asp Tyr Glu Lys
               180                 185                 190
   His Lys Val Tyr Ala Cys Glu Val Thr His Gln Gly Leu Ser Ser Pro
           195                 200                 205
   Val Thr Lys Ser Phe Asn Arg Gly Glu Cys
       210                 215
<210> 123
   <211> 117
   <212> PRT
   <213> Oryctolagus cuniculus
<400> 123
   Gln Ser Val Glu Ala Ser Gly Gly Arg Leu Val Thr Pro Gly Thr Pro
   1               5                   10                  15
   Leu Thr Leu Thr Cys Thr Ala Ser Gly Phe Ser Leu Ser Thr Tyr Trp
               20                  25                  30
   Met Ser Trp Val Arg Gln Ala Pro Gly Lys Gly Leu Glu Trp Ile Gly
           35                  40                  45
   Asp Ile Tyr Phe Ser Asn Glu Glu Thr Asn Tyr Ala Ser Trp Ala Lys
       50                  55                  60
   Gly Arg Phe Thr Ile Ser Lys Thr Ser Thr Thr Val Asp Leu Asn Val
   65                  70                  75                  80
   Ile Ser Pro Thr Thr Glu Asp Thr Ala Thr Tyr Phe Cys Ala Arg Gly
                   85                  90                  95
   Ser Pro Asp Val Asp Ile Gly Ile Asp Met Trp Gly Pro Gly Thr Leu
               100                 105                 110
   Val Thr Val Ser Ser
           115
<210> 124
   <211> 447
   <212> PRT
   <213> Synthetic
<400> 124
   Gln Ser Val Glu Ala Ser Gly Gly Arg Leu Val Thr Pro Gly Thr Pro
   1               5                   10                  15
   Leu Thr Leu Thr Cys Thr Ala Ser Gly Phe Ser Leu Ser Thr Tyr Trp
               20                  25                  30
   Met Ser Trp Val Arg Gln Ala Pro Gly Lys Gly Leu Glu Trp Ile Gly
           35                  40                  45
   Asp Ile Tyr Phe Ser Asn Glu Glu Thr Asn Tyr Ala Ser Trp Ala Lys
       50                  55                  60
   Gly Arg Phe Thr Ile Ser Lys Thr Ser Thr Thr Val Asp Leu Asn Val
   65                  70                  75                  80
   Ile Ser Pro Thr Thr Glu Asp Thr Ala Thr Tyr Phe Cys Ala Arg Gly
                   85                  90                  95
   Ser Pro Asp Val Asp Ile Gly Ile Asp Met Trp Gly Pro Gly Thr Leu
               100                 105                 110
   Val Thr Val Ser Ser Ala Ser Thr Lys Gly Pro Ser Val Phe Pro Leu
           115                 120                 125
   Ala Pro Ser Ser Lys Ser Thr Ser Gly Gly Thr Ala Ala Leu Gly Cys
       130                 135                 140
   Leu Val Lys Asp Tyr Phe Pro Glu Pro Val Thr Val Ser Trp Asn Ser
   145                 150                 155                 160
   Gly Ala Leu Thr Ser Gly Val His Thr Phe Pro Ala Val Leu Gln Ser
                   165                 170                 175
   Ser Gly Leu Tyr Ser Leu Ser Ser Val Val Thr Val Pro Ser Ser Ser
               180                 185                 190
   Leu Gly Thr Gln Thr Tyr Ile Cys Asn Val Asn His Lys Pro Ser Asn
           195                 200                 205
   Thr Lys Val Asp Lys Arg Val Glu Pro Lys Ser Cys Asp Lys Thr His
       210                 215                 220
   Thr Cys Pro Pro Cys Pro Ala Pro Glu Leu Leu Gly Gly Pro Ser Val
   225                 230                 235                 240
   Phe Leu Phe Pro Pro Lys Pro Lys Asp Thr Leu Met Ile Ser Arg Thr
                   245                 250                 255
   Pro Glu Val Thr Cys Val Val Val Asp Val Ser His Glu Asp Pro Glu
               260                 265                 270
   Val Lys Phe Asn Trp Tyr Val Asp Gly Val Glu Val His Asn Ala Lys
           275                 280                 285
   Thr Lys Pro Arg Glu Glu Gln Tyr Ala Ser Thr Tyr Arg Val Val Ser
       290                 295                 300
   Val Leu Thr Val Leu His Gln Asp Trp Leu Asn Gly Lys Glu Tyr Lys
   305                 310                 315                 320
   Cys Lys Val Ser Asn Lys Ala Leu Pro Ala Pro Ile Glu Lys Thr Ile
                   325                 330                 335
   Ser Lys Ala Lys Gly Gln Pro Arg Glu Pro Gln Val Tyr Thr Leu Pro
               340                 345                 350
   Pro Ser Arg Glu Glu Met Thr Lys Asn Gln Val Ser Leu Thr Cys Leu
           355                 360                 365
   Val Lys Gly Phe Tyr Pro Ser Asp Ile Ala Val Glu Trp Glu Ser Asn
   370 375 380
   Gly Gln Pro Glu Asn Asn Tyr Lys Thr Thr Pro Pro Val Leu Asp Ser
   385 390 395 400
   Asp Gly Ser Phe Phe Leu Tyr Ser Lys Leu Thr Val Asp Lys Ser Arg
   405 410 415
   Trp Gln Gln Gly Asn Val Phe Ser Cys Ser Val Met His Glu Ala Leu
   420 425 430
   His Asn His Tyr Thr Gln Lys Ser Leu Ser Leu Ser Pro Gly Lys
   435 440 445
<210> 125
   <211> 13
   <212> PRT
   <213> Oryctolagus cuniculus
<400> 125
   Gln Ser Ser Gln Asn Val Tyr Lys Asn Asn Tyr Leu Ser
   1 5 10
<210> 126
   <211> 7
   <212> PRT
   <213> Oryctolagus cuniculus
<400> 126
   Lys Ala Ser Thr Leu Ala Ser
   1 5
<210> 127
   <211> 11
   <212> PRT
   <213> Oryctolagus cuniculus
<400> 127
   Ala Gly Gly Tyr Thr Ser Ser Ser Asp Asn Ala
   1 5 10
<210> 128
   <211> 5
   <212> PRT
   <213> Oryctolagus cuniculus
<400> 128
   Thr Tyr Trp Met Ser
   1 5
<210> 129
   <211> 17
   <212> PRT
   <213> Oryctolagus cuniculus
<400> 129
   Asp Ile Tyr Phe Ser Asn Glu Glu Thr Asn Tyr Ala Ser Trp Ala Lys
   1               5                   10                  15
   Gly
<210> 130
   <211> 11
   <212> PRT
   <213> Oryctolagus cuniculus
<400> 130
   Gly Ser Pro Asp Val Asp Ile Gly Ile Asp Met
   1               5                   10
<210> 131
   <211> 112
   <212> PRT
   <213> Synthetic
<400> 131
   Asp Ile Gln Met Thr Gln Ser Pro Ser Ser Leu Ser Ala Ser Val Gly
   1               5                   10                  15
   Asp Arg Val Thr Ile Thr Cys Gln Ser Ser Gln Asn Val Tyr Lys Asn
               20                  25                  30
   Asn Tyr Leu Ser Trp Tyr Gln Gln Lys Pro Gly Lys Val Pro Lys Leu
           35                  40                  45
   Leu Ile Tyr Lys Ala Ser Thr Leu Ala Ser Gly Val Pro Ser Arg Phe
       50                  55                  60
   Ser Gly Ser Gly Ser Gly Thr Asp Phe Thr Leu Thr Ile Ser Ser Leu
   65                  70                  75                  80
   Gln Pro Glu Asp Val Ala Thr Tyr Tyr Cys Ala Gly Gly Tyr Thr Ser
                   85                  90                  95
   Ser Ser Asp Asn Ala Phe Gly Gly Gly Thr Lys Val Glu Ile Lys Arg
               100                 105                 110
<210> 132
   <211> 218
   <212> PRT
   <213> Synthetic
<400> 132
   Asp Ile Gln Met Thr Gln Ser Pro Ser Ser Leu Ser Ala Ser Val Gly
   1               5                   10                  15
   Asp Arg Val Thr Ile Thr Cys Gln Ser Ser Gln Asn Val Tyr Lys Asn
               20                  25                  30
   Asn Tyr Leu Ser Trp Tyr Gln Gln Lys Pro Gly Lys Val Pro Lys Leu
           35                  40                  45
   Leu Ile Tyr Lys Ala Ser Thr Leu Ala Ser Gly Val Pro Ser Arg Phe
       50                  55                  60
   Ser Gly Ser Gly Ser Gly Thr Asp Phe Thr Leu Thr Ile Ser Ser Leu
   65                  70                  75                  80
   Gln Pro Glu Asp Val Ala Thr Tyr Tyr Cys Ala Gly Gly Tyr Thr Ser
                   85                  90                  95
   Ser Ser Asp Asn Ala Phe Gly Gly Gly Thr Lys Val Glu Ile Lys Arg
               100                 105                 110
   Thr Val Ala Ala Pro Ser Val Phe Ile Phe Pro Pro Ser Asp Glu Gln
           115                 120                 125
   Leu Lys Ser Gly Thr Ala Ser Val Val Cys Leu Leu Asn Asn Phe Tyr
       130                 135                 140
   Pro Arg Glu Ala Lys Val Gln Trp Lys Val Asp Asn Ala Leu Gln Ser
   145                 150                 155                 160
   Gly Asn Ser Gln Glu Ser Val Thr Glu Gln Asp Ser Lys Asp Ser Thr
                   165                 170                 175
   Tyr Ser Leu Ser Ser Thr Leu Thr Leu Ser Lys Ala Asp Tyr Glu Lys
               180                 185                 190
   His Lys Val Tyr Ala Cys Glu Val Thr His Gln Gly Leu Ser Ser Pro
           195                 200                 205
   Val Thr Lys Ser Phe Asn Arg Gly Glu Cys
       210                 215
<210> 133
   <211> 120
   <212> PRT
   <213> Synthetic
<400> 133
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Val Ser Thr Tyr
               20                  25                  30
   Trp Met Ser Trp Val Arg Gln Ala Pro Gly Lys Gly Leu Glu Trp Val
           35                  40                  45
   Gly Asp Ile Tyr Phe Ser Asn Glu Glu Thr Asn Tyr Ala Ser Ser Ala
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Gly Ser Pro Asp Val Asp Ile Gly Ile Asp Met Trp Gly Pro
               100                 105                 110
   Gly Thr Leu Val Thr Val Ser Ser
           115                 120
<210> 134
   <211> 450
   <212> PRT
   <213> Synthetic
<400> 134
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Val Ser Thr Tyr
               20                  25                  30
   Trp Met Ser Trp Val Arg Gln Ala Pro Gly Lys Gly Leu Glu Trp Val
           35                  40                  45
   Gly Asp Ile Tyr Phe Ser Asn Glu Glu Thr Asn Tyr Ala Ser Ser Ala
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Gly Ser Pro Asp Val Asp Ile Gly Ile Asp Met Trp Gly Pro
               100                 105                 110
   Gly Thr Leu Val Thr Val Ser Ser Ala Ser Thr Lys Gly Pro Ser Val
           115                 120                 125
   Phe Pro Leu Ala Pro Ser Ser Lys Ser Thr Ser Gly Gly Thr Ala Ala
       130                 135                 140
   Leu Gly Cys Leu Val Lys Asp Tyr Phe Pro Glu Pro Val Thr Val Ser
   145                 150                 155                 160
   Trp Asn Ser Gly Ala Leu Thr Ser Gly Val His Thr Phe Pro Ala Val
                   165                 170                 175
   Leu Gln Ser Ser Gly Leu Tyr Ser Leu Ser Ser Val Val Thr Val Pro
               180                 185                 190
   Ser Ser Ser Leu Gly Thr Gln Thr Tyr Ile Cys Asn Val Asn His Lys
           195                 200                 205
   Pro Ser Asn Thr Lys Val Asp Lys Arg Val Glu Pro Lys Ser Cys Asp
       210                 215                 220
   Lys Thr His Thr Cys Pro Pro Cys Pro Ala Pro Glu Leu Leu Gly Gly
   225                 230                 235                 240
   Pro Ser Val Phe Leu Phe Pro Pro Lys Pro Lys Asp Thr Leu Met Ile
                   245                 250                 255
   Ser Arg Thr Pro Glu Val Thr Cys Val Val Val Asp Val Ser His Glu
               260                 265                 270
   Asp Pro Glu Val Lys Phe Asn Trp Tyr Val Asp Gly Val Glu Val His
           275                 280                 285
   Asn Ala Lys Thr Lys Pro Arg Glu Glu Gln Tyr Ala Ser Thr Tyr Arg
       290                 295                 300
   Val Val Ser Val Leu Thr Val Leu His Gln Asp Trp Leu Asn Gly Lys
   305                 310                 315                 320
   Glu Tyr Lys Cys Lys Val Ser Asn Lys Ala Leu Pro Ala Pro Ile Glu
                   325                 330                 335
   Lys Thr Ile Ser Lys Ala Lys Gly Gln Pro Arg Glu Pro Gln Val Tyr
               340                 345                 350
   Thr Leu Pro Pro Ser Arg Glu Glu Met Thr Lys Asn Gln Val Ser Leu
           355                 360                 365
   Thr Cys Leu Val Lys Gly Phe Tyr Pro Ser Asp Ile Ala Val Glu Trp
       370                 375                 380
   Glu Ser Asn Gly Gln Pro Glu Asn Asn Tyr Lys Thr Thr Pro Pro Val
   385                 390                 395                 400
   Leu Asp Ser Asp Gly Ser Phe Phe Leu Tyr Ser Lys Leu Thr Val Asp
                   405                 410                 415
   Lys Ser Arg Trp Gln Gln Gly Asn Val Phe Ser Cys Ser Val Met His
               420                 425                 430
   Glu Ala Leu His Asn His Tyr Thr Gln Lys Ser Leu Ser Leu Ser Pro
           435                 440                 445
   Gly Lys
       450
<210> 135
   <211> 13
   <212> PRT
   <213> Oryctolagus cuniculus
<400> 135
   Gln Ser Ser Gln Asn Val Tyr Lys Asn Asn Tyr Leu Ser
   1               5                   10
<210> 136
   <211> 7
   <212> PRT
   <213> Oryctolagus cuniculus
<400> 136
   Lys Ala Ser Thr Leu Ala Ser
   1               5
<210> 137
   <211> 11
   <212> PRT
   <213> Oryctolagus cuniculus
<400> 137
   Ala Gly Gly Tyr Thr Ser Ser Ser Asp Asn Ala
   1 5 10
<210> 138
   <211> 5
   <212> PRT
   <213> Oryctolagus cuniculus
<400> 138
   Thr Tyr Trp Met Ser
   1 5
<210> 139
   <211> 17
   <212> PRT
   <213> Synthetic
<400> 139
   Asp Ile Tyr Phe Ser Asn Glu Glu Thr Asn Tyr Ala Ser Ser Ala Lys
   1 5 10 15
   Gly
<210> 140
   <211> 11
   <212> PRT
   <213> Oryctolagus cuniculus
<400> 140
   Gly Ser Pro Asp Val Asp Ile Gly Ile Asp Met
   1 5 10
<210> 141
   <211> 112
   <212> PRT
   <213> Oryctolagus cuniculus
<400> 141
   Ala Ala Val Leu Thr Gln Thr Pro Ser Pro Val Ser Ala Ala Val Gly
   1 5 10 15
   Asp Thr Val Thr Ile Lys Cys Gln Ser Ser Gln Ser Val Tyr Lys Asn
   20 25 30
   Asn Tyr Leu Ser Trp Tyr Gln Gln Lys Pro Gly Gln Pro Pro Lys Leu
   35 40 45
   Leu Ile Tyr Asp Ala Ser Asn Leu Pro Ser Gly Val Pro Ser Arg Phe
   50 55 60
   Ser Gly Ser Gly Ser Gly Thr Gln Phe Thr Leu Thr Ile Ser Gly Val
   65 70 75 80
   Gln Cys Asp Asp Ala Ala Thr Tyr Tyr Cys Leu Gly Asp Tyr Asp Asp
   85 90 95
   Asp Thr Asp Asn Gly Phe Gly Gly Gly Thr Glu Val Val Val Lys Arg
   100 105 110
<210> 142
   <211> 218
   <212> PRT
   <213> Synthetic
<400> 142
   Ala Ala Val Leu Thr Gln Thr Pro Ser Pro Val Ser Ala Ala Val Gly
   1 5 10 15
   Asp Thr Val Thr Ile Lys Cys Gln Ser Ser Gln Ser Val Tyr Lys Asn
   20 25 30
   Asn Tyr Leu Ser Trp Tyr Gln Gln Lys Pro Gly Gln Pro Pro Lys Leu
   35 40 45
   Leu Ile Tyr Asp Ala Ser Asn Leu Pro Ser Gly Val Pro Ser Arg Phe
   50 55 60
   Ser Gly Ser Gly Ser Gly Thr Gln Phe Thr Leu Thr Ile Ser Gly Val
   65 70 75 80
   Gln Cys Asp Asp Ala Ala Thr Tyr Tyr Cys Leu Gly Asp Tyr Asp Asp
   85 90 95
   Asp Thr Asp Asn Gly Phe Gly Gly Gly Thr Glu Val Val Val Lys Arg
   100 105 110
   Thr Val Ala Ala Pro Ser Val Phe Ile Phe Pro Pro Ser Asp Glu Gln
   115 120 125
   Leu Lys Ser Gly Thr Ala Ser Val Val Cys Leu Leu Asn Asn Phe Tyr
   130 135 140
   Pro Arg Glu Ala Lys Val Gln Trp Lys Val Asp Asn Ala Leu Gln Ser
   145 150 155 160
   Gly Asn Ser Gln Glu Ser Val Thr Glu Gln Asp Ser Lys Asp Ser Thr
   165 170 175
   Tyr Ser Leu Ser Ser Thr Leu Thr Leu Ser Lys Ala Asp Tyr Glu Lys
   180 185 190
   His Lys Val Tyr Ala Cys Glu Val Thr His Gln Gly Leu Ser Ser Pro
   195 200 205
   Val Thr Lys Ser Phe Asn Arg Gly Glu Cys
   210 215
<210> 143
   <211> 112
   <212> PRT
   <213> Oryctolagus cuniculus
<400> 143
   Gln Ser Val Glu Glu Ser Gly Gly Arg Leu Val Thr Pro Gly Thr Pro
   1 5 10 15
   Leu Thr Leu Thr Cys Thr Val Ser Gly Ile Asp Leu Ser Ser Tyr Ala
   20 25 30
   Met Ile Trp Val Arg Gln Ala Pro Gly Lys Gly Leu Glu Tyr Ile Gly
   35 40 45
   Ile Ile Trp Ser Gly Gly Thr Tyr Tyr Ala Thr Trp Ala Lys Gly Arg
   50 55 60
   Phe Thr Ile Ser Lys Thr Ser Thr Thr Val Asp Leu Gln Ile Thr Ser
   65 70 75 80
   Pro Thr Thr Glu Asp Ala Ala Thr Tyr Phe Cys Ala Ala Gly Gly Gly
   85 90 95
   Ser Ile Tyr Asp Val Trp Gly Pro Gly Thr Leu Val Thr Val Ser Ser
   100 105 110
<210> 144
   <211> 442
   <212> PRT
   <213> Synthetic
<400> 144
   Gln Ser Val Glu Glu Ser Gly Gly Arg Leu Val Thr Pro Gly Thr Pro
   1 5 10 15
   Leu Thr Leu Thr Cys Thr Val Ser Gly Ile Asp Leu Ser Ser Tyr Ala
   20 25 30
   Met Ile Trp Val Arg Gln Ala Pro Gly Lys Gly Leu Glu Tyr Ile Gly
   35 40 45
   Ile Ile Trp Ser Gly Gly Thr Tyr Tyr Ala Thr Trp Ala Lys Gly Arg
   50 55 60
   Phe Thr Ile Ser Lys Thr Ser Thr Thr Val Asp Leu Gln Ile Thr Ser
   65 70 75 80
   Pro Thr Thr Glu Asp Ala Ala Thr Tyr Phe Cys Ala Ala Gly Gly Gly
   85 90 95
   Ser Ile Tyr Asp Val Trp Gly Pro Gly Thr Leu Val Thr Val Ser Ser
   100 105 110
   Ala Ser Thr Lys Gly Pro Ser Val Phe Pro Leu Ala Pro Ser Ser Lys
   115 120 125
   Ser Thr Ser Gly Gly Thr Ala Ala Leu Gly Cys Leu Val Lys Asp Tyr
   130 135 140
   Phe Pro Glu Pro Val Thr Val Ser Trp Asn Ser Gly Ala Leu Thr Ser
   145 150 155 160
   Gly Val His Thr Phe Pro Ala Val Leu Gln Ser Ser Gly Leu Tyr Ser
   165 170 175
   Leu Ser Ser Val Val Thr Val Pro Ser Ser Ser Leu Gly Thr Gln Thr
   180 185 190
   Tyr Ile Cys Asn Val Asn His Lys Pro Ser Asn Thr Lys Val Asp Lys
   195 200 205
   Arg Val Glu Pro Lys Ser Cys Asp Lys Thr His Thr Cys Pro Pro Cys
   210 215 220
   Pro Ala Pro Glu Leu Leu Gly Gly Pro Ser Val Phe Leu Phe Pro Pro
   225 230 235 240
   Lys Pro Lys Asp Thr Leu Met Ile Ser Arg Thr Pro Glu Val Thr Cys
   245 250 255
   Val Val Val Asp Val Ser His Glu Asp Pro Glu Val Lys Phe Asn Trp
   260 265 270
   Tyr Val Asp Gly Val Glu Val His Asn Ala Lys Thr Lys Pro Arg Glu
   275 280 285
   Glu Gln Tyr Ala Ser Thr Tyr Arg Val Val Ser Val Leu Thr Val Leu
   290 295 300
   His Gln Asp Trp Leu Asn Gly Lys Glu Tyr Lys Cys Lys Val Ser Asn
   305 310 315 320
   Lys Ala Leu Pro Ala Pro Ile Glu Lys Thr Ile Ser Lys Ala Lys Gly
   325 330 335
   Gln Pro Arg Glu Pro Gln Val Tyr Thr Leu Pro Pro Ser Arg Glu Glu
   340 345 350
   Met Thr Lys Asn Gln Val Ser Leu Thr Cys Leu Val Lys Gly Phe Tyr
   355 360 365
   Pro Ser Asp Ile Ala Val Glu Trp Glu Ser Asn Gly Gln Pro Glu Asn
   370 375 380
   Asn Tyr Lys Thr Thr Pro Pro Val Leu Asp Ser Asp Gly Ser Phe Phe
   385 390 395 400
   Leu Tyr Ser Lys Leu Thr Val Asp Lys Ser Arg Trp Gln Gln Gly Asn
   405 410 415
   Val Phe Ser Cys Ser Val Met His Glu Ala Leu His Asn His Tyr Thr
   420 425 430
   Gln Lys Ser Leu Ser Leu Ser Pro Gly Lys
   435 440
<210> 145
   <211> 13
   <212> PRT
   <213> Oryctolagus cuniculus
<400> 145
   Gln Ser Ser Gln Ser Val Tyr Lys Asn Asn Tyr Leu Ser
   1 5 10
<210> 146
   <211> 7
   <212> PRT
   <213> Oryctolagus cuniculus
<400> 146
   Asp Ala Ser Asn Leu Pro Ser
   1 5
<210> 147
   <211> 11
   <212> PRT
   <213> Oryctolagus cuniculus
<400> 147
   Leu Gly Asp Tyr Asp Asp Asp Thr Asp Asn Gly
   1               5                   10
<210> 148
   <211> 5
   <212> PRT
   <213> Oryctolagus cuniculus
<400> 148
   Ser Tyr Ala Met Ile
   1               5
<210> 149
   <211> 15
   <212> PRT
   <213> Oryctolagus cuniculus
<400> 149
   Ile Ile Trp Ser Gly Gly Thr Tyr Tyr Ala Thr Trp Ala Lys Gly
   1               5                   10                  15
<210> 150
   <211> 8
   <212> PRT
   <213> Oryctolagus cuniculus
<400> 150
   Gly Gly Gly Ser Ile Tyr Asp Val
   1               5
<210> 151
   <211> 112
   <212> PRT
   <213> Oryctolagus cuniculus
<400> 151
   Ala Leu Val Met Thr Gln Thr Pro Ser Ser Thr Ser Glu Pro Val Gly
   1               5                   10                  15
   Gly Thr Val Thr Ile Asn Cys Gln Ala Ser Gln Asn Ile Gly Asn Asp
               20                  25                  30
   Leu Ser Trp Tyr Gln Gln Lys Pro Gly Gln Pro Pro Glu Leu Leu Ile
           35                  40                  45
   Tyr Ser Thr Ser Lys Leu Ala Thr Gly Val Pro Lys Arg Phe Ser Gly
   50 55 60
   Ser Arg Ser Gly Thr Gln Phe Thr Leu Thr Ile Ser Asp Leu Glu Cys
   65 70 75 80
   Asp Asp Ala Ala Thr Tyr Tyr Cys Leu Gly Val Tyr Ser Tyr Ile Ser
   85 90 95
   Asp Asp Gly Asn Ala Phe Gly Gly Gly Thr Glu Val Val Val Lys Arg
   100 105 110
<210> 152
   <211> 218
   <212> **PRT**
   <213> Synthetic
<400> 152
   Ala Leu Val Met Thr Gln Thr Pro Ser Ser Thr Ser Glu Pro Val Gly
   1 5 10 15
   Gly Thr Val Thr Ile Asn Cys Gln Ala Ser Gln Asn Ile Gly Asn Asp
   20 25 30
   Leu Ser Trp Tyr Gln Gln Lys Pro Gly Gln Pro Pro Glu Leu Leu Ile
   35 40 45
   Tyr Ser Thr Ser Lys Leu Ala Thr Gly Val Pro Lys Arg Phe Ser Gly
   50 55 60
   Ser Arg Ser Gly Thr Gln Phe Thr Leu Thr Ile Ser Asp Leu Glu Cys
   65 70 75 80
   Asp Asp Ala Ala Thr Tyr Tyr Cys Leu Gly Val Tyr Ser Tyr Ile Ser
   85 90 95
   Asp Asp Gly Asn Ala Phe Gly Gly Gly Thr Glu Val Val Val Lys Arg
   100 105 110
   Thr Val Ala Ala Pro Ser Val Phe Ile Phe Pro Pro Ser Asp Glu Gln
   115 120 125
   Leu Lys Ser Gly Thr Ala Ser Val Val Cys Leu Leu Asn Asn Phe Tyr
   130 135 140
   Pro Arg Glu Ala Lys Val Gln Trp Lys Val Asp Asn Ala Leu Gln Ser
   145 150 155 160
   Gly Asn Ser Gln Glu Ser Val Thr Glu Gln Asp Ser Lys Asp Ser Thr
   165 170 175
   Tyr Ser Leu Ser Ser Thr Leu Thr Leu Ser Lys Ala Asp Tyr Glu Lys
   180 185 190
   His Lys Val Tyr Ala Cys Glu Val Thr His Gln Gly Leu Ser Ser Pro
   195 200 205
   Val Thr Lys Ser Phe Asn Arg Gly Glu Cys
   210 215
<210> 153
   <211> 120
   <212> PRT
   <213> Oryctolagus cuniculus
<400> 153
   Gln Ser Val Glu Glu Phe Gly Gly Arg Leu Val Thr Pro Gly Thr Pro
   1 5 10 15
   Leu Thr Leu Thr Cys Thr Val Ser Gly Phe Ser Leu Asn Asn Tyr Ala
   20 25 30
   Met Thr Trp Val Arg Gln Ala Pro Gly Lys Gly Leu Glu Trp Ile Gly
   35 40 45
   Ile Ile Gly Ser Ile Gly Thr Thr Tyr Tyr Ala Ser Trp Ala Lys Gly
   50 55 60
   Arg Phe Phe Ile Ser Lys Thr Ser Thr Thr Val Asp Leu Lys Ile Ile
   65 70 75 80
   Ser Pro Thr Thr Glu Asp Thr Ala Thr Tyr Phe Cys Ala Arg Asp Ala
   85 90 95
   Gly Val Thr Val Asp Gly Tyr Gly Tyr Tyr Phe Asn Ile Trp Gly Pro
   100 105 110
   Gly Thr Leu Val Thr Val Ser Ser
   115 120
<210> 154
   <211> 450
   <212> PRT
   <213> Synthetic
<400> 154
   Gln Ser Val Glu Glu Phe Gly Gly Arg Leu Val Thr Pro Gly Thr Pro
   1               5                   10                  15
   Leu Thr Leu Thr Cys Thr Val Ser Gly Phe Ser Leu Asn Asn Tyr Ala
               20                  25                  30
   Met Thr Trp Val Arg Gln Ala Pro Gly Lys Gly Leu Glu Trp Ile Gly
           35                  40                  45
   Ile Ile Gly Ser Ile Gly Thr Thr Tyr Tyr Ala Ser Trp Ala Lys Gly
       50                  55                  60
   Arg Phe Phe Ile Ser Lys Thr Ser Thr Thr Val Asp Leu Lys Ile Ile
   65                  70                  75                  80
   Ser Pro Thr Thr Glu Asp Thr Ala Thr Tyr Phe Cys Ala Arg Asp Ala
                   85                  90                  95
   Gly Val Thr Val Asp Gly Tyr Gly Tyr Tyr Phe Asn Ile Trp Gly Pro
               100                 105                 110
   Gly Thr Leu Val Thr Val Ser Ser Ala Ser Thr Lys Gly Pro Ser Val
           115                 120                 125
   Phe Pro Leu Ala Pro Ser Ser Lys Ser Thr Ser Gly Gly Thr Ala Ala
       130                 135                 140
   Leu Gly Cys Leu Val Lys Asp Tyr Phe Pro Glu Pro Val Thr Val Ser
   145                 150                 155                 160
   Trp Asn Ser Gly Ala Leu Thr Ser Gly Val His Thr Phe Pro Ala Val
                   165                 170                 175
   Leu Gln Ser Ser Gly Leu Tyr Ser Leu Ser Ser Val Val Thr Val Pro
               180                 185                 190
   Ser Ser Ser Leu Gly Thr Gln Thr Tyr Ile Cys Asn Val Asn His Lys
           195                 200                 205
   Pro Ser Asn Thr Lys Val Asp Lys Arg Val Glu Pro Lys Ser Cys Asp
       210                 215                 220
   Lys Thr His Thr Cys Pro Pro Cys Pro Ala Pro Glu Leu Leu Gly Gly
   225                 230                 235                 240
   Pro Ser Val Phe Leu Phe Pro Pro Lys Pro Lys Asp Thr Leu Met Ile
                   245                 250                 255
   Ser Arg Thr Pro Glu Val Thr Cys Val Val Val Asp Val Ser His Glu
   260 265 270
   Asp Pro Glu Val Lys Phe Asn Trp Tyr Val Asp Gly Val Glu Val His
   275 280 285
   Asn Ala Lys Thr Lys Pro Arg Glu Glu Gln Tyr Ala Ser Thr Tyr Arg
   290 295 300
   Val Val Ser Val Leu Thr Val Leu His Gln Asp Trp Leu Asn Gly Lys
   305 310 315 320
   Glu Tyr Lys Cys Lys Val Ser Asn Lys Ala Leu Pro Ala Pro Ile Glu
   325 330 335
   Lys Thr Ile Ser Lys Ala Lys Gly Gln Pro Arg Glu Pro Gln Val Tyr
   340 345 350
   Thr Leu Pro Pro Ser Arg Glu Glu Met Thr Lys Asn Gln Val Ser Leu
   355 360 365
   Thr Cys Leu Val Lys Gly Phe Tyr Pro Ser Asp Ile Ala Val Glu Trp
   370 375 380
   Glu Ser Asn Gly Gln Pro Glu Asn Asn Tyr Lys Thr Thr Pro Pro Val
   385 390 395 400
   Leu Asp Ser Asp Gly Ser Phe Phe Leu Tyr Ser Lys Leu Thr Val Asp
   405 410 415
   Lys Ser Arg Trp Gln Gln Gly Asn Val Phe Ser Cys Ser Val Met His
   420 425 430
   Glu Ala Leu His Asn His Tyr Thr Gln Lys Ser Leu Ser Leu Ser Pro
   435 440 445
   Gly Lys
   450
<210> 155
   <211> 11
   <212> PRT
   <213> Oryctolagus cuniculus
<400> 155
   Gln Ala Ser Gln Asn Ile Gly Asn Asp Leu Ser
   1 5 10
<210> 156
   <211> 7
   <212> PRT
   <213> Oryctolagus cuniculus
<400> 156
   Ser Thr Ser Lys Leu Ala Thr
   1 5
<210> 157
   <211> 13
   <212> PRT
   <213> Oryctolagus cuniculus
<400> 157
   Leu Gly Val Tyr Ser Tyr Ile Ser Asp Asp Gly Asn Ala
   1 5 10
<210> 158
   <211> 5
   <212> **PRT**
   <213> Oryctolagus cuniculus
<400> 158
   Asn Tyr Ala Met Thr
   1 5
<210> 159
   <211> 16
   <212> PRT
   <213> Oryctolagus cuniculus
<400> 159
   Ile Ile Gly Ser Ile Gly Thr Thr Tyr Tyr Ala Ser Trp Ala Lys Gly
   1 5 10 15
<210> 160
   <211> 15
   <212> PRT
   <213> Oryctolagus cuniculus
<400> 160
   Asp Ala Gly Val Thr Val Asp Gly Tyr Gly Tyr Tyr Phe Asn Ile
   1 5 10 15
<210> 161
   <211> 112
   <212> PRT
   <213> Oryctolagus cuniculus
<400> 161
   Ala Ile Glu Met Thr Gln Thr Pro Phe Ser Val Ser Ala Ala Val Gly
   1               5                   10                  15
   Gly Thr Val Thr Ile Lys Cys Gln Ala Ser Gln Thr Ile Ser Asn Tyr
               20                  25                  30
   Leu Ala Trp Tyr Gln Gln Lys Pro Gly Gln Pro Pro Lys Leu Leu Ile
           35                  40                  45
   Tyr Gly Ala Ser Asn Leu Glu Ser Gly Val Pro Ser Arg Phe Lys Gly
       50                  55                  60
   Ser Gly Ser Gly Thr Gln Phe Thr Leu Thr Ile Ser Asp Leu Glu Cys
   65                  70                  75                  80
   Asp Asp Ala Ala Thr Tyr Tyr Cys Gln Gln Gly Tyr Thr Ile Ser Asn
                   85                  90                  95
   Val Asp Asn Asn Val Phe Gly Gly Gly Thr Glu Val Val Val Lys Arg
               100                 105                 110
<210> 162
   <211> 218
   <212> PRT
   <213> Synthetic
<400> 162
   Ala Ile Glu Met Thr Gln Thr Pro Phe Ser Val Ser Ala Ala Val Gly
   1               5                   10                  15
   Gly Thr Val Thr Ile Lys Cys Gln Ala Ser Gln Thr Ile Ser Asn Tyr
               20                  25                  30
   Leu Ala Trp Tyr Gln Gln Lys Pro Gly Gln Pro Pro Lys Leu Leu Ile
           35                  40                  45
   Tyr Gly Ala Ser Asn Leu Glu Ser Gly Val Pro Ser Arg Phe Lys Gly
       50                  55                  60
   Ser Gly Ser Gly Thr Gln Phe Thr Leu Thr Ile Ser Asp Leu Glu Cys
   65                  70                  75                  80
   Asp Asp Ala Ala Thr Tyr Tyr Cys Gln Gln Gly Tyr Thr Ile Ser Asn
                   85                  90                  95
   Val Asp Asn Asn Val Phe Gly Gly Gly Thr Glu Val Val Val Lys Arg
               100                 105                 110
   Thr Val Ala Ala Pro Ser Val Phe Ile Phe Pro Pro Ser Asp Glu Gln
           115                 120                 125
   Leu Lys Ser Gly Thr Ala Ser Val Val Cys Leu Leu Asn Asn Phe Tyr
       130                 135                 140
   Pro Arg Glu Ala Lys Val Gln Trp Lys Val Asp Asn Ala Leu Gln Ser
   145                 150                 155                 160
   Gly Asn Ser Gln Glu Ser Val Thr Glu Gln Asp Ser Lys Asp Ser Thr
                   165                 170                 175
   Tyr Ser Leu Ser Ser Thr Leu Thr Leu Ser Lys Ala Asp Tyr Glu Lys
               180                 185                 190
   His Lys Val Tyr Ala Cys Glu Val Thr His Gln Gly Leu Ser Ser Pro
           195                 200                 205
   Val Thr Lys Ser Phe Asn Arg Gly Glu Cys
       210                 215
<210> 163
   <211> 115
   <212> PRT
   <213> Oryctolagus cuniculus
<400> 163
   Gln Ser Leu Glu Glu Ser Gly Gly Arg Leu Val Thr Pro Gly Gly Ser
   1               5                   10                  15
   Leu Thr Leu Thr Cys Ala Ala Ser Gly Phe Ser Leu Thr Gly Tyr Asn
               20                  25                  30
   Leu Val Trp Val Arg Gln Ala Pro Gly Lys Gly Leu Glu Trp Ile Gly
           35                  40                  45
   Phe Ile Ser Tyr Gly Asp Thr Thr Tyr Tyr Ala Ser Trp Ala Lys Gly
       50                  55                  60
   Arg Phe Thr Ile Ser Lys Thr Ser Thr Thr Val Thr Leu Thr Ile Thr
   65                  70                  75                  80
   Asp Leu Gln Pro Ser Asp Thr Gly Thr Tyr Phe Cys Ala Arg Glu Thr
                   85                  90                  95
   Ala Asn Thr Tyr Asp Tyr Gly Ile Trp Gly Pro Gly Thr Leu Val Thr
               100                 105                 110
   Val Ser Ser
                                   115
<210> 164
   <211> 445
   <212> PRT
   <213> Synthetic
<400> 164
   Gln Ser Leu Glu Glu Ser Gly Gly Arg Leu Val Thr Pro Gly Gly Ser
   1               5                   10                  15
   Leu Thr Leu Thr Cys Ala Ala Ser Gly Phe Ser Leu Thr Gly Tyr Asn
               20                  25                  30
   Leu Val Trp Val Arg Gln Ala Pro Gly Lys Gly Leu Glu Trp Ile Gly
           35                  40                  45
   Phe Ile Ser Tyr Gly Asp Thr Thr Tyr Tyr Ala Ser Trp Ala Lys Gly
       50                  55                  60
   Arg Phe Thr Ile Ser Lys Thr Ser Thr Thr Val Thr Leu Thr Ile Thr
   65                  70                  75                  80
   Asp Leu Gln Pro Ser Asp Thr Gly Thr Tyr Phe Cys Ala Arg Glu Thr
                   85                  90                  95
   Ala Asn Thr Tyr Asp Tyr Gly Ile Trp Gly Pro Gly Thr Leu Val Thr
               100                 105                 110
   Val Ser Ser Ala Ser Thr Lys Gly Pro Ser Val Phe Pro Leu Ala Pro
           115                 120                 125
   Ser Ser Lys Ser Thr Ser Gly Gly Thr Ala Ala Leu Gly Cys Leu Val
       130                 135                 140
   Lys Asp Tyr Phe Pro Glu Pro Val Thr Val Ser Trp Asn Ser Gly Ala
   145                 150                 155                 160
   Leu Thr Ser Gly Val His Thr Phe Pro Ala Val Leu Gln Ser Ser Gly
                   165                 170                 175
   Leu Tyr Ser Leu Ser Ser Val Val Thr Val Pro Ser Ser Ser Leu Gly
               180                 185                 190
   Thr Gln Thr Tyr Ile Cys Asn Val Asn His Lys Pro Ser Asn Thr Lys
           195                 200                 205
   Val Asp Lys Arg Val Glu Pro Lys Ser Cys Asp Lys Thr His Thr Cys
   210 215 220
   Pro Pro Cys Pro Ala Pro Glu Leu Leu Gly Gly Pro Ser Val Phe Leu
   225 230 235 240
   Phe Pro Pro Lys Pro Lys Asp Thr Leu Met Ile Ser Arg Thr Pro Glu
   245 250 255
   Val Thr Cys Val Val Val Asp Val Ser His Glu Asp Pro Glu Val Lys
   260 265 270
   Phe Asn Trp Tyr Val Asp Gly Val Glu Val His Asn Ala Lys Thr Lys
   275 280 285
   Pro Arg Glu Glu Gln Tyr Ala Ser Thr Tyr Arg Val Val Ser Val Leu
   290 295 300
   Thr Val Leu His Gln Asp Trp Leu Asn Gly Lys Glu Tyr Lys Cys Lys
   305 310 315 320
   Val Ser Asn Lys Ala Leu Pro Ala Pro Ile Glu Lys Thr Ile Ser Lys
   325 330 335
   Ala Lys Gly Gln Pro Arg Glu Pro Gln Val Tyr Thr Leu Pro Pro Ser
   340 345 350
   Arg Glu Glu Met Thr Lys Asn Gln Val Ser Leu Thr Cys Leu Val Lys
   355 360 365
   Gly Phe Tyr Pro Ser Asp Ile Ala Val Glu Trp Glu Ser Asn Gly Gln
   370 375 380
   Pro Glu Asn Asn Tyr Lys Thr Thr Pro Pro Val Leu Asp Ser Asp Gly
   385 390 395 400
   Ser Phe Phe Leu Tyr Ser Lys Leu Thr Val Asp Lys Ser Arg Trp Gln
   405 410 415
   Gln Gly Asn Val Phe Ser Cys Ser Val Met His Glu Ala Leu His Asn
   420 425 430
   His Tyr Thr Gln Lys Ser Leu Ser Leu Ser Pro Gly Lys
   435 440 445
<210> 165
   <211> 11
   <212> PRT
   <213> Oryctolagus cuniculus
<400> 165
   Gln Ala Ser Gln Thr Ile Ser Asn Tyr Leu Ala
   1 5 10
<210> 166
   <211> 7
   <212> PRT
   <213> Oryctolagus cuniculus
<400> 166
   Gly Ala Ser Asn Leu Glu Ser
   1 5
<210> 167
   <211> 13
   <212> PRT
   <213> Oryctolagus cuniculus
<400> 167
   Gln Gln Gly Tyr Thr Ile Ser Asn Val Asp Asn Asn Val
   1 5 10
<210> 168
   <211> 5
   <212> PRT
   <213> Oryctolagus cuniculus
<400> 168
   Gly Tyr Asn Leu Val
   1 5
<210> 169
   <211> 16
   <212> PRT
   <213> Oryctolagus cuniculus
<400> 169
   Phe Ile Ser Tyr Gly Asp Thr Thr Tyr Tyr Ala Ser Trp Ala Lys Gly
   1 5 10 15
<210> 170
   <211> 10
   <212> PRT
   <213> Oryctolagus cuniculus
<400> 170
   Glu Thr Ala Asn Thr Tyr Asp Tyr Gly Ile
   1 5 10
<210> 171
   <211> 112
   <212> PRT
   <213> Synthetic
<400> 171
   Asp Ile Gln Met Thr Gln Ser Pro Ser Thr Leu Ser Ala Ser Val Gly
   1               5                   10                  15
   Asp Arg Val Thr Ile Thr Cys Gln Ala Ser Gln Thr Ile Ser Asn Tyr
               20                  25                  30
   Leu Ala Trp Tyr Gln Gln Lys Pro Gly Lys Ala Pro Lys Leu Leu Ile
           35                  40                  45
   Tyr Gly Ala Ser Asn Leu Glu Ser Gly Val Pro Ser Arg Phe Ser Gly
       50                  55                  60
   Ser Gly Ser Gly Thr Glu Phe Thr Leu Thr Ile Ser Ser Leu Gln Pro
   65                  70                  75                  80
   Asp Asp Phe Ala Thr Tyr Tyr Cys Gln Gln Gly Tyr Thr Ile Ser Asn
                   85                  90                  95
   Val Asp Asn Asn Val Phe Gly Gly Gly Thr Lys Val Glu Ile Lys Arg
               100                 105                 110
<210> 172
   <211> 218
   <212> PRT
   <213> Synthetic
<400> 172
   Asp Ile Gln Met Thr Gln Ser Pro Ser Thr Leu Ser Ala Ser Val Gly
   1               5                   10                  15
   Asp Arg Val Thr Ile Thr Cys Gln Ala Ser Gln Thr Ile Ser Asn Tyr
               20                  25                  30
   Leu Ala Trp Tyr Gln Gln Lys Pro Gly Lys Ala Pro Lys Leu Leu Ile
           35                  40                  45
   Tyr Gly Ala Ser Asn Leu Glu Ser Gly Val Pro Ser Arg Phe Ser Gly
       50                  55                  60
   Ser Gly Ser Gly Thr Glu Phe Thr Leu Thr Ile Ser Ser Leu Gln Pro
   65                  70                  75                  80
   Asp Asp Phe Ala Thr Tyr Tyr Cys Gln Gln Gly Tyr Thr Ile Ser Asn
                   85                  90                  95
   Val Asp Asn Asn Val Phe Gly Gly Gly Thr Lys Val Glu Ile Lys Arg
               100                 105                 110
   Thr Val Ala Ala Pro Ser Val Phe Ile Phe Pro Pro Ser Asp Glu Gln
           115                 120                 125
   Leu Lys Ser Gly Thr Ala Ser Val Val Cys Leu Leu Asn Asn Phe Tyr
       130                 135                 140
   Pro Arg Glu Ala Lys Val Gln Trp Lys Val Asp Asn Ala Leu Gln Ser
   145                 150                 155                 160
   Gly Asn Ser Gln Glu Ser Val Thr Glu Gln Asp Ser Lys Asp Ser Thr
                   165                 170                 175
   Tyr Ser Leu Ser Ser Thr Leu Thr Leu Ser Lys Ala Asp Tyr Glu Lys
               180                 185                 190
   His Lys Val Tyr Ala Cys Glu Val Thr His Gln Gly Leu Ser Ser Pro
           195                 200                 205
   Val Thr Lys Ser Phe Asn Arg Gly Glu Cys
       210                 215
<210> 173
   <211> 118
   <212> PRT
   <213> Synthetic
<400> 173
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Val Ser Gly Tyr
               20                  25                  30
   Asn Leu Val Trp Val Arg Gln Ala Pro Gly Lys Gly Leu Glu Trp Val
           35                  40                  45
   Gly Phe Ile Ser Tyr Gly Asp Thr Thr Tyr Tyr Ala Ser Ser Ala Lys
       50                  55                  60
   Gly Arg Phe Thr Ile Ser Arg Asp Asn Ser Lys Asn Thr Leu Tyr Leu
   65                  70                  75                  80
   Gln Met Asn Ser Leu Arg Ala Glu Asp Thr Ala Val Tyr Tyr Cys Ala
                   85                  90                  95
   Arg Glu Thr Ala Asn Thr Tyr Asp Tyr Gly Ile Trp Gly Gln Gly Thr
               100                 105                 110
   Leu Val Thr Val Ser Ser
           115
<210> 174
   <211> 448
   <212> PRT
   <213> Synthetic
<400> 174
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Val Ser Gly Tyr
               20                  25                  30
   Asn Leu Val Trp Val Arg Gln Ala Pro Gly Lys Gly Leu Glu Trp Val
           35                  40                  45
   Gly Phe Ile Ser Tyr Gly Asp Thr Thr Tyr Tyr Ala Ser Ser Ala Lys
       50                  55                  60
   Gly Arg Phe Thr Ile Ser Arg Asp Asn Ser Lys Asn Thr Leu Tyr Leu
   65                  70                  75                  80
   Gln Met Asn Ser Leu Arg Ala Glu Asp Thr Ala Val Tyr Tyr Cys Ala
                   85                  90                  95
   Arg Glu Thr Ala Asn Thr Tyr Asp Tyr Gly Ile Trp Gly Gln Gly Thr
               100                 105                 110
   Leu Val Thr Val Ser Ser Ala Ser Thr Lys Gly Pro Ser Val Phe Pro
           115                 120                 125
   Leu Ala Pro Ser Ser Lys Ser Thr Ser Gly Gly Thr Ala Ala Leu Gly
       130                 135                 140
   Cys Leu Val Lys Asp Tyr Phe Pro Glu Pro Val Thr Val Ser Trp Asn
   145                 150                 155                 160
   Ser Gly Ala Leu Thr Ser Gly Val His Thr Phe Pro Ala Val Leu Gln
                   165                 170                 175
   Ser Ser Gly Leu Tyr Ser Leu Ser Ser Val Val Thr Val Pro Ser Ser
               180                 185                 190
   Ser Leu Gly Thr Gln Thr Tyr Ile Cys Asn Val Asn His Lys Pro Ser
   195 200 205
   Asn Thr Lys Val Asp Lys Arg Val Glu Pro Lys Ser Cys Asp Lys Thr
   210 215 220
   His Thr Cys Pro Pro Cys Pro Ala Pro Glu Leu Leu Gly Gly Pro Ser
   225 230 235 240
   Val Phe Leu Phe Pro Pro Lys Pro Lys Asp Thr Leu Met Ile Ser Arg
   245 250 255
   Thr Pro Glu Val Thr Cys Val Val Val Asp Val Ser His Glu Asp Pro
   260 265 270
   Glu Val Lys Phe Asn Trp Tyr Val Asp Gly Val Glu Val His Asn Ala
   275 280 285
   Lys Thr Lys Pro Arg Glu Glu Gln Tyr Ala Ser Thr Tyr Arg Val Val
   290 295 300
   Ser Val Leu Thr Val Leu His Gln Asp Trp Leu Asn Gly Lys Glu Tyr
   305 310 315 320
   Lys Cys Lys Val Ser Asn Lys Ala Leu Pro Ala Pro Ile Glu Lys Thr
   325 330 335
   Ile Ser Lys Ala Lys Gly Gln Pro Arg Glu Pro Gln Val Tyr Thr Leu
   340 345 350
   Pro Pro Ser Arg Glu Glu Met Thr Lys Asn Gln Val Ser Leu Thr Cys
   355 360 365
   Leu Val Lys Gly Phe Tyr Pro Ser Asp Ile Ala Val Glu Trp Glu Ser
   370 375 380
   Asn Gly Gln Pro Glu Asn Asn Tyr Lys Thr Thr Pro Pro Val Leu Asp
   385 390 395 400
   Ser Asp Gly Ser Phe Phe Leu Tyr Ser Lys Leu Thr Val Asp Lys Ser
   405 410 415
   Arg Trp Gln Gln Gly Asn Val Phe Ser Cys Ser Val Met His Glu Ala
   420 425 430
   Leu His Asn His Tyr Thr Gln Lys Ser Leu Ser Leu Ser Pro Gly Lys
   435 440 445
<210> 175
   <211> 11
   <212> PRT
   <213> Oryctolagus cuniculus
<400> 175
   Gln Ala Ser Gln Thr Ile Ser Asn Tyr Leu Ala
   1 5 10
<210> 176
   <211> 7
   <212> PRT
   <213> Oryctolagus cuniculus
<400> 176
   Gly Ala Ser Asn Leu Glu Ser
   1 5
<210> 177
   <211> 13
   <212> PRT
   <213> Oryctolagus cuniculus
<400> 177
   Gln Gln Gly Tyr Thr Ile Ser Asn Val Asp Asn Asn Val
   1 5 10
<210> 178
   <211> 5
   <212> PRT
   <213> Oryctolagus cuniculus
<400> 178
   Gly Tyr Asn Leu Val
   1 5
<210> 179
   <211> 16
   <212> PRT
   <213> Synthetic
<400> 179
   Phe Ile Ser Tyr Gly Asp Thr Thr Tyr Tyr Ala Ser Ser Ala Lys Gly
   1 5 10 15
<210> 180
   <211> 10
   <212> PRT
   <213> Oryctolagus cuniculus
<400> 180
   Glu Thr Ala Asn Thr Tyr Asp Tyr Gly Ile
   1               5                   10
<210> 181
   <211> 112
   <212> PRT
   <213> Oryctolagus cuniculus
<400> 181
   Ala Ala Val Leu Thr Gln Thr Pro Ser Pro Val Ser Ala Ala Val Gly
   1               5                   10                  15
   Gly Thr Val Ser Ile Ser Cys Gln Ser Ser Gln Asn Val Tyr Lys Asn
               20                  25                  30
   Asn Tyr Leu Ser Trp Tyr Gln Gln Lys Pro Gly Gln Pro Pro Lys Leu
           35                  40                  45
   Leu Ile Tyr Lys Ala Ser Thr Leu Ala Ser Gly Val Pro Ser Arg Phe
       50                  55                  60
   Lys Gly Ser Gly Ser Gly Thr Asp Phe Thr Leu Thr Ile Ser Asp Val
   65                  70                  75                  80
   Gln Cys Asp Ala Ala Ala Thr Tyr Tyr Cys Ala Gly Gly Tyr Ser Ser
                   85                  90                  95
   Ser Ser Asp Asn Ala Phe Gly Gly Gly Thr Glu Val Val Val Lys Arg
               100                 105                 110
<210> 182
   <211> 218
   <212> PRT
   <213> Synthetic
<400> 182
   Ala Ala Val Leu Thr Gln Thr Pro Ser Pro Val Ser Ala Ala Val Gly
   1               5                   10                  15
   Gly Thr Val Ser Ile Ser Cys Gln Ser Ser Gln Asn Val Tyr Lys Asn
               20                  25                  30
   Asn Tyr Leu Ser Trp Tyr Gln Gln Lys Pro Gly Gln Pro Pro Lys Leu
           35                  40                  45
   Leu Ile Tyr Lys Ala Ser Thr Leu Ala Ser Gly Val Pro Ser Arg Phe
       50                  55                  60
   Lys Gly Ser Gly Ser Gly Thr Asp Phe Thr Leu Thr Ile Ser Asp Val
   65                  70                  75                  80
   Gln Cys Asp Ala Ala Ala Thr Tyr Tyr Cys Ala Gly Gly Tyr Ser Ser
                   85                  90                  95
   Ser Ser Asp Asn Ala Phe Gly Gly Gly Thr Glu Val Val Val Lys Arg
               100                 105                 110
   Thr Val Ala Ala Pro Ser Val Phe Ile Phe Pro Pro Ser Asp Glu Gln
           115                 120                 125
   Leu Lys Ser Gly Thr Ala Ser Val Val Cys Leu Leu Asn Asn Phe Tyr
       130                 135                 140
   Pro Arg Glu Ala Lys Val Gln Trp Lys Val Asp Asn Ala Leu Gln Ser
   145                 150                 155                 160
   Gly Asn Ser Gln Glu Ser Val Thr Glu Gln Asp Ser Lys Asp Ser Thr
                   165                 170                 175
   Tyr Ser Leu Ser Ser Thr Leu Thr Leu Ser Lys Ala Asp Tyr Glu Lys
               180                 185                 190
   His Lys Val Tyr Ala Cys Glu Val Thr His Gln Gly Leu Ser Ser Pro
           195                 200                 205
   Val Thr Lys Ser Phe Asn Arg Gly Glu Cys
       210                 215
<210> 183
   <211> 117
   <212> PRT
   <213> Oryctolagus cuniculus
<400> 183
   Gln Ser Val Glu Ala Ser Gly Gly Arg Leu Val Met Pro Gly Gly Ser
   1               5                   10                  15
   Leu Thr Leu Thr Cys Thr Ala Ser Gly Phe Ser Leu Ser Thr Tyr Trp
               20                  25                  30
   Met Ser Trp Val Arg Gln Ala Pro Gly Lys Gly Leu Glu Trp Ile Gly
           35                  40                  45
   Asp Ile Tyr Phe Ser Asn Glu Glu Thr Asn Tyr Ala Thr Trp Ala Lys
       50                  55                  60
   Gly Arg Phe Thr Ile Ser Lys Thr Ser Thr Thr Val Asp Leu Asn Val
   65                  70                  75                  80
   Ile Ser Pro Thr Thr Glu Asp Thr Ala Thr Tyr Phe Cys Ala Arg Gly
                   85                  90                  95
   Ser Pro Asp Val Glu Ile Ala Ile Asp Met Trp Gly Gln Gly Thr Leu
               100                 105                 110
   Val Thr Val Ser Ser
           115
<210> 184
   <211> 447
   <212> PRT
   <213> Synthetic
<400> 184
   Gln Ser Val Glu Ala Ser Gly Gly Arg Leu Val Met Pro Gly Gly Ser
   1               5                   10                  15
   Leu Thr Leu Thr Cys Thr Ala Ser Gly Phe Ser Leu Ser Thr Tyr Trp
               20                  25                  30
   Met Ser Trp Val Arg Gln Ala Pro Gly Lys Gly Leu Glu Trp Ile Gly
           35                  40                  45
   Asp Ile Tyr Phe Ser Asn Glu Glu Thr Asn Tyr Ala Thr Trp Ala Lys
       50                  55                  60
   Gly Arg Phe Thr Ile Ser Lys Thr Ser Thr Thr Val Asp Leu Asn Val
   65                  70                  75                  80
   Ile Ser Pro Thr Thr Glu Asp Thr Ala Thr Tyr Phe Cys Ala Arg Gly
                   85                  90                  95
   Ser Pro Asp Val Glu Ile Ala Ile Asp Met Trp Gly Gln Gly Thr Leu
               100                 105                 110
   Val Thr Val Ser Ser Ala Ser Thr Lys Gly Pro Ser Val Phe Pro Leu
           115                 120                 125
   Ala Pro Ser Ser Lys Ser Thr Ser Gly Gly Thr Ala Ala Leu Gly Cys
       130                 135                 140
   Leu Val Lys Asp Tyr Phe Pro Glu Pro Val Thr Val Ser Trp Asn Ser
   145                 150                 155                 160
   Gly Ala Leu Thr Ser Gly Val His Thr Phe Pro Ala Val Leu Gln Ser
                   165                 170                 175
   Ser Gly Leu Tyr Ser Leu Ser Ser Val Val Thr Val Pro Ser Ser Ser
               180                 185                 190
   Leu Gly Thr Gln Thr Tyr Ile Cys Asn Val Asn His Lys Pro Ser Asn
           195                 200                 205
   Thr Lys Val Asp Lys Arg Val Glu Pro Lys Ser Cys Asp Lys Thr His
       210                 215                 220
   Thr Cys Pro Pro Cys Pro Ala Pro Glu Leu Leu Gly Gly Pro Ser Val
   225                 230                 235                 240
   Phe Leu Phe Pro Pro Lys Pro Lys Asp Thr Leu Met Ile Ser Arg Thr
                   245                 250                 255
   Pro Glu Val Thr Cys Val Val Val Asp Val Ser His Glu Asp Pro Glu
               260                 265                 270
   Val Lys Phe Asn Trp Tyr Val Asp Gly Val Glu Val His Asn Ala Lys
           275                 280                 285
   Thr Lys Pro Arg Glu Glu Gln Tyr Ala Ser Thr Tyr Arg Val Val Ser
       290                 295                 300
   Val Leu Thr Val Leu His Gln Asp Trp Leu Asn Gly Lys Glu Tyr Lys
   305                 310                 315                 320
   Cys Lys Val Ser Asn Lys Ala Leu Pro Ala Pro Ile Glu Lys Thr Ile
                   325                 330                 335
   Ser Lys Ala Lys Gly Gln Pro Arg Glu Pro Gln Val Tyr Thr Leu Pro
               340                 345                 350
   Pro Ser Arg Glu Glu Met Thr Lys Asn Gln Val Ser Leu Thr Cys Leu
           355                 360                 365
   Val Lys Gly Phe Tyr Pro Ser Asp Ile Ala Val Glu Trp Glu Ser Asn
       370                 375                 380
   Gly Gln Pro Glu Asn Asn Tyr Lys Thr Thr Pro Pro Val Leu Asp Ser
   385                 390                 395                 400
   Asp Gly Ser Phe Phe Leu Tyr Ser Lys Leu Thr Val Asp Lys Ser Arg
                   405                 410                 415
   Trp Gln Gln Gly Asn Val Phe Ser Cys Ser Val Met His Glu Ala Leu
   420 425 430
   His Asn His Tyr Thr Gln Lys Ser Leu Ser Leu Ser Pro Gly Lys
   435 440 445
<210> 185
   <211> 13
   <212> PRT
   <213> Oryctolagus cuniculus
<400> 185
   Gln Ser Ser Gln Asn Val Tyr Lys Asn Asn Tyr Leu Ser
   1 5 10
<210> 186
   <211> 7
   <212> PRT
   <213> Oryctolagus cuniculus
<400> 186
   Lys Ala Ser Thr Leu Ala Ser
   1 5
<210> 187
   <211> 11
   <212> PRT
   <213> Oryctolagus cuniculus
<400> 187
   Ala Gly Gly Tyr Ser Ser Ser Ser Asp Asn Ala
   1 5 10
<210> 188
   <211> 5
   <212> PRT
   <213> Oryctolagus cuniculus
<400> 188
   Thr Tyr Trp Met Ser
   1 5
<210> 189
   <211> 17
   <212> PRT
   <213> Oryctolagus cuniculus
<400> 189
   Asp Ile Tyr Phe Ser Asn Glu Glu Thr Asn Tyr Ala Thr Trp Ala Lys
   1 5 10 15
   Gly
<210> 190
   <211> 11
   <212> PRT
   <213> Oryctolagus cuniculus
<400> 190
   Gly Ser Pro Asp Val Glu Ile Ala Ile Asp Met
   1               5                   10
<210> 191
   <211> 112
   <212> PRT
   <213> Synthetic
<400> 191
   Asp Ile Gln Met Thr Gln Ser Pro Ser Ser Leu Ser Ala Ser Val Gly
   1               5                   10                  15
   Asp Arg Val Thr Ile Thr Cys Gln Ser Ser Gln Asn Val Tyr Lys Asn
               20                  25                  30
   Asn Tyr Leu Ser Trp Tyr Gln Gln Lys Pro Gly Lys Val Pro Lys Leu
           35                  40                  45
   Leu Ile Tyr Lys Ala Ser Thr Leu Ala Ser Gly Val Pro Ser Arg Phe
       50                  55                  60
   Ser Gly Ser Gly Ser Gly Thr Asp Phe Thr Leu Thr Ile Ser Ser Leu
   65                  70                  75                  80
   Gln Pro Glu Asp Val Ala Thr Tyr Tyr Cys Ala Gly Gly Tyr Thr Ser
                   85                  90                  95
   Ser Ser Asp Asn Ala Phe Gly Gly Gly Thr Lys Val Glu Ile Lys Arg
               100                 105                 110
<210> 192
   <211> 218
   <212> PRT
   <213> Synthetic
<400> 192
   Asp Ile Gln Met Thr Gln Ser Pro Ser Ser Leu Ser Ala Ser Val Gly
   1               5                   10                  15
   Asp Arg Val Thr Ile Thr Cys Gln Ser Ser Gln Asn Val Tyr Lys Asn
   20 25 30
   Asn Tyr Leu Ser Trp Tyr Gln Gln Lys Pro Gly Lys Val Pro Lys Leu
   35 40 45
   Leu Ile Tyr Lys Ala Ser Thr Leu Ala Ser Gly Val Pro Ser Arg Phe
   50 55 60
   Ser Gly Ser Gly Ser Gly Thr Asp Phe Thr Leu Thr Ile Ser Ser Leu
   65 70 75 80
   Gln Pro Glu Asp Val Ala Thr Tyr Tyr Cys Ala Gly Gly Tyr Thr Ser
   85 90 95
   Ser Ser Asp Asn Ala Phe Gly Gly Gly Thr Lys Val Glu Ile Lys Arg
   100 105 110
   Thr Val Ala Ala Pro Ser Val Phe Ile Phe Pro Pro Ser Asp Glu Gln
   115 120 125
   Leu Lys Ser Gly Thr Ala Ser Val Val Cys Leu Leu Asn Asn Phe Tyr
   130 135 140
   Pro Arg Glu Ala Lys Val Gln Trp Lys Val Asp Asn Ala Leu Gln Ser
   145 150 155 160
   Gly Asn Ser Gln Glu Ser Val Thr Glu Gln Asp Ser Lys Asp Ser Thr
   165 170 175
   Tyr Ser Leu Ser Ser Thr Leu Thr Leu Ser Lys Ala Asp Tyr Glu Lys
   180 185 190
   His Lys Val Tyr Ala Cys Glu Val Thr His Gln Gly Leu Ser Ser Pro
   195 200 205
   Val Thr Lys Ser Phe Asn Arg Gly Glu Cys
   210 215
<210> 193
   <211> 120
   <212> PRT
   <213> Synthetic
<400> 193
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Val Ser Thr Tyr
   20 25 30
   Trp Met Ser Trp Val Arg Gln Ala Pro Gly Lys Gly Leu Glu Trp Val
   35 40 45
   Gly Asp Ile Tyr Phe Ser Asn Glu Glu Thr Asn Tyr Ala Thr Ser Ala
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Arg Gly Ser Pro Asp Val Glu Ile Ala Ile Asp Met Trp Gly Gln
   100 105 110
   Gly Thr Leu Val Thr Val Ser Ser
   115 120
<210> 194
   <211> 450
   <212> PRT
   <213> Synthetic
<400> 194
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Val Ser Thr Tyr
   20 25 30
   Trp Met Ser Trp Val Arg Gln Ala Pro Gly Lys Gly Leu Glu Trp Val
   35 40 45
   Gly Asp Ile Tyr Phe Ser Asn Glu Glu Thr Asn Tyr Ala Thr Ser Ala
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Arg Gly Ser Pro Asp Val Glu Ile Ala Ile Asp Met Trp Gly Gln
   100 105 110
   Gly Thr Leu Val Thr Val Ser Ser Ala Ser Thr Lys Gly Pro Ser Val
           115                 120                 125
   Phe Pro Leu Ala Pro Ser Ser Lys Ser Thr Ser Gly Gly Thr Ala Ala
       130                 135                 140
   Leu Gly Cys Leu Val Lys Asp Tyr Phe Pro Glu Pro Val Thr Val Ser
   145                 150                 155                 160
   Trp Asn Ser Gly Ala Leu Thr Ser Gly Val His Thr Phe Pro Ala Val
                   165                 170                 175
   Leu Gln Ser Ser Gly Leu Tyr Ser Leu Ser Ser Val Val Thr Val Pro
               180                 185                 190
   Ser Ser Ser Leu Gly Thr Gln Thr Tyr Ile Cys Asn Val Asn His Lys
           195                 200                 205
   Pro Ser Asn Thr Lys Val Asp Lys Arg Val Glu Pro Lys Ser Cys Asp
       210                 215                 220
   Lys Thr His Thr Cys Pro Pro Cys Pro Ala Pro Glu Leu Leu Gly Gly
   225                 230                 235                 240
   Pro Ser Val Phe Leu Phe Pro Pro Lys Pro Lys Asp Thr Leu Met Ile
                   245                 250                 255
   Ser Arg Thr Pro Glu Val Thr Cys Val Val Val Asp Val Ser His Glu
               260                 265                 270
   Asp Pro Glu Val Lys Phe Asn Trp Tyr Val Asp Gly Val Glu Val His
           275                 280                 285
   Asn Ala Lys Thr Lys Pro Arg Glu Glu Gln Tyr Ala Ser Thr Tyr Arg
       290                 295                 300
   Val Val Ser Val Leu Thr Val Leu His Gln Asp Trp Leu Asn Gly Lys
   305                 310                 315                 320
   Glu Tyr Lys Cys Lys Val Ser Asn Lys Ala Leu Pro Ala Pro Ile Glu
                   325                 330                 335
   Lys Thr Ile Ser Lys Ala Lys Gly Gln Pro Arg Glu Pro Gln Val Tyr
               340                 345                 350
   Thr Leu Pro Pro Ser Arg Glu Glu Met Thr Lys Asn Gln Val Ser Leu
           355                 360                 365
   Thr Cys Leu Val Lys Gly Phe Tyr Pro Ser Asp Ile Ala Val Glu Trp
   370 375 380
   Glu Ser Asn Gly Gln Pro Glu Asn Asn Tyr Lys Thr Thr Pro Pro Val
   385 390 395 400
   Leu Asp Ser Asp Gly Ser Phe Phe Leu Tyr Ser Lys Leu Thr Val Asp
   405 410 415
   Lys Ser Arg Trp Gln Gln Gly Asn Val Phe Ser Cys Ser Val Met His
   420 425 430
   Glu Ala Leu His Asn His Tyr Thr Gln Lys Ser Leu Ser Leu Ser Pro
   435 440 445
   Gly Lys
   450
<210> 195
   <211> 13
   <212> PRT
   <213> Oryctolagus cuniculus
<400> 195
   Gln Ser Ser Gln Asn Val Tyr Lys Asn Asn Tyr Leu Ser
   1 5 10
<210> 196
   <211> 7
   <212> PRT
   <213> Oryctolagus cuniculus
<400> 196
   Lys Ala Ser Thr Leu Ala Ser
   1 5
<210> 197
   <211> 11
   <212> PRT
   <213> Oryctolagus cuniculus
<400> 197
   Ala Gly Gly Tyr Thr Ser Ser Ser Asp Asn Ala
   1 5 10
<210> 198
   <211> 5
   <212> PRT
   <213> Oryctolagus cuniculus
<400> 198
   Thr Tyr Trp Met Ser
   1 5
<210> 199
   <211> 17
   <212> PRT
   <213> Synthetic
<400> 199
   Asp Ile Tyr Phe Ser Asn Glu Glu Thr Asn Tyr Ala Thr Ser Ala Lys
   1 5 10 15
   Gly
<210> 200
   <211> 11
   <212> PRT
   <213> Synthetic
<400> 200
   Gly Ser Pro Asp Val Glu Ile Ala Ile Asp Met
   1 5 10
<210> 201
   <211> 333
   <212> DNA
   <213> Oryctolagus cuniculus
<400> 201
   gcccttgtga tgacccagac tccatcctcc gtgtctgcag ctgtgggagg cacagtcacc 60
   atcaattgcc aggccagtca gaacatttac agcaatttag cctggtatca acagagacca 120
   gggcagcgtc ccaagctcct gatctatggt gcatccaatc tggatgctgg ggtcccatcg 180
   cggttcagag gcagtggatc tgggacagag tacactctca ccatcagcga cctggagtgt 240
   gacgatgttg gcacttacta ctgtcaaagt gcttttgata gtgatagtac tgaaaatact 300
   ttcggcggag ggaccgaggt ggtggtcaaa cgt 333
<210> 202
   <211> 654
   <212> DNA
   <213> Synthetic
<400> 202
   gcccttgtga tgacccagac tccatcctcc gtgtctgcag ctgtgggagg cacagtcacc 60
   atcaattgcc aggccagtca gaacatttac agcaatttag cctggtatca acagagacca 120
   gggcagcgtc ccaagctcct gatctatggt gcatccaatc tggatgctgg ggtcccatcg 180
   cggttcagag gcagtggatc tgggacagag tacactctca ccatcagcga cctggagtgt 240
   gacgatgttg gcacttacta ctgtcaaagt gcttttgata gtgatagtac tgaaaatact 300
   ttcggcggag ggaccgaggt ggtggtcaaa cgtacggtag cggccccatc tgtcttcatc 360
   ttcccgccat ctgatgagca gttgaaatct ggaactgcct ctgttgtgtg cctgctgaat 420
   aacttctatc ccagagaggc caaagtacag tggaaggtgg ataacgccct ccaatcgggt 480
   aactcccagg agagtgtcac agagcaggac agcaaggaca gcacctacag cctcagcagc 540
   accctgacgc tgagcaaagc agactacgag aaacacaaag tctacgcctg cgaagtcacc 600
   catcagggcc tgagctcgcc cgtcacaaag agcttcaaca ggggagagtg ttag 654
<210> 203
   <211> 354
   <212> DNA
   <213> Oryctolagus cuniculus
<400> 203
   cagtcgctgg aggagtccgg gggtcgcctg gtcacgcctg ggacacccct gacactcacc 60
   tgcacagtct ctggcttctc cctcagtagc tatgcaatga gctgggtccg ccaggctcca 120
   gggaaggggc tggaatggat cggagtcatt actagtattg gtagcacagt ctacgcgagc 180
   tgggcgaaag gccgattcac catctccaaa acctcgacca cggtggatct gaaaatcacc 240
   agtccgacaa ccgaggacac ggccacctat ttctgtgcca gaggctacga tgactatgat 300
   gagatgacct actttaacat ctggggccag gggaccctcg tcaccgtctc gagc 354
<210> 204
   <211> 1347
   <212> DNA
   <213> Synthetic
<400> 204
   cagtcgctgg aggagtccgg gggtcgcctg gtcacgcctg ggacacccct gacactcacc 60
   tgcacagtct ctggcttctc cctcagtagc tatgcaatga gctgggtccg ccaggctcca 120
   gggaaggggc tggaatggat cggagtcatt actagtattg gtagcacagt ctacgcgagc 180
   tgggcgaaag gccgattcac catctccaaa acctcgacca cggtggatct gaaaatcacc 240
   agtccgacaa ccgaggacac ggccacctat ttctgtgcca gaggctacga tgactatgat 300
   gagatgacct actttaacat ctggggccag gggaccctcg tcaccgtctc gagcgcctcc 360
   accaagggcc catcggtctt ccccctggca ccctcctcca agagcacctc tgggggcaca 420
   gcggccctgg gctgcctggt caaggactac ttccccgaac cggtgacggt gtcgtggaac 480
   tcaggcgccc tgaccagcgg cgtgcacacc ttcccggctg tcctacagtc ctcaggactc 540
   tactccctca gcagcgtggt gaccgtgccc tccagcagct tgggcaccca gacctacatc 600
   tgcaacgtga atcacaagcc cagcaacacc aaggtggaca agagagttga gcccaaatct 660
   tgtgacaaaa ctcacacatg cccaccgtgc ccagcacctg aactcctggg gggaccgtca 720
   gtcttcctct tccccccaaa acccaaggac accctcatga tctcccggac ccctgaggtc 780
   acatgcgtgg tggtggacgt gagccacgaa gaccctgagg tcaagttcaa ctggtacgtg 840
   gacggcgtgg aggtgcataa tgccaagaca aagccgcggg aggagcagta cgccagcacg 900
   taccgtgtgg tcagcgtcct caccgtcctg caccaggact ggctgaatgg caaggagtac 960
   aagtgcaagg tctccaacaa agccctccca gcccccatcg agaaaaccat ctccaaagcc 1020
   aaagggcagc cccgagaacc acaggtgtac accctgcccc catcccggga ggagatgacc 1080
   aagaaccagg tcagcctgac ctgcctggtc aaaggcttct atcccagcga catcgccgtg 1140
   gagtgggaga gcaatgggca gccggagaac aactacaaga ccacgcctcc cgtgctggac 1200
   tccgacggct ccttcttcct ctacagcaag ctcaccgtgg acaagagcag gtggcagcag 1260
   gggaacgtct tctcatgctc cgtgatgcat gaggctctgc acaaccacta cacgcagaag 1320
   agcctctccc tgtctccggg taaatga 1347
<210> 205
   <211> 33
   <212> DNA
   <213> Oryctolagus cuniculus
<400> 205
   caggccagtc agaacattta cagcaattta gcc 33
<210> 206
   <211> 21
   <212> DNA
   <213> Oryctolagus cuniculus
<400> 206
   ggtgcatcca atctggatgc t 21
<210> 207
   <211> 36
   <212> DNA
   <213> Oryctolagus cuniculus
<400> 207
   caaagtgctt ttgatagtga tagtactgaa aatact 36
<210> 208
   <211> 15
   <212> DNA
   <213> Oryctolagus cuniculus
<400> 208
   agctatgcaa tgagc 15
<210> 209
   <211> 48
   <212> DNA
   <213> Oryctolagus cuniculus
<400> 209
   gtcattacta gtattggtag cacagtctac gcgagctggg cgaaaggc 48
<210> 210
   <211> 39
   <212> DNA
   <213> Oryctolagus cuniculus
<400> 210
   ggctacgatg actatgatga gatgacctac tttaacatc 39
<210> 211
   <211> 333
   <212> DNA
   <213> Synthetic
<400> 211
   gacatccaga tgacccagtc tccttccacc ctgtctgcat ctgtaggaga cagagtcacc 60
   atcacttgcc aggccagtca gaacatttac agcaacttag cctggtatca gcagaaacca 120
   ggaaaagccc ctaagctcct gatctatggt gcatccaatc tggatgctgg agtcccatca 180
   aggttctctg gcagtggatc tgggacagag tacactctca ccatcagcag cctgcagcct 240
   gatgattttg caacttacta ctgccaaagt gcttttgata gtgatagtac tgaaaacact 300
   ttcggcggag gaaccaaggt ggaaatcaaa cgt 333
<210> 212
   <211> 654
   <212> DNA
   <213> Synthetic
<400> 212
   gacatccaga tgacccagtc tccttccacc ctgtctgcat ctgtaggaga cagagtcacc     60
   atcacttgcc aggccagtca gaacatttac agcaacttag cctggtatca gcagaaacca    120
   ggaaaagccc ctaagctcct gatctatggt gcatccaatc tggatgctgg agtcccatca    180
   aggttctctg gcagtggatc tgggacagag tacactctca ccatcagcag cctgcagcct    240
   gatgattttg caacttacta ctgccaaagt gcttttgata gtgatagtac tgaaaacact    300
   ttcggcggag gaaccaaggt ggaaatcaaa cgtacggtag cggccccatc tgtcttcatc    360
   ttcccgccat ctgatgagca gttgaaatct ggaactgcct ctgttgtgtg cctgctgaat    420
   aacttctatc ccagagaggc caaagtacag tggaaggtgg ataacgccct ccaatcgggt    480
   aactcccagg agagtgtcac agagcaggac agcaaggaca gcacctacag cctcagcagc    540
   accctgacgc tgagcaaagc agactacgag aaacacaaag tctacgcctg cgaagtcacc    600
   catcagggcc tgagctcgcc cgtcacaaag agcttcaaca ggggagagtg ttag          654
<210> 213
   <211> 363
   <212> DNA
   <213> Synthetic
<400> 213
   gaggtgcagc tggtggagtc tgggggaggc ttggtccagc ctggggggtc cctgagactc     60
   tcctgtgcag cctctggatt caccgtcagt agctatgcaa tgagctgggt ccgtcaggct    120
   ccagggaagg ggctggagtg ggtcggagtc attactagta ttggtagcac agtctacgcg    180
   agcagcgcga aaggccgatt caccatctcc agagacaatt ccaagaacac cctgtatctt    240
   caaatgaaca gcctgagagc tgaggacact gctgtgtatt actgtgctag aggctacgat    300
   gactatgatg agatgaccta ctttaacatc tggggccaag ggaccctcgt caccgtctcg    360
   agc                                                                  363
<210> 214
   <211> 1356
   <212> DNA
   <213> Synthetic
<400> 214
   gaggtgcagc tggtggagtc tgggggaggc ttggtccagc ctggggggtc cctgagactc 60
   tcctgtgcag cctctggatt caccgtcagt agctatgcaa tgagctgggt ccgtcaggct 120
   ccagggaagg ggctggagtg ggtcggagtc attactagta ttggtagcac agtctacgcg 180
   agcagcgcga aaggccgatt caccatctcc agagacaatt ccaagaacac cctgtatctt 240
   caaatgaaca gcctgagagc tgaggacact gctgtgtatt actgtgctag aggctacgat 300
   gactatgatg agatgaccta ctttaacatc tggggccaag ggaccctcgt caccgtctcg 360
   agcgcctcca ccaagggccc atcggtcttc cccctggcac cctcctccaa gagcacctct 420
   gggggcacag cggccctggg ctgcctggtc aaggactact tccccgaacc ggtgacggtg 480
   tcgtggaact caggcgccct gaccagcggc gtgcacacct tcccggctgt cctacagtcc 540
   tcaggactct actccctcag cagcgtggtg accgtgccct ccagcagctt gggcacccag 600
   acctacatct gcaacgtgaa tcacaagccc agcaacacca aggtggacaa gagagttgag 660
   cccaaatctt gtgacaaaac tcacacatgc ccaccgtgcc cagcacctga actcctgggg 720
   ggaccgtcag tcttcctctt ccccccaaaa cccaaggaca ccctcatgat ctcccggacc 780
   cctgaggtca catgcgtggt ggtggacgtg agccacgaag accctgaggt caagttcaac 840
   tggtacgtgg acggcgtgga ggtgcataat gccaagacaa agccgcggga ggagcagtac 900
   gccagcacgt accgtgtggt cagcgtcctc accgtcctgc accaggactg gctgaatggc 960
   aaggagtaca agtgcaaggt ctccaacaaa gccctcccag cccccatcga gaaaaccatc 1020
   tccaaagcca aagggcagcc ccgagaacca caggtgtaca ccctgccccc atcccgggag 1080
   gagatgacca agaaccaggt cagcctgacc tgcctggtca aaggcttcta tcccagcgac 1140
   atcgccgtgg agtgggagag caatgggcag ccggagaaca actacaagac cacgcctccc 1200
   gtgctggact ccgacggctc cttcttcctc tacagcaagc tcaccgtgga caagagcagg 1260
   tggcagcagg ggaacgtctt ctcatgctcc gtgatgcatg aggctctgca caaccactac 1320
   acgcagaaga gcctctccct gtctccgggt aaatga 1356
<210> 215
   <211> 33
   <212> DNA
   <213> Oryctolagus cuniculus
<400> 215
   caggccagtc agaacattta cagcaactta gcc 33
<210> 216
   <211> 21
   <212> DNA
   <213> Oryctolagus cuniculus
<400> 216
   ggtgcatcca atctggatgc t 21
<210> 217
   <211> 36
   <212> DNA
   <213> Oryctolagus cuniculus
<400> 217
   caaagtgctt ttgatagtga tagtactgaa aacact 36
<210> 218
   <211> 15
   <212> DNA
   <213> Oryctolagus cuniculus
<400> 218
   agctatgcaa tgagc 15
<210> 219
   <211> 48
   <212> DNA
   <213> Synthetic
<400> 219
   gtcattacta gtattggtag cacagtctac gcgagcagcg cgaaaggc 48
<210> 220
   <211> 39
   <212> DNA
   <213> Oryctolagus cuniculus
<400> 220
   ggctacgatg actatgatga gatgacctac tttaacatc 39
<210> 221
   <211> 336
   <212> DNA
   <213> Oryctolagus cuniculus
<400> 221
   gcagccgtgc tgacccagac accatcgccc gtgtctgcag ctatgggaga cacagtcacc      60
   atcaagtgcc agtccagtca gagtgtttat aagaacaact acttatcctg gtatcagcag     120
   aaaccagggc agcctcccag gctcctgatc tatgatgcat ccaatctgcc atctggggtc     180
   ccatcacggt tcagcggcag tggatctggg acacagttca ctctcaccat cagcggcgtg     240
   cagtgtgacg atgctgccac ttactactgt ctaggcgatt atgatgatga tgctgataat     300
   gctttcggcg gagggaccga ggtggtggtc aaacgt                               336
<210> 222
   <211> 657
   <212> DNA
   <213> Synthetic
<400> 222
   gcagccgtgc tgacccagac accatcgccc gtgtctgcag ctatgggaga cacagtcacc    60
   atcaagtgcc agtccagtca gagtgtttat aagaacaact acttatcctg gtatcagcag   120
   aaaccagggc agcctcccag gctcctgatc tatgatgcat ccaatctgcc atctggggtc   180
   ccatcacggt tcagcggcag tggatctggg acacagttca ctctcaccat cagcggcgtg   240
   cagtgtgacg atgctgccac ttactactgt ctaggcgatt atgatgatga tgctgataat   300
   gctttcggcg gagggaccga ggtggtggtc aaacgtacgg tagcggcccc atctgtcttc   360
   atcttcccgc catctgatga gcagttgaaa tctggaactg cctctgttgt gtgcctgctg   420
   aataacttct atcccagaga ggccaaagta cagtggaagg tggataacgc cctccaatcg   480
   ggtaactccc aggagagtgt cacagagcag gacagcaagg acagcaccta cagcctcagc   540
   agcaccctga cgctgagcaa agcagactac gagaaacaca aagtctacgc ctgcgaagtc   600
   acccatcagg gcctgagctc gcccgtcaca aagagcttca acaggggaga gtgttag      657
<210> 223
   <211> 339
   <212> DNA
   <213> Oryctolagus cuniculus
<400> 223
   cagtcggtgg aggagtccgg gggtcgcctg gtcacgcctg ggacacccct gacactcacc    60
   tgcacagtct ctggattctc cctcagtagc tatgtaatga tctgggtccg ccaggctcca   120
   gggaaggggc tggaatacat cggaatcact tggagtgctg gtacatacta cgcgagctgg   180
   gcgaaaggcc gattcaccat ctccaaaacc tcgtcgacca cggtggatct gaaaatcacc   240
   agtccgacaa ccgaggacac ggccacctat ttctgtgccg gaggtggtgg tagtatttat   300
   gatatttggg gcccgggcac cctggtcacc gtctcgagc                          339
<210> 224
   <211> 1332
   <212> DNA
   <213> Synthetic
<400> 224
   cagtcggtgg aggagtccgg gggtcgcctg gtcacgcctg ggacacccct gacactcacc      60
   tgcacagtct ctggattctc cctcagtagc tatgtaatga tctgggtccg ccaggctcca     120
   gggaaggggc tggaatacat cggaatcact tggagtgctg gtacatacta cgcgagctgg     180
   gcgaaaggcc gattcaccat ctccaaaacc tcgtcgacca cggtggatct gaaaatcacc     240
   agtccgacaa ccgaggacac ggccacctat ttctgtgccg gaggtggtgg tagtatttat     300
   gatatttggg gcccgggcac cctggtcacc gtctcgagcg cctccaccaa gggcccatcg     360
   gtcttccccc tggcaccctc ctccaagagc acctctgggg gcacagcggc cctgggctgc     420
   ctggtcaagg actacttccc cgaaccggtg acggtgtcgt ggaactcagg cgccctgacc     480
   agcggcgtgc acaccttccc ggctgtccta cagtcctcag gactctactc cctcagcagc     540
   gtggtgaccg tgccctccag cagcttgggc acccagacct acatctgcaa cgtgaatcac     600
   aagcccagca acaccaaggt ggacaagaga gttgagccca aatcttgtga caaaactcac     660
   acatgcccac cgtgcccagc acctgaactc ctggggggac cgtcagtctt cctcttcccc     720
   ccaaaaccca aggacaccct catgatctcc cggacccctg aggtcacatg cgtggtggtg     780
   gacgtgagcc acgaagaccc tgaggtcaag ttcaactggt acgtggacgg cgtggaggtg     840
   cataatgcca agacaaagcc gcgggaggag cagtacgcca gcacgtaccg tgtggtcagc     900
   gtcctcaccg tcctgcacca ggactggctg aatggcaagg agtacaagtg caaggtctcc     960
   aacaaagccc tcccagcccc catcgagaaa accatctcca aagccaaagg gcagccccga    1020
   gaaccacagg tgtacaccct gcccccatcc cgggaggaga tgaccaagaa ccaggtcagc    1080
   ctgacctgcc tggtcaaagg cttctatccc agcgacatcg ccgtggagtg ggagagcaat    1140
   gggcagccgg agaacaacta caagaccacg cctcccgtgc tggactccga cggctccttc    1200
   ttcctctaca gcaagctcac cgtggacaag agcaggtggc agcaggggaa cgtcttctca    1260
   tgctccgtga tgcatgaggc tctgcacaac cactacacgc agaagagcct ctccctgtct    1320
   ccgggtaaat ga                                                        1332
<210> 225
   <211> 39
   <212> DNA
   <213> Oryctolagus cuniculus
<400> 225
   cagtccagtc agagtgttta taagaacaac tacttatcc 39
<210> 226
   <211> 21
   <212> DNA
   <213> Oryctolagus cuniculus
<400> 226
   gatgcatcca atctgccatc t 21
<210> 227
   <211> 33
   <212> DNA
   <213> Oryctolagus cuniculus
<400> 227
   ctaggcgatt atgatgatga tgctgataat gct 33
<210> 228
   <211> 15
   <212> DNA
   <213> Oryctolagus cuniculus
<400> 228
   agctatgtaa tgatc 15
<210> 229
   <211> 45
   <212> DNA
   <213> Oryctolagus cuniculus
<400> 229
   atcacttgga gtgctggtac atactacgcg agctgggcga aaggc 45
<210> 230
   <211> 24
   <212> DNA
   <213> Oryctolagus cuniculus
<400> 230
   ggtggtggta gtatttatga tatt 24
<210> 231
   <211> 336
   <212> DNA
   <213> Synthetic
<400> 231
   gacatccaga tgacccagtc tccttccacc ctgtctgcat ctgtaggaga cagagtcacc 60
   atcacttgcc agtccagtca gagtgtctat aagaacaact acttatcctg gtatcagcag 120
   aaaccaggaa aagcccctaa gctcctgatc tatgatgcat ccaatctgcc atctggagtc 180
   ccatcaaggt tcagcggcag tggatctgga acagaattca ctctcaccat cagcagcctg 240
   cagcctgatg attttgcaac ttattactgc ctaggcgatt atgatgatga tgctgataat 300
   gctttcggcg gaggaaccaa ggtggaaatc aaacgt 336
<210> 232
   <211> 657
   <212> DNA
   <213> Synthetic
<400> 232
   gacatccaga tgacccagtc tccttccacc ctgtctgcat ctgtaggaga cagagtcacc 60
   atcacttgcc agtccagtca gagtgtctat aagaacaact acttatcctg gtatcagcag 120
   aaaccaggaa aagcccctaa gctcctgatc tatgatgcat ccaatctgcc atctggagtc 180
   ccatcaaggt tcagcggcag tggatctgga acagaattca ctctcaccat cagcagcctg 240
   cagcctgatg attttgcaac ttattactgc ctaggcgatt atgatgatga tgctgataat 300
   gctttcggcg gaggaaccaa ggtggaaatc aaacgtacgg tagcggcccc atctgtcttc 360
   atcttcccgc catctgatga gcagttgaaa tctggaactg cctctgttgt gtgcctgctg 420
   aataacttct atcccagaga ggccaaagta cagtggaagg tggataacgc cctccaatcg 480
   ggtaactccc aggagagtgt cacagagcag gacagcaagg acagcaccta cagcctcagc 540
   agcaccctga cgctgagcaa agcagactac gagaaacaca aagtctacgc ctgcgaagtc 600
   acccatcagg gcctgagctc gcccgtcaca aagagcttca acaggggaga gtgttag 657
<210> 233
   <211> 345
   <212> DNA
   <213> Synthetic
<400> 233
   gaggtgcagc tggtggagtc tgggggaggc ttggtccagc ctggggggtc cctgagactc 60
   tcctgtgcag cctctggatt caccgtcagt agctatgtaa tgatctgggt ccgtcaggct 120
   ccagggaagg ggctggagta catcggaatc acttggagtg ctggtacata ctacgcgagc 180
   agtgcgaaag gccgattcac catctccaga gacaattcca agaacaccct gtatcttcaa 240
   atgaacagcc tgagagctga ggacactgct gtgtattact gtgctggagg tggtggtagt 300
   atctatgata tttggggcca agggaccctc gtcaccgtct cgagc 345
<210> 234
   <211> 1338
   <212> DNA
   <213> Synthetic
<400> 234
   gaggtgcagc tggtggagtc tgggggaggc ttggtccagc ctggggggtc cctgagactc 60
   tcctgtgcag cctctggatt caccgtcagt agctatgtaa tgatctgggt ccgtcaggct 120
   ccagggaagg ggctggagta catcggaatc acttggagtg ctggtacata ctacgcgagc 180
   agtgcgaaag gccgattcac catctccaga gacaattcca agaacaccct gtatcttcaa 240
   atgaacagcc tgagagctga ggacactgct gtgtattact gtgctggagg tggtggtagt 300
   atctatgata tttggggcca agggaccctc gtcaccgtct cgagcgcctc caccaagggc 360
   ccatcggtct tccccctggc accctcctcc aagagcacct ctgggggcac agcggccctg 420
   ggctgcctgg tcaaggacta cttccccgaa ccggtgacgg tgtcgtggaa ctcaggcgcc 480
   ctgaccagcg gcgtgcacac cttcccggct gtcctacagt cctcaggact ctactccctc 540
   agcagcgtgg tgaccgtgcc ctccagcagc ttgggcaccc agacctacat ctgcaacgtg 600
   aatcacaagc ccagcaacac caaggtggac aagagagttg agcccaaatc ttgtgacaaa 660
   actcacacat gcccaccgtg cccagcacct gaactcctgg ggggaccgtc agtcttcctc 720
   ttccccccaa aacccaagga caccctcatg atctcccgga cccctgaggt cacatgcgtg 780
   gtggtggacg tgagccacga agaccctgag gtcaagttca actggtacgt ggacggcgtg 840
   gaggtgcata atgccaagac aaagccgcgg gaggagcagt acgccagcac gtaccgtgtg 900
   gtcagcgtcc tcaccgtcct gcaccaggac tggctgaatg gcaaggagta caagtgcaag 960
   gtctccaaca aagccctccc agcccccatc gagaaaacca tctccaaagc caaagggcag 1020
   ccccgagaac cacaggtgta caccctgccc ccatcccggg aggagatgac caagaaccag 1080
   gtcagcctga cctgcctggt caaaggcttc tatcccagcg acatcgccgt ggagtgggag 1140
   agcaatgggc agccggagaa caactacaag accacgcctc ccgtgctgga ctccgacggc 1200
   tccttcttcc tctacagcaa gctcaccgtg gacaagagca ggtggcagca ggggaacgtc 1260
   ttctcatgct ccgtgatgca tgaggctctg cacaaccact acacgcagaa gagcctctcc 1320
   ctgtctccgg gtaaatga 1338
<210> 235
   <211> 39
   <212> DNA
   <213> Oryctolagus cuniculus
<400> 235
   cagtccagtc agaatgttta taagaacaac tacttatcc 39
<210> 236
   <211> 21
   <212> DNA
   <213> Oryctolagus cuniculus
<400> 236
   aaggcatcca ctctggcatc t 21
<210> 237
   <211> 32
   <212> DNA
   <213> Oryctolagus cuniculus
<400> 237
   gcaggcggtt ataccagtag tagtgataat gc 32
<210> 238
   <211> 15
   <212> DNA
   <213> Oryctolagus cuniculus
<400> 238
   agctatgtaa tgatc 15
<210> 239
   <211> 45
   <212> DNA
   <213> Synthetic
<400> 239
   atcacttgga gtgctggtac atactacgcg agcagtgcga aaggc 45
<210> 240
   <211> 24
   <212> DNA
   <213> Oryctolagus cuniculus
<400> 240
   ggtggtggta gtatctatga tatt 24
<210> 241
   <211> 333
   <212> DNA
   <213> Oryctolagus cuniculus
<400> 241
   gcctatgata tgacccagac tccagcctct gtggaggtag ctgtgggagg cacagtcacc 60
   atcaagtgcc aggccagtca gagcatttac agcaatttag cctggtatca gcagagacca 120
   gggcagcctc ccaagctcct gatctatgat gcatccactc tggaatctgg ggtcccatcg 180
   cggttcaaag gcagtggatc tgggacagag tacactctca ccatcagcgg cgtggagtgt 240
   gccgatgctg cctcttacta ctgtcaacag ggttttactg ttagtgatat tgataatgct 300
   ttcggcggag ggaccgaggt ggtggtcaaa cgt 333
<210> 242
   <211> 654
   <212> DNA
   <213> Synthetic
<400> 242
   gcctatgata tgacccagac tccagcctct gtggaggtag ctgtgggagg cacagtcacc 60
   atcaagtgcc aggccagtca gagcatttac agcaatttag cctggtatca gcagagacca 120
   gggcagcctc ccaagctcct gatctatgat gcatccactc tggaatctgg ggtcccatcg 180
   cggttcaaag gcagtggatc tgggacagag tacactctca ccatcagcgg cgtggagtgt 240
   gccgatgctg cctcttacta ctgtcaacag ggttttactg ttagtgatat tgataatgct 300
   ttcggcggag ggaccgaggt ggtggtcaaa cgtacggtag cggccccatc tgtcttcatc 360
   ttcccgccat ctgatgagca gttgaaatct ggaactgcct ctgttgtgtg cctgctgaat 420
   aacttctatc ccagagaggc caaagtacag tggaaggtgg ataacgccct ccaatcgggt 480
   aactcccagg agagtgtcac agagcaggac agcaaggaca gcacctacag cctcagcagc 540
   accctgacgc tgagcaaagc agactacgag aaacacaaag tctacgcctg cgaagtcacc 600
   catcagggcc tgagctcgcc cgtcacaaag agcttcaaca ggggagagtg ttag 654
<210> 243
   <211> 354
   <212> DNA
   <213> Oryctolagus cuniculus
<400> 243
   cagtcggtgg aggagtccgg gggtcgcctg gtcacgcctg ggacacccct gacactcacc 60
   tgcacagtct ctggattctc cctcagtaac tatgcagtgg gctgggtccg ccaggctcca 120
   gggaaggggc tggaatggat cggaatcatt ggtcgtaatg gtaacacatg gtacgcgagc 180
   tgggcaagag gccgattcac catctccaaa acctcgacca cggtggatct gaaaatcacc 240
   agtccgacaa gcgaggacac ggccacatat ttctgtgcca gaggatatgg ccgtagtgtt 300
   gcttattacg tctttaacat ctggggccca ggcaccctcg tcaccgtctc gagc 354
<210> 244
   <211> 1347
   <212> DNA
   <213> Synthetic
<400> 244
   cagtcggtgg aggagtccgg gggtcgcctg gtcacgcctg ggacacccct gacactcacc 60
   tgcacagtct ctggattctc cctcagtaac tatgcagtgg gctgggtccg ccaggctcca 120
   gggaaggggc tggaatggat cggaatcatt ggtcgtaatg gtaacacatg gtacgcgagc 180
   tgggcaagag gccgattcac catctccaaa acctcgacca cggtggatct gaaaatcacc 240
   agtccgacaa gcgaggacac ggccacatat ttctgtgcca gaggatatgg ccgtagtgtt 300
   gcttattacg tctttaacat ctggggccca ggcaccctcg tcaccgtctc gagcgcctcc 360
   accaagggcc catcggtctt ccccctggca ccctcctcca agagcacctc tgggggcaca 420
   gcggccctgg gctgcctggt caaggactac ttccccgaac cggtgacggt gtcgtggaac 480
   tcaggcgccc tgaccagcgg cgtgcacacc ttcccggctg tcctacagtc ctcaggactc 540
   tactccctca gcagcgtggt gaccgtgccc tccagcagct tgggcaccca gacctacatc 600
   tgcaacgtga atcacaagcc cagcaacacc aaggtggaca agagagttga gcccaaatct 660
   tgtgacaaaa ctcacacatg cccaccgtgc ccagcacctg aactcctggg gggaccgtca 720
   gtcttcctct tccccccaaa acccaaggac accctcatga tctcccggac ccctgaggtc 780
   acatgcgtgg tggtggacgt gagccacgaa gaccctgagg tcaagttcaa ctggtacgtg 840
   gacggcgtgg aggtgcataa tgccaagaca aagccgcggg aggagcagta cgccagcacg 900
   taccgtgtgg tcagcgtcct caccgtcctg caccaggact ggctgaatgg caaggagtac 960
   aagtgcaagg tctccaacaa agccctccca gcccccatcg agaaaaccat ctccaaagcc 1020
   aaagggcagc cccgagaacc acaggtgtac accctgcccc catcccggga ggagatgacc 1080
   aagaaccagg tcagcctgac ctgcctggtc aaaggcttct atcccagcga catcgccgtg 1140
   gagtgggaga gcaatgggca gccggagaac aactacaaga ccacgcctcc cgtgctggac 1200
   tccgacggct ccttcttcct ctacagcaag ctcaccgtgg acaagagcag gtggcagcag 1260
   gggaacgtct tctcatgctc cgtgatgcat gaggctctgc acaaccacta cacgcagaag 1320
   agcctctccc tgtctccggg taaatga 1347
<210> 245
   <211> 33
   <212> DNA
   <213> Oryctolagus cuniculus
<400> 245
   caggccagtc agagcattta cagcaattta gcc 33
<210> 246
   <211> 21
   <212> DNA
   <213> Oryctolagus cuniculus
<400> 246
   gatgcatcca ctctggaatc t 21
<210> 247
   <211> 36
   <212> DNA
   <213> Oryctolagus cuniculus
<400> 247
   caacagggtt ttactgttag tgatattgat aatgct 36
<210> 248
   <211> 15
   <212> DNA
   <213> Oryctolagus cuniculus
<400> 248
   aactatgcag tgggc 15
<210> 249
   <211> 48
   <212> DNA
   <213> Oryctolagus cuniculus
<400> 249
   atcattggtc gtaatggtaa cacatggtac gcgagctggg caagaggc 48
<210> 250
   <211> 39
   <212> DNA
   <213> Oryctolagus cuniculus
<400> 250
   ggatatggcc gtagtgttgc ttattacgtc tttaacatc 39
<210> 251
   <211> 333
   <212> DNA
   <213> Synthetic
<400> 251
   gacatccaga tgacccagtc tccttccacc ctgtctgcat ctgtaggaga cagagtcacc 60
   atcacttgcc aggccagtca gagcatttac agcaatcttg cctggtatca gcagaaacca 120
   ggaaaagccc ctaagctcct gatctatgat gcatccactc tggaatctgg agtcccatca 180
   aggttcagcg gcagtggatc tgggacagag tacactctca ccatcagcag cctgcagcct 240
   gatgattttg caacttacta ctgccaacag ggttttactg ttagtgatat tgataatgct 300
   ttcggcggag gaaccaaggt ggaaatcaaa cgt 333
<210> 252
   <211> 654
   <212> DNA
   <213> Synthetic
<400> 252
   gacatccaga tgacccagtc tccttccacc ctgtctgcat ctgtaggaga cagagtcacc      60
   atcacttgcc aggccagtca gagcatttac agcaatcttg cctggtatca gcagaaacca     120
   ggaaaagccc ctaagctcct gatctatgat gcatccactc tggaatctgg agtcccatca     180
   aggttcagcg gcagtggatc tgggacagag tacactctca ccatcagcag cctgcagcct     240
   gatgattttg caacttacta ctgccaacag ggttttactg ttagtgatat tgataatgct     300
   ttcggcggag gaaccaaggt ggaaatcaaa cgtacggtag cggccccatc tgtcttcatc     360
   ttcccgccat ctgatgagca gttgaaatct ggaactgcct ctgttgtgtg cctgctgaat     420
   aacttctatc ccagagaggc caaagtacag tggaaggtgg ataacgccct ccaatcgggt     480
   aactcccagg agagtgtcac agagcaggac agcaaggaca gcacctacag cctcagcagc     540
   accctgacgc tgagcaaagc agactacgag aaacacaaag tctacgcctg cgaagtcacc     600
   catcagggcc tgagctcgcc cgtcacaaag agcttcaaca ggggagagtg ttag           654
<210> 253
   <211> 363
   <212> DNA
   <213> Synthetic
<400> 253
   gaggtgcagc tggtggagtc tgggggaggc ttggtccagc ctggggggtc cctgagactc      60
   tcctgtgcag cctctggatt caccgtcagt aactatgcag tgggctgggt ccgtcaggct     120
   ccagggaagg ggctggagtg ggtcggaatc attggtcgta atggtaacac atggtacgcg     180
   agctctgcaa gaggccgatt caccatctcc agagacaatt ccaagaacac cctgtatctt     240
   caaatgaaca gcctgagagc tgaggacact gctgtgtatt actgtgctag aggatatggc     300
   cgtagtgttg cttattacgt ctttaacatc tggggcccag ggaccctcgt caccgtctcg     360
   agc                                                                   363
<210> 254
   <211> 1356
   <212> DNA
   <213> Synthetic
<400> 254
   gaggtgcagc tggtggagtc tgggggaggc ttggtccagc ctggggggtc cctgagactc 60
   tcctgtgcag cctctggatt caccgtcagt aactatgcag tgggctgggt ccgtcaggct 120
   ccagggaagg ggctggagtg ggtcggaatc attggtcgta atggtaacac atggtacgcg 180
   agctctgcaa gaggccgatt caccatctcc agagacaatt ccaagaacac cctgtatctt 240
   caaatgaaca gcctgagagc tgaggacact gctgtgtatt actgtgctag aggatatggc 300
   cgtagtgttg cttattacgt ctttaacatc tggggcccag ggaccctcgt caccgtctcg 360
   agcgcctcca ccaagggccc atcggtcttc cccctggcac cctcctccaa gagcacctct 420
   gggggcacag cggccctggg ctgcctggtc aaggactact tccccgaacc ggtgacggtg 480
   tcgtggaact caggcgccct gaccagcggc gtgcacacct tcccggctgt cctacagtcc 540
   tcaggactct actccctcag cagcgtggtg accgtgccct ccagcagctt gggcacccag 600
   acctacatct gcaacgtgaa tcacaagccc agcaacacca aggtggacaa gagagttgag 660
   cccaaatctt gtgacaaaac tcacacatgc ccaccgtgcc cagcacctga actcctgggg 720
   ggaccgtcag tcttcctctt ccccccaaaa cccaaggaca ccctcatgat ctcccggacc 780
   cctgaggtca catgcgtggt ggtggacgtg agccacgaag accctgaggt caagttcaac 840
   tggtacgtgg acggcgtgga ggtgcataat gccaagacaa agccgcggga ggagcagtac 900
   gccagcacgt accgtgtggt cagcgtcctc accgtcctgc accaggactg gctgaatggc 960
   aaggagtaca agtgcaaggt ctccaacaaa gccctcccag cccccatcga gaaaaccatc 1020
   tccaaagcca aagggcagcc ccgagaacca caggtgtaca ccctgccccc atcccgggag 1080
   gagatgacca agaaccaggt cagcctgacc tgcctggtca aaggcttcta tcccagcgac 1140
   atcgccgtgg agtgggagag caatgggcag ccggagaaca actacaagac cacgcctccc 1200
   gtgctggact ccgacggctc cttcttcctc tacagcaagc tcaccgtgga caagagcagg 1260
   tggcagcagg ggaacgtctt ctcatgctcc gtgatgcatg aggctctgca caaccactac 1320
   acgcagaaga gcctctccct gtctccgggt aaatga 1356
<210> 255
   <211> 33
   <212> DNA
   <213> Oryctolagus cuniculus
<400> 255
   caggccagtc agagcattta cagcaatctt gcc 33
<210> 256
   <211> 21
   <212> DNA
   <213> Oryctolagus cuniculus
<400> 256
   gatgcatcca ctctggaatc t 21
<210> 257
   <211> 36
   <212> DNA
   <213> Oryctolagus cuniculus
<400> 257
   caacagggtt ttactgttag tgatattgat aatgct 36
<210> 258
   <211> 15
   <212> DNA
   <213> Oryctolagus cuniculus
<400> 258
   aactatgcag tgggc 15
<210> 259
   <211> 48
   <212> DNA
   <213> Synthetic
<400> 259
   atcattggtc gtaatggtaa cacatggtac gcgagctctg caagaggc 48
<210> 260
   <211> 39
   <212> DNA
   <213> Oryctolagus cuniculus
<400> 260
   ggatatggcc gtagtgttgc ttattacgtc tttaacatc 39
<210> 261
   <211> 336
   <212> DNA
   <213> Oryctolagus cuniculus
<400> 261
   gccgatgttg tgatgaccca gactccagcc tccgtgtctc aacctgtggg aggcacagtc        60
   accatcaagt gccaggccag tgaggacatt tataacttat tggcctggta tcagcagaaa       120
   ccagggcagc ctcccaagct cctgatctat tctgcatcca ctctggcatc tggggtccca       180
   tcgcggttca aaggcagtgg atctgggaca gagtacactc tcaccatcag cggcctggag       240
   tgtgccgatg ctgccactta ctactgtcaa aacaattatc ttgttactac ttatggtgtt       300
   gctttcggcg gagggaccga ggtggtggtc aaacgt                                 336
<210> 262
   <211> 657
   <212> DNA
   <213> Synthetic
<400> 262
   gccgatgttg tgatgaccca gactccagcc tccgtgtctc aacctgtggg aggcacagtc       60
   accatcaagt gccaggccag tgaggacatt tataacttat tggcctggta tcagcagaaa      120
   ccagggcagc ctcccaagct cctgatctat tctgcatcca ctctggcatc tggggtccca      180
   tcgcggttca aaggcagtgg atctgggaca gagtacactc tcaccatcag cggcctggag      240
   tgtgccgatg ctgccactta ctactgtcaa aacaattatc ttgttactac ttatggtgtt      300
   gctttcggcg gagggaccga ggtggtggtc aaacgtacgg tagcggcccc atctgtcttc      360
   atcttcccgc catctgatga gcagttgaaa tctggaactg cctctgttgt gtgcctgctg      420
   aataacttct atcccagaga ggccaaagta cagtggaagg tggataacgc cctccaatcg      480
   ggtaactccc aggagagtgt cacagagcag gacagcaagg acagcaccta cagcctcagc      540
   agcaccctga cgctgagcaa agcagactac gagaaacaca aagtctacgc ctgcgaagtc      600
   acccatcagg gcctgagctc gcccgtcaca aagagcttca acaggggaga gtgttag         657
<210> 263
   <211> 360
   <212> DNA
   <213> Oryctolagus cuniculus
<400> 263
   caggagcagc tgaaggagtc cgggggtcgc ctggtcacgc ctgggacacc cctgacactc       60
   acctgtacag tctctggatt ctccctcagt agctatgcaa tgatctgggt ccgccaggct      120
   ccagggaagg ggctggaata catcggatac attgatactg atactagcgc atactacgcg      180
   agctgggtga aaggccgatt caccatctcc agaacctcga ccacggtgga tctcaaaatc      240
   actagtccga caaccgagga cacggccacc tatttctgtg ccagatctta tgctgcttat      300
   ggtggttatc ctgctacttt tgatccctgg ggcccaggca ccctggtcac cgtctcgagc      360
<210> 264
   <211> 1353
   <212> DNA
   <213> Synthetic
<400> 264 cagaagagcc tctccctgtc tccgggtaaa tga 1353
<210> 265
   <211> 33
   <212> DNA
   <213> Oryctolagus cuniculus
<400> 265
   caggccagtg aggacattta taacttattg gcc 33
<210> 266
   <211> 21
   <212> DNA
   <213> Oryctolagus cuniculus
<400> 266
   tctgcatcca ctctggcatc t 21
<210> 267
   <211> 36
   <212> DNA
   <213> Oryctolagus cuniculus
<400> 267
   caaaacaatt atcttgttac tacttatggt gttgct 36
<210> 268
   <211> 15
   <212> DNA
   <213> Oryctolagus cuniculus
<400> 268
   agctatgcaa tgatc 15
<210> 269
   <211> 48
   <212> DNA
   <213> Oryctolagus cuniculus
<400> 269
   tacattgata ctgatactag cgcatactac gcgagctggg tgaaaggc 48
<210> 270
   <211> 36
   <212> DNA
   <213> Oryctolagus cuniculus
<400> 270
   tcttatgctg cttatggtgg ttatcctgct actttt 36
<210> 271
   <211> 333
   <212> DNA
   <213> Synthetic
<400> 271
   gacatccaga tgacccagtc tccatcctcc ctgtctgcat ctgtaggaga cagagtcacc 60
   atcacttgcc aggccagtga ggacatttac aacttattgg cctggtatca gcagaaacca 120
   gggaaagtcc ctaagctcct gatctattct gcatccactc tggcatctgg ggtcccatct 180
   cgtttcagtg gcagtggatc tgggacagat tacactctca ccatcagcag cctgcagcct 240
   gaagatgttg caacttatta ctgtcaaaac aactatcttg ttactactta tggtgttgct 300
   ttcggcggag gaaccaaggt ggaaatcaaa cgt 333
<210> 272
   <211> 654
   <212> DNA
   <213> Synthetic
<400> 272
   gacatccaga tgacccagtc tccatcctcc ctgtctgcat ctgtaggaga cagagtcacc        60
   atcacttgcc aggccagtga ggacatttac aacttattgg cctggtatca gcagaaacca       120
   gggaaagtcc ctaagctcct gatctattct gcatccactc tggcatctgg ggtcccatct       180
   cgtttcagtg gcagtggatc tgggacagat tacactctca ccatcagcag cctgcagcct       240
   gaagatgttg caacttatta ctgtcaaaac aactatcttg ttactactta tggtgttgct       300
   ttcggcggag gaaccaaggt ggaaatcaaa cgtacggtag cggccccatc tgtcttcatc       360
   ttcccgccat ctgatgagca gttgaaatct ggaactgcct ctgttgtgtg cctgctgaat       420
   aacttctatc ccagagaggc caaagtacag tggaaggtgg ataacgccct ccaatcgggt       480
   aactcccagg agagtgtcac agagcaggac agcaaggaca gcacctacag cctcagcagc       540
   accctgacgc tgagcaaagc agactacgag aaacacaaag tctacgcctg cgaagtcacc       600
   catcagggcc tgagctcgcc cgtcacaaag agcttcaaca ggggagagtg ttag             654
<210> 273
   <211> 366
   <212> DNA
   <213> Synthetic
<400> 273
   caggtacagc tggtggagtc tggtggaggc gtggtccagc ctgggaggtc cctgagactc        60
   tcctgtgcag cttctggatt caccttcagt agctatgcaa tgatctgggt ccgccaggct       120
   ccagggaagg ggctggaata catcggatac attgatactg atactagcgc atactacgca       180
   agcagtgtga aaggccgatt caccatctcc agagacaatt ccaagaacac gctgtacctg       240
   caaatgtcta gcctgagagc cgaggacacg gctgtgtatt actgtgctag atcttatgct       300
   gcttatggtg gttatcctgc tacttttgat ccctggggcc aaggtaccct cgtcaccgtc       360
   tcgagc                                                                  366
<210> 274
   <211> 1359
   <212> DNA
   <213> Synthetic
<400> 274
   caggtacagc tggtggagtc tggtggaggc gtggtccagc ctgggaggtc cctgagactc      60
   tcctgtgcag cttctggatt caccttcagt agctatgcaa tgatctgggt ccgccaggct     120
   ccagggaagg ggctggaata catcggatac attgatactg atactagcgc atactacgca     180
   agcagtgtga aaggccgatt caccatctcc agagacaatt ccaagaacac gctgtacctg     240
   caaatgtcta gcctgagagc cgaggacacg gctgtgtatt actgtgctag atcttatgct     300
   gcttatggtg gttatcctgc tacttttgat ccctggggcc aaggtaccct cgtcaccgtc     360
   tcgagcgcct ccaccaaggg cccatcggtc ttccccctgg caccctcctc caagagcacc     420
   tctgggggca cagcggccct gggctgcctg gtcaaggact acttccccga accggtgacg     480
   gtgtcgtgga actcaggcgc cctgaccagc ggcgtgcaca ccttcccggc tgtcctacag     540
   tcctcaggac tctactccct cagcagcgtg gtgaccgtgc cctccagcag cttgggcacc     600
   cagacctaca tctgcaacgt gaatcacaag cccagcaaca ccaaggtgga caagagagtt     660
   gagcccaaat cttgtgacaa aactcacaca tgcccaccgt gcccagcacc tgaactcctg     720
   gggggaccgt cagtcttcct cttcccccca aaacccaagg acaccctcat gatctcccgg     780
   acccctgagg tcacatgcgt ggtggtggac gtgagccacg aagaccctga ggtcaagttc     840
   aactggtacg tggacggcgt ggaggtgcat aatgccaaga caaagccgcg ggaggagcag     900
   tacgccagca cgtaccgtgt ggtcagcgtc ctcaccgtcc tgcaccagga ctggctgaat     960
   ggcaaggagt acaagtgcaa ggtctccaac aaagccctcc cagcccccat cgagaaaacc    1020
   atctccaaag ccaaagggca gccccgagaa ccacaggtgt acaccctgcc cccatcccgg    1080
   gaggagatga ccaagaacca ggtcagcctg acctgcctgg tcaaaggctt ctatcccagc    1140
   gacatcgccg tggagtggga gagcaatggg cagccggaga acaactacaa gaccacgcct    1200
   cccgtgctgg actccgacgg ctccttcttc ctctacagca agctcaccgt ggacaagagc    1260
   aggtggcagc aggggaacgt cttctcatgc tccgtgatgc atgaggctct gcacaaccac    1320
   tacacgcaga agagcctctc cctgtctccg ggtaaatga                           1359
<210> 275
   <211> 33
   <212> DNA
   <213> Oryctolagus cuniculus
<400> 275
   caggccagtg aggacattta caacttattg gcc 33
<210> 276
   <211> 21
   <212> DNA
   <213> Oryctolagus cuniculus
<400> 276
   tctgcatcca ctctggcatc t 21
<210> 277
   <211> 36
   <212> DNA
   <213> Oryctolagus cuniculus
<400> 277
   caaaacaact atcttgttac tacttatggt gttgct 36
<210> 278
   <211> 15
   <212> DNA
   <213> Oryctolagus cuniculus
<400> 278
   agctatgcaa tgatc 15
<210> 279
   <211> 48
   <212> DNA
   <213> Synthetic
<400> 279
   tacattgata ctgatactag cgcatactac gcaagcagtg tgaaaggc 48
<210> 280
   <211> 42
   <212> DNA
   <213> Oryctolagus cuniculus
<400> 280
   tcttatgctg cttatggtgg ttatcctgct acttttgatc cc 42
<210> 281
   <211> 333
   <212> DNA
   <213> Oryctolagus cuniculus
<400> 281
   gcctatgata tgacccagac tccagcctcc gtgtctgcag ctgtgggagg cacagtcacc      60
   atcaagtgcc aggccagtga gaacattggt agctacttag cctggtatca gcagaaacca     120
   gggcagcctc ccgaactcct gatctacagg gcgtccactc tggcatctgg ggtcccatcg     180
   cggttcaaag gcagtggatc tgggacacag ttcactctca ccatcagcgg cgtggagtgt     240
   gccgatgctg ccacttacta ctgtcaacag ggttataata gtgagaatct tgataatgct     300
   ttcggcggag ggaccgaggt ggtggtcaaa cgt                                  333
<210> 282
   <211> 654
   <212> DNA
   <213> Synthetic
<400> 282
   gcctatgata tgacccagac tccagcctcc gtgtctgcag ctgtgggagg cacagtcacc      60
   atcaagtgcc aggccagtga gaacattggt agctacttag cctggtatca gcagaaacca     120
   gggcagcctc ccgaactcct gatctacagg gcgtccactc tggcatctgg ggtcccatcg     180
   cggttcaaag gcagtggatc tgggacacag ttcactctca ccatcagcgg cgtggagtgt     240
   gccgatgctg ccacttacta ctgtcaacag ggttataata gtgagaatct tgataatgct     300
   ttcggcggag ggaccgaggt ggtggtcaaa cgtacggtag cggccccatc tgtcttcatc     360
   ttcccgccat ctgatgagca gttgaaatct ggaactgcct ctgttgtgtg cctgctgaat     420
   aacttctatc ccagagaggc caaagtacag tggaaggtgg ataacgccct ccaatcgggt     480
   aactcccagg agagtgtcac agagcaggac agcaaggaca gcacctacag cctcagcagc     540
   accctgacgc tgagcaaagc agactacgag aaacacaaag tctacgcctg cgaagtcacc     600
   catcagggcc tgagctcgcc cgtcacaaag agcttcaaca ggggagagtg ttag           654
<210> 283
   <211> 345
   <212> DNA
   <213> Oryctolagus cuniculus
<400> 283
   cagtcggtgg aggagtccgg gggtcgcctg gtcacgcctg ggacacccct gacactcacc      60
   tgcacagtct ctggaatcga cctcagtatg tattcaatgg gctgggtccg ccaggctcca     120
   gggaaggggc tggaatacat cggatggatt agttatggtg gtactgcata ttacgcgagc     180
   tgggcgaagg gccgattcac catctccaaa acctcgacca cggtggagct gaagatcacc     240
   agtccgacaa tcgaggacac ggccacctat ttctgtgcca gagagactcc tgttaattat     300
   tatttggaca tttggggcca ggggaccctc gtcaccgtct cgagc                     345
<210> 284
   <211> 1338
   <212> DNA
   <213> Synthetic
<400> 284
   cagtcggtgg aggagtccgg gggtcgcctg gtcacgcctg ggacacccct gacactcacc      60
   tgcacagtct ctggaatcga cctcagtatg tattcaatgg gctgggtccg ccaggctcca     120
   gggaaggggc tggaatacat cggatggatt agttatggtg gtactgcata ttacgcgagc     180
   tgggcgaagg gccgattcac catctccaaa acctcgacca cggtggagct gaagatcacc     240
   agtccgacaa tcgaggacac ggccacctat ttctgtgcca gagagactcc tgttaattat     300
   tatttggaca tttggggcca ggggaccctc gtcaccgtct cgagcgcctc caccaagggc 360
   ccatcggtct tccccctggc accctcctcc aagagcacct ctgggggcac agcggccctg 420
   ggctgcctgg tcaaggacta cttccccgaa ccggtgacgg tgtcgtggaa ctcaggcgcc 480
   ctgaccagcg gcgtgcacac cttcccggct gtcctacagt cctcaggact ctactccctc 540
   agcagcgtgg tgaccgtgcc ctccagcagc ttgggcaccc agacctacat ctgcaacgtg 600
   aatcacaagc ccagcaacac caaggtggac aagagagttg agcccaaatc ttgtgacaaa 660
   actcacacat gcccaccgtg cccagcacct gaactcctgg ggggaccgtc agtcttcctc 720
   ttccccccaa aacccaagga caccctcatg atctcccgga cccctgaggt cacatgcgtg 780
   gtggtggacg tgagccacga agaccctgag gtcaagttca actggtacgt ggacggcgtg 840
   gaggtgcata atgccaagac aaagccgcgg gaggagcagt acgccagcac gtaccgtgtg 900
   gtcagcgtcc tcaccgtcct gcaccaggac tggctgaatg gcaaggagta caagtgcaag 960
   gtctccaaca aagccctccc agcccccatc gagaaaacca tctccaaagc caaagggcag 1020
   ccccgagaac cacaggtgta caccctgccc ccatcccggg aggagatgac caagaaccag 1080
   gtcagcctga cctgcctggt caaaggcttc tatcccagcg acatcgccgt ggagtgggag 1140
   agcaatgggc agccggagaa caactacaag accacgcctc ccgtgctgga ctccgacggc 1200
   tccttcttcc tctacagcaa gctcaccgtg gacaagagca ggtggcagca ggggaacgtc 1260
   ttctcatgct ccgtgatgca tgaggctctg cacaaccact acacgcagaa gagcctctcc 1320
   ctgtctccgg gtaaatga 1338
<210> 285
   <211> 33
   <212> DNA
   <213> Oryctolagus cuniculus
<400> 285
   caggccagtg agaacattgg tagctactta gcc 33
<210> 286
   <211> 21
   <212> DNA
   <213> Oryctolagus cuniculus
<400> 286
   agggcgtcca ctctggcatc t 21
<210> 287
   <211> 36
   <212> DNA
   <213> Oryctolagus cuniculus
<400> 287
   caacagggtt ataatagtga gaatcttgat aatgct 36
<210> 288
   <211> 15
   <212> DNA
   <213> Oryctolagus cuniculus
<400> 288
   atgtattcaa tgggc 15
<210> 289
   <211> 48
   <212> DNA
   <213> Oryctolagus cuniculus
<400> 289
   tggattagtt atggtggtac tgcatattac gcgagctggg cgaagggc 48
<210> 290
   <211> 30
   <212> DNA
   <213> Oryctolagus cuniculus
<400> 290
   gagactcctg ttaattatta tttggacatt 30
<210> 291
   <211> 333
   <212> DNA
   <213> Synthetic
<400> 291
   gcctatgata tgacccagtc tccatcctcc ctgtctgcat ctgtaggaga cagagtcacc 60
   atcacttgcc aggccagtga gaacattggt agctacttag cctggtatca gcagaaacca 120
   gggaaagtcc ctaagctcct gatctatagg gcttccactc tggcatctgg ggtcccatct 180
   cgtttcagtg gcagtggatc tgggacagat ttcactctca ccatcagcag cctgcagcct 240
   gaagatgttg caacttatta ctgtcaacag ggttacaata gtgagaatct tgataatgct 300
   ttcggcggag gaaccaaggt ggaaatcaaa cgt 333
<210> 292
   <211> 654
   <212> DNA
   <213> Synthetic
<400> 292
   gcctatgata tgacccagtc tccatcctcc ctgtctgcat ctgtaggaga cagagtcacc 60
   atcacttgcc aggccagtga gaacattggt agctacttag cctggtatca gcagaaacca 120
   gggaaagtcc ctaagctcct gatctatagg gcttccactc tggcatctgg ggtcccatct 180
   cgtttcagtg gcagtggatc tgggacagat ttcactctca ccatcagcag cctgcagcct 240
   gaagatgttg caacttatta ctgtcaacag ggttacaata gtgagaatct tgataatgct 300
   ttcggcggag gaaccaaggt ggaaatcaaa cgtacggtag cggccccatc tgtcttcatc 360
   ttcccgccat ctgatgagca gttgaaatct ggaactgcct ctgttgtgtg cctgctgaat 420
   aacttctatc ccagagaggc caaagtacag tggaaggtgg ataacgccct ccaatcgggt 480
   aactcccagg agagtgtcac agagcaggac agcaaggaca gcacctacag cctcagcagc 540
   accctgacgc tgagcaaagc agactacgag aaacacaaag tctacgcctg cgaagtcacc 600
   catcagggcc tgagctcgcc cgtcacaaag agcttcaaca ggggagagtg ttag 654
<210> 293
   <211> 354
   <212> DNA
   <213> Synthetic
<400> 293
   caggtacagc tggtggagtc tggtggaggc gtggtccagc ctgggaggtc cctgagactc 60
   tcctgtgcag cttctggatt caccttcagt atgtattcaa tgggctgggt ccgccaggct 120
   ccagggaagg ggctggaata catcggatgg attagttatg gtggtactgc atactacgct 180
   agcagcgcta agggccgatt caccatctcc agagacaatt ccaagaacac gctgtacctg 240
   caaatgtcta gcctgagagc cgaggacacg gctgtgtatt actgtgctag agagactcct 300
   gttaattact acttggacat ttggggccaa ggtaccctcg tcaccgtctc gagc 354
<210> 294
   <211> 1347
   <212> DNA
   <213> Synthetic
<400> 294
   caggtacagc tggtggagtc tggtggaggc gtggtccagc ctgggaggtc cctgagactc 60
   tcctgtgcag cttctggatt caccttcagt atgtattcaa tgggctgggt ccgccaggct 120
   ccagggaagg ggctggaata catcggatgg attagttatg gtggtactgc atactacgct 180
   agcagcgcta agggccgatt caccatctcc agagacaatt ccaagaacac gctgtacctg 240
   caaatgtcta gcctgagagc cgaggacacg gctgtgtatt actgtgctag agagactcct 300
   gttaattact acttggacat ttggggccaa ggtaccctcg tcaccgtctc gagcgcctcc 360
   accaagggcc catcggtctt ccccctggca ccctcctcca agagcacctc tgggggcaca 420
   gcggccctgg gctgcctggt caaggactac ttccccgaac cggtgacggt gtcgtggaac 480
   tcaggcgccc tgaccagcgg cgtgcacacc ttcccggctg tcctacagtc ctcaggactc 540
   tactccctca gcagcgtggt gaccgtgccc tccagcagct tgggcaccca gacctacatc 600
   tgcaacgtga atcacaagcc cagcaacacc aaggtggaca agagagttga gcccaaatct 660
   tgtgacaaaa ctcacacatg cccaccgtgc ccagcacctg aactcctggg gggaccgtca 720
   gtcttcctct tccccccaaa acccaaggac accctcatga tctcccggac ccctgaggtc 780
   acatgcgtgg tggtggacgt gagccacgaa gaccctgagg tcaagttcaa ctggtacgtg 840
   gacggcgtgg aggtgcataa tgccaagaca aagccgcggg aggagcagta cgccagcacg 900
   taccgtgtgg tcagcgtcct caccgtcctg caccaggact ggctgaatgg caaggagtac 960
   aagtgcaagg tctccaacaa agccctccca gcccccatcg agaaaaccat ctccaaagcc 1020
   aaagggcagc cccgagaacc acaggtgtac accctgcccc catcccggga ggagatgacc 1080
   aagaaccagg tcagcctgac ctgcctggtc aaaggcttct atcccagcga catcgccgtg 1140
   gagtgggaga gcaatgggca gccggagaac aactacaaga ccacgcctcc cgtgctggac 1200
   tccgacggct ccttcttcct ctacagcaag ctcaccgtgg acaagagcag gtggcagcag 1260
   gggaacgtct tctcatgctc cgtgatgcat gaggctctgc acaaccacta cacgcagaag 1320
   agcctctccc tgtctccggg taaatga 1347
<210> 295
   <211> 33
   <212> DNA
   <213> Oryctolagus cuniculus
<400> 295
   caggccagtg agaacattgg tagctactta gcc 33
<210> 296
   <211> 21
   <212> DNA
   <213> Oryctolagus cuniculus
<400> 296
   agggcttcca ctctggcatc t 21
<210> 297
   <211> 36
   <212> DNA
   <213> Oryctolagus cuniculus
<400> 297
   caacagggtt acaatagtga gaatcttgat aatgct 36
<210> 298
   <211> 15
   <212> DNA
   <213> Oryctolagus cuniculus
<400> 298
   atgtattcaa tgggc 15
<210> 299
   <211> 48
   <212> DNA
   <213> Synthetic
<400> 299
   tggattagtt atggtggtac tgcatactac gctagcagcg ctaagggc 48
<210> 300
   <211> 30
   <212> DNA
   <213> Oryctolagus cuniculus
<400> 300
   gagactcctg ttaattacta cttggacatt 30
<210> 301
   <211> 327
   <212> DNA
   <213> Oryctolagus cuniculus
<400> 301
   gcattcgaat tgacccagac tccatcctcc gtggaggcag ctgtgggagg cacagtcacc 60
   atcaagtgcc aggccagtca gaacattgtt accaatttag cctggtatca acagaaacca 120
   gggcagcctc ccaagctcct gatctatggt gcatccactc tggcatctgg ggtctcatcg 180
   cggttcaaag gcagtggatc tgggacacag ttcactctca ccatcagcga cctggagtgt 240
   gccgatgctg ccacttattt ctgtcagagc tatgatggtt ttaatagtgc tgggttcggc 300
   ggagggaccg aggtggtggt caaacgt 327
<210> 302
   <211> 648
   <212> DNA
   <213> Synthetic
<400> 302
   gcattcgaat tgacccagac tccatcctcc gtggaggcag ctgtgggagg cacagtcacc 60
   atcaagtgcc aggccagtca gaacattgtt accaatttag cctggtatca acagaaacca 120
   gggcagcctc ccaagctcct gatctatggt gcatccactc tggcatctgg ggtctcatcg 180
   cggttcaaag gcagtggatc tgggacacag ttcactctca ccatcagcga cctggagtgt 240
   gccgatgctg ccacttattt ctgtcagagc tatgatggtt ttaatagtgc tgggttcggc 300
   ggagggaccg aggtggtggt caaacgtacg gtagcggccc catctgtctt catcttcccg 360
   ccatctgatg agcagttgaa atctggaact gcctctgttg tgtgcctgct gaataacttc 420
   tatcccagag aggccaaagt acagtggaag gtggataacg ccctccaatc gggtaactcc 480
   caggagagtg tcacagagca ggacagcaag gacagcacct acagcctcag cagcaccctg 540
   acgctgagca aagcagacta cgagaaacac aaagtctacg cctgcgaagt cacccatcag 600
   ggcctgagct cgcccgtcac aaagagcttc aacaggggag agtgttag 648
<210> 303
   <211> 360
   <212> DNA
   <213> Oryctolagus cuniculus
<400> 303
   cagtcgctgg aggagtccgg gggtcgcctg gtcacgcctg ggacacccct gacactcacc 60
   tgcacagcct ctggattctc cctcagtggc tacgacatga gctgggtccg ccaggctcca 120
   ggaaaggggc tggaatacat cggactcatt agttatgatg gtaacacata ctacgcgacc 180
   tgggcgaaag gccgattcac catctccaaa acctcgacca cggtggatct gaaaatcacc 240
   agtccgacaa ccgaggacac ggccacctat ttctgtgcca gaagtcttta tgctggtcct 300
   aatgctggta tcggaccgtt taacatctgg ggccagggga ccctcgtcac cgtctcgagc 360
<210> 304
   <211> 1353
   <212> DNA
   <213> Synthetic
<400> 304
   cagtcgctgg aggagtccgg gggtcgcctg gtcacgcctg ggacacccct gacactcacc        60
   tgcacagcct ctggattctc cctcagtggc tacgacatga gctgggtccg ccaggctcca       120
   ggaaaggggc tggaatacat cggactcatt agttatgatg gtaacacata ctacgcgacc       180
   tgggcgaaag gccgattcac catctccaaa acctcgacca cggtggatct gaaaatcacc       240
   agtccgacaa ccgaggacac ggccacctat ttctgtgcca gaagtcttta tgctggtcct       300
   aatgctggta tcggaccgtt taacatctgg ggccagggga ccctcgtcac cgtctcgagc       360
   gcctccacca agggcccatc ggtcttcccc ctggcaccct cctccaagag cacctctggg       420
   ggcacagcgg ccctgggctg cctggtcaag gactacttcc ccgaaccggt gacggtgtcg       480
   tggaactcag gcgccctgac cagcggcgtg cacaccttcc cggctgtcct acagtcctca       540
   ggactctact ccctcagcag cgtggtgacc gtgccctcca gcagcttggg cacccagacc       600
   tacatctgca acgtgaatca caagcccagc aacaccaagg tggacaagag agttgagccc       660
   aaatcttgtg acaaaactca cacatgccca ccgtgcccag cacctgaact cctgggggga       720
   ccgtcagtct tcctcttccc cccaaaaccc aaggacaccc tcatgatctc ccggacccct       780
   gaggtcacat gcgtggtggt ggacgtgagc cacgaagacc ctgaggtcaa gttcaactgg       840
   tacgtggacg gcgtggaggt gcataatgcc aagacaaagc cgcgggagga gcagtacgcc       900
   agcacgtacc gtgtggtcag cgtcctcacc gtcctgcacc aggactggct gaatggcaag       960
   gagtacaagt gcaaggtctc caacaaagcc ctcccagccc ccatcgagaa aaccatctcc      1020
   aaagccaaag ggcagccccg agaaccacag gtgtacaccc tgcccccatc ccgggaggag      1080
   atgaccaaga accaggtcag cctgacctgc ctggtcaaag gcttctatcc cagcgacatc      1140
   gccgtggagt gggagagcaa tgggcagccg gagaacaact acaagaccac gcctcccgtg      1200
   ctggactccg acggctcctt cttcctctac agcaagctca ccgtggacaa gagcaggtgg      1260
   cagcagggga acgtcttctc atgctccgtg atgcatgagg ctctgcacaa ccactacacg      1320
   cagaagagcc tctccctgtc tccgggtaaa tga                                  1353
<210> 305
   <211> 33
   <212> DNA
   <213> Oryctolagus cuniculus
<400> 305
   caggccagtc agaacattgt taccaattta gcc 33
<210> 306
   <211> 21
   <212> DNA
   <213> Oryctolagus cuniculus
<400> 306
   ggtgcatcca ctctggcatc t 21
<210> 307
   <211> 30
   <212> DNA
   <213> Oryctolagus cuniculus
<400> 307
   cagagctatg atggttttaa tagtgctggg 30
<210> 308
   <211> 15
   <212> DNA
   <213> Oryctolagus cuniculus
<400> 308
   ggctacgaca tgagc 15
<210> 309
   <211> 48
   <212> DNA
   <213> Oryctolagus cuniculus
<400> 309
   ctcattagtt atgatggtaa cacatactac gcgacctggg cgaaaggc 48
<210> 310
   <211> 45
   <212> DNA
   <213> Oryctolagus cuniculus
<400> 310
   agtctttatg ctggtcctaa tgctggtatc ggaccgttta acatc 45
<210> 311
   <211> 327
   <212> DNA
   <213> Synthetic
<400> 311
   gcattccaga tgacccagtc tccatcctcc ctgtctgcat ctgtaggaga cagagtcacc 60
   atcacttgcc aggccagtca gaacattgtt accaacttag cctggtatca gcagaaacca 120
   gggaaagtcc ctaagctcct gatctatggt gcatccactc tggcatctgg ggtcccatct 180
   cgtttcagtg gcagtggatc tgggacagat ttcactctca ccatcagcag cctgcagcct 240
   gaagatgttg caacttatta ctgtcagagc tatgatggtt tcaatagtgc tggtttcggc 300
   ggaggaacca aggtggaaat caaacgt 327
<210> 312
   <211> 648
   <212> DNA
   <213> Synthetic
<400> 312
   gcattccaga tgacccagtc tccatcctcc ctgtctgcat ctgtaggaga cagagtcacc 60
   atcacttgcc aggccagtca gaacattgtt accaacttag cctggtatca gcagaaacca 120
   gggaaagtcc ctaagctcct gatctatggt gcatccactc tggcatctgg ggtcccatct 180
   cgtttcagtg gcagtggatc tgggacagat ttcactctca ccatcagcag cctgcagcct 240
   gaagatgttg caacttatta ctgtcagagc tatgatggtt tcaatagtgc tggtttcggc 300
   ggaggaacca aggtggaaat caaacgtacg gtagcggccc catctgtctt catcttcccg 360
   ccatctgatg agcagttgaa atctggaact gcctctgttg tgtgcctgct gaataacttc 420
   tatcccagag aggccaaagt acagtggaag gtggataacg ccctccaatc gggtaactcc 480
   caggagagtg tcacagagca ggacagcaag gacagcacct acagcctcag cagcaccctg 540
   acgctgagca aagcagacta cgagaaacac aaagtctacg cctgcgaagt cacccatcag 600
   ggcctgagct cgcccgtcac aaagagcttc aacaggggag agtgttag 648
<210> 313
   <211> 369
   <212> DNA
   <213> Synthetic
<400> 313
   caggtacagc tggtggagtc tggtggaggc gtggtccagc ctgggaggtc cctgagactc 60
   tcctgtgcag cttctggatt ctccctcagt ggctacgaca tgagctgggt ccgtcaggct 120
   ccaggcaagg gactggagtg ggtgggactc attagttatg atggtaacac atactacgcg 180
   acctccgcga aaggccgatt caccatctcc agagacaatt ccaagaacac gctgtacctg 240
   caaatgtcta gcctgagagc cgaggacacg gctgtgtatt actgtgctag aagtctttat 300
   gctggtccta atgctggtat cggaccgttt aacatctggg gccaaggtac cctcgtcacc 360
   gtctcgagc 369
<210> 314
   <211> 1362
   <212> DNA
   <213> Synthetic
<400> 314
   caggtacagc tggtggagtc tggtggaggc gtggtccagc ctgggaggtc cctgagactc     60
   tcctgtgcag cttctggatt ctccctcagt ggctacgaca tgagctgggt ccgtcaggct    120
   ccaggcaagg gactggagtg ggtgggactc attagttatg atggtaacac atactacgcg    180
   acctccgcga aaggccgatt caccatctcc agagacaatt ccaagaacac gctgtacctg    240
   caaatgtcta gcctgagagc cgaggacacg gctgtgtatt actgtgctag aagtctttat    300
   gctggtccta atgctggtat cggaccgttt aacatctggg gccaaggtac cctcgtcacc    360
   gtctcgagcg cctccaccaa gggcccatcg gtcttccccc tggcaccctc ctccaagagc    420
   acctctgggg gcacagcggc cctgggctgc ctggtcaagg actacttccc cgaaccggtg    480
   acggtgtcgt ggaactcagg cgccctgacc agcggcgtgc acaccttccc ggctgtccta    540
   cagtcctcag gactctactc cctcagcagc gtggtgaccg tgccctccag cagcttgggc    600
   acccagacct acatctgcaa cgtgaatcac aagcccagca acaccaaggt ggacaagaga    660
   gttgagccca aatcttgtga caaaactcac acatgcccac cgtgcccagc acctgaactc    720
   ctggggggac cgtcagtctt cctcttcccc ccaaaaccca aggacaccct catgatctcc    780
   cggacccctg aggtcacatg cgtggtggtg gacgtgagcc acgaagaccc tgaggtcaag    840
   ttcaactggt acgtggacgg cgtggaggtg cataatgcca agacaaagcc gcgggaggag    900
   cagtacgcca gcacgtaccg tgtggtcagc gtcctcaccg tcctgcacca ggactggctg    960
   aatggcaagg agtacaagtg caaggtctcc aacaaagccc tcccagcccc catcgagaaa   1020
   accatctcca aagccaaagg gcagccccga gaaccacagg tgtacaccct gcccccatcc   1080
   cgggaggaga tgaccaagaa ccaggtcagc ctgacctgcc tggtcaaagg cttctatccc   1140
   agcgacatcg ccgtggagtg ggagagcaat gggcagccgg agaacaacta caagaccacg   1200
   cctcccgtgc tggactccga cggctccttc ttcctctaca gcaagctcac cgtggacaag   1260
   agcaggtggc agcaggggaa cgtcttctca tgctccgtga tgcatgaggc tctgcacaac   1320
   cactacacgc agaagagcct ctccctgtct ccgggtaaat ga                       1362
<210> 315
   <211> 33
   <212> DNA
   <213> Oryctolagus cuniculus
<400> 315
   caggccagtc agaacattgt taccaactta gcc 33
<210> 316
   <211> 21
   <212> DNA
   <213> Oryctolagus cuniculus
<400> 316
   ggtgcatcca ctctggcatc t 21
<210> 317
   <211> 29
   <212> DNA
   <213> Oryctolagus cuniculus
<400> 317
   cagagctatg atggtttcaa tagtgctgg 29
<210> 318
   <211> 15
   <212> DNA
   <213> Oryctolagus cuniculus
<400> 318
   ggctacgaca tgagc 15
<210> 319
   <211> 48
   <212> DNA
   <213> Synthetic
<400> 319
   ctcattagtt atgatggtaa cacatactac gcgacctccg cgaaaggc 48
<210> 320
   <211> 45
   <212> DNA
   <213> Oryctolagus cuniculus
<400> 320
   agtctttatg ctggtcctaa tgctggtatc ggaccgttta acatc 45
<210> 321
   <211> 336
   <212> DNA
   <213> Oryctolagus cuniculus
<400> 321
   gccgccgtgc tgacccagac tccatctccc gtgtctgcag ctgtgggagg cacagtcagc 60
   atcagttgcc agtccagtca gaatgtttat aagaacaact acttatcctg gtatcagcag 120
   aaaccagggc agcctcccaa gctcctgatc tacaaggcat ccactctggc atctggggtc 180
   ccatcgcggt tcaaaggcgg tggatctggg acagatttca ctctcaccat cagcgacgtg 240
   cagtgtgacg ctgctgccac ttactactgt gcaggcggtt ataccagtag tagtgataat 300
   gctttcggcg gagggaccga ggtggtggtc aaacgt 336
<210> 322
   <211> 656
   <212> DNA
   <213> Synthetic
<400> 322
   gccgccgtgc tgacccagac tccatctccc gtgtctgcag ctgtgggagg cacagtcagc      60
   atcagttgcc agtccagtca gaatgtttat aagaacaact acttatcctg gtatcagcag     120
   aaaccagggc agcctcccaa gctcctgatc tacaaggcat ccactctggc atctggggtc     180
   ccatcgcggt tcaaaggcgg tggatctggg acagatttca ctctcaccat cagcgacgtg     240
   cagtgtgacg ctgctgccac ttactactgt gcaggcggtt ataccagtag tagtgataat     300
   gctttcggcg gagggaccga ggtggtggtc aaacgtacgg tagcggcccc atctgtcttc     360
   atcttcccgc catctgatga gcagttgaaa tctggaactg cctctgttgt gtgcctgctg     420
   aataacttct atcccagaga ggccaaagta cagtggaagg tggataacgc cctccaatcg     480
   ggtaactccc aggagagtgt cacagagcag gacagcaagg acagcaccta cagcctcagc     540
   agcaccctga cgctgagcaa agcagactac gagaaacaca aagtctacgc ctgcgaagtc     600
   acccatcagg gcctgagctc gcccgtcaca aagagcttca acaggggaga gtgtta         656
<210> 323
   <211> 351
   <212> DNA
   <213> Oryctolagus cuniculus
<400> 323
   cagtcggtgg aggcgtccgg gggtcgcctg gtcacgcctg ggacacccct gacactcacc      60
   tgcacagcct ctggattctc cctcagtacc tactggatga gctgggtccg ccaggctcca     120
   gggaaggggc tggaatggat cggagacatt tattttagta atgaagaaac aaactacgcg     180
   agctgggcga aaggccgatt taccatctcc aaaacctcga ccacggtgga tctgaatgtc     240
   atcagtccga caaccgagga cacggccacc tatttctgtg ccagaggttc tcctgatgtt     300
   gatattggta tagatatgtg gggcccgggc accctcgtca ccgtctcgag c               351
<210> 324
   <211> 1344
   <212> DNA
   <213> Synthetic
<400> 324
   cagtcggtgg aggcgtccgg gggtcgcctg gtcacgcctg ggacacccct gacactcacc      60
   tgcacagcct ctggattctc cctcagtacc tactggatga gctgggtccg ccaggctcca     120
   gggaaggggc tggaatggat cggagacatt tattttagta atgaagaaac aaactacgcg    180
   agctgggcga aaggccgatt taccatctcc aaaacctcga ccacggtgga tctgaatgtc    240
   atcagtccga caaccgagga cacggccacc tatttctgtg ccagaggttc tcctgatgtt    300
   gatattggta tagatatgtg gggcccgggc accctcgtca ccgtctcgag cgcctccacc     360
   aagggcccat cggtcttccc cctggcaccc tcctccaaga gcacctctgg gggcacagcg    420
   gccctgggct gcctggtcaa ggactacttc cccgaaccgg tgacggtgtc gtggaactca    480
   ggcgccctga ccagcggcgt gcacaccttc ccggctgtcc tacagtcctc aggactctac    540
   tccctcagca gcgtggtgac cgtgccctcc agcagcttgg gcacccagac ctacatctgc    600
   aacgtgaatc acaagcccag caacaccaag gtggacaaga gagttgagcc caaatcttgt    660
   gacaaaactc acacatgccc accgtgccca gcacctgaac tcctgggggg accgtcagtc    720
   ttcctcttcc ccccaaaacc caaggacacc ctcatgatct cccggacccc tgaggtcaca    780
   tgcgtggtgg tggacgtgag ccacgaagac cctgaggtca agttcaactg gtacgtggac    840
   ggcgtggagg tgcataatgc caagacaaag ccgcgggagg agcagtacgc cagcacgtac    900
   cgtgtggtca gcgtcctcac cgtcctgcac caggactggc tgaatggcaa ggagtacaag    960
   tgcaaggtct ccaacaaagc cctcccagcc cccatcgaga aaaccatctc caaagccaaa   1020
   gggcagcccc gagaaccaca ggtgtacacc ctgcccccat cccgggagga gatgaccaag   1080
   aaccaggtca gcctgacctg cctggtcaaa ggcttctatc ccagcgacat cgccgtggag   1140
   tgggagagca atgggcagcc ggagaacaac tacaagacca cgcctcccgt gctggactcc   1200
   gacggctcct tcttcctcta cagcaagctc accgtggaca agagcaggtg gcagcagggg   1260
   aacgtcttct catgctccgt gatgcatgag gctctgcaca accactacac gcagaagagc   1320
   ctctccctgt ctccgggtaa atga                                          1344
<210> 325
   <211> 39
   <212> DNA
   <213> Oryctolagus cuniculus
<400> 325
   cagtccagtc agaatgttta taagaacaac tacttatcc 39
<210> 326
   <211> 21
   <212> DNA
   <213> Oryctolagus cuniculus
<400> 326
   aaggcatcca ctctggcatc t 21
<210> 327
   <211> 33
   <212> DNA
   <213> Oryctolagus cuniculus
<400> 327
   gcaggcggtt ataccagtag tagtgataat gct 33
<210> 328
   <211> 15
   <212> DNA
   <213> Oryctolagus cuniculus
<400> 328
   acctactgga tgagc 15
<210> 329
   <211> 51
   <212> DNA
   <213> Oryctolagus cuniculus
<400> 329
   gacatttatt ttagtaatga agaaacaaac tacgcgagct gggcgaaagg c 51
<210> 330
   <211> 33
   <212> DNA
   <213> Oryctolagus cuniculus
<400> 330
   ggttctcctg atgttgatat tggtatagat atg 33
<210> 331
   <211> 336
   <212> DNA
   <213> Synthetic
<400> 331
   gacatccaga tgacccagtc tccatcctcc ctgtctgcat ctgtaggaga cagagtcacc      60
   atcacttgcc agtccagtca gaatgtttat aagaacaact acttatcctg gtatcagcag     120
   aaaccaggga aagtccctaa gctcctgatc tataaggcat ccactctggc atctggggtc     180
   ccatctcgtt tcagtggcag tggatctggg acagatttca ctctcaccat cagcagcctg     240
   cagcctgaag atgttgcaac ttattactgt gcaggcggtt ataccagtag tagtgataat     300
   gctttcggcg gaggaaccaa ggtggaaatc aaacgt                               336
<210> 332
   <211> 657
   <212> DNA
   <213> Synthetic
<400> 332
   gacatccaga tgacccagtc tccatcctcc ctgtctgcat ctgtaggaga cagagtcacc 60
   atcacttgcc agtccagtca gaatgtttat aagaacaact acttatcctg gtatcagcag 120
   aaaccaggga aagtccctaa gctcctgatc tataaggcat ccactctggc atctggggtc 180
   ccatctcgtt tcagtggcag tggatctggg acagatttca ctctcaccat cagcagcctg 240
   cagcctgaag atgttgcaac ttattactgt gcaggcggtt ataccagtag tagtgataat 300
   gctttcggcg gaggaaccaa ggtggaaatc aaacgtacgg tagcggcccc atctgtcttc 360
   atcttcccgc catctgatga gcagttgaaa tctggaactg cctctgttgt gtgcctgctg 420
   aataacttct atcccagaga ggccaaagta cagtggaagg tggataacgc cctccaatcg 480
   ggtaactccc aggagagtgt cacagagcag gacagcaagg acagcaccta cagcctcagc 540
   agcaccctga cgctgagcaa agcagactac gagaaacaca aagtctacgc ctgcgaagtc 600
   acccatcagg gcctgagctc gcccgtcaca aagagcttca acaggggaga gtgttag 657
<210> 333
   <211> 360
   <212> DNA
   <213> Synthetic
<400> 333
   gaggtgcagc tggtggagtc tgggggaggc ttggtccagc ctggggggtc cctgagactc 60
   tcctgtgcag cctctggatt caccgtcagt acctactgga tgagctgggt ccgtcaggct 120
   ccagggaagg ggctggagtg ggtcggagac atttacttta gtaatgaaga aacaaactac 180
   gcgagcagcg cgaaaggccg attcaccatc tccagagaca attccaagaa caccctgtat 240
   cttcaaatga acagcctgag agctgaggac actgctgtgt attactgtgc tagaggttct 300
   cctgatgttg atattggtat agatatgtgg ggcccaggga ccctcgtcac cgtctcgagc 360
<210> 334
   <211> 1353
   <212> DNA
   <213> Synthetic
<400> 334
   gaggtgcagc tggtggagtc tgggggaggc ttggtccagc ctggggggtc cctgagactc 60
   tcctgtgcag cctctggatt caccgtcagt acctactgga tgagctgggt ccgtcaggct 120
   ccagggaagg ggctggagtg ggtcggagac atttacttta gtaatgaaga aacaaactac 180
   gcgagcagcg cgaaaggccg attcaccatc tccagagaca attccaagaa caccctgtat 240
   cttcaaatga acagcctgag agctgaggac actgctgtgt attactgtgc tagaggttct 300
   cctgatgttg atattggtat agatatgtgg ggcccaggga ccctcgtcac cgtctcgagc 360
   gcctccacca agggcccatc ggtcttcccc ctggcaccct cctccaagag cacctctggg 420
   ggcacagcgg ccctgggctg cctggtcaag gactacttcc ccgaaccggt gacggtgtcg 480
   tggaactcag gcgccctgac cagcggcgtg cacaccttcc cggctgtcct acagtcctca 540
   ggactctact ccctcagcag cgtggtgacc gtgccctcca gcagcttggg cacccagacc 600
   tacatctgca acgtgaatca caagcccagc aacaccaagg tggacaagag agttgagccc 660
   aaatcttgtg acaaaactca cacatgccca ccgtgcccag cacctgaact cctgggggga 720
   ccgtcagtct tcctcttccc cccaaaaccc aaggacaccc tcatgatctc ccggacccct 780
   gaggtcacat gcgtggtggt ggacgtgagc cacgaagacc ctgaggtcaa gttcaactgg 840
   tacgtggacg gcgtggaggt gcataatgcc aagacaaagc cgcgggagga gcagtacgcc 900
   agcacgtacc gtgtggtcag cgtcctcacc gtcctgcacc aggactggct gaatggcaag 960
   gagtacaagt gcaaggtctc caacaaagcc ctcccagccc ccatcgagaa aaccatctcc 1020
   aaagccaaag ggcagccccg agaaccacag gtgtacaccc tgcccccatc ccgggaggag 1080
   atgaccaaga accaggtcag cctgacctgc ctggtcaaag gcttctatcc cagcgacatc 1140
   gccgtggagt gggagagcaa tgggcagccg gagaacaact acaagaccac gcctcccgtg 1200
   ctggactccg acggctcctt cttcctctac agcaagctca ccgtggacaa gagcaggtgg 1260
   cagcagggga acgtcttctc atgctccgtg atgcatgagg ctctgcacaa ccactacacg 1320
   cagaagagcc tctccctgtc tccgggtaaa tga 1353
<210> 335
   <211> 39
   <212> DNA
   <213> Oryctolagus cuniculus
<400> 335
   cagtccagtc agaatgttta taagaacaac tacttatcc 39
<210> 336
   <211> 21
   <212> DNA
   <213> Oryctolagus cuniculus
<400> 336
   aaggcatcca ctctggcatc t 21
<210> 337
   <211> 33
   <212> DNA
   <213> Oryctolagus cuniculus
<400> 337
   gcaggcggtt ataccagtag tagtgataat gct 33
<210> 338
   <211> 15
   <212> DNA
   <213> Oryctolagus cuniculus
<400> 338
   acctactgga tgagc 15
<210> 339
   <211> 51
   <212> DNA
   <213> Synthetic
<400> 339
   gacatttact ttagtaatga agaaacaaac tacgcgagca gcgcgaaagg c 51
<210> 340
   <211> 33
   <212> DNA
   <213> Oryctolagus cuniculus
<400> 340
   ggttctcctg atgttgatat tggtatagat atg 33
<210> 341
   <211> 336
   <212> DNA
   <213> Oryctolagus cuniculus
<400> 341
   gcagccgtgc tgacccagac accatcgccc gtgtctgcag ctgtgggaga cacagtcacc 60
   atcaagtgcc agtccagtca gagtgtttat aagaacaact acttatcctg gtatcagcag 120
   aaaccagggc agcctcccaa gctcctgatc tatgatgcat ccaatctgcc atctggggtc 180
   ccatcacggt tcagcggcag tggatctggg acacagttca ctctcaccat cagcggcgtg 240
   cagtgtgacg atgctgccac ttactactgt ctaggcgatt atgatgatga tactgataat 300
   ggtttcggcg gagggaccga ggtggtggtc aaacgt 336
<210> 342
   <211> 657
   <212> DNA
   <213> Synthetic
<400> 342
   gcagccgtgc tgacccagac accatcgccc gtgtctgcag ctgtgggaga cacagtcacc       60
   atcaagtgcc agtccagtca gagtgtttat aagaacaact acttatcctg gtatcagcag      120
   aaaccagggc agcctcccaa gctcctgatc tatgatgcat ccaatctgcc atctggggtc      180
   ccatcacggt tcagcggcag tggatctggg acacagttca ctctcaccat cagcggcgtg      240
   cagtgtgacg atgctgccac ttactactgt ctaggcgatt atgatgatga tactgataat      300
   ggtttcggcg gagggaccga ggtggtggtc aaacgtacgg tagcggcccc atctgtcttc      360
   atcttcccgc catctgatga gcagttgaaa tctggaactg cctctgttgt gtgcctgctg      420
   aataacttct atcccagaga ggccaaagta cagtggaagg tggataacgc cctccaatcg      480
   ggtaactccc aggagagtgt cacagagcag gacagcaagg acagcaccta cagcctcagc      540
   agcaccctga cgctgagcaa agcagactac gagaaacaca aagtctacgc ctgcgaagtc      600
   acccatcagg gcctgagctc gcccgtcaca aagagcttca acaggggaga gtgttag         657
<210> 343
   <211> 336
   <212> DNA
   <213> Oryctolagus cuniculus
<400> 343
   cagtcggtgg aggagtccgg gggtcgcctg gtcacgcctg ggacacccct gacactcacc       60
   tgcacagtct ctggaatcga cctcagtagc tatgcaatga tctgggtccg ccaggctcca      120
   gggaaggggc tggaatacat cggaatcatt tggagtggtg gcacctacta cgcgacctgg      180
   gcgaaaggcc gattcaccat ctccaaaacc tcgaccacgg tggatctgca aatcaccagt      240
   ccgacaaccg aggacgcggc cacctatttc tgtgccgcag gtggtggtag tatttatgat      300
   gtttggggcc cgggcaccct ggtcaccgtc tcgagc                               336
<210> 344
   <211> 1329
   <212> DNA
   <213> Synthetic
<400> 344
   cagtcggtgg aggagtccgg gggtcgcctg gtcacgcctg ggacacccct gacactcacc 60
   tgcacagtct ctggaatcga cctcagtagc tatgcaatga tctgggtccg ccaggctcca 120
   gggaaggggc tggaatacat cggaatcatt tggagtggtg gcacctacta cgcgacctgg 180
   gcgaaaggcc gattcaccat ctccaaaacc tcgaccacgg tggatctgca aatcaccagt 240
   ccgacaaccg aggacgcggc cacctatttc tgtgccgcag gtggtggtag tatttatgat 300
   gtttggggcc cgggcaccct ggtcaccgtc tcgagcgcct ccaccaaggg cccatcggtc 360
   ttccccctgg caccctcctc caagagcacc tctgggggca cagcggccct gggctgcctg 420
   gtcaaggact acttccccga accggtgacg gtgtcgtgga actcaggcgc cctgaccagc 480
   ggcgtgcaca ccttcccggc tgtcctacag tcctcaggac tctactccct cagcagcgtg 540
   gtgaccgtgc cctccagcag cttgggcacc cagacctaca tctgcaacgt gaatcacaag 600
   cccagcaaca ccaaggtgga caagagagtt gagcccaaat cttgtgacaa aactcacaca 660
   tgcccaccgt gcccagcacc tgaactcctg gggggaccgt cagtcttcct cttcccccca 720
   aaacccaagg acaccctcat gatctcccgg acccctgagg tcacatgcgt ggtggtggac 780
   gtgagccacg aagaccctga ggtcaagttc aactggtacg tggacggcgt ggaggtgcat 840
   aatgccaaga caaagccgcg ggaggagcag tacgccagca cgtaccgtgt ggtcagcgtc 900
   ctcaccgtcc tgcaccagga ctggctgaat ggcaaggagt acaagtgcaa ggtctccaac 960
   aaagccctcc cagcccccat cgagaaaacc atctccaaag ccaaagggca gccccgagaa 1020
   ccacaggtgt acaccctgcc cccatcccgg gaggagatga ccaagaacca ggtcagcctg 1080
   acctgcctgg tcaaaggctt ctatcccagc gacatcgccg tggagtggga gagcaatggg 1140
   cagccggaga acaactacaa gaccacgcct cccgtgctgg actccgacgg ctccttcttc 1200
   ctctacagca agctcaccgt ggacaagagc aggtggcagc aggggaacgt cttctcatgc 1260
   tccgtgatgc atgaggctct gcacaaccac tacacgcaga agagcctctc cctgtctccg 1320
   ggtaaatga 1329
<210> 345
   <211> 39
   <212> DNA
   <213> Oryctolagus cuniculus
<400> 345
   cagtccagtc agagtgttta taagaacaac tacttatcc 39
<210> 346
   <211> 21
   <212> DNA
   <213> Oryctolagus cuniculus
<400> 346
   gatgcatcca atctgccatc t 21
<210> 347
   <211> 33
   <212> DNA
   <213> Oryctolagus cuniculus
<400> 347
   ctaggcgatt atgatgatga tactgataat ggt 33
<210> 348
   <211> 15
   <212> DNA
   <213> Oryctolagus cuniculus
<400> 348
   agctatgcaa tgatc 15
<210> 349
   <211> 45
   <212> DNA
   <213> Oryctolagus cuniculus
<400> 349
   atcatttgga gtggtggcac ctactacgcg acctgggcga aaggc 45
<210> 350
   <211> 24
   <212> DNA
   <213> Oryctolagus cuniculus
<400> 350
   ggtggtggta gtatttatga tgtt 24
<210> 351
   <211> 336
   <212> DNA
   <213> Oryctolagus cuniculus
<400> 351
   gccctggtga tgacccagac tccatcctcc acgtctgaac cagtgggagg cacagtcacc    60
   atcaattgcc aggctagtca gaatattggt aacgacctat cctggtatca gcagaaacca   120
   gggcagcctc ccgagctcct aatctattct acatccaaac tggcaactgg ggtcccaaag   180
   cggttcagtg gcagcagatc tgggacacag ttcactctca ccatcagcga cctggagtgt   240
   gacgatgctg ccacttacta ctgtctaggt gtttatagtt atattagtga tgatggtaat   300
   gctttcggcg gagggaccga ggtggtggtc aaacgt                             336
<210> 352
   <211> 657
   <212> DNA
   <213> Synthetic
<400> 352
   gccctggtga tgacccagac tccatcctcc acgtctgaac cagtgggagg cacagtcacc      60
   atcaattgcc aggctagtca gaatattggt aacgacctat cctggtatca gcagaaacca     120
   gggcagcctc ccgagctcct aatctattct acatccaaac tggcaactgg ggtcccaaag     180
   cggttcagtg gcagcagatc tgggacacag ttcactctca ccatcagcga cctggagtgt     240
   gacgatgctg ccacttacta ctgtctaggt gtttatagtt atattagtga tgatggtaat     300
   gctttcggcg gagggaccga ggtggtggtc aaacgtacgg tagcggcccc atctgtcttc     360
   atcttcccgc catctgatga gcagttgaaa tctggaactg cctctgttgt gtgcctgctg     420
   aataacttct atcccagaga ggccaaagta cagtggaagg tggataacgc cctccaatcg     480
   ggtaactccc aggagagtgt cacagagcag gacagcaagg acagcaccta cagcctcagc     540
   agcaccctga cgctgagcaa agcagactac gagaaacaca aagtctacgc ctgcgaagtc     600
   acccatcagg gcctgagctc gcccgtcaca aagagcttca acaggggaga gtgttag        657
<210> 353
   <211> 360
   <212> DNA
   <213> Oryctolagus cuniculus
<400> 353
   cagtcggtgg aggagttcgg gggtcgcctg gtcacgcctg ggacacccct gacactcacc      60
   tgcaccgtct ctggattctc cctcaataac tatgcaatga cctgggtccg ccaggctcca     120
   gggaaggggc tggagtggat cgggatcatt ggtagtattg gtaccacata ctacgcgagc     180
   tgggcgaaag gccgattctt catctccaaa acctcgacca ctgtggatct gaaaatcatt     240
   agtccgacaa ccgaggacac ggccacctat ttctgtgcca gagatgctgg cgttactgtt     300
   gatggttatg gctactactt taacatctgg ggcccaggca ccctcgtcac cgtctcgagc     360
<210> 354
   <211> 1353
   <212> DNA
   <213> Synthetic
<400> 354
   cagtcggtgg aggagttcgg gggtcgcctg gtcacgcctg ggacacccct gacactcacc 60
   tgcaccgtct ctggattctc cctcaataac tatgcaatga cctgggtccg ccaggctcca 120
   gggaaggggc tggagtggat cgggatcatt ggtagtattg gtaccacata ctacgcgagc 180
   tgggcgaaag gccgattctt catctccaaa acctcgacca ctgtggatct gaaaatcatt 240
   agtccgacaa ccgaggacac ggccacctat ttctgtgcca gagatgctgg cgttactgtt 300
   gatggttatg gctactactt taacatctgg ggcccaggca ccctcgtcac cgtctcgagc 360
   gcctccacca agggcccatc ggtcttcccc ctggcaccct cctccaagag cacctctggg 420
   ggcacagcgg ccctgggctg cctggtcaag gactacttcc ccgaaccggt gacggtgtcg 480
   tggaactcag gcgccctgac cagcggcgtg cacaccttcc cggctgtcct acagtcctca 540
   ggactctact ccctcagcag cgtggtgacc gtgccctcca gcagcttggg cacccagacc 600
   tacatctgca acgtgaatca caagcccagc aacaccaagg tggacaagag agttgagccc 660
   aaatcttgtg acaaaactca cacatgccca ccgtgcccag cacctgaact cctgggggga 720
   ccgtcagtct tcctcttccc cccaaaaccc aaggacaccc tcatgatctc ccggacccct 780
   gaggtcacat gcgtggtggt ggacgtgagc cacgaagacc ctgaggtcaa gttcaactgg 840
   tacgtggacg gcgtggaggt gcataatgcc aagacaaagc cgcgggagga gcagtacgcc 900
   agcacgtacc gtgtggtcag cgtcctcacc gtcctgcacc aggactggct gaatggcaag 960
   gagtacaagt gcaaggtctc caacaaagcc ctcccagccc ccatcgagaa aaccatctcc 1020
   aaagccaaag ggcagccccg agaaccacag gtgtacaccc tgcccccatc ccgggaggag 1080
   atgaccaaga accaggtcag cctgacctgc ctggtcaaag gcttctatcc cagcgacatc 1140
   gccgtggagt gggagagcaa tgggcagccg gagaacaact acaagaccac gcctcccgtg 1200
   ctggactccg acggctcctt cttcctctac agcaagctca ccgtggacaa gagcaggtgg 1260
   cagcagggga acgtcttctc atgctccgtg atgcatgagg ctctgcacaa ccactacacg 1320
   cagaagagcc tctccctgtc tccgggtaaa tga 1353
<210> 355
   <211> 33
   <212> DNA
   <213> Oryctolagus cuniculus
<400> 355
   caggctagtc agaatattgg taacgaccta tcc 33
<210> 356
   <211> 21
   <212> DNA
   <213> Oryctolagus cuniculus
<400> 356
   tctacatcca aactggcaac t 21
<210> 357
   <211> 39
   <212> DNA
   <213> Oryctolagus cuniculus
<400> 357
   ctaggtgttt atagttatat tagtgatgat ggtaatgct 39
<210> 358
   <211> 15
   <212> DNA
   <213> Oryctolagus cuniculus
<400> 358
   aactatgcaa tgacc 15
<210> 359
   <211> 48
   <212> DNA
   <213> Oryctolagus cuniculus
<400> 359
   atcattggta gtattggtac cacatactac gcgagctggg cgaaaggc 48
<210> 360
   <211> 45
   <212> DNA
   <213> Oryctolagus cuniculus
<400> 360
   gatgctggcg ttactgttga tggttatggc tactacttta acatc 45
<210> 361
   <211> 336
   <212> DNA
   <213> Oryctolagus cuniculus
<400> 361
   gccatcgaaa tgacccagac tccattctcc gtgtctgcag ctgtgggagg cacagtcacc 60
   atcaagtgcc aggccagtca gaccattagc aactacttag cctggtatca gcagaaacca 120
   gggcagcctc ccaagctcct gatctatggt gcatccaatc tggaatctgg ggtcccatcg 180
   cggttcaaag gcagtggatc tgggacacag ttcactctca ccatcagcga cctggagtgt 240
   gacgatgctg ccacttacta ctgtcaacag ggttatacta tcagtaatgt tgataacaat 300
   gttttcggcg gagggaccga ggtggtggtc aaacgt 336
<210> 362
   <211> 657
   <212> DNA
   <213> Synthetic
<400> 362
   gccatcgaaa tgacccagac tccattctcc gtgtctgcag ctgtgggagg cacagtcacc      60
   atcaagtgcc aggccagtca gaccattagc aactacttag cctggtatca gcagaaacca     120
   gggcagcctc ccaagctcct gatctatggt gcatccaatc tggaatctgg ggtcccatcg     180
   cggttcaaag gcagtggatc tgggacacag ttcactctca ccatcagcga cctggagtgt     240
   gacgatgctg ccacttacta ctgtcaacag ggttatacta tcagtaatgt tgataacaat     300
   gttttcggcg gagggaccga ggtggtggtc aaacgtacgg tagcggcccc atctgtcttc     360
   atcttcccgc catctgatga gcagttgaaa tctggaactg cctctgttgt gtgcctgctg     420
   aataacttct atcccagaga ggccaaagta cagtggaagg tggataacgc cctccaatcg     480
   ggtaactccc aggagagtgt cacagagcag gacagcaagg acagcaccta cagcctcagc     540
   agcaccctga cgctgagcaa agcagactac gagaaacaca aagtctacgc ctgcgaagtc     600
   acccatcagg gcctgagctc gcccgtcaca aagagcttca acaggggaga gtgttag        657
<210> 363
   <211> 345
   <212> DNA
   <213> Oryctolagus cuniculus
<400> 363
   cagtcgctgg aggagtccgg gggtcgcctg gtcacgcctg ggggatccct gacactcacc      60
   tgcgcagcct ctggattctc cctcactggc tacaacttgg tctgggtccg ccaggctcca     120
   gggaaggggc tggagtggat cggattcatt agttatggtg ataccacata ctacgcgagc     180
   tgggcgaaag gccgattcac catctccaaa acctcgacca cggtgactct gacgatcacc     240
   gatctgcaac cttcagacac gggcacctat ttctgtgcca gagagactgc taatacttat     300
   gattatggca tctggggccc aggcaccctc gtcaccgtct cgagc                     345
<210> 364
   <211> 1338
   <212> DNA
   <213> Synthetic
<400> 364
   cagtcgctgg aggagtccgg gggtcgcctg gtcacgcctg ggggatccct gacactcacc      60
   tgcgcagcct ctggattctc cctcactggc tacaacttgg tctgggtccg ccaggctcca 120
   gggaaggggc tggagtggat cggattcatt agttatggtg ataccacata ctacgcgagc 180
   tgggcgaaag gccgattcac catctccaaa acctcgacca cggtgactct gacgatcacc 240
   gatctgcaac cttcagacac gggcacctat ttctgtgcca gagagactgc taatacttat 300
   gattatggca tctggggccc aggcaccctc gtcaccgtct cgagcgcctc caccaagggc 360
   ccatcggtct tccccctggc accctcctcc aagagcacct ctgggggcac agcggccctg 420
   ggctgcctgg tcaaggacta cttccccgaa ccggtgacgg tgtcgtggaa ctcaggcgcc 480
   ctgaccagcg gcgtgcacac cttcccggct gtcctacagt cctcaggact ctactccctc 540
   agcagcgtgg tgaccgtgcc ctccagcagc ttgggcaccc agacctacat ctgcaacgtg 600
   aatcacaagc ccagcaacac caaggtggac aagagagttg agcccaaatc ttgtgacaaa 660
   actcacacat gcccaccgtg cccagcacct gaactcctgg ggggaccgtc agtcttcctc 720
   ttccccccaa aacccaagga caccctcatg atctcccgga cccctgaggt cacatgcgtg 780
   gtggtggacg tgagccacga agaccctgag gtcaagttca actggtacgt ggacggcgtg 840
   gaggtgcata atgccaagac aaagccgcgg gaggagcagt acgccagcac gtaccgtgtg 900
   gtcagcgtcc tcaccgtcct gcaccaggac tggctgaatg gcaaggagta caagtgcaag 960
   gtctccaaca aagccctccc agcccccatc gagaaaacca tctccaaagc caaagggcag 1020
   ccccgagaac cacaggtgta caccctgccc ccatcccggg aggagatgac caagaaccag 1080
   gtcagcctga cctgcctggt caaaggcttc tatcccagcg acatcgccgt ggagtgggag 1140
   agcaatgggc agccggagaa caactacaag accacgcctc ccgtgctgga ctccgacggc 1200
   tccttcttcc tctacagcaa gctcaccgtg gacaagagca ggtggcagca ggggaacgtc 1260
   ttctcatgct ccgtgatgca tgaggctctg cacaaccact acacgcagaa gagcctctcc 1320
   ctgtctccgg gtaaatga 1338
<210> 365
   <211> 33
   <212> DNA
   <213> Oryctolagus cuniculus
<400> 365
   caggccagtc agaccattag caactactta gcc 33
<210> 366
   <211> 21
   <212> DNA
   <213> Oryctolagus cuniculus
<400> 366
   ggtgcatcca atctggaatc t 21
<210> 367
   <211> 39
   <212> DNA
   <213> Oryctolagus cuniculus
<400> 367
   caacagggtt atactatcag taatgttgat aacaatgtt 39
<210> 368
   <211> 15
   <212> DNA
   <213> Oryctolagus cuniculus
<400> 368
   ggctacaact tggtc 15
<210> 369
   <211> 48
   <212> DNA
   <213> Oryctolagus cuniculus
<400> 369
   ttcattagtt atggtgatac cacatactac gcgagctggg cgaaaggc 48
<210> 370
   <211> 30
   <212> DNA
   <213> Oryctolagus cuniculus
<400> 370
   gagactgcta atacttatga ttatggcatc 30
<210> 371
   <211> 336
   <212> DNA
   <213> Synthetic
<400> 371
   gacatccaga tgacccagtc tccttccacc ctgtctgcat ctgtaggaga cagagtcacc 60
   atcacttgtc aggctagtca gaccattagc aactacttag cctggtatca gcagaaacca 120
   ggaaaagccc ctaagctcct gatctatggt gcatccaatc tggaatctgg agtcccatca 180
   aggttcagcg gcagtggatc tggaacagaa ttcactctca ccatcagcag cctgcagcct 240
   gatgattttg caacttacta ctgtcaacag ggttatacta tcagtaatgt tgataacaat 300
   gttttcggcg gaggaaccaa ggtggaaatc aaacgt 336
<210> 372
   <211> 657
   <212> DNA
   <213> Synthetic
<400> 372
   gacatccaga tgacccagtc tccttccacc ctgtctgcat ctgtaggaga cagagtcacc        60
   atcacttgtc aggctagtca gaccattagc aactacttag cctggtatca gcagaaacca       120
   ggaaaagccc ctaagctcct gatctatggt gcatccaatc tggaatctgg agtcccatca       180
   aggttcagcg gcagtggatc tggaacagaa ttcactctca ccatcagcag cctgcagcct       240
   gatgattttg caacttacta ctgtcaacag ggttatacta tcagtaatgt tgataacaat       300
   gttttcggcg gaggaaccaa ggtggaaatc aaacgtacgg tagcggcccc atctgtcttc       360
   atcttcccgc catctgatga gcagttgaaa tctggaactg cctctgttgt gtgcctgctg       420
   aataacttct atcccagaga ggccaaagta cagtggaagg tggataacgc cctccaatcg       480
   ggtaactccc aggagagtgt cacagagcag gacagcaagg acagcaccta cagcctcagc       540
   agcaccctga cgctgagcaa agcagactac gagaaacaca aagtctacgc ctgcgaagtc       600
   acccatcagg gcctgagctc gcccgtcaca aagagcttca acaggggaga gtgttag          657
<210> 373
   <211> 354
   <212> DNA
   <213> Synthetic
<400> 373
   gaggtgcagc tggtggagtc tgggggaggc ttggtccagc ctggggggtc cctgagactc        60
   tcctgtgcag cctctggatt caccgtcagt ggctacaact tggtctgggt ccgtcaggct       120
   ccagggaagg ggctggagtg ggtcggattc attagttatg gtgataccac atactacgct       180
   agctctgcta aaggccgatt caccatctcc agagacaatt ccaagaacac cctgtatctt       240
   caaatgaaca gcctgagagc tgaggacact gctgtgtatt actgtgctag agagactgct       300
   aatacttatg attatggcat ctggggccaa gggaccctcg tcaccgtctc gagc             354
<210> 374
   <211> 1347
   <212> DNA
   <213> Synthetic
<400> 374
   gaggtgcagc tggtggagtc tgggggaggc ttggtccagc ctggggggtc cctgagactc 60
   tcctgtgcag cctctggatt caccgtcagt ggctacaact tggtctgggt ccgtcaggct 120
   ccagggaagg ggctggagtg ggtcggattc attagttatg gtgataccac atactacgct 180
   agctctgcta aaggccgatt caccatctcc agagacaatt ccaagaacac cctgtatctt 240
   caaatgaaca gcctgagagc tgaggacact gctgtgtatt actgtgctag agagactgct 300
   aatacttatg attatggcat ctggggccaa gggaccctcg tcaccgtctc gagcgcctcc 360
   accaagggcc catcggtctt ccccctggca ccctcctcca agagcacctc tgggggcaca 420
   gcggccctgg gctgcctggt caaggactac ttccccgaac cggtgacggt gtcgtggaac 480
   tcaggcgccc tgaccagcgg cgtgcacacc ttcccggctg tcctacagtc ctcaggactc 540
   tactccctca gcagcgtggt gaccgtgccc tccagcagct tgggcaccca gacctacatc 600
   tgcaacgtga atcacaagcc cagcaacacc aaggtggaca agagagttga gcccaaatct 660
   tgtgacaaaa ctcacacatg cccaccgtgc ccagcacctg aactcctggg gggaccgtca 720
   gtcttcctct tccccccaaa acccaaggac accctcatga tctcccggac ccctgaggtc 780
   acatgcgtgg tggtggacgt gagccacgaa gaccctgagg tcaagttcaa ctggtacgtg 840
   gacggcgtgg aggtgcataa tgccaagaca aagccgcggg aggagcagta cgccagcacg 900
   taccgtgtgg tcagcgtcct caccgtcctg caccaggact ggctgaatgg caaggagtac 960
   aagtgcaagg tctccaacaa agccctccca gcccccatcg agaaaaccat ctccaaagcc 1020
   aaagggcagc cccgagaacc acaggtgtac accctgcccc catcccggga ggagatgacc 1080
   aagaaccagg tcagcctgac ctgcctggtc aaaggcttct atcccagcga catcgccgtg 1140
   gagtgggaga gcaatgggca gccggagaac aactacaaga ccacgcctcc cgtgctggac 1200
   tccgacggct ccttcttcct ctacagcaag ctcaccgtgg acaagagcag gtggcagcag 1260
   gggaacgtct tctcatgctc cgtgatgcat gaggctctgc acaaccacta cacgcagaag 1320
   agcctctccc tgtctccggg taaatga 1347
<210> 375
   <211> 33
   <212> DNA
   <213> Oryctolagus cuniculus
<400> 375
   caggctagtc agaccattag caactactta gcc 33
<210> 376
   <211> 21
   <212> DNA
   <213> Oryctolagus cuniculus
<400> 376
   ggtgcatcca atctggaatc t 21
<210> 377
   <211> 39
   <212> DNA
   <213> Oryctolagus cuniculus
<400> 377
   caacagggtt atactatcag taatgttgat aacaatgtt 39
<210> 378
   <211> 15
   <212> DNA
   <213> Oryctolagus cuniculus
<400> 378
   ggctacaact tggtc 15
<210> 379
   <211> 48
   <212> DNA
   <213> Synthetic
<400> 379
   ttcattagtt atggtgatac cacatactac gctagctctg ctaaaggc 48
<210> 380
   <211> 30
   <212> DNA
   <213> Oryctolagus cuniculus
<400> 380
   gagactgcta atacttatga ttatggcatc 30
<210> 381
   <211> 336
   <212> DNA
   <213> Oryctolagus cuniculus
<400> 381
   gccgccgtgc tgacccagac tccatctccc gtgtctgcag ctgtgggagg cacagtcagc       60
   atcagttgcc agtccagtca gaatgtttat aagaacaact atttatcctg gtatcagcag      120
   aaaccagggc agcctcccaa gctcctgatc tacaaggctt ccactctggc atctggggtc      180
   ccatcgcggt tcaaaggcag tggatctggg acagatttca ctctcaccat cagcgacgtg      240
   cagtgtgacg ctgctgccac ttactactgt gcaggcggtt atagtagtag tagtgataat      300
   gctttcggcg gagggaccga ggtggtggtc aaacgt                                336
<210> 382
   <211> 657
   <212> DNA
   <213> Synthetic
<400> 382
   gccgccgtgc tgacccagac tccatctccc gtgtctgcag ctgtgggagg cacagtcagc      60
   atcagttgcc agtccagtca gaatgtttat aagaacaact atttatcctg gtatcagcag     120
   aaaccagggc agcctcccaa gctcctgatc tacaaggctt ccactctggc atctggggtc     180
   ccatcgcggt tcaaaggcag tggatctggg acagatttca ctctcaccat cagcgacgtg     240
   cagtgtgacg ctgctgccac ttactactgt gcaggcggtt atagtagtag tagtgataat     300
   gctttcggcg gagggaccga ggtggtggtc aaacgtacgg tagcggcccc atctgtcttc     360
   atcttcccgc catctgatga gcagttgaaa tctggaactg cctctgttgt gtgcctgctg     420
   aataacttct atcccagaga ggccaaagta cagtggaagg tggataacgc cctccaatcg     480
   ggtaactccc aggagagtgt cacagagcag gacagcaagg acagcaccta cagcctcagc     540
   agcaccctga cgctgagcaa agcagactac gagaaacaca aagtctacgc ctgcgaagtc     600
   acccatcagg gcctgagctc gcccgtcaca aagagcttca acaggggaga gtgttag        657
<210> 383
   <211> 351
   <212> DNA
   <213> Oryctolagus cuniculus
<400> 383
   cagtcggtgg aggcgtccgg gggtcgtctg gtcatgcctg gaggatccct gacactcacc      60
   tgcacagcct ctggattctc cctcagtacc tactggatgt cctgggtccg ccaggctcca     120
   gggaaggggc tggaatggat cggagacatt tattttagta atgaggaaac aaactacgcg     180
   acctgggcga aaggccgatt taccatctcc aaaacctcga ccacggtgga tctgaatgtc     240
   atcagtccga caaccgagga cacggccacc tatttctgtg caagaggttc tcctgatgtt     300
   gagattgcta tagatatgtg gggccagggc accctcgtca ccgtctcgag c                351
<210> 384
   <211> 1344
   <212> DNA
   <213> Synthetic
<400> 384
   cagtcggtgg aggcgtccgg gggtcgtctg gtcatgcctg gaggatccct gacactcacc      60
   tgcacagcct ctggattctc cctcagtacc tactggatgt cctgggtccg ccaggctcca     120
   gggaaggggc tggaatggat cggagacatt tattttagta atgaggaaac aaactacgcg     180
   acctgggcga aaggccgatt taccatctcc aaaacctcga ccacggtgga tctgaatgtc     240
   atcagtccga caaccgagga cacggccacc tatttctgtg caagaggttc tcctgatgtt     300
   gagattgcta tagatatgtg gggccagggc accctcgtca ccgtctcgag cgcctccacc     360
   aagggcccat cggtcttccc cctggcaccc tcctccaaga gcacctctgg gggcacagcg     420
   gccctgggct gcctggtcaa ggactacttc cccgaaccgg tgacggtgtc gtggaactca     480
   ggcgccctga ccagcggcgt gcacaccttc ccggctgtcc tacagtcctc aggactctac     540
   tccctcagca gcgtggtgac cgtgccctcc agcagcttgg gcacccagac ctacatctgc     600
   aacgtgaatc acaagcccag caacaccaag gtggacaaga gagttgagcc caaatcttgt     660
   gacaaaactc acacatgccc accgtgccca gcacctgaac tcctgggggg accgtcagtc     720
   ttcctcttcc ccccaaaacc caaggacacc ctcatgatct cccggacccc tgaggtcaca     780
   tgcgtggtgg tggacgtgag ccacgaagac cctgaggtca agttcaactg gtacgtggac     840
   ggcgtggagg tgcataatgc caagacaaag ccgcgggagg agcagtacgc cagcacgtac     900
   cgtgtggtca gcgtcctcac cgtcctgcac caggactggc tgaatggcaa ggagtacaag     960
   tgcaaggtct ccaacaaagc cctcccagcc cccatcgaga aaaccatctc caaagccaaa    1020
   gggcagcccc gagaaccaca ggtgtacacc ctgcccccat cccgggagga gatgaccaag    1080
   aaccaggtca gcctgacctg cctggtcaaa ggcttctatc ccagcgacat cgccgtggag    1140
   tgggagagca atgggcagcc ggagaacaac tacaagacca cgcctcccgt gctggactcc    1200
   gacggctcct tcttcctcta cagcaagctc accgtggaca agagcaggtg gcagcagggg    1260
   aacgtcttct catgctccgt gatgcatgag gctctgcaca accactacac gcagaagagc    1320
   ctctccctgt ctccgggtaa atga                                           1344
<210> 385
   <211> 39
   <212> DNA
   <213> Oryctolagus cuniculus
<400> 385
   cagtccagtc agaatgttta taagaacaac tatttatcc 39
<210> 386
   <211> 21
   <212> DNA
   <213> Oryctolagus cuniculus
<400> 386
   aaggcttcca ctctggcatc t 21
<210> 387
   <211> 33
   <212> DNA
   <213> Oryctolagus cuniculus
<400> 387
   gcaggcggtt atagtagtag tagtgataat gct 33
<210> 388
   <211> 15
   <212> DNA
   <213> Oryctolagus cuniculus
<400> 388
   acctactgga tgtcc 15
<210> 389
   <211> 51
   <212> DNA
   <213> Oryctolagus cuniculus
<400> 389
   gacatttatt ttagtaatga ggaaacaaac tacgcgacct gggcgaaagg c 51
<210> 390
   <211> 33
   <212> DNA
   <213> Oryctolagus cuniculus
<400> 390
   ggttctcctg atgttgagat tgctatagat atg 33
<210> 391
   <211> 336
   <212> DNA
   <213> Synthetic
<400> 391
   gacatccaga tgacccagtc tccatcctcc ctgtctgcat ctgtaggaga cagagtcacc      60
   atcacttgcc agtccagtca gaatgtttat aagaacaact acttatcctg gtatcagcag     120
   aaaccaggga aagtccctaa gctcctgatc tataaggcat ccactctggc atctggggtc     180
   ccatctcgtt tcagtggcag tggatctggg acagatttca ctctcaccat cagcagcctg     240
   cagcctgaag atgttgcaac ttattactgt gcaggcggtt ataccagtag tagtgataat     300
   gctttcggcg gaggaaccaa ggtggaaatc aaacgt                               336
<210> 392
   <211> 657
   <212> DNA
   <213> Synthetic
<400> 392
   gacatccaga tgacccagtc tccatcctcc ctgtctgcat ctgtaggaga cagagtcacc    60
   atcacttgcc agtccagtca gaatgtttat aagaacaact acttatcctg gtatcagcag   120
   aaaccaggga aagtccctaa gctcctgatc tataaggcat ccactctggc atctggggtc   180
   ccatctcgtt tcagtggcag tggatctggg acagatttca ctctcaccat cagcagcctg   240
   cagcctgaag atgttgcaac ttattactgt gcaggcggtt ataccagtag tagtgataat   300
   gctttcggcg gaggaaccaa ggtggaaatc aaacgtacgg tagcggcccc atctgtcttc   360
   atcttcccgc catctgatga gcagttgaaa tctggaactg cctctgttgt gtgcctgctg   420
   aataacttct atcccagaga ggccaaagta cagtggaagg tggataacgc cctccaatcg   480
   ggtaactccc aggagagtgt cacagagcag gacagcaagg acagcaccta cagcctcagc   540
   agcaccctga cgctgagcaa agcagactac gagaaacaca aagtctacgc ctgcgaagtc   600
   acccatcagg gcctgagctc gcccgtcaca aagagcttca acaggggaga gtgttag      657
<210> 393
   <211> 360
   <212> DNA
   <213> Synthetic
<400> 393
   gaggtgcagc tggtggagtc tgggggaggc ttggtccagc ctggggggtc cctgagactc    60
   tcctgtgcag cctctggatt caccgtcagt acctactgga tgagctgggt ccgtcaggct   120
   ccagggaagg ggctggagtg ggtcggagac atttacttta gtaatgaaga aacaaactac   180
   gcgaccagcg cgaaaggccg attcaccatc tccagagaca attccaagaa caccctgtat   240
   cttcaaatga acagcctgag agctgaggac actgctgtgt attactgtgc tagaggttct   300
   cctgatgttg agattgctat agatatgtgg ggccaaggga ccctcgtcac cgtctcgagc   360
<210> 394
   <211> 1353
   <212> DNA
   <213> Synthetic
<400> 394
   gaggtgcagc tggtggagtc tgggggaggc ttggtccagc ctggggggtc cctgagactc 60
   tcctgtgcag cctctggatt caccgtcagt acctactgga tgagctgggt ccgtcaggct 120
   ccagggaagg ggctggagtg ggtcggagac atttacttta gtaatgaaga aacaaactac 180
   gcgaccagcg cgaaaggccg attcaccatc tccagagaca attccaagaa caccctgtat 240
   cttcaaatga acagcctgag agctgaggac actgctgtgt attactgtgc tagaggttct 300
   cctgatgttg agattgctat agatatgtgg ggccaaggga ccctcgtcac cgtctcgagc 360
   gcctccacca agggcccatc ggtcttcccc ctggcaccct cctccaagag cacctctggg 420
   ggcacagcgg ccctgggctg cctggtcaag gactacttcc ccgaaccggt gacggtgtcg 480
   tggaactcag gcgccctgac cagcggcgtg cacaccttcc cggctgtcct acagtcctca 540
   ggactctact ccctcagcag cgtggtgacc gtgccctcca gcagcttggg cacccagacc 600
   tacatctgca acgtgaatca caagcccagc aacaccaagg tggacaagag agttgagccc 660
   aaatcttgtg acaaaactca cacatgccca ccgtgcccag cacctgaact cctgggggga 720
   ccgtcagtct tcctcttccc cccaaaaccc aaggacaccc tcatgatctc ccggacccct 780
   gaggtcacat gcgtggtggt ggacgtgagc cacgaagacc ctgaggtcaa gttcaactgg 840
   tacgtggacg gcgtggaggt gcataatgcc aagacaaagc cgcgggagga gcagtacgcc 900
   agcacgtacc gtgtggtcag cgtcctcacc gtcctgcacc aggactggct gaatggcaag 960
   gagtacaagt gcaaggtctc caacaaagcc ctcccagccc ccatcgagaa aaccatctcc 1020
   aaagccaaag ggcagccccg agaaccacag gtgtacaccc tgcccccatc ccgggaggag 1080
   atgaccaaga accaggtcag cctgacctgc ctggtcaaag gcttctatcc cagcgacatc 1140
   gccgtggagt gggagagcaa tgggcagccg gagaacaact acaagaccac gcctcccgtg 1200
   ctggactccg acggctcctt cttcctctac agcaagctca ccgtggacaa gagcaggtgg 1260
   cagcagggga acgtcttctc atgctccgtg atgcatgagg ctctgcacaa ccactacacg 1320
   cagaagagcc tctccctgtc tccgggtaaa tga 1353
<210> 395
   <211> 39
   <212> DNA
   <213> Oryctolagus cuniculus
<400> 395
   cagtccagtc agaatgttta taagaacaac tacttatcc 39
<210> 396
   <211> 21
   <212> DNA
   <213> Oryctolagus cuniculus
<400> 396
   aaggcatcca ctctggcatc t 21
<210> 397
   <211> 33
   <212> DNA
   <213> Oryctolagus cuniculus
<400> 397
   gcaggcggtt ataccagtag tagtgataat gct 33
<210> 398
   <211> 15
   <212> DNA
   <213> Oryctolagus cuniculus
<400> 398
   acctactgga tgagc 15
<210> 399
   <211> 51
   <212> DNA
   <213> Synthetic
<400> 399
   gacatttact ttagtaatga agaaacaaac tacgcgacca gcgcgaaagg c 51
<210> 400
   <211> 33
   <212> DNA
   <213> Synthetic
<400> 400
   ggttctcctg atgttgagat tgctatagat atg 33
<210> 401
   <211> 217
   <212> PRT
   <213> Synthetic
<400> 401
   Asp Ile Gln Met Thr Gln Ser Pro Ser Thr Leu Ser Ala Ser Val Gly
   1               5                   10                  15
   Asp Arg Val Thr Ile Thr Cys Gln Ala Ser Gln Ser Ile Tyr Ser Asn
               20                  25                  30
   Leu Ala Trp Tyr Gln Gln Lys Pro Gly Lys Ala Pro Lys Leu Leu Ile
           35                  40                  45
   Tyr Asp Ala Ser Thr Leu Glu Ser Gly Val Pro Ser Arg Phe Ser Gly
       50                  55                  60
   Ser Gly Ser Gly Thr Glu Tyr Thr Leu Thr Ile Ser Ser Leu Gln Pro
   65                  70                  75                  80
   Asp Asp Phe Ala Thr Tyr Tyr Cys Gln Gln Gly Phe Thr Val Ser Asp
                   85                  90                  95
   Ile Asp Asn Ala Phe Gly Gly Gly Thr Lys Val Glu Ile Lys Arg Thr
               100                 105                 110
   Val Ala Ala Pro Ser Val Phe Ile Phe Pro Pro Ser Asp Glu Gln Leu
           115                 120                 125
   Lys Ser Gly Thr Ala Ser Val Val Cys Leu Leu Asn Asn Phe Tyr Pro
       130                 135                 140
   Arg Glu Ala Lys Val Gln Trp Lys Val Asp Asn Ala Leu Gln Ser Gly
   145                 150                 155                 160
   Asn Ser Gln Glu Ser Val Thr Glu Gln Asp Ser Lys Asp Ser Thr Tyr
                   165                 170                 175
   Ser Leu Ser Ser Thr Leu Thr Leu Ser Lys Ala Asp Tyr Glu Lys His
               180                 185                 190
   Lys Val Tyr Ala Cys Glu Val Thr His Gln Gly Leu Ser Ser Pro Val
           195                 200                 205
   Thr Lys Ser Phe Asn Arg Gly Glu Cys
       210                 215
<210> 402
   <211> 451
   <212> PRT
   <213> Synthetic
<400> 402
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Val Ser Asn Tyr
   20 25 30
   Ala Val Gly Trp Val Arg Gln Ala Pro Gly Lys Gly Leu Glu Trp Val
   35 40 45
   Gly Ile Ile Gly Arg Asn Gly Asn Thr Trp Tyr Ala Ser Ser Ala Arg
   50 55 60
   Gly Arg Phe Thr Ile Ser Arg Asp Asn Ser Lys Asn Thr Leu Tyr Leu
   65 70 75 80
   Gln Met Asn Ser Leu Arg Ala Glu Asp Thr Ala Val Tyr Tyr Cys Ala
   85 90 95
   Arg Gly Tyr Gly Arg Ser Val Ala Tyr Tyr Val Phe Asn Ile Trp Gly
   100 105 110
   Pro Gly Thr Leu Val Thr Val Ser Ser Ala Ser Thr Lys Gly Pro Ser
   115 120 125
   Val Phe Pro Leu Ala Pro Ser Ser Lys Ser Thr Ser Gly Gly Thr Ala
   130 135 140
   Ala Leu Gly Cys Leu Val Lys Asp Tyr Phe Pro Glu Pro Val Thr Val
   145 150 155 160
   Ser Trp Asn Ser Gly Ala Leu Thr Ser Gly Val His Thr Phe Pro Ala
   165 170 175
   Val Leu Gln Ser Ser Gly Leu Tyr Ser Leu Ser Ser Val Val Thr Val
   180 185 190
   Pro Ser Ser Ser Leu Gly Thr Gln Thr Tyr Ile Cys Asn Val Asn His
   195 200 205
   Lys Pro Ser Asn Thr Lys Val Asp Ala Arg Val Glu Pro Lys Ser Cys
   210 215 220
   Asp Lys Thr His Thr Cys Pro Pro Cys Pro Ala Pro Glu Leu Leu Gly
   225 230 235 240
   Gly Pro Ser Val Phe Leu Phe Pro Pro Lys Pro Lys Asp Thr Leu Met
   245 250 255
   Ile Ser Arg Thr Pro Glu Val Thr Cys Val Val Val Asp Val Ser His
               260                 265                 270
   Glu Asp Pro Glu Val Lys Phe Asn Trp Tyr Val Asp Gly Val Glu Val
           275                 280                 285
   His Asn Ala Lys Thr Lys Pro Arg Glu Glu Gln Tyr Ala Ser Thr Tyr
       290                 295                 300
   Arg Val Val Ser Val Leu Thr Val Leu His Gln Asp Trp Leu Asn Gly
   305                 310                 315                 320
   Lys Glu Tyr Lys Cys Lys Val Ser Asn Lys Ala Leu Pro Ala Pro Ile
                   325                 330                 335
   Glu Lys Thr Ile Ser Lys Ala Lys Gly Gln Pro Arg Glu Pro Gln Val
               340                 345                 350
   Tyr Thr Leu Pro Pro Ser Arg Glu Glu Met Thr Lys Asn Gln Val Ser
           355                 360                 365
   Leu Thr Cys Leu Val Lys Gly Phe Tyr Pro Ser Asp Ile Ala Val Glu
       370                 375                 380
   Trp Glu Ser Asn Gly Gln Pro Glu Asn Asn Tyr Lys Thr Thr Pro Pro
   385                 390                 395                 400
   Val Leu Asp Ser Asp Gly Ser Phe Phe Leu Tyr Ser Lys Leu Thr Val
                   405                 410                 415
   Asp Lys Ser Arg Trp Gln Gln Gly Asn Val Phe Ser Cys Ser Val Met
               420                 425                 430
   His Glu Ala Leu His Asn His Tyr Thr Gln Lys Ser Leu Ser Leu Ser
           435                 440                 445
   Pro Gly Lys
       450
<210> 403
   <211> 654
   <212> DNA
   <213> Synthetic
<400> 403
   gacatccaga tgacccagtc tccttccacc ctgtctgcat ctgtaggaga cagagtcacc 60
   atcacttgcc aggccagtca gagcatttac agcaatcttg cctggtatca gcagaaacca 120
   ggaaaagccc ctaagctcct gatctatgat gcatccactc tggaatctgg agtcccatca 180
   aggttcagcg gcagtggatc tgggacagag tacactctca ccatcagcag cctgcagcct 240
   gatgattttg caacttacta ctgccaacag ggttttactg ttagtgatat tgataatgct 300
   ttcggcggag gaaccaaggt ggaaatcaaa cgtacggtag cggccccatc tgtcttcatc 360
   ttcccgccat ctgatgagca gttgaaatct ggaactgcct ctgttgtgtg cctgctgaat 420
   aacttctatc ccagagaggc caaagtacag tggaaggtgg ataacgccct ccaatcgggt 480
   aactcccagg agagtgtcac agagcaggac agcaaggaca gcacctacag cctcagcagc 540
   accctgacgc tgagcaaagc agactacgag aaacacaaag tctacgcctg cgaagtcacc 600
   catcagggcc tgagctcgcc cgtcacaaag agcttcaaca ggggagagtg ttag 654
<210> 404
   <211> 1356
   <212> DNA
   <213> Synthetic
<400> 404
   gaggtgcagc tggtggagtc tgggggaggc ttggtccagc ctggggggtc cctgagactc 60
   tcctgtgcag cctctggatt caccgtcagt aactatgcag tgggctgggt ccgtcaggct 120
   ccagggaagg ggctggagtg ggtcggaatc attggtcgta atggtaacac atggtacgcg 180
   agctctgcaa gaggccgatt caccatctcc agagacaatt ccaagaacac cctgtatctt 240
   caaatgaaca gcctgagagc tgaggacact gctgtgtatt actgtgctag aggatatggc 300
   cgtagtgttg cttactacgt ctttaacatc tggggcccag ggaccctcgt caccgtctcg 360
   agcgcctcca ccaagggccc atcggtcttc cccctggcac cctcctccaa gagcacctct 420
   gggggcacag cggccctggg ctgcctggtc aaggactact tccccgaacc ggtgacggtg 480
   tcgtggaact caggcgccct gaccagcggc gtgcacacct tcccggctgt cctacagtcc 540
   tcaggactct actccctcag cagcgtggtg accgtgccct ccagcagctt gggcacccag 600
   acctacatct gcaacgtgaa tcacaagccc agcaacacca aggtggacgc gagagttgag 660
   cccaaatctt gtgacaaaac tcacacatgc ccaccgtgcc cagcacctga actcctgggg 720
   ggaccgtcag tcttcctctt ccccccaaaa cccaaggaca ccctcatgat ctcccggacc 780
   cctgaggtca catgcgtggt ggtggacgtg agccacgaag accctgaggt caagttcaac 840
   tggtacgtgg acggcgtgga ggtgcataat gccaagacaa agccgcggga ggagcagtac 900
   gccagcacgt accgtgtggt cagcgtcctc accgtcctgc accaggactg gctgaatggc 960
   aaggagtaca agtgcaaggt ctccaacaaa gccctcccag cccccatcga gaaaaccatc 1020
   tccaaagcca aagggcagcc ccgagaacca caggtgtaca ccctgccccc atcccgggag 1080
   gagatgacca agaaccaggt cagcctgacc tgcctggtca aaggcttcta tcccagcgac 1140
   atcgccgtgg agtgggagag caatgggcag ccggagaaca actacaagac cacgcctccc 1200
   gtgctggact ccgacggctc cttcttcctc tacagcaagc tcaccgtgga caagagcagg 1260
   tggcagcagg ggaacgtctt ctcatgctcc gtgatgcatg aggctctgca caaccactac 1320
   acgcagaaga gcctctccct gtctccgggt aaatga 1356
<210> 405
   <211> 217
   <212> PRT
   <213> Synthetic
<400> 405
   Asp Ile Gln Met Thr Gln Ser Pro Ser Thr Leu Ser Ala Ser Val Gly
   1               5                   10                  15
   Asp Arg Val Thr Ile Thr Cys Gln Ala Ser Gln Ser Ile Tyr Ser Asn
               20                  25                  30
   Leu Ala Trp Tyr Gln Gln Lys Pro Gly Lys Ala Pro Lys Leu Leu Ile
           35                  40                  45
   Tyr Asp Ala Ser Thr Leu Glu Ser Gly Val Pro Ser Arg Phe Ser Gly
       50                  55                  60
   Ser Gly Ser Gly Thr Glu Tyr Thr Leu Thr Ile Ser Ser Leu Gln Pro
   65                  70                  75                  80
   Asp Asp Phe Ala Thr Tyr Tyr Cys Gln Gln Gly Phe Thr Val Ser Asp
                   85                  90                  95
   Ile Asp Asn Ala Phe Gly Gly Gly Thr Lys Val Glu Ile Lys Arg Thr
               100                 105                 110
   Val Ala Ala Pro Ser Val Phe Ile Phe Pro Pro Ser Asp Glu Gln Leu
           115                 120                 125
   Lys Ser Gly Thr Ala Ser Val Val Cys Leu Leu Asn Asn Phe Tyr Pro
       130                 135                 140
   Arg Glu Ala Lys Val Gln Trp Lys Val Asp Asn Ala Leu Gln Ser Gly
   145                 150                 155                 160
   Asn Ser Gln Glu Ser Val Thr Glu Gln Asp Ser Lys Asp Ser Thr Tyr
                   165                 170                 175
   Ser Leu Ser Ser Thr Leu Thr Leu Ser Lys Ala Asp Tyr Glu Lys His
               180                 185                 190
   Lys Val Tyr Ala Cys Glu Val Thr His Gln Gly Leu Ser Ser Pro Val
           195                 200                 205
   Thr Lys Ser Phe Asn Arg Gly Glu Cys
       210                 215
<210> 406
   <211> 228
   <212> PRT
   <213> Synthetic
<400> 406
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Val Ser Asn Tyr
               20                  25                  30
   Ala Val Gly Trp Val Arg Gln Ala Pro Gly Lys Gly Leu Glu Trp Val
           35                  40                  45
   Gly Ile Ile Gly Arg Asn Gly Asn Thr Trp Tyr Ala Ser Ser Ala Arg
       50                  55                  60
   Gly Arg Phe Thr Ile Ser Arg Asp Asn Ser Lys Asn Thr Leu Tyr Leu
   65                  70                  75                  80
   Gln Met Asn Ser Leu Arg Ala Glu Asp Thr Ala Val Tyr Tyr Cys Ala
                   85                  90                  95
   Arg Gly Tyr Gly Arg Ser Val Ala Tyr Tyr Val Phe Asn Ile Trp Gly
               100                 105                 110
   Pro Gly Thr Leu Val Thr Val Ser Ser Ala Ser Thr Lys Gly Pro Ser
           115                 120                 125
   Val Phe Pro Leu Ala Pro Ser Ser Lys Ser Thr Ser Gly Gly Thr Ala
       130                 135                 140
   Ala Leu Gly Cys Leu Val Lys Asp Tyr Phe Pro Glu Pro Val Thr Val
   145                 150                 155                 160
   Ser Trp Asn Ser Gly Ala Leu Thr Ser Gly Val His Thr Phe Pro Ala
                   165                 170                 175
   Val Leu Gln Ser Ser Gly Leu Tyr Ser Leu Ser Ser Val Val Thr Val
               180                 185                 190
   Pro Ser Ser Ser Leu Gly Thr Gln Thr Tyr Ile Cys Asn Val Asn His
           195                 200                 205
   Lys Pro Ser Asn Thr Lys Val Asp Ala Arg Val Glu Pro Lys Ser Cys
       210                 215                 220
   Asp Lys Thr His
   225
<210> 407
   <211> 217
   <212> PRT
   <213> Synthetic
<400> 407
   Asp Ile Gln Met Thr Gln Ser Pro Ser Thr Leu Ser Ala Ser Val Gly
   1               5                   10                  15
   Asp Arg Val Thr Ile Thr Cys Gln Ala Ser Gln Ser Ile Tyr Ser Asn
               20                  25                  30
   Leu Ala Trp Tyr Gln Gln Lys Pro Gly Lys Ala Pro Lys Leu Leu Ile
           35                  40                  45
   Tyr Asp Ala Ser Thr Leu Glu Ser Gly Val Pro Ser Arg Phe Ser Gly
       50                  55                  60
   Ser Gly Ser Gly Thr Glu Tyr Thr Leu Thr Ile Ser Ser Leu Gln Pro
   65                  70                  75                  80
   Asp Asp Phe Ala Thr Tyr Tyr Cys Gln Gln Gly Phe Thr Val Ser Asp
                   85                  90                  95
   Ile Asp Asn Ala Phe Gly Gly Gly Thr Lys Val Glu Ile Lys Arg Thr
               100                 105                 110
   Val Ala Ala Pro Ser Val Phe Ile Phe Pro Pro Ser Asp Glu Gln Leu
           115                 120                 125
   Lys Ser Gly Thr Ala Ser Val Val Cys Leu Leu Asn Asn Phe Tyr Pro
       130                 135                 140
   Arg Glu Ala Lys Val Gln Trp Lys Val Asp Asn Ala Leu Gln Ser Gly
   145                 150                 155                 160
   Asn Ser Gln Glu Ser Val Thr Glu Gln Asp Ser Lys Asp Ser Thr Tyr
                   165                 170                 175
   Ser Leu Ser Ser Thr Leu Thr Leu Ser Lys Ala Asp Tyr Glu Lys His
               180                 185                 190
   Lys Val Tyr Ala Cys Glu Val Thr His Gln Gly Leu Ser Ser Pro Val
           195                 200                 205
   Thr Lys Ser Phe Asn Arg Gly Glu Cys
       210                 215
<210> 408
   <211> 228
   <212> PRT
   <213> Synthetic
<400> 408
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Val Ser Asn Tyr
               20                  25                  30
   Ala Val Gly Trp Val Arg Gln Ala Pro Gly Lys Gly Leu Glu Trp Val
           35                  40                  45
   Gly Ile Ile Gly Arg Asn Gly Asn Thr Trp Tyr Ala Ser Ser Ala Arg
       50                  55                  60
   Gly Arg Phe Thr Ile Ser Arg Asp Asn Ser Lys Asn Thr Leu Tyr Leu
   65                  70                  75                  80
   Gln Met Asn Ser Leu Arg Ala Glu Asp Thr Ala Val Tyr Tyr Cys Ala
                   85                  90                  95
   Arg Gly Tyr Gly Arg Ser Val Ala Tyr Tyr Val Phe Asn Ile Trp Gly
               100                 105                 110
   Pro Gly Thr Leu Val Thr Val Ser Ser Ala Ser Thr Lys Gly Pro Ser
           115                 120                 125
   Val Phe Pro Leu Ala Pro Ser Ser Lys Ser Thr Ser Gly Gly Thr Ala
       130                 135                 140
   Ala Leu Gly Cys Leu Val Lys Asp Tyr Phe Pro Glu Pro Val Thr Val
   145                 150                 155                 160
   Ser Trp Asn Ser Gly Ala Leu Thr Ser Gly Val His Thr Phe Pro Ala
                   165                 170                 175
   Val Leu Gln Ser Ser Gly Leu Tyr Ser Leu Ser Ser Val Val Thr Val
               180                 185                 190
   Pro Ser Ser Ser Leu Gly Thr Gln Thr Tyr Ile Cys Asn Val Asn His
           195                 200                 205
   Lys Pro Ser Asn Thr Lys Val Asp Ala Arg Val Glu Pro Lys Ser Cys
       210                 215                 220
   Asp Lys Thr His
   225
<210> 409
   <211> 654
   <212> DNA
   <213> Synthetic
<400> 409
   gacatccaga tgacccagtc tccttccacc ctgtctgcat ctgtaggaga cagagtcacc      60
   atcacttgcc aggccagtca gagcatttac agcaatcttg cctggtatca gcagaaacca     120
   ggaaaagccc ctaagctcct gatctatgat gcatccactc tggaatctgg agtcccatca     180
   aggttcagcg gcagtggatc tgggacagag tacactctca ccatcagcag cctgcagcct     240
   gatgattttg caacttacta ctgccaacag ggttttactg ttagtgatat tgataatgct     300
   ttcggcggag gaaccaaggt ggaaatcaaa cgtacggtag cggccccatc tgtcttcatc     360
   ttcccgccat ctgatgagca gttgaaatct ggaactgcct ctgttgtgtg cctgctgaat     420
   aacttctatc ccagagaggc caaagtacag tggaaggtgg ataacgccct ccaatcgggt     480
   aactcccagg agagtgtcac agagcaggac agcaaggaca gcacctacag cctcagcagc     540
   accctgacgc tgagcaaagc agactacgag aaacacaaag tctacgcctg cgaagtcacc     600
   catcagggcc tgagctcgcc cgtcacaaag agcttcaaca ggggagagtg ttag           654
<210> 410
   <211> 687
   <212> DNA
   <213> Synthetic
<400> 410
   gaggtgcagc tggtggagtc tgggggaggc ttggtccagc ctggggggtc cctgagactc        60
   tcctgtgcag cctctggatt caccgtcagt aactatgcag tgggctgggt ccgtcaggct       120
   ccagggaagg ggctggagtg ggtcggaatc attggtcgta atggtaacac atggtacgcg       180
   agctctgcaa gaggccgatt caccatctcc agagacaatt ccaagaacac cctgtatctt       240
   caaatgaaca gcctgagagc tgaggacact gctgtgtatt actgtgctag aggatatggc       300
   cgtagtgttg cttactacgt ctttaacatc tggggcccag ggaccctcgt caccgtctcg       360
   agcgcctcca ccaagggccc atcggtcttc cccctggcac cctcctccaa gagcacctct       420
   gggggcacag cggccctggg ctgcctggtc aaggactact tccccgaacc ggtgacggtg       480
   tcgtggaact caggcgccct gaccagcggc gtgcacacct tcccggctgt cctacagtcc       540
   tcaggactct actccctcag cagcgtggtg accgtgccct ccagcagctt gggcacccag       600
   acctacatct gcaacgtgaa tcacaagccc agcaacacca aggtggacgc gagagttgag       660
   cccaaatctt gtgacaaaac tcactag                                           687
<210> 411
   <211> 120
   <212> PRT
   <213> Homo sapiens
<400> 411
   Ser Ser Ser His Pro Ile Phe His Arg Gly Glu Phe Ser Val Cys Asp
   1               5                   10                  15
   Ser Val Ser Val Trp Val Gly Asp Lys Thr Thr Ala Thr Asp Ile Lys
               20                  25                  30
   Gly Lys Glu Val Met Val Leu Gly Glu Val Asn Ile Asn Asn Ser Val
           35                  40                  45
   Phe Lys Gln Tyr Phe Phe Glu Thr Lys Cys Arg Asp Pro Asn Pro Val
       50                  55                  60
   Asp Ser Gly Cys Arg Gly Ile Asp Ser Lys His Trp Asn Ser Tyr Cys
   65                  70                  75                  80
   Thr Thr Thr His Thr Phe Val Lys Ala Leu Thr Met Asp Gly Lys Gln
                   85                  90                  95
   Ala Ala Trp Arg Phe Ile Arg Ile Asp Thr Ala Cys Val Cys Val Leu
               100                 105                 110
   Ser Arg Lys Ala Val Arg Arg Ala
           115                 120
<210> 412
   <211> 105
   <212> PRT
   <213> Homo sapiens
<400> 412
<210> 413
   <211> 330
   <212> PRT
   <213> Homo sapiens
<400> 413
   Ala Ser Thr Lys Gly Pro Ser Val Phe Pro Leu Ala Pro Ser Ser Lys
   1               5                   10                  15
   Ser Thr Ser Gly Gly Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Gln Thr
   65                  70                  75                  80
   Tyr Ile Cys Asn Val Asn His Lys Pro Ser Asn Thr Lys Val Asp Lys
                   85                  90                  95
   Arg Val Glu Pro Lys Ser Cys Asp Lys Thr His Thr Cys Pro Pro Cys
               100                 105                 110
   Pro Ala Pro Glu Leu Leu Gly Gly Pro Ser Val Phe Leu Phe Pro Pro
           115                 120                 125
   Lys Pro Lys Asp Thr Leu Met Ile Ser Arg Thr Pro Glu Val Thr Cys
   130 135 140
   Val Val Val Asp Val Ser His Glu Asp Pro Glu Val Lys Phe Asn Trp
   145 150 155 160
   Tyr Val Asp Gly Val Glu Val His Asn Ala Lys Thr Lys Pro Arg Glu
   165 170 175
   Glu Gln Tyr Ala Ser Thr Tyr Arg Val Val Ser Val Leu Thr Val Leu
   180 185 190
   His Gln Asp Trp Leu Asn Gly Lys Glu Tyr Lys Cys Lys Val Ser Asn
   195 200 205
   Lys Ala Leu Pro Ala Pro Ile Glu Lys Thr Ile Ser Lys Ala Lys Gly
   210 215 220
   Gln Pro Arg Glu Pro Gln Val Tyr Thr Leu Pro Pro Ser Arg Glu Glu
   225 230 235 240
   Met Thr Lys Asn Gln Val Ser Leu Thr Cys Leu Val Lys Gly Phe Tyr
   245 250 255
   Pro Ser Asp Ile Ala Val Glu Trp Glu Ser Asn Gly Gln Pro Glu Asn
   260 265 270
   Asn Tyr Lys Thr Thr Pro Pro Val Leu Asp Ser Asp Gly Ser Phe Phe
   275 280 285
   Leu Tyr Ser Lys Leu Thr Val Asp Lys Ser Arg Trp Gln Gln Gly Asn
   290 295 300
   Val Phe Ser Cys Ser Val Met His Glu Ala Leu His Asn His Tyr Thr
   305 310 315 320
   Gln Lys Ser Leu Ser Leu Ser Pro Gly Lys
   325 330
<210> 414
   <211> 18
   <212> PRT
   <213> Artificial
<220>
   <223> Secretion peptide
<400> 414
   Met Arg Ser Leu Leu Ile Leu Val Leu Cys Phe Leu Pro Leu Ala Ala
   1 5 10 15
   Leu Gly
<210> 415
   <211> 16
   <212> PRT
   <213> Artificial
<220>
   <223> Secretion peptide
<400> 415
   Met Arg Leu Leu Leu Leu Leu Leu Leu Leu Pro Leu Ala Ala Leu Gly
   1 5 10 15
<210> 416
   <211> 19
   <212> PRT
   <213> Artificial
<220>
   <223> Secretion peptide
<400> 416
   Met Leu Leu Gln Ala Phe Leu Phe Leu Leu Ala Gly Phe Ala Ala Lys
   1 5 10 15
   Ile Ser Ala
<210> 417
   <211> 35
   <212> PRT
   <213> Artificial
<220>
   <223> Secretion peptide
<400> 417
   Met Arg Phe Pro Ser Ile Phe Thr Ala Val Leu Phe Ala Ala Ser Ser
   1 5 10 15
   Ala Leu Ala Ala Pro Val Asn Thr Thr Thr Glu Glu Gly Val Ser Leu
   20 25 30
   Glu Lys Arg
   35
<210> 418
   <211> 22
   <212> PRT
   <213> Artificial
<220>
   <223> Secretion peptide
<400> 418
   Met Arg Phe Pro Ser Ile Phe Thr Ala Val Leu Phe Ala Ala Ser Ser
   1               5                   10                  15
   Ala Leu Ala Ala Pro Val
               20
<210> 419
   <211> 27
   <212> PRT
   <213> Artificial
<220>
   <223> Secretion peptide
<400> 419
   Met Arg Phe Pro Ser Ile Phe Thr Ala Val Leu Phe Ala Ala Ser Ser
   1               5                   10                  15
   Ala Leu Ala Ala Pro Val Ser Leu Glu Lys Arg
               20                  25
<210> 420
   <211> 41
   <212> PRT
   <213> Artificial
<220>
   <223> Secretion peptide
<400> 420
   Met Arg Phe Pro Ser Ile Phe Thr Ala Val Leu Phe Ala Ala Ser Ser
   1               5                   10                  15
   Ala Leu Ala Ala Pro Val Asn Thr Thr Thr Glu Glu Gly Val Ser Leu
               20                  25                  30
   Glu Lys Arg Glu Ala Glu Ala Glu Ala
           35                  40
<210> 421
   <211> 85
   <212> PRT
   <213> Artificial
<220>
   <223> Secretion peptide
<400> 421
   Met Arg Phe Pro Ser Ile Phe Thr Ala Val Leu Phe Ala Ala Ser Ser
   1               5                   10                  15
   Ala Leu Ala Ala Pro Val Asn Thr Thr Thr Glu Asp Glu Thr Ala Gln
               20                  25                  30
   Ile Pro Ala Glu Ala Val Ile Gly Tyr Ser Asp Leu Glu Gly Asp Phe
           35                  40                  45
   Asp Val Ala Val Leu Pro Phe Ser Asn Ser Thr Asn Asn Gly Leu Leu
       50                  55                  60
   Phe Ile Asn Thr Thr Ile Ala Ser Ile Ala Ala Lys Glu Glu Gly Val
   65                  70                  75                  80
   Ser Leu Glu Lys Arg
                   85
<210> 422
   <211> 44
   <212> PRT
   <213> Artificial
<220>
   <223> Secretion peptide
<400> 422
   Met Thr Lys Pro Thr Gln Val Leu Val Arg Ser Val Ser Ile Leu Phe
   1               5                   10                  15
   Phe Ile Thr Leu Leu His Leu Val Val Ala Leu Asn Asp Val Ala Gly
               20                  25                  30
   Pro Ala Glu Thr Ala Pro Val Ser Leu Leu Pro Arg
           35                  40
<210> 423
   <211> 22
   <212> PRT
   <213> Artificial
<220>
   <223> Secretion peptide
<400> 423
   Met Phe Ser Pro Ile Leu Ser Leu Glu Ile Ile Leu Ala Leu Ala Thr
   1               5                   10                  15
   Leu Gln Ser Val Phe Ala
               20
<210> 424
   <211> 22
   <212> PRT
   <213> Artificial
<220>
   <223> Secretion peptide
<400> 424
   Met Gln Thr Leu Leu Val Ser Ser Leu Val Val Ser Leu Ala Ala Ala
   1               5                   10                  15
   Leu Pro His Tyr Ile Arg
               20
<210> 425
   <211> 22
   <212> PRT
   <213> Artificial
<220>
   <223> Secretion peptide
<400> 425
   Met Ile Phe Leu Lys Leu Ile Lys Ser Ile Val Ile Gly Leu Gly Leu
   1               5                   10                  15
   Val Ser Ala Ile Gln Ala
               20
<210> 426
   <211> 24
   <212> PRT
   <213> Artificial
<220>
   <223> Secretion peptide
<400> 426
   Met Lys Trp Val Thr Phe Ile Ser Leu Leu Phe Leu Phe Ser Ser Ala
   1               5                   10                  15
   Tyr Ser Arg Gly Val Phe Arg Arg
               20
<210> 427
   <211> 18
   <212> PRT
   <213> Artificial
<220>
   <223> Secretion peptide
<400> 427
   Met Lys Trp Val Thr Phe Ile Ser Leu Leu Phe Leu Phe Ser Ser Ala
   1               5                   10                  15
   Tyr Ser
<210> 428
   <211> 24
   <212> PRT
   <213> Artificial
<220>
   <223> Secretion peptide
<400> 428
   Met Ala Leu Trp Met Arg Leu Leu Pro Leu Leu Ala Leu Leu Ala Leu
   1               5                   10                  15
   Trp Gly Pro Asp Pro Ala Ala Ala
               20
<210> 429
   <211> 24
   <212> PRT
   <213> Artificial
<220>
   <223> Secretion peptide
<400> 429
   Met Lys Tyr Thr Ser Tyr Ile Leu Ala Phe Gln Leu Cys Ile Val Leu
   1               5                   10                  15
   Gly Ser Leu Gly Cys Asp Leu Pro
               20
<210> 430
   <211> 26
   <212> PRT
   <213> Artificial
<220>
   <223> Secretion peptide
<400> 430
   Met Ala Ala Asp Ser Gln Thr Pro Trp Leu Leu Thr Phe Ser Leu Leu
   1               5                   10                  15
   Cys Leu Leu Trp Pro Gln Glu Pro Gly Ala
               20                  25
<210> 431
   <211> 27
   <212> PRT
   <213> Artificial
<220>
   <223> Secretion peptide
<400> 431
   Met Lys Lys Asn Arg Met Met Met Met Ile Trp Ser Val Gly Val Val
   1               5                   10                  15
   Trp Met Leu Leu Leu Val Gly Gly Ser Tyr Gly
               20                  25
<210> 432
   <211> 18
   <212> PRT
   <213> Artificial
<220>
   <223> Secretion peptide
<400> 432
   Met Gln Lys Leu Ile Ile Phe Ala Leu Val Val Leu Cys Val Gly Ser
   1               5                   10                  15
   Glu Ala
<210> 433
   <211> 18
   <212> PRT
   <213> Artificial
<220>
   <223> Secretion peptide
<400> 433
   Met Lys Ala Leu Ile Val Leu Gly Leu Val Leu Leu Ser Val Thr Val
   1               5                   10                  15
   Gln Gly
<210> 434
   <211> 20
   <212> PRT
   <213> Artificial
<220>
   <223> Secretion peptide
<400> 434
   Met Val Asp Gly Val Met Ile Leu Pro Val Leu Ile Met Ile Ala Leu
   1               5                   10                  15
   Pro Ser Pro Ser
               20
<210> 435
   <211> 19
   <212> PRT
   <213> Artificial
<220>
   <223> Secretion peptide
<400> 435
   Met Gly Ala Ala Ala Lys Leu Ala Phe Ala Val Phe Leu Ile Ser Cys
   1               5                   10                  15
   Ser Ser Gly
<210> 436
   <211> 31
   <212> PRT
   <213> Artificial
<220>
   <223> Secretion peptide
<400> 436
   Met Leu Ser Leu Lys Pro Ser Trp Leu Thr Leu Ala Ala Leu Met Tyr
   1               5                   10                  15
   Ala Met Leu Leu Val Val Val Pro Phe Ala Lys Pro Val Arg Ala
               20                  25                  30
<210> 437
   <211> 22
   <212> PRT
   <213> Artificial
<220>
   <223> Secretion peptide
<400> 437
   Met Asp Met Arg Val Pro Ala Gln Leu Leu Gly Leu Leu Leu Leu Trp
   1               5                   10                  15
   Leu Pro Gly Ala Lys Cys
               20
<210> 438
   <211> 651
   <212> DNA
   <213> Artificial
<220>
   <223> Humanized antibody
<400> 438
   gctatccaga tgacccagtc tccttcctcc ctgtctgcat ctgtaggaga cagagtcacc      60
   atcacttgcc aggccagtca gagcattaac aatgagttat cctggtatca gcagaaacca     120
   gggaaagccc ctaagctcct gatctatagg gcatccactc tggcatctgg ggtcccatca     180
   aggttcagcg gcagtggatc tgggacagac ttcactctca ccatcagcag cctgcagcct     240
   gatgattttg caacttatta ctgccaacag ggttatagtc tgaggaacat tgataatgct     300
   ttcggcggag ggaccaaggt ggaaatcaaa cgtacggtgg ctgcaccatc tgtcttcatc     360
   ttcccgccat ctgatgagca gttgaaatct ggaactgcct ctgttgtgtg cctgctgaat     420
   aacttctatc ccagagaggc caaagtacag tggaaggtgg ataacgccct ccaatcgggt     480
   aactcccagg agagtgtcac agagcaggac agcaaggaca gcacctacag cctcagcagc     540
   accctgacgc tgagcaaagc agactacgag aaacacaaag tctacgcctg cgaagtcacc     600
   catcagggcc tgagctcgcc cgtcacaaag agcttcaaca ggggagagtg t              651
<210> 439
   <211> 1350
   <212> DNA
   <213> Oryctolagus cuniculus
<220>
   <223> Humanized antibody
<400> 439
   gaggtgcagc tggtggagtc tgggggaggc ttggtccagc ctggggggtc cctgagactc      60
   tcctgtgcag cctctggatt ctccctcagt aactactacg tgacctgggt ccgtcaggct     120
   ccagggaagg ggctggagtg ggtcggcatc atctatggta gtgatgaaac cgcctacgct     180
   acctccgcta taggccgatt caccatctcc agagacaatt ccaagaacac cctgtatctt     240
   caaatgaaca gcctgagagc tgaggacact gctgtgtatt actgtgctag agatgatagt     300
   agtgactggg atgcaaagtt caacttgtgg ggccaaggga ccctcgtcac cgtctcgagc     360
   gcctccacca agggcccatc ggtcttcccc ctggcaccct cctccaagag cacctctggg     420
   ggcacagcgg ccctgggctg cctggtcaag gactacttcc ccgaaccggt gacggtgtcg     480
   tggaactcag gcgccctgac cagcggcgtg cacaccttcc cggctgtcct acagtcctca     540
   ggactctact ccctcagcag cgtggtgacc gtgccctcca gcagcttggg cacccagacc     600
   tacatctgca acgtgaatca caagcccagc aacaccaagg tggacaagag agttgagccc     660
   aaatcttgtg acaaaactca cacatgccca ccgtgcccag cacctgaact cctgggggga     720
   ccgtcagtct tcctcttccc cccaaaaccc aaggacaccc tcatgatctc ccggacccct     780
   gaggtcacat gcgtggtggt ggacgtgagc cacgaagacc ctgaggtcaa gttcaactgg     840
   tacgtggacg gcgtggaggt gcataatgcc aagacaaagc cgcgggagga gcagtacgcc     900
   agcacgtacc gtgtggtcag cgtcctcacc gtcctgcacc aggactggct gaatggcaag     960
   gagtacaagt gcaaggtctc caacaaagcc ctcccagccc ccatcgagaa aaccatctcc    1020
   aaagccaaag ggcagccccg agaaccacag gtgtacaccc tgcccccatc ccgggaggag    1080
   atgaccaaga accaggtcag cctgacctgc ctggtcaaag gcttctatcc cagcgacatc    1140
   gccgtggagt gggagagcaa tgggcagccg gagaacaact acaagaccac gcctcccgtg    1200
   ctggactccg acggctcctt cttcctctac agcaagctca ccgtggacaa gagcaggtgg    1260
   cagcagggga acgtcttctc atgctccgtg atgcatgagg ctctgcacaa ccactacacg    1320
   cagaagagcc tctccctgtc tccgggtaaa                                     1350
<210> 440
   <211> 660
   <212> DNA
   <213> Artificial Sequence
<220>
   <223> Humanized antibody
<400> 440
   caagtgctga cccagtctcc atcctccctg tctgcatctg taggagacag agtcaccatc 60
   aattgccagg ccagtcagag tgtttatcat aacacctacc tggcctggta tcagcagaaa 120
   ccagggaaag ttcctaagca actgatctat gatgcatcca ctctggcatc tggggtccca 180
   tctcgtttca gtggcagtgg atctgggaca gatttcactc tcaccatcag cagcctgcag 240
   cctgaagatg ttgcaactta ttactgtctg ggcagttatg attgtactaa tggtgattgt 300
   tttgttttcg gcggaggaac caaggtggaa atcaaacgta cggtggctgc accatctgtc 360
   ttcatcttcc cgccatctga tgagcagttg aaatctggaa ctgcctctgt tgtgtgcctg 420
   ctgaataact tctatcccag agaggccaaa gtacagtgga aggtggataa cgccctccaa 480
   tcgggtaact cccaggagag tgtcacagag caggacagca aggacagcac ctacagcctc 540
   agcagcaccc tgacgctgag caaagcagac tacgagaaac acaaagtcta cgcctgcgaa 600
   gtcacccatc agggcctgag ctcgcccgtc acaaagagct tcaacagggg agagtgttag 660
<210> 441
   <211> 1326
   <212> DNA
   <213> Artificial Sequence
<220>
   <223> Humanized antibody
<400> 441
   gaggtgcagc ttgtggagtc tgggggaggc ttggtccagc ctggggggtc cctgagactc      60
   tcctgtgcag tctctggaat cgacctcagt ggctactaca tgaactgggt ccgtcaggct     120
   ccagggaagg ggctggagtg ggtcggagtc attggtatta atggtgccac atactacgcg     180
   agctgggcga aaggccgatt caccatctcc agagacaatt ccaagaccac ggtgtatctt     240
   caaatgaaca gcctgagagc tgaggacact gctgtgtatt tctgtgctag aggggacatc     300
   tggggccaag ggaccctcgt caccgtctcg agcgcctcca ccaagggccc atcggtcttc     360
   cccctggcac cctcctccaa gagcacctct gggggcacag cggccctggg ctgcctggtc     420
   aaggactact tccccgaacc ggtgacggtg tcgtggaact caggcgccct gaccagcggc     480
   gtgcacacct tcccggctgt cctacagtcc tcaggactct actccctcag cagcgtggtg     540
   accgtgccct ccagcagctt gggcacccag acctacatct gcaacgtgaa tcacaagccc     600
   agcaacacca aggtggacgc gagagttgag cccaaatctt gtgacaaaac tcacacatgc     660
   ccaccgtgcc cagcacctga actcctgggg ggaccgtcag tcttcctctt ccccccaaaa     720
   cccaaggaca ccctcatgat ctcccggacc cctgaggtca catgcgtggt ggtggacgtg     780
   agccacgaag accctgaggt caagttcaac tggtacgtgg acggcgtgga ggtgcataat     840
   gccaagacaa agccgcggga ggagcagtac gccagcacgt accgtgtggt cagcgtcctc     900
   accgtcctgc accaggactg gctgaatggc aaggagtaca agtgcaaggt ctccaacaaa     960
   gccctcccag cccccatcga gaaaaccatc tccaaagcca aagggcagcc ccgagaacca    1020
   caggtgtaca ccctgccccc atcccgggag gagatgacca agaaccaggt cagcctgacc    1080
   tgcctggtca aaggcttcta tcccagcgac atcgccgtgg agtgggagag caatgggcag    1140
   ccggagaaca actacaagac cacgcctccc gtgctggact ccgacggctc cttcttcctc    1200
   tacagcaagc tcaccgtgga caagagcagg tggcagcagg ggaacgtctt ctcatgctcc    1260
   gtgatgcatg aggctctgca caaccactac acgcagaaga gcctctccct gtctccgggt    1320
   aaatga                                                               1326

## Description

### FIELD

The present disclosure generally relates to methods for producing heterologous proteins in transformed cells. In particular, the present disclosure provides improved methods of producing multi-subunit proteins, including antibodies and other multi-subunit proteins, which may or may not be secreted, with decreased production of undesired side-products and/or increased yield. In exemplary embodiments, the transformed cells are a yeast, such as *Pichia pastoris* or *Saccharomyces cerevisiae.*

### BACKGROUND

Conventional antibodies are tetrameric proteins composed of two identical light chains and two identical heavy chains. Pure human antibodies of a specific type can be difficult or impossible to purify from natural sources in sufficient amounts for many purposes. As a consequence, biotechnology and pharmaceutical companies have turned to recombinant DNA-based methods to prepare antibodies on a large scale. The production of functional antibodies generally involves not just the synthesis of the two polypeptides but also a number of post-translational events, including proteolytic processing of the N-terminal secretion signal sequence; proper folding and assembly of the polypeptides into tetramers; formation of disulfide bonds; and typically includes a specific N-linked glycosylation. All of these events take place in the eukaryotic cell secretory pathway, an organelle complex unique to eukaryotic cells.

Recombinant synthesis of such complex proteins has typically relied on cultures of higher eukaryotic cells to produce biologically active material, with cultured mammalian cells being very commonly used. However, mammalian tissue culture-based production systems incur significant added expense and complication relative to microbial fermentation methods. Additionally, products derived from mammalian cell culture may require additional safety testing to ensure freedom from mammalian pathogens (including viruses) that might be present in the cultured cells or animal-derived products used in culture, such as serum.

Prior work has help to establish the yeast *Pichia pastoris* as a cost-effective platform for producing functional antibodies that are potentially suitable for research, diagnostic, and therapeutic use. *See* co-owned U.S. Patents 7,935,340 and 7,927,863, each of which is incorporated by reference herein in its entirety. Methods are also known in the literature for design and optimization of *P. pastoris* fermentations for expression of recombinant proteins, including optimization of the cell density, broth volume, substrate feed rate, and the length of each phase of the reaction. *See* Zhang et al., "Rational Design and Optimization of Fed-Batch and Continuous Fermentations" in Cregg, J. M., Ed., 2007, Pichia Protocols (2nd edition), Methods in Molecular Biology, vol. 389, Humana Press, Totowa, N.J., pgs. 43-63.

Though recombinant multi-subunit proteins can be produced from cultured cells, undesired side-products may also be produced. For example, the cultured cells may produce the desired multi-subunit protein along with free monomers, complexes having incorrect stoichiometry, or proteins having undesired or aberrant glycosylation. Purification of the desired multi-subunit protein can increase production cost, and the steps involved in purification may decrease total yield of active complexes. Moreover, even after purification, undesired side-products may be present in amounts that cause concern. For example, glycosylated side-products may be present in amounts that increase the risk of an immune reaction after administration, while aberrant complexes or aggregates may decrease specific activity and may also be potentially immunogenic.

WO 00/56903 A2 describes improved methods for producing proteins in transformed Pichia. WO 02/48382 A2 describes a method for the production of a heterologous protein by a fungus. Tapani et al. (ACTA Radiologica (Stockholm, Sweden: 1987) Nov 1996, 37:6, 923-926) describes toxicity of ethanol in low concentrations - experimental evaluation in cell culture. WO 2008/063302 A2 describes novel P. pastoris pastoris promoters, and the use thereof to direct expression of proteins in yeast, preferably using a haploid mating strategy. Muhlbauer et al. (Alcohol, vol. 24, no. 3, 1 July 2001, pages 179-187) describes impaired immunoglobulin M production by incubation of hybridoma cells with ethanol. Baumann et al. (Biotechnology and Bioengineering, 100:1, 177-183, 1 May 2008) describes that hypoxic fed-batch cultivation of Pichia pastoris increases specific and volumetric productivity of recombinant proteins. David et al. (The Journal of Biological Chemistry, 25 June 1983, pages 7702-7706) describes studies on the effect of ethanol on eukaryotic protein synthesis in vitro. Van de Laar et al. (Biotechnology and Bioengineering, 96:3, 483-494, 15 February 2007) describes increased heterologous protein production by Saccharomyces cerevisiae growing on ethanol as sole carbon source.

Further, WO 2012/075340 A2, citeable under Article 54(3) EPC, discloses anti-NGF compositions and use thereof.

### SUMMARY

Most IgG1 antibody molecules are stabilized by a total of 16 intra-chain and inter-chain disulfide bridges. The intra-chain disulfide bridges stabilize the folding of the IgG domains in both heavy and light chains, while the inter-chain disulfide bridges stabilize the association between heavy and light chains. As a result of these bonds, antibodies form a stable complex containing two heavy chains and two light chains (H2L2). However, due to improper disulfide bond formation, product-associated variants are sometimes found in recombinant antibody preparations, including a complex having one light and one heavy chain (H1L1) and a complex having two heavy chains and one light chain (H2L1). Further, higher order complexes may also form in which additional inter-chain disulfide bonds form, resulting in a greater number of covalently linked subunits.

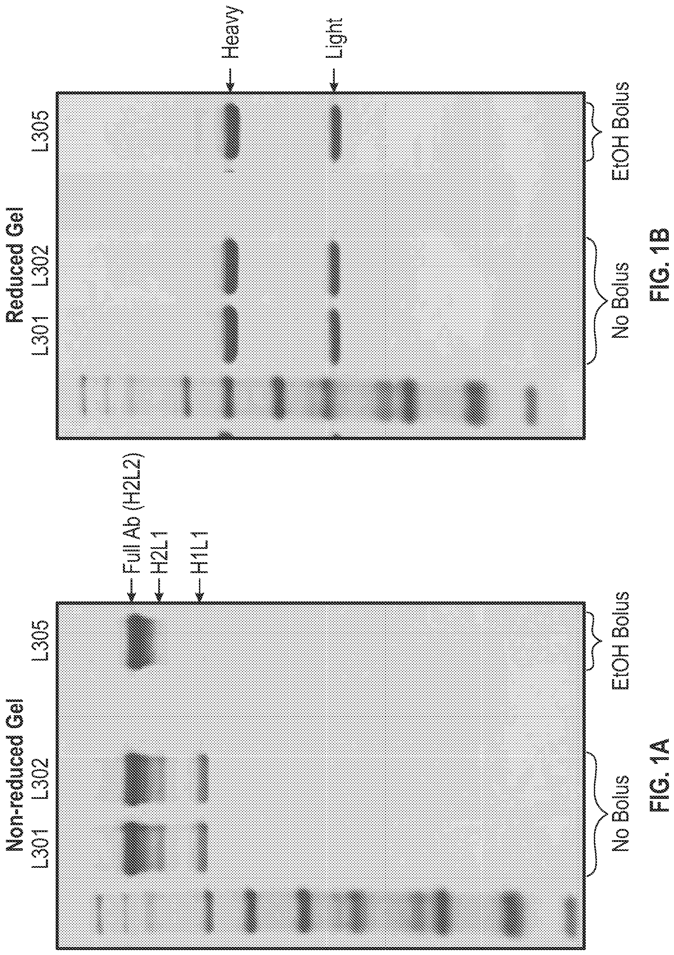

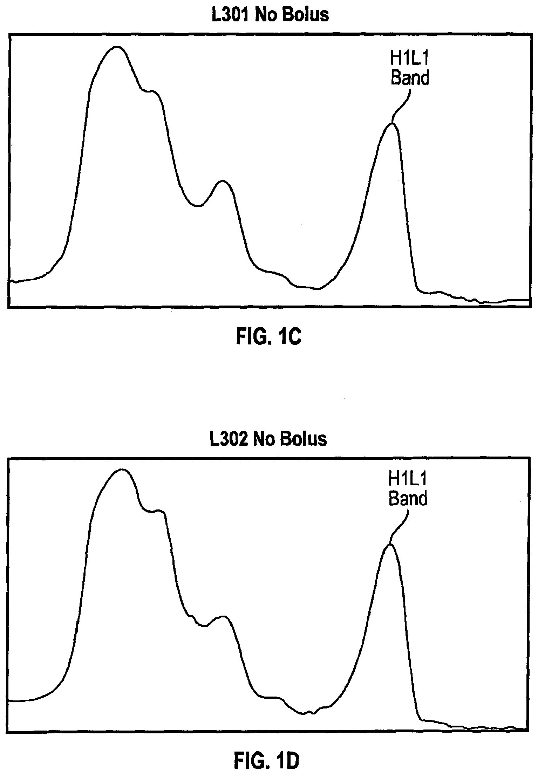

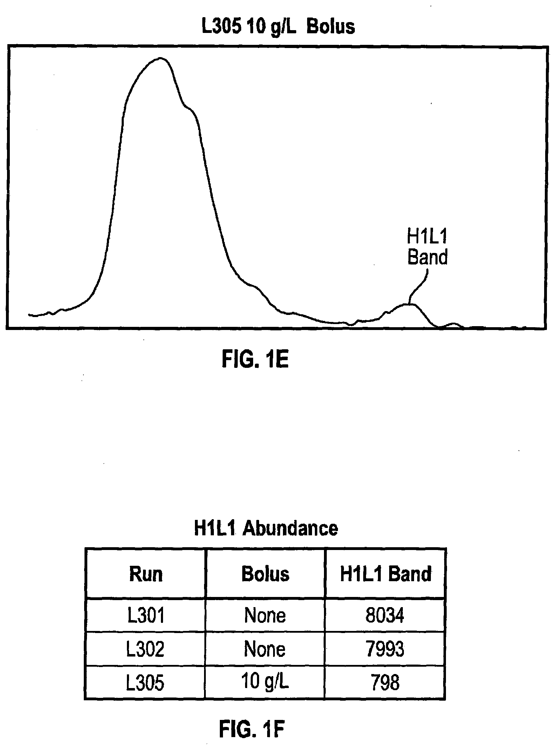

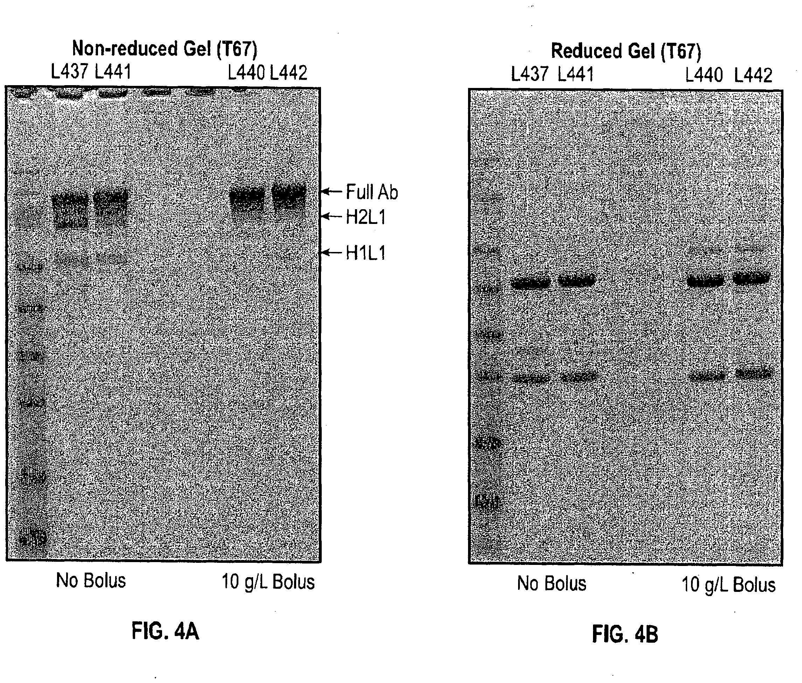

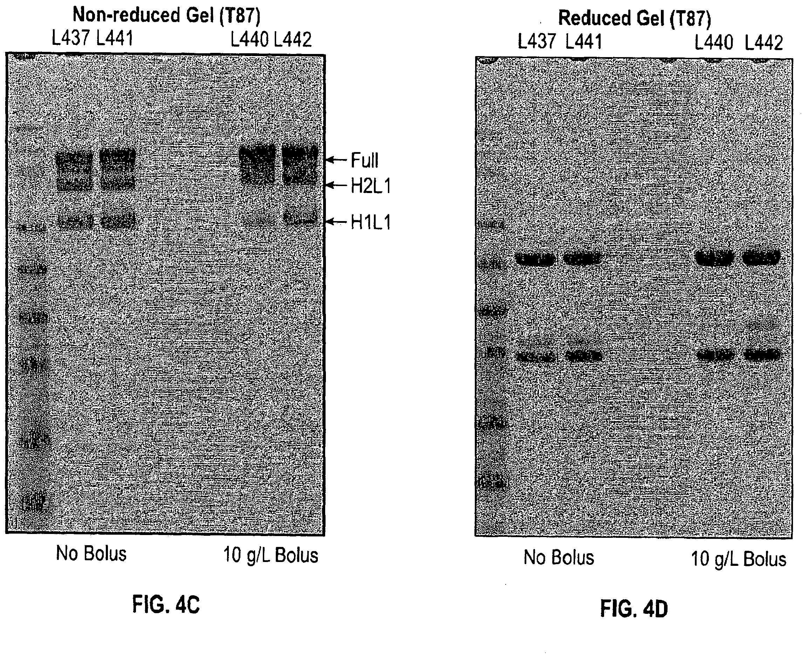

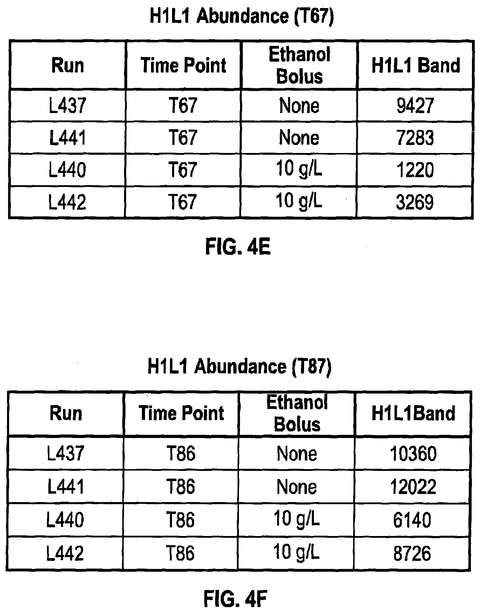

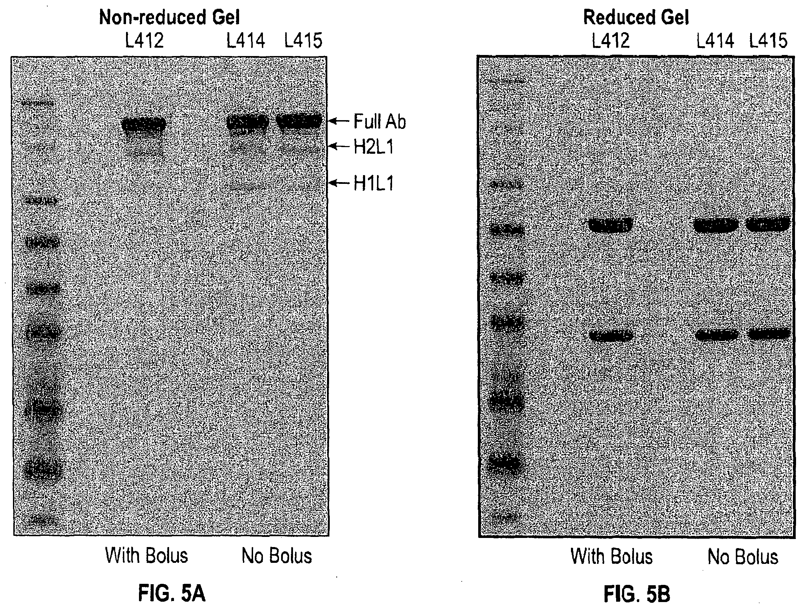

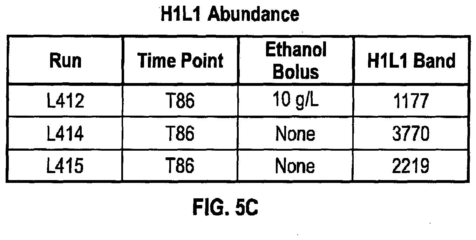

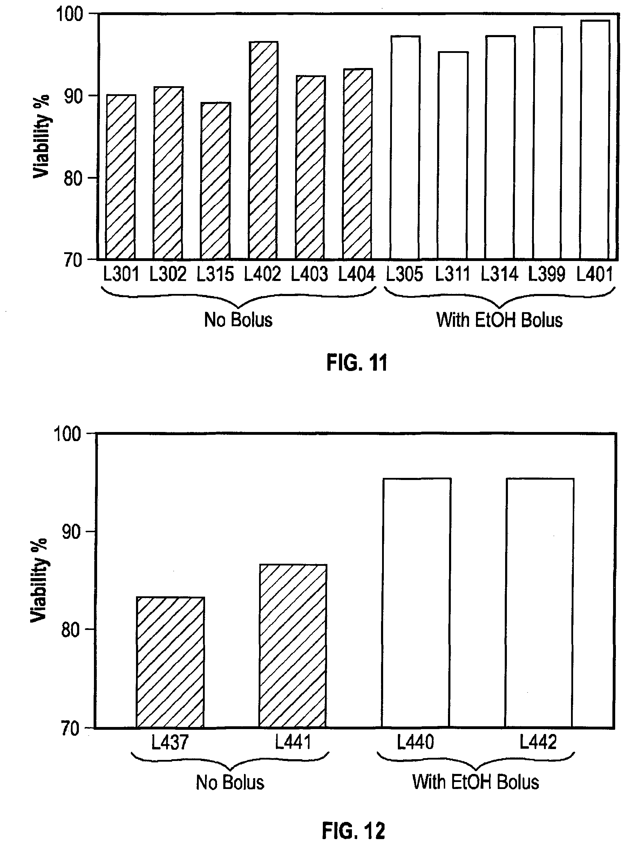

As further described below, Applicants have now identified methods of decreasing the production of these complexes containing aberrant disulfide bonds during recombinant production of antibodies from yeast culture. Specifically, the method involves addition of an ethanol bolus to the culture, and resulted in decreased production of the H1L1, H2L1, and H4L4 products-associated variants, and increased purity of the desired H2L2 product. The H1L1 and H2L1 complexes were detected by non-reduced, denaturing SDS-PAGE, and the H4L4 complexes were detected by size exclusion chromatography. Using the subject methods, proper disulfide bond formation was facilitated, resulting in increased antibody purity. This was demonstrated for three different antibodies, all three of which exhibited improved purity when produced with the addition of the ethanol bolus (FIGS. 1-6). These three antibodies are not only are different in sequence but also recognize three different antigens. Moreover, when produced in the absence of an ethanol bolus, two of the antibodies contained greater amounts of the H1L1 product (FIGS. 1-4), compared with the third antibody (FIG. 5). The two antibodies containing greater amounts of the H1L1 product have a non-canonical or additional disulfide bridge, whereas the third does not. The antibody exemplified in FIGS. 1, 2 and 3 has an additional intra-chain disulfide bridge in the variable light chain domain, while the antibody exemplified in FIG. 4, has an additional intra-chain disulfide bridge in its heavy chain. It has been reported in the literature that the presence of disulfide-bridges in overexpressed proteins increases intracellular stress in the host (see Gasser et al, Biotechnology and Bioengineering, Vol. 94, No. 2, pg. 353- 61, June 5, 2006; Inan et al, Biotechnology And Bioengineering, Vol. 93, No. 4, pg. 771-78, March 5, 2006; Li et al, Biochem Biophys Res Commun. 2010 November 19; 402(3): 519-524). This increased stress can also lead to lower viability, as is demonstrated in FIGS. 11, 12, and 13 where both antibodies with the extra intra-chain disulfide bridge have lower viability under the "no-bolus" conditions. The addition of the ethanol bolus, therefore leads to increased viability and increased purity. This may be of use in particular when difficult to express proteins with multiple disulfide bridges are being expressed.

In one aspect, the present invention provides a method of producing a multi-subunit complex, comprising (a) providing a culture comprising *Pichia pastoris* cells comprising genes that provide for the expression of the subunits of said multi-subunit complex; (b) adding a bolus of ethanol to said culture, which results in an ethanol concentration in said culture of between 0.5% and 1.5% (w/v); and (c) adding a feed comprising at least one fermentable carbon source to said culture, and culturing said culture to produce said multi-subunit complex, wherein said multi-subunit complex comprises an entire or full-length antibody comprising heavy and light chain polypeptides, wherein said multi-subunit complex is not an anti-NGF antibody containing the polypeptide sequences SEQ ID NO: 401 and SEQ ID NO: 402.

The ethanol bolus may enhance the formation of stable disulfide bonds relative to the same method effected in the absence of the bolus of ethanol.

The method may decrease the relative abundance of one or more product- associated variants relative to the same method effected in the absence of the bolus of ethanol.

The method may decrease the relative abundance of product-associated variants having a higher or lower apparent molecular weight than said desired multi-subunit complex as detected by size exclusion chromatography or gel electrophoresis relative to the same method effected in the absence of the bolus of ethanol.

The method may decrease the relative abundance of complexes having aberrant stoichiometry relative to the same method effected in the absence of the bolus of ethanol.

The method may decrease the relative abundance of complexes having aberrant disulfide bonds relative to the same method effected in the absence of the bolus of ethanol.

The method may decrease the relative abundance of complexes having reduced cysteines relative to the same method effected in the absence of the bolus of ethanol.

The method may decrease the relative abundance of complexes having aberrant glycosylation relative to the same method effected in the absence of the bolus of ethanol.

The method may modulate the formation or stability of inter-heavy chain disulfide bonds.

The method may modulate the formation or stability of disulfide bonds linking the light and heavy chains of said antibody.

The method may decrease the relative abundance of one or more product-associated variants relative to the same method effected in the absence of the bolus of ethanol.

Said product-associated variants may comprise one or more of the H1L1, H2L1, and H4L4 product-associate variants.

The method increase the purity of said antibody relative to said method effected in the absence of said bolus of ethanol.

Step (b) may result in a concentration of ethanol in said culture of between 0.7% and 1.5%, or between 0.8% and 1.25%.

Step (b) may result in a concentration of ethanol in said culture that may be 0.5%, 0.6%, 0.7%, 0.8% or 0.9% (w/v).

Step (b) may result in a concentration of ethanol in said culture that may be 1.5%, 1.4%, 1.3%, 1.2%, 1.1%, 1.0%, 0.9%, 0.8%, 0.7%, 0.6%, or 0.5% (w/v).

Step (b) may comprise adding ethanol to said culture, adding a carrier comprising ethanol to said culture, adding said cells to a medium or carrier comprising ethanol, or replacing part of the culture medium.

Said bolus of ethanol may be added to the culture medium over a period of time between 1 and 20 minutes.

Step (c) may comprise providing oxygen to said cells.

Said providing oxygen may comprise agitating said culture.

Said providing oxygen may comprise contacting said culture with a gas mixture comprising oxygen.

Step (c) may comprise adding a feed comprising a carbon source to said culture.

Said feed may comprise at least one fermentable carbon source.

Said feed may comprise one or more of glucose, ethanol, citrate, sorbitol, xylose, trehalose, arabinose, galactose, fructose, melil0.5%,lactose, maltose, rhamnose, ribose, mannose, mannitol, and raffinose.

The method may further comprise maintaining the concentration of ethanol between an upper set point and a lower set point during step (c).

Said lower set point may be 0.5%, 0.6%, 0.7%, 0.8% or 0.9% (w/v).

Said upper set point may be 1.5%, 1.4%, 1.3%, 1.2%, 1.1%, 1.0%, 0.9%, 0.8%, 0.7%, or 0.6% (w/v).

Said upper set point may be at most 1.5%, 1.4%, 1.3, 1.2%, or 1.1% (w/v).

The method may further comprise maintaining the concentration of ethanol at a set point during step (c).

Said set point may be 0.5%, 0.6%, 0.7%, 0.8%, 0.9%, 01.%, 01.1%, 01.2%, 01.3%, 01.4%, or 01.5% (w/v).

Step (c) may comprise maintaining the concentration of ethanol in said culture between 0.7% and 1.5%, or between 0.8% and 1.25%.

The concentration of ethanol in said culture may be maintained by controlling production of ethanol by said cells or by addition of ethanol to said culture.

The step of controlling production of ethanol may comprise controlling one or more of the concentration of glucose, availability of oxygen, intensity of agitation, gas pressure, flow rate of supplied air or other gas mixture, viscosity of the culture, culture density, concentration of oxygen in the supplied air or other gas mixture, and temperature.

The time between step (a) and step (b) may be less than about 72 hours, less than about 48 hours, less than about 24 hours, less than about 12 hours, less than about 9 hours, less than about 6 hours, less than about 5 hours, less than about 4 hours, less than about 3 hours, less than about 90 minutes, less than about 30 minutes, less than about 5 minutes, or less than about 1 minute.

The time between step (b) and step (c) may be less than about 10 hours, less than about 9 hours, less than about 8 hours, less than about 7 hours, less than about 6 hours, less than about 5 hours, less than about 4 hours, less than about 3 hours, less than about 2 hours, less than about 90 minutes, less than about 80 minutes, less than about 70 minutes, less than about 60 minutes, less than about 50 minutes, less than about 40 minutes, less than about 30 minutes, less than about 20 minutes, less than about 10 minutes, less than about 5 minutes, or less than about 1 minute.

The culture of step (a) may be produced by adding a carbon source to said culture, and culturing said culture until the carbon source may be depleted.

Said carbon source may comprise one or more of: glycerol, glucose, ethanol, citrate, sorbitol, xylose, trehalose, arabinose, galactose, fructose, melibiose, lactose, maltose, rhamnose, ribose, mannose, mannitol, and raffinose.

The depletion of the carbon source may be determined by detecting a decrease in the metabolic activity of said *Pichia pastoris* cells.

Said decrease in the metabolic activity of said *Pichia pastoris* cells may be identified. by detecting a decrease in the consumption of oxygen by said *Pichia pastoris* cells, by detecting an increase in pH in the culture, by detecting stabilization of the wet cell mass, or by detecting an increase in the concentration of ammonia in the culture.

Said decrease in the consumption of oxygen by said *Pichia pastoris* cells may be identified by detecting an increase in the concentration of dissolved oxygen in said culture.

The genes that provide for expression of said multi-subunit complex may be integrated into one or more genomic loci.

At least one of said genomic loci may be selected from the group consisting of the pGAP locus, 3' AOX TT locus; PpURA5; OCH1; AOX1; HIS4; GAP; pGAP; 3' AOX TT; ARG; and the HIS4 TT locus.

At least one of the genes encoding said subunits of the multi-subunit complex may be expressed under control of an inducible or constitutive promoter.

Said inducible promoter may be selected from the group consisting of the AOX1, CUP1, tetracycline inducible, thiamine inducible, and FLD1 promoters.

At least one of the genes encoding said subunits of the multi-subunit complex may be expressed under control of a promoter selected from the group consisting of: the CUP1, AOX1, ICL1, glyceraldehyde-3-phosphate dehydrogenase (GAP), FLD1, ADH1, alcohol dehydrogenase II, GAL4, PHO3, PHO5, and Pyk promoters, tetracycline inducible promoters, thiamine inducible promoters, chimeric promoters derived therefrom, yeast promoters, mammalian promoters, insect promoters, plant promoters, reptile promoters, amphibian promoters, viral promoters, and avian promoters.

Said *Pichia pastoris* cell may be diploid, tetraploid, or polyploid cells.

The method may further comprise purifying said multi-subunit complex from said *Pichia pastoris* cells or from the culture medium.

Said multi-subunit complex may be purified from an intracellular component, cytoplasm, nucleoplasm, or a membrane of said *Pichia pastoris* cells.

Said *Pichia pastoris* cells secrete said multi-subunit complex into the culture medium.

Said multi-subunit complex may be purified from said culture medium.

Said multi-subunit complex may comprise a monospecific or bispecific antibody.

Said multi-subunit complex may comprise a human antibody or a humanized antibody.

Said humanized antibody may be of mouse, rat, rabbit, goat, sheep, or cow origin.

Said humanized antibody may be of rabbit origin.

Said multi-subunit complex may comprise a monovalent, bivalent, or multivalent antibody.

Said antibody may be purified from said culture by protein A and/or protein G affinity.

At least one of the genes that provide for expression of a subunit of said multi-subunit complex in at least one of said *Pichia pastoris* cells in said panel may be optimized for expression in said eukaryotic cell.

Said multi-subunit complex may comprise an antibody and the purity of said antibody may be assessed by measuring the fraction of the antibody produced by said eukaryotic cell that may be contained in antibody complexes having the expected apparent hydrodynamic radius, may be contained in antibody complexes having the expected molecular weight, and / or specifically binds a target of said antibody.

Said multi-subunit complex comprises an antibody and the yield of said antibody may be assessed by determining the amount of antibody produced by said *Pichia pastoris* cell discounting any product-associated variants that may be abnormally glycosylated, contained in antibody complexes other than complexes having the expected apparent hydrodynamic radius, contained in antibody complexes having the expected molecular weight, and / or that fail to specifically bind to the target of said antibody.

The molecular weight of said antibody complexes may be determined by non-reducing SDS-PAGE.

Said multi-subunit complex comprises an antibody, said method may further comprise purifying said antibody.

Said culture cell may produce a supernatant antibody titer of at least 100 mg / L, at least 150 mg / L, at least 200 mg / L, at least 250 mg / L, at least 300 mg / L, between 100 and 300 mg / L, between 100 and 500 mg / L, between 100 and 1000 mg / L, at least 1000 mg / L, at least 1250 mg/liter, at least 1500 mg/liter, at least about 1750 mg/liter, at least about 2000 mg/liter, at least about 10000 mg/liter, or more.

One or more subunits of said multi-subunit complex may be expressed from more than one gene copy.

Said multi-subunit complex comprises an antibody which may be expressed from between 1-10 copies of a gene encoding the light chain of said antibody and from 1-10 copies of a gene encoding the heavy chain of said antibody.

The genes that provide for expression of said multi-subunit complex may be integrated into genome of said cells.

The genes that provide for expression of said multi-subunit complex may be contained on an extrachromosomal element, plasmid, or artificial chromosome.

Said cells may comprise more copies of the gene that provide for the expression of the light chain of said antibody than copies of the gene that provide for expression of the heavy chain of said antibody.

The respective number of copies of the gene encoding the heavy chain of said antibody and the number of copies of the gene encoding the light chain of said antibody in said cells may be: 2 and 2, 2 and 3, 3 and 3, 3 and 4, 3 and 5, 4 and 3, 4 and 4, 4 and 5, 4 and 6, 5 and 4, 5 and 5, 5 and 6, or 5 and 7.

The respective number of copies of the gene encoding the heavy chain of said antibody and the number of copies of the gene encoding the light chain of said antibody in said cells maybe: 2 and 1, 3 and 1, 4 and 1, 5 and 1, 6 and 1, 7 and 1, 8 and 1, 9 and 1, 10 and 1, 1 and 2,2 and 2, 3 and 2, 4 and 2, 5 and 2, 6 and 2, 7 and 2, 8 and 2, 9 and 2, 10 and 2, 1 and 3, 2 and 3, 3 and 3, 4 and 3, 5 and 3,6 and 3, 7 and 3, 8 and 3,9 and 3, 10 and 3, 1 and 4, 2 and 4, 3 and 4, 4 and 4, 5 and 4,6 and 4, 7 and 4, 8 and 4,9 and 4, 10 and 4, 1 and 5, 2 and 5, 3 and 5, 4 and 5, 5 and 5, 6 and 5, 7 and 5, 8 and 5, 9 and 5, 10 and 5, 1 and 6, 2 and 6, 3 and 6, 4 and 6, 5 and 6, 6 and 6, 7 and 6, 8 and 6, 9 and 6, 10 and 6, 1 and 7, 2 and 7,3 and 7,4 and 7, 5 and 7, 6 and 7, 7 and 7, 8 and 7, 9 and 7, 10 and 7, 1 and 8, 2 and 8, 3 and 8, 4 and 8, 5 and 8, 6 and 8, 7 and 8, 8 and 8, 9 and 8, 10 and 8, 1 and 9, 2 and 9, 3 and 9, 4 and 9, 5 and 9, 6 and 9, 7 and 9, 8 and 9, 9 and 9, 10 and 9, 1 and 10, 2 and 10, 3 and 10, 4 and 10, 5 and 10, 6 and 10, 7 and 10, 8 and 10, 9 and 10, 10 and 10.

The culture of step (c) may be grown in a production medium.

Said production medium may be a minimal medium.

Said minimal medium lacks selective agents.

Said minimal medium lacks pre-formed amino acids or other complex biomolecules.

The production medium may be a complex medium.

The complex medium may comprise one or more of yeast extract, soy peptones, and other plant peptones.

The culture of step (c) may be grown to a high cell density.

Said high cell density may be at least 50 g/L.

Said high cell density may be at least 100 g/L.

Said high cell density may be at least 300 g/L.

Said high cell density may be at least 400 g/L.

Said high cell density may be at least 500 g/L.

Said high cell density may be at least 750 g/L.

The *Pichia pastoris* cells may be cultured for at least 20 doublings and maintain high levels of expression of said multi-subunit complex after said at least 20 doublings.

The cells of step (c) may be cultured for at least 50 doublings and maintain high levels of expression of said multi-subunit complex after said at least 50 doublings.

The cells of step (c) may be cultured for at least 100 doublings and maintain high levels of expression of said multi-subunit complex after said at least 100 doublings.

At least one subunit of said multi-subunit complex may comprise a secretion signal.

Said multi-subunit complex may comprise an antibody.

The secretion signal may comprise one or more polypeptides selected from the group consisting of: SEQ ID NOS: 414 to 437 and any combination thereof.

Said multi-subunit complex may be not any of the antibodies disclosed in WO2012/075340 (PCT/US11/62963, filed December 1, 2011), US 2012/0148490 ( U.S. Ser. No. 13/309,295, filed December 1, 2011), US 2012/0141485 ( U.S. Ser. No. 13/309,153, filed December 1, 2011), US 2012/0164067 ( U.S. Ser. No. 13/308,665 filed on December 1,2011), and US 2012/0141484 ( U.S. Ser. No 13/308,831, filed December 1, 2011).

Said multi-subunit complex may not be Ab1-NGF, Ab2-NGF, Ab3-NGF, Ab4-NGF, Ab5-NGF, Ab6-NGF, Ab7-NGF, Ab8-NGF, Ab9-NGF, Ab10-NGF, Ab11-NGF, Ab12-NGF, Ab13-NGF, Ab14-NGF, Ab15-NGF, Ab16-NGF, Ab17-NGF, Ab18-NGF, Ab19-NGF, Ab20-NGF, and Ab21-NGF, or an Fab2 or Fab1 fragment thereof.

Said multi-subunit complex may not contain at least one, at least two, at least three, at least four, at least five, or at least all six of the complementarity determining regions (CDRs) contained in any of the following antibodies: Ab1-NGF, Ab2-NGF, Ab3-NGF, Ab4-NGF, Ab5-NGF, Ab6-NGF, Ab7-NGF, Ab8-NGF, Ab9-NGF, Ab10-NGF, Ab11-NGF, Ab12-NGF, Ab13-NGF, Ab14-NGF, Ab15-NGF, Ab16-NGF, Ab17-NGF, Ab18-NGF, Ab19-NGF, Ab20-NGF, or Ab21-NGF and optionally having binding specificity for NGF.

Said multi-subunit complex may not comprise or consist of the light and heavy chain polypeptide sequences of SEQ ID NOs: 51 and 401, respectively, SEQ ID NOs: 53 and 402, respectively, SEQ ID NOs: 405 and 406, respectively, and SEQ ID NOs: 407 and 408, respectively.

Said multi-subunit complex may not comprise an antibody containing at least one, at least two, at least three, at least four, at least five, or at least all six of the CDRs of SEQ ID NOs: 55, 56, 57, 58, 59, and 60, and optionally having binding specificity for NGF.

Said multi-subunit complex may not comprise any of the antibodies or antibody coding sequences disclosed herein in the sections entitled "Anti-NGF Antibodies and Binding Fragments Thereof Having Binding Activity for NGF" and "Polynucleotides Encoding Anti-NGF Antibody Polypeptides." The present disclosure further provides methods of producing a multi-subunit complex, which may comprise: culturing a host cell providing a culture comprising eukaryotic cells that expresses said multi-subunit complex, adding a bolus of ethanol to said culture, and culturing said culture to produce said multi-subunit complex. The multi-subunit complex may comprise one or more disulfide bonds, and may be an antibody.

According to the description, the ethanol bolus concentration (expressed as % w/v) may be between about 0.1% and about 5%, such as at least about 0.1%, at least about 0.2%, at least about 0.3%, at least about 0.4%, at least about 0.5%, at least about 0.6%, at least about 0.7%, at least about 0.8%, at least about 0.9%, at least about 1%, up to about 1%, up to about 1.1%, up to about 1.2%, up to about 1.3%, up to about 1.4%, up to about 1.5%, up to about 1.6%, up to about 1.7%, up to about 1.8%, up to about 1.9%, up to about 2%, up to about 3%, up to about 4%, or up to about 5%, such as between about 0.1% and about 1.9%, between about 0.2% and about 1.8%, between about 0.3% and about 1.7%, between about 0.4% and about 1.6%, between about 0.5% and about 1.5%, between about 0.6% and about 1.4%, between about 0.7% and about 1.3%, between about 0.8% and about 1.2%, or between about 0.9% and about 1.1%, such as about 0.1%, about 0.2%, about 0.3%, about 0.4%, about 0.5%, about 0.6%, about 0.7%, about 0.8%, about 0.9%, about 1%, about 1.1%, about 1.2%, about 1.3%, about 1.4%, about 1.5%, about 1.6%, about 1.7%, about 1.8%, about 1.9%, about 2%, about 2.5%, about 3%, about 4%, or about 5%.

The method may further comprise purification of said desired multi-subunit complex.

In some examples the ethanol concentration may be controlled subsequent to addition of the ethanol bolus, which may be used to maintain the ethanol concentration at a desired set point or within a desired set point range. The set point (expressed as % w/v) may be between about 0.1% and about 4%, at least about 0.01%, at least about 0.02%, at least about 0.04%, at least about 0.06%, at least about 0.08%, at least about 0.1%, at least about 0.15%, at least about 0.2%, at least about 0.25%, at least about 0.3%, at least about 0.35%, at least about 0.4%, at least about 0.45%, at least about 0.5%, at least about 0.6%, at least about 0.7%, at least about 0.8%, at least about 0.9%, at least about 1%, at least about 1.2%, at least about 1.4%, at least about 1.6%, at least about 1.8%, at least about 2%, up to about 4%, up to about 3.75%, up to about 3.5%, up to about 3.25%, up to about 3%, up to about 2.75%, up to about 2.5%, up to about 2.25%, up to about 2%, up to about 1.75%, up to about 1.5%, up to about 1.25%, up to about 1%, between about 0.01% and about 4%, between about 0.02% and about 3.75%, between about 0.04% and about 3.5%, between about 0.08% and about 3.25%, between about 0.1% and about 3%, between about 0.2% and about 2.75%, between about 0.3% and about 2.5%, between about 0.4% and about 2.25%, between about 0.5% and about 2%, between about 0.6% and about 1.75%, between about 0.7% and about 1.5%, or between about 0.8% and about 1.25%. For example, the set point may be the same as the bolus concentration or within plus or minus 1%, 2%, 5%, 10%, 15%, 20%, 25%, 30%, 35%, 40%,45%, 50%, 60%, 70%, 80%, 90% or 100% of the bolus concentration.

The ethanol concentration set point may be maintained by controlling ethanol production by yeast cells during fermentation. For example, the ethanol concentration may be increased by increasing the concentration of glucose (e.g., increasing the rate of glucose feed), decreasing the availability of oxygen, by decreasing the intensity of agitation (e.g., lowering the fermenter input power), decreasing the gas pressure in the fermenter, decreasing the flow rate of supplied air or other gas mixture, increasing the viscosity of the culture, or decreasing the concentration of oxygen in the supplied air or other gas mixture (e.g., if oxygen supplementation is being used). Ethanol production may also be increased by increasing the fermentation temperature. Likewise, the ethanol concentration may be decreased by decreasing the glucose concentration (e.g., decreasing the rate of glucose feed), decreased by increasing the availability of oxygen, by increasing the intensity of agitation (e.g., increasing the fermenter input power), increasing the gas pressure in the fermenter, increasing the flow rate of supplied air or other gas mixture, decreasing the viscosity of the culture, or increasing the concentration of oxygen in the supplied air or other gas mixture (e.g., if oxygen supplementation is being used). Ethanol production may also be decreased by decreasing the fermentation temperature.

Using the methods of the present disclosure, the relative abundance of undesired side-product(s) may be decreased by at least 10%, at least 20%, at least 30%, at least 40%, at least 50%, at least 60%, at least 70%, at least 80%, at least 90%, at least 95%, at least 96%, at least 97%, at least 98%, at least 99%, or down to undetectable levels compared to initial abundance levels, relative to conventional methods. Exemplary undesired side-products whose relative abundance may be so decreased may include one or more species having a different apparent molecular weight than the desired multi-subunit complex. For example, apparent molecular weight may be affected by differences in stoichiometry, folding, complex assembly, and/or glycosylation. For example, such undesired side products may be detected using size exclusion chromatography and/or gel electrophoresis, and may have a higher or lower apparent molecular weight than the desired multi-subunit complex. In some examples, the undesired side-products may be detected under reducing conditions. In other examples, the undesired side-products may be detected under non-reducing conditions.

In some examples, the present disclosure also provides improved methods and compositions of matter that provide for the recombinant production of antibodies and other multi-subunit complexes, with a higher yield. In some examples, the yield may be increased by at least 10%, at least 20%, at least 30%, at least 40%, at least 50%, at least 100%, or more (relative to conventional methods) using the methods disclosed herein.

In some examples, the host cell in which the multi-subunit proteins may be produced may be a yeast, for example in a *Pichia* species such as *P. pastoris* or another methylotrophic yeast, or in a *Saccharomyces* species such as *S. cerevisiae,* or another yeast such as a *Schizosaccharomyces* (e.g., *S. pombe).* Other examples of methylotrophic yeast which may be utilized in the present invention include *Pichia angusta* (also known in the art as *Hansenula polymorpha), Pichia guillermordii, Pichia methanolica, Pichia inositovera, Ogataea nitratoaversa, and Candida boidnii.*

The host cell may be a eukaryotic cell, such as a yeast cell, such as a methylotrophic yeast, such as a yeast of the genus *Pichia.* Exemplary methylotrophic yeasts of the genus *Pichia* include *Pichia pastoris, Pichia angusta, Pichia guillermordii, Pichia methanolica,* and *Pichia inositovera.* The host cell may be produced by mating, e.g., by mating two haploid yeast cells that each contain one or more copies of at least one gene encoding a subunit of the multi-subunit complex.

In a preferred example, the methylotrophic yeasts of the genus *Pichia* is *Pichia pastoris.* The host cell may be a diploid or tetraploid cell.

At least one of said genes encoding said subunits of the desired multi-subunit complex, such as said desired antibody light chain and / or heavy chain, may be expressed under control of an inducible or constitutive promoter, such as CUP1 (induced by the level of copper in the medium; see Koller et al., Yeast 2000; 16: 651-656.), tetracycline inducible promoters (see, e.g., Staib et al., Antimicrobial Agents And Chemotherapy, Jan. 2008, p. 146-156), thiamine inducible promoters, AOX1, ICL1, glyceraldehyde-3-phosphate dehydrogenase (GAP), FLD1, ADH1, alcohol dehydrogenase II, GAL4, PHO3, PHO5, and Pyk promoters, chimeric promoters derived therefrom, yeast promoters, mammalian promoters, insect promoters, plant promoters, reptile promoters, amphibian promoters, viral promoters, and avian promoters.

The host cell may secrete said desired multi-subunit complex into the culture medium. Alternatively or in addition, said desired multi-subunit complex may be retained in said host cell and may be isolated therefrom.

The desired multi-subunit complex may comprise an antibody, such as a monospecific or bispecific antibody. The antibody may be an antibody that specifically binds any antigen.

The desired multi-subunit complex may be an antibody other than any of the antibodies (e.g., an antibody other than any of the anti-NGF antibodies) disclosed in WO 2012/075340 (PCT/US11/62963, filed December 1, 2011), US 2012/0148490 (U.S. Ser. No. 13/309,295, filed December 1, 2011), US 2012/0141485 (U.S. Ser. No. 13/309,153, filed December 1, 2011), US 2012/0164067 (U.S. Ser. No. 13/308,665 filed on December 1, 2011), and US 2012/0141484 (U.S. Ser. No. 13/308,831, filed December 1, 2011). In an example, the desired multi-subunit complex may not be any of the following antibodies: Ab1-NGF, Ab2-NGF, Ab3-NGF, Ab4-NGF, Ab5-NGF, Ab6-NGF, Ab7-NGF, Ab8-NGF, Ab9-NGF, Ab10-NGF, Ab11-NGF, Ab12-NGF, Ab13-NGF, Ab14-NGF, Ab15-NGF, Ab16-NGF, Ab17-NGF, Ab18-NGF, Ab19-NGF, Ab20-NGF, and Ab21-NGF. In a further example, the desired multi-subunit complex may not be an Fab2 fragment of any of the following antibodies: Ab1-NGF, Ab2-NGF, Ab3-NGF, Ab4-NGF, Ab5-NGF, Ab6-NGF, Ab7-NGF, Ab8-NGF, Ab9-NGF, Ab10-NGF, Ab11-NGF, Ab12-NGF, Ab13-NGF, Ab14-NGF, Ab15-NGF, Ab16-NGF, Ab17-NGF, Ab18-NGF, Ab19-NGF,Ab20-NGF, and Ab21-NGF. In a further example, the desired multi-subunit complex may not be an Fab1 fragment of any of the following antibodies: Ab1-NGF, Ab2-NGF, Ab3-NGF, Ab4-NGF, Ab5-NGF, Ab6-NGF, Ab7-NGF, Ab8-NGF, Ab9-NGF, Ab10-NGF, Ab11-NGF, Ab12-NGF, Ab13-NGF, Ab14-NGF, Ab15-NGF, Ab16-NGF, Ab17-NGF, Ab18-NGF, Ab19-NGF,Ab20-NGF, and Ab21-NGF. In a further example, the desired multi-subunit complex may not comprise an antibody containing at least one, at least two, at least three, at least four, at least five, or at least all six of the complementarity determining regions (CDRs) contained in any of the following antibodies: Ab1-NGF, Ab2-NGF, Ab3-NGF, Ab4-NGF, Ab5-NGF, Ab6-NGF, Ab7-NGF, Ab8-NGF, Ab9-NGF, Ab10-NGF, Ab11-NGF, Ab12-NGF, Ab13-NGF, Ab14-NGF, Ab15-NGF, Ab16-NGF, Ab17-NGF, Ab18-NGF, Ab19-NGF,Ab20-NGF, and Ab21-NGF and optionally having binding specificity for NGF. For example, the desired multi-subunit complex may not comprise or may not consist of the light and heavy chain polypeptide sequences SEQ ID NOs: 51 and 401, respectively, and/or SEQ ID NOs: 53 and 402, respectively, and/or SEQ ID NOs: 405 and 406, respectively, and/or SEQ ID NOs: 407 and 408, respectively. As a further example, the desired multi-subunit complex may not comprise an antibody containing at least one, at least two, at least three, at least four, at least five, or at least all six of the CDRs of SEQ ID NOs: 55, 56, 57, 58, 59, and 60, and optionally having binding specificity for NGF.

The desired multi-subunit complex may comprise an antibody of any type. Exemplary antibody types include antibodies of any mammalian species, e.g., human, mouse, rat, rabbit, goat, sheep, cow, etc. Preferably, the antibody is a human antibody or a humanized antibody that may be of rabbit origin. The desired antibody may be a monovalent, bivalent, or multivalent antibody.

At least one of said genes that provide for expression of a subunit of the desired multi-subunit complex, such as the light chain and/or heavy chain of a desired antibody, in at least one of said host cells in said panel may be optimized for expression in said host cell (e.g., by selecting preferred codons and/or altering the percentage AT through codon selection).

The purity of said desired multi-subunit complex, such as a desired antibody, may be assessed by measuring the fraction of the desired multi-subunit complex produced by said host cell that is non-glycosylated, is contained in complexes having the expected apparent hydrodynamic radius and/or apparent molecular weight (e.g., measured by size exclusion chromatography), has the expected electrophoretic mobility (e.g., detected by gel electrophoresis, such as SDS-PAGE, and optionally Western blotting), and / or by measuring the specific activity of the multi-subunit complex (e.g., specific binding a target of a desired antibody).

The desired multi-subunit complex may be an antibody, and yield of said antibody may be assessed by determining the amount of desired antibody produced by said host cell discounting any product-associated variants that are glycosylated, contained in antibody complexes other than complexes having the expected apparent molecular weight or hydrodynamic radius, and / or that fail to specifically bind to the target of said desired antibody.

The subject methods may produce a supernatant antibody titer of at least 100 mg / L, at least 150 mg / L, at least 200 mg / L, at least 250 mg / L, at least 300 mg / L, between 100 and 300 mg / L, between 100 and 500 mg / L, between 100 and 1000 mg / L or in excess of 1000 mg/L e.g., as high as 1200 mg/L, as high as 10,000 mg / L, or higher.

In another aspect, the host cell that produces a desired multi-subunit complex may be a diploid or tetraploid cell of the genus *Pichia,* such as a *Pichia pastoris* cell. The genes that provide for expression of the subunits of said desired multi-subunit complex, such as the light chain and heavy chain of a desired antibody, may be integrated into genome of said host cell, and/or may be contained on an extrachromosomal element, plasmid, or artificial chromosome.

In another aspect, the host cell that produces a desired multi-subunit complex may be engineered to increase yield and/or purity. As described therein, yield and purity of an antibody or other multi-subunit complex can be greatly improved by altering the number of copies per cell of the genes encoding each subunit. For example, where the desired multi-subunit complex is an antibody, the host cell may comprise more copies of the gene that provide for the expression of the light chain than copies of the gene that provide for expression of the heavy chain. In exemplary embodiments, the host cell may comprise from 1-10 copies of a gene encoding the light chain and from 1-10 copies of a gene encoding the heavy chain. The respective number of copies of the gene encoding the heavy chain and the number of copies of the gene encoding the light chain in said host cell may be: 2 and 2, 2 and 3, 3 and 3, 3 and 4, 3 and 5, 4 and 3, 4 and 4, 4 and 5, 4 and 6, 5 and 4, 5 and 5, 5 and 6, or 5 and 7, respectively. Additional exemplary combinations of heavy and light chain gene copy numbers include any combination of up to ten copies of the heavy and/or light chain gene, such as H2xL1, H3xLl, H4xL1, H5xL1, H6xL1, H7xL1, H8xL1, H9xL1, H10xL1, H1xL2, H2xL2, H3xL2, H4xL2, H5xL2, H6xL2, H7xL2, H8xL2, H9xL2, H10xL2, H1xL3, H2xL3, H3xL3, H4xL3, H5xL3, H6xL3, H7xL3, H8xL3, H9xL3, H10xL3, H1xL4, H2xL4, H3xL4, H4xL4, H5xL4, H6xL4, H7xL4, H8xL4, H9xL4, H10xL4, H1xL5, H2xL5, H3xL5, H4xL5, H5xL5, H6xL5, H7xL5, H8xL5, H9xL5, H10xL5, H1xL6, H2xL6, H3xL6, H4xL6, H5xL6, H6xL6, H7xL6, H8xL6, H9xL6, H10xL6, H1xL7, H2xL7, H3xL7, H4xL7, H5xL7, H6xL7, H7xL7, H8xL7, H9xL7, H10xL7, H1xL8, H2xL8, H3xL8, H4xL8, H5xL8, H6xL8, H7xL8, H8xL8, H9xL8, H10xL8, H1xL9, H2xL9, H3xL9, H4xL9, H5xL9, H6xL9, H7xL9, H8xL9, H9xL9, H10xL9, H1xL10, H2xL10, H3xL10, H4xL10, H5xL10, H6xL10, H7xL10, H8xL10, H9xL10, H10xL10, where the number following the "H" identifies the number of copies of the heavy chain gene, and the number following the "L" identifies the number of copies of the light chain gene. For example, the specified number of heavy and light chain gene copies may be tandemly integrated into a single locus, or into multiple loci (any or all of which may contain more than one copy). Optionally, each genomic locus may contain no more than three or four tandemly integrated gene copies, thereby promoting copy number stability during propagation and/or antibody production.

Culturing most typically involves proving cells with an energy source, oxygen, and nutrients. Methods are also known in the literature for design and optimization of P. *pastoris* fermentations for expression of recombinant proteins, including optimization of the cell density, broth volume, substrate feed rate, and the length of each phase of the reaction. *See* Zhang et al., "Rational Design and Optimization of Fed-Batch and Continuous Fermentations" in Cregg, J. M., Ed., 2007, Pichia Protocols (2nd edition), Methods in Molecular Biology, vol. 389, Humana Press, Totowa, N.J., pgs. 43-63. The culture may be provided with a gas mixture comprising oxygen, such as air with or without oxygen supplementation. The yeast culture may be cultured in a culture medium which may be a minimal medium, may lack selective agents, and / or may lack pre-formed amino acids or other complex biomolecules. The culture medium may also be a complex medium (e.g., containing yeast extract and/or plant peptone(s)). The medium may include a nitrogen source (e.g., methylamine chloride, NH4SO4, yeast extract, soy peptone, other plant peptones, etc.). Exemplary minimal media include minimal dextrose medium (MD) (1.34% yeast nitrogen base (YNB) (w/o amino acids), 4 × 10⁻⁵% biotin, and 2% glucose.), buffered minimal glycerol complex medium (BMGY) (1% yeast extract, 2% peptone, 1% glycerol, 1.34% YNB (w/o amino acids), 4 x 10-5% biotin and 100 mM potassium phosphate (pH 6.0)). Media may include one or more salts (such as sodium chloride, calcium, magnesium, and phosphate), buffers (such as potassium phosphate, Tris, or HEPES), nucleosides (such as adenosine and thymidine), antibiotics (e.g., added to inhibit growth of contaminants and/or for maintenance of a selectable marker), trace elements, and glucose or another energy source. Any supplements and substitutions may also be included at appropriate concentrations that would be known to those skilled in the art.

The culture may be grown to a high cell density, such as at least 50 g/L, at least 100 g/L, at least 300 g/L, at least 400 g/L, at least 500 g/L, or at least 700 g/L. These culture densities are illustrative rather than limiting, and suitable culture densities may be readily determined by those of ordinary skill in the art.

The yeast cells may be cultured for at least 20 doublings and maintain high levels of expression of said antibody after said at least 20 doublings.

The yeast cells may be cultured for at least 50 doublings and maintain high levels of expression of said antibody after said at least 50 doublings.

The yeast cells may be cultured for at least 100 doublings and maintain high levels of expression of said antibody after said at least 100 doublings.

In another aspect, the present disclosure provides a culture medium containing a stable diploid *Pichia* yeast culture produced according to any of the foregoing methods, wherein the culture medium may comprise expression levels of said desired antibody which may be at least about 50 mg/liter, 100 mg/liter, 500 mg/liter, 750 mg/liter, 1000 mg/liter, 1250 mg/liter, 1500 mg/liter, 1750 mg/liter, 2000 mg/liter, or more. These yield values are illustrative rather than limiting. Optionally, yield may be optimized, for example using the methods and general approach described in Zhang et al. (2007), *supra.* For example, yield may be optimized by varying temperature, pH, media composition (e.g., carbon source, carbon source concentration, mixture of two or more carbon sources, nitrogen source and concentration, concentration of salts and nutrients including KH₂PO₄, K₂HPO₄, MgSO₄, potassium sulfate, sodium citrate, potassium sulfate, sodium citrate, trace metals such as cobalt chloride, cupric sulfate, sodium iodide, manganese sulfate, sodium molybdate, boric acid, zinc chloride, ferrous sulfate, vitamins such as biotin, inositol, thiamine, peptone, yeast extract, casamino acids, urea, ammonium phosphate or other ammonium ions, L-arginine-hydrochloride), time, culture density, oxygenation, and other factors that influence yield. For example, yield, expression, and/or purity of the desired multi-subunit complex may in some instances be improved by maintaining the temperature at a desired set point, e.g., a set point between about 15°C and about 30 °C, such as between about 17 °C and about 25 °C). Without intent to be limited by theory, it is hypothesized that controlling the temperature may assist intracellular trafficking through the folding and post-translational processing pathways, and/or may decrease the activity of cellular proteases. Likewise, yield, expression, and/or purity of the desired multi-subunit complex may in some instances be improved by maintaining the pH of the culture medium at a desired set point, e.g., a set point between pH 3 to pH 8, such as between pH 4 and pH 7.

In another aspect, the present disclosure provides a culture medium containing a stable diploid *Pichia pastoris* yeast culture produced according to any of the foregoing methods that expresses said desired antibody into a culture medium wherein the cell density of said diploid cells in said culture may be at least about 50 g/L, 100 g/L, 300 g/L, 400 g/L, 500 g/L, 700 g/L or more. These culture densities are illustrative rather than limiting, and suitable culture densities may be readily determined by those of ordinary skill in the art.

At least one subunit of said antibody or other multi-subunit protein may comprise a secretion signal, such as the S. chicken lysozyme (CLY) signal peptide; CLY-L8; S. cerevisiae invertase (SUC2) signal peptide; MF-alpha (Prepro); MF-alpha (Pre)-apv; MF-alpha (Pre)-apvSLEKR; MF-alpha (Prepro)-(EA)3; αF signal peptide; KILM1 signal peptide; repressible acid phosphatase (PHO1) signal peptide; A. niger GOX signal peptide; Schwanniomyces occidentalis glucoamylase gene (GAM1) signal peptide; human serum albumin (HSA) signal peptide without pro-sequence; human serum albumin (HSA) signal peptide with pro-sequence; ISN signal peptide; IFN signal peptide; HGH signal peptide; phytohaemagglutinin (PHA); Silkworm lysozyme; Human lysozyme (LYZ1); activin receptor type-1; activin type II receptor; P. pastoris immunoglobulin binding protein (PpBiP); human antibody 3D6 light chain leader; and any combination thereof.

The host cell may be produced by mating two haploid yeast cells that each contain one or more copies of a gene encoding one or more subunits of said antibody or other multi-subunit protein.

### BRIEF DESCRIPTION OF THE DRAWINGS

**FIG.** 1A-B. Purity of a recombinantly produced Ab-A was improved by a bolus addition of ethanol prior to the start of a glucose feed in yeast cultures from which the antibodies were produced. Antibodies were harvested after 97 hours of culture and purified by protein-A affinity, then purity was assessed by SDS-PAGE using a non-reduced gel **(****FIG.** 1A) to resolve the desired full antibody (arrow, "Full Ab (H2L2)") from undesired product-associated variants. Complexes having aberrant stoichiometry were identified based on their molecular weight, affinity for protein A, and additional studies further described below, as a "half antibody" species containing one heavy and one light chain (arrow, "H1L1") and a complex containing two heavy chains and one light chain ("H2L1"). The relative abundance of the H2L1 and H1L1 complexes was greatly decreased by the bolus addition of ethanol during antibody production. Compare **FIG.** 1A, lanes 2-3 (no bolus) to lane 5 (with bolus). **FIG.** 1B shows the same samples processed under reducing conditions, which separated each of the full antibody, H1L1, and H2L1 complexes into individual heavy and light chains, confirming that the H1L1 and H2L1 complexes are composed of full-length heavy and light chains. Lane order in **FIGS.** 1A-B: Lane 1: molecular weight marker; lanes 2 and 3: control samples prepared from fermentation cultures without a bolus addition of ethanol; lane 4: no sample; lane 5: sample prepared from fermentation cultures with a bolus addition of ethanol.
**FIGS.** 1C-E show the gel band density plotted along the length of the non-reduced gel **(****FIG.** 1A, lanes 2, 3, and 5, respectively); arrows identify the peaks corresponding to the H1L1 species. **FIG.** 1F tabulates the area contained in the H1L1 peaks shown in **FIGS.** 1C-E, demonstrating approximately 90% reduction in the relative abundance of the H1L1 complexes. H2L1 complex abundance was not quantified due to the incomplete resolution from the full antibody peaks.

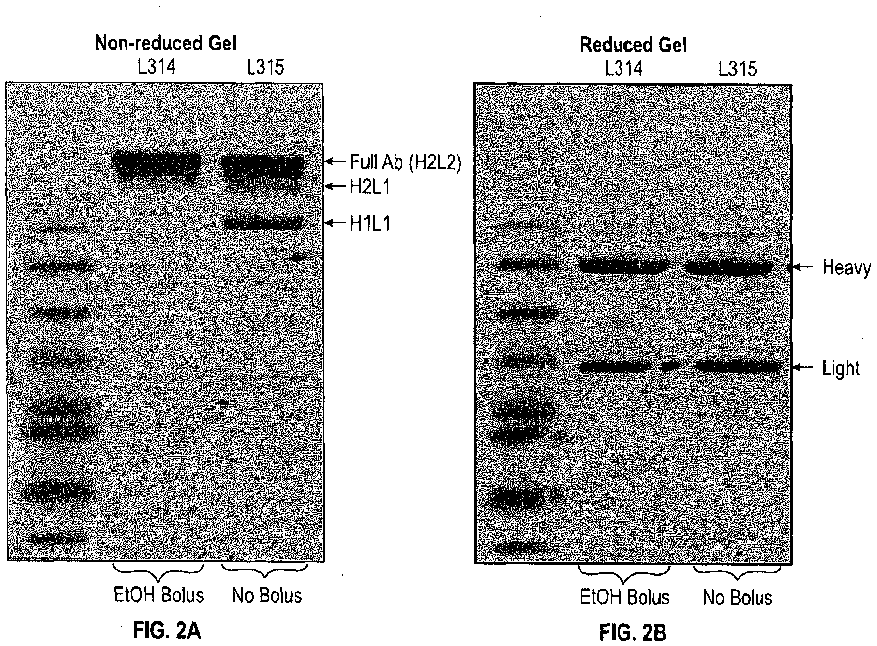

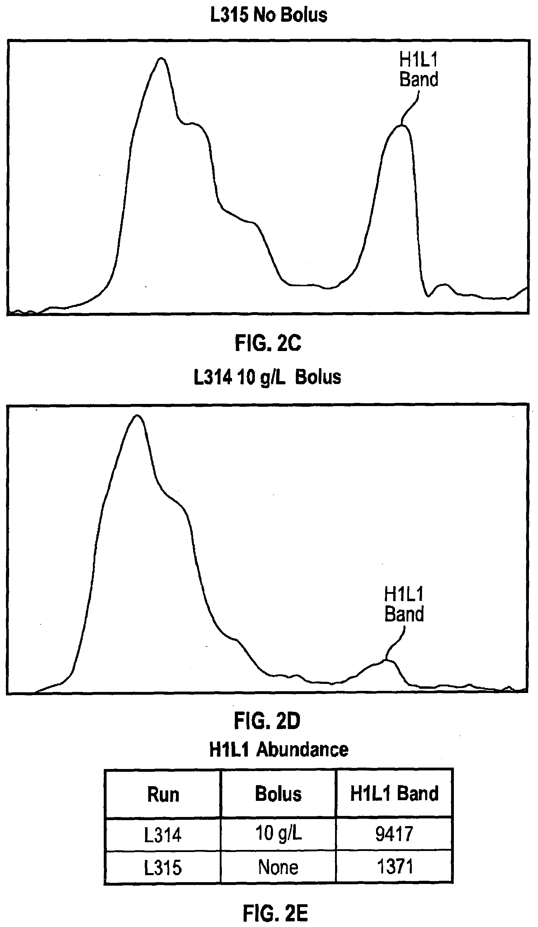

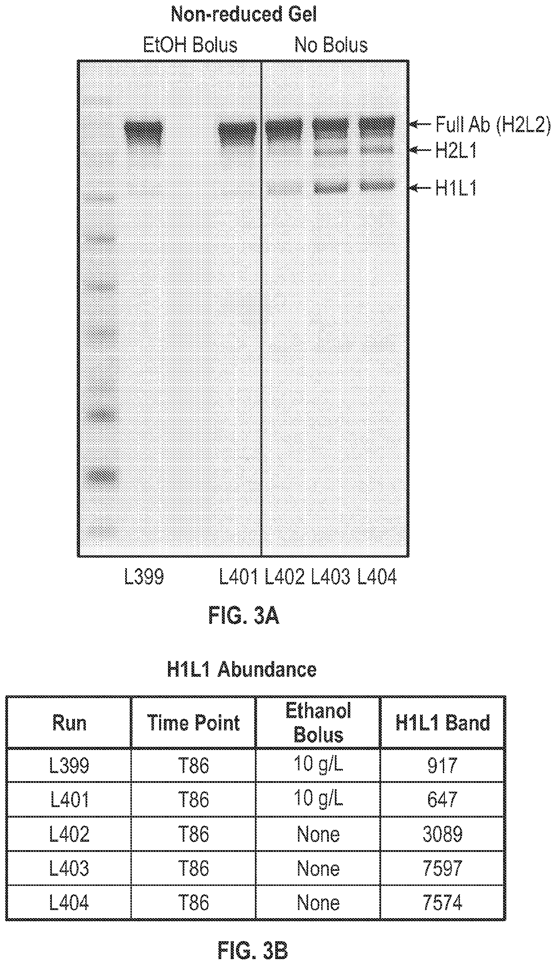

**FIGS.** 2A-B and 3A-B demonstrate reproducibility of the improvement in purity of Ab-A by a bolus addition of ethanol to the yeast cultures. Antibodies were harvested after 87 or 86 hours of culture (FIGs. 2 and 3, respectively) and purified by protein-A affinity, then purity was assessed by SDS-PAGE using a non-reduced gel. The abundance of the H1L1 and H2L1 complexes (arrows) were again decreased by the bolus addition of ethanol. Compare **FIG.** 2A, lane 3 (no bolus) to lane 2 (with bolus), and FIG. 3A, lanes 4-6 (no bolus) with lanes 2 and 4 (with bolus). **FIG.** 2B shows the same samples as in FIG. 2A processed under reducing conditions, again confirming that the observed product-associated variants are composed of full-length heavy and light chains. Lane order in **FIGS.** 2A-B: Lane 1: molecular weight marker; lane 2: sample prepared from a fermentation culture with a bolus addition of ethanol; lane 3: control sample prepared from a fermentation culture with no bolus addition of ethanol. Lane order in **FIG.** 3A: Lane 1: molecular weight marker; lanes 2 and 4: samples prepared from fermentation cultures with a bolus addition of ethanol; lane 3: no sample; lanes 5-7: control samples prepared from fermentation cultures with no bolus addition of ethanol.
**FIGS.** 2C and 2D show the gel band density plotted along the length of the non-reduced gel **(****FIG.** 2A, lanes 2, and 3, respectively); arrows identify the peaks corresponding to the H1L1 species. **FIGS.** 2E and 3B tabulate the area contained in the H1L1 peaks shown in **FIGS.** 2C and **FIG.** 3A, demonstrating approximately 85% reduction in the relative abundance of H1L1 complexes in **FIG.** 2A and approximately 87% average reduction in the relative abundance of H1L1 complexes in **FIG.** 3A.
**FIG.** 4A-D. Purity of a second recombinant antibody ("Ab-B") was also improved by a bolus addition of ethanol prior to the production phase of a fermentation process. Samples of fermentation culture broth were harvested after 67 hours ("T67) or 87 hours ("T87") of culture (FIGs. 4A-B and 4C-D, respectively) and antibodies were purified by protein-A affinity. Purity was then assessed by SDS-PAGE using non-reduced gels (FIGS. 4A and 4C). At both assessed time points, the abundance of the half-antibody species (H1L1) and the H2L1 complex was greatly decreased in fermentation cultures prepared that received a bolus addition of ethanol, relative to control cultures that did not receive a bolus addition of ethanol. Compare **FIG.** 4A, lanes 2-3 (no bolus) to lanes 6-7 (with bolus), and FIG. 4C, lanes 2-3 (no bolus) to lanes 6-7 (with bolus). **FIGs.** 4B and 4D shows the same samples processed under reducing conditions. Lane order in **FIGS.** 4A-D: Lane 1: molecular weight marker; lanes 2-3: control sample prepared from fermentation cultures with no bolus addition of ethanol; lanes 4-5: no sample; lanes 6-7: samples prepared from fermentation cultures with a bolus addition of ethanol.
**FIGS.** 4E and 4F tabulate the area contained in the H1L1 peaks shown in **FIGS.** 4A (T67) and 4C (T87), respectively, demonstrating that the bolus addition of ethanol produced about a 73% reduction in the relative abundance of H1L1 complexes at the earlier time point shown **FIG.** 4A and about a 34% average reduction in the relative abundance of H1L1 complexes at the later time point shown in **FIG.** 4C.
**FIG.** 5A-B. Purity of a third recombinant antibody (Ab-C) was also improved by a bolus addition of ethanol prior to the production phase of fermentation. Antibodies were harvested after 86 hours of culture and purified by protein-A affinity, then purity was assessed by SDS-PAGE using a non-reduced gel (FIGS. 5A). The H1L1 and H2L1 complexes were less abundant in the Ab-C product even without the addition of a bolus of ethanol, leaving less room for improvement. Nonetheless, the abundance of the half-antibody species (H1L1) and the H2L1 complex was noticeably decreased in fermentation cultures that received a bolus addition of ethanol, relative to control cultures that did not receive a bolus addition of ethanol. Compare **FIG.** 5A, lanes 5-6 (no bolus) to lane 3 (with bolus). **FIG.** 5B shows the same samples processed under reducing conditions. Lane order in **FIGS.** 5A-B: Lane 1: molecular weight marker, lane 2: no sample; lane 3: sample prepared from fermentation cultures that received a bolus addition of ethanol; lane 4: no sample; lanes 5-6: control sample prepared from fermentation cultures that did not receive a bolus addition of ethanol.
**FIG.** 5C tabulates the area contained in the H1L1 peaks shown in **FIGS. 5A****,** demonstrating about a 61% average reduction in the relative abundance of H1L1 complexes by the bolus addition of ethanol.

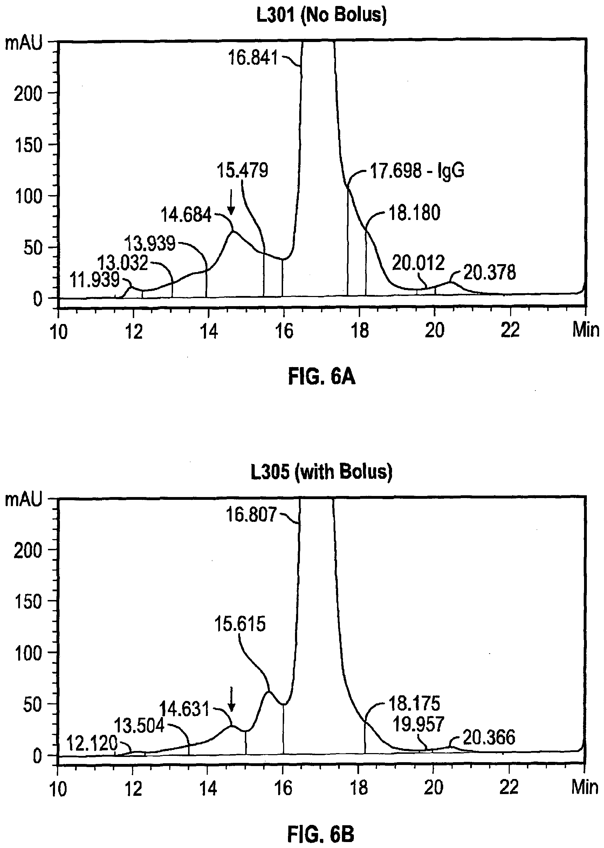

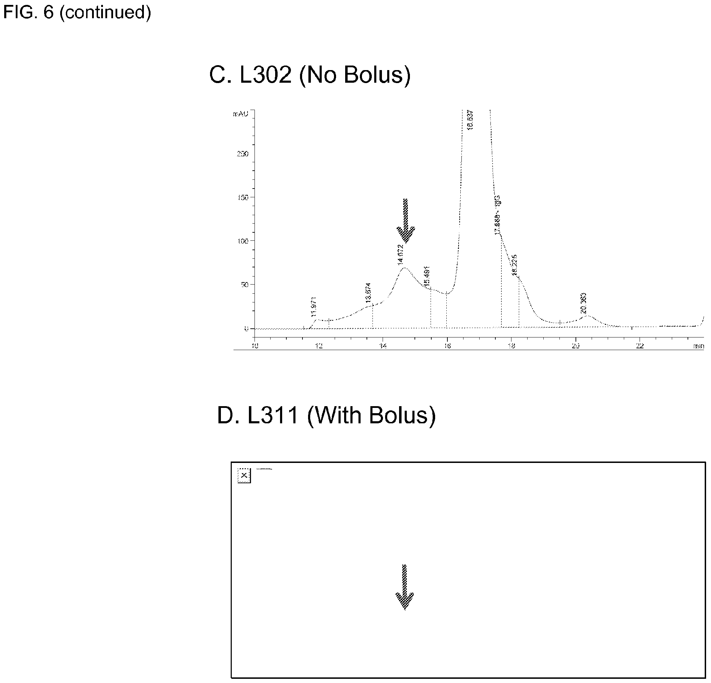

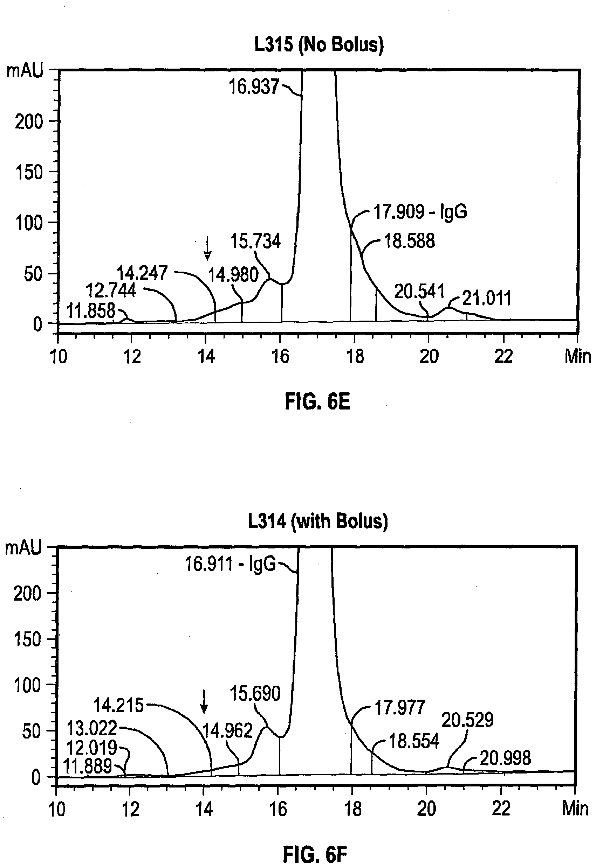

**FIG.** 6A-F shows assessment of the relative purity of the Ab-A preparations shown in **FIGS.** 1-3 by size exclusion chromatography. In each panel, the main peak contains the full antibody containing two heavy and two light chains (H2L2). The H1L1 species was not resolved from the main peak by this method (thought to be due H1L1 dimers forming by non-covalent association which is retained under the conditions used). However, other undesired product-associated variants were detected, including higher molecular weight species (left of main peak) and lower molecular weight species (right of main peak). A prominent peak though to correspond to antibody dimers containing two full antibodies (H4L4) was detected (arrow) and the relative abundance of these was decreased in samples prepared from fermentation cultures that received a bolus addition of ethanol. Compare FIGS. 6A, 6C, and 6E (no bolus) to FIGS. 6B, 6D, and 6F (with bolus).

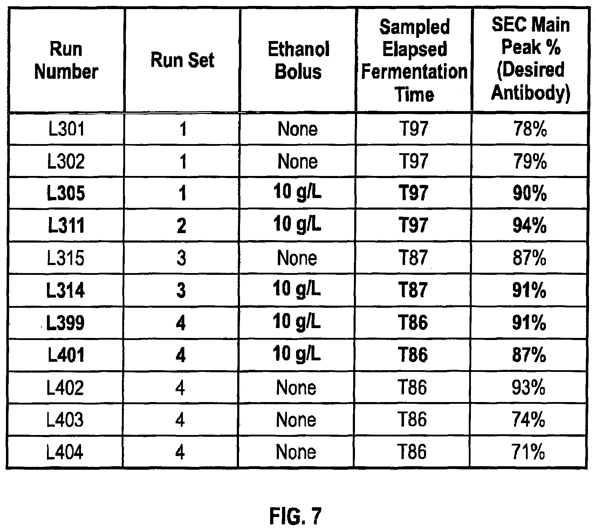

**FIG. 7** summarizes quantitation of the amount of product-associated variants detected by SEC for the six Ab-A samples shown in FIG. 6 and five additional samples. For each identified sample (col. 1), the run set number (col. 2, identifying fermentation runs that were conducted in parallel), bolus added (either 10g/L or none, col. 3), and elapsed culture time before culture samples were taken and processed (col. 4) are shown, together with the fraction of protein detected in the main peak ("SEC Main Peak %," col. 5). The bolus addition of ethanol at the end of the growth phase increased the average percentage contained in the main peak, from 80.3% up to 90.6%.

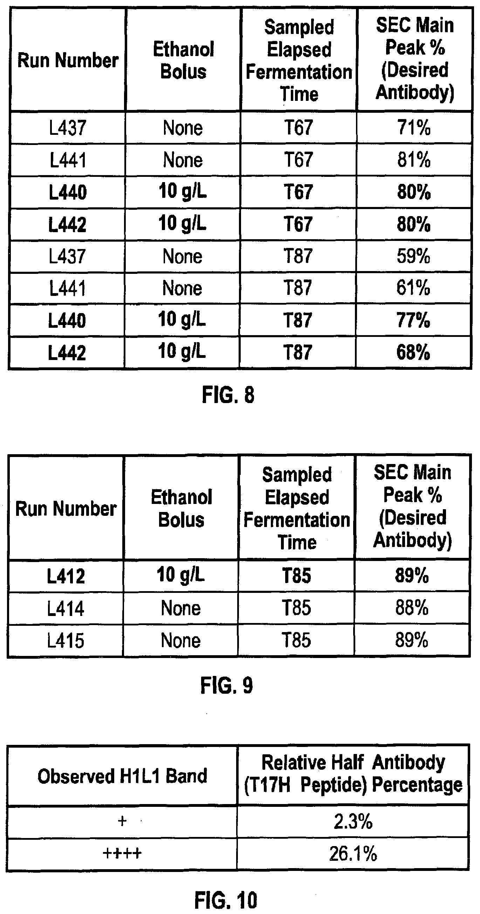

FIG. 8 summarizes quantitation of the amount of product-associated variants detected by SEC for Ab-B antibody samples shown in FIG. 4. For each fermentation run (col. 1), the bolus added at the end of the growth phase (either 10g/L or none, col. 2), and elapsed culture time before culture samples were taken and processed (col. 3) are shown, together with the fraction of protein detected in the main peak ("SEC Main Peak %," col. 4). Overall purity was increased, with the main peak increasing from 76% to 79% at T67 and from 60% to 73% at T87.
FIG. 9 summarizes quantitation of the amount of product-associated variants detected by SEC for the Ab-C antibody samples shown in FIG. 5. For each identified fermentation run (col. 1), the bolus added (either 10g/L or none, col. 2), and elapsed culture time before the sample was taken and processed (col. 3) are shown, together with the fraction of protein detected in the main peak ("SEC Main Peak %," col. 4). There was little difference in overall purity as detected by this method, with about 89% of product contained in main peak with or without the ethanol bolus. This was apparently due to the high initial purity of the Ab-C antibody even without the bolus addition. Additionally, SEC did not resolve the H1L1 species from the full antibody and accordingly the decreased production of this species due to the ethanol bolus was not reflected in SEC results.
FIG. 10 summarizes the results of mass spectrometry measurement of the quantity of a free heavy chain (lacking a disulfide bond to a second heavy chain) in Ab-A antibody samples containing high or low amounts of the H1L1 band. As expected, the amount of free heavy chain correlated with the amount of the H1L1 band, confirming the identity thereof as containing one heavy and one light chain and lacking a disulfide bond to a second heavy chain.

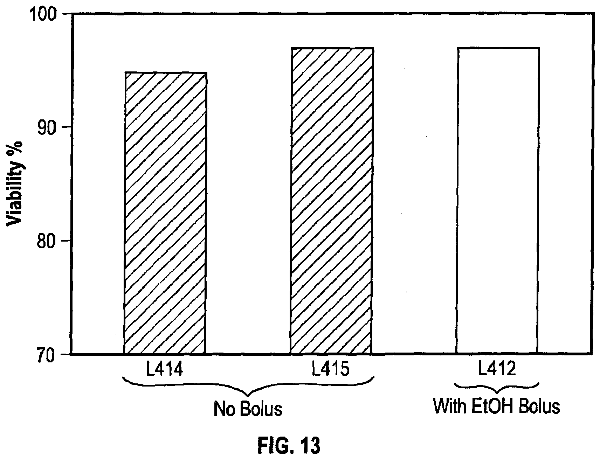

FIGS. 11-13 show the correlation between addition of an ethanol bolus and cell viability. The addition of an ethanol bolus generally improved cell viability and antibody purity for the Ab-A antibody (FIG. 11) and the Ab-B antibody (FIG. 12). These results suggest that the improvement in cell viability may account for at least part of the improvement in antibody purity from the ethanol bolus addition. Consistent with these results, the Ab-C antibody culture exhibited greater antibody purity and cell viability than the Ab-A and Ab-B cultures (FIG. 13). Apparently because the viability of the Ab-C antibody-producing cultures was already high in these experiments, there was little room for improvement and the cultures exhibited little improvement in viability from the bolus addition of ethanol. In FIGS. 11-13, filled bars indicate no bolus, while open bars indicate a bolus addition of ethanol. Viability was determined from fermentation cultures sampled with 1.5 hours of the time at which samples were collected for purity analyses (as identified in the preceding slides).

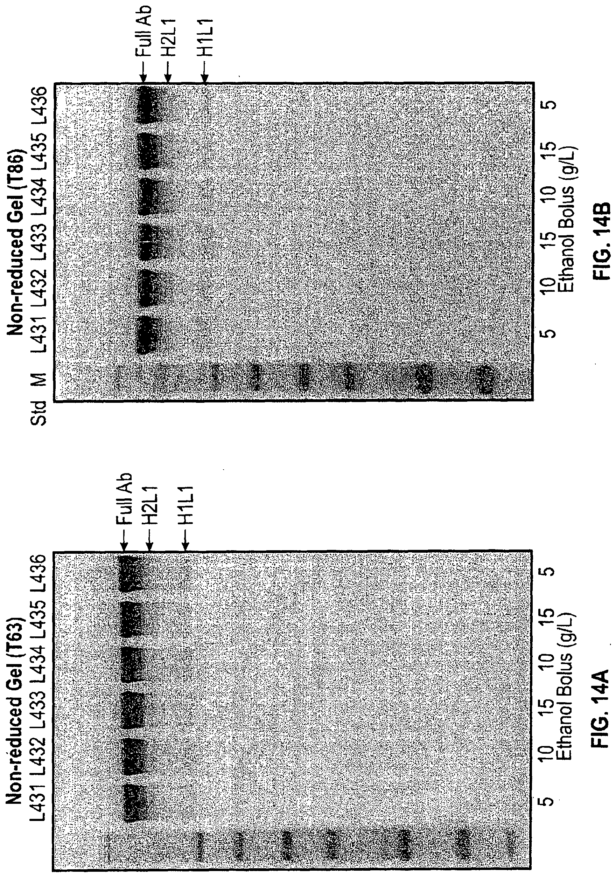

FIG. 14 shows that a wide range of ethanol bolus concentrations can produce the same improvement in antibody purity. Ab-A was produced with a bolus addition of ethanol between 5 g/L (0.5% w/v) and 15 g/L (1.5% w/v) and purified by protein A affinity, then the purity was analyzed by non-reduced SDS-PAGE. Each culture exhibited similarly low levels of the H2L1 and H1L1 complexes at 63 hours (FIG. 14A) and 86 hours (FIG. 14B). Lane order in FIGS. 14A-B: lane 1: molecular weight markers; lanes 2 and 7: 5 g/L bolus; lanes 3 and 5: 10 g/L bolus; lanes 4 and 6: 15 g/L bolus.

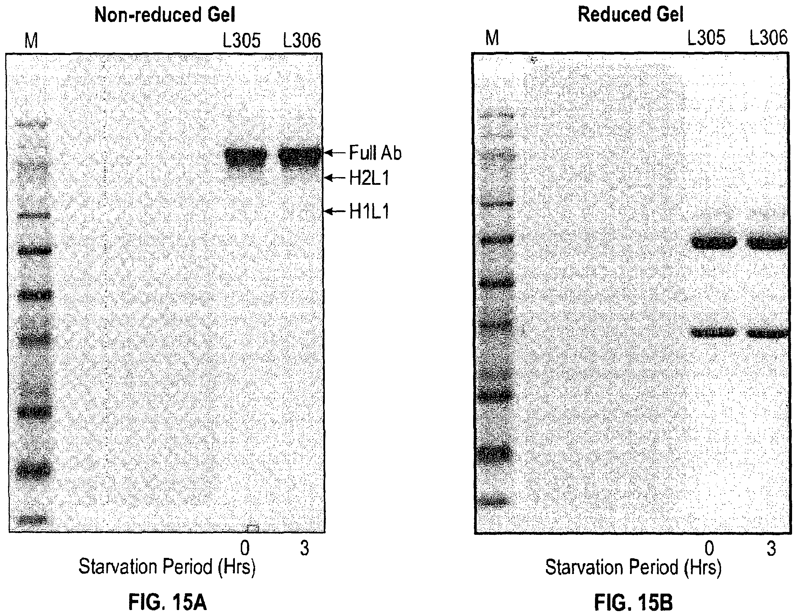

FIG. 15 shows that the time elapsed between the dissolved oxygen spike and the ethanol bolus addition can vary considerably while giving similar improvement in antibody purity. Ab-A was produced with a bolus addition of ethanol of 10 g/L (1% w/v) and purified by protein A affinity, then the purity was analyzed by non-reduced SDS-PAGE (FIG. 15A). The "starvation period," the time between the dissolved oxygen spike (indicating exhaustion of the carbon source in the culture) and the bolus addition of ethanol, was varied between 0 and 3 hours. Each culture exhibited similarly low levels of the H2L1 and H1L1 complexes irrespective of the duration of the starvation period, indicating that antibody purity is relatively insensitive to absence of a starvation period or a starvation period of at least up to three hours. The same samples were analyzed on a reduced gel (FIG. 15B). Lane order in FIGS. 15A-B: lane 1: molecular weight markers; lanes 2-4: no sample; lane 5: 0 hours starvation period; lane 6: 3 hours starvation period.

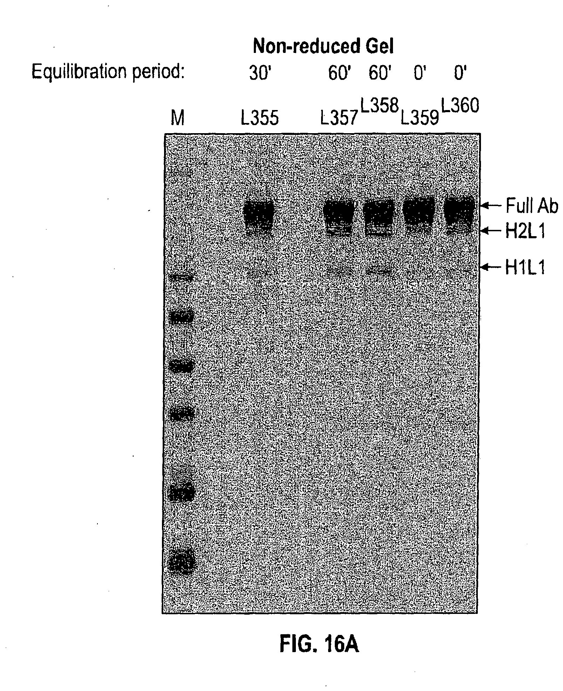

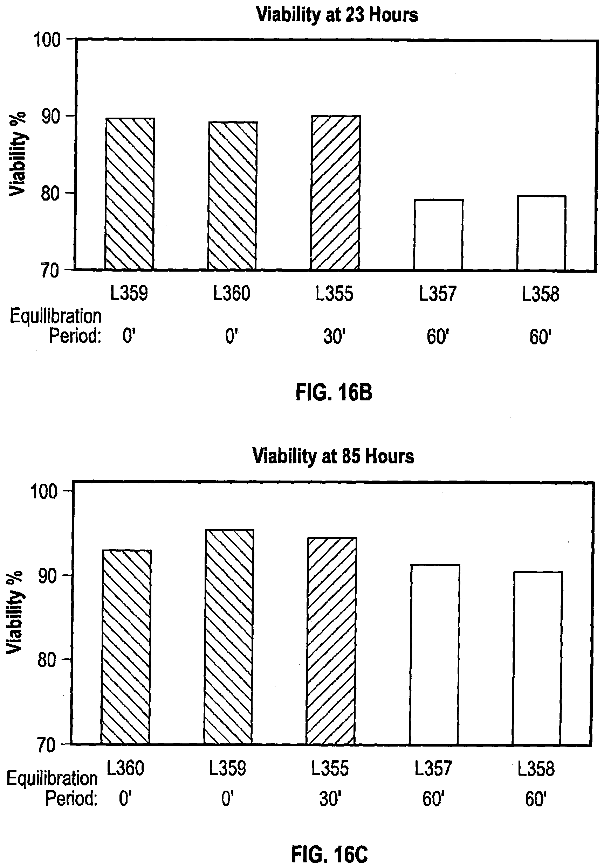

FIG. 16 shows the effect of the equilibration period (the time between ethanol bolus addition and feed start) on antibody purity. The Ab-B antibody was produced with a bolus addition of ethanol of 10 g/L (1% w/v) and purified by protein A affinity, then the purity was analyzed by non-reduced SDS-PAGE (FIG. 16A). The duration of the equilibration period was either 0, 30, or 60 minutes. The 60 minute equilibration period resulted in a lower antibody purity (higher abundance of the H2L1 and H1L1 complexes). Culture viability was also markedly lower with a 60 minute equilibration period, particularly earlier in the culture (at 23 hours, FIG. 16B); viability had improved somewhat by the end of the culture (at 85 hours, FIG. 16C). Lane order in FIG. 16A: lane 1: molecular weight markers; lanes 2 and 4: no sample; lane 3: 30 minutes equilibration; lanes 5 and 6: 60 minutes equilibration time; lanes 7 and 8: 0 minutes equilibration time. In FIGS. 16B-C, filled bars indicate an equilibration period of zero minutes, hatched bars indicate an equilibration period of 30 minutes and open bars indicate an equilibration period of 60 minutes.

### DETAILED DESCRIPTION

Applicants have unexpectedly discovered that purity of multi-subunit complexes expressed from yeast can be greatly improved by addition of a bolus of ethanol to the culture media. A single bolus addition of ethanol was demonstrated to improve purity over a sustained period of production, for up to at least 97 hours.

The present disclosure provides improved methods and compositions of matter that provide for the recombinant production of antibodies and other multi-subunit complexes with increased purity and decreased production of one or more undesired side-products. In some examples, relative to the desired multi-subunit complex the undesired side product(s) may exhibit one or more of: altered stoichiometry, aberrant glycosylation, differences in apparent molecular weight, differences in disulfide bonds, differences in hydrodynamic radius, fragments and/or truncated forms of one or more subunits. Undesired side-products may exhibit one or more additional differences as well. Undesired side-products may also be detected by their effects on a preparation, e.g., alteration in the level of specific activity, immunogenicity, or other effects on physical constitution and/or function of the desired multi-subunit complex.

For example, when the desired multi-subunit complex is an antibody, the undesired side products may include an H1L1 or "half antibody" species (i.e., containing a heavy chain and a light chain, wherein the heavy chain is not linked by a disulfide bond to another heavy chain), and/or a H2L1 species (i.e., containing two heavy chains and one light chain, but lacking a second light chain).

Though not intending to be limited by theory, it is hypothesized that a rapid increase in ethanol concentration (which can be brought about by a bolus addition of ethanol) can cause sustained changes in gene expression which confer a lasting improvement in the production of properly folded and assembled multi-subunit complexes and/or increases processing of improperly folded or misassembled multi-subunit complexes, leading to improved purity in the multi-subunit complex. Additionally, it was demonstrated that the improved antibody purity correlated with improved viability of the yeast in the culture, and based thereon Applicants hypothesize that the improved viability may account (at least in part) for the improved purity, though this theory is not intended to be limiting.

In a preferred example, the heterologous multi-subunit complex is an antibody or antibody fragment, such as a humanized antibody, comprised of two heavy chain subunits and two light chain subunits. Preferred host cells include yeasts, and particularly preferred yeasts include methylotrophic yeast strains, *e.g., Pichia pastoris, Hansenula polymorpha* (*Pichia angusta*)*, Pichia guillermordii, Pichia methanolica, Pichia inositovera,* and others *(see, e.g.,* U.S. Patent 4,812,405, 4,818,700, 4,929,555, 5,736,383, 5,955,349, 5,888,768, and 6,258,559). The host cell may be produced by methods known in the art, such as transformation, mating, sporulation, etc.

In a preferred example, the host cell may comprise more than one copy of one or more of the genes encoding the heterologous protein subunits. For example, multiple copies of a subunit gene may be integrated in tandem into one or more chromosomal loci. Tandemly integrated gene copies are preferably retained in a stable number of copies during culture for the production of the multi-subunit complex. For example, in the examples described below, gene copy numbers were generally stable for *P. pastoris* strains containing three to four tandemly integrated copies of light and heavy chain antibody genes.

One or more of the genes encoding the heterologous protein subunits are preferably integrated into one or more chromosomal loci of a host cell. Any suitable chromosomal locus may be utilized for integration, including intergenic sequences, promoters sequences, coding sequences, termination sequences, regulatory sequences, etc. Exemplary chromosomal loci that may be used in *P. pastoris* include PpURA5; *OCH1*; *AOX1*; *HIS4*; and *GAP.* The encoding genes may also be integrated into one or more random chromosomal loci rather than being targeted. In preferred embodiments, the chromosomal loci are selected from the group consisting of the pGAP locus, 3' AOX TT, and the HIS4 TT locus. In additional exemplary embodiments, the genes encoding the heterologous protein subunits may be contained in one or more extrachromosomal elements, for example one or more plasmids or artificial chromosomes.

In some examples, the multi-subunit protein may comprise two, three, four, five, six, or more non-identical subunits. Additionally, each subunit may be present one or more times in each multi-subunit protein. For example, the multi-subunit protein may be a multi-specific antibody such as a bi-specific antibody comprising two non-identical light chains and two non-identical heavy chains.

The subunits may be expressed from monocistronic genes, polycistronic genes, or any combination thereof. Each polycistronic gene may comprise multiple copies of the same subunit, or may comprise one or more copies of each different subunit.

Exemplary methods that may be used for manipulation of *Pichia pastoris* (including methods of culturing, transforming, and mating) are disclosed in Published Applications including U.S. 20080003643, U.S. 20070298500, and U.S. 20060270045, and in Higgins, D. R., and Cregg, J. M., Eds. 1998. Pichia Protocols. Methods in Molecular Biology. Humana Press, Totowa, N.J., and Cregg, J. M., Ed., 2007, Pichia Protocols (2nd edition), Methods in Molecular Biology. Humana Press, Totowa, N.J.

An exemplary expression cassette that may be utilized is composed of the glyceraldehyde dehydrogenase gene (GAP gene) promoter, fused to sequences encoding a secretion signal, followed by the sequence of the gene to be expressed, followed by sequences encoding a *P. pastoris* transcriptional termination signal from the *P. pastoris* alcohol oxidase I gene (AOX1). The Zeocin resistance marker gene may provide a means of enrichment for strains that contain multiple integrated copies of an expression vector in a strain by selecting for transformants that are resistant to higher levels of Zeocin. Similarly, G418 or Kanamycin resistance marker genes may be used to provide a means of enrichment for strains that contain multiple integrated copies of an expression vector in a strain by selecting for transformants that are resistant to higher levels of Geneticin or Kanamycin.

Host strains that may be utilized include auxotrophic *P*. *pastoris* or other *Pichia* strains, for example, strains having mutations in met1, lys3, ura3 and ade1 or other auxotrophy-associated genes. Preferred mutations are incapable of giving rise to revertants at any appreciable frequency and are preferably partial or even more preferably full deletion mutants. Preferably, prototrophic diploid or tetraploid strains are produced by mating a complementing sets of auxotrophic strains.

Transformation of haploid *P. pastoris* strains and genetic manipulation of the *P. pastoris* sexual cycle may be performed as described in Pichia Protocols (1998, 2007), *supra.*

Prior to transformation, each expression vector may be linearized by restriction enzyme cleavage within a region homologous to the target genomic locus (e.g., the GAP promoter sequence) to direct the integration of the vectors into the target locus in the host cell. Samples of each vector may then be individually transformed into cultures of the desired strains by electroporation or other methods, and successful transformants may be selected by means of a selectable marker, e.g., antibiotic resistance or complementation of an auxotrophy. Isolates may be picked, streaked for single colonies under selective conditions and then examined to confirm the number of copies of the gene encoding the subunit of the multi-subunit complex (e.g., a desired antibody) by Southern Blot or PCR assay on genomic DNA extracted from each strain. Optionally, expression of the expected subunit gene product may be confirmed, e.g., by FACS, Western Blot, colony lift and immunoblot, and other means known in the art. Optionally, haploid isolates are transformed additional times to introduce additional heterologous genes, e.g., additional copies of the same subunit integrated at a different locus, and / or copies of a different subunit. The haploid strains are then mated to generate diploid strains (or strains of higher ploidy) able to synthesize the multi-protein complex. Presence of each expected subunit gene may be confirmed by Southern blotting, PCR, and other detection means known in the art. Where the desired multi-protein complex is an antibody, its expression may also be confirmed by a colony lift/immunoblot method (Wung et al. Biotechniques 21 808-812 (1996) and / or by FACS.

This transformation protocol is optionally repeated to target a heterologous gene into a second locus, which may be the same gene or a different gene than was targeted into the first locus. When the construct to be integrated into the second locus encodes a protein that is the same as or highly similar to the sequence encoded by the first locus, its sequence may be varied to decrease the likelihood of undesired integration into the first locus. For example, the sequence to be integrated into the second locus may have differences in the promoter sequence, termination sequence, codon usage, and/or other tolerable sequence differences relative to the sequence integrated into the first locus.

To mate *P. pastoris* haploid strains, each strain to be crossed can be patched together onto mating plates. For example, multiple matings can be conveniently performed at the same time by streaking each strain to be mated across a plate suitable for its growth, and the mating partners may be streaked across a second plate (preferably the plates are rich media such as YPD). Typically, after one or two days incubation at 30° C., cells from the two plates can be replica plated in a crisscross fashion onto a mating plate, resulting in a cross-hatched pattern with each pair of strains being co-plated and having the opportunity to mate at the intersection of a pair of the original streak lines. The mating plate can then be incubated (e.g., at 30° C.) to stimulate the initiation of mating between strains. After about two days, the cells on the mating plates can be streaked, patched, or replica plated onto media selective for the desired diploid strains (e.g., where the mated strains have complementary autotrophies, drop-out or minimal medium plates may be used). These plates can be incubated (e.g., at 30° C.) for a suitable duration (e.g., about three days) to allow for the selective growth of the desired diploid strains. Colonies that arise can be picked and streaked for single colonies to isolate and purify each diploid strain.

Expression vectors for use in the methods of the invention may further include yeast specific sequences, including a selectable auxotrophic or drug marker for identifying transformed yeast strains. A drug marker may further be used to amplify copy number of the vector in a yeast host cell, e.g., by culturing a population of cells in an elevated concentration of the drug, thereby selecting transformants that express elevated levels of the resistance gene.

In an example, one or more of the genes encoding the heterologous protein subunits are coupled to an inducible promoter. Suitable exemplary promoters include the alcohol oxidase 1 gene promoter, formaldehyde dehydrogenase genes (FLD; see U.S. Pub. No. 2007/0298500), and other inducible promoters known in the art. The alcohol oxidase 1 gene promoter, is tightly repressed during growth of the yeast on most common carbon sources, such as glucose, glycerol, or ethanol, but is highly induced during growth on methanol (Tschopp et al., 1987; U.S. Pat. No. 4,855,231 to Stroman, D. W., et al). For production of foreign proteins, strains may be initially grown on a repressing carbon source to generate biomass and then shifted to methanol as the sole (or main) carbon and energy source to induce expression of the foreign gene. One advantage of this regulatory system is that *P*. *pastoris* strains transformed with foreign genes whose expression products are toxic to the cells can be maintained by growing under repressing conditions.

In another example, one or more of the heterologous genes may be coupled to a regulated promoter, whose expression level can be upregulated under appropriate conditions. Exemplary regulated promoters include the CUP1 promoter (induced by the level of copper in the medium), tetracycline inducible promoters, thiamine inducible promoters, the AOX1 promoter, and the FLD1 promoter.

Though much of the present disclosure describes production of antibodies, the methods described herein are readily adapted to other multi-subunit complexes as well. Without intent to be limited by theory, it is believed that the yield and purity of multi-subunit complexes can be greatly influenced by the concentration and stoichiometry of the subunits, which are in turn influenced by the level of expression of the genes responsible for production of each subunit. The methods disclosed herein may readily be utilized to improve the yield and / or purity of any recombinant multi-subunit complex comprising two or more different subunits. Additionally, the present methods are not limited to production of multi-protein complexes but may also be readily adapted for use with ribonucleoprotein (RNP) complexes including telomerase, hnRNPs, Ribosomes, snRNPs, signal recognition particles, prokaryotic and eukaryotic RNase P complexes, and any other complexes that contain multiple distinct protein and / or RNA subunits. The host cell that expresses the multi-subunit complex may be produced by methods known in the art. For example, a panel of diploid or tetraploid yeast cells containing differing combinations of gene copy numbers may be generated by mating cells containing varying numbers of copies of the individual subunit genes (which numbers of copies preferably are known in advance of mating).

### Definitions

It is to be understood that this invention is not limited to the particular methodology, protocols, cell lines, animal species or genera, and reagents described, as such may vary. It is also to be understood that the terminology used herein is for the purpose of describing particular embodiments only, and is not intended to limit the scope of the present invention which will be limited only by the appended claims.

As used herein the singular forms "a", "and", and "the" include plural referents unless the context clearly dictates otherwise. Thus, for example, reference to "a cell" includes a plurality of such cells and reference to "the protein" includes reference to one or more proteins and equivalents thereof known to those skilled in the art, and so forth. All technical and scientific terms used herein have the same meaning as commonly understood to one of ordinary skill in the art to which this invention belongs unless clearly indicated otherwise.

*Bolus addition:* In the present disclosure, "bolus addition" generally refers to rapid change in concentration of a substance (such as ethanol) in contact with cultured cells (for example, in a culture medium). For example, the substance may be added to the cultured cells in a single addition, a succession of more than one addition, and/or infused over a period of time (e.g., over about 1, 2, 3, 4, 5, 6, 7, 8, 9, 10, 15, 20, 25, 30, 40, 50, 60, 90, or 120 minutes). The substance may also be added by replacing the culture medium in part or in full, for example by concentrating the cells (using centrifugation, filtration, settling, or other methods), removing part or all of the medium, and adding the substance, or by adding the cells to a medium containing the substance. The substance may be admixed with a carrier (e.g., culture media, water, saline, etc.). For example, a bolus addition of ethanol may comprise the addition of pure or concentrated ethanol (e.g., 100%, 95%, 70%, 50%, 60%, 40%, 30%, 20%, etc.) to the culture medium in an amount sufficient to produce the desired concentration. As another example, the cells may be added to a medium containing ethanol, e.g., by adding an inoculum containing the cells to a medium containing ethanol.

*Bolus concentration:* In the present disclosure, "bolus concentration" generally refers to the concentration that results from a bolus addition of a substance (e.g., ethanol).

*Mating competent yeast species:* In the present disclosure this is intended to broadly encompass any diploid or tetraploid yeast which can be grown in culture. Such species of yeast may exist in a haploid, diploid, or other polyploid form. The cells of a given ploidy may, under appropriate conditions, proliferate for an indefinite number of generations in that form. Diploid cells can also sporulate to form haploid cells. Sequential mating can result in tetraploid strains through further mating or fusion of diploid strains. The present invention contemplates the use of haploid yeast, as well as diploid or other polyploid yeast cells produced, for example, by mating or fusion (e.g., spheroplast fusion).

In one example, the mating competent yeast is a member of the *Saccharomycetaceae* family, which includes the genera *Arxiozyma*; *Ascobotryozyma*; *Citeromyces*; *Debaryomyces*; *Dekkera*; *Eremothecium*; *Issatchenkia*; *Kazachstania*; *Kluyveromyces*; *Kodamaea*; *Lodderomyces*; *Pachysolen*; *Pichia*; *Saccharomyces*; *Saturnispora*; *Tetrapisispora*; *Torulaspora*; *Williopsis*; and *Zygosaccharomyces.* Other types of yeast potentially useful in the invention include *Yarrowia*; *Rhodosporidium*; *Candida*; *Hansenula*; *Filobasium*; *Sporidiobolus*; *Bullera*; *Leucosporidium* and *Filobasidella*.

In a preferred example, the mating competent yeast is a member of the genus *Pichia or is another methylotroph*. In a further preferred example, the mating competent yeast of the genus *Pichia* is one of the following species: *Pichiapastoris*, *Pichia methanolica,* and *Hansenula polymorpha* (*Pichia angusta*). According to the invention, the mating competent yeast of the genus *Pichia* is the species *Pichia pastoris.*

*Haploid Yeast Cell:* A cell having a single copy of each gene of its normal genomic (chromosomal) complement.

*Polyploid Yeast Cell*: A cell having more than one copy of its normal genomic (chromosomal) complement.

*Diploid Yeast Cell*: A cell having two copies (alleles) of essentially every gene of its normal genomic complement, typically formed by the process of fusion (mating) of two haploid cells.

*Tetraploid Yeast Cell*: A cell having four copies (alleles) of essentially every gene of its normal genomic complement, typically formed by the process of fusion (mating) of two diploid cells. Tetraploids may carry two, three, four, or more different expression cassettes. Such tetraploids might be obtained in *S*. *cerevisiae* by selective mating homozygotic heterothallic a/a and alpha/alpha diploids and in *Pichia* by sequential mating of haploids to obtain auxotrophic diploids. For example, a [met his] haploid can be mated with [ade his] haploid to obtain diploid [his]; and a [met arg] haploid can be mated with [ade arg] haploid to obtain diploid [arg]; then the diploid [his] can be mated with the diploid [arg] to obtain a tetraploid prototroph. It will be understood by those of skill in the art that reference to the benefits and uses of diploid cells may also apply to tetraploid cells.

*Yeast Mating:* The process by which two yeast cells fuse to form a single yeast cell. The fused cells may be haploid cells or cells of higher ploidy (e.g., mating two diploid cells to produce a tetraploid cell).

*Meiosis:* The process by which a diploid yeast cell undergoes reductive division to form four haploid spore products. Each spore may then germinate and form a haploid vegetatively growing cell line.

*Selectable Marker*: A selectable marker is a gene or gene fragment that confers a growth phenotype (physical growth characteristic) on a cell receiving that gene as, for example through a transformation event. The selectable marker allows that cell to survive and grow in a selective growth medium under conditions in which cells that do not receive that selectable marker gene cannot grow. Selectable marker genes generally fall into several types, including positive selectable marker genes such as a gene that confers on a cell resistance to an antibiotic or other drug, temperature when two temperature sensitive ("ts") mutants are crossed or a ts mutant is transformed; negative selectable marker genes such as a biosynthetic gene that confers on a cell the ability to grow in a medium without a specific nutrient needed by all cells that do not have that biosynthetic gene, or a mutagenized biosynthetic gene that confers on a cell inability to grow by cells that do not have the wild type gene; and the like. Suitable markers include but are not limited to: ZEO; NEO (G418); LYS3; MET1; MET3a; ADE1; ADE3; URA3; and the like.

*Integrated:* A genetic element (typically a heterologous genetic element) that are covalently joined into a chromosome of an organism.

*Tandemly integrated:* Two or more copies of a genetic element that are integrated in adjacent locations in a chromosome. The two or more copies do not necessarily have the orientation; e.g., for transcribed genes, some copies may be transcribed from the Watson strand and others from the Crick strand.

*Host cell:* In the context of the present disclosure, the term host cell refers to a cell (e.g., a eukaryotic cell, such as a *Pichia* cell) which contains a heterologous gene. For example, the heterologous gene may provide for the expression of a subunit of a desired multi-subunit complex, a gene involved in protein folding (e.g., a chaperone), expression, or secretion, and/or another desired gene. The heterologous gene may be integrated into the genome of the eukaryotic cell or contained in extrachromosomal element such as a plasmid or artificial chromosome.

*Expression Vector*: These DNA vectors contain elements that facilitate manipulation for the expression of a foreign protein within the target host cell. Conveniently, manipulation of sequences and production of DNA for transformation is first performed in a bacterial host, e.g. E. coli, and usually vectors will include sequences to facilitate such manipulations, including a bacterial origin of replication and appropriate bacterial selection marker. Selection markers encode proteins necessary for the survival or growth of transformed host cells grown in a selective culture medium. Host cells not transformed with the vector containing the selection gene will not survive in the culture medium. Typical selection genes encode proteins that (a) confer resistance to antibiotics or other toxins, (b) complement auxotrophic deficiencies, or (c) supply critical nutrients not available from complex media. Exemplary vectors and methods for transformation of yeast are described, for example, in Burke, D., Dawson, D., & Stearns, T. (2000). Methods in yeast genetics: a Cold Spring Harbor Laboratory course manual. Plainview, N.Y.: Cold Spring Harbor Laboratory Press.

Expression vectors for use in the methods of the invention may further include yeast specific sequences, including a selectable auxotrophic or drug marker for identifying transformed yeast strains. A drug marker may further be used to select for amplification of copy number of the vector in a yeast host cell.

The polypeptide coding sequence of interest is typically operably linked to transcriptional and translational regulatory sequences that provide for expression of the polypeptide in yeast cells. These vector components may include, but are not limited to, one or more of the following: an enhancer element, a promoter, and a transcription termination sequence. Sequences for the secretion of the polypeptide may also be included, e.g. a signal sequence, and the like. A yeast origin of replication is optional, as expression vectors are often integrated into the yeast genome.

Though optional, in one embodiment of the invention, one or more subunit of the multi-subunit complex is operably linked, or fused, to a secretion sequence that provides for secretion of the expressed polypeptide into the culture media, which can facilitate harvesting and purification of the heterologous multi-subunit complex. Even more preferably, the secretion sequences provide for optimized secretion of the polypeptide from the host cells (e.g., yeast diploid cells), such as through selecting preferred codons and/or altering the percentage AT through codon selection. It is known in the art that secretion efficiency and / or stability can be affected by the choice of secretion sequence and the optimal secretion sequence can vary between different proteins *(see, e.g.,* Koganesawa et al., Protein Eng. 2001 Sep;14(9):705-10. Many potentially suitable secretion signals are known in the art and can readily be tested for their effect upon yield and/or purity of a particular heterologous multi-subunit complex. Any secretion sequences may potentially be used, including those present in secreted proteins of yeasts and other species, as well as engineered secretion sequences. Exemplary secretion sequences that may be utilized include: chicken lysozyme (CLY) signal peptide (MRSLLILVLCFLPLAALG (SEQ ID NO:414)), CLY-L8 (MRLLLLLLLLPLAALG (SEQ ID NO:415)), *S*. *cerevisiae* invertase (SUC2) signal peptide (MLLQAFLFLLAGFAAKISA (SEQ ID NO:416)), MF-alpha (Prepro) (MRFPSIFTAVLFAASSALA-APVNTTTE-EGVSLEKR (SEQ ID NO:417)), MF-alpha (Pre)-apv (MRFPSIFTAVLFAASSALA-APV (SEQ ID NO:418)), MF-alpha (Pre)-apv-SLEKR (MRFPSIFTAVLFAASSALA-APVSLEKR (SEQ ID NO:419)), MF-alpha (Prepro)-(EA)3 (MRFPSIFTAVLFAASSALA-APVNTTTE-EGVSLEKR-EAEAEA (SEQ ID NO:420)), αF signal peptide (MRFPSIFTAVLFAASSALA-APVNTTTE-DETAQIPAEAVIGYSDLEGDFDVAVLPFSNSTNNGLLFINTTIASIAAKE-EGVSLEKR (SEQ ID NO:421)), KILM1 signal peptide (MTKPTQVLVRSVSILFFITLLHLVVALNDVAGPAETAPVSLLPR (SEQ ID NO:422)), repressible acid phosphatase (PHO1) signal peptide (MFSPILSLEIILALATLQSVFA (SEQ ID NO:423)), A. niger GOX signal peptide (MQTLLVSSLVVSLAAALPHYIR (SEQ ID NO:424)), *Schwanniomyces occidentalis* glucoamylase gene (GAM1) signal peptide (MIFLKLIKSIVIGLGLVSAIQA (SEQ ID NO:425)), human serum albumin (HSA) signal peptide with pro-sequence (MKWVTFISLLFLFSSAYSRGVFRR (SEQ ID NO:426)), human serum albumin (HSA) signal peptide without pro-sequence (MKWVTFISLLFLFSSAYS (SEQ ID NO:427)), ISN signal peptide (MALWMRLLPLLALLALWGPDPAAA (SEQ ID NO:428)), IFN signal peptide (MKYTSYILAFQLCIVLGSLGCDLP (SEQ ID NO:429)), HGH signal peptide (MAADSQTPWLLTFSLLCLLWPQEPGA (SEQ ID NO:430)), phytohaemagglutinin (PHA) (MKKNRMMMMIWSVGVVWMLLLVGGSYG (SEQ ID NO:431)), Silkworm lysozyme (MQKLIIFALVVLCVGSEA (SEQ ID NO:432)), Human lysozyme (LYZ1) (MKALIVLGLVLLSVTVQG (SEQ ID NO:433)), activin receptor type-1 (MVDGVMILPVLIMIALPSPS (SEQ ID NO:434)), activin type II receptor (MGAAAKLAFAVFLISCSSG (SEQ ID NO:435)), P. pastoris immunoglobulin binding protein (PpBiP) (MLSLKPSWLTLAALMYAMLLVVVPFAKPVRA (SEQ ID NO:436)), and human antibody 3D6 light chain leader (MDMRVPAQLLGLLLLWLPGAKC (SEQ ID NO:437)). See Hashimoto et al., Protein Engineering vol. 11 no. 2 pp.75-77, 1998; Oka et al., Biosci Biotechnol Biochem. 1999 Nov; 63(11):1977-83; Gellissen et al., FEMS Yeast Research 5 (2005) 1079-1096; Ma et al., Hepatology. 2005 Dec;42(6): 1355-63; Raemaekers et al., Eur J Biochem. 1999 Oct 1;265(1):394-403; Koganesawa et al., Protein Eng. (2001) 14 (9): 705-710; Daly et al., Protein Expr Purif. 2006 Apr;46(2):456-67 ; Damasceno et al., Appl Microbiol Biotechnol (2007) 74:381-389; and Felgenhauer et al., Nucleic Acids Res. 1990 Aug 25;18(16):4927. The multi-subunit complex may also be secreted into the culture media without being operably linked or fused to a secretion signal. For example, it has been demonstrated that some heterologous polypeptides are secreted into the culture media when expressed in *P. pastoris* even without being linked or fused to a secretion signal. Additionally, the multi-subunit complex may be purified from host cells (which, for example, may be preferable if the complex is poorly secreted) using methods known in the art.

Media or cells comprising a desired multi-subunit complex may be recovered from the culture. Optionally, the secreted proteins may be purified. For example, cells comprising a desired multi-subunit complex may be lysed using mechanical, chemical, enzymatic, and/or osmotic methods (e.g., freezing with liquid nitrogen, using a homogenizer, spheroplasting, sonication, agitation in the presence of glass beads, using detergents, etc.). The desired multi-subunit complex may be concentrated, filtered, dialyzed, etc., using methods known in the art. The desired multi-subunit complex may be purified based on, for example, its molecular mass (e.g., size exclusion chromatography), isoelectric point (e.g., isoelectric focusing), electrophoretic mobility (e.g., gel electrophoresis), hydrophobic interaction chromatography (e.g., HPLC), charge (e.g., ion exchange chromatography), affinity (e.g., in the case of an antibody, binding to protein A, protein G, and/or an epitope to which the desired antibody binds), and/or glycosylation state (e.g., detected by lectin binding affinity). Multiple purification steps may be performed to obtain the desired level of purity. In an exemplary embodiment, the desired multi-subunit complex may be comprise an immunoglobulin constant domain and may be purified using protein A or protein G affinity, size exclusion chromatography, and lack of binding to lectin (to remove glycosylated forms). Optionally the A protease inhibitor, such as phenyl methyl sulfonyl fluoride (PMSF) may be added to inhibit proteolytic degradation during purification.

Nucleic acids are "operably linked" when placed into a functional relationship with another nucleic acid sequence. For example, DNA for a signal sequence is operably linked to DNA for a polypeptide if it is expressed as a preprotein that participates in the secretion of the polypeptide; a promoter or enhancer is operably linked to a coding sequence if it affects the transcription of the sequence. Generally, "operably linked" means that the DNA sequences being linked are contiguous, and, in the case of a secretory leader, contiguous and in reading frame. However, enhancers do not have to be contiguous. Linking may be accomplished by ligation at convenient restriction sites or alternatively via a PCR/recombination method familiar to those skilled in the art (Gateway® Technology; Invitrogen, Carlsbad Calif.). If such sites do not exist, the synthetic oligonucleotide adapters or linkers may be used in accordance with conventional practice. Desired nucleic acids (including nucleic acids comprising operably linked sequences) may also be produced by chemical synthesis.

Promoters are untranslated sequences located upstream (5') to the start codon of a structural gene (generally within about 100 to 1000 bp) that control the transcription and translation of particular nucleic acid sequences to which they are operably linked. Such promoters fall into several classes: inducible, constitutive, and repressible promoters (that increase levels of transcription in response to absence of a repressor). Inducible promoters may initiate increased levels of transcription from DNA under their control in response to some change in culture conditions, e.g., the presence or absence of a nutrient or a change in temperature.

The yeast promoter fragment may also serve as the site for homologous recombination and integration of the expression vector into the same site in the yeast genome; alternatively a selectable marker is used as the site for homologous recombination. *Pichia* transformation is described in Cregg et al. (1985) Mol. Cell. Biol. 5:3376-3385.

Examples of suitable promoters from *Pichia* include the CUP1 (induced by the level of copper in the medium), tetracycline inducible promoters, thiamine inducible promoters, AOX1 promoter (Cregg et al. (1989) Mol. Cell. Biol. 9:1316-1323); ICL1 promoter (Menendez et al. (2003) Yeast 20(13):1097-108); glyceraldehyde-3-phosphate dehydrogenase promoter (GAP) (Waterham et al. (1997) Gene 186(1):37-44); and FLD1 promoter (Shen et al. (1998) Gene 216(1):93-102). The GAP promoter is a strong constitutive promoter and the CUP1, AOX and FLD1 promoters are inducible.

Other yeast promoters include ADH1, alcohol dehydrogenase II, GAL4, PHO3, PHO5, Pyk, and chimeric promoters derived therefrom. Additionally, non-yeast promoters may be used in the invention such as mammalian, insect, plant, reptile, amphibian, viral, and avian promoters. Most typically the promoter will comprise a mammalian promoter (potentially endogenous to the expressed genes) or will comprise a yeast or viral promoter that provides for efficient transcription in yeast systems.

The polypeptides of interest may be produced recombinantly not only directly, but also as a fusion polypeptide with a heterologous polypeptide, e.g. a signal sequence or other polypeptide having a specific cleavage site at the N-terminus of the mature protein or polypeptide. In general, the signal sequence may be a component of the vector, or it may be a part of the polypeptide coding sequence that is inserted into the vector. The heterologous signal sequence selected preferably is one that is recognized and processed through one of the standard pathways available within the host cell. The *S*. *cerevisiae* alpha factor pre-pro signal has proven effective in the secretion of a variety of recombinant proteins from *P. pastoris.* Other yeast signal sequences include the alpha mating factor signal sequence, the invertase signal sequence, and signal sequences derived from other secreted yeast polypeptides. Additionally, these signal peptide sequences may be engineered to provide for enhanced secretion in diploid yeast expression systems. Other secretion signals of interest also include mammalian signal sequences, which may be heterologous to the protein being secreted, or may be a native sequence for the protein being secreted. Signal sequences include pre-peptide sequences, and in some instances may include propeptide sequences. Many such signal sequences are known in the art, including the signal sequences found on immunoglobulin chains, e.g., K28 preprotoxin sequence, PHA-E, FACE, human MCP-1, human serum albumin signal sequences, human Ig heavy chain, human Ig light chain, and the like. For example, see Hashimoto et. al. Protein Eng 11(2) 75 (1998); and Kobayashi et. al. Therapeutic Apheresis 2(4) 257 (1998).

Transcription may be increased by inserting a transcriptional activator sequence into the vector. These activators are cis-acting elements of DNA, usually about from 10 to 300 bp, which act on a promoter to increase its transcription. Transcriptional enhancers are relatively orientation and position independent, having been found 5' and 3' to the transcription unit, within an intron, as well as within the coding sequence itself. The enhancer may be spliced into the expression vector at a position 5' or 3' to the coding sequence, but is preferably located at a site 5' from the promoter.

Expression vectors used in eukaryotic host cells may also contain sequences necessary for the termination of transcription and for stabilizing the mRNA. Such sequences are commonly available from 3' to the translation termination codon, in untranslated regions of eukaryotic or viral DNAs or cDNAs. These regions contain nucleotide segments transcribed as polyadenylated fragments in the untranslated portion of the mRNA.

Construction of suitable vectors containing one or more of the above-listed components employs standard ligation techniques or PCR/recombination methods. Isolated plasmids or DNA fragments are cleaved, tailored, and re-ligated in the form desired to generate the plasmids required or via recombination methods. For analysis to confirm correct sequences in plasmids constructed, the ligation mixtures are used to transform host cells, and successful transformants selected by antibiotic resistance (e.g. ampicillin or Zeocin) where appropriate. Plasmids from the transformants are prepared, analyzed by restriction endonuclease digestion and/or sequenced.

As an alternative to restriction and ligation of fragments, recombination methods based on att sites and recombination enzymes may be used to insert DNA sequences into a vector. Such methods are described, for example, by Landy (1989) Ann. Rev. Biochem. 58:913-949; and are known to those of skill in the art. Such methods utilize intermolecular DNA recombination that is mediated by a mixture of lambda and E. coli-encoded recombination proteins. Recombination occurs between specific attachment (att) sites on the interacting DNA molecules. For a description of att sites see Weisberg and Landy (1983) Site-Specific Recombination in Phage Lambda, in Lambda II, Weisberg, ed. (Cold Spring Harbor, N.Y.: Cold Spring Harbor Press), pp. 211-250. The DNA segments flanking the recombination sites are switched, such that after recombination, the att sites are hybrid sequences comprised of sequences donated by each parental vector. The recombination can occur between DNAs of any topology.

Att sites may be introduced into a sequence of interest by ligating the sequence of interest into an appropriate vector; generating a PCR product containing att B sites through the use of specific primers; generating a cDNA library cloned into an appropriate vector containing att sites; and the like.

*Monocistronic and polycistronic genes.* A monocistronic gene encodes an RNA that contains the genetic information to translate only a single protein. A polycistronic gene encodes an mRNA that contains the genetic information to translate more than one protein. The proteins encoded in a polycistronic gene may have the same or different sequences or a combination thereof. Dicistronic or bicistronic refers to a polycistronic gene that encodes two proteins. Polycistronic genes optionally include one or more internal ribosome entry site (IRES) elements to facilitate cap-independent initiation of translation, which may be situated at a location that can drive translation of the downstream protein coding region independently of the 5'-cap structure bound to the 5' end of the mRNA molecule. Any known IRES sequence (e.g., viral, eukaryotic, or artificial in origin) may be used. For example, the cricket paralysis virus IRES sequence in the intergenic region (IGR) may be used, as described in Thompson et al. (2001) PNAS 98:12972-12977. Optionally, IRES function may be potentiated by genetic alteration, e.g., by causing constitutive expression of eIF2 kinase GCN2 or disrupting two initiator tRNA(met) genes disrupted (id.).

*Folding,* as used herein, refers to the three-dimensional structure of polypeptides and proteins, where interactions between amino acid residues act to stabilize the structure. While non-covalent interactions are important in determining structure, usually the proteins of interest will have intra- and/or intermolecular covalent disulfide bonds formed by two cysteine residues. For naturally occurring proteins and polypeptides or derivatives and variants thereof, the proper folding is typically the arrangement that results in optimal biological activity, and can conveniently be monitored by assays for activity, e.g. ligand binding, enzymatic activity, etc.

In some instances, for example where the desired product is of synthetic origin, assays based on biological activity will be less meaningful. The proper folding of such molecules may be determined on the basis of physical properties, energetic considerations, modeling studies, and the like.

The expression host may be further modified by the introduction of sequences encoding one or more enzymes that enhance folding and disulfide bond formation, i.e. foldases, chaperoning, etc. Such sequences may be constitutively or inducibly expressed in the yeast host cell, using vectors, markers, etc. as known in the art. Preferably the sequences, including transcriptional regulatory elements sufficient for the desired pattern of expression, are stably integrated in the yeast genome through a targeted methodology.

For example, the eukaryotic PDI is not only an efficient catalyst of protein cysteine oxidation and disulfide bond isomerization, but also exhibits chaperone activity. Co-expression of PDI can facilitate the production of active proteins having multiple disulfide bonds. Also of interest is the expression of BIP (immunoglobulin heavy chain binding protein); cyclophilin; and the like. In one embodiment of the invention, the multi-subunit complex may be expressed from a yeast strain produced by mating, wherein each of the haploid parental strains expresses a distinct folding enzyme, e.g. one strain may express BIP, and the other strain may express PDI or combinations thereof.

The terms "desired protein" or "target protein" are used interchangeably and refer generally to a heterologous multi-subunit protein such as an antibody (e.g., a humanized antibody) or a binding portion thereof described herein.

The term "antibody" includes any polypeptide chain-containing molecular structure with a specific shape that fits to and recognizes an epitope, where one or more non-covalent binding interactions stabilize the complex between the molecular structure and the epitope. The archetypal antibody molecule is the immunoglobulin, and all types of immunoglobulins, IgG, IgM, IgA, IgE, IgD, etc., from all sources, e.g. human, rodent, rabbit, cow, sheep, pig, dog, other mammals, chicken, other avians, etc., are considered to be "antibodies." A preferred source for producing antibodies useful as starting material according to the invention is rabbits. Numerous antibody coding sequences have been described; and others may be raised by methods well-known in the art. Examples thereof include chimeric antibodies, human antibodies and other non-human mammalian antibodies, humanized antibodies, single chain antibodies such as scFvs, camelbodies, nanobodies, IgNAR (single-chain antibodies derived from sharks), small-modular immunopharmaceuticals (SMIPs), and antibody fragments such as Fabs, Fab', F(ab')₂ and the like. See Streltsov V A, et al., Structure of a shark IgNAR antibody variable domain and modeling of an early-developmental isotype, Protein Sci. 2005 November; 14(11):2901-9. Epub 2005 Sep. 30; Greenberg A S, et al., A new antigen receptor gene family that undergoes rearrangement and extensive somatic diversification in sharks, Nature. 1995 Mar. 9; 374(6518):168-73; Nuttall S D, et al., Isolation of the new antigen receptor from wobbegong sharks, and use as a scaffold for the display of protein loop libraries, Mol Immunol. 2001 August; 38(4):313-26; Hamers-Casterman C, et al., Naturally occurring antibodies devoid of light chains, Nature. 1993 Jun. 3; 363(6428):446-8; Gill D S, et al., Biopharmaceutical drug discovery using novel protein scaffolds, Curr Opin Biotechnol. 2006 December; 17(6):653-8. Epub 2006 Oct. 19.

For example, antibodies or antigen binding fragments may be produced by genetic engineering. In this technique, as with other methods, antibody-producing cells are sensitized to the desired antigen or immunogen. The messenger RNA isolated from antibody producing cells is used as a template to make cDNA using PCR amplification. A library of vectors, each containing one heavy chain gene and one light chain gene retaining the initial antigen specificity, is produced by insertion of appropriate sections of the amplified immunoglobulin cDNA into the expression vectors. A combinatorial library is constructed by combining the heavy chain gene library with the light chain gene library. This results in a library of clones which co-express a heavy and light chain (resembling the Fab fragment or antigen binding fragment of an antibody molecule). The vectors that carry these genes are co-transfected into a host cell. When antibody gene synthesis is induced in the transfected host, the heavy and light chain proteins self-assemble to produce active antibodies that can be detected by screening with the antigen or immunogen.

Antibody coding sequences of interest include those encoded by native sequences, as well as nucleic acids that, by virtue of the degeneracy of the genetic code, are not identical in sequence to the disclosed nucleic acids, and variants thereof. Variant polypeptides can include amino acid (aa) substitutions, additions or deletions. The amino acid substitutions can be conservative amino acid substitutions or substitutions to eliminate non-essential amino acids, such as to alter a glycosylation site, or to minimize misfolding by substitution or deletion of one or more cysteine residues that are not necessary for function. Variants can be designed so as to retain or have enhanced biological activity of a particular region of the protein (e.g., a functional domain, catalytic amino acid residues, etc). Variants also include fragments of the polypeptides disclosed herein, particularly biologically active fragments and/or fragments corresponding to functional domains. Techniques for in vitro mutagenesis of cloned genes are known. Also included in the subject invention are polypeptides that have been modified using ordinary molecular biological techniques so as to improve their resistance to proteolytic degradation or to optimize solubility properties or to render them more suitable as a therapeutic agent.

Chimeric antibodies may be made by recombinant means by combining the variable light and heavy chain regions (VL and VH), obtained from antibody producing cells of one species with the constant light and heavy chain regions from another. Typically chimeric antibodies utilize rodent or rabbit variable regions and human constant regions, in order to produce an antibody with predominantly human domains. The production of such chimeric antibodies is well known in the art, and may be achieved by standard means (as described, e.g., in , U.S. Pat. No. 5,624,659). It is further contemplated that the human constant regions of chimeric antibodies of the invention may be selected from IgG1, IgG2, IgG3, and IgG4 constant regions.

Humanized antibodies are engineered to contain even more human-like immunoglobulin domains, and incorporate only the complementarity-determining regions of the animal-derived antibody. This is accomplished by carefully examining the sequence of the hyper- variable loops of the variable regions of the monoclonal antibody, and fitting them to the structure of the human antibody chains. Although facially complex, the process is straightforward in practice. See, e.g., , U.S. Pat. No. 6,187,287. Methods of humanizing antibodies have been described previously in issued , U.S. Patent No. 7935340. In some instances, a determination of whether additional rabbit framework residues are required to maintain activity is necessary. In some instances the humanized antibodies still requires some critical rabbit framework residues to be retained to minimize loss of affinity or activity. In these cases, it is necessary to change single or multiple framework amino acids from human germline sequences back to the original rabbit amino acids in order to have desired activity. These changes are determined experimentally to identify which rabbit residues are necessary to preserve affinity and activity.

In addition to entire immunoglobulins (or their recombinant counterparts), immunoglobulin fragments comprising the epitope binding site (e.g., Fab', F(ab')₂, or other fragments) may be synthesized. "Fragment," or minimal immunoglobulins may be designed utilizing recombinant immunoglobulin techniques. For instance "Fv" immunoglobulins for use in the present invention may be produced by synthesizing a fused variable light chain region and a variable heavy chain region. Combinations of antibodies are also of interest, e.g. diabodies, which comprise two distinct Fv specificities. In another embodiment of the invention, SMIPs (small molecule immunopharmaceuticals), camelbodies, nanobodies, and IgNAR are encompassed by immunoglobulin fragments.

Immunoglobulins and fragments thereof may be modified post-translationally, e.g. to add effector moieties such as chemical linkers, detectable moieties, such as fluorescent dyes, enzymes, toxins, substrates, bioluminescent materials, radioactive materials, chemiluminescent moieties and the like, or specific binding moieties, such as streptavidin, avidin, or biotin, and the like may be utilized in the methods and compositions of the present invention. Examples of additional effector molecules are provided infra.

*Product-associated variant:* a product other than the desired product (e.g., the desired multi-subunit complex) which is present in a preparation of the desired product and related to the desired product. Exemplary product-associated variants include truncated or elongated peptides, products having different glycosylation than the desired glycosylation (e.g., if an aglycosylated product is desired then any glycosylated product would be considered to be a product-associated variant), complexes having abnormal stoichiometry, improper assembly, abnormal disulfide linkages, abnormal or incomplete folding, aggregation, protease cleavage, or other abnormalities. Exemplary product-associated variants may exhibit alterations in one or more of molecular mass (e.g., detected by size exclusion chromatography), isoelectric point (e.g., detected by isoelectric focusing), electrophoretic mobility (e.g., detected by gel electrophoresis), phosphorylation state (e.g., detected by mass spectrometry), charge to mass ratio (e.g., detected by mass spectrometry), mass or identity of proteolytic fragments (e.g., detected by mass spectrometry or gel electrophoresis), hydrophobicity (e.g., detected by HPLC), charge (e.g., detected by ion exchange chromatography), affinity (*e.g*., in the case of an antibody, detected by binding to protein A, protein G, and/or an epitope to which the desired antibody binds), and glycosylation state (e.g., detected by lectin binding affinity). Where the desired protein is an antibody, the term product-associate variant may include a glyco-heavy variant and/or half antibody species (described below).

Exemplary product-associated variants include variant forms that contain aberrant disulfide bonds. For example, most IgG1 antibody molecules are stabilized by a total of 16 intra-chain and inter-chain disulfide bridges, which stabilize the folding of the IgG domains in both heavy and light chains, while the inter-chain disulfide bridges stabilize the association between heavy and light chains. Other antibody types likewise contain characteristic stabilizing intra-chain and inter-chain disulfide bonds. Further, some antibodies (including Ab-A and Ab-B disclosed herein) contain additional disulfide bonds referred to as non-canonical disulfide bonds. Thus, aberrant inter-chain disulfide bonds may result in abnormal complex stoichiometry, due to the absence of a stabilizing covalent linkage, and/or disulfide linkages to additional subunits. Additionally, aberrant disulfide bonds (whether inter-chain or intra-chain) may decrease structural stability of the antibody, which may result in decreased activity, decreased stability, increased propensity to form aggregates, and/or increased immunogenicity. Product-associated variants containing aberrant disulfide bonds may be detected in a variety of ways, including non-reduced denaturing SDS-PAGE, capillary electrophoresis, cIEX, mass spectrometry (optionally with chemical modification to produce a mass shift in free cysteines), size exclusion chromatography, HPLC, changes in light scattering, and any other suitable methods known in the art. *See, e.g.,* The Protein Protocols Handbook 2002, Part V, 581-583, DOI: 10.1385/1-59259-169-8:581;

*Half antibody, half-antibody species,* or *H1L1* refer to a protein complex that includes a single heavy and single light antibody chain, but lacks a covalent linkage to a second heavy and light antibody chain. Two half antibodies may remain non-covalently associated under some conditions (which may give behavior similar to a full antibody, e.g., apparent molecular weight determined by size exclusion chromatography). Similarly, *H2L1* refers to a protein complex that includes two heavy antibody chains and single light antibody chain, but lacks a covalent linkage to a second light antibody chain; these complexes may also non-covalently associate with another light antibody chain (and likewise give similar behavior to a full antibody). Like full antibodies, half antibody species and H2L1 species can dissociate under reducing conditions into individual heavy and light chains. Half antibody species and H2L1 species can be detected on a non-reduced SDS-PAGE gel as a species migrating at a lower apparent molecular weight than the full antibody, e.g., H1L1 migrates at approximately half the apparent molecular weight of the full antibody (e.g., about 75 kDa).

*Glyco-heavy variant* refers to a glycosylated product-associated variant sometimes present in antibody preparations and which contains at least a partial Fc sequence. The glyco-heavy variant is characterized by decreased electrophoretic mobility observable by SDS-PAGE (relative to a normal heavy chain), lectin binding affinity, binding to an anti-Fc antibody, and apparent higher molecular weight of antibody complexes containing the glyco-heavy variant as determined by size exclusion chromatography. See U.S. Provisional Application Ser. No. 61/525,307 (Atty. Docket No. 67858.730200), filed August 31, 2011.

The term "polyploid yeast that stably expresses or expresses a desired secreted heterologous polypeptide for prolonged time" refers to a yeast culture that secretes said polypeptide for at least several days to a week, more preferably at least a month, still more preferably at least 1-6 months, and even more preferably for more than a year at threshold expression levels, typically at least 50-500 mg/liter (after about 90 hours in culture) and preferably substantially greater.

The term "polyploidal yeast culture that secretes desired amounts of recombinant polypeptide" refers to cultures that stably or for prolonged periods secrete at least at least 50-500 mg/liter, and most preferably 500-1000 mg/liter or more.

A polynucleotide sequence "corresponds" to a polypeptide sequence if translation of the polynucleotide sequence in accordance with the genetic code yields the polypeptide sequence (i.e., the polynucleotide sequence "encodes" the polypeptide sequence), one polynucleotide sequence "corresponds" to another polynucleotide sequence if the two sequences encode the same polypeptide sequence.

A "heterologous" region or domain of a DNA construct is an identifiable segment of DNA within a larger DNA molecule that is not found in association with the larger molecule in nature. Thus, when the heterologous region encodes a mammalian gene, the gene will usually be flanked by DNA that does not flank the mammalian genomic DNA in the genome of the source organism. Another example of a heterologous region is a construct where the coding sequence itself is not found in nature (e.g., a cDNA where the genomic coding sequence contains introns, or synthetic sequences having codons different than the native gene). Allelic variations or naturally-occurring mutational events do not give rise to a heterologous region of DNA as defined herein.

A "coding sequence" is an in-frame sequence of codons that (in view of the genetic code) correspond to or encode a protein or peptide sequence. Two coding sequences correspond to each other if the sequences or their complementary sequences encode the same amino acid sequences. A coding sequence in association with appropriate regulatory sequences may be transcribed and translated into a polypeptide. A polyadenylation signal and transcription termination sequence will usually be located 3' to the coding sequence. A "promoter sequence" is a DNA regulatory region capable of binding RNA polymerase in a cell and initiating transcription of a downstream (3' direction) coding sequence. Promoter sequences typically contain additional sites for binding of regulatory molecules (e.g., transcription factors) which affect the transcription of the coding sequence. A coding sequence is "under the control" of the promoter sequence or "operatively linked" to the promoter when RNA polymerase binds the promoter sequence in a cell and transcribes the coding sequence into mRNA, which is then in turn translated into the protein encoded by the coding sequence.

Vectors are used to introduce a foreign substance, such as DNA, RNA or protein, into an organism or host cell. Typical vectors include recombinant viruses (for polynucleotides) and liposomes (for polypeptides). A "DNA vector" is a replicon, such as plasmid, phage or cosmid, to which another polynucleotide segment may be attached so as to bring about the replication of the attached segment. An "expression vector" is a DNA vector which contains regulatory sequences which will direct polypeptide synthesis by an appropriate host cell. This usually means a promoter to bind RNA polymerase and initiate transcription of mRNA, as well as ribosome binding sites and initiation signals to direct translation of the mRNA into a polypeptide(s). Incorporation of a polynucleotide sequence into an expression vector at the proper site and in correct reading frame, followed by transformation of an appropriate host cell by the vector, enables the production of a polypeptide encoded by said polynucleotide sequence.

"Amplification" of polynucleotide sequences is the in vitro production of multiple copies of a particular nucleic acid sequence. The amplified sequence is usually in the form of DNA. A variety of techniques for carrying out such amplification are described in the following review articles: Van Brunt 1990, Bio/Technol., 8(4):291-294; and Gill and Ghaemi, Nucleosides Nucleotides Nucleic Acids. 2008 Mar;27(3):224-43. Polymerase chain reaction or PCR is a prototype of nucleic acid amplification, and use of PCR herein should be considered exemplary of other suitable amplification techniques.

The general structure of antibodies in most vertebrates (including mammals) is now well understood (Edelman, G. M., Ann. N.Y. Acad. Sci., 190: 5 (1971)). Conventional antibodies consist of two identical light polypeptide chains of molecular weight approximately 23,000 daltons (the "light chain"), and two identical heavy chains of molecular weight 53,000-70,000 (the "heavy chain"). The four chains are joined by disulfide bonds in a "Y" configuration wherein the light chains bracket the heavy chains starting at the mouth of the "Y" configuration. The "branch" portion of the "Y" configuration is designated the F_{ab} region; the stem portion of the "Y" configuration is designated the F_{c} region. The amino acid sequence orientation runs from the N-terminal end at the top of the "Y" configuration to the C-terminal end at the bottom of each chain. The N-terminal end possesses the variable region having specificity for the antigen that elicited it, and is approximately 100 amino acids in length, there being slight variations between light and heavy chain and from antibody to antibody.

The variable region is linked in each chain to a constant region that extends the remaining length of the chain and that within a particular class of antibody does not vary with the specificity of the antibody (i.e., the antigen eliciting it). There are five known major classes of constant regions that determine the class of the immunoglobulin molecule (IgG, IgM, IgA, IgD, and IgE corresponding to gamma, mu, alpha, delta, and epsilon heavy chain constant regions). The constant region or class determines subsequent effector function of the antibody, including activation of complement (Kabat, E. A., Structural Concepts in Immunology and Immunochemistry, 2nd Ed., p. 413-436, Holt, Rinehart, Winston (1976)), and other cellular responses (Andrews, D. W., et al., Clinical Immunobiology, pp 1-18, W. B. Sanders (1980); Kohl, S., et al., Immunology, 48: 187 (1983)); while the variable region determines the antigen with which it will react. Light chains are classified as either kappa or lambda. Each heavy chain class can be paired with either kappa or lambda light chain. The light and heavy chains are covalently bonded to each other, and the "tail" portions of the two heavy chains are bonded to each other by covalent disulfide linkages when the immunoglobulins are generated either by hybridomas or by B cells.

The expression "variable region" or "VR" refers to the domains within each pair of light and heavy chains in an antibody that are involved directly in binding the antibody to the antigen. Each heavy chain has at one end a variable domain (V_{H}) followed by a number of constant domains. Each light chain has a variable domain (V_{L}) at one end and a constant domain at its other end; the constant domain of the light chain is aligned with the first constant domain of the heavy chain, and the light chain variable domain is aligned with the variable domain of the heavy chain.

The expressions "complementarity determining region," "hypervariable region," or "CDR" refer to one or more of the hyper-variable or complementarity determining regions (CDRs) found in the variable regions of light or heavy chains of an antibody (See Kabat, E. A. et al., Sequences of Proteins of Immunological Interest, National Institutes of Health, Bethesda, Md., (1987)). These expressions include the hypervariable regions as defined by Kabat et al. ("Sequences of Proteins of Immunological Interest," Kabat E., et al., US Dept. of Health and Human Services, 1983) or the hypervariable loops in 3-dimensional structures of antibodies (Chothia and Lesk, J Mol. Biol. 196 901-917 (1987)). The CDRs in each chain are held in close proximity by framework regions and, with the CDRs from the other chain, contribute to the formation of the antigen binding site. Within the CDRs there are select amino acids that have been described as the selectivity determining regions (SDRs) which represent the critical contact residues used by the CDR in the antibody-antigen interaction (Kashmiri, S., Methods, 36:25-34 (2005)).

The expressions "framework region" or "FR" refer to one or more of the framework regions within the variable regions of the light and heavy chains of an antibody (See Kabat, E. A. et al., Sequences of Proteins of Immunological Interest, National Institutes of Health, Bethesda, Md., (1987)). These expressions include those amino acid sequence regions interposed between the CDRs within the variable regions of the light and heavy chains of an antibody.

### Anti-NGF Antibodies and Binding Fragments Thereof Having Binding Activity for NGF

### Antibody Ab1

The description contemplates methods of treating pain and the specific pain associated disorders using antibody Ab1 or fragments thereof, or an antibody or antibody fragment that binds to the same or overlapping epitope as Ab1, for example as set forth below, alone or is association with another active agent, e.g., an NSAID or opioid analgesic, in a therapeutically effective amount which inhibits the association of NGF with TrkA and the association of NGF with p75 and/or inhibits or prevents pain. In one example, the description includes chimeric antibodies having binding specificity to NGF and possessing a variable light chain sequence comprising the sequence set forth below:

Thedescriptionoptionally contemplates methods of treating pain and the specific pain associated disorders alone or is association with another active agent, e.g., an NSAID or opioid analgesic, wherein the antibodies include chimeric antibodies having binding specificity to NGF and possessing a light chain sequence comprising the sequence set forth below:

The description optionally contemplates methods of treating pain and the specific pain associated disorders alone or is association with another active agent, e.g., an NSAID or opioid analgesic, wherein the antibodies include chimeric antibodies having binding specificity to NGF and possessing a variable heavy chain sequence comprising the sequence set forth below:

The description optionally contemplates methods of treating pain and the specific pain associated disorders alone or is association with another active agent, e.g., an NSAID or opioid analgesic, wherein the antibodies include chimeric antibodies having binding specificity to NGF and possessing a heavy chain sequence comprising the sequence set forth below:

The description optionally contemplates methods of treating pain and the specific pain associated disorders alone or is association with another active agent, e.g., an NSAID or opioid analgesic, wherein the antibodies comprise one or more of the polypeptide sequences of SEQ ID NO: 5; SEQ ID NO: 6; and SEQ ID NO: 7 which correspond to the complementarity-determining regions (CDRs, or hypervariable regions) of the variable light chain sequence of SEQ ID NO: 1 or the light chain sequence of SEQ ID NO: 2, and/or one or more of the polypeptide sequences of SEQ ID NO: 8; SEQ ID NO: 9; and SEQ ID NO: 10 which correspond to the complementarity-determining regions (CDRs, or hypervariable regions) of the variable heavy chain sequence of SEQ ID NO: 3 or the heavy chain sequence of SEQ ID NO: 4, or combinations of these polypeptide sequences. In another example of the description, the antibodies of the description or fragments thereof comprise, or alternatively consist of, combinations of one or more of the CDRs, the variable heavy and variable light chain sequences, and the heavy and light chain sequences set forth above, including all of them.

The description optionally contemplates methods of treating pain and the specific pain associated disorders alone or is association with another active agent, e.g., an NSAID or opioid analgesic, wherein the antibody is a fragment having binding specificity to NGF. In one example of the description, antibody fragments of the invention comprise, or alternatively consist of, the polypeptide sequence of SEQ ID NO: 1 or SEQ ID NO: 2. In another example of the description, antibody fragments of the invention comprise, or alternatively consist of, the polypeptide sequence of SEQ ID NO: 3 or SEQ ID NO: 4.

In a further example of the description, fragments of the antibody having binding specificity to NGF for treatment or prevention of pain comprise, or alternatively consist of, one or more of the polypeptide sequences of SEQ ID NO: 5; SEQ ID NO: 6; and SEQ ID NO: 7 which correspond to the complementarity-determining regions (CDRs, or hypervariable regions) of the variable light chain sequence of SEQ ID NO: 1 or the light chain sequence of SEQ ID NO: 2.

The description optionally contemplates methods of treating pain and the specific pain associated disorders alone or is association with another active agent, e.g., an NSAID or opioid analgesic, wherein the antibodies include fragments having binding specificity to NGF comprise, or alternatively consist of, one or more of the polypeptide sequences of SEQ ID NO: 8; SEQ ID NO: 9; and SEQ ID NO: 10 which correspond to the complementarity-determining regions (CDRs, or hypervariable regions) of the variable heavy chain sequence of SEQ ID NO: 3 or the heavy chain sequence of SEQ ID NO: 4.

The description also optionally contemplates antibody fragments which include one or more of the antibody fragments described herein. In one example of the description, fragments of the antibodies having binding specificity to NGF comprise, or alternatively consist of, one, two, three or more, including all of the following antibody fragments: the variable light chain region of SEQ ID NO: 1; the variable heavy chain region of SEQ ID NO: 3; the complementarity-determining regions (SEQ ID NO: 5; SEQ ID NO: 6; and SEQ ID NO: 7) of the variable light chain region of SEQ ID NO: 1; and the complementarity-determining regions (SEQ ID NO: 8; SEQ ID NO: 9; and SEQ ID NO: 10) of the variable heavy chain region of SEQ ID NO: 3.

In a particularly preferred optional example of the description, the chimeric anti-NGF antibody is Ab1, comprising, or alternatively consisting of, SEQ ID NO: 2 and SEQ ID NO: 4, and having at least one of the biological activities set forth herein.

In a further particularly preferred optional example of the description, antibody fragments for use herein comprise, or alternatively consist of, Fab (fragment antigen binding) fragments having binding specificity for NGF or another Fab or monovalent antibody fragment that binds to the same or overlapping epitope as Ab1. With respect to antibody Ab1, the Fab fragment includes the variable light chain sequence of SEQ ID NO: 1 and the variable heavy chain sequence of SEQ ID NO: 3. This example of the description further contemplates additions, deletions, and variants of SEQ ID NO: 1 and/or SEQ ID NO: 3 in said Fab while retaining binding specificity for NGF.

In one optional example of the description, described herein (infra), Fab fragments may be produced by enzymatic digestion (e.g., papain) of Ab1. In another example of the description, anti-NGF antibodies such as or Fab fragments thereof may be produced via expression in mammalian cells such as CHO, NSO or HEK 293 cells, fungal, insect, plant or microbial systems such as yeast cells (for example diploid yeast such as diploid Pichia) and other yeast strains. Suitable Pichia species include, but are not limited to, Pichia pastoris.

### Antibody Ab2

The description optionally contemplates methods of treating pain and the specific pain associated disorders alone or is association with another active agent, e.g., an NSAID or opioid analgesic, wherein the antibodies include chimeric or humanized antibodies having binding specificity to NGF wherein the antibody is antibody Ab2 or fragments thereof, or an antibody or antibody fragment that binds to the same or overlapping epitope as Ab2, for example as set forth below, in a therapeutically effective amount which inhibits the association of NGF with TrkA and the association of NGF with p75. In one example, the description includes chimeric or humanized antibodies having binding specificity to NGF and possessing a variable light chain sequence comprising the sequence set forth below:

The description also optionally includes chimeric or humanized antibodies for treatment or prevention of pain and pain associated conditions having binding specificity to NGF and possessing a light chain sequence comprising the sequence set forth below:

The description further optionally includes chimeric or humanized antibodies having binding specificity to NGF for treatment or prevention of pain and pain associated conditions and possessing a variable heavy chain sequence comprising the sequence set forth below:

The description also optionally includes chimeric or humanized antibodies having binding specificity to NGF for treatment or prevention of pain and pain associated conditions and possessing a heavy chain sequence comprising the sequence set forth below:

The description further optionally contemplates antibodies for treatment or prevention of pain and pain associated conditions comprising one or more of the polypeptide sequences of SEQ ID NO: 15; SEQ ID NO: 16; and SEQ ID NO: 17 which correspond to the complementarity-determining regions (CDRs, or hypervariable regions) of the variable light chain sequence of SEQ ID NO: 11 or the light chain sequence of SEQ ID NO: 12, and/or one or more of the polypeptide sequences of SEQ ID NO: 18; SEQ ID NO: 19; and SEQ ID NO: 20 which correspond to the complementarity-determining regions (CDRs, or hypervariable regions) of the variable heavy chain sequence of SEQ ID NO: 13 or the heavy chain sequence of SEQ ID NO: 14, or combinations of these polypeptide sequences. In another example of the description, the antibodies of the description or fragments thereof comprise, or alternatively consist of, combinations of one or more of the CDRs, the variable heavy and variable light chain sequences, and the heavy and light chain sequences set forth above, including all of them.

The description also optionally contemplates fragments of the antibody having binding specificity to NGF for treatment or prevention of pain and pain associated conditions. In one example of the description, antibody fragments of the description comprise, or alternatively consist of, the polypeptide sequence of SEQ ID NO: 11 or SEQ ID NO: 12. In another example of the description, antibody fragments of the description comprise, or alternatively consist of, the polypeptide sequence of SEQ ID NO: 13 or SEQ ID NO: 14.

In a further example of the description, fragments of the antibody having binding specificity to NGF for treatment or prevention of pain and pain associated conditions optionally comprise, or alternatively consist of, one or more of the polypeptide sequences of SEQ ID NO: 15; SEQ ID NO: 16; and SEQ ID NO: 17 which correspond to the complementarity-determining regions (CDRs, or hypervariable regions) of the variable light chain sequence of SEQ ID NO: 11 or the light chain sequence of SEQ ID NO: 12.

In a further optional example of the description, fragments of the antibody having binding specificity to NGF for treatment or prevention of pain and pain associated conditions comprise, or alternatively consist of, one or more of the polypeptide sequences of SEQ ID NO: 18; SEQ ID NO: 19; and SEQ ID NO: 20 which correspond to the complementarity-determining regions (CDRs, or hypervariable regions) of the variable heavy chain sequence of SEQ ID NO: 13 or the heavy chain sequence of SEQ ID NO: 14.

The description also optionally contemplates antibody fragments which include one or more of the antibody fragments described herein. In one example of the description, fragments of the antibodies having binding specificity to NGF for treatment or prevention of pain and pain associated conditions comprise, or alternatively consist of, one, two, three or more, including all of the following antibody fragments: the variable light chain region of SEQ ID NO: 11; the variable heavy chain region of SEQ ID NO: 13; the complementarity-determining regions (SEQ ID NO: 15; SEQ ID NO: 16; and SEQ ID NO: 17) of the variable light chain region of SEQ ID NO: 11; and the complementarity-determining regions (SEQ ID NO: 18; SEQ ID NO: 19; and SEQ ID NO: 20) of the variable heavy chain region of SEQ ID NO: 13.

In a particularly preferred optional example of the description, the chimeric or humanized anti-NGF antibody for treatment or prevention of pain and pain associated conditions optionally is Ab2, comprising, or alternatively consisting of, SEQ ID NO: 12 and SEQ ID NO: 14, and having at least one of the biological activities set forth herein.

In a further particularly preferred optional example of the description, antibody fragments for treatment or prevention of pain and pain associated conditions comprise, or alternatively consist of, Fab (fragment antigen binding) fragments having binding specificity for NGF or a Fab or other monovalent antibody fragment that binds to the same or overlapping epitope as Ab2,. With respect to antibody Ab2, the Fab fragment includes the variable light chain sequence of SEQ ID NO: 11 and the variable heavy chain sequence of SEQ ID NO: 13. This example of the description further contemplates additions, deletions, and variants of SEQ ID NO: 11 and/or SEQ ID NO: 13 in said Fab while retaining binding specificity for NGF.

In one optional example of the description described herein (infra), Fab fragments may be produced by enzymatic digestion (e.g., papain) of Ab2. In another embodiment of the invention, anti-NGF antibodies such as Ab2 or Fab fragments thereof may be produced via expression in mammalian cells such as CHO, NSO or HEK 293 cells, fungal, insect, plant or microbial systems such as yeast cells (for example diploid yeast such as diploid Pichia) and other yeast strains. Suitable Pichia species include, but are not limited to, Pichia pastoris.

### Antibody Ab3

The description contemplates methods of treating pain and the specific pain associated disorders alone or is association with another active agent, e.g., an NSAID or opioid analgesic, wherein the antibodies include chimeric or humanized antibodies having binding specificity to NGF wherein the antibody is Ab3 or fragments thereof, or another antibody or antibody fragment that binds to the same or overlapping epitope as Ab3, for example as set forth below, in a therapeutically effective amount which inhibits the association of NGF with TrkA without appreciably inhibiting the association of NGF with p75. In one example, the description includes chimeric antibodies having binding specificity to NGF and possessing a variable light chain sequence comprising the sequence set forth below:

The description also includes chimeric antibodies or treatment or prevention of pain and pain associated conditions having binding specificity to NGF and possessing a light chain sequence comprising the sequence set forth below:

The description further includes chimeric antibodies for treatment or prevention of pain and pain associated conditions having binding specificity to NGF and possessing a variable heavy chain sequence comprising the sequence set forth below:

The description also includes chimeric antibodies for treatment or prevention of pain and pain associated conditions having binding specificity to NGF and possessing a heavy chain sequence comprising the sequence set forth below:

The description further contemplates antibodies for treatment or prevention of pain and pain associated conditions comprising one or more of the polypeptide sequences of SEQ ID NO: 25; SEQ ID NO: 26; and SEQ ID NO: 27 which correspond to the complementarity-determining regions (CDRs, or hypervariable regions) of the variable light chain sequence of SEQ ID NO: 21 or the light chain sequence of SEQ ID NO: 22, and/or one or more of the polypeptide sequences of SEQ ID NO: 28; SEQ ID NO: 29; and SEQ ID NO: 30 which correspond to the complementarity-determining regions (CDRs, or hypervariable regions) of the variable heavy chain sequence of SEQ ID NO: 23 or the heavy chain sequence of SEQ ID NO: 24, or combinations of these polypeptide sequences. In another example of the description the antibodies of the description or fragments thereof comprise, or alternatively consist of, combinations of one or more of the CDRs, the variable heavy and variable light chain sequences, and the heavy and light chain sequences set forth above, including all of them.

The description also contemplates fragments of the antibody for treatment or prevention of pain and pain associated conditions having binding specificity to NGF. In one example of the description, antibody fragments of the description comprise, or alternatively consist of, the polypeptide sequence of SEQ ID NO: 21 or SEQ ID NO: 22. In another example of the description, antibody fragments of the description comprise, or alternatively consist of, the polypeptide sequence of SEQ ID NO: 23 or SEQ ID NO: 24.

In a further optional example of the description fragments of the antibody having binding specificity to NGF for treatment or prevention of pain and pain associated conditions comprise, or alternatively consist of, one or more of the polypeptide sequences of SEQ ID NO: 25; SEQ ID NO: 26; and SEQ ID NO: 27 which correspond to the complementarity-determining regions (CDRs, or hypervariable regions) of the variable light chain sequence of SEQ ID NO: 21 or the light chain sequence of SEQ ID NO: 22.

In a further example of the description fragments of the antibody having binding specificity to NGF for treatment or prevention of pain and pain associated conditions comprise, or alternatively consist of, one or more of the polypeptide sequences of SEQ ID NO: 28; SEQ ID NO: 29; and SEQ ID NO: 30 which correspond to the complementarity-determining regions (CDRs, or hypervariable regions) of the variable heavy chain sequence of SEQ ID NO: 23 or the heavy chain sequence of SEQ ID NO: 24.

The description also optionally contemplates antibody fragments which include one or more of the antibody fragments described herein for treatment or prevention of pain and pain associated conditions. In one example of the description fragments of the antibodies having binding specificity to NGF comprise, or alternatively consist of, one, two, three or more, including all of the following antibody fragments: the variable light chain region of SEQ ID NO: 21; the variable heavy chain region of SEQ ID NO: 23; the complementarity-determining regions (SEQ ID NO: 25; SEQ ID NO: 26; and SEQ ID NO: 27) of the variable light chain region of SEQ ID NO: 21; and the complementarity-determining regions (SEQ ID NO: 28; SEQ ID NO: 29; and SEQ ID NO: 30) of the variable heavy chain region of SEQ ID NO: 23.

In a particularly preferred optional example of the description the chimeric anti-NGF antibody for treatment or prevention of pain and pain associated conditions is Ab3, comprising, or alternatively consisting of, SEQ ID NO: 22 and SEQ ID NO: 24, and having at least one of the biological activities set forth herein.

In a further particularly preferred optional example of the description, antibody fragments for treatment or prevention of pain and pain associated conditions comprise, or alternatively consist of, Fab (fragment antigen binding) fragments having binding specificity for NGF or another Fab or monovalent antibody fragment that binds to the same or overlapping epitope as Ab3. With respect to antibody Ab3, the Fab fragment includes the variable light chain sequence of SEQ ID NO: 21 and the variable heavy chain sequence of SEQ ID NO: 23. This example of the description, further contemplates additions, deletions, and variants of SEQ ID NO: 21 and/or SEQ ID NO: 23 in said Fab while retaining binding specificity for NGF.

In one optional example of the description described herein (infra), Fab fragments may be produced by enzymatic digestion (e.g., papain) of Ab3. In another embodiment of the invention, anti-NGF antibodies such as Ab3 or Fab fragments thereof may be produced via expression in mammalian cells such as CHO, NSO or HEK 293 cells, fungal, insect, plant or microbial systems such as yeast cells (for example diploid yeast such as diploid Pichia) and other yeast strains. Suitable Pichia species include, but are not limited to, Pichia pastoris.

### Antibody Ab4

The description contemplates methods of treating pain and the specific pain associated disorders alone or is association with another active agent, e.g., an NSAID or opioid analgesic, wherein the antibodies include chimeric or humanized antibodies having binding specificity to NGF wherein the antibody is antibody Ab4 or fragments thereof, or another antibody or antibody fragment that binds to the same or overlapping epitope as Ab4, for example as set forth below, in a therapeutically effective amount which inhibits the association of NGF with TrkA without appreciably inhibiting the association of NGF with p75 and/or for preventing or effectively treating pain. In one example, the description, includes humanized antibodies for treatment or prevention of pain and pain associated conditions having binding specificity to NGF and possessing a variable light chain sequence comprising the sequence set forth below:

The description also includes chimeric or humanized antibodies for treatment or prevention of pain and pain associated conditions having binding specificity to NGF and possessing a light chain sequence comprising the sequence set forth below:

The description further includes humanized antibodies for treatment or prevention of pain and pain associated conditions having binding specificity to NGF and possessing a variable heavy chain sequence comprising the sequence set forth below:

The description also includes chimeric or humanized antibodies for treatment or prevention of pain and pain associated conditions having binding specificity to NGF and possessing a heavy chain sequence comprising the sequence set forth below:

The description further contemplates antibodies for treatment or prevention of pain and pain associated conditions comprising one or more of the polypeptide sequences of SEQ ID NO: 35; SEQ ID NO: 36; and SEQ ID NO: 37 which correspond to the complementarity-determining regions (CDRs, or hypervariable regions) of the variable light chain sequence of SEQ ID NO: 31 or the light chain sequence of SEQ ID NO: 32, and/or one or more of the polypeptide sequences of SEQ ID NO: 38; SEQ ID NO: 39; and SEQ ID NO: 40 which correspond to the complementarity-determining regions (CDRs, or hypervariable regions) of the variable heavy chain sequence of SEQ ID NO: 33 or the heavy chain sequence of SEQ ID NO: 34, or combinations of these polypeptide sequences. In another example of the description, the antibodies of the description or fragments thereof comprise, or alternatively consist of, combinations of one or more of the CDRs, the variable heavy and variable light chain sequences, and the heavy and light chain sequences set forth above, including all of them.

The description also contemplates fragments of the antibody having binding specificity to NGF for treatment or prevention of pain and pain associated conditions. In one example of the description, antibody fragments of the description comprise, or alternatively consist of, the polypeptide sequence of SEQ ID NO: 31 or SEQ ID NO: 32. In another embodiment of the invention, antibody fragments of the invention comprise, or alternatively consist of, the polypeptide sequence of SEQ ID NO: 33 or SEQ ID NO: 34.

In a further example of the description, fragments of the antibody having binding specificity to NGF for treatment or prevention of pain and pain associated conditions comprise, or alternatively consist of, one or more of the polypeptide sequences of SEQ ID NO: 35; SEQ ID NO: 36; and SEQ ID NO: 37 which correspond to the complementarity-determining regions (CDRs, or hypervariable regions) of the variable light chain sequence of SEQ ID NO: 31 or the light chain sequence of SEQ ID NO: 32.

In a further example of the description, fragments of the antibody having binding specificity to NGF for treatment or prevention of pain and pain associated conditions comprise, or alternatively consist of, one or more of the polypeptide sequences of SEQ ID NO: 38; SEQ ID NO: 39; and SEQ ID NO: 40 which correspond to the complementarity-determining regions (CDRs, or hypervariable regions) of the variable heavy chain sequence of SEQ ID NO: 33 or the heavy chain sequence of SEQ ID NO: 34.

The description also optional contemplates antibody fragments for treatment or prevention of pain and pain associated conditions which include one or more of the antibody fragments described herein. In one example of the description, fragments of the antibodies having binding specificity to NGF comprise, or alternatively consist of, one, two, three or more, including all of the following antibody fragments: the variable light chain region of SEQ ID NO: 31; the variable heavy chain region of SEQ ID NO: 33; the complementarity-determining regions (SEQ ID NO: 35; SEQ ID NO: 36; and SEQ ID NO: 37) of the variable light chain region of SEQ ID NO: 31; and the complementarity-determining regions (SEQ ID NO: 38; SEQ ID NO: 39; and SEQ ID NO: 40) of the variable heavy chain region of SEQ ID NO: 33.

In a particularly preferred example of the description, the chimeric or humanized anti-NGF antibody for treatment or prevention of pain and pain associated conditions is Ab4, comprising, or alternatively consisting of, SEQ ID NO: 32 and SEQ ID NO: 34, and having at least one of the biological activities set forth herein.

In a further particularly preferred example of the description, antibody fragments for treatment or prevention of pain and pain associated conditions comprise, or alternatively consist of, Fab (fragment antigen binding) fragments having binding specificity for NGF or another Fab or monovalent antibody fragment that binds to the same or overlapping epitope as Ab14. With respect to antibody Ab4, the Fab fragment includes the variable light chain sequence of SEQ ID NO: 31 and the variable heavy chain sequence of SEQ ID NO: 33. This example of the description further contemplates additions, deletions, and variants of SEQ ID NO: 31 and/or SEQ ID NO: 33 in said Fab while retaining binding specificity for NGF.

In one example of the description described herein (infra), Fab fragments for treatment or prevention of pain and pain associated conditions may be produced by enzymatic digestion (e.g., papain) of Ab4. In another example of the description, anti-NGF antibodies such as Ab4 or Fab fragments thereof may be produced via expression in mammalian cells such as CHO, NSO or HEK 293 cells, fungal, insect, plant or microbial systems such as yeast cells (for example diploid yeast such as diploid Pichia) and other yeast strains. Suitable Pichia species include, but are not limited to, Pichia pastoris.

### Antibody Ab5

The description contemplates methods of treating pain and the specific pain associated disorders alone or is association with another active agent, e.g., an NSAID or opioid analgesic, wherein the antibodies optionally include Ab5 or fragments thereof, or another antibody or antibody fragment that binds to the same or overlapping epitope as Ab5, for example as set forth below, in a therapeutically effective amount which inhibits the association of NGF with TrkA and the association of NGF with p75. In one example, the description includes chimeric antibodies having binding specificity to NGF and possessing a variable light chain sequence comprising the sequence set forth below:

The description also optionally includes chimeric antibodies for treatment or prevention of pain and pain associated conditions having binding specificity to NGF and possessing a light chain sequence comprising the sequence set forth below:

The description further optionally includes chimeric antibodies for treatment or prevention of pain and pain associated conditions having binding specificity to NGF and possessing a variable heavy chain sequence comprising the sequence set forth below:

The description also optionally includes chimeric antibodies for treatment or prevention of pain and pain associated conditions having binding specificity to NGF and possessing a heavy chain sequence comprising the sequence set forth below:

The description further optionally contemplates antibodies for treatment or prevention of pain and pain associated conditions comprising one or more of the polypeptide sequences of SEQ ID NO: 45; SEQ ID NO: 46; and SEQ ID NO: 47 which correspond to the complementarity-determining regions (CDRs, or hypervariable regions) of the variable light chain sequence of SEQ ID NO: 41 or the light chain sequence of SEQ ID NO: 42, and/or one or more of the polypeptide sequences of SEQ ID NO: 48; SEQ ID NO: 49; and SEQ ID NO: 50 which correspond to the complementarity-determining regions (CDRs, or hypervariable regions) of the variable heavy chain sequence of SEQ ID NO: 43 or the heavy chain sequence of SEQ ID NO: 44, or combinations of these polypeptide sequences. In another example of the description, the antibodies of the description or fragments thereof optionally comprise, or alternatively consist of, combinations of one or more of the CDRs, the variable heavy and variable light chain sequences, and the heavy and light chain sequences set forth above, including all of them.

The description also optionally contemplates fragments of the antibody having binding specificity to NGF for treatment or prevention of pain and pain associated conditions. In one example of the description, antibody fragments of the description comprise, or alternatively consist of, the polypeptide sequence of SEQ ID NO: 41 or SEQ ID NO: 42. In another optional example of the description, antibody fragments of the description for treatment or prevention of pain and pain associated conditions comprise, or alternatively consist of, the polypeptide sequence of SEQ ID NO: 43 or SEQ ID NO: 44.

In a further example of the description, fragments of the antibody having binding specificity to NGF comprise, or alternatively consist of, one or more of the polypeptide sequences of SEQ ID NO: 45; SEQ ID NO: 46; and SEQ ID NO: 47 which correspond to the complementarity-determining regions (CDRs, or hypervariable regions) of the variable light chain sequence of SEQ ID NO: 41 or the light chain sequence of SEQ ID NO: 42.

In a further optional example of the description, fragments of the antibody having binding specificity to NGF for treatment or prevention of pain and pain associated conditions optionally comprise, or alternatively consist of, one or more of the polypeptide sequences of SEQ ID NO: 48; SEQ ID NO: 49; and SEQ ID NO: 50 which correspond to the complementarity-determining regions (CDRs, or hypervariable regions) of the variable heavy chain sequence of SEQ ID NO: 43 or the heavy chain sequence of SEQ ID NO: 44.

The description also optionally contemplates antibody fragments for treatment or prevention of pain and pain associated conditions which include one or more of the antibody fragments described herein. In one example of the description, fragments of the antibodies having binding specificity to NGF comprise, or alternatively consist of, one, two, three or more, including all of the following antibody fragments: the variable light chain region of SEQ ID NO: 41; the variable heavy chain region of SEQ ID NO: 43; the complementarity-determining regions (SEQ ID NO: 45; SEQ ID NO: 46; and SEQ ID NO: 47) of the variable light chain region of SEQ ID NO: 41; and the complementarity-determining regions (SEQ ID NO: 48; SEQ ID NO: 49; and SEQ ID NO: 50) of the variable heavy chain region of SEQ ID NO: 43.

In a particularly preferred optional example of the description, the optionally included chimeric anti-NGF antibody for treatment or prevention of pain and pain associated conditions is Ab5, comprising, or alternatively consisting of, SEQ ID NO: 42 and SEQ ID NO: 44, and having at least one of the biological activities set forth herein.

In a further particularly preferred optional example of the description, antibody fragments for treatment or prevention of pain and pain associated conditions comprise, or alternatively consist of, Fab (fragment antigen binding) fragments having binding specificity for NGF or another Fab or antibody fragment that binds to the same or overlapping epitope as Ab5. With respect to antibody Ab5, the Fab fragment includes the variable light chain sequence of SEQ ID NO: 41 and the variable heavy chain sequence of SEQ ID NO: 43. This example of the description further contemplates additions, deletions, and variants of SEQ ID NO: 41 and/or SEQ ID NO: 43 in said Fab while retaining binding specificity for NGF.

In one example of the description described herein (infra), Fab fragments for treatment or prevention of pain and pain associated conditions may be produced by enzymatic digestion (e.g., papain) of Ab5. In another example of the description, anti-NGF antibodies such as Ab5 or Fab fragments thereof may be produced via expression in mammalian cells such as CHO, NSO or HEK 293 cells, fungal, insect, plant or microbial systems such as yeast cells (for example diploid yeast such as diploid Pichia) and other yeast strains. Suitable Pichia species include, but are not limited to, Pichia pastoris.

### Antibody Ab6

The description optionally contemplates methods of treating pain and the specific pain associated disorders alone or is association with another active agent, e.g., an NSAID or opioid analgesic, wherein the antibodies include Ab6 or fragments thereof, or another antibody or antibody fragment that binds to the same or overlapping epitope as Ab6, for example as set forth below, in a therapeutically effective amount which inhibits the association of NGF with TrkA and the association of NGF with p75. In one example, the description includes chimeric or humanized antibodies having binding specificity to NGF and possessing a variable light chain sequence comprising the sequence set forth below:

The description also optionally includes chimeric or humanized antibodies for treatment or prevention of pain and pain associated conditions having binding specificity to NGF and possessing a light chain sequence comprising the sequence set forth below:

The description further optionally includes chimeric or humanized antibodies for treatment or prevention of pain and pain associated conditions having binding specificity to NGF and possessing a variable heavy chain sequence comprising the sequence set forth below:

The description also optionally includes chimeric or humanized antibodies for treatment or prevention of pain and pain associated conditions having binding specificity to NGF and possessing a heavy chain sequence comprising the sequence set forth below:

The description further optionally contemplates antibodies for treatment or prevention of pain and pain associated conditions comprising one or more of the polypeptide sequences of SEQ ID NO: 55; SEQ ID NO: 56; and SEQ ID NO: 57 which correspond to the complementarity-determining regions (CDRs, or hypervariable regions) of the variable light chain sequence of SEQ ID NO: 51 or the light chain sequence of SEQ ID NO: 52, and/or one or more of the polypeptide sequences of SEQ ID NO: 58; SEQ ID NO: 59; and SEQ ID NO: 60 which correspond to the complementarity-determining regions (CDRs, or hypervariable regions) of the variable heavy chain sequence of SEQ ID NO: 53 or the heavy chain sequence of SEQ ID NO: 54, or combinations of these polypeptide sequences. In another optional example of the description, the antibodies of the description or fragments thereof comprise, or alternatively consist of, combinations of one or more of the CDRs, the variable heavy and variable light chain sequences, and the heavy and light chain sequences set forth above, including all of them.

The description also optionally contemplates fragments of the antibody for treatment or prevention of pain and pain associated conditions having binding specificity to NGF. In one example of the description, antibody fragments of the description comprise, or alternatively consist of, the polypeptide sequence of SEQ ID NO: 51 or SEQ ID NO: 52. In another example of the description, antibody fragments of the description comprise, or alternatively consist of, the polypeptide sequence of SEQ ID NO: 53 or SEQ ID NO: 54.

In a further optional example of the description, fragments of the antibody having binding specificity to NGF comprise, or alternatively consist of, one or more of the polypeptide sequences of SEQ ID NO: 55; SEQ ID NO: 56; and SEQ ID NO: 57 which correspond to the complementarity-determining regions (CDRs, or hypervariable regions) of the variable light chain sequence of SEQ ID NO: 51 or the light chain sequence of SEQ ID NO: 52.

In a further optional example of the description, fragments of the antibody having binding specificity to NGF for treatment or prevention of pain and pain associated conditions comprise, or alternatively consist of, one or more of the polypeptide sequences of SEQ ID NO: 58; SEQ ID NO: 59; and SEQ ID NO: 60 which correspond to the complementarity-determining regions (CDRs, or hypervariable regions) of the variable heavy chain sequence of SEQ ID NO: 53 or the heavy chain sequence of SEQ ID NO: 54.

The description also optionally contemplates antibody fragments for treatment or prevention of pain and pain associated conditions which include one or more of the antibody fragments described herein. In one example of the description, fragments of the antibodies having binding specificity to NGF comprise, or alternatively consist of, one, two, three or more, including all of the following antibody fragments: the variable light chain region of SEQ ID NO: 51; the variable heavy chain region of SEQ ID NO: 53; the complementarity-determining regions (SEQ ID NO: 55; SEQ ID NO: 56; and SEQ ID NO: 57) of the variable light chain region of SEQ ID NO: 51; and the complementarity-determining regions (SEQ ID NO: 58; SEQ ID NO: 59; and SEQ ID NO: 60) of the variable heavy chain region of SEQ ID NO: 53.

In a particularly preferred optional example of the description, the chimeric or humanized anti-NGF antibody for treatment or prevention of pain and pain associated conditions is Ab6, comprising, or alternatively consisting of, SEQ ID NO: 52 and SEQ ID NO: 54, and having at least one of the biological activities set forth herein.

In a further particularly preferred optional example of the description, antibody fragments for treatment or prevention of pain and pain associated conditions comprise, or alternatively consist of, Fab (fragment antigen binding) fragments having binding specificity for NGF or another Fab or monovalent antibody fragment that binds to the same or overlapping epitope as Ab6. With respect to antibody Ab6, the Fab fragment includes the variable light chain sequence of SEQ ID NO: 51 and the variable heavy chain sequence of SEQ ID NO: 53. This optional example of the description further contemplates additions, deletions, and variants of SEQ ID NO: 51 and/or SEQ ID NO: 53 in said Fab while retaining binding specificity for NGF.

In one optional example of the description described herein (infra), Fab fragments for treatment or prevention of pain and pain associated conditions may be produced by enzymatic digestion (e.g., papain) of Ab6. In another example of the description, anti-NGF antibodies such as Ab6 or Fab fragments thereof may be produced via expression in mammalian cells such as CHO, NSO or HEK 293 cells, fungal, insect, plant or microbial systems such as yeast cells (for example diploid yeast such as diploid Pichia) and other yeast strains. Suitable Pichia species include, but are not limited to, Pichia pastoris.

### Antibody Ab7

The description optionally contemplates methods of treating pain and the specific pain associated disorders alone or is association with another active agent, e.g., an NSAID or opioid analgesic, wherein the antibodies include Ab7 or fragments thereof, or another antibody or antibody fragment that binds to the same or overlapping epitope as Ab7, for example as set forth below, in a therapeutically effective amount which inhibits the association of NGF with TrkA and the association of NGF with p75. In one example, the description, includes chimeric antibodies for treatment or prevention of pain and pain associated conditions having binding specificity to NGF and possessing a variable light chain sequence comprising the sequence set forth below:

The description also optionally includes chimeric antibodies for treatment or prevention of pain and pain associated conditions having binding specificity to NGF and possessing a light chain sequence comprising the sequence set forth below:

The description further optionally includes chimeric antibodies for treatment or prevention of pain and pain associated conditions having binding specificity to NGF and possessing a variable heavy chain sequence comprising the sequence set forth below:

The description also optionally includes chimeric antibodies for treatment or prevention of pain and pain associated conditions having binding specificity to NGF and possessing a heavy chain sequence comprising the sequence set forth below:

The description further optionally contemplates antibodies for treatment or prevention of pain and pain associated conditions comprising one or more of the polypeptide sequences of SEQ ID NO: 65; SEQ ID NO: 66; and SEQ ID NO: 67 which correspond to the complementarity-determining regions (CDRs, or hypervariable regions) of the variable light chain sequence of SEQ ID NO: 61 or the light chain sequence of SEQ ID NO: 62, and/or one or more of the polypeptide sequences of SEQ ID NO: 68; SEQ ID NO: 69; and SEQ ID NO: 70 which correspond to the complementarity-determining regions (CDRs, or hypervariable regions) of the variable heavy chain sequence of SEQ ID NO: 63 or the heavy chain sequence of SEQ ID NO: 64, or combinations of these polypeptide sequences. In another example of the description, the antibodies of the description or fragments thereof comprise, or alternatively consist of, combinations of one or more of the CDRs, the variable heavy and variable light chain sequences, and the heavy and light chain sequences set forth above, including all of them.

The description also optionally contemplates fragments of the antibody for treatment or prevention of pain and pain associated conditions having binding specificity to NGF. In one (example of the description, antibody fragments of the description comprise, or alternatively consist of, the polypeptide sequence of SEQ ID NO: 61 or SEQ ID NO: 62. In another example of the description, antibody fragments of the description comprise, or alternatively consist of, the polypeptide sequence of SEQ ID NO: 63 or SEQ ID NO: 64.

In a further optional example of the description, fragments of the antibody for treatment or prevention of pain and pain associated conditions having binding specificity to NGF comprise, or alternatively consist of, one or more of the polypeptide sequences of SEQ ID NO: 65; SEQ ID NO: 66; and SEQ ID NO: 67 which correspond to the complementarity-determining regions (CDRs, or hypervariable regions) of the variable light chain sequence of SEQ ID NO: 61 or the light chain sequence of SEQ ID NO: 62.

In a further optional example of the description, fragments of the antibody for treatment or prevention of pain and pain associated conditions having binding specificity to NGF comprise, or alternatively consist of, one or more of the polypeptide sequences of SEQ ID NO: 68; SEQ ID NO: 69; and SEQ ID NO: 70 which correspond to the complementarity-determining regions (CDRs, or hypervariable regions) of the variable heavy chain sequence of SEQ ID NO: 63 or the heavy chain sequence of SEQ ID NO: 64.

The description also optionally contemplates antibody fragments for treatment or prevention of pain and pain associated conditions which include one or more of the antibody fragments described herein. In one example of the description, fragments of the antibodies having binding specificity to NGF comprise, or alternatively consist of, one, two, three or more, including all of the following antibody fragments: the variable light chain region of SEQ ID NO: 61; the variable heavy chain region of SEQ ID NO: 63; the complementarity-determining regions (SEQ ID NO: 65; SEQ ID NO: 66; and SEQ ID NO: 67) of the variable light chain region of SEQ ID NO: 61; and the complementarity-determining regions (SEQ ID NO: 68; SEQ ID NO: 69; and SEQ ID NO: 70) of the variable heavy chain region of SEQ ID NO: 63.

In a particularly preferred optional example of the description, the chimeric anti-NGF antibody for treatment or prevention of pain and pain associated conditions is Ab7, comprising, or alternatively consisting of, SEQ ID NO: 62 and SEQ ID NO: 64, and having at least one of the biological activities set forth herein.

In a further particularly optionally preferred example of the description, antibody fragments for treatment or prevention of pain and pain associated conditions comprise, or alternatively consist of, Fab (fragment antigen binding) fragments having binding specificity for NGF or another Fab or monovalent antibody fragment that binds to the same or overlapping epitope as Ab7. With respect to antibody Ab7, the Fab fragment includes the variable light chain sequence of SEQ ID NO: 61 and the variable heavy chain sequence of SEQ ID NO: 63. This example of the description, further contemplates additions, deletions, and variants of SEQ ID NO: 61 and/or SEQ ID NO: 63 in said Fab while retaining binding specificity for NGF.

In one optional example of the description, described herein (infra), Fab fragments for treatment or prevention of pain and pain associated conditions may be produced by enzymatic digestion (e.g., papain) of Ab7. In another example of the description, anti-NGF antibodies such as Ab7 or Fab fragments thereof may be produced via expression in mammalian cells such as CHO, NSO or HEK 293 cells, fungal, insect, plant or microbial systems such as yeast cells (for example diploid yeast such as diploid Pichia) and other yeast strains. Suitable Pichia species include, but are not limited to, Pichia pastoris.

### Antibody Ab8

The description optionally contemplates methods of treating pain and the specific pain associated disorders alone or is association with another active agent, e.g., an NSAID or opioid analgesic, wherein the antibodies include Ab8 or fragments thereof, or another antibody or fragment that binds to the same or overlapping epitope as Ab8, for example as set forth below, in a therapeutically effective amount which inhibits the association of NGF with TrkA and the association of NGF with p75. In one example, the description, includes chimeric or humanized antibodies having binding specificity to NGF and possessing a variable light chain sequence comprising the sequence set forth below:

The description also optionally includes chimeric or humanized antibodies for treatment or prevention of pain and pain associated conditions having binding specificity to NGF and possessing a light chain sequence comprising the sequence set forth below:

The description further optionally includes chimeric or humanized antibodies for treatment or prevention of pain and pain associated conditions having binding specificity to NGF and possessing a variable heavy chain sequence comprising the sequence set forth below:

The description also optionally includes chimeric or humanized antibodies for treatment or prevention of pain and pain associated conditions having binding specificity to NGF and possessing a heavy chain sequence comprising the sequence set forth below:

The description further optionally contemplates antibodies for treatment or prevention of pain and pain associated conditions comprising one or more of the polypeptide sequences of SEQ ID NO: 75; SEQ ID NO: 76; and SEQ ID NO: 77 which correspond to the complementarity-determining regions (CDRs, or hypervariable regions) of the variable light chain sequence of SEQ ID NO: 71 or the light chain sequence of SEQ ID NO: 72, and/or one or more of the polypeptide sequences of SEQ ID NO: 78; SEQ ID NO: 79; and SEQ ID NO: 80 which correspond to the complementarity-determining regions (CDRs, or hypervariable regions) of the variable heavy chain sequence of SEQ ID NO: 73 or the heavy chain sequence of SEQ ID NO: 74, or combinations of these polypeptide sequences. In another optional example of the description, the antibodies of the description, or fragments thereof comprise, or alternatively consist of, combinations of one or more of the CDRs, the variable heavy and variable light chain sequences, and the heavy and light chain sequences set forth above, including all of them.

The description also optionally contemplates fragments of the antibody for treatment or prevention of pain and pain associated conditions having binding specificity to NGF. In one example of the description, antibody fragments of the description comprise, or alternatively consist of, the polypeptide sequence of SEQ ID NO: 71 or SEQ ID NO: 72. In another example of the description, antibody fragments of the description comprise, or alternatively consist of, the polypeptide sequence of SEQ ID NO: 73 or SEQ ID NO: 74.

In a further optional example of the description, fragments of the antibody having binding specificity to NGF for treatment or prevention of pain and pain associated conditions comprise, or alternatively consist of, one or more of the polypeptide sequences of SEQ ID NO: 75; SEQ ID NO: 76; and SEQ ID NO: 77 which correspond to the complementarity-determining regions (CDRs, or hypervariable regions) of the variable light chain sequence of SEQ ID NO: 71 or the light chain sequence of SEQ ID NO: 72.

In a further optional example of the description, fragments of the antibody having binding specificity to NGF for treatment or prevention of pain and pain associated conditions comprise, or alternatively consist of, one or more of the polypeptide sequences of SEQ ID NO: 78; SEQ ID NO: 79; and SEQ ID NO: 80 which correspond to the complementarity-determining regions (CDRs, or hypervariable regions) of the variable heavy chain sequence of SEQ ID NO: 73 or the heavy chain sequence of SEQ ID NO: 74.

The description also optionally contemplates antibody fragments for treatment or prevention of pain and pain associated conditions which include one or more of the antibody fragments described herein. In one example of the description, fragments of the antibodies having binding specificity to NGF comprise, or alternatively consist of, one, two, three or more, including all of the following antibody fragments: the variable light chain region of SEQ ID NO: 71; the variable heavy chain region of SEQ ID NO: 73; the complementarity-determining regions (SEQ ID NO: 75; SEQ ID NO: 76; and SEQ ID NO: 77) of the variable light chain region of SEQ ID NO: 71; and the complementarity-determining regions (SEQ ID NO: 78; SEQ ID NO: 79; and SEQ ID NO: 80) of the variable heavy chain region of SEQ ID NO: 73.

In a particularly preferred optional example of the description, the chimeric or humanized anti-NGF antibody for treatment or prevention of pain and pain associated conditions is Ab8, comprising, or alternatively consisting of, SEQ ID NO: 72 and SEQ ID NO: 74, and having at least one of the biological activities set forth herein.

In a further particularly preferred optional example of the description, antibody fragments for treatment or prevention of pain and pain associated conditions comprise, or alternatively consist of, Fab (fragment antigen binding) fragments having binding specificity for NGF. With respect to antibody Ab8, the Fab fragment includes the variable light chain sequence of SEQ ID NO: 71 and the variable heavy chain sequence of SEQ ID NO: 73 or another Fab or monovalent antibody fragment that binds to the same or overlapping epitope as Ab8. This embodiment of the invention further contemplates additions, deletions, and variants of SEQ ID NO: 71 and/or SEQ ID NO: 73 in said Fab while retaining binding specificity for NGF.

In one optional example of the description described herein (infra), Fab fragments for treatment or prevention of pain and pain associated conditions may be produced by enzymatic digestion (e.g., papain) of Ab8. In another example of the description, anti-NGF antibodies such as Ab8 or Fab fragments thereof may be produced via expression in mammalian cells such as CHO, NSO or HEK 293 cells, fungal, insect, plant or microbial systems such as yeast cells (for example diploid yeast such as diploid Pichia) and other yeast strains. Suitable Pichia species include, but are not limited to, Pichia pastoris.

### Antibody Ab9

The description optionally contemplates methods of treating pain and the specific pain associated disorders alone or is association with another active agent, e.g., an NSAID or opioid analgesic, wherein the antibodies include Ab9 or fragments thereof, or another antibody or antibody fragment that binds to the same or overlapping epitope as Ab9, for example as set forth below, in a therapeutically effective amount which inhibits the association of NGF with TrkA and the association of NGF with p75. In one optional example, the description, includes chimeric antibodies for treatment or prevention of pain and pain associated conditions having binding specificity to NGF and possessing a variable light chain sequence comprising the sequence set forth below:

The description also optionally includes chimeric antibodies for treatment or prevention of pain and pain associated conditions having binding specificity to NGF and possessing a light chain sequence comprising the sequence set forth below:

The description further optionally includes chimeric antibodies for treatment or prevention of pain and pain associated conditions having binding specificity to NGF and possessing a variable heavy chain sequence comprising the sequence set forth below:

The description also optionally includes chimeric antibodies for treatment or prevention of pain and pain associated conditions having binding specificity to NGF and possessing a heavy chain sequence comprising the sequence set forth below:

The description further optionally contemplates antibodies for treatment or prevention of pain and pain associated conditions comprising one or more of the polypeptide sequences of SEQ ID NO: 85; SEQ ID NO: 86; and SEQ ID NO: 87 which correspond to the complementarity-determining regions (CDRs, or hypervariable regions) of the variable light chain sequence of SEQ ID NO: 81 or the light chain sequence of SEQ ID NO: 82, and/or one or more of the polypeptide sequences of SEQ ID NO: 88; SEQ ID NO: 89; and SEQ ID NO: 90 which correspond to the complementarity-determining regions (CDRs, or hypervariable regions) of the variable heavy chain sequence of SEQ ID NO: 83 or the heavy chain sequence of SEQ ID NO: 84, or combinations of these polypeptide sequences. In another optional example of the description, the antibodies of the description or fragments thereof comprise, or alternatively consist of, combinations of one or more of the CDRs, the variable heavy and variable light chain sequences, and the heavy and light chain sequences set forth above, including all of them.

The description also optionally contemplates fragments of the antibody for treatment or prevention of pain and pain associated conditions having binding specificity to NGF. In one example of the description, antibody fragments of the description comprise, or alternatively consist of, the polypeptide sequence of SEQ ID NO: 81 or SEQ ID NO: 82. In another example of the description, antibody fragments of the invention comprise, or alternatively consist of, the polypeptide sequence of SEQ ID NO: 83 or SEQ ID NO: 84.

In a further optional example of the description, fragments of the antibody having binding specificity to NGF for treatment or prevention of pain and pain associated conditions comprise, or alternatively consist of, one or more of the polypeptide sequences of SEQ ID NO: 85; SEQ ID NO: 86; and SEQ ID NO: 87 which correspond to the complementarity-determining regions (CDRs, or hypervariable regions) of the variable light chain sequence of SEQ ID NO: 81 or the light chain sequence of SEQ ID NO: 82.

In a further optional example of the description, fragments of the antibody having binding specificity to NGF for treatment or prevention of pain and pain associated conditions comprise, or alternatively consist of, one or more of the polypeptide sequences of SEQ ID NO: 88; SEQ ID NO: 89; and SEQ ID NO: 90 which correspond to the complementarity-determining regions (CDRs, or hypervariable regions) of the variable heavy chain sequence of SEQ ID NO: 83 or the heavy chain sequence of SEQ ID NO: 84.

The description also optionally contemplates antibody fragments for treatment or prevention of pain and pain associated conditions which include one or more of the antibody fragments described herein. In one example of the description, fragments of the antibodies having binding specificity to NGF comprise, or alternatively consist of, one, two, three or more, including all of the following antibody fragments: the variable light chain region of SEQ ID NO: 81; the variable heavy chain region of SEQ ID NO: 83; the complementarity-determining regions (SEQ ID NO: 85; SEQ ID NO: 86; and SEQ ID NO: 87) of the variable light chain region of SEQ ID NO: 81; and the complementarity-determining regions (SEQ ID NO: 88; SEQ ID NO: 89; and SEQ ID NO: 90) of the variable heavy chain region of SEQ ID NO: 83.

In a particularly preferred optional example of the description, the chimeric anti-NGF antibody for treatment or prevention of pain and pain associated conditions is Ab9, comprising, or alternatively consisting of, SEQ ID NO: 82 and SEQ ID NO: 84, and having at least one of the biological activities set forth herein.

In a further particularly preferred optional example of the description, antibody fragments for treatment or prevention of pain and pain associated conditions comprise, or alternatively consist of, Fab (fragment antigen binding) fragments having binding specificity for NGF. With respect to antibody Ab9, the Fab fragment includes the variable light chain sequence of SEQ ID NO: 81 and the variable heavy chain sequence of SEQ ID NO: 83 or another Fab or monovalent antibody fragment that binds to the same or overlapping epitope as Ab9. This example of the description further contemplates additions, deletions, and variants of SEQ ID NO: 81 and/or SEQ ID NO: 83 in said Fab while retaining binding specificity for NGF.

In one example of the description described herein (infra), Fab fragments for treatment or prevention of pain and pain associated conditions may be produced by enzymatic digestion (e.g., papain) of Ab9. In another optional example of the description, anti-NGF antibodies such as Ab9 or Fab fragments thereof may be produced via expression in mammalian cells such as CHO, NSO or HEK 293 cells, fungal, insect, plant or microbial systems such as yeast cells (for example diploid yeast such as diploid Pichia) and other yeast strains. Suitable Pichia species include, but are not limited to, Pichia pastoris.

### Antibody Ab10

The description optionally contemplates methods of treating pain and the specific pain associated disorders alone or is association with another active agent, e.g., an NSAID or opioid analgesic, wherein the antibodies include Ab10 or fragments thereof, or another antibody or antibody fragment that binds to the same or overlapping epitope as Ab10, for example as set forth below, in a therapeutically effective amount which inhibits the association of NGF with TrkA and the association of NGF with p75. In one example, the description includes chimeric or humanized antibodies for treatment or prevention of pain and pain associated conditions having binding specificity to NGF and possessing a variable light chain sequence comprising the sequence set forth below:

The description also optionally includes chimeric or humanized antibodies for treatment or prevention of pain and pain associated conditions having binding specificity to NGF and possessing a light chain sequence comprising the sequence set forth below:

The description further optionally includes chimeric or humanized antibodies for treatment or prevention of pain and pain associated conditions having binding specificity to NGF and possessing a variable heavy chain sequence comprising the sequence set forth below:

The description also optionally includes chimeric or humanized antibodies for treatment or prevention of pain and pain associated conditions having binding specificity to NGF and possessing a heavy chain sequence comprising the sequence set forth below:

The description further optionally contemplates antibodies for treatment or prevention of pain and pain associated conditions comprising one or more of the polypeptide sequences of SEQ ID NO: 95; SEQ ID NO: 96; and SEQ ID NO: 97 which correspond to the complementarity-determining regions (CDRs, or hypervariable regions) of the variable light chain sequence of SEQ ID NO: 91 or the light chain sequence of SEQ ID NO: 92, and/or one or more of the polypeptide sequences of SEQ ID NO: 98; SEQ ID NO: 99; and SEQ ID NO: 100 which correspond to the complementarity-determining regions (CDRs, or hypervariable regions) of the variable heavy chain sequence of SEQ ID NO: 93 or the heavy chain sequence of SEQ ID NO: 94, or combinations of these polypeptide sequences. In another example of the description, the antibodies of the description or fragments thereof comprise, or alternatively consist of, combinations of one or more of the CDRs, the variable heavy and variable light chain sequences, and the heavy and light chain sequences set forth above, including all of them.

The description also optionally contemplates fragments of the antibody for treatment or prevention of pain and pain associated conditions having binding specificity to NGF. In one example of the description, antibody fragments of the description comprise, or alternatively consist of, the polypeptide sequence of SEQ ID NO: 91 or SEQ ID NO: 92. In another example of the description, antibody fragments of the description comprise, or alternatively consist of, the polypeptide sequence of SEQ ID NO: 93 or SEQ ID NO: 94.

In a further example of the description, fragments of the antibody having binding specificity to NGF for treatment or prevention of pain and pain associated conditions optionally comprise, or alternatively consist of, one or more of the polypeptide sequences of SEQ ID NO: 95; SEQ ID NO: 96; and SEQ ID NO: 97 which correspond to the complementarity-determining regions (CDRs, or hypervariable regions) of the variable light chain sequence of SEQ ID NO: 91 or the light chain sequence of SEQ ID NO: 92.

In a further optional example of the description, fragments of the antibody having binding specificity to NGF for treatment or prevention of pain and pain associated conditions comprise, or alternatively consist of, one or more of the polypeptide sequences of SEQ ID NO: 98; SEQ ID NO: 99; and SEQ ID NO: 100 which correspond to the complementarity-determining regions (CDRs, or hypervariable regions) of the variable heavy chain sequence of SEQ ID NO: 93 or the heavy chain sequence of SEQ ID NO: 94.

The description also optionally contemplates antibody fragments for treatment or prevention of pain and pain associated conditions which include one or more of the antibody fragments described herein. In one example of the description, fragments of the antibodies for treatment or prevention of pain and pain associated conditions having binding specificity to NGF comprise, or alternatively consist of, one, two, three or more, including all of the following antibody fragments: the variable light chain region of SEQ ID NO: 91; the variable heavy chain region of SEQ ID NO: 93; the complementarity-determining regions (SEQ ID NO: 95; SEQ ID NO: 96; and SEQ ID NO: 97) of the variable light chain region of SEQ ID NO: 91; and the complementarity-determining regions (SEQ ID NO: 98; SEQ ID NO: 99; and SEQ ID NO: 100) of the variable heavy chain region of SEQ ID NO: 93.

In a particularly preferred optional example of the description, the chimeric or humanized anti-NGF antibody for treatment or prevention of pain and pain associated conditions is Ab10, comprising, or alternatively consisting of, SEQ ID NO: 92 and SEQ ID NO: 94, and having at least one of the biological activities set forth herein.

In a further particularly preferred optional example of the description, antibody fragments for treatment or prevention of pain and pain associated conditions comprise, or alternatively consist of, Fab (fragment antigen binding) fragments having binding specificity for NGF. With respect to antibody Ab10, the Fab fragment for treatment or prevention of pain and pain associated conditions includes the variable light chain sequence of SEQ ID NO: 91 and the variable heavy chain sequence of SEQ ID NO: 93 or another Fab or monovalent antibody fragment that binds to the same or overlapping epitope as Ab10. This example of the description further optionally contemplates additions, deletions, and variants of SEQ ID NO: 91 and/or SEQ ID NO: 93 in said Fab while retaining binding specificity for NGF.

In one optional example of the description described herein (infra), Fab fragments for treatment or prevention of pain and pain associated conditions may be produced by enzymatic digestion (e.g., papain) of Ab10. In another example of the description, anti-NGF antibodies such as Ab10 or Fab fragments thereof may be produced via expression in mammalian cells such as CHO, NSO or HEK 293 cells, fungal, insect, plant or microbial systems such as yeast cells (for example diploid yeast such as diploid Pichia) and other yeast strains. Suitable Pichia species include, but are not limited to, Pichia pastoris.

### Antibody Ab11

The description optionally contemplates methods of treating pain and the specific pain associated disorders alone or is association with another active agent, e.g., an NSAID or opioid analgesic, wherein the antibodies include 1 or fragments thereof, or another antibody or antibody fragment that binds to the same or overlapping epitope as Ab11, for example as set forth below, in a therapeutically effective amount which inhibits the association of NGF with TrkA and the association of NGF with p75. In one optional example, the description includes chimeric antibodies having binding specificity to NGF and possessing a variable light chain sequence comprising the sequence set forth below:

The description also optionally includes chimeric antibodies for treatment or prevention of pain and pain associated conditions having binding specificity to NGF and possessing a light chain sequence comprising the sequence set forth below:

The description further optionally includes chimeric antibodies for treatment or prevention of pain and pain associated conditions having binding specificity to NGF and possessing a variable heavy chain sequence comprising the sequence set forth below:

The description also optionally includes chimeric antibodies for treatment or prevention of pain and pain associated conditions having binding specificity to NGF and possessing a heavy chain sequence comprising the sequence set forth below:

The description further optionally contemplates antibodies for treatment or prevention of pain and pain associated conditions comprising one or more of the polypeptide sequences of SEQ ID NO: 105; SEQ ID NO: 106; and SEQ ID NO: 107 which correspond to the complementarity-determining regions (CDRs, or hypervariable regions) of the variable light chain sequence of SEQ ID NO: 101 or the light chain sequence of SEQ ID NO: 102, and/or one or more of the polypeptide sequences of SEQ ID NO: 108; SEQ ID NO: 109; and SEQ ID NO: 110 which correspond to the complementarity-determining regions (CDRs, or hypervariable regions) of the variable heavy chain sequence of SEQ ID NO: 103 or the heavy chain sequence of SEQ ID NO: 104, or combinations of these polypeptide sequences. In another optional example of the description, the antibodies of the description or fragments thereof optionally comprise, or alternatively consist of, combinations of one or more of the CDRs, the variable heavy and variable light chain sequences, and the heavy and light chain sequences set forth above, including all of them.

The description also optionally contemplates fragments of the antibody having binding specificity to NGF for treatment or prevention of pain and pain associated conditions. In one example of the description, antibody fragments of the description comprise, or alternatively consist of, the polypeptide sequence of SEQ ID NO: 101 or SEQ ID NO: 102. In another optional example of the description, antibody fragments of the description comprise, or alternatively consist of, the polypeptide sequence of SEQ ID NO: 103 or SEQ ID NO: 104.

In a further optional example of the description, fragments of the antibody having binding specificity to NGF for treatment or prevention of pain and pain associated conditions comprise, or alternatively consist of, one or more of the polypeptide sequences of SEQ ID NO: 105; SEQ ID NO: 106; and SEQ ID NO: 107 which correspond to the complementarity-determining regions (CDRs, or hypervariable regions) of the variable light chain sequence of SEQ ID NO: 101 or the light chain sequence of SEQ ID NO: 102.

In a further optional example of the description, fragments of the antibody for treatment or prevention of pain and pain associated conditions having binding specificity to NGF comprise, or alternatively consist of, one or more of the polypeptide sequences of SEQ ID NO: 108; SEQ ID NO: 109; and SEQ ID NO: 110 which correspond to the complementarity-determining regions (CDRs, or hypervariable regions) of the variable heavy chain sequence of SEQ ID NO: 103 or the heavy chain sequence of SEQ ID NO: 104.

The description also optionally contemplates antibody fragments for treatment or prevention of pain and pain associated conditions which include one or more of the antibody fragments described herein. In one example of the description, fragments of the antibodies having binding specificity to NGF comprise, or alternatively consist of, one, two, three or more, including all of the following antibody fragments: the variable light chain region of SEQ ID NO: 101; the variable heavy chain region of SEQ ID NO: 103; the complementarity-determining regions (SEQ ID NO: 105; SEQ ID NO: 106; and SEQ ID NO: 107) of the variable light chain region of SEQ ID NO: 101; and the complementarity-determining regions (SEQ ID NO: 108; SEQ ID NO: 109; and SEQ ID NO: 110) of the variable heavy chain region of SEQ ID NO: 103.

In a particularly preferred optional example of the description, the chimeric anti-NGF antibody for treatment or prevention of pain and pain associated conditions is Ab11, comprising, or alternatively consisting of, SEQ ID NO: 102 and SEQ ID NO: 104, and having at least one of the biological activities set forth herein.

In a further particularly preferred optional example of the description, antibody fragments for treatment or prevention of pain and pain associated conditions comprise, or alternatively consist of, Fab (fragment antigen binding) fragments having binding specificity for NGF. With respect to antibody Ab11, the Fab fragment includes the variable light chain sequence of SEQ ID NO: 101 and the variable heavy chain sequence of SEQ ID NO: 103 or comprises another Fab or monovalent antibody fragment that binds to the same or overlapping epitope as 1. This example of the description further contemplates additions, deletions, and variants of SEQ ID NO: 101 and/or SEQ ID NO: 103 in said Fab while retaining binding specificity for NGF.

In one optional example of the description described herein (infra), Fab fragments may for treatment or prevention of pain and pain associated conditions be produced by enzymatic digestion (e.g., papain) of Ab11. In another optional example of the description, anti-NGF antibodies such as Ab11 or Fab fragments thereof may be produced via expression in mammalian cells such as CHO, NSO or HEK 293 cells, fungal, insect, plant or microbial systems such as yeast cells (for example diploid yeast such as diploid Pichia) and other yeast strains. Suitable Pichia species include, but are not limited to, Pichia pastoris.

### Antibody Ab12

The description optionally contemplates methods of treating pain and the specific pain associated disorders alone or is association with another active agent, e.g., an NSAID or opioid analgesic, wherein the antibodies include Ab12 or fragments thereof, f or another antibody or antibody fragment that binds to the same or overlapping epitope as Ab12, or example as set forth below, in a therapeutically effective amount which inhibits the association of NGF with TrkA and the association of NGF with p75. In one example, the description includes chimeric or humanized antibodies for treatment or prevention of pain and pain associated conditions having binding specificity to NGF and possessing a variable light chain sequence comprising the sequence set forth below:

The description also optionally includes chimeric or humanized antibodies for treatment or prevention of pain and pain associated conditions having binding specificity to NGF and possessing a light chain sequence comprising the sequence set forth below:

The description further optionally includes chimeric or humanized antibodies for treatment or prevention of pain and pain associated conditions having binding specificity to NGF and possessing a variable heavy chain sequence comprising the sequence set forth below:

The description also optionally includes chimeric or humanized antibodies for treatment or prevention of pain and pain associated conditions having binding specificity to NGF and possessing a heavy chain sequence comprising the sequence set forth below:

The description further optionally contemplates antibodies for treatment or prevention of pain and pain associated conditions comprising one or more of the polypeptide sequences of SEQ ID NO: 115; SEQ ID NO: 116; and SEQ ID NO: 117 which correspond to the complementarity-determining regions (CDRs, or hypervariable regions) of the variable light chain sequence of SEQ ID NO: 111 or the light chain sequence of SEQ ID NO: 112, and/or one or more of the polypeptide sequences of SEQ ID NO: 118; SEQ ID NO: 119; and SEQ ID NO: 120 which correspond to the complementarity-determining regions (CDRs, or hypervariable regions) of the variable heavy chain sequence of SEQ ID NO: 113 or the heavy chain sequence of SEQ ID NO: 114, or combinations of these polypeptide sequences. In another optional example of the description, the antibodies of the description or fragments thereof comprise, or alternatively consist of, combinations of one or more of the CDRs, the variable heavy and variable light chain sequences, and the heavy and light chain sequences set forth above, including all of them.

The description also optionally contemplates fragments of the antibody having binding specificity to NGF for treatment or prevention of pain and pain associated conditions. In one optional example of the description, antibody fragments of the description comprise, or alternatively consist of, the polypeptide sequence of SEQ ID NO: 111 or SEQ ID NO: 112. In another example of the description, antibody fragments of the description comprise, or alternatively consist of, the polypeptide sequence of SEQ ID NO: 113 or SEQ ID NO: 114.

In a further optional example of the description, fragments of the antibody having binding specificity to NGF for treatment or prevention of pain and pain associated conditions comprise, or alternatively consist of, one or more of the polypeptide sequences of SEQ ID NO: 115; SEQ ID NO: 116; and SEQ ID NO: 117 which correspond to the complementarity-determining regions (CDRs, or hypervariable regions) of the variable light chain sequence of SEQ ID NO: 111 or the light chain sequence of SEQ ID NO: 112.

In a further optional example of the description, fragments of the antibody having binding specificity to NGF for treatment or prevention of pain and pain associated conditions comprise, or alternatively consist of, one or more of the polypeptide sequences of SEQ ID NO: 118; SEQ ID NO: 119; and SEQ ID NO: 120 which correspond to the complementarity-determining regions (CDRs, or hypervariable regions) of the variable heavy chain sequence of SEQ ID NO: 113 or the heavy chain sequence of SEQ ID NO: 114.

The description also optionally contemplates antibody fragments which include one or more of the antibody fragments described herein. In one optional example of the description, fragments of the antibodies having binding specificity to NGF for treatment or prevention of pain and pain associated conditions comprise, or alternatively consist of, one, two, three or more, including all of the following antibody fragments: the variable light chain region of SEQ ID NO: 111; the variable heavy chain region of SEQ ID NO: 113; the complementarity-determining regions (SEQ ID NO: 115; SEQ ID NO: 116; and SEQ ID NO: 117) of the variable light chain region of SEQ ID NO: 111; and the complementarity-determining regions (SEQ ID NO: 118; SEQ ID NO: 119; and SEQ ID NO: 120) of the variable heavy chain region of SEQ ID NO: 113.

In a particularly preferred optional example of the description, the chimeric or humanized anti-NGF antibody for treatment or prevention of pain and pain associated conditions is Ab12, comprising, or alternatively consisting of, SEQ ID NO: 112 and SEQ ID NO: 114, and having at least one of the biological activities set forth herein.

In a further particularly preferred optional example of the description, antibody fragments for treatment or prevention of pain and pain associated conditions comprise, or alternatively consist of, Fab (fragment antigen binding) fragments having binding specificity for NGF. With respect to antibody Ab12, the Fab fragment includes the variable light chain sequence of SEQ ID NO: 111 and the variable heavy chain sequence of SEQ ID NO: 113 or comprises another Fab or monovalent antibody fragment that binds to the same or overlapping epitope as Ab12. This optional example of the description further contemplates additions, deletions, and variants of SEQ ID NO: 111 and/or SEQ ID NO: 113 in said Fab while retaining binding specificity for NGF.

In one optional example of the description described herein (infra), Fab fragments for treatment or prevention of pain and pain associated conditions may be produced by enzymatic digestion (e.g., papain) of Ab12. In another example of the description, anti-NGF antibodies such as Ab12 or Fab fragments thereof for treatment or prevention of pain and pain associated conditions may be produced via expression in mammalian cells such as CHO, NSO or HEK 293 cells, fungal, insect, plant or microbial systems such as yeast cells (for example diploid yeast such as diploid Pichia) and other yeast strains. Suitable Pichia species include, but are not limited to, Pichia pastoris.

### Antibody Ab13

The description optionally contemplates methods of treating pain and the specific pain associated disorders alone or is association with another active agent, e.g., an NSAID or opioid analgesic, wherein the antibodies include Ab13 or fragments thereof, or another antibody or antibody fragment that binds to the same or overlapping epitope as Ab13, for example as set forth below, in a therapeutically effective amount which inhibits the association of NGF with TrkA and the association of NGF with p75. In one example, the description includes chimeric antibodies for treatment or prevention of pain and pain associated conditions having binding specificity to NGF and possessing a variable light chain sequence comprising the sequence set forth below:

The description also optionally includes chimeric antibodies for treatment or prevention of pain and pain associated conditions having binding specificity to NGF and possessing a light chain sequence comprising the sequence set forth below:

The description further optionally includes chimeric antibodies for treatment or prevention of pain and pain associated conditions having binding specificity to NGF and possessing a variable heavy chain sequence comprising the sequence set forth below:

The description also optionally includes chimeric antibodies for treatment or prevention of pain and pain associated conditions having binding specificity to NGF and possessing a heavy chain sequence comprising the sequence set forth below:

The description further optionally contemplates antibodies for treatment or prevention of pain and pain associated conditions comprising one or more of the polypeptide sequences of SEQ ID NO: 125; SEQ ID NO: 126; and SEQ ID NO: 127 which correspond to the complementarity-determining regions (CDRs, or hypervariable regions) of the variable light chain sequence of SEQ ID NO: 121 or the light chain sequence of SEQ ID NO: 122, and/or one or more of the polypeptide sequences of SEQ ID NO: 128; SEQ ID NO: 129; and SEQ ID NO: 130 which correspond to the complementarity-determining regions (CDRs, or hypervariable regions) of the variable heavy chain sequence of SEQ ID NO: 123 or the heavy chain sequence of SEQ ID NO: 124, or combinations of these polypeptide sequences. In another optional example of the description, the antibodies of the description or fragments thereof comprise, or alternatively consist of, combinations of one or more of the CDRs, the variable heavy and variable light chain sequences, and the heavy and light chain sequences set forth above, including all of them.

The description also optionally contemplates fragments of the antibody having binding specificity to NGF for treatment or prevention of pain and pain associated conditions. In example of the description, antibody fragments of the description comprise, or alternatively consist of, the polypeptide sequence of SEQ ID NO: 121 or SEQ ID NO: 122. In another example of the description, antibody fragments of the description comprise, or alternatively consist of, the polypeptide sequence of SEQ ID NO: 123 or SEQ ID NO: 124.

In a further optional example of the description, fragments of the antibody having binding specificity to NGF for treatment or prevention of pain and pain associated conditions comprise, or alternatively consist of, one or more of the polypeptide sequences of SEQ ID NO: 125; SEQ ID NO: 126; and SEQ ID NO: 127 which correspond to the complementarity-determining regions (CDRs, or hypervariable regions) of the variable light chain sequence of SEQ ID NO: 121 or the light chain sequence of SEQ ID NO: 122.

In a further optional example of the description, fragments of the antibody having binding specificity to NGF for treatment or prevention of pain and pain associated conditions comprise, or alternatively consist of, one or more of the polypeptide sequences of SEQ ID NO: 128; SEQ ID NO: 129; and SEQ ID NO: 130 which correspond to the complementarity-determining regions (CDRs, or hypervariable regions) of the variable heavy chain sequence of SEQ ID NO: 123 or the heavy chain sequence of SEQ ID NO: 124.

The description also optionally contemplates antibody fragments for treatment or prevention of pain and pain associated conditions which include one or more of the antibody fragments described herein. In one example of the description, fragments of the antibodies having binding specificity to NGF comprise, or alternatively consist of, one, two, three or more, including all of the following antibody fragments: the variable light chain region of SEQ ID NO: 121; the variable heavy chain region of SEQ ID NO: 123; the complementarity-determining regions (SEQ ID NO: 125; SEQ ID NO: 126; and SEQ ID NO: 127) of the variable light chain region of SEQ ID NO: 121; and the complementarity-determining regions (SEQ ID NO: 128; SEQ ID NO: 129; and SEQ ID NO: 130) of the variable heavy chain region of SEQ ID NO: 123.

In a particularly preferred optional example of the description, the chimeric anti-NGF antibody for treatment or prevention of pain and pain associated conditions is Ab13, comprising, or alternatively consisting of, SEQ ID NO: 122 and SEQ ID NO: 124, and having at least one of the biological activities set forth herein.

In a further particularly preferred optional example of the description, antibody fragments for treatment or prevention of pain and pain associated conditions comprise, or alternatively consist of, Fab (fragment antigen binding) fragments having binding specificity for NGF. With respect to antibody Abl3, the Fab fragment includes the variable light chain sequence of SEQ ID NO: 121 and the variable heavy chain sequence of SEQ ID NO: 123 or comprises another Fab or monovalent antibody fragment that binds to the same or overlapping epitope as Ab13. This optional example of the description further contemplates additions, deletions, and variants of SEQ ID NO: 121 and/or SEQ ID NO: 123 in said Fab while retaining binding specificity for NGF.

In one optional example of the description described herein (infra), Fab fragments for treatment or prevention of pain and pain associated conditions may be produced by enzymatic digestion (e.g., papain) of Ab13. In another example of the description, anti-NGF antibodies such as Ab13 or Fab fragments thereof may be produced via expression in mammalian cells such as CHO, NSO or HEK 293 cells, fungal, insect, plant or microbial systems such as yeast cells (for example diploid yeast such as diploid Pichia) and other yeast strains. Suitable Pichia species include, but are not limited to, Pichia pastoris.

### Antibody Ab14

The description optionally contemplates methods of treating pain and the specific pain associated disorders alone or is association with another active agent, e.g., an NSAID or opioid analgesic, wherein the antibodies include chimeric antibodies having binding specificity to NGF wherein the antibody is Ab14 or fragments thereof, or another antibody or fragment that binds to the same or overlapping epitope as Ab14, for example as set forth below, in a therapeutically effective amount which inhibits the association of NGF with TrkA and the association of NGF with p75. In one optional example, the description, includes chimeric or humanized antibodies having binding specificity to NGF and possessing a variable light chain sequence comprising the sequence set forth below:

The description also optionally includes chimeric or humanized antibodies for treatment or prevention of pain and pain associated conditions having binding specificity to NGF and possessing a light chain sequence comprising the sequence set forth below:

The description further optionally includes chimeric or humanized antibodies for treatment or prevention of pain and pain associated conditions having binding specificity to NGF and possessing a variable heavy chain sequence comprising the sequence set forth below:

The description also optionally includes chimeric or humanized antibodies for treatment or prevention of pain and pain associated conditions having binding specificity to NGF and possessing a heavy chain sequence comprising the sequence set forth below:

The description further optionally contemplates antibodies for treatment or prevention of pain and pain associated conditions comprising one or more of the polypeptide sequences of SEQ ID NO: 135; SEQ ID NO: 136; and SEQ ID NO: 137 which correspond to the complementarity-determining regions (CDRs, or hypervariable regions) of the variable light chain sequence of SEQ ID NO: 131 or the light chain sequence of SEQ ID NO: 132, and/or one or more of the polypeptide sequences of SEQ ID NO: 138; SEQ ID NO: 139; and SEQ ID NO: 140 which correspond to the complementarity-determining regions (CDRs, or hypervariable regions) of the variable heavy chain sequence of SEQ ID NO: 133 or the heavy chain sequence of SEQ ID NO: 134, or combinations of these polypeptide sequences. In another optional example of the description, the antibodies of the description or fragments thereof comprise, or alternatively consist of, combinations of one or more of the CDRs, the variable heavy and variable light chain sequences, and the heavy and light chain sequences set forth above, including all of them.

The description also optionally contemplates fragments of the antibody having binding specificity to NGF for treatment or prevention of pain and pain associated conditions. In one example of the description, antibody fragments of the description comprise, or alternatively consist of, the polypeptide sequence of SEQ ID NO: 131 or SEQ ID NO: 132. In another example of the description, antibody fragments of the description comprise, or alternatively consist of, the polypeptide sequence of SEQ ID NO: 133 or SEQ ID NO: 134.

In a further optional example of the description, fragments of the antibody having binding specificity to NGF for treatment or prevention of pain and pain associated conditions optionally comprise, or alternatively consist of, one or more of the polypeptide sequences of SEQ ID NO: 135; SEQ ID NO: 136; and SEQ ID NO: 137 which correspond to the complementarity-determining regions (CDRs, or hypervariable regions) of the variable light chain sequence of SEQ ID NO: 131 or the light chain sequence of SEQ ID NO: 132.

In a further optional example of the description, fragments of the antibody having binding specificity to NGF for treatment or prevention of pain and pain associated conditions comprise, or alternatively consist of, one or more of the polypeptide sequences of SEQ ID NO: 138; SEQ ID NO: 139; and SEQ ID NO: 140 which correspond to the complementarity-determining regions (CDRs, or hypervariable regions) of the variable heavy chain sequence of SEQ ID NO: 133 or the heavy chain sequence of SEQ ID NO: 134.

The description also optionally contemplates antibody fragments for treatment or prevention of pain and pain associated conditions which include one or more of the antibody fragments described herein. In one example of the description, fragments of the antibodies having binding specificity to NGF comprise, or alternatively consist of, one, two, three or more, including all of the following antibody fragments: the variable light chain region of SEQ ID NO: 131; the variable heavy chain region of SEQ ID NO: 133; the complementarity-determining regions (SEQ ID NO: 135; SEQ ID NO: 136; and SEQ ID NO: 137) of the variable light chain region of SEQ ID NO: 131; and the complementarity-determining regions (SEQ ID NO: 138; SEQ ID NO: 139; and SEQ ID NO: 140) of the variable heavy chain region of SEQ ID NO: 133.

In a particularly preferred optional example of the description, the chimeric or humanized anti-NGF antibody for treatment or prevention of pain and pain associated conditions is Ab14, comprising, or alternatively consisting of, SEQ ID NO: 132 and SEQ ID NO: 134, and having at least one of the biological activities set forth herein.

In a further particularly preferred optional example of the description, antibody fragments for treatment or prevention of pain and pain associated conditions comprise, or alternatively consist of, Fab (fragment antigen binding) fragments having binding specificity for NGF. With respect to antibody Ab14, the Fab fragment includes the variable light chain sequence of SEQ ID NO: 131 and the variable heavy chain sequence of SEQ ID NO: 133 or another Fab or monovalent antibody fragment that binds to the same or overlapping epitope as

Ab14. This example of the description further contemplates additions, deletions, and variants of SEQ ID NO: 131 and/or SEQ ID NO: 133 in said Fab while retaining binding specificity for NGF.

In one optional example of the description described herein (infra), Fab fragments for treatment or prevention of pain and pain associated conditions may be produced by enzymatic digestion (e.g., papain) of Ab14. In another example of the description, anti-NGF antibodies such as Ab14 or Fab fragments thereof for treatment or prevention of pain and pain associated conditions may be produced via expression in mammalian cells such as CHO, NSO or HEK 293 cells, fungal, insect, plant or microbial systems such as yeast cells (for example diploid yeast such as diploid Pichia) and other yeast strains. Suitable Pichia species include, but are not limited to, Pichia pastoris.

### Antibody Ab15

The description contemplates methods of treating pain and the specific pain associated disorders alone or is association with another active agent, e.g., an NSAID or opioid analgesic, wherein the antibodies include chimeric antibodies having binding specificity to NGF wherein the antibody is Ab15 or fragments thereof, for example as set forth below, or comprises another antibody or antibody fragment that binds to the same or overlapping epitope as Ab15, in a therapeutically effective amount which inhibits the association of NGF with TrkA without appreciably inhibiting the association of NGF with p75. In one example, the description includes chimeric antibodies for treatment or prevention of pain and pain associated conditions having binding specificity to NGF and possessing a variable light chain sequence comprising the sequence set forth below:

The description also includes chimeric antibodies for treatment or prevention of pain and pain associated conditions having binding specificity to NGF and possessing a light chain sequence comprising the sequence set forth below:

The description further includes chimeric antibodies for treatment or prevention of pain and pain associated conditions having binding specificity to NGF and possessing a variable heavy chain sequence comprising the sequence set forth below:

The description also includes chimeric antibodies for treatment or prevention of pain and pain associated conditions having binding specificity to NGF and possessing a heavy chain sequence comprising the sequence set forth below:

The description further contemplates antibodies for treatment or prevention of pain and pain associated conditions comprising one or more of the polypeptide sequences of SEQ ID NO: 145; SEQ ID NO: 146; and SEQ ID NO: 147 which correspond to the complementarity-determining regions (CDRs, or hypervariable regions) of the variable light chain sequence of SEQ ID NO: 141 or the light chain sequence of SEQ ID NO: 142, and/or one or more of the polypeptide sequences of SEQ ID NO: 148; SEQ ID NO: 149; and SEQ ID NO: 150 which correspond to the complementarity-determining regions (CDRs, or hypervariable regions) of the variable heavy chain sequence of SEQ ID NO: 143 or the heavy chain sequence of SEQ ID NO: 144, or combinations of these polypeptide sequences. In another example of the description, the antibodies of the description or fragments thereof for treatment or prevention of pain and pain associated conditions comprise, or alternatively consist of, combinations of one or more of the CDRs, the variable heavy and variable light chain sequences, and the heavy and light chain sequences set forth above, including all of them.

The description also contemplates fragments of the antibody having binding specificity to NGF for treatment or prevention of pain and pain associated conditions. In one example of the description, antibody fragments of the description comprise, or alternatively consist of, the polypeptide sequence of SEQ ID NO: 141 or SEQ ID NO: 142. In another example of the description, antibody fragments of the description comprise, or alternatively consist of, the polypeptide sequence of SEQ ID NO: 143 or SEQ ID NO: 144.

In a further example of the description, fragments of the antibody having binding specificity to NGF for treatment or prevention of pain and pain associated conditions comprise, or alternatively consist of, one or more of the polypeptide sequences of SEQ ID NO: 145; SEQ ID NO: 146; and SEQ ID NO: 147 which correspond to the complementarity-determining regions (CDRs, or hypervariable regions) of the variable light chain sequence of SEQ ID NO: 141 or the light chain sequence of SEQ ID NO: 142.

In a further example of the description, fragments of the antibody having binding specificity to NGF for treatment or prevention of pain and pain associated conditions comprise, or alternatively consist of, one or more of the polypeptide sequences of SEQ ID NO: 148; SEQ ID NO: 149; and SEQ ID NO: 150 which correspond to the complementarity-determining regions (CDRs, or hypervariable regions) of the variable heavy chain sequence of SEQ ID NO: 143 or the heavy chain sequence of SEQ ID NO: 144.

The description also contemplates antibody fragments for treatment or prevention of pain and pain associated conditions which include one or more of the antibody fragments described herein. In one example of the description, fragments of the antibodies having binding specificity to NGF for treatment or prevention of pain and pain associated conditions comprise, or alternatively consist of, one, two, three or more, including all of the following antibody fragments: the variable light chain region of SEQ ID NO: 141; the variable heavy chain region of SEQ ID NO: 143; the complementarity-determining regions (SEQ ID NO: 145; SEQ ID NO: 146; and SEQ ID NO: 147) of the variable light chain region of SEQ ID NO: 141; and the complementarity-determining regions (SEQ ID NO: 148; SEQ ID NO: 149; and SEQ ID NO: 150) of the variable heavy chain region of SEQ ID NO: 143.

In a particularly preferred example of the description, the chimeric anti-NGF antibody for treatment or prevention of pain and pain associated conditions is Abl5, comprising, or alternatively consisting of, SEQ ID NO: 142 and SEQ ID NO: 144, and having at least one of the biological activities set forth herein.

In a further particularly preferred example of the description, antibody fragments for treatment or prevention of pain and pain associated conditions comprise, or alternatively consist of, Fab (fragment antigen binding) fragments having binding specificity for NGF. With respect to antibody Ab15, the Fab fragment includes the variable light chain sequence of SEQ ID NO: 141 and the variable heavy chain sequence of SEQ ID NO: 143 or comprises another Fab that binds to the same or overlapping epitope as Ab15. This example of the description further contemplates additions, deletions, and variants of SEQ ID NO: 141 and/or SEQ ID NO: 143 in said Fab while retaining binding specificity for NGF.

In one example of the description described herein (infra), Fab fragments for treatment or prevention of pain and pain associated conditions may be produced by enzymatic digestion (e.g., papain) of Ab15. In another example of the description, anti-NGF antibodies for treatment or prevention of pain and pain associated conditions such as Ab15 or Fab fragments thereof may be produced via expression in mammalian cells such as CHO, NSO or HEK 293 cells, fungal, insect, plant or microbial systems such as yeast cells (for example diploid yeast such as diploid Pichia) and other yeast strains. Suitable Pichia species include, but are not limited to, Pichia pastoris.

### Antibody Ab16

The description contemplates methods of treating pain and the specific pain associated disorders alone or is association with another active agent, e.g., an NSAID or opioid analgesic, wherein the antibodies include chimeric antibodies having binding specificity to NGF wherein the antibody is Ab16 or fragments thereof, for example as set forth below, or comprises another antibody or antibody fragment that binds to the same or overlapping epitope as Ab16, in a therapeutically effective amount which inhibits the association of NGF with TrkA without appreciably inhibiting the association of NGF with p75. In one example, the description includes chimeric or humanized antibodies for treatment or prevention of pain and pain associated conditions having binding specificity to NGF and possessing a variable light chain sequence comprising the sequence set forth below:

The description also includes chimeric or humanized antibodies for treatment or prevention of pain and pain associated conditions having binding specificity to NGF and possessing a light chain sequence comprising the sequence set forth below:

The description further includes chimeric or humanized antibodies for treatment or prevention of pain and pain associated conditions having binding specificity to NGF and possessing a variable heavy chain sequence comprising the sequence set forth below:

The description also includes chimeric or humanized antibodies for treatment or prevention of pain and pain associated conditions having binding specificity to NGF and possessing a heavy chain sequence comprising the sequence set forth below:

The description further contemplates antibodies for treatment or prevention of pain and pain associated conditions comprising one or more of the polypeptide sequences of SEQ ID NO: 155; SEQ ID NO: 156; and SEQ ID NO: 157 which correspond to the complementarity-determining regions (CDRs, or hypervariable regions) of the variable light chain sequence of SEQ ID NO: 151 or the light chain sequence of SEQ ID NO: 152, and/or one or more of the polypeptide sequences of SEQ ID NO: 158; SEQ ID NO: 159; and SEQ ID NO: 160 which correspond to the complementarity-determining regions (CDRs, or hypervariable regions) of the variable heavy chain sequence of SEQ ID NO: 153 or the heavy chain sequence of SEQ ID NO: 154, or combinations of these polypeptide sequences. In another example of the description, the antibodies of the description or fragments thereof for treatment or prevention of pain and pain associated conditions comprise, or alternatively consist of, combinations of one or more of the CDRs, the variable heavy and variable light chain sequences, and the heavy and light chain sequences set forth above, including all of them.

The description also contemplates fragments of the antibody having binding specificity to NGF for treatment or prevention of pain and pain associated conditions. In one example of the description, antibody fragments of the description comprise, or alternatively consist of, the polypeptide sequence of SEQ ID NO: 151 or SEQ ID NO: 152. In another example of the description, antibody fragments of the description for treatment or prevention of pain and pain associated conditions comprise, or alternatively consist of, the polypeptide sequence of SEQ ID NO: 153 or SEQ ID NO: 154.

In a further example of the description, fragments of the antibody having binding specificity to NGF for treatment or prevention of pain and pain associated conditions comprise, or alternatively consist of, one or more of the polypeptide sequences of SEQ ID NO: 155; SEQ ID NO: 156; and SEQ ID NO: 157 which correspond to the complementarity-determining regions (CDRs, or hypervariable regions) of the variable light chain sequence of SEQ ID NO: 151 or the light chain sequence of SEQ ID NO: 152.

In a further example of the description, fragments of the antibody having binding specificity to NGF for treatment or prevention of pain and pain associated conditions comprise, or alternatively consist of, one or more of the polypeptide sequences of SEQ ID NO: 158; SEQ ID NO: 159; and SEQ ID NO: 160 which correspond to the complementarity-determining regions (CDRs, or hypervariable regions) of the variable heavy chain sequence of SEQ ID NO: 153 or the heavy chain sequence of SEQ ID NO: 154.

The description also contemplates antibody fragments for treatment or prevention of pain and pain associated conditions which include one or more of the antibody fragments described herein. In one example of the description, fragments of the antibodies having binding specificity to NGF comprise, or alternatively consist of, one, two, three or more, including all of the following antibody fragments: the variable light chain region of SEQ ID NO: 151; the variable heavy chain region of SEQ ID NO: 153; the complementarity-determining regions (SEQ ID NO: 155; SEQ ID NO: 156; and SEQ ID NO: 157) of the variable light chain region of SEQ ID NO: 151; and the complementarity-determining regions (SEQ ID NO: 158; SEQ ID NO: 159; and SEQ ID NO: 160) of the variable heavy chain region of SEQ ID NO: 153.

In a particularly preferred example of the description, the chimeric or humanized anti-NGF antibody for treatment or prevention of pain and pain associated conditions is Ab16, comprising, or alternatively consisting of, SEQ ID NO: 152 and SEQ ID NO: 154, and having at least one of the biological activities set forth herein.

In a further particularly preferred example of the description, antibody fragments for treatment or prevention of pain and pain associated conditions comprise, or alternatively consist of, Fab (fragment antigen binding) fragments having binding specificity for NGF. With respect to antibody Abl6, the Fab fragment includes the variable light chain sequence of SEQ ID NO: 151 and the variable heavy chain sequence of SEQ ID NO: 153 or comprises another Fab or another bivalent or monovalent antibody fragment that binds to the same or overlapping epitope as Ab16. This example of the description further contemplates additions, deletions, and variants of SEQ ID NO: 151 and/or SEQ ID NO: 153 in said Fab while retaining binding specificity for NGF.

In one example of the description described herein (infra), Fab fragments for treatment or prevention of pain and pain associated conditions may be produced by enzymatic digestion (e.g., papain) of Ab16. In another example of the description, anti-NGF antibodies for treatment or prevention of pain and pain associated conditions such as Ab16 or Fab fragments thereof may be produced via expression in mammalian cells such as CHO, NSO or HEK 293 cells, fungal, insect, plant or microbial systems such as yeast cells (for example diploid yeast such as diploid Pichia) and other yeast strains. Suitable Pichia species include, but are not limited to, Pichia pastoris.

### Antibody Ab17

The description optionally contemplates methods of treating pain and the specific pain associated disorders alone or is association with another active agent, e.g., an NSAID or opioid analgesic, wherein the antibodies include chimeric antibodies having binding specificity to NGF wherein the antibody is Ab17 or fragments thereof, for example as set forth below, or comprises another antibody or antibody fragment that binds to the same or overlapping epitope as Ab17, in a therapeutically effective amount which inhibits the association of NGF with TrkA and the association of NGF with p75. In one example, the description includes chimeric antibodies for treatment or prevention of pain and pain associated conditions having binding specificity to NGF and possessing a variable light chain sequence comprising the sequence set forth below:

The description also optionally includes chimeric antibodies for treatment or prevention of pain and pain associated conditions having binding specificity to NGF and possessing a light chain sequence comprising the sequence set forth below:

The description, further optionally includes chimeric antibodies for treatment or prevention of pain and pain associated conditions having binding specificity to NGF and possessing a variable heavy chain sequence comprising the sequence set forth below:

The description also optionally includes chimeric antibodies for treatment or prevention of pain and pain associated conditions having binding specificity to NGF and possessing a heavy chain sequence comprising the sequence set forth below:

The description further optionally contemplates antibodies for treatment or prevention of pain and pain associated conditions comprising one or more of the polypeptide sequences of SEQ ID NO: 165; SEQ ID NO: 166; and SEQ ID NO: 167 which correspond to the complementarity-determining regions (CDRs, or hypervariable regions) of the variable light chain sequence of SEQ ID NO: 161 or the light chain sequence of SEQ ID NO: 162, and/or one or more of the polypeptide sequences of SEQ ID NO: 168; SEQ ID NO: 169; and SEQ ID NO: 170 which correspond to the complementarity-determining regions (CDRs, or hypervariable regions) of the variable heavy chain sequence of SEQ ID NO: 163 or the heavy chain sequence of SEQ ID NO: 164, or combinations of these polypeptide sequences. In another optional example of the description, the antibodies of the description or fragments thereof for treatment or prevention of pain and pain associated conditions comprise, or alternatively consist of, combinations of one or more of the CDRs, the variable heavy and variable light chain sequences, and the heavy and light chain sequences set forth above, including all of them.

The description also optionally contemplates fragments of the antibody having binding specificity to NGF for treatment or prevention of pain and pain associated conditions. In one example of the description, antibody fragments of the description comprise, or alternatively consist of, the polypeptide sequence of SEQ ID NO: 161 or SEQ ID NO: 162. In another optional example of the description, antibody fragments of the description comprise, or alternatively consist of, the polypeptide sequence of SEQ ID NO: 163 or SEQ ID NO: 164.

In a further optional example of the description, fragments of the antibody having binding specificity to NGF for treatment or prevention of pain and pain associated conditions comprise, or alternatively consist of, one or more of the polypeptide sequences of SEQ ID NO: 165; SEQ ID NO: 166; and SEQ ID NO: 167 which correspond to the complementarity-determining regions (CDRs, or hypervariable regions) of the variable light chain sequence of SEQ ID NO: 161 or the light chain sequence of SEQ ID NO: 162.

In a further optional example of the description, fragments of the antibody having binding specificity to NGF for treatment or prevention of pain and pain associated conditions comprise, or alternatively consist of, one or more of the polypeptide sequences of SEQ ID NO: 168; SEQ ID NO: 169; and SEQ ID NO: 170 which correspond to the complementarity-determining regions (CDRs, or hypervariable regions) of the variable heavy chain sequence of SEQ ID NO: 163 or the heavy chain sequence of SEQ ID NO: 164.

The description also optionally contemplates antibody fragments for treatment or prevention of pain and pain associated conditions which include one or more of the antibody fragments described herein. In one example of the description, fragments of the antibodies for treatment or prevention of pain and pain associated conditions having binding specificity to NGF comprise, or alternatively consist of, one, two, three or more, including all of the following antibody fragments: the variable light chain region of SEQ ID NO: 161; the variable heavy chain region of SEQ ID NO: 163; the complementarity-determining regions (SEQ ID NO: 165; SEQ ID NO: 166; and SEQ ID NO: 167) of the variable light chain region of SEQ ID NO: 161; and the complementarity-determining regions (SEQ ID NO: 168; SEQ ID NO: 169; and SEQ ID NO: 170) of the variable heavy chain region of SEQ ID NO: 163.

In a particularly preferred optional example of the description, the chimeric anti-NGF antibody for treatment or prevention of pain and pain associated conditions is Ab17, comprising, or alternatively consisting of, SEQ ID NO: 162 and SEQ ID NO: 164, and having at least one of the biological activities set forth herein.

In a further particularly preferred optional example of the description, antibody fragments for treatment or prevention of pain and pain associated conditions comprise, or alternatively consist of, Fab (fragment antigen binding) fragments having binding specificity for NGF. With respect to antibody Ab17, the Fab fragment includes the variable light chain sequence of SEQ ID NO: 161 and the variable heavy chain sequence of SEQ ID NO: 163 or comprises another Fab or monovalent or bivalent antibody fragment that binds to the same or overlapping epitope as Ab15,. This example of the description further contemplates additions, deletions, and variants of SEQ ID NO: 161 and/or SEQ ID NO: 163 in said Fab while retaining binding specificity for NGF.

In one optional example of the description described herein (infra), Fab fragments for treatment or prevention of pain and pain associated conditions may be produced by enzymatic digestion (e.g., papain) of Ab17. In another example of the description, anti-NGF antibodies such as Ab17 or Fab fragments thereof may be produced via expression in mammalian cells such as CHO, NSO or HEK 293 cells, fungal, insect, plant or microbial systems such as yeast cells (for example diploid yeast such as diploid Pichia) and other yeast strains. Suitable Pichia species include, but are not limited to, Pichia pastoris.

### Antibody Ab18

The description optionally contemplates methods of treating pain and the specific pain associated disorders alone or is association with another active agent, e.g., an NSAID or opioid analgesic, wherein the antibodies include chimeric antibodies having binding specificity to NGF wherein the antibody is Ab18 or fragments thereof, for example as set forth below, or comprises another antibody or antibody fragment that binds to the same or overlapping epitope as Ab18, in a therapeutically effective amount which inhibits the association of NGF with TrkA and the association of NGF with p75. In one example, the description includes chimeric or humanized antibodies for treatment or prevention of pain and pain associated conditions having binding specificity to NGF and possessing a variable light chain sequence comprising the sequence set forth below:

The description also optionally includes chimeric or humanized antibodies for treatment or prevention of pain and pain associated conditions having binding specificity to NGF and possessing a light chain sequence comprising the sequence set forth below:

The description further optionally includes chimeric or humanized antibodies for treatment or prevention of pain and pain associated conditions having binding specificity to NGF and possessing a variable heavy chain sequence comprising the sequence set forth below:

The description also optionally includes chimeric or humanized antibodies for treatment or prevention of pain and pain associated conditions having binding specificity to NGF and possessing a heavy chain sequence comprising the sequence set forth below:

The description further optionally contemplates antibodies for treatment or prevention of pain and pain associated conditions comprising one or more of the polypeptide sequences of SEQ ID NO: 175; SEQ ID NO: 176; and SEQ ID NO: 177 which correspond to the complementarity-determining regions (CDRs, or hypervariable regions) of the variable light chain sequence of SEQ ID NO: 171 or the light chain sequence of SEQ ID NO: 172, and/or one or more of the polypeptide sequences of SEQ ID NO: 178; SEQ ID NO: 179; and SEQ ID NO: 180 which correspond to the complementarity-determining regions (CDRs, or hypervariable regions) of the variable heavy chain sequence of SEQ ID NO: 173 or the heavy chain sequence of SEQ ID NO: 174, or combinations of these polypeptide sequences. In another example of the description, the antibodies of the description or fragments thereof for treatment or prevention of pain and pain associated conditions comprise, or alternatively consist of, combinations of one or more of the CDRs, the variable heavy and variable light chain sequences, and the heavy and light chain sequences set forth above, including all of them.

The description also optionally contemplates fragments of the antibody having binding specificity to NGF for treatment or prevention of pain and pain associated conditions. In one example of the description, antibody fragments of the description comprise, or alternatively consist of, the polypeptide sequence of SEQ ID NO: 171 or SEQ ID NO: 172. In another example of the description, antibody fragments of the description comprise, or alternatively consist of, the polypeptide sequence of SEQ ID NO: 173 or SEQ ID NO: 174.

In a further optional example of the description, fragments of the antibody having binding specificity to NGF for treatment or prevention of pain and pain associated conditions comprise, or alternatively consist of, one or more of the polypeptide sequences of SEQ ID NO: 175; SEQ ID NO: 176; and SEQ ID NO: 177 which correspond to the complementarity-determining regions (CDRs, or hypervariable regions) of the variable light chain sequence of SEQ ID NO: 171 or the light chain sequence of SEQ ID NO: 172.

In a further optional example of the description, fragments of the antibody having binding specificity to NGF for treatment or prevention of pain and pain associated conditions comprise, or alternatively consist of, one or more of the polypeptide sequences of SEQ ID NO: 178; SEQ ID NO: 179; and SEQ ID NO: 180 which correspond to the complementarity-determining regions (CDRs, or hypervariable regions) of the variable heavy chain sequence of SEQ ID NO: 173 or the heavy chain sequence of SEQ ID NO: 174.

The description also optional contemplates antibody fragments for treatment or prevention of pain and pain associated conditions which include one or more of the antibody fragments described herein. In one example of the description, fragments of the antibodies having binding specificity to NGF for treatment or prevention of pain and pain associated conditions comprise, or alternatively consist of, one, two, three or more, including all of the following antibody fragments: the variable light chain region of SEQ ID NO: 171; the variable heavy chain region of SEQ ID NO: 173; the complementarity-determining regions (SEQ ID NO: 175; SEQ ID NO: 176; and SEQ ID NO: 177) of the variable light chain region of SEQ ID NO: 171; and the complementarity-determining regions (SEQ ID NO: 178; SEQ ID NO: 179; and SEQ ID NO: 180) of the variable heavy chain region of SEQ ID NO: 173.

In a particularly preferred optional example of the description, the chimeric or humanized anti-NGF antibody for treatment or prevention of pain and pain associated conditions is Ab18, comprising, or alternatively consisting of, SEQ ID NO: 172 and SEQ ID NO: 174, and having at least one of the biological activities set forth herein.

In a further particularly preferred optional example of the description, antibody fragments comprise, or alternatively consist of, Fab (fragment antigen binding) fragments having binding specificity for NGF. With respect to antibody Ab18, the Fab fragment includes the variable light chain sequence of SEQ ID NO: 171 and the variable heavy chain sequence of SEQ ID NO: 173 or comprise another Fab or antibody fragment that binds to the same or overlapping epitope as Ab18. This example of the description further contemplates additions, deletions, and variants of SEQ ID NO: 171 and/or SEQ ID NO: 173 in said Fab while retaining binding specificity for NGF.

In one optional example of the description described herein (infra), Fab fragments for treatment or prevention of pain and pain associated conditions may be produced by enzymatic digestion (e.g., papain) of Ab18. In another example of the description, anti-NGF antibodies such as Ab18 or Fab fragments thereof may be produced via expression in mammalian cells such as CHO, NSO or HEK 293 cells, fungal, insect, plant or microbial systems such as yeast cells (for example diploid yeast such as diploid Pichia) and other yeast strains. Suitable Pichia species include, but are not limited to, Pichia pastoris.

### Antibody Ab19

The description optionally contemplates methods of treating pain and the specific pain associated disorders alone or is association with another active agent, e.g., an NSAID or opioid analgesic, wherein the antibodies include chimeric antibodies having binding specificity to NGF wherein the antibody is Ab19 or fragments thereof, for example as set forth below, or comprises another antibody or antibody fragment that binds to the same or overlapping epitope as Ab19, in a therapeutically effective amount which inhibits the association of NGF with TrkA and the association of NGF with p75. In one example, the description includes chimeric antibodies for treatment or prevention of pain and pain associated conditions having binding specificity to NGF and possessing a variable light chain sequence comprising the sequence set forth below:

The description also optionally includes chimeric antibodies for treatment or prevention of pain and pain associated conditions having binding specificity to NGF and possessing a light chain sequence comprising the sequence set forth below:

The description, further optionally includes chimeric antibodies for treatment or prevention of pain and pain associated conditions having binding specificity to NGF and possessing a variable heavy chain sequence comprising the sequence set forth below:

The description also optionally includes chimeric antibodies for treatment or prevention of pain and pain associated conditions having binding specificity to NGF and possessing a heavy chain sequence comprising the sequence set forth below:

The description further optionally contemplates antibodies for treatment or prevention of pain and pain associated conditions comprising one or more of the polypeptide sequences of SEQ ID NO: 185; SEQ ID NO: 186; and SEQ ID NO: 187 which correspond to the complementarity-determining regions (CDRs, or hypervariable regions) of the variable light chain sequence of SEQ ID NO: 181 or the light chain sequence of SEQ ID NO: 182, and/or one or more of the polypeptide sequences of SEQ ID NO: 188; SEQ ID NO: 189; and SEQ ID NO: 190 which correspond to the complementarity-determining regions (CDRs, or hypervariable regions) of the variable heavy chain sequence of SEQ ID NO: 183 or the heavy chain sequence of SEQ ID NO: 184, or combinations of these polypeptide sequences. In another example of the description, the antibodies of the description or fragments thereof for treatment or prevention of pain and pain associated conditions comprise, or alternatively consist of, combinations of one or more of the CDRs, the variable heavy and variable light chain sequences, and the heavy and light chain sequences set forth above, including all of them.

The description also optionally contemplates fragments of the antibody having binding specificity to NGF for treatment or prevention of pain and pain associated conditions. In one example of the description, antibody fragments of the description comprise, or alternatively consist of, the polypeptide sequence of SEQ ID NO: 181 or SEQ ID NO: 182. In another example of the description, antibody fragments of the description comprise, or alternatively consist of, the polypeptide sequence of SEQ ID NO: 183 or SEQ ID NO: 184.

In a further optional example of the description, fragments of the antibody having binding specificity to NGF for treatment or prevention of pain and pain associated conditions comprise, or alternatively consist of, one or more of the polypeptide sequences of SEQ ID NO: 185; SEQ ID NO: 186; and SEQ ID NO: 187 which correspond to the complementarity-determining regions (CDRs, or hypervariable regions) of the variable light chain sequence of SEQ ID NO: 181 or the light chain sequence of SEQ ID NO: 182.

In a further optional example of the description, fragments of the antibody having binding specificity to NGF for treatment or prevention of pain and pain associated conditions comprise, or alternatively consist of, one or more of the polypeptide sequences of SEQ ID NO: 188; SEQ ID NO: 189; and SEQ ID NO: 190 which correspond to the complementarity-determining regions (CDRs, or hypervariable regions) of the variable heavy chain sequence of SEQ ID NO: 183 or the heavy chain sequence of SEQ ID NO: 184.

The description also optionally contemplates antibody fragments which include one or more of the antibody fragments described herein for treatment or prevention of pain and pain associated conditions. In one optional example of the description, fragments of the antibodies having binding specificity to NGF comprise, or alternatively consist of, one, two, three or more, including all of the following antibody fragments: the variable light chain region of SEQ ID NO: 181; the variable heavy chain region of SEQ ID NO: 183; the complementarity-determining regions (SEQ ID NO: 185; SEQ ID NO: 186; and SEQ ID NO: 187) of the variable light chain region of SEQ ID NO: 181; and the complementarity-determining regions (SEQ ID NO: 188; SEQ ID NO: 189; and SEQ ID NO: 190) of the variable heavy chain region of SEQ ID NO: 183.

In a particularly preferred optional example of the description, the chimeric anti-NGF antibody for treatment or prevention of pain and pain associated conditions is Ab19, comprising, or alternatively consisting of, SEQ ID NO: 182 and SEQ ID NO: 184, and having at least one of the biological activities set forth herein.

In a further particularly preferred optional example of the description, antibody fragments for treatment or prevention of pain and pain associated conditions comprise, or alternatively consist of, Fab (fragment antigen binding) fragments having binding specificity for NGF. With respect to antibody Ab19, the Fab fragment includes the variable light chain sequence of SEQ ID NO: 181 and the variable heavy chain sequence of SEQ ID NO: 183 or comprises another Fab or antibody fragment that binds to the same or overlapping epitope as Ab19. This optional example of the description further contemplates additions, deletions, and variants of SEQ ID NO: 181 and/or SEQ example of the description, e retaining binding specificity for NGF.

In one optional example of the description described herein (infra), Fab fragments for treatment or prevention of pain and pain associated conditions may be produced by enzymatic digestion (e.g., papain) of Ab19. In another example of the description, anti-NGF antibodies such as Ab19 or Fab fragments thereof may be produced via expression in mammalian cells such as CHO, NSO or HEK 293 cells, fungal, insect, plant or microbial systems such as yeast cells (for example diploid yeast such as diploid Pichia) and other yeast strains. Suitable Pichia species include, but are not limited to, Pichia pastoris.

### Antibody Ab20

The description optionally contemplates methods of treating pain and the specific pain associated disorders alone or is association with another active agent, e.g., an NSAID or opioid analgesic, wherein the antibodies include chimeric antibodies having binding specificity to NGF wherein the antibody is Ab20 or fragments thereof, for example as set forth below, or comprises another antibody or antibody fragment that binds to the same or overlapping epitope as Ab20, in a therapeutically effective amount which inhibits the association of NGF with TrkA and the association of NGF with p75. In one example, the description includes chimeric or humanized antibodies for treatment or prevention of pain and pain associated conditions having binding specificity to NGF and possessing a variable light chain sequence comprising the sequence set forth below:

The description also optionally includes chimeric or humanized antibodies for treatment or prevention of pain and pain associated conditions having binding specificity to NGF and possessing a light chain sequence comprising the sequence set forth below:

The description further optionally includes chimeric or humanized antibodies for treatment or prevention of pain and pain associated conditions having binding specificity to NGF and possessing a variable heavy chain sequence comprising the sequence set forth below:

The description also optionally includes chimeric or humanized antibodies for treatment or prevention of pain and pain associated conditions having binding specificity to NGF and possessing a heavy chain sequence comprising the sequence set forth below:

The description further optionally contemplates antibodies for treatment or prevention of pain and pain associated conditions comprising one or more of the polypeptide sequences of SEQ ID NO: 195; SEQ ID NO: 196; and SEQ ID NO: 197 which correspond to the complementarity-determining regions (CDRs, or hypervariable regions) of the variable light chain sequence of SEQ ID NO: 191 or the light chain sequence of SEQ ID NO: 192, and/or one or more of the polypeptide sequences of SEQ ID NO: 198; SEQ ID NO: 199; and SEQ ID NO: 200 which correspond to the complementarity-determining regions (CDRs, or hypervariable regions) of the variable heavy chain sequence of SEQ ID NO: 193 or the heavy chain sequence of SEQ ID NO: 194, or combinations of these polypeptide sequences. In another example of the description, the antibodies of the description or fragments thereof for treatment or prevention of pain and pain associated conditions comprise, or alternatively consist of, combinations of one or more of the CDRs, the variable heavy and variable light chain sequences, and the heavy and light chain sequences set forth above, including all of them.

The description also optionally contemplates fragments of the antibody having binding specificity to NGF. In one example of the description, antibody fragments of the description for treatment or prevention of pain and pain associated conditions comprise, or alternatively consist of, the polypeptide sequence of SEQ ID NO: 191 or SEQ ID NO: 192. In another example of the description, antibody fragments of the description comprise, or alternatively consist of, the polypeptide sequence of SEQ ID NO: 193 or SEQ ID NO: 194.

In a further optional example of the description, fragments of the antibody having binding specificity to NGF for treatment or prevention of pain and pain associated conditions comprise, or alternatively consist of, one or more of the polypeptide sequences of SEQ ID NO: 195; SEQ ID NO: 196; and SEQ ID NO: 197 which correspond to the complementarity-determining regions (CDRs, or hypervariable regions) of the variable light chain sequence of SEQ ID NO: 191 or the light chain sequence of SEQ ID NO: 192.

In a further optional example of the description, fragments of the antibody having binding specificity to NGF comprise, or alternatively consist of, one or more of the polypeptide sequences of SEQ ID NO: 198; SEQ ID NO: 199; and SEQ ID NO: 200 which correspond to the complementarity-determining regions (CDRs, or hypervariable regions) of the variable heavy chain sequence of SEQ ID NO: 193 or the heavy chain sequence of SEQ ID NO: 194.

The description also optionally contemplates antibody fragments for treatment or prevention of pain and pain associated conditions which include one or more of the antibody fragments described herein. In one example of the description, fragments of the antibodies having binding specificity to NGF comprise, or alternatively consist of, one, two, three or more, including all of the following antibody fragments: the variable light chain region of SEQ ID NO: 191; the variable heavy chain region of SEQ ID NO: 193; the complementarity-determining regions (SEQ ID NO: 195; SEQ ID NO: 196; and SEQ ID NO: 197) of the variable light chain region of SEQ ID NO: 191; and the complementarity-determining regions (SEQ ID NO: 198; SEQ ID NO: 199; and SEQ ID NO: 200) of the variable heavy chain region of SEQ ID NO: 193.

In a particularly preferred optional example of the description, the chimeric or humanized anti-NGF antibody for treatment or prevention of pain and pain associated conditions is Ab20, comprising, or alternatively consisting of, SEQ ID NO: 192 and SEQ ID NO: 194, and having at least one of the biological activities set forth herein.

In a further particularly preferred optional example of the description, antibody fragments for treatment or prevention of pain and pain associated conditions comprise, or alternatively consist of, Fab (fragment antigen binding) fragments having binding specificity for NGF. With respect to antibody Ab20, the Fab fragment includes the variable light chain sequence of SEQ ID NO: 191 and the variable heavy chain sequence of SEQ ID NO: 193. This embodiment of the invention further contemplates additions, deletions, and variants of SEQ ID NO: 191 and/or SEQ ID NO: 193 in said Fab while retaining binding specificity for NGF.

In one optional example of the description, described herein (infra), Fab fragments for treatment or prevention of pain and pain associated conditions may be produced by enzymatic digestion (e.g., papain) of Ab20. In another example of the description, anti-NGF antibodies for treatment or prevention of pain and pain associated conditions such as Ab20 or Fab fragments thereof may be produced via expression in mammalian cells such as CHO, NSO or HEK 293 cells, fungal, insect, plant or microbial systems such as yeast cells (for example diploid yeast such as diploid Pichia) and other yeast strains. Suitable Pichia species include, but are not limited to, Pichia pastoris.

### Antibody Ab21

The description optionally contemplates methods of treating pain and the specific pain associated disorders alone or is association with another active agent, e.g., an NSAID or opioid analgesic, wherein the antibodies include chimeric antibodies having binding specificity to NGF wherein the antibody is Ab21 or fragments thereof, or another antibody or antibody fragment that binds to the same or overlapping epitope as Ab5, for example as set forth below, in a therapeutically effective amount which inhibits the association of NGF with TrkA and the association of NGF with p75. In one example, the description includes chimeric or humanized antibodies having binding specificity to NGF and possessing a variable light chain sequence comprising the sequence set forth below:

The description also optionally includes chimeric or humanized antibodies for treatment or prevention of pain and pain associated conditions having binding specificity to NGF and possessing a light chain sequence comprising the sequence set forth below:

The description further optionally includes chimeric or humanized antibodies for treatment or prevention of pain and pain associated conditions having binding specificity to NGF and possessing a variable heavy chain sequence comprising the sequence set forth below:

The description also optionally includes chimeric or humanized antibodies for treatment or prevention of pain and pain associated conditions having binding specificity to NGF and possessing a heavy chain sequence comprising the sequence set forth below:

The description further optionally contemplates antibodies for treatment or prevention of pain and pain associated conditions comprising one or more of the polypeptide sequences of SEQ ID NO: 55; SEQ ID NO: 56; and SEQ ID NO: 57 which correspond to the complementarity-determining regions (CDRs, or hypervariable regions) of the variable light chain sequence of SEQ ID NO: 51 or the light chain sequence of SEQ ID NO: 401, and/or one or more of the polypeptide sequences of SEQ ID NO: 58; SEQ ID NO: 59; and SEQ ID NO: 60 which correspond to the complementarity-determining regions (CDRs, or hypervariable regions) of the variable heavy chain sequence of SEQ ID NO: 53 or the heavy chain sequence of SEQ ID NO: 402, or combinations of these polypeptide sequences. In another optional example of the description, the antibodies of the description or fragments thereof comprise, or alternatively consist of, combinations of one or more of the CDRs, the variable heavy and variable light chain sequences, and the heavy and light chain sequences set forth above, including all of them.

The description also optionally contemplates fragments of the antibody for treatment or prevention of pain and pain associated conditions having binding specificity to NGF. In one example of the description, antibody fragments of the description for treatment or prevention of pain and pain associated conditions comprise, or alternatively consist of, the polypeptide sequence of SEQ ID NO: 51 or SEQ ID NO: 401. In another example of the description, antibody fragments of the description for treatment or prevention of pain and pain associated conditions comprise, or alternatively consist of, the polypeptide sequence of SEQ ID NO: 53 or SEQ ID NO: 402.

In a further optional example of the description, fragments of the antibody having binding specificity to NGF for treatment or prevention of pain and pain associated conditions comprise, or alternatively consist of, one or more of the polypeptide sequences of SEQ ID NO: 55; SEQ ID NO: 56; and SEQ ID NO: 57 which correspond to the complementarity-determining regions (CDRs, or hypervariable regions) of the variable light chain sequence of SEQ ID NO: 51 or the light chain sequence of SEQ ID NO: 401.

In a further optional example of the description, fragments of the antibody having binding specificity to NGF for treatment or prevention of pain and pain associated conditions comprise, or alternatively consist of, one or more of the polypeptide sequences of SEQ ID NO: 58; SEQ ID NO: 59; and SEQ ID NO: 60 which correspond to the complementarity-determining regions (CDRs, or hypervariable regions) of the variable heavy chain sequence of SEQ ID NO: 53 or the heavy chain sequence of SEQ ID NO: 402.

The description also optionally contemplates antibody fragments for treatment or prevention of pain and pain associated conditions which include one or more of the antibody fragments described herein. In one example of the description, fragments of the antibodies having binding specificity to NGF comprise, or alternatively consist of, one, two, three or more, including all of the following antibody fragments: the variable light chain region of SEQ ID NO: 51; the variable heavy chain region of SEQ ID NO: 53; the complementarity-determining regions (SEQ ID NO: 55; SEQ ID NO: 56; and SEQ ID NO: 57) of the variable light chain region of SEQ ID NO: 51; and the complementarity-determining regions (SEQ ID NO: 58; SEQ ID NO: 59; and SEQ ID NO: 60) of the variable heavy chain region of SEQ ID NO: 53.

In a particularly preferred optional example of the description, the chimeric or humanized anti-NGF antibody for treatment or prevention of pain and pain associated conditions is Ab21, comprising, or alternatively consisting of, SEQ ID NO: 401 and SEQ ID NO: 402, and having at least one of the biological activities set forth herein.

In a further particularly preferred optional example of the description, antibody fragments for treatment or prevention of pain and pain associated conditions comprise, or alternatively consist of, Fab (fragment antigen binding) fragments having binding specificity for NGF. With respect to antibody Ab21, the Fab fragment includes the variable light chain sequence of SEQ ID NO: 51 and the variable heavy chain sequence of SEQ ID NO: 53 or another Fab or monovalent antibody fragment that binds to the same or overlapping epitope as Ab5,. This example of the description further contemplates additions, deletions, and variants of SEQ ID NO: 51 and/or SEQ ID NO: 53 in said Fab while retaining binding specificity for NGF.

In one optional example of the description described herein (infra), Fab fragments for treatment or prevention of pain and pain associated conditions may be produced by enzymatic digestion (e.g., papain) of Ab21. In another example of the description, anti-NGF antibodies for treatment or prevention of pain and pain associated conditions such as Ab21 or Fab fragments thereof may be produced via expression in mammalian cells such as CHO, NSO or HEK 293 cells, fungal, insect, plant or microbial systems such as yeast cells (for example diploid yeast such as diploid Pichia) and other yeast strains. Suitable Pichia species include, but are not limited to, Pichia pastoris.

### Antibody fragment Fab1

The description optionally contemplates methods of treating pain using antibody fragment Fab1 or fragments thereof, for example as set forth below, in a therapeutically effective amount which inhibits the association of NGF with TrkA and the association of NGF with p75. In one example, the description optionally includes Fab antibody fragments for treatment or prevention of pain and pain associated conditions having binding specificity to NGF and possessing a light chain sequence comprising the sequence set forth below:

The description further optionally includes Fab antibody fragments for treatment or prevention of pain and pain associated conditions having binding specificity to NGF and possessing a heavy chain sequence comprising the sequence set forth below:

The description further optionally contemplates antibody fragments for treatment or prevention of pain and pain associated conditions comprising one or more of the polypeptide sequences of SEQ ID NO: 55; SEQ ID NO: 56; and SEQ ID NO: 57 which correspond to the complementarity-determining regions (CDRs, or hypervariable regions) of the variable light chain sequence of SEQ ID NO: 51 or the light chain sequence of SEQ ID NO: 405, and/or one or more of the polypeptide sequences of SEQ ID NO: 58; SEQ ID NO: 59; and SEQ ID NO: 60 which correspond to the complementarity-determining regions (CDRs, or hypervariable regions) of the variable heavy chain sequence of SEQ ID NO: 53 or the heavy chain sequence of SEQ ID NO: 406, or combinations of these polypeptide sequences. In another optional example of the description, antibody fragments of the description for treatment or prevention of pain and pain associated conditions comprise, or alternatively consist of, combinations of one or more of the CDRs, the variable heavy and variable light chain sequences, and the heavy and light chain sequences set forth above, including all of them.

The description also optionally contemplates fragments of the antibody for treatment or prevention of pain and pain associated conditions having binding specificity to NGF. In one example of the description, antibody fragments of the description comprise, or alternatively consist of, the polypeptide sequence of SEQ ID NO: 51 or SEQ ID NO: 405. In another example of the description, antibody fragments of the description comprise, or alternatively consist of, the polypeptide sequence of SEQ ID NO: 53 or SEQ ID NO: 406.

In a further optional example of the description, antibody fragments for treatment or prevention of pain and pain associated conditions having binding specificity to NGF comprise, or alternatively consist of, one or more of the polypeptide sequences of SEQ ID NO: 55; SEQ ID NO: 56; and SEQ ID NO: 5 example of the nd to the complementarity-determining regions (CDRs, or hypervariable regions) of the variable light chain sequence of SEQ ID NO: 51 or the light chain sequence of SEQ ID NO: 405.

In a further optional example of the description, antibody fragments for treatment or prevention of pain and pain associated conditions having binding specificity to NGF comprise, or alternatively consist of, one or more of the polypeptide sequences of SEQ ID NO: 58; SEQ ID NO: 59; and SEQ ID NO: 60 which correspond to the complementarity-determining regions (CDRs, or hypervariable regions) of the variable heavy chain sequence of SEQ ID NO: 53 or the heavy chain sequence of SEQ ID NO: 406.

The description also optionally contemplates antibody fragments for treatment or prevention of pain and pain associated conditions which include one or more of the antibody fragments described herein. In one example of the description, fragments of the antibodies having binding specificity to NGF comprise, or alternatively consist of, one, two, three or more, including all of the following antibody fragments: the variable light chain region of SEQ ID NO: 51; the variable heavy chain region of SEQ ID NO: 53; the complementarity-determining regions (SEQ ID NO: 55; SEQ ID NO: 56; and SEQ ID NO: 57) of the variable light chain region of SEQ ID NO: 51; and the complementarity-determining regions (SEQ ID NO: 58; SEQ ID NO: 59; and SEQ ID NO: 60) of the variable heavy chain region of SEQ ID NO: 53.

In a particularly preferred optional example of the description, the anti-NGF antibody fragment for treatment or prevention of pain and pain associated conditions is Fab1, comprising SEQ ID NO: 405 and SEQ ID NO: 406, or another Fab or antibody fragment that binds to the same or overlapping epitope as Fab1, and having at least one of the biological activities set forth herein. In one example of the description, antibody fragment Fab1 may be produced by enzymatic digestion (e.g., papain) of Ab21.

### Antibody fragment Fab2

The description optionally contemplates methods of treating pain using antibody fragment Fab2 or fragments thereof, for example as set forth below, in a therapeutically effective amount which inhibits the association of NGF with TrkA and the association of NGF with p75. In one example, the description includes Fab antibody fragments for treatment or prevention of pain and pain associated conditions having binding specificity to NGF and possessing a light chain sequence comprising the sequence set forth below:

The description further optionally includes Fab antibody fragments for treatment or prevention of pain and pain associated conditions having binding specificity to NGF and possessing a heavy chain sequence comprising the sequence set forth below:

The description further optionally contemplates antibody fragments for treatment or prevention of pain and pain associated conditions comprising one or more of the polypeptide sequences of SEQ ID NO: 55; SEQ ID NO: 56; and SEQ ID NO: 57 which correspond to the complementarity-determining regions (CDRs, or hypervariable regions) of the variable light chain sequence of SEQ ID NO: 51 or the light chain sequence of SEQ ID NO: 407, and/or one or more of the polypeptide sequences of SEQ ID NO: 58; SEQ ID NO: 59; and SEQ ID NO: 60 which correspond to the complementarity-determining regions (CDRs, or hypervariable regions) of the variable heavy chain sequence of SEQ ID NO: 53 or the heavy chain sequence of SEQ ID NO: 408, or combinations of these polypeptide sequences. In another example of the description, antibody fragments of the description comprise, or alternatively consist of, combinations of one or more of the CDRs, the variable heavy and variable light chain sequences, and the heavy and light chain sequences set forth above, including all of them.

The description also optionally contemplates fragments of the antibody for treatment or prevention of pain and pain associated conditions having binding specificity to NGF. In one example of the description, antibody fragments of the invention for treatment or prevention of pain and pain associated conditions comprise, or alternatively consist of, the polypeptide sequence of SEQ ID NO: 51 or SEQ ID NO: 407. In another example of the description, antibody fragments of the description for treatment or prevention of pain and pain associated conditions comprise, or alternatively consist of, the polypeptide sequence of SEQ ID NO: 53 or SEQ ID NO: 408.

In a further optional example of the description, antibody fragments for treatment or prevention of pain and pain associated conditions having binding specificity to NGF comprise, or alternatively consist of, one or more of the polypeptide sequences of SEQ ID NO: 55; SEQ ID NO: 56; and SEQ ID NO: 57 which correspond to the complementarity-determining regions (CDRs, or hypervariable regions) of the variable light chain sequence of SEQ ID NO: 51 or the light chain sequence of SEQ ID NO: 407.

In a further optional example of the description, antibody fragments for treatment or prevention of pain and pain associated conditions having binding specificity to NGF comprise, or alternatively consist of, one or more of the polypeptide sequences of SEQ ID NO: 58; SEQ ID NO: 59; and SEQ ID NO: 60 which correspond to the complementarity-determining regions (CDRs, or hypervariable regions) of the variable heavy chain sequence of SEQ ID NO: 53 or the heavy chain sequence of SEQ ID NO: 408.

The description also optionally contemplates antibody fragments for treatment or prevention of pain and pain associated conditions which include one or more of the antibody fragments described herein. In one example of the description, fragments of the antibodies having binding specificity to NGF for treatment or prevention of pain and pain associated conditions comprise, or alternatively consist of, one, two, three or more, including all of the following antibody fragments: the variable light chain region of SEQ ID NO: 51; the variable heavy chain region of SEQ ID NO: 53; the complementarity-determining regions (SEQ ID NO: 55; SEQ ID NO: 56; and SEQ ID NO: 57) of the variable light chain region of SEQ ID NO: 51; and the complementarity-determining regions (SEQ ID NO: 58; SEQ ID NO: 59; and SEQ ID NO: 60) of the variable heavy chain region of SEQ ID NO: 53.

In a particularly preferred optional example of the description, the anti-NGF antibody fragment for treatment or prevention of pain and pain associated conditions is Fab2, comprising SEQ ID NO: 407 and SEQ ID NO: 408, or another Fab or antibody fragment that binds to the same or overlapping epitope as Fab2, and having at least one of the biological activities set forth herein.

In another optional example of the description described herein (infra), Fab fragments for treatment or prevention of pain and pain associated conditions may be produced via expression in mammalian cells such as CHO, NSO or HEK 293 cells, fungal, insect, plant or microbial systems such as yeast cells (for example diploid yeast such as diploid Pichia) and other yeast strains. Suitable Pichia species include, but are not limited to, Pichia pastoris. In one example of the description, antibody fragment Fab2 may be produced by expression in Pichia pastoris using protocols set forth herein in the examples.

In another example, antibody fragments may be present in one or more of the following non-limiting forms: Fab, Fab', F(ab')₂, Fv and single chain Fv antibody forms. In a preferred example, the anti-NGF antibodies described herein further comprises the kappa constant light chain sequence comprising the sequence set forth below:

In another preferred optional example, the anti-NGF antibodies described herein for treatment or prevention of pain and pain associated conditions further comprises the gamma-1 constant heavy chain polypeptide sequence comprising the sequence set forth below:

In another optional example, the description contemplates an isolated anti-NGF antibody for treatment or prevention of pain and pain associated conditions comprising a V_{H} polypeptide sequence selected from: SEQ ID NO: 3, 13, 23, 33, 43, 53, 63, 73, 83, 93, 103, 113, 123, 133, 143, 153, 163, 173, 183, 193, or 402, or a variant thereof; and further comprising a V_{L} polypeptide sequence selected from: SEQ ID NO: 1, 11, 21, 31, 41, 51, 61, 71, 81, 91, 101, 111, 121, 131, 141, 151, 161, 171, 181, 191, or 401, or a variant thereof, wherein one or more of the framework residues (FR residues) in said V_{H} or V_{L} polypeptide has been substituted with another amino acid residue resulting in an anti-NGF antibody that specifically binds NGF. The description contemplates humanized and chimeric forms of these antibodies for treatment or prevention of pain and pain associated conditions. The chimeric antibodies may include an Fc derived from IgG1, IgG2, IgG3, IgG4, IgG5, IgG6, IgG7, IgG8, IgG9, IgG10, IgG11, IgG12, IgG13, IgG14, IgG15, IgG16, IgG17, IgG18 or IgG19 constant regions.

In one example of the description, the antibodies or V_{H} or V_{L} polypeptides originate or are selected from one or more rabbit B cell populations prior to initiation of the humanization process referenced herein.

In another example of the description, the anti-NGF antibodies and fragments thereof for treatment or prevention of pain and pain associated conditions do not have binding specificity for p75 or TrkA. In a further example of the description, there is contemplated methods for treating pain comprising using the anti-NGF antibodies and fragments thereof to inhibit the association of NGF with p75 and/or TrkA. In another example of the description, there is contemplated methods for treating pain comprising using anti-NGF antibodies and fragments thereof to inhibit the association of NGF with TrkA and/or multimers thereof and/or antagonizes the biological effects thereof. In another example of the description, there is contemplated methods for treating pain comprising using anti-NGF antibodies and fragments thereof to inhibit the association of NGF with p75 and/or multimers thereof and the association of NGF with TrkA and/or multimers thereof, and antagonizes the biological effects of p75 and TrkA.

As stated supra, antibodies and fragments thereof may be modified post-translationally to add effector moieties such as chemical linkers, detectable moieties such as for example fluorescent dyes, enzymes, substrates, bioluminescent materials, radioactive materials, and chemiluminescent moieties, or functional moieties such as for example streptavidin, avidin, biotin, a cytotoxin, a cytotoxic agent, and radioactive materials.

Regarding detectable moieties, further exemplary enzymes include, but are not limited to, horseradish peroxidase, acetylcholinesterase, alkaline phosphatase, beta-galactosidase and luciferase. Further exemplary fluorescent materials include, but are not limited to, rhodamine, fluorescein, fluorescein isothiocyanate, umbelliferone, dichlorotriazinylamine, phycoerythrin and dansyl chloride. Further exemplary chemiluminescent moieties include, but are not limited to, luminol. Further exemplary bioluminescent materials include, but are not limited to, luciferin and aequorin. Further exemplary radioactive materials include, but are not limited to, Iodine 125 (¹²⁵I), Carbon 14 (¹⁴C), Sulfur 35 (³⁵S), Tritium (³H) and Phosphorus 32 (³²P).

Regarding functional moieties, exemplary cytotoxic agents include, but are not limited to, methotrexate, aminopterin, 6-mercaptopurine, 6-thioguanine, cytarabine, 5-fluorouracil decarbazine; alkylating agents such as mechlorethamine, thioepa chlorambucil, melphalan, carmustine (BSNU), mitomycin C, lomustine (CCNU), 1-methylnitrosourea, cyclothosphamide, mechlorethamine, busulfan, dibromomannitol, streptozotocin, mitomycin C, cis-dichlorodiamine platinum (II) (DDP) cisplatin and carboplatin (paraplatin); anthracyclines include daunorubicin (formerly daunomycin), doxorubicin (adriamycin), detorubicin, carminomycin, idarubicin, epirubicin, mitoxantrone and bisantrene; antibiotics include dactinomycin (actinomycin D), bleomycin, calicheamicin, mithramycin, and anthramycin (AMC); and antimytotic agents such as the vinca alkaloids, vincristine and vinblastine. Other cytotoxic agents include paclitaxel (taxol), ricin, pseudomonas exotoxin, gemcitabine, cytochalasin B, gramicidin D, ethidium bromide, emetine, etoposide, tenoposide, colchicin, dihydroxy anthracin dione, 1-dehydrotestosterone, glucocorticoids, procaine, tetracaine, lidocaine, propranolol, puromycin, procarbazine, hydroxyurea, asparaginase, corticosteroids, mytotane (O,P'-(DDD)), interferons, and mixtures of these cytotoxic agents.

Further cytotoxic agents include, but are not limited to, chemotherapeutic agents such as carboplatin, cisplatin, paclitaxel, gemcitabine, calicheamicin, doxorubicin, 5-fluorouracil, mitomycin C, actinomycin D, cyclophosphamide, vincristine and bleomycin. Toxic enzymes from plants and bacteria such as ricin, diphtheria toxin and Pseudomonas toxin may be conjugated to the humanized or chimeric antibodies, or binding fragments thereof, to generate cell-type-specific-killing reagents (Youle, et al., Proc. Nat'l Acad. Sci. USA 77:5483 (1980); Gilliland, et al., Proc. Nat'l Acad. Sci. USA 77:4539 (1980); Krolick, et al., Proc. Nat'l Acad. Sci. USA 77:5419 (1980)).

Other cytotoxic agents include cytotoxic ribonucleases as described by Goldenberg in U.S. Pat. No. 6,653,104. Examples of the description also relate to radioimmunoconjugates where a radionuclide that emits alpha or beta particles is stably coupled to the antibody, or binding fragments thereof, with or without the use of a complex-forming agent. Such radionuclides include beta-emitters such asPhosphorus-32 (³²P), Scandium-47 (⁴⁷Sc), Copper-67 (⁶⁷Cu), Gallium-67 (⁶⁷Ga), Yttrium-88 (⁸⁸Y), Yttrium-90 (⁹⁰Y), Iodine-125 (¹²⁵I), Iodine-131 (¹³¹I), Samarium-153 (¹⁵³Sm), Lutetium-177 (¹⁷⁷Lu), Rhenium-186 (¹⁸⁶Re) or Rhenium-188 (¹⁸⁸Re), and alpha-emitters such as Astatine-211 (²¹¹At), Lead-212 (²¹²Pb), Bismuth-212 (²¹²Bi) or -213 (²¹³Bi) or Actinium-225 (²²⁵Ac).

Further exemplary radioactive materials include, but are not limited to, Iodine 125 (¹²⁵I), Carbon 14 (¹⁴C), Sulfur 35 (³⁵S), Tritium (³H) and Phosphorus 32 (³²P).

Regarding functional moieties, exemplary cytotoxic agents include, but are not limited to, methotrexate, aminopterin, 6-mercaptopurine, 6-thioguanine, cytarabine, 5-fluorouracil decarbazine; alkylating agents such as mechlorethamine, thioepa chlorambucil, melphalan, carmustine (BSNU), mitomycin C, lomustine (CCNU), 1-methylnitrosourea, cyclothosphamide, mechlorethamine, busulfan, dibromomannitol, streptozotocin, mitomycin C, cis-dichlorodiamine platinum (II) (DDP) cisplatin and carboplatin (paraplatin); anthracyclines include daunorubicin (formerly daunomycin), doxorubicin (adriamycin), detorubicin, carminomycin, idarubicin, epirubicin, mitoxantrone and bisantrene; antibiotics include dactinomycin (actinomycin D), bleomycin, calicheamicin, mithramycin, and anthramycin (AMC); and antimytotic agents such as the vinca alkaloids, vincristine and vinblastine. Other cytotoxic agents include paclitaxel (taxol), ricin, pseudomonas exotoxin, gemcitabine, cytochalasin B, gramicidin D, ethidium bromide, emetine, etoposide, tenoposide, colchicin, dihydroxy anthracin dione, 1-dehydrotestosterone, glucocorticoids, procaine, tetracaine, lidocaine, propranolol, puromycin, procarbazine, hydroxyurea, asparaginase, corticosteroids, mytotane (O,P'-(DDD)), interferons, and mixtures of these cytotoxic agents.

Further cytotoxic agents include, but are not limited to, chemotherapeutic agents such as carboplatin, cisplatin, paclitaxel, gemcitabine, calicheamicin, doxorubicin, 5-fluorouracil, mitomycin C, actinomycin D, cyclophosphamide, vincristine and bleomycin. Toxic enzymes from plants and bacteria such as ricin, diphtheria toxin and Pseudomonas toxin may be conjugated to the humanized or chimeric antibodies, or binding fragments thereof, to generate cell-type-specific-killing reagents (Youle, et al., Proc. Nat'l Acad. Sci. USA 77:5483 (1980); Gilliland, et al., Proc. Nat'l Acad. Sci. USA 77:4539 (1980); Krolick, et al., Proc. Nat'l Acad. Sci. USA 77:5419 (1980)).

Other cytotoxic agents include cytotoxic ribonucleases as described by Goldenberg in U.S. Pat. No. 6,653,104. Examples of the description also relate to radioimmunoconjugates where a radionuclide that emits alpha or beta particles is stably coupled to the antibody, or binding fragments thereof, with or without the use of a complex-forming agent. Such radionuclides include beta-emitters such asPhosphorus-32 (³²P), Scandium-47 (⁴⁷Sc), Copper-67 (⁶⁷Cu), Gallium-67 (⁶⁷Ga), Yttrium-88 (⁸⁸Y), Yttrium-90 (⁹⁰Y), Iodine-125 (¹²⁵I), Iodine-131 (¹³¹I), Samarium-153 (¹⁵³Sm), Lutetium-177 (¹⁷⁷Lu), Rhenium-186 (¹⁸⁶Re) or Rhenium-188 (¹⁸⁸Re), and alpha-emitters such as Astatine-211 (²¹¹At), Lead-212 (²¹²Pb), Bismuth-212 (²¹²Bi) or -213 (²¹³Bi) or Actinium-225 (²²⁵Ac)..

Methods are known in the art for conjugating an antibody or binding fragment thereof to a detectable moiety and the like, such as for example those methods described by Hunter et al, Nature 144:945 (1962); David et al, Biochemistry 13:1014 (1974); Pain et al, J. Immunol. Meth. 40:219 (1981); and Nygren, J., Histochem. and Cytochem. 30:407 (1982).

Examples described herein further include variants and equivalents that are substantially homologous to the antibodies, antibody fragments, diabodies, SMIPs, camelbodies, nanobodies, IgNAR, polypeptides, variable regions and CDRs set forth herein. These may contain, e.g., conservative substitution mutations, (i.e., the substitution of one or more amino acids by similar amino acids). For example, conservative substitution refers to the substitution of an amino acid with another within the same general class, e.g., one acidic amino acid with another acidic amino acid, one basic amino acid with another basic amino acid, or one neutral amino acid by another neutral amino acid. What is intended by a conservative amino acid substitution is well known in the art.

In another example, the description contemplates polypeptide sequences having at least 90% or greater sequence homology to any one or more of the polypeptide sequences of antibody fragments, variable regions and CDRs set forth herein. More preferably, the description contemplates polypeptide sequences having at least 95% or greater sequence homology, even more preferably at least 98% or greater sequence homology, and still more preferably at least 99% or greater sequence homology to any one or more of the polypeptide sequences of antibody fragments, variable regions and CDRs set forth herein. Methods for determining homology between nucleic acid and amino acid sequences are well known to those of ordinary skill in the art.

In another example, the description further contemplates the above-recited polypeptide homologs of the antibody fragments, variable regions and CDRs set forth herein further having anti-NGF activity. Non-limiting examples of anti-NGF activity are set forth herein.

In another example, the description further contemplates the generation and use of anti-idiotypic antibodies that bind any of the foregoing sequences. In an example, such an anti-idiotypic antibody could be administered to a subject who has received an anti-NGF antibody to modulate, reduce, or neutralize, the effect of the anti-NGF antibody. Such anti-idiotypic antibodies could also be useful for treatment of an autoimmune disease characterized by the presence of anti-NGF antibodies. A further exemplary use of such anti-idiotypic antibodies is for detection of the anti-NGF antibodies of the present invention, for example to monitor the levels of the anti-NGF antibodies present in a subject's blood or other bodily fluids.

The present description also contemplates anti-NGF antibodies comprising any of the polypeptide or polynucleotide sequences described herein substituted for any of the other polynucleotide sequences described herein. For example, without limitation thereto, the present description contemplates antibodies comprising the combination of any of the variable light chain and variable heavy chain sequences described herein, and further contemplates antibodies resulting from substitution of any of the CDR sequences described herein for any of the other CDR sequences described herein.

### Polynucleotides Encoding Anti-NGF Antibody Polypeptides

### Antibody Ab1

The description is further optionally directed to the use of polynucleotides set forth below to produce antibody Ab1 polypeptides for treatment or prevention of pain and pain associated conditions having binding specificity to NGF, which inhibit the association of NGF with TrkA and the association of NGF with p75, in methods of treating pain in an individual comprising administering to said individual antibody Ab1 polypeptides. In one example of the description, polynucleotides of the description comprise, or alternatively consist of, the following polynucleotide sequence encoding the variable light chain polypeptide sequence of SEQ ID NO: 1:

In one optional example of the description, polynucleotides of the description comprise, or alternatively consist of, the following polynucleotide sequence encoding the light chain polypeptide sequence of SEQ ID NO: 2:

In another optional example of the description, polynucleotides of the description comprise, or alternatively consist of, the following polynucleotide sequence encoding the variable heavy chain polypeptide sequence of SEQ ID NO: 3:

In one optional example of the description, polynucleotides of the description comprise, or alternatively consist of, the following polynucleotide sequence encoding the heavy chain polypeptide sequence of SEQ ID NO: 4:

In a further optional example of the description, polynucleotides encoding antibody fragments for treatment or prevention of pain and pain associated conditions having binding specificity to NGF comprise, or alternatively consist of, one or more of the polynucleotide sequences of SEQ ID NO: 205; SEQ ID NO: 206; and SEQ ID NO: 207 which correspond to polynucleotides encoding the complementarity-determining regions (CDRs, or hypervariable regions) of the light chain variable sequence of SEQ ID NO: 1 or the light chain sequence of SEQ ID NO: 2.

In a further optional example of the description, polynucleotides encoding antibody fragments for treatment or prevention of pain and pain associated conditions having binding specificity to NGF comprise, or alternatively consist of, one or more of the polynucleotide sequences of SEQ ID NO: 208; SEQ ID NO: 209; and SEQ ID NO: 210 which correspond to polynucleotides encoding the complementarity-determining regions (CDRs, or hypervariable regions) of the heavy chain variable sequence of SEQ ID NO: 3 or the heavy chain sequence of SEQ ID NO: 4.

The description also optionally contemplates polynucleotide sequences including one or more of the polynucleotide sequences encoding antibody fragments for treatment or prevention of pain and pain associated conditions described herein. In one example of the description, polynucleotides encoding antibody fragments having binding specificity to NGF comprise, or alternatively consist of, one, two, three or more, including all of the following polynucleotides encoding antibody fragments: the polynucleotide SEQ ID NO: 201 encoding the light chain variable sequence of SEQ ID NO: 1; the polynucleotide SEQ ID NO: 202 encoding the light chain sequence of SEQ ID NO: 2; the polynucleotide SEQ ID NO: 203 encoding the heavy chain variable sequence of SEQ ID NO: 3; the polynucleotide SEQ ID NO: 204 encoding the heavy chain sequence of SEQ ID NO: 4; polynucleotides encoding the complementarity-determining regions (SEQ ID NO: 205; SEQ ID NO: 206; and SEQ ID NO: 207) of the light chain variable sequence of SEQ ID NO: 1 or the light chain sequence of SEQ ID NO: 2; and polynucleotides encoding the complementarity-determining regions (SEQ ID NO: 208; SEQ ID NO: 209; and SEQ ID NO: 210) of the heavy chain variable sequence of SEQ ID NO: 3 or the heavy chain sequence of SEQ ID NO: 4.

In a preferred optional example of the description, polynucleotides of the description comprise, or alternatively consist of, polynucleotides encoding Fab (fragment antigen binding) fragments for treatment or prevention of pain and pain associated conditions having binding specificity for NGF. With respect to antibody Ab1, the polynucleotides encoding the full length Ab1 antibody comprise, or alternatively consist of, the polynucleotide SEQ ID NO: 202 encoding the light chain sequence of SEQ ID NO: 2 and the polynucleotide SEQ ID NO: 204 encoding the heavy chain sequence of SEQ ID NO: 4.

Another optional example of the description contemplates these polynucleotides incorporated into an expression vector for expression in mammalian cells such as CHO, NSO, HEK-293, or in fungal, insect, plant or microbial systems such as yeast cells such as the yeast Pichia. Suitable Pichia species include, but are not limited to, Pichia pastoris. In one example of the description described herein (infra), Fab fragments for treatment or prevention of pain and pain associated conditions may be produced by enzymatic digestion (e.g., papain) of Ab1 following expression of the full-length polynucleotides in a suitable host. In another example of the description, anti-NGF antibodies such as Ab1 or Fab fragments thereof may be produced via expression of Ab1 polynucleotides in mammalian cells such as CHO, NSO or HEK 293 cells, fungal, insect, plant or microbial systems such as yeast cells (for example diploid yeast such as diploid Pichia) and other yeast strains. Suitable Pichia species include, but are not limited to, Pichia pastoris.

### Antibody Ab2

The description is further optionally directed to the use of polynucleotides set forth below to produce antibody Ab2 polypeptides which inhibit the association of NGF with TrkA and the association of NGF with p75, for treatment or prevention of pain and pain associated conditions having binding specificity to NGF in methods of treating pain in an individual comprising administering to said individual antibody Ab2 polypeptides. The description is further directed to polynucleotides encoding antibody polypeptides having binding specificity to NGF for treatment or prevention of pain and pain associated conditions. In one example of the description, polynucleotides of the description comprise, or alternatively consist of, the following polynucleotide sequence encoding the variable light chain polypeptide sequence of SEQ ID NO: 11:

In one optional example of the description, polynucleotides of the description comprise, or alternatively consist of, the following polynucleotide sequence encoding the light chain polypeptide sequence of SEQ ID NO: 12:

In another optional example of the description, polynucleotides of the description comprise, or alternatively consist of, the following polynucleotide sequence encoding the variable heavy chain polypeptide sequence of SEQ ID NO: 13:

In one optional example of the description, polynucleotides of the description comprise, or alternatively consist of, the following polynucleotide sequence encoding the heavy chain polypeptide sequence of SEQ ID NO: 14:

In a further optional example of the description, polynucleotides encoding antibody fragments for treatment or prevention of pain and pain associated conditions having binding specificity to NGF comprise, or alternatively consist of, one or more of the polynucleotide sequences of SEQ ID NO: 215; SEQ ID NO: 216; and SEQ ID NO: 217 which correspond to polynucleotides encoding the complementarity-determining regions (CDRs, or hypervariable regions) of the light chain variable sequence of SEQ ID NO: 11 or the light chain sequence of SEQ ID NO: 12.

In a further optional example of the description, polynucleotides encoding antibody fragments for treatment or prevention of pain and pain associated conditions having binding specificity to NGF comprise, or alternatively consist of, one or more of the polynucleotide sequences of SEQ ID NO: 218; SEQ ID NO: 219; and SEQ ID NO: 220 which correspond to polynucleotides encoding the complementarity-determining regions (CDRs, or hypervariable regions) of the heavy chain variable sequence of SEQ ID NO: 13 or the heavy chain sequence of SEQ ID NO: 14.

The description also optional contemplates polynucleotide sequences including one or more of the polynucleotide sequences encoding antibody fragments for treatment or prevention of pain and pain associated conditions described herein. In one example of the description, polynucleotides encoding antibody fragments having binding specificity to NGF comprise, or alternatively consist of, one, two, three or more, including all of the following polynucleotides encoding antibody fragments: the polynucleotide SEQ ID NO: 211 encoding the light chain variable sequence of SEQ ID NO: 11; the polynucleotide SEQ ID NO: 212 encoding the light chain sequence of SEQ ID NO: 12; the polynucleotide SEQ ID NO: 213 encoding the heavy chain variable sequence of SEQ ID NO: 13; the polynucleotide SEQ ID NO: 214 encoding the heavy chain sequence of SEQ ID NO: 14; polynucleotides encoding the complementarity-determining regions (SEQ ID NO: 215; SEQ ID NO: 216; and SEQ ID NO: 217) of the light chain variable sequence of SEQ ID NO: 11 or the light chain sequence of SEQ ID NO: 12; and polynucleotides encoding the complementarity-determining regions (SEQ ID NO: 218; SEQ ID NO: 219; and SEQ ID NO: 220) of the heavy chain variable sequence of SEQ ID NO: 13 or the heavy chain sequence of SEQ ID NO: 14.

In a preferred optional example of the description, polynucleotides of the description comprise, or alternatively consist of, polynucleotides encoding Fab (fragment antigen binding) fragments for treatment or prevention of pain and pain associated conditions having binding specificity for NGF. With respect to antibody Ab2, the polynucleotides encoding the full length Ab2 antibody comprise, or alternatively consist of, the polynucleotide SEQ ID NO: 212 encoding the light chain sequence of SEQ ID NO: 12 and the polynucleotide SEQ ID NO: 214 encoding the heavy chain sequence of SEQ ID NO: 14.

Another optional example of the description contemplates these polynucleotides incorporated into an expression vector for expression in mammalian cells such as CHO, NSO, HEK-293, or in fungal, insect, plant or microbial systems such as yeast cells such as the yeast Pichia. Suitable Pichia species include, but are not limited to, Pichia pastoris. In one example of the description described herein (infra), Fab fragments may be produced by enzymatic digestion (e.g., papain) of Ab2 following expression of the full-length polynucleotides in a suitable host. In another example of the description, anti-NGF antibodies such as Ab2 or Fab fragments thereof may be produced via expression of Ab2 polynucleotides in mammalian cells such as CHO, NSO or HEK 293 cells, fungal, insect, plant or microbial systems such as yeast cells (for example diploid yeast such as diploid Pichia) and other yeast strains. Suitable Pichia species include, but are not limited to, Pichia pastoris.

### Antibody Ab3

The description is further directed to the use of polynucleotides set forth below to produce antibody Ab3 polypeptides having binding specificity to NGF, which inhibit the association of NGF with TrkA without appreciably inhibiting the association of NGF with p75, in methods of treating pain in an individual comprising administering to said individual antibody Ab3 polypeptides.The description is further directed to polynucleotides encoding antibody polypeptides having binding specificity to NGF. In one example of the description, polynucleotides of the description comprise, or alternatively consist of, the following polynucleotide sequence encoding the variable light chain polypeptide sequence of SEQ ID NO: 21:

In one example of the description, polynucleotides of the description comprise, or alternatively consist of, the following polynucleotide sequence encoding the light chain polypeptide sequence of SEQ ID NO: 22:

In another example of the description, polynucleotides of the description comprise, or alternatively consist of, the following polynucleotide sequence encoding the variable heavy chain polypeptide sequence of SEQ ID NO: 23:

In one example of the description, polynucleotides of the description comprise, or alternatively consist of, the following polynucleotide sequence encoding the heavy chain polypeptide sequence of SEQ ID NO: 24:

In a further example of the description, polynucleotides encoding antibody fragments having binding specificity to NGF comprise, or alternatively consist of, one or more of the polynucleotide sequences of SEQ ID NO: 225; SEQ ID NO: 226; and SEQ ID NO: 227 which correspond to polynucleotides encoding the complementarity-determining regions (CDRs, or hypervariable regions) of the light chain variable sequence of SEQ ID NO: 21 or the light chain sequence of SEQ ID NO: 22.

In a further example of the description, polynucleotides encoding antibody fragments for treatment or prevention of pain and pain associated conditions having binding specificity to NGF comprise, or alternatively consist of, one or more of the polynucleotide sequences of SEQ ID NO: 228; SEQ ID NO: 229; and SEQ ID NO: 230 which correspond to polynucleotides encoding the complementarity-determining regions (CDRs, or hypervariable regions) of the heavy chain variable sequence of SEQ ID NO: 23 or the heavy chain sequence of SEQ ID NO: 24.

The description also contemplates polynucleotide sequences including one or more of the polynucleotide sequences encoding antibody fragments described herein for treatment or prevention of pain and pain associated conditions. In one example of the description, polynucleotides encoding antibody fragments having binding specificity to NGF comprise, or alternatively consist of, one, two, three or more, including all of the following polynucleotides encoding antibody fragments: the polynucleotide SEQ ID NO: 221 encoding the light chain variable sequence of SEQ ID NO: 21; the polynucleotide SEQ ID NO: 222 encoding the light chain sequence of SEQ ID NO: 22; the polynucleotide SEQ ID NO: 223 encoding the heavy chain variable sequence of SEQ ID NO: 23; the polynucleotide SEQ ID NO: 224 encoding the heavy chain sequence of SEQ ID NO: 24; polynucleotides encoding the complementarity-determining regions (SEQ ID NO: 225; SEQ ID NO: 226; and SEQ ID NO: 227) of the light chain variable sequence of SEQ ID NO: 21 or the light chain sequence of SEQ ID NO: 22; and polynucleotides encoding the complementarity-determining regions (SEQ ID NO: 228; SEQ ID NO: 229; and SEQ ID NO: 230) of the heavy chain variable sequence of SEQ ID NO: 23 or the heavy chain sequence of SEQ ID NO: 24.

In a preferred example of the description, polynucleotides of the description comprise, or alternatively consist of, polynucleotides encoding Fab (fragment antigen binding) fragments for treatment or prevention of pain and pain associated conditions having binding specificity for NGF. With respect to antibody Ab3, the polynucleotides encoding the full length Ab3 antibody comprise, or alternatively consist of, the polynucleotide SEQ ID NO: 222 encoding the light chain sequence of SEQ ID NO: 22 and the polynucleotide SEQ ID NO: 224 encoding the heavy chain sequence of SEQ ID NO: 24.

Another example of the description contemplates these polynucleotides incorporated into an expression vector for expression in mammalian cells such as CHO, NSO, HEK-293, or in fungal, insect, plant or microbial systems such as yeast cells such as the yeast Pichia. Suitable Pichia species include, but are not limited to, Pichia pastoris. In one example of the description described herein (infra), Fab fragments may be produced by enzymatic digestion (e.g., papain) of Ab3 following expression of the full-length polynucleotides in a suitable host. In another example of the description, anti-NGF antibodies such as Ab3 or Fab fragments thereof may be produced via expression of Ab3 polynucleotides in mammalian cells such as CHO, NSO or HEK 293 cells, fungal, insect, plant or microbial systems such as yeast cells (for example diploid yeast such as diploid Pichia) and other yeast strains. Suitable Pichia species include, but are not limited to, Pichia pastoris.

### Antibody Ab4

The description is further directed to the use of polynucleotides set forth below to produce antibody Ab4 polypeptides having binding specificity to NGF, which inhibit the association of NGF with TrkA without appreciably inhibiting the association of NGF with p75, in methods of treating pain in an individual comprising administering to said individual antibody Ab4 polypeptides. The description is further directed to polynucleotides encoding antibody polypeptides for treatment or prevention of pain and pain associated conditions having binding specificity to NGF. In one example of the description, polynucleotides of the description comprise, or alternatively consist of, the following polynucleotide sequence encoding the variable light chain polypeptide sequence of SEQ ID NO: 31:

In one example of the description, polynucleotides of the description comprise, or alternatively consist of, the following polynucleotide sequence encoding the light chain polypeptide sequence of SEQ ID NO: 32:

In another example of the description, polynucleotides of the description comprise, or alternatively consist of, the following polynucleotide sequence encoding the variable heavy chain polypeptide sequence of SEQ ID NO: 33:

In one example of the description, polynucleotides of the description comprise, or alternatively consist of, the following polynucleotide sequence encoding the heavy chain polypeptide sequence of SEQ ID NO: 34:

In a further example of the description, polynucleotides encoding antibody fragments for treatment or prevention of pain and pain associated conditions having binding specificity to NGF comprise, or alternatively consist of, one or more of the polynucleotide sequences of SEQ ID NO: 235; SEQ ID NO: 236; and SEQ ID NO: 237 which correspond to polynucleotides encoding the complementarity-determining regions (CDRs, or hypervariable regions) of the light chain variable sequence of SEQ ID NO: 31 or the light chain sequence of SEQ ID NO: 32.

In a further example of the description, polynucleotides encoding antibody fragments for treatment or prevention of pain and pain associated conditions having binding specificity to NGF comprise, or alternatively consist of, one or more of the polynucleotide sequences of SEQ ID NO: 238; SEQ ID NO: 239; and SEQ ID NO: 240 which correspond to polynucleotides encoding the complementarity-determining regions (CDRs, or hypervariable regions) of the heavy chain variable sequence of SEQ ID NO: 33 or the heavy chain sequence of SEQ ID NO: 34.

The description also contemplates polynucleotide sequences including one or more of the polynucleotide sequences encoding antibody fragments described herein. In one example of the description, polynucleotides encoding antibody fragments for treatment or prevention of pain and pain associated conditions having binding specificity to NGF comprise, or alternatively consist of, one, two, three or more, including all of the following polynucleotides encoding antibody fragments: the polynucleotide SEQ ID NO: 231 encoding the light chain variable sequence of SEQ ID NO: 31; the polynucleotide SEQ ID NO: 232 encoding the light chain sequence of SEQ ID NO: 32; the polynucleotide SEQ ID NO: 233 encoding the heavy chain variable sequence of SEQ ID NO: 33; the polynucleotide SEQ ID NO: 234 encoding the heavy chain sequence of SEQ ID NO: 34; polynucleotides encoding the complementarity-determining regions (SEQ ID NO: 235; SEQ ID NO: 236; and SEQ ID NO: 237) of the light chain variable sequence of SEQ ID NO: 31 or the light chain sequence of SEQ ID NO: 32; and polynucleotides encoding the complementarity-determining regions (SEQ ID NO: 238; SEQ ID NO: 239; and SEQ ID NO: 240) of the heavy chain variable sequence of SEQ ID NO: 33 or the heavy chain sequence of SEQ ID NO: 34.

In a preferred example of the description, polynucleotides of the description comprise, or alternatively consist of, polynucleotides encoding Fab (fragment antigen binding) fragments for treatment or prevention of pain and pain associated conditions having binding specificity for NGF. With respect to antibody Ab4, the polynucleotides encoding the full length Ab4 antibody comprise, or alternatively consist of, the polynucleotide SEQ ID NO: 232 encoding the light chain sequence of SEQ ID NO: 32 and the polynucleotide SEQ ID NO: 234 encoding the heavy chain sequence of SEQ ID NO: 34.

Another example of the description, contemplates these polynucleotides incorporated into an expression vector for expression in mammalian cells such as CHO, NSO, HEK-293, or in fungal, insect, plant or microbial systems such as yeast cells such as the yeast Pichia. Suitable Pichia species include, but are not limited to, Pichia pastoris. In one example of the description described herein (infra), Fab fragments may be produced by enzymatic digestion (e.g., papain) of Ab4 following expression of the full-length polynucleotides in a suitable host. In another example of the description, anti-NGF antibodies for treatment or prevention of pain and pain associated conditions such as Ab4 or Fab fragments thereof may be produced via expression of Ab4 polynucleotides in mammalian cells such as CHO, NSO or HEK 293 cells, fungal, insect, plant or microbial systems such as yeast cells (for example diploid yeast such as diploid Pichia) and other yeast strains. Suitable Pichia species include, but are not limited to, Pichia pastoris.

### Antibody Ab5

The description is further optionally directed to the use of polynucleotides set forth below to produce antibody Ab5 polypeptides for treatment or prevention of pain and pain associated conditions having binding specificity to NGF, which inhibit the association of NGF with TrkA and the association of NGF with p75, in methods of treating pain in an individual comprising administering to said individual antibody Ab5 polypeptides. The description is further directed to polynucleotides encoding antibody polypeptides for treatment or prevention of pain and pain associated conditions having binding specificity to NGF. In one example of the description, polynucleotides of the description comprise, or alternatively consist of, the following polynucleotide sequence encoding the variable light chain polypeptide sequence of SEQ ID NO: 41:

In one optional example of the description, polynucleotides of the description comprise, or alternatively consist of, the following polynucleotide sequence encoding the light chain polypeptide sequence of SEQ ID NO: 42:

**In** another optional example of the description, polynucleotides of the description comprise, or alternatively consist of, the following polynucleotide sequence encoding the variable heavy chain polypeptide sequence of SEQ ID NO: 43:

In one optional example of the description, polynucleotides of the description comprise, or alternatively consist of, the following polynucleotide sequence encoding the heavy chain polypeptide sequence of SEQ ID NO: 44:

In a further optional example of the description, polynucleotides encoding for treatment or prevention of pain and pain associated conditions fragments having binding specificity to NGF comprise, or alternatively consist of, one or more of the polynucleotide sequences of SEQ ID NO: 245; SEQ ID NO: 246; and SEQ ID NO: 247 which correspond to polynucleotides encoding the complementarity-determining regions (CDRs, or hypervariable regions) of the light chain variable sequence of SEQ ID NO: 41 or the light chain sequence of SEQ ID NO: 42.

In a further optional example of the description, polynucleotides encoding antibody fragments for treatment or prevention of pain and pain associated conditions having binding specificity to NGF comprise, or alternatively consist of, one or more of the polynucleotide sequences of SEQ ID NO: 248; SEQ ID NO: 249; and SEQ ID NO: 250 which correspond to polynucleotides encoding the complementarity-determining regions (CDRs, or hypervariable regions) of the heavy chain variable sequence of SEQ ID NO: 43 or the heavy chain sequence of SEQ ID NO: 44.

The description also optionally contemplates polynucleotide sequences including one or more of the polynucleotide sequences encoding antibody fragments for treatment or prevention of pain and pain associated conditions described herein. In one example of the description, polynucleotides encoding antibody fragments for treatment or prevention of pain and pain associated conditions having binding specificity to NGF comprise, or alternatively consist of, one, two, three or more, including all of the following polynucleotides encoding antibody fragments: the polynucleotide SEQ ID NO: 241 encoding the light chain variable sequence of SEQ ID NO: 41; the polynucleotide SEQ ID NO: 242 encoding the light chain sequence of SEQ ID NO: 42; the polynucleotide SEQ ID NO: 243 encoding the heavy chain variable sequence of SEQ ID NO: 43; the polynucleotide SEQ ID NO: 244 encoding the heavy chain sequence of SEQ ID NO: 44; polynucleotides encoding the complementarity-determining regions (SEQ ID NO: 245; SEQ ID NO: 246; and SEQ ID NO: 247) of the light chain variable sequence of SEQ ID NO: 41 or the light chain sequence of SEQ ID NO: 42; and polynucleotides encoding the complementarity-determining regions (SEQ ID NO: 248; SEQ ID NO: 249; and SEQ ID NO: 250) of the heavy chain variable sequence of SEQ ID NO: 43 or the heavy chain sequence of SEQ ID NO: 44.

In a preferred optional example of the description, polynucleotides of the description comprise, or alternatively consist of, polynucleotides encoding Fab (fragment antigen binding) fragments having binding specificity for NGF. With respect to antibody Ab5, the polynucleotides encoding the full length Ab5 antibody comprise, or alternatively consist of, the polynucleotide SEQ ID NO: 242 encoding the light chain sequence of SEQ ID NO: 42 and the polynucleotide SEQ ID NO: 244 encoding the heavy chain sequence of SEQ ID NO: 44.

Another optional example of the description contemplates these polynucleotides incorporated into an expression vector for expression in mammalian cells such as CHO, NSO, HEK-293, or in fungal, insect, plant or microbial systems such as yeast cells such as the yeast Pichia. Suitable Pichia species include, but are not limited to, Pichia pastoris. In one example of the description described herein (infra), Fab fragments may be produced by enzymatic digestion (e.g., papain) of Ab5 following expression of the full-length polynucleotides in a suitable host. In another optional example of the description, anti-NGF antibodies such as Ab5 or Fab fragments thereof may be produced via expression of Ab5 polynucleotides in mammalian cells such as CHO, NSO or HEK 293 cells, fungal, insect, plant or microbial systems such as yeast cells (for example diploid yeast such as diploid Pichia) and other yeast strains. Suitable Pichia species include, but are not limited to, Pichia pastoris.

### Antibody Ab6

The description is further optionally directed to the use of polynucleotides set forth below to produce antibody Ab6 polypeptides having binding specificity to NGF, which inhibits the association of NGF with TrkA and the association of NGF with p75, in methods of treating pain in an individual comprising administering to said individual antibody Ab6 polypeptides. The description is further directed to polynucleotides encoding for treatment or prevention of pain and pain associated conditions polypeptides having binding specificity to NGF. In one example of the description, polynucleotides of the description comprise, or alternatively consist of, the following polynucleotide sequence encoding the variable light chain polypeptide sequence of SEQ ID NO: 51:

In one optional example of the description, polynucleotides of the description comprise, or alternatively consist of, the following polynucleotide sequence encoding the light chain polypeptide sequence of SEQ ID NO: 52:

In another optional example of the description, polynucleotides of the description comprise, or alternatively consist of, the following polynucleotide sequence encoding the variable heavy chain polypeptide sequence of SEQ ID NO: 53:

In one optional example of the description, polynucleotides of the description comprise, or alternatively consist of, the following polynucleotide sequence encoding the heavy chain polypeptide sequence of SEQ ID NO: 54:

In a further optional example of the description, polynucleotides encoding antibody fragments for treatment or prevention of pain and pain associated conditions having binding specificity to NGF comprise, or alternatively consist of, one or more of the polynucleotide sequences of SEQ ID NO: 255; SEQ ID NO: 256; and SEQ ID NO: 257 which correspond to polynucleotides encoding the complementarity-determining regions (CDRs, or hypervariable regions) of the light chain variable sequence of SEQ ID NO: 51 or the light chain sequence of SEQ ID NO: 52.

In a further optional example of the description, polynucleotides encoding for treatment or prevention of pain and pain associated conditions fragments having binding specificity to NGF comprise, or alternatively consist of, one or more of the polynucleotide sequences of SEQ ID NO: 258; SEQ ID NO: 259; and SEQ ID NO: 260 which correspond to polynucleotides encoding the complementarity-determining regions (CDRs, or hypervariable regions) of the heavy chain variable sequence of SEQ ID NO: 53 or the heavy chain sequence of SEQ ID NO: 54.

The description also optionally contemplates polynucleotide sequences including one or more of the polynucleotide sequences encoding antibody fragments for treatment or prevention of pain and pain associated conditions described herein. In one example of the description, polynucleotides encoding antibody fragments for treatment or prevention of pain and pain associated conditions having binding specificity to NGF comprise, or alternatively consist of, one, two, three or more, including all of the following polynucleotides encoding antibody fragments: the polynucleotide SEQ ID NO: 251 encoding the light chain variable sequence of SEQ ID NO: 51; the polynucleotide SEQ ID NO: 252 encoding the light chain sequence of SEQ ID NO: 52; the polynucleotide SEQ ID NO: 253 encoding the heavy chain variable sequence of SEQ ID NO: 53; the polynucleotide SEQ ID NO: 254 encoding the heavy chain sequence of SEQ ID NO: 54; polynucleotides encoding the complementarity-determining regions (SEQ ID NO: 255; SEQ ID NO: 256; and SEQ ID NO: 257) of the light chain variable sequence of SEQ ID NO: 51 or the light chain sequence of SEQ ID NO: 52; and polynucleotides encoding the complementarity-determining regions (SEQ ID NO: 258; SEQ ID NO: 259; and SEQ ID NO: 260) of the heavy chain variable sequence of SEQ ID NO: 53 or the heavy chain sequence of SEQ ID NO: 54.

In a preferred optional example of the description, polynucleotides of the description comprise, or alternatively consist of, polynucleotides encoding Fab (fragment antigen binding) fragments having binding specificity for NGF. With respect to antibody Ab6, the polynucleotides encoding the full length Ab6 antibody comprise, or alternatively consist of, the polynucleotide SEQ ID NO: 252 encoding the light chain sequence of SEQ ID NO: 52 and the polynucleotide SEQ ID NO: 254 encoding the heavy chain sequence of SEQ ID NO: 54.

Another optional example of the description contemplates these polynucleotides incorporated into an expression vector for expression in mammalian cells such as CHO, NSO, HEK-293, or in fungal, insect, plant or microbial systems such as yeast cells such as the yeast Pichia. Suitable Pichia species include, but are not limited to, Pichia pastoris. In one example of the description described herein (infra), Fab fragments may be produced by enzymatic digestion (e.g., papain) of Ab6 following expression of the full-length polynucleotides in a suitable host. In another example of the description, anti-NGF antibodies such as Ab6 or Fab fragments thereof may be produced via expression of Ab6 polynucleotides in mammalian cells such as CHO, NSO or HEK 293 cells, fungal, insect, plant or microbial systems such as yeast cells (for example diploid yeast such as diploid Pichia) and other yeast strains. Suitable Pichia species include, but are not limited to, Pichia pastoris.

### Antibody Ab7

The description optionally is further directed to the use of polynucleotides set forth below to produce antibody Ab7 polypeptides having binding specificity to NGF, which inhibit the association of NGF with TrkA and the association of NGF with p75. in methods of treating pain in an individual comprising administering to said individual antibody Ab7 polypeptides. The description is further directed to polynucleotides encoding antibody polypeptides for treatment or prevention of pain and pain associated conditions having binding specificity to NGF. In one example of the description, polynucleotides of the description comprise, or alternatively consist of, the following polynucleotide sequence encoding the variable light chain polypeptide sequence of SEQ ID NO: 61:

In one optional example of the description, polynucleotides of the description comprise, or alternatively consist of, the following polynucleotide sequence encoding the light chain polypeptide sequence of SEQ ID NO: 62:

In another optional example of the description, polynucleotides of the description comprise, or alternatively consist of, the following polynucleotide sequence encoding the variable heavy chain polypeptide sequence of SEQ ID NO: 63:

In one optional example of the description, polynucleotides of the description comprise, or alternatively consist of, the following polynucleotide sequence encoding the heavy chain polypeptide sequence of SEQ ID NO: 64:

In a further optional example of the description, polynucleotides encoding antibody fragments for treatment or prevention of pain and pain associated conditions having binding specificity to NGF comprise, or alternatively consist of, one or more of the polynucleotide sequences of SEQ ID NO: 265; SEQ ID NO: 266; and SEQ ID NO: 267 which correspond to polynucleotides encoding the complementarity-determining regions (CDRs, or hypervariable regions) of the light chain variable sequence of SEQ ID NO: 61 or the light chain sequence of SEQ ID NO: 62.

In a further optional example of the description, polynucleotides encoding antibody fragments for treatment or prevention of pain and pain associated conditions having binding specificity to NGF comprise, or alternatively consist of, one or more of the polynucleotide sequences of SEQ ID NO: 268; SEQ ID NO: 269; and SEQ ID NO: 270 which correspond to polynucleotides encoding the complementarity-determining regions (CDRs, or hypervariable regions) of the heavy chain variable sequence of SEQ ID NO: 63 or the heavy chain sequence of SEQ ID NO: 64.

The description also optionally contemplates polynucleotide sequences including one or more of the polynucleotide sequences encoding antibody fragments for treatment or prevention of pain and pain associated conditions described herein. In one example of the description polynucleotides encoding antibody fragments for treatment or prevention of pain and pain associated conditions having binding specificity to NGF comprise, or alternatively consist of, one, two, three or more, including all of the following polynucleotides encoding antibody fragments: the polynucleotide SEQ ID NO: 261 encoding the light chain variable sequence of SEQ ID NO: 61; the polynucleotide SEQ ID NO: 262 encoding the light chain sequence of SEQ ID NO: 62; the polynucleotide SEQ ID NO: 263 encoding the heavy chain variable sequence of SEQ ID NO: 63; the polynucleotide SEQ ID NO: 264 encoding the heavy chain sequence of SEQ ID NO: 64; polynucleotides encoding the complementarity-determining regions (SEQ ID NO: 265; SEQ ID NO: 266; and SEQ ID NO: 267) of the light chain variable sequence of SEQ ID NO: 61 or the light chain sequence of SEQ ID NO: 62; and polynucleotides encoding the complementarity-determining regions (SEQ ID NO: 268; SEQ ID NO: 269; and SEQ ID NO: 270) of the heavy chain variable sequence of SEQ ID NO: 63 or the heavy chain sequence of SEQ ID NO: 64.

In a preferred optional example of the description, polynucleotides of the description comprise, or alternatively consist of, polynucleotides encoding Fab (fragment antigen binding) fragments having binding specificity for NGF. With respect to antibody Ab7, the polynucleotides encoding the full length Ab7 antibody comprise, or alternatively consist of, the polynucleotide SEQ ID NO: 262 encoding the light chain sequence of SEQ ID NO: 62 and the polynucleotide SEQ ID NO: 264 encoding the heavy chain sequence of SEQ ID NO: 64.

Another optional example of the description contemplates these polynucleotides incorporated into an expression vector for expression in mammalian cells such as CHO, NSO, HEK-293, or in fungal, insect, plant or microbial systems such as yeast cells such as the yeast Pichia. Suitable Pichia species include, but are not limited to, Pichia pastoris. In one example of the description described herein (infra), Fab fragments may be produced by enzymatic digestion (e.g., papain) of Ab7 following expression of the full-length polynucleotides in a suitable host. In another example of the description, anti-NGF antibodies such as Ab7 or Fab fragments thereof may be produced via expression of Ab7 polynucleotides in mammalian cells such as CHO, NSO or HEK 293 cells, fungal, insect, plant or microbial systems such as yeast cells (for example diploid yeast such as diploid Pichia) and other yeast strains. Suitable Pichia species include, but are not limited to, Pichia pastoris.

### Antibody Ab8

The description is further optionally directed to the use of polynucleotides set forth below to produce antibody Ab8 polypeptides having binding specificity to NGF, which inhibits the association of NGF with TrkA and the association of NGF with p75, in methods of treating pain in an individual comprising administering to said individual antibody Ab8 polypeptides. The description is further optionally directed to polynucleotides encoding antibody polypeptides for treatment or prevention of pain and pain associated conditions having binding specificity to NGF. In one example of the description, polynucleotides of the description comprise, or alternatively consist of, the following polynucleotide sequence encoding the variable light chain polypeptide sequence of SEQ ID NO: 71:

In one optional example of the description, polynucleotides of the description comprise, or alternatively consist of, the following polynucleotide sequence encoding the light chain polypeptide sequence of SEQ ID NO: 72:

In another optional example of the description, polynucleotides of the description comprise, or alternatively consist of, the following polynucleotide sequence encoding the variable heavy chain polypeptide sequence of SEQ ID NO: 73:

In one optional example of the description, polynucleotides of the description comprise, or alternatively consist of, the following polynucleotide sequence encoding the heavy chain polypeptide sequence of SEQ ID NO: 74:

In a further optional example of the description, polynucleotides encoding antibody fragments for treatment or prevention of pain and pain associated conditions having binding specificity to NGF comprise, or alternatively consist of, one or more of the polynucleotide sequences of SEQ ID NO: 275; SEQ ID NO: 276; and SEQ ID NO: 277 which correspond to polynucleotides encoding the complementarity-determining regions (CDRs, or hypervariable regions) of the light chain variable sequence of SEQ ID NO: 71 or the light chain sequence of SEQ ID NO: 72.

In a further optional example of the description, polynucleotides encoding antibody fragments for treatment or prevention of pain and pain associated conditions having binding specificity to NGF comprise, or alternatively consist of, one or more of the polynucleotide sequences of SEQ ID NO: 278; SEQ ID NO: 279; and SEQ ID NO: 280 which correspond to polynucleotides encoding the complementarity-determining regions (CDRs, or hypervariable regions) of the heavy chain variable sequence of SEQ ID NO: 73 or the heavy chain sequence of SEQ ID NO: 74.

The description also optionally contemplates polynucleotide sequences including one or more of the polynucleotide sequences encoding antibody fragments for treatment or prevention of pain and pain associated conditions described herein. In one example of the description, polynucleotides encoding antibody fragments for treatment or prevention of pain and pain associated conditions having binding specificity to NGF comprise, or alternatively consist of, one, two, three or more, including all of the following polynucleotides encoding antibody fragments: the polynucleotide SEQ ID NO: 271 encoding the light chain variable sequence of SEQ ID NO: 71; the polynucleotide SEQ ID NO: 272 encoding the light chain sequence of SEQ ID NO: 72; the polynucleotide SEQ ID NO: 273 encoding the heavy chain variable sequence of SEQ ID NO: 73; the polynucleotide SEQ ID NO: 274 encoding the heavy chain sequence of SEQ ID NO: 74; polynucleotides encoding the complementarity-determining regions (SEQ ID NO: 275; SEQ ID NO: 276; and SEQ ID NO: 277) of the light chain variable sequence of SEQ ID NO: 71 or the light chain sequence of SEQ ID NO: 72; and polynucleotides encoding the complementarity-determining regions (SEQ ID NO: 278; SEQ ID NO: 279; and SEQ ID NO: 280) of the heavy chain variable sequence of SEQ ID NO: 73 or the heavy chain sequence of SEQ ID NO: 74.

In a preferred optional example of the description, polynucleotides of the description comprise, or alternatively consist of, polynucleotides encoding Fab (fragment antigen binding) fragments having binding specificity for NGF. With respect to antibody Ab8, the polynucleotides encoding the full length Ab8 antibody comprise, or alternatively consist of, the polynucleotide SEQ ID NO: 272 encoding the light chain sequence of SEQ ID NO: 72 and the polynucleotide SEQ ID NO: 274 encoding the heavy chain sequence of SEQ ID NO: 74.

Another optional example of the description, contemplates these polynucleotides incorporated into an expression vector for expression in mammalian cells such as CHO, NSO, HEK-293, or in fungal, insect, plant or microbial systems such as yeast cells such as the yeast Pichia. Suitable Pichia species include, but are not limited to, Pichia pastoris. In one example of the description described herein (infra), Fab fragments may be produced by enzymatic digestion (e.g., papain) of Ab8 following expression of the full-length polynucleotides in a suitable host. In another example of the description, anti-NGF antibodies such as Ab8 or Fab fragments thereof may be produced via expression of Ab8 polynucleotides in mammalian cells such as CHO, NSO or HEK 293 cells, fungal, insect, plant or microbial systems such as yeast cells (for example diploid yeast such as diploid Pichia) and other yeast strains. Suitable Pichia species include, but are not limited to, Pichia pastoris.

### Antibody Ab9

The description is further optionally directed to the use of polynucleotides set forth below to produce antibody Ab9 p example of the description, ecificity to NGF, which inhibit the association of NGF with TrkA and the association of NGF with p75, in methods of treating pain in an individual comprising administering to said individual antibody Ab9 polypeptides. The description is further optionally directed to polynucleotides encoding antibody polypeptides for treatment or prevention of pain and pain associated conditions having binding specificity to NGF. In one example of the description, polynucleotides of the description comprise, or alternatively consist of, the following polynucleotide sequence encoding the variable light chain polypeptide sequence of SEQ ID NO: 81:

In one optional example of the description, polynucleotides of the description comprise, or alternatively consist of, the following polynucleotide sequence encoding the light chain polypeptide sequence of SEQ ID NO: 82:

In another optional example of the description, polynucleotides of the description comprise, or alternatively consist of, the following polynucleotide sequence encoding the variable heavy chain polypeptide sequence of SEQ ID NO: 83:

In one optional example of the description, polynucleotides of the description comprise, or alternatively consist of, the following polynucleotide sequence encoding the heavy chain polypeptide sequence of SEQ ID NO: 84:

In a further optional example of the description, polynucleotides encoding antibody fragments for treatment or prevention of pain and pain associated conditions having binding specificity to NGF comprise, or alternatively consist of, one or more of the polynucleotide sequences of SEQ ID NO: 285; SEQ ID NO: 286; and SEQ ID NO: 287 which correspond to polynucleotides encoding the complementarity-determining regions (CDRs, or hypervariable regions) of the light chain variable sequence of SEQ ID NO: 81 or the light chain sequence of SEQ ID NO: 82.

In a further optional example of the description, polynucleotides encoding antibody fragments for treatment or prevention of pain and pain associated conditions having binding specificity to NGF comprise, or alternatively consist of, one or more of the polynucleotide sequences of SEQ ID NO: 288; SEQ ID NO: 289; and SEQ ID NO: 290 which correspond to polynucleotides encoding the complementarity-determining regions (CDRs, or hypervariable regions) of the heavy chain variable sequence of SEQ ID NO: 83 or the heavy chain sequence of SEQ ID NO: 84.

The description also optionally contemplates polynucleotide sequences including one or more of the polynucleotide sequences encoding antibody fragments for treatment or prevention of pain and pain associated conditions described herein. In one optional example of the description, polynucleotides encoding antibody fragments for treatment or prevention of pain and pain associated conditions having binding specificity to NGF comprise, or alternatively consist of, one, two, three or more, including all of the following polynucleotides encoding antibody fragments: the polynucleotide SEQ ID NO: 281 encoding the light chain variable sequence of SEQ ID NO: 81; the polynucleotide SEQ ID NO: 282 encoding the light chain sequence of SEQ ID NO: 82; the polynucleotide SEQ ID NO: 283 encoding the heavy chain variable sequence of SEQ ID NO: 83; the polynucleotide SEQ ID NO: 284 encoding the heavy chain sequence of SEQ ID NO: 84; polynucleotides encoding the complementarity-determining regions (SEQ ID NO: 285; SEQ ID NO: 286; and SEQ ID NO: 287) of the light chain variable sequence of SEQ ID NO: 81 or the light chain sequence of SEQ ID NO: 82; and polynucleotides encoding the complementarity-determining regions (SEQ ID NO: 288; SEQ ID NO: 289; and SEQ ID NO: 290) of the heavy chain variable sequence of SEQ ID NO: 83 or the heavy chain sequence of SEQ ID NO: 84.

In a preferred optional example of the description, polynucleotides of the description comprise, or alternatively consist of, polynucleotides encoding Fab (fragment antigen binding) fragments having binding specificity for NGF. With respect to antibody Ab9, the polynucleotides encoding the full length Ab9 antibody comprise, or alternatively consist of, the polynucleotide SEQ ID NO: 282 encoding the light chain sequence of SEQ ID NO: 82 and the polynucleotide SEQ ID NO: 284 encoding the heavy chain sequence of SEQ ID NO: 84.

Another optional example of the description contemplates these polynucleotides incorporated into an expression vector for expression in mammalian cells such as CHO, NSO, HEK-293, or in fungal, insect, plant or microbial systems such as yeast cells such as the yeast Pichia. Suitable Pichia species include, but are not limited to, Pichia pastoris. In one example of the description described herein (infra), Fab fragments may be produced by enzymatic digestion (e.g., papain) of Ab9 following expression of the full-length polynucleotides in a suitable host. In another example of the description, anti-NGF antibodies such as Ab9 or Fab fragments thereof may be produced via expression of Ab9 polynucleotides in mammalian cells such as CHO, NSO or HEK 293 cells, fungal, insect, plant or microbial systems such as yeast cells (for example diploid yeast such as diploid Pichia) and other yeast strains. Suitable Pichia species include, but are not limited to, Pichia pastoris.

### Antibody Ab10

The description is further optionally directed to the use of polynucleotides set forth below to produce antibody Ab10 polypeptides having binding specificity to NGF, which inhibit the association of NGF with TrkA and the association of NGF with p75, in methods of treating pain in an individual comprising administering to said individual antibody Ab10 polypeptides. The description is further optionally directed to polynucleotides encoding antibody polypeptides having binding specificity to NGF. In one example of the description, polynucleotides of the description comprise, or alternatively consist of, the following polynucleotide sequence encoding the variable light chain polypeptide sequence of SEQ ID NO: 91:

In one optional example of the description, polynucleotides of the description comprise, or alternatively consist of, the following polynucleotide sequence encoding the light chain polypeptide sequence of SEQ ID NO: 92:

In another optional example of the description, polynucleotides of the description comprise, or alternatively consist of, the following polynucleotide sequence encoding the variable heavy chain polypeptide sequence of SEQ ID NO: 93:

In one optional example of the description, polynucleotides of the description comprise, or alternatively consist of, the following polynucleotide sequence encoding the heavy chain polypeptide sequence of SEQ ID NO: 94:

In a further optional example of the description, polynucleotides encoding antibody fragments for treatment or prevention of pain and pain associated conditions having binding specificity to NGF comprise, or alternatively consist of, one or more of the polynucleotide sequences of SEQ ID NO: 295; SEQ ID NO: 296; and SEQ ID NO: 297 which correspond to polynucleotides encoding the complementarity-determining regions (CDRs, or hypervariable regions) of the light chain variable sequence of SEQ ID NO: 91 or the light chain sequence of SEQ ID NO: 92.

In a further optional example of the description, polynucleotides encoding antibody fragments for treatment or prevention of pain and pain associated conditions having binding specificity to NGF comprise, or alternatively consist of, one or more of the polynucleotide sequences of SEQ ID NO: 298; SEQ ID NO: 299; and SEQ ID NO: 300 which correspond to polynucleotides encoding the complementarity-determining regions (CDRs, or hypervariable regions) of the heavy chain variable sequence of SEQ ID NO: 93 or the heavy chain sequence of SEQ ID NO: 94.

The description also optionally contemplates polynucleotide sequences including one or more of the polynucleotide sequences encoding antibody fragments for treatment or prevention of pain and pain associated conditions described herein. In one example of the description, polynucleotides encoding antibody fragments for treatment or prevention of pain and pain associated conditions having binding specificity to NGF comprise, or alternatively consist of, one, two, three or more, including all of the following polynucleotides encoding antibody fragments: the polynucleotide SEQ ID NO: 291 encoding the light chain variable sequence of SEQ ID NO: 91; the polynucleotide SEQ ID NO: 292 encoding the light chain sequence of SEQ ID NO: 92; the polynucleotide SEQ ID NO: 293 encoding the heavy chain variable sequence of SEQ ID NO: 93; the polynucleotide SEQ ID NO: 294 encoding the heavy chain sequence of SEQ ID NO: 94; polynucleotides encoding the complementarity-determining regions (SEQ ID NO: 295; SEQ ID NO: 296; and SEQ ID NO: 297) of the light chain variable sequence of SEQ ID NO: 91 or the light chain sequence of SEQ ID NO: 92; and polynucleotides encoding the complementarity-determining regions (SEQ ID NO: 298; SEQ ID NO: 299; and SEQ ID NO: 300) of the heavy chain variable sequence of SEQ ID NO: 93 or the heavy chain sequence of SEQ ID NO: 94.

In a preferred optional example of the description, polynucleotides of the description comprise, or alternatively consist of, polynucleotides encoding Fab (fragment antigen binding) fragments having binding specificity for NGF. With respect to antibody Ab10, the polynucleotides encoding the full length Ab10 antibody comprise, or alternatively consist of, the polynucleotide SEQ ID NO: 292 encoding the light chain sequence of SEQ ID NO: 92 and the polynucleotide SEQ ID NO: 294 encoding the heavy chain sequence of SEQ ID NO: 94.

Another optional example of the description contemplates these polynucleotides incorporated into an expression vector for expression in mammalian cells such as CHO, NSO, HEK-293, or in fungal, insect, plant or microbial systems such as yeast cells such as the yeast Pichia. Suitable Pichia species include, but are not limited to, Pichia pastoris. In one example of the description described herein (infra), Fab fragments may be produced by enzymatic digestion (e.g., papain) of AblO following expression of the full-length polynucleotides in a suitable host. In another example of the description, anti-NGF antibodies such as Ab10 or Fab fragments thereof may be produced via expression of AblO polynucleotides in mammalian cells such as CHO, NSO or HEK 293 cells, fungal, insect, plant or microbial systems such as yeast cells (for example diploid yeast such as diploid Pichia) and other yeast strains. Suitable Pichia species include, but are not limited to, Pichia pastoris.

### Antibody Ab11

The description is further optionally directed to the use of polynucleotides set forth below to produce antibody Ab11 polypeptides having binding specificity to NGF, which inhibits the association of NGF with TrkA and the association of NGF with p75, in methods of treating pain in an individual comprising administering to said individual antibody Ab11 polypeptides. The description is further directed to polynucleotides encoding antibody polypeptides having binding specificity to NGF. In one example of the description, polynucleotides of the description comprise, or alternatively consist of, the following polynucleotide sequence encoding the variable light chain polypeptide sequence of SEQ ID NO: 101:

In one optional example of the description, polynucleotides of the description comprise, or alternatively consist of, the following polynucleotide sequence encoding the light chain polypeptide sequence of SEQ ID NO: 102:

In another optional example of the description, polynucleotides of the description comprise, or alternatively consist of, the following polynucleotide sequence encoding the variable heavy chain polypeptide sequence of SEQ ID NO: 103:

In one optional example of the description, polynucleotides of the description comprise, or alternatively consist of, the following polynucleotide sequence encoding the heavy chain polypeptide sequence of SEQ ID NO: 104:

In a further optional example of the description, polynucleotides encoding antibody fragments for treatment or prevention of pain and pain associated conditions having binding specificity to NGF comprise, or alternatively consist of, one or more of the polynucleotide sequences of SEQ ID NO: 305; SEQ ID NO: 306; and SEQ ID NO: 307 which correspond to polynucleotides encoding the complementarity-determining regions (CDRs, or hypervariable regions) of the light chain variable sequence of SEQ ID NO: 101 or the light chain sequence of SEQ ID NO: 102.

In a further optional example of the description, polynucleotides encoding for treatment or prevention of pain and pain associated conditions fragments having binding specificity to NGF comprise, or alternatively consist of, one or more of the polynucleotide sequences of SEQ ID NO: 308; SEQ ID NO: 309; and SEQ ID NO: 310 which correspond to polynucleotides encoding the complementarity-determining regions (CDRs, or hypervariable regions) of the heavy chain variable sequence of SEQ ID NO: 103 or the heavy chain sequence of SEQ ID NO: 104.

The description also optionally contemplates polynucleotide sequences including one or more of the polynucleotide sequences encoding antibody fragments for treatment or prevention of pain and pain associated conditions described herein. In one example of the description, polynucleotides encoding antibody fragments for treatment or prevention of pain and pain associated conditions having binding specificity to NGF comprise, or alternatively consist of, one, two, three or more, including all of the following polynucleotides encoding antibody fragments: the polynucleotide SEQ ID NO: 301 encoding the light chain variable sequence of SEQ ID NO: 101; the polynucleotide SEQ ID NO: 302 encoding the light chain sequence of SEQ ID NO: 102; the polynucleotide SEQ ID NO: 303 encoding the heavy chain variable sequence of SEQ ID NO: 103; the polynucleotide SEQ ID NO: 304 encoding the heavy chain sequence of SEQ ID NO: 104; polynucleotides encoding the complementarity-determining regions (SEQ ID NO: 305; SEQ ID NO: 306; and SEQ ID NO: 307) of the light chain variable sequence of SEQ ID NO: 101 or the light chain sequence of SEQ ID NO: 102; and polynucleotides encoding the complementarity-determining regions (SEQ ID NO: 308; SEQ ID NO: 309; and SEQ ID NO: 310) of the heavy chain variable sequence of SEQ ID NO: 103 or the heavy chain sequence of SEQ ID NO: 104.

In a preferred optional example of the description, polynucleotides of the description comprise, or alternatively consist of, polynucleotides encoding Fab (fragment antigen binding) fragments having binding specificity for NGF. With respect to antibody Ab11, the polynucleotides encoding the full length Ab11 antibody comprise, or alternatively consist of, the polynucleotide SEQ ID NO: 302 encoding the light chain sequence of SEQ ID NO: 102 and the polynucleotide SEQ ID NO: 304 encoding the heavy chain sequence of SEQ ID NO: 104.

Another optional example of the description contemplates these polynucleotides incorporated into an expression vector for expression in mammalian cells such as CHO, NSO, HEK-293, or in fungal, insect, plant or microbial systems such as yeast cells such as the yeast Pichia. Suitable Pichia species include, but are not limited to, Pichia pastoris. In one example of the description described herein (infra), Fab fragments may be produced by enzymatic digestion (e.g., papain) of Ab11 following expression of the full-length polynucleotides in a suitable host. In another optional example of the description, anti-NGF antibodies such as Ab11 or Fab fragments thereof may be produced via expression of Ab11 polynucleotides in mammalian cells such as CHO, NSO or HEK 293 cells, fungal, insect, plant or microbial systems such as yeast cells (for example diploid yeast such as diploid Pichia) and other yeast strains. Suitable Pichia species include, but are not limited to, Pichia pastoris.

### Antibody Ab12

The description is further optionally directed to the use of polynucleotides set forth below to produce antibody Ab12 polypeptides having binding specificity to NGF, which inhibit the association of NGF with TrkA and the association of NGF with p75, in methods of treating pain in an individual comprising administering to said individual antibody Ab12 polypeptides. The description is further optionally directed to polynucleotides encoding antibody polypeptides for treatment or prevention of pain and pain associated conditions having binding specificity to NGF. In one example of the description, polynucleotides of the description comprise, or alternatively consist of, the following polynucleotide sequence encoding the variable light chain polypeptide sequence of SEQ ID NO: 111:

In one optional example of the description, polynucleotides of the description comprise, or alternatively consist of, the following polynucleotide sequence encoding the light chain polypeptide sequence of SEQ ID NO: 112:

In another optional example of the description, polynucleotides of the description comprise, or alternatively consist of, the following polynucleotide sequence encoding the variable heavy chain polypeptide sequence of SEQ ID NO: 113:

In one optional example of the description, polynucleotides of the description comprise, or alternatively consist of, the following polynucleotide sequence encoding the heavy chain polypeptide sequence of SEQ ID NO: 114:

In a further optional example of the description, polynucleotides encoding antibody fragments for treatment or prevention of pain and pain associated conditions having binding specificity to NGF comprise, or alternatively consist of, one or more of the polynucleotide sequences of SEQ ID NO: 315; SEQ ID NO: 316; and SEQ ID NO: 317 which correspond to polynucleotides encoding the complementarity-determining regions (CDRs, or hypervariable regions) of the light chain variable sequence of SEQ ID NO: 111 or the light chain sequence of SEQ ID NO: 112.

In a further optional example of the description, polynucleotides encoding antibody fragments for treatment or prevention of pain and pain associated conditions having binding specificity to NGF comprise, or alternatively consist of, one or more of the polynucleotide sequences of SEQ ID NO: 318; SEQ ID NO: 319; and SEQ ID NO: 320 which correspond to polynucleotides encoding the complementarity-determining regions (CDRs, or hypervariable regions) of the heavy chain variable sequence of SEQ ID NO: 113 or the heavy chain sequence of SEQ ID NO: 114.

The description also optionally contemplates polynucleotide sequences including one or more of the polynucleotide sequences encoding antibody fragments for treatment or prevention of pain and pain associated conditions described herein. In one example of the description, polynucleotides encoding antibody fragments for treatment or prevention of pain and pain associated conditions having binding specificity to NGF comprise, or alternatively consist of, one, two, three or more, including all of the following polynucleotides encoding antibody fragments: the polynucleotide SEQ ID NO: 311 encoding the light chain variable sequence of SEQ ID NO: 111; the polynucleotide SEQ ID NO: 312 encoding the light chain sequence of SEQ ID NO: 112; the polynucleotide SEQ ID NO: 313 encoding the heavy chain variable sequence of SEQ ID NO: 113; the polynucleotide SEQ ID NO: 314 encoding the heavy chain sequence of SEQ ID NO: 114; polynucleotides encoding the complementarity-determining regions (SEQ ID NO: 315; SEQ ID NO: 316; and SEQ ID NO: 317) of the light chain variable sequence of SEQ ID NO: 111 or the light chain sequence of SEQ ID NO: 112; and polynucleotides encoding the complementarity-determining regions (SEQ ID NO: 318; SEQ ID NO: 319; and SEQ ID NO: 320) of the heavy chain variable sequence of SEQ ID NO: 113 or the heavy chain sequence of SEQ ID NO: 114.

In a preferred optional example of the description, polynucleotides of the description comprise, or alternatively consist of, polynucleotides encoding Fab (fragment antigen binding) fragments having binding specificity for NGF. With respect to antibody Ab12, the polynucleotides encoding the full length Ab12 antibody comprise, or alternatively consist of, the polynucleotide SEQ ID NO: 312 encoding the light chain sequence of SEQ ID NO: 112 and the polynucleotide SEQ ID NO: 314 encoding the heavy chain sequence of SEQ ID NO: 114.

Another optional example of the description contemplates these polynucleotides incorporated into an expression vector for expression in mammalian cells such as CHO, NSO, HEK-293, or in fungal, insect, plant or microbial systems such as yeast cells such as the yeast Pichia. Suitable Pichia species include, but are not limited to, Pichia pastoris. In one example of the description described herein (infra), Fab fragments may be produced by enzymatic digestion (e.g., papain) of Ab12 following expression of the full-length polynucleotides in a suitable host. In another example of the description, anti-NGF antibodies such as Ab12 or Fab fragments thereof may be produced via expression of Ab12 polynucleotides in mammalian cells such as CHO, NSO or HEK 293 cells, fungal, insect, plant or microbial systems such as yeast cells (for example diploid yeast such as diploid Pichia) and other yeast strains. Suitable Pichia species include, but are not limited to, Pichia pastoris.

### Antibody Ab13

The description is further optionally directed to the use of polynucleotides set forth below to produce antibody Ab13 polypeptides having binding specificity to NGF, which inhibit the association of NGF with TrkA and the association of NGF with p75, in methods of treating pain in an individual comprising administering to said individual antibody Ab13 polypeptides. The description is further optionally directed to polynucleotides encoding antibody polypeptides having binding specificity to NGF. In one example of the description, polynucleotides of the description comprise, or alternatively consist of, the following polynucleotide sequence encoding the variable light chain polypeptide sequence of SEQ ID NO: 121:

**In** one optional example of the description, polynucleotides of the description comprise, or alternatively consist of, the following polynucleotide sequence encoding the light chain polypeptide sequence of SEQ ID NO: 122:

In another optional example of the description, polynucleotides of the description comprise, or alternatively consist of, the following polynucleotide sequence encoding the variable heavy chain polypeptide sequence of SEQ ID NO: 123:

In one optional example of the description, polynucleotides of the description comprise, or alternatively consist of, the following polynucleotide sequence encoding the heavy chain polypeptide sequence of SEQ ID NO: 124:

In a further optional example of the description, polynucleotides encoding antibody fragments for treatment or prevention of pain and pain associated conditions having binding specificity to NGF comprise, or alternatively consist of, one or more of the polynucleotide sequences of SEQ ID NO: 325; SEQ ID NO: 326; and SEQ ID NO: 327 which correspond to polynucleotides encoding the complementarity-determining regions (CDRs, or hypervariable regions) of the light chain variable sequence of SEQ ID NO: 121 or the light chain sequence of SEQ ID NO: 122.

In a further optional example of the description, , polynucleotides encoding antibody fragments for treatment or prevention of pain and pain associated conditions having binding specificity to NGF comprise, or alternatively consist of, one or more of the polynucleotide sequences of SEQ ID NO: 328; SEQ ID NO: 329; and SEQ ID NO: 330 which correspond to polynucleotides encoding the complementarity-determining regions (CDRs, or hypervariable regions) of the heavy chain variable sequence of SEQ ID NO: 123 or the heavy chain sequence of SEQ ID NO: 124.

The description also optionally contemplates polynucleotide sequences including one or more of the polynucleotide sequences encoding antibody fragments for treatment or prevention of pain and pain associated conditions described herein. In one example of the description, polynucleotides encoding antibody fragments for treatment or prevention of pain and pain associated conditions having binding specificity to NGF comprise, or alternatively consist of, one, two, three or more, including all of the following polynucleotides encoding antibody fragments: the polynucleotide SEQ ID NO: 321 encoding the light chain variable sequence of SEQ ID NO: 121; the polynucleotide SEQ ID NO: 322 encoding the light chain sequence of SEQ ID NO: 122; the polynucleotide SEQ ID NO: 323 encoding the heavy chain variable sequence of SEQ ID NO: 123; the polynucleotide SEQ ID NO: 324 encoding the heavy chain sequence of SEQ ID NO: 124; polynucleotides encoding the complementarity-determining regions (SEQ ID NO: 325; SEQ ID NO: 326; and SEQ ID NO: 327) of the light chain variable sequence of SEQ ID NO: 121 or the light chain sequence of SEQ ID NO: 122; and polynucleotides encoding the complementarity-determining regions (SEQ ID NO: 328; SEQ ID NO: 329; and SEQ ID NO: 330) of the heavy chain variable sequence of SEQ ID NO: 123 or the heavy chain sequence of SEQ ID NO: 124.

In a preferred optional example of the description, polynucleotides of the description comprise, or alternatively consist of, polynucleotides encoding Fab (fragment antigen binding) fragments having binding specificity for NGF. With respect to antibody Ab13, the polynucleotides encoding the full length Ab13 antibody comprise, or alternatively consist of, the polynucleotide SEQ ID NO: 322 encoding the light chain sequence of SEQ ID NO: 122 and the polynucleotide SEQ ID NO: 324 encoding the heavy chain sequence of SEQ ID NO: 124.

Another optional example of the description contemplates these polynucleotides incorporated into an expression vector for expression in mammalian cells such as CHO, NSO, HEK-293, or in fungal, insect, plant or microbial systems such as yeast cells such as the yeast Pichia. Suitable Pichia species include, but are not limited to, Pichia pastoris. In one example of the description described herein (infra), Fab fragments may be produced by enzymatic digestion (e.g., papain) of Ab13 following expression of the full-length polynucleotides in a suitable host. In another example of the description, anti-NGF antibodies such as Ab13 or Fab fragments thereof may be produced via expression of Ab13 polynucleotides in mammalian cells such as CHO, NSO or HEK 293 cells, fungal, insect, plant or microbial systems such as yeast cells (for example diploid yeast such as diploid Pichia) and other yeast strains. Suitable Pichia species include, but are not limited to, Pichia pastoris.

### Antibody Ab14

The description is further optionally directed to the use of polynucleotides set forth below to produce antibody Ab14 polypeptides having binding specificity to NGF, which inhibit the association of NGF with TrkA and the association of NGF with p75, in methods of treating pain in an individual comprising administering to said individual antibody Ab14 polypeptides. The description is further optionally directed to polynucleotides encoding antibody polypeptides having binding specificity to NGF. In one example of the description, polynucleotides of the description comprise, or alternatively consist of, the following polynucleotide sequence encoding the variable light chain polypeptide sequence of SEQ ID NO: 131:

In one optional example of the description, polynucleotides of the description comprise, or alternatively consist of, the following polynucleotide sequence encoding the light chain polypeptide sequence of SEQ ID NO: 132:

In another optional example of the description, polynucleotides of the description comprise, or alternatively consist of, the following polynucleotide sequence encoding the variable heavy chain polypeptide sequence of SEQ ID NO: 133:

In one optional example of the description, polynucleotides of the description comprise, or alternatively consist of, the following polynucleotide sequence encoding the heavy chain polypeptide sequence of SEQ ID NO: 134:

In a further optional example of the description, polynucleotides encoding antibody fragments for treatment or prevention of pain and pain associated conditions having binding specificity to NGF comprise, or alternatively consist of, one or more of the polynucleotide sequences of SEQ ID NO: 335; SEQ ID NO: 336; and SEQ ID NO: 337 which correspond to polynucleotides encoding the complementarity-determining regions (CDRs, or hypervariable regions) of the light chain variable sequence of SEQ ID NO: 131 or the light chain sequence of SEQ ID NO: 132.

In a further optional example of the description, polynucleotides encoding antibody fragments for treatment or prevention of pain and pain associated conditions having binding specificity to NGF comprise, or alternatively consist of, one or more of the polynucleotide sequences of SEQ ID NO: 338; SEQ ID NO: 339; and SEQ ID NO: 340 which correspond to polynucleotides encoding the complementarity-determining regions (CDRs, or hypervariable regions) of the heavy chain variable sequence of SEQ ID NO: 133 or the heavy chain sequence of SEQ ID NO: 134.

The description also optionally contemplates polynucleotide sequences including one or more of the polynucleotide sequences encoding antibody fragments for treatment or prevention of pain and pain associated conditions described herein. In one example of the description, polynucleotides encoding antibody fragments for treatment or prevention of pain and pain associated conditions having binding specificity to NGF comprise, or alternatively consist of, one, two, three or more, including all of the following polynucleotides encoding antibody fragments: the polynucleotide SEQ ID NO: 331 encoding the light chain variable sequence of SEQ ID NO: 131; the polynucleotide SEQ ID NO: 332 encoding the light chain sequence of SEQ ID NO: 132; the polynucleotide SEQ ID NO: 333 encoding the heavy chain variable sequence of SEQ ID NO: 133; the polynucleotide SEQ ID NO: 334 encoding the heavy chain sequence of SEQ ID NO: 134; polynucleotides encoding the complementarity-determining regions (SEQ ID NO: 335; SEQ ID NO: 336; and SEQ ID NO: 337) of the light chain variable sequence of SEQ ID NO: 131 or the light chain sequence of SEQ ID NO: 132; and polynucleotides encoding the complementarity-determining regions (SEQ ID NO: 338; SEQ ID NO: 339; and SEQ ID NO: 340) of the heavy chain variable sequence of SEQ ID NO: 133 or the heavy chain sequence of SEQ ID NO: 134.

In a preferred optional example of the description, polynucleotides of the description comprise, or alternatively consist of, polynucleotides encoding Fab (fragment antigen binding) fragments having binding specificity for NGF. With respect to antibody Ab14, the polynucleotides encoding the full length Ab14 antibody comprise, or alternatively consist of, the polynucleotide SEQ ID NO: 332 encoding the light chain sequence of SEQ ID NO: 132 and the polynucleotide SEQ ID NO: 334 encoding the heavy chain sequence of SEQ ID NO: 134.

Another optional example of the description contemplates these polynucleotides incorporated into an expression vector for expression in mammalian cells such as CHO, NSO, HEK-293, or in fungal, insect, plant or microbial systems such as yeast cells such as the yeast Pichia. Suitable Pichia species include, but are not limited to, Pichia pastoris. In one example of the description described herein (infra), Fab fragments may be produced by enzymatic digestion (e.g., papain) of Ab14 following expression of the full-length polynucleotides in a suitable host. In another example of the description, anti-NGF antibodies such as Ab14 or Fab fragments thereof may be produced via expression of Ab14 polynucleotides in mammalian cells such as CHO, NSO or HEK 293 cells, fungal, insect, plant or microbial systems such as yeast cells (for example diploid yeast such as diploid Pichia) and other yeast strains. Suitable Pichia species include, but are not limited to, Pichia pastoris.

### Antibody Ab15

The description is further directed to the use of polynucleotides set forth below to produce antibody Ab15 polypeptides having binding specificity to NGF, which inhibit the association of NGF with TrkA without appreciably inhibiting the association of NGF with p75, in methods of treating pain in an individual comprising administering to said individual antibody Ab15 polypeptides. The description is further directed to polynucleotides encoding antibody polypeptides for treatment or prevention of pain and pain associated conditions having binding specificity to NGF. In one example of the description, polynucleotides of the description comprise, or alternatively consist of, the following polynucleotide sequence encoding the variable light chain polypeptide sequence of SEQ ID NO: 141:

In one example of the description, polynucleotides of the description comprise, or alternatively consist of, the following polynucleotide sequence encoding the light chain polypeptide sequence of SEQ ID NO: 142:

In another example of the description, polynucleotides of the description comprise, or alternatively consist of, the following polynucleotide sequence encoding the variable heavy chain polypeptide sequence of SEQ ID NO: 143:

In one example of the description, polynucleotides of the description comprise, or alternatively consist of, the following polynucleotide sequence encoding the heavy chain polypeptide sequence of SEQ ID NO: 144:

In a further example of the description, polynucleotides encoding antibody fragments for treatment or prevention of pain and pain associated conditions having binding specificity to NGF comprise, or alternatively consist of, one or more of the polynucleotide sequences of SEQ ID NO: 345; SEQ ID NO: 346; and SEQ ID NO: 347 which correspond to polynucleotides encoding the complementarity-determining regions (CDRs, or hypervariable regions) of the light chain variable sequence of SEQ ID NO: 141 or the light chain sequence of SEQ ID NO: 142.

In a further example of the description, polynucleotides encoding antibody fragments for treatment or prevention of pain and pain associated conditions having binding specificity to NGF comprise, or alternatively consist of, one or more of the polynucleotide sequences of SEQ ID NO: 348; SEQ ID NO: 349; and SEQ ID NO: 350 which correspond to polynucleotides encoding the complementarity-determining regions (CDRs, or hypervariable regions) of the heavy chain variable sequence of SEQ ID NO: 143 or the heavy chain sequence of SEQ ID NO: 144.

The description also contemplates polynucleotide sequences including one or more of the polynucleotide sequences encoding antibody fragments for treatment or prevention of pain and pain associated conditions described herein. In one example of the description, polynucleotides encoding antibody fragments for treatment or prevention of pain and pain associated conditions having binding specificity to NGF comprise, or alternatively consist of, one, two, three or more, including all of the following polynucleotides encoding antibody fragments: the polynucleotide SEQ ID NO: 341 encoding the light chain variable sequence of SEQ ID NO: 141; the polynucleotide SEQ ID NO: 342 encoding the light chain sequence of SEQ ID NO: 142; the polynucleotide SEQ ID NO: 343 encoding the heavy chain variable sequence of SEQ ID NO: 143; the polynucleotide SEQ ID NO: 344 encoding the heavy chain sequence of SEQ ID NO: 144; polynucleotides encoding the complementarity-determining regions (SEQ ID NO: 345; SEQ ID NO: 346; and SEQ ID NO: 347) of the light chain variable sequence of SEQ ID NO: 141 or the light chain sequence of SEQ ID NO: 142; and polynucleotides encoding the complementarity-determining regions (SEQ ID NO: 348; SEQ ID NO: 349; and SEQ ID NO: 350) of the heavy chain variable sequence of SEQ ID NO: 143 or the heavy chain sequence of SEQ ID NO: 144.

In a preferred example of the description, polynucleotides of the description comprise, or alternatively consist of, polynucleotides encoding Fab (fragment antigen binding) fragments having binding specificity for NGF. With respect to antibody Ab15, the polynucleotides encoding the full length Ab15 antibody comprise, or alternatively consist of, the polynucleotide SEQ ID NO: 342 encoding the light chain sequence of SEQ ID NO: 142 and the polynucleotide SEQ ID NO: 344 encoding the heavy chain sequence of SEQ ID NO: 144.

Another example of the description contemplates these polynucleotides incorporated into an expression vector for expression in mammalian cells such as CHO, NSO, HEK-293, or in fungal, insect, plant or microbial systems such as yeast cells such as the yeast Pichia. Suitable Pichia species include, but are not limited to, Pichia pastoris. In one example of the description described herein (infra), Fab fragments may be produced by enzymatic digestion (e.g., papain) of Ab15 following expression of the full-length polynucleotides in a suitable host. In another example of the description, anti-NGF antibodies such as Ab15 or Fab fragments thereof may be produced via expression of Ab15 polynucleotides in mammalian cells such as CHO, NSO or HEK 293 cells, fungal, insect, plant or microbial systems such as yeast cells (for example diploid yeast such as diploid Pichia) and other yeast strains. Suitable Pichia species include, but are not limited to, Pichia pastoris.

### Antibody Ab16

The description is further directed to the use of polynucleotides set forth below to produce antibody Ab16 polypeptides for treatment or prevention of pain and pain associated conditions having binding specificity to NGF, which inhibit the association of NGF with TrkA without appreciably inhibiting the association of NGF with p75, in methods of treating pain in an individual comprising administering to said individual antibody Ab16 polypeptides. The description is further directed to polynucleotides encoding antibody polypeptides for treatment or prevention of pain and pain associated conditions having binding specificity to NGF. In one example of the description, polynucleotides of the description comprise, or alternatively consist of, the following polynucleotide sequence encoding the variable light chain polypeptide sequence of SEQ ID NO: 151:

In one example of the description, polynucleotides of the description comprise, or alternatively consist of, the following polynucleotide sequence encoding the light chain polypeptide sequence of SEQ ID NO: 152:

In another example of the description, polynucleotides of the description comprise, or alternatively consist of, the following polynucleotide sequence encoding the variable heavy chain polypeptide sequence of SEQ ID NO: 153:

In one example of the description, polynucleotides of the description comprise, or alternatively consist of, the following polynucleotide sequence encoding the heavy chain polypeptide sequence of SEQ ID NO: 154:

In a further example of the description, polynucleotides encoding antibody fragments for treatment or prevention of pain and pain associated conditions having binding specificity to NGF comprise, or alternatively consist of, one or more of the polynucleotide sequences of SEQ ID NO: 355; SEQ ID NO: 356; and SEQ ID NO: 357 which correspond to polynucleotides encoding the complementarity-determining regions (CDRs, or hypervariable regions) of the light chain variable sequence of SEQ ID NO: 151 or the light chain sequence of SEQ ID NO: 152.

In a further example of the description, polynucleotides encoding antibody fragments for treatment or prevention of pain and pain associated conditions having binding specificity to NGF comprise, or alternatively consist of, one or more of the polynucleotide sequences of SEQ ID NO: 358; SEQ ID NO: 359; and SEQ ID NO: 360 which correspond to polynucleotides encoding the complementarity-determining regions (CDRs, or hypervariable regions) of the heavy chain variable sequence of SEQ ID NO: 153 or the heavy chain sequence of SEQ ID NO: 154.

The description also contemplates polynucleotide sequences including one or more of the polynucleotide sequences encoding antibody fragments for treatment or prevention of pain and pain associated conditions described herein. In one example of the description, polynucleotides encoding antibody fragments for treatment or prevention of pain and pain associated conditions having binding specificity to NGF comprise, or alternatively consist of, one, two, three or more, including all of the following polynucleotides encoding antibody fragments: the polynucleotide SEQ ID NO: 351 encoding the light chain variable sequence of SEQ ID NO: 151; the polynucleotide SEQ ID NO: 352 encoding the light chain sequence of SEQ ID NO: 152; the polynucleotide SEQ ID NO: 353 encoding the heavy chain variable sequence of SEQ ID NO: 153; the polynucleotide SEQ ID NO: 354 encoding the heavy chain sequence of SEQ ID NO: 154; polynucleotides encoding the complementarity-determining regions (SEQ ID NO: 355; SEQ ID NO: 356; and SEQ ID NO: 357) of the light chain variable sequence of SEQ ID NO: 151 or the light chain sequence of SEQ ID NO: 152; and polynucleotides encoding the complementarity-determining regions (SEQ ID NO: 358; SEQ ID NO: 359; and SEQ ID NO: 360) of the heavy chain variable sequence of SEQ ID NO: 153 or the heavy chain sequence of SEQ ID NO: 154.

In a preferred example of the description, polynucleotides of the description comprise, or alternatively consist of, polynucleotides encoding Fab (fragment antigen binding) fragments having binding specificity for NGF. With respect to antibody Ab16, the polynucleotides encoding the full length Ab16 antibody comprise, or alternatively consist of, the polynucleotide SEQ ID NO: 352 encoding the light chain sequence of SEQ ID NO: 152 and the polynucleotide SEQ ID NO: 354 encoding the heavy chain sequence of SEQ ID NO: 154.

Another example of the description contemplates these polynucleotides incorporated into an expression vector for expression in mammalian cells such as CHO, NSO, HEK-293, or in fungal, insect, plant or microbial systems such as yeast cells such as the yeast Pichia. Suitable Pichia species include, but are not limited to, Pichia pastoris. In one example of the description described herein (infra), Fab fragments may be produced by enzymatic digestion (e.g., papain) of Ab16 following expression of the full-length polynucleotides in a suitable host. In another example of the description, anti-NGF antibodies such as Ab16 or Fab fragments thereof may be produced via expression of Ab16 polynucleotides in mammalian cells such as CHO, NSO or HEK 293 cells, fungal, insect, plant or microbial systems such as yeast cells (for example diploid yeast such as diploid Pichia) and other yeast strains. Suitable Pichia species include, but are not limited to, Pichia pastoris.

### Antibody Ab17

The description is further optionally directed to the use of polynucleotides set forth below to produce antibody Ab17 polypeptides having binding specificity to NGF, which inhibit the association of NGF with TrkA without appreciably inhibiting the association of NGF with p75, in methods of treating pain in an individual comprising administering to said individual antibody Ab17 polypeptides. The description is further optionally directed to polynucleotides encoding antibody polypeptides for treatment or prevention of pain and pain associated conditions having binding specificity to NGF. In one optional example of the description, polynucleotides of the description comprise, or alternatively consist of, the following polynucleotide sequence encoding the variable light chain polypeptide sequence of SEQ ID NO: 161:

In one optional example of the description, polynucleotides of the description comprise, or alternatively consist of, the following polynucleotide sequence encoding the light chain polypeptide sequence of SEQ ID NO: 162:

In another optional example of the description, polynucleotides of the description comprise, or alternatively consist of, the following polynucleotide sequence encoding the variable heavy chain polypeptide sequence of SEQ ID NO: 163:

**In** one optional example of the description, polynucleotides of the description comprise, or alternatively consist of, the following polynucleotide sequence encoding the heavy chain polypeptide sequence of SEQ ID NO: 164:

In a further optional example of the description, polynucleotides encoding antibody fragments for treatment or prevention of pain and pain associated conditions having binding specificity to NGF comprise, or alternatively consist of, one or more of the polynucleotide sequences of SEQ ID NO: 365; SEQ ID NO: 366; and SEQ ID NO: 367 which correspond to polynucleotides encoding the complementarity-determining regions (CDRs, or hypervariable regions) of the light chain variable sequence of SEQ ID NO: 161 or the light chain sequence of SEQ ID NO: 162.

In a further optional example of the description, polynucleotides encoding antibody fragments for treatment or prevention of pain and pain associated conditions having binding specificity to NGF comprise, or alternatively consist of, one or more of the polynucleotide sequences of SEQ ID NO: 368; SEQ ID NO: 369; and SEQ ID NO: 370 which correspond to polynucleotides encoding the complementarity-determining regions (CDRs, or hypervariable regions) of the heavy chain variable sequence of SEQ ID NO: 163 or the heavy chain sequence of SEQ ID NO: 164.

The description also optionally contemplates polynucleotide sequences including one or more of the polynucleotide sequences encoding antibody fragments for treatment or prevention of pain and pain associated conditions described herein. In one example of the description, polynucleotides encoding antibody fragments for treatment or prevention of pain and pain associated conditions having binding specificity to NGF comprise, or alternatively consist of, one, two, three or more, including all of the following polynucleotides encoding antibody fragments: the polynucleotide SEQ ID NO: 361 encoding the light chain variable sequence of SEQ ID NO: 161; the polynucleotide SEQ ID NO: 362 encoding the light chain sequence of SEQ ID NO: 162; the polynucleotide SEQ ID NO: 363 encoding the heavy chain variable sequence of SEQ ID NO: 163; the polynucleotide SEQ ID NO: 364 encoding the heavy chain sequence of SEQ ID NO: 164; polynucleotides encoding the complementarity-determining regions (SEQ ID NO: 365; SEQ ID NO: 366; and SEQ ID NO: 367) of the light chain variable sequence of SEQ ID NO: 161 or the light chain sequence of SEQ ID NO: 162; and polynucleotides encoding the complementarity-determining regions (SEQ ID NO: 368; SEQ ID NO: 369; and SEQ ID NO: 370) of the heavy chain variable sequence of SEQ ID NO: 163 or the heavy chain sequence of SEQ ID NO: 164.

In a preferred optional example of the description, polynucleotides of the description comprise, or alternatively consist of, polynucleotides encoding Fab (fragment antigen binding) fragments having binding specificity for NGF. With respect to antibody Ab17, the polynucleotides encoding the full length Ab17 antibody comprise, or alternatively consist of, the polynucleotide SEQ ID NO: 362 encoding the light chain sequence of SEQ ID NO: 162 and the polynucleotide SEQ ID NO: 364 encoding the heavy chain sequence of SEQ ID NO: 164.

Another optional example of the description contemplates these polynucleotides incorporated into an expression vector for expression in mammalian cells such as CHO, NSO, HEK-293, or in fungal, insect, plant or microbial systems such as yeast cells such as the yeast Pichia. Suitable Pichia species include, but are not limited to, Pichia pastoris. In one optional example of the description described herein (infra), Fab fragments may be produced by enzymatic digestion (e.g., papain) of Ab17 following expression of the full-length polynucleotides in a suitable host. In another example of the description, anti-NGF antibodies such as Ab17 or Fab fragments thereof may be produced via expression of Ab17 polynucleotides in mammalian cells such as CHO, NSO or HEK 293 cells, fungal, insect, plant or microbial systems such as yeast cells (for example diploid yeast such as diploid Pichia) and other yeast strains. Suitable Pichia species include, but are not limited to, Pichia pastoris.

### Antibody Ab18

The description is further optionally directed to the use of polynucleotides set forth below to produce antibody Ab18 polypeptides having binding specificity to NGF, which inhibit the association of NGF with TrkA and the association of NGF with p75, in methods of treating pain in an individual comprising administering to said individual antibody Ab18 polypeptides. The description is further directed to polynucleotides encoding antibody polypeptides for treatment or prevention of pain and pain associated conditions having binding specificity to NGF. In one optional example of the description, polynucleotides of the description comprise, or alternatively consist of, the following polynucleotide sequence encoding the variable light chain polypeptide sequence of SEQ ID NO: 171:

In one optional example of the description, polynucleotides of the description comprise, or alternatively consist of, the following polynucleotide sequence encoding the light chain polypeptide sequence of SEQ ID NO: 172:

In another optional example of the description, polynucleotides of the description comprise, or alternatively consist of, the following polynucleotide sequence encoding the variable heavy chain polypeptide sequence of SEQ ID NO: 173:

In one optional example of the description, polynucleotides of the description comprise, or alternatively consist of, the following polynucleotide sequence encoding the heavy chain polypeptide sequence of SEQ ID NO: 174:

In a further optional example of the description, polynucleotides encoding antibody fragments for treatment or prevention of pain and pain associated conditions having binding specificity to NGF comprise, or alternatively consist of, one or more of the polynucleotide sequences of SEQ ID NO: 375; SEQ ID NO: 376; and SEQ ID NO: 377 which correspond to polynucleotides encoding the complementarity-determining regions (CDRs, or hypervariable regions) of the light chain variable sequence of SEQ ID NO: 171 or the light chain sequence of SEQ ID NO: 172.

In a further optional example of the description, polynucleotides encoding antibody fragments for treatment or prevention of pain and pain associated conditions having binding specificity to NGF comprise, or alternatively consist of, one or more of the polynucleotide sequences of SEQ ID NO: 378; SEQ ID NO: 379; and SEQ ID NO: 380 which correspond to polynucleotides encoding the complementarity-determining regions (CDRs, or hypervariable regions) of the heavy chain variable sequence of SEQ ID NO: 173 or the heavy chain sequence of SEQ ID NO: 174.

The description also optionally contemplates polynucleotide sequences including one or more of the polynucleotide sequences encoding antibody fragments for treatment or prevention of pain and pain associated conditions described herein. In one optional example of the description, polynucleotides encoding antibody fragments for treatment or prevention of pain and pain associated conditions having binding specificity to NGF comprise, or alternatively consist of, one, two, three or more, including all of the following polynucleotides encoding antibody fragments: the polynucleotide SEQ ID NO: 371 encoding the light chain variable sequence of SEQ ID NO: 171; the polynucleotide SEQ ID NO: 372 encoding the light chain sequence of SEQ ID NO: 172; the polynucleotide SEQ ID NO: 373 encoding the heavy chain variable sequence of SEQ ID NO: 173; the polynucleotide SEQ ID NO: 374 encoding the heavy chain sequence of SEQ ID NO: 174; polynucleotides encoding the complementarity-determining regions (SEQ ID NO: 375; SEQ ID NO: 376; and SEQ ID NO: 377) of the light chain variable sequence of SEQ ID NO: 171 or the light chain sequence of SEQ ID NO: 172; and polynucleotides encoding the complementarity-determining regions (SEQ ID NO: 378; SEQ ID NO: 379; and SEQ ID NO: 380) of the heavy chain variable sequence of SEQ ID NO: 173 or the heavy chain sequence of SEQ ID NO: 174.

In a preferred optional example of the description, polynucleotides of the description comprise, or alternatively consist of, polynucleotides encoding Fab (fragment antigen binding) fragments for treatment or prevention of pain and pain associated conditions having binding specificity for NGF. With respect to antibody Ab18, the polynucleotides encoding the full length Ab18 antibody comprise, or alternatively consist of, the polynucleotide SEQ ID NO: 372 encoding the light chain sequence of SEQ ID NO: 172 and the polynucleotide SEQ ID NO: 374 encoding the heavy chain sequence of SEQ ID NO: 174.

Another optional example of the description, contemplates these polynucleotides incorporated into an expression vector for expression in mammalian cells such as CHO, NSO, HEK-293, or in fungal, insect, plant or microbial systems such as yeast cells such as the yeast Pichia. Suitable Pichia species include, but are not limited to, Pichia pastoris. In one example of the description, described herein (infra), Fab fragments may be produced by enzymatic digestion (e.g., papain) of Ab18 following expression of the full-length polynucleotides in a suitable host. In another example of the description, anti-NGF antibodies such as Ab18 or Fab fragments thereof may be produced via expression of Ab18 polynucleotides in mammalian cells such as CHO, NSO or HEK 293 cells, fungal, insect, plant or microbial systems such as yeast cells (for example diploid yeast such as diploid Pichia) and other yeast strains. Suitable Pichia species include, but are not limited to, Pichia pastoris.

### Antibody Ab19

The description is further optionally directed to the use of polynucleotides set forth below to produce antibody Ab19 polypeptides having binding specificity to NGF, which inhibit the association of NGF with TrkA and the association of NGF with p75. in methods of treating pain in an individual comprising administering to said individual antibody Ab19 polypeptides. The description is further optionally directed to polynucleotides encoding antibody polypeptides for treatment or prevention of pain and pain associated conditions having binding specificity to NGF. In one example of the description, polynucleotides of the description comprise, or alternatively consist of, the following polynucleotide sequence encoding the variable light chain polypeptide sequence of SEQ ID NO: 181:

In one optional example of the description, polynucleotides of the description comprise, or alternatively consist of, the following polynucleotide sequence encoding the light chain polypeptide sequence of SEQ ID NO: 182:

In another optional example of the description, polynucleotides of the description comprise, or alternatively consist of, the following polynucleotide sequence encoding the variable heavy chain polypeptide sequence of SEQ ID NO: 183:

In one optional example of the description, polynucleotides of the description comprise, or alternatively consist of, the following polynucleotide sequence encoding the heavy chain polypeptide sequence of SEQ ID NO: 184:

In a further optional example of the description, polynucleotides encoding antibody fragments for treatment or prevention of pain and pain associated conditions having binding specificity to NGF comprise, or alternatively consist of, one or more of the polynucleotide sequences of SEQ ID NO: 385; SEQ ID NO: 386; and SEQ ID NO: 387 which correspond to polynucleotides encoding the complementarity-determining regions (CDRs, or hypervariable regions) of the light chain variable sequence of SEQ ID NO: 181 or the light chain sequence of SEQ ID NO: 182.

In a further optional example of the description, polynucleotides encoding antibody fragments for treatment or prevention of pain and pain associated conditions having binding specificity to NGF comprise, or alternatively consist of, one or more of the polynucleotide sequences of SEQ ID NO: 388; SEQ ID NO: 389; and SEQ ID NO: 390 which correspond to polynucleotides encoding the complementarity-determining regions (CDRs, or hypervariable regions) of the heavy chain variable sequence of SEQ ID NO: 183 or the heavy chain sequence of SEQ ID NO: 184.

The description also optionally contemplates polynucleotide sequences including one or more of the polynucleotide sequences encoding antibody fragments for treatment or prevention of pain and pain associated conditions described herein. In one example of the description, polynucleotides encoding antibody fragments for treatment or prevention of pain and pain associated conditions having binding specificity to NGF comprise, or alternatively consist of, one, two, three or more, including all of the following polynucleotides encoding antibody fragments: the polynucleotide SEQ ID NO: 381 encoding the light chain variable sequence of SEQ ID NO: 181; the polynucleotide SEQ ID NO: 382 encoding the light chain sequence of SEQ ID NO: 182; the polynucleotide SEQ ID NO: 383 encoding the heavy chain variable sequence of SEQ ID NO: 183; the polynucleotide SEQ ID NO: 384 encoding the heavy chain sequence of SEQ ID NO: 184; polynucleotides encoding the complementarity-determining regions (SEQ ID NO: 385; SEQ ID NO: 386; and SEQ ID NO: 387) of the light chain variable sequence of SEQ ID NO: 181 or the light chain sequence of SEQ ID NO: 182; and polynucleotides encoding the complementarity-determining regions (SEQ ID NO: 388; SEQ ID NO: 389; and SEQ ID NO: 390) of the heavy chain variable sequence of SEQ ID NO: 183 or the heavy chain sequence of SEQ ID NO: 184.

In a preferred optional example of the description, polynucleotides of the description comprise, or alternatively consist of, polynucleotides encoding Fab (fragment antigen binding) fragments having binding specificity for NGF. With respect to antibody Ab19, the polynucleotides encoding the full length Ab19 antibody comprise, or alternatively consist of, the polynucleotide SEQ ID NO: 382 encoding the light chain sequence of SEQ ID NO: 182 and the polynucleotide SEQ ID NO: 384 encoding the heavy chain sequence of SEQ ID NO: 184.

Another optional example of the description contemplates these polynucleotides incorporated into an expression vector for expression in mammalian cells such as CHO, NSO, HEK-293, or in fungal, insect, plant or microbial systems such as yeast cells such as the yeast Pichia. Suitable Pichia species include, but are not limited to, Pichia pastoris. In one optional embodiment of the invention described herein (infra), Fab fragments may be produced by enzymatic digestion (e.g., papain) of Ab19 following expression of the full-length polynucleotides in a suitable host. In another example of the description, anti-NGF antibodies such as Ab19 or Fab fragments thereof may be produced via expression of Ab19 polynucleotides in mammalian cells such as CHO, NSO or HEK 293 cells, fungal, insect, plant or microbial systems such as yeast cells (for example diploid yeast such as diploid Pichia) and other yeast strains. Suitable Pichia species include, but are not limited to, Pichia pastoris.

### Antibody Ab20

The description is further optionally directed to the use of polynucleotides set forth below to produce antibody Ab20 polypeptides for treatment or prevention of pain and pain associated conditions having binding specificity to NGF, which inhibits the association of NGF with TrkA and the association of NGF with p75, in methods of treating pain in an individual comprising administering to said individual antibody Ab20 polypeptides. The description is further directed to polynucleotides encoding antibody polypeptides having binding specificity to NGF. In one optional example of the description, polynucleotides of the description comprise, or alternatively consist of, the following polynucleotide sequence encoding the variable light chain polypeptide sequence of SEQ ID NO: 191:

In one optional example of the description, polynucleotides of the description comprise, or alternatively consist of, the following polynucleotide sequence encoding the light chain polypeptide sequence of SEQ ID NO: 192:

In another optional example of the description, polynucleotides of the description comprise, or alternatively consist of, the following polynucleotide sequence encoding the variable heavy chain polypeptide sequence of SEQ ID NO: 193:

In one optional example of the description, polynucleotides of the description comprise, or alternatively consist of, the following polynucleotide sequence encoding the heavy chain polypeptide sequence of SEQ ID NO: 194:

In a further optional example of the description, polynucleotides encoding antibody fragments for treatment or prevention of pain and pain associated conditions having binding specificity to NGF comprise, or alternatively consist of, one or more of the polynucleotide sequences of SEQ ID NO: 395; SEQ ID NO: 396; and SEQ ID NO: 397 which correspond to polynucleotides encoding the complementarity-determining regions (CDRs, or hypervariable regions) of the light chain variable sequence of SEQ ID NO: 191 or the light chain sequence of SEQ ID NO: 192.

In a further optional example of the description, polynucleotides encoding antibody fragments for treatment or prevention of pain and pain associated conditions having binding specificity to NGF comprise, or alternatively consist of, one or more of the polynucleotide sequences of SEQ ID NO: 398; SEQ ID NO: 399; and SEQ ID NO: 400 which correspond to polynucleotides encoding the complementarity-determining regions (CDRs, or hypervariable regions) of the heavy chain variable sequence of SEQ ID NO: 193 or the heavy chain sequence of SEQ ID NO: 194.

The description also optionally contemplates polynucleotide sequences including one or more of the polynucleotide sequences encoding antibody fragments for treatment or prevention of pain and pain associated conditions described herein. In one example of the description, polynucleotides encoding antibody fragments for treatment or prevention of pain and pain associated conditions having binding specificity to NGF comprise, or alternatively consist of, one, two, three or more, including all of the following polynucleotides encoding antibody fragments: the polynucleotide SEQ ID NO: 391 encoding the light chain variable sequence of SEQ ID NO: 191; the polynucleotide SEQ ID NO: 392 encoding the light chain sequence of SEQ ID NO: 192; the polynucleotide SEQ ID NO: 393 encoding the heavy chain variable sequence of SEQ ID NO: 193; the polynucleotide SEQ ID NO: 394 encoding the heavy chain sequence of SEQ ID NO: 194; polynucleotides encoding the complementarity-determining regions (SEQ ID NO: 395; SEQ ID NO: 396; and SEQ ID NO: 397) of the light chain variable sequence of SEQ ID NO: 191 or the light chain sequence of SEQ ID NO: 192; and polynucleotides encoding the complementarity-determining regions (SEQ ID NO: 398; SEQ ID NO: 399; and SEQ ID NO: 400) of the heavy chain variable sequence of SEQ ID NO: 193 or the heavy chain sequence of SEQ ID NO: 194.

In a preferred optional example of the description, polynucleotides of the description comprise, or alternatively consist of, polynucleotides encoding Fab (fragment antigen binding) fragments having binding specificity for NGF. With respect to antibody Ab20, the polynucleotides encoding the full length Ab20 antibody comprise, or alternatively consist of, the polynucleotide SEQ ID NO: 392 encoding the light chain sequence of SEQ ID NO: 192 and the polynucleotide SEQ ID NO: 394 encoding the heavy chain sequence of SEQ ID NO: 194.

Another optional example of the description contemplates these polynucleotides incorporated into an expression vector for expression in mammalian cells such as CHO, NSO, HEK-293, or in fungal, insect, plant or microbial systems such as yeast cells such as the yeast Pichia. Suitable Pichia species include, but are not limited to, Pichia pastoris. In one example of the description described herein (infra), Fab fragments may be produced by enzymatic digestion (e.g., papain) of Ab20 following expression of the full-length polynucleotides in a suitable host. In another example of the description, anti-NGF antibodies such as Ab20 or Fab fragments thereof may be produced via expression of Ab20 polynucleotides in mammalian cells such as CHO, NSO or HEK 293 cells, fungal, insect, plant or microbial systems such as yeast cells (for example diploid yeast such as diploid Pichia) and other yeast strains. Suitable Pichia species include, but are not limited to, Pichia pastoris.

### Antibody Ab21

The description is further optionally directed to the use of polynucleotides set forth below to produce antibody Ab21 polypeptides having binding specificity to NGF, which inhibit the association of NGF with TrkA and the association of NGF with p75 in methods of treating pain in an individual comprising administering to said individual antibody Ab21 polypeptides. The description is further optionally directed to polynucleotides encoding antibody polypeptides for treatment or prevention of pain and pain associated conditions having binding specificity to NGF. In one example of the description, polynucleotides of the description comprise, or alternatively consist of, the following polynucleotide sequence encoding the variable light chain polypeptide sequence of SEQ ID NO: 51:

In one example of the description, polynucleotides of the description optionally comprise, or alternatively consist of, the following polynucleotide sequence encoding the light chain polypeptide sequence of SEQ ID NO: 401:

In another optional example of the description, polynucleotides of the description comprise, or alternatively consist of, the following polynucleotide sequence encoding the variable heavy chain polypeptide sequence of SEQ ID NO: 53:

In one optional example of the description, polynucleotides of the description comprise, or alternatively consist of, the following polynucleotide sequence encoding the heavy chain polypeptide sequence of SEQ ID NO: 402:

In a further optional example of the description, polynucleotides encoding antibody fragments for treatment or prevention of pain and pain associated conditions having binding specificity to NGF comprise, or alternatively consist of, one or more of the polynucleotide sequences of SEQ ID NO: 255; SEQ ID NO: 256; and SEQ ID NO: 257 which correspond to polynucleotides encoding the complementarity-determining regions (CDRs, or hypervariable regions) of the light chain variable sequence of SEQ ID NO: 51 or the light chain sequence of SEQ ID NO: 401.

In a further optional example of the description, polynucleotides encoding antibody fragments for treatment or prevention of pain and pain associated conditions having binding specificity to NGF comprise, or alternatively consist of, one or more of the polynucleotide sequences of SEQ ID NO: 258; SEQ ID NO: 259; and SEQ ID NO: 260 which correspond to polynucleotides encoding the complementarity-determining regions (CDRs, or hypervariable regions) of the heavy chain variable sequence of SEQ ID NO: 53 or the heavy chain sequence of SEQ ID NO: 402.

The description, also optionally contemplates polynucleotide sequences including one or more of the polynucleotide sequences encoding antibody fragments for treatment or prevention of pain and pain associated conditions described herein. In one example of the description, polynucleotides encoding antibody fragments for treatment or prevention of pain and pain associated conditions having binding specificity to NGF comprise, or alternatively consist of, one, two, three or more, including all of the following polynucleotides encoding antibody fragments: the polynucleotide SEQ ID NO: 251 encoding the light chain variable sequence of SEQ ID NO: 51; the polynucleotide SEQ ID NO: 403 encoding the light chain sequence of SEQ ID NO: 401; the polynucleotide SEQ ID NO: 253 encoding the heavy chain variable sequence of SEQ ID NO: 53; the polynucleotide SEQ ID NO: 404 encoding the heavy chain sequence of SEQ ID NO: 402; polynucleotides encoding the complementarity-determining regions (SEQ ID NO: 255; SEQ ID NO: 256; and SEQ ID NO: 257) of the light chain variable sequence of SEQ ID NO: 51 or the light chain sequence of SEQ ID NO: 401; and polynucleotides encoding the complementarity-determining regions (SEQ ID NO: 258; SEQ ID NO: 259; and SEQ ID NO: 260) of the heavy chain variable sequence of SEQ ID NO: 53 or the heavy chain sequence of SEQ ID NO: 402.

In a preferred optional example of the description, polynucleotides of the description comprise, or alternatively consist of, polynucleotides encoding Fab (fragment antigen binding) fragments having binding specificity for NGF. With respect to antibody Ab21, the polynucleotides encoding the full length Ab21 antibody comprise, or alternatively consist of, the polynucleotide SEQ ID NO: 403 encoding the light chain sequence of SEQ ID NO: 401 and the polynucleotide SEQ ID NO: 404 encoding the heavy chain sequence of SEQ ID NO: 402.

Another optional example of the description contemplates these polynucleotides incorporated into an expression vector for expression in mammalian cells such as CHO, NSO, HEK-293, or in fungal, insect, plant or microbial systems such as yeast cells such as the yeast Pichia. Suitable Pichia species include, but are not limited to, Pichia pastoris. In one optional example of the description described herein (infra), Fab fragments may be produced by enzymatic digestion (e.g., papain) of Ab21 following expression of the full-length polynucleotides in a suitable host. In another example of the description, anti-NGF antibodies such as Ab21 or Fab fragments thereof may be produced via expression of Ab21 polynucleotides in mammalian cells such as CHO, NSO or HEK 293 cells, fungal, insect, plant or microbial systems such as yeast cells (for example diploid yeast such as diploid Pichia) and other yeast strains. Suitable Pichia species include, but are not limited to, Pichia pastoris.

### Antibody fragment Fab2

The description is further optionally directed to the use of polynucleotides set forth below to produce antibody fragment Fab2 polypeptides that inhibit the association of NGF with TrkA and p75 for treatment or prevention of pain and pain associated conditions having binding specificity to NGF in methods of treating pain in an individual comprising administering to said individual antibody Ab1 polypeptides. The description is further directed to polynucleotides encoding antibody fragment polypeptides for treatment or prevention of pain and pain associated conditions having binding specificity to NGF. In one example of the description, Fab polynucleotides of the description comprise, or alternatively consist of, the following polynucleotide sequence encoding the light chain polypeptide sequence of SEQ ID NO: 407:

In another optional example of the description, polynucleotides of the description comprise, or alternatively consist of, the following polynucleotide sequence encoding the heavy chain polypeptide sequence of SEQ ID NO: 408:

In a further optional example of the description, polynucleotides encoding Fab antibody fragments having binding specificity to NGF comprise one or more of the polynucleotide sequences of SEQ ID NO: 255; SEQ ID NO: 256; and SEQ ID NO: 257 which correspond to polynucleotides encoding the complementarity-determining regions (CDRs, or hypervariable regions) of the light chain variable sequence of SEQ ID NO: 51 or the light chain sequence of SEQ ID NO: 409.

In a further optional example of the description, polynucleotides encoding Fab antibody fragments for treatment or prevention of pain and pain associated conditions having binding specificity to NGF comprise one or more of the polynucleotide sequences of SEQ ID NO: 258; SEQ ID NO: 259; and SEQ ID NO: 260 which correspond to polynucleotides encoding the complementarity-determining regions (CDRs, or hypervariable regions) of the heavy chain variable sequence of SEQ ID NO: 53 or the heavy chain sequence of SEQ ID NO: 410.

The description also optionally contemplates polynucleotide sequences including one or more of the polynucleotide sequences encoding antibody fragments for treatment or prevention of pain and pain associated conditions described herein. In one example of the description, polynucleotides encoding antibody fragments for treatment or prevention of pain and pain associated conditions having binding specificity to NGF comprise, or alternatively consist of, one, two, three or more, including all of the following polynucleotides encoding antibody fragments: the polynucleotide SEQ ID NO: 251 encoding the light chain variable sequence of SEQ ID NO: 51; the polynucleotide SEQ ID NO: 409 encoding the light chain sequence of SEQ ID NO: 407; the polynucleotide SEQ ID NO: 253 encoding the heavy chain variable sequence of SEQ ID NO: 53; the polynucleotide SEQ ID NO: 410 encoding the heavy chain sequence of SEQ ID NO: 408; polynucleotides encoding the complementarity-determining regions (SEQ ID NO: 255; SEQ ID NO: 256; and SEQ ID NO: 257) of the light chain variable sequence of SEQ ID NO: 51 or the light chain sequence of SEQ ID NO: 407; and polynucleotides encoding the complementarity-determining regions (SEQ ID NO: 258; SEQ ID NO: 259; and SEQ ID NO: 260) of the heavy chain variable sequence of SEQ ID NO: 53 or the heavy chain sequence of SEQ ID NO: 408.

In a preferred optional example of the description, polynucleotides of the description comprise, or alternatively consist of, polynucleotides encoding Fab (fragment antigen binding) fragments having binding specificity for NGF. With respect to antibody fragment Fab2, the polynucleotides encoding the Fab fragment include the polynucleotide SEQ ID NO: 409 encoding the light chain sequence of SEQ ID NO: 407 and the polynucleotide SEQ ID NO: 410 encoding the heavy chain sequence of SEQ ID NO: 408.

Another example of the description contemplates these polynucleotides incorporated into an expression vector for expression in mammalian cells such as CHO, NSO, HEK-293, or in fungal, insect, plant or microbial systems such as yeast cells such as the yeast Pichia. Suitable Pichia species include, but are not limited to, Pichia pastoris. In one example of the description described herein (infra), Fab fragments may be produced via expression of Fab2 polynucleotides in mammalian cells such as CHO, NSO or HEK 293 cells, fungal, insect, plant or microbial systems such as yeast cells (for example diploid yeast such as diploid Pichia) and other yeast strains. Suitable Pichia species include, but are not limited to, Pichia pastoris.

In one example, the description is optionally directed to an isolated polynucleotide comprising a polynucleotide encoding an anti-NGF V_{H} antibody amino acid sequence selected from SEQ ID NO: 3, 13, 23, 33, 43, 53, 63, 73, 83, 93, 103, 113, 123, 133, 143, 153, 163, 173, 183, 193, or 402, or encoding a variant thereof wherein at least one framework residue (FR residue) has been substituted with an amino acid present at the corresponding position in a rabbit anti-NGF antibody V_{H} polypeptide or a conservative amino acid substitution.

In another optional example, the description is directed to an isolated polynucleotide comprising the polynucleotide sequence encoding an anti-NGF V_{L} antibody amino acid sequence of 1, 11, 21, 31, 41, 51, 61, 71, 81, 91, 101, 111, 121, 131, 141, 151, 161, 171, 181, 191, or 401, or encoding a variant thereof wherein at least one framework residue (FR residue) has been substituted with an amino acid present at the corresponding position in a rabbit anti-NGF antibody V_{L} polypeptide or a conservative amino acid substitution.

In yet another optional example, the description is directed to one or more heterologous polynucleotides comprising a sequence encoding the polypeptides contained in SEQ ID NO:1 and SEQ ID NO:3; SEQ ID NO:11 and SEQ ID NO:13; SEQ ID NO:21 and SEQ ID NO:23; SEQ ID NO:31 and SEQ ID NO:33; SEQ ID NO:411 and SEQ ID NO:43; SEQ ID NO:51 and SEQ ID NO:53, SEQ ID NO:61 and SEQ ID NO:63; SEQ ID NO:71 and SEQ ID NO:73; SEQ ID NO:81 and SEQ ID NO:83; SEQ ID NO:91 and SEQ ID NO:93; SEQ ID NO:101 and SEQ ID NO:103; SEQ ID NO:111 and SEQ ID NO:113; SEQ ID NO:121 and SEQ ID NO:123; SEQ ID NO:131 and SEQ ID NO:133; SEQ ID NO:141 and SEQ ID NO:143; SEQ ID NO:151 and SEQ ID NO:153; SEQ ID NO:161 and SEQ ID NO:163; SEQ ID NO:171 and SEQ ID NO:173; SEQ ID NO:181 and SEQ ID NO:183; SEQ ID NO:191 and SEQ ID NO:193; or SEQ ID NO:401 and SEQ ID NO:403.

In another example, the description is optionally directed to an isolated polynucleotide that expresses a polypeptide containing at least one CDR polypeptide derived from an anti-NGF antibody wherein said expressed polypeptide alone specifically binds NGF or specifically binds NGF when expressed in association with another polynucleotide sequence that expresses a polypeptide containing at least one CDR polypeptide derived from an anti-NGF antibody for treatment or prevention of pain and pain associated conditions wherein said at least one CDR is selected from those contained in the V_{L} or V_{H} polypeptides of SEQ ID NO: 1, 3, 11, 13, 21, 23, 31, 33, 41, 43, 51, 53, 61, 63, 71, 73, 81, 83, 91, 93, 101, 103, 111, 113, 121, 123, 131, 133, 141, 143, 151, 153, 161, 163, 171, 173, 181, 183, 191, 193, 401 or SEQ ID NO:403.

Host cells and vectors comprising said polynucleotides are also contemplated.

The description further optionally contemplates vectors comprising the polynucleotide sequences encoding the variable heavy and light chain polypeptide sequences, as well as the individual complementarity-determining regions (CDRs, or hypervariable regions), as set forth herein, as well as host cells comprising said vector sequences. In one example of the description, the host cell is a yeast cell. In another example of the description, the yeast host cell belongs to the genus Pichia.

### EXAMPLES

The following examples are put forth so as to provide those of ordinary skill in the art with a complete disclosure and description of how to make and use the subject invention, and are not intended to limit the scope of what is regarded as the invention. Efforts have been made to ensure accuracy with respect to the numbers used (e.g. amounts, temperature, concentrations, etc.) but some experimental errors and deviations should be allowed for. Unless otherwise indicated, parts are parts by weight, molecular weight is average molecular weight, temperature is in degrees centigrade; and pressure is at or near atmospheric.

### EXAMPLE 1

This example describes culture methods that improved the purity of recombinant antibodies produced from cultured *P. pastoris* cells. When a bolus of ethanol was added during culturing, the resulting antibodies exhibited a great decrease in the concentration of an undesired product-associated variant.

### Methods

To generate the inoculum, diploid *P. pastoris* was grown using a medium composed of the following nutrients (percentages are given as w/v): yeast extract 3%, anhydrous dextrose 2%, YNB 1.34%, Biotin 0.004% and 100 mM potassium phosphate (pH 6.0). The inoculum medium for runs L355, L357, L358, L359 and L360 was composed of the following nutrients (percentages are given as w/v): yeast extract 3%, glycerol 2%, YNB 1.34%, Biotin 0.004%, 200 mM potassium phosphate (pH 6.0) The inoculum was grown for approximately 24 hours to 29 hours in a shaking incubator at 30°C and 300 rpm. A 10% inoculum was then added to Labfors 2.5L working volume vessels containing 1 L sterile growth medium. The growth medium was composed of the following nutrients: potassium sulfate 18.2 g/L, ammonium phosphate monobasic 36.4 g/L, potassium phosphate dibasic 12.8 g/L, magnesium sulfate heptahydrate 3.72 g/L, sodium citrate dihydrate 10 g/L, glycerol 40 g/L, yeast extract 30 g/L, PTM1 trace metals 4.35 mL/L, and antifoam 204 1.67 mL/L. The PTM1 trace metal solution was comprised of the following components: cupric sulfate pentahydrate 6 g/L, sodium iodide 0.08 g/L, manganese sulfate hydrate 3 g/L, sodium molybdate dihyrate 0.2 g/L, boric acid 0.02 g/L, cobalt chloride 0.5 g/L, zinc chloride 20 g/L, ferrous sulfate heptahydrate 65 g/L, biotin 0.2 g/L, and sulfuric acid 5 mL/L. The yeast strain was engineered to express Ab-A antibody from four integrated genomic copies of the heavy chain coding sequence (SEQ ID NO: 441) and 3 copies of the light chain coding sequence (SEQ ID NO: 440). The heavy chain gene copies were integrated into the pGAP locus (3 copies) and HIS4 TT locus (1 copy) while the 3 light chain gene copies were integrated into the pGAP locus. The antibody chain gene copies were each under the control of the GAP promoter. The bioreactor process control parameters were set as follows: Agitation 1000 rpm, airflow 1.35 standard liter per minute, temperature 28°C and pH was controlled (at 6) using ammonium hydroxide. No oxygen supplementation was provided.

Following addition of the inoculum, fermentation cultures were grown for approximately 12 to 16 hours (the "growth phase"). The growth phase ended when the initial glycerol in the medium was consumed, as which was detected by a dissolved oxygen ("DO") spike (a sudden increase in the dissolved oxygen concentration). The cultures were then starved for approximately three hours after the dissolved oxygen spike ("starvation phase") for run L306. For other runs, the ethanol bolus was added immediately after DO spike. A bolus of ethanol was then added to the reactor to give a final concentration of 1% ethanol (w/v). Control cultures were treated identically, except that the bolus addition of ethanol was omitted. The fermentation cultures were allowed to equilibrate for 15 to 30 minutes ("equilibration phase"). After the equilibration phase, feed was added at a constant rate of 30 g/L/hr for 40 minutes ("transition phase"). For the remainder of the culture ("production phase") the ethanol concentration was detected using an ethanol sensing probe (Raven Biotech) which was used to control the feed rate, with the feed rate being set at 15 g/L/hr when the ethanol concentration was below the set point, or 7.5 g/L/hr when the ethanol concentration was above the set point. In instances in which the high feed rate of 15 g/L/hr was not high enough to maintain ethanol at set point (which occurred in the L315 fermentation run), the high feed rate was set to 22.5 g/L/hr while the low feed rate was set to 15 g/L/hr. The same set point was maintained whether or not an ethanol bolus had been added to the culture (production of ethanol by the yeast caused the set point to be reached without the bolus addition of ethanol). The feed was composed of the following components: yeast extract 50 g/L, anhydrous dextrose 500 g/L, magnesium sulfate heptahydrate 3 g/L, PTM1 trace metals 12 mL/L, and sodium citrate dihydrate 0.5g/L The total fermentation time was typically 85 hours to 97 hours in these experiments, though longer and shorter times can also be used.

After the production phase, fermentation cultures had PEI (polyethyleneimine) and EDTA (ethylenediaminetetraacetic acid) added to 0.05% w/v and 3 mM final concentrations respectively. The cultures were then spun in a centrifuge and antibodies were purified from the culture supernatant by Protein A affinity. Briefly, approximately 20 mL of 0.2µ clarified supernatants from harvested fermentation broth were diluted with the same volume of equilibration buffer (20 mM Histidine pH6). From this diluted broth, 20 mL were then loaded onto a pre-equilibrated 1 mL HiTrap MabSelect Sure column (GE, Piscataway, NJ). The column was subsequently washed using 40 column volumes of equilibration buffer. The antibody bound onto the column was eluted using a step gradient into 100% elution buffer (100 mM Citric Acid pH 3.0). One mL fractions were collected and immediately neutralized using 100µL of 2M Tris buffer pH 8.0. Protein containing fractions were determined by measuring absorbance at 280nM and protein-containing fractions were pooled.

Protein A purified antibodies were analyzed for purity by SDS-PAGE. For non-reduced samples, SDS-PAGE was carried out using precast polyacrylamide gels (NuPAGE® Bis-Tris Gels) containing a 4%-12% polyacrylamide gradient, using NuPAGE® MES SDS running buffer and NuPAGE® LDS Sample Buffer (all from Invitrogen, Carlsbad, Ca.) in accord with the manufacturer's instructions. Proteins were then visualized by Coomassie blue staining. Reduced samples were processed in the same manner except that samples were reduced prior to loading using the NuPAGE® Sample Reducing Agent (Invitrogen, Carlsbad, Ca.) in accord with the manufacturer's instructions.

### Results

To determine the effect of a bolus addition of ethanol during culture on antibody purity, the antibody Ab-A was produced from yeast cultures with or without the addition of a bolus of ethanol to a final concentration of 1% (10 g/L) at the end of the growth phase and prior to the production phase. The production phase was continued for 97 hours **(****FIG. 1****),** 87 hours **(****FIG. 2****),** or 86 hours **(****FIG. 3****).** The antibody produced by each culture was then harvested from the culture media, purified by Protein A affinity chromatography.

SDS-PAGE was used to detect the relative abundance of the full antibody, the "half antibody" or H1L1 complex (containing one heavy and one light chain) and the H2L1 complex (containing two heavy chains and one light chain). The abundance of the H1L1 and H2L1 complexes was greatly decreased in the cultures that were produced with the bolus addition of ethanol. This improvement was reproduced in three experiments shown in **FIG.** 1A, compare lanes 2 and 3 (with bolus) to 5 (no bolus); **FIG. 2A****,** compare lane 2 (with bolus) to 3 (no bolus); **FIG. 3A****,** compare lanes 2 and 4 (with bolus) to lanes 5-7 (no bolus). Under reducing conditions, the H1L1, H2L1, and full antibody species were each separated into individual heavy and light chains, confirming the identity of the 75 kDa band as consisting of one light and one heavy chain joined by a disulfide-linkage (**FIGS.** 1B and 2B; lane order is the same as **FIGS.** 1A and 1B, respectively).

The decrease in abundance of the H1L1 species was then quantified using ImageJ to plot the gel band density along the length of the non-reduced gels (**FIGS.** 1C-E and 2C-D, respectively corresponding to **FIG.** 1A, lanes 2, 3, and 5, and **FIG. 2A****,** lanes 2 and 3, and FIG. 3B, corresponding to lanes 2 and 4-6 of FIG. 3A). The area under the H1L1 peak was quantified and results are tabulated in **FIGS.** 1F, 2E, and 3B. Based on these measurements, the addition of an ethanol bolus prior to antibody production decreased the relative abundance of the 75 kDa band by about 90% in **FIG.** 1A, by about 85% in **FIG.** 2A, and by about 87% in **FIG.** 3A.

In summary, these results demonstrate that the concentration of the H1L1 species was greatly decreased by the bolus addition of ethanol to the culture, resulting in decreased production of the H1L1 species between about 85% to 90%.

### EXAMPLE 2

This example extends the results obtained in Example 1 by demonstrating that the same methods produced a similar improvement in antibody purity when used during production of two additional antibodies.

### Methods

Antibodies Ab-B and Ab-C were recombinantly produced using the methods described in Example 1. Antibody Ab-C was expressed from a yeast strain engineered contain four copies of the heavy chain coding sequence (SEQ ID NO: 439) and three copies of the light chain coding sequence (SEQ ID NO: 438). Samples were taken from the reactors and culture supernatant containing antibodies was collected after a total fermentation time of 67 hours (T67) or 87 hours (T87) for the Ab-B antibody and for 86 hours (T86) for the Ab-C antibody, purified by Protein A affinity, and analyzed by SDS-PAGE as described in Example 1.

### Results

Antibodies Ab-B (FIG. 4) and Ab-C (FIG. 5) were produced with or without a bolus addition of ethanol to a final concentration of 1% at the end of the growth phase and prior to the production phase. For the antibodies produced without the bolus addition of ethanol, the H1L1 or half antibody species, and the H2L1 species were each observed as a prominent band (**FIG.** 4A, lines 6 and 7; FIG. 4C, lanes 6 and 7; **FIG. 5A****,** lanes 5 and 6). The intensity of these bands were greatly decreased for the culture produced with a bolus addition of ethanol (**FIG.** 4A, lanes 2-3; FIG. 4C, lanes 2-3; **FIG.** 5A, lane 3). Under reducing conditions, the H1L1 and H2L1 bands were separated into individual heavy and light chains, confirming the identity of these species as consisting full length heavy and light chains (**FIGS.** 4B, 4D, and 5B; lane order is the same as in **FIGS.** 4A, 4D, and 5A, respectively).

The decrease in abundance of the H1L1 species was then quantified using ImageJ to plot the gel band density along the length of the non-reduced gels. **FIGS.** 4E and 4F tabulate the area contained in the H1L1 peaks shown in **FIGS.** 4A (T67) and 4C (T87), respectively, demonstrating that the bolus addition of ethanol produced about a 73% reduction in the relative abundance of H1L1 complexes at the earlier time point shown **FIG.** 4A and about a 34% average reduction in the relative abundance of H1L1 complexes at the later time point shown in **FIG.** 4C. Similarly, **FIG.** 5C tabulates the area contained in the H1L1 peaks shown in **FIGS.** 5A, demonstrating about a 61% average reduction in the relative abundance of H1L1 complexes by the bolus addition of ethanol.

In summary, these results demonstrate that the concentration of the H1L1 and H2L1 species were decreased by between about 61% and 73% by the bolus addition of ethanol to the culture for two additional antibodies having binding specificity for different targets.

### EXAMPLE 3

This example further describes the improved purity of recombinant antibodies produced from cultured *P. pastoris* cells through addition of a bolus of ethanol during culturing. In addition to greatly decreasing the abundance of the H1L1 and H2L1 species, this example further demonstrates a decrease in the concentration of other product-associated variants.

### Methods

Recombinant antibodies Ab-A, Ab-B, and Ab-C were prepared and purified from *P. pastoris* cultures as described in Examples 1 and 2. Antibodies were produced either with or without a bolus addition of ethanol to a final concentration of 1% (w/v) at the end of the growth phase and prior to the production phase. To analyze the purity of protein A purified antibody preparations, size exclusion high-performance liquid chromatography (SE-HPLC) was used. Briefly, an Agilent (Santa Clara, CA) 1200 Series HPLC with UV detection instrument was used. For sample separation, a TSKgel 3000SW_{XL} 7.8x300 mm column connected with a TSKgel Guard SW_{XL} 6x40 mm from Tosoh Bioscience (King of Prussia, PA) was used. A solution of 100 mM sodium phosphate, 200 mM sodium chloride pH 6.5 was used as mobile phase with a flow rate of 0.5 mL/min in isocratic mode and absorbance at UV 215nm was monitored. Before injection of samples the column was equilibrated until a stable baseline was achieved. Samples were diluted to a concentration of 1 mg/mL using mobile phase and a 30 µL volume was injected. To monitor column performance, BioRad (Hercules, CA) gel filtration standards were used.

### Results

The Ab-A antibody preparations described in Example 1 and additional preparations produced using the same methods were expressed in yeast, purified by protein A affinity, and then analyzed for purity using size exclusion chromatography (SEC). Under the condition used, the half antibody (H1L1) species co-elutes with the full antibody, which is thought to be due to non-covalent association between pairs of half antibodies. However, this method allows purity to be assessed with respect to other product-associated variants, such complexes having aberrant stoichiometry, fragments, glycosylated forms, and aggregates.

SE-HPLC data are shown for Ab-A samples produced without (FIGS. 6A, 6C, and 6E) or with (FIGS. 6B, 6D, and 6F) a bolus addition of ethanol. A product-associated variant consisting of an aggregate of two full antibodies (containing four heavy and four light chains) was detected (arrow), and the abundance thereof was reduced on average in the samples prepared with the bolus addition. FIG. 7 shows quantification of the purity of Ab-A by determining the percentage of the antibody preparation contained in the main peak (containing the full antibody). The bolus addition of ethanol increased the average percentage contained in the main peak from 80.3% up to 90.6%.

Similar analysis was performed for the Ab-B and Ab-C antibody preparations described in Example 2, quantified in FIGS. 8 and 9, respectively. Overall purity of the Ab-B antibody was improved, with the average fraction in the main peak increasing from 76% to 79% at T67 and from 60% to 73% at T87. For the Ab-C antibody, there was little detectable difference in antibody purity assessed by this method, apparently due to the high initial purity of the Ab-C antibody even without the bolus addition.

In summary, these results demonstrate that a bolus addition of ethanol to the culture can decrease the concentration of other product-associated variants in addition to the half-antibody species.

### EXAMPLE 4

This example describes the further confirmation of the identity of the 75 kDa product associated variant as a half antibody species containing only one antibody heavy chain and only one antibody light chain. This hypothesis was based on several observations. First, the 75 kDa band was present in protein A purified samples (see Example 1) indicating that it contained at least the protein A binding portion of an antibody heavy chain. Second, the 75 kDa band was prominent in non-reduced samples analyzed by SDS-PAGE (see, e.g., **FIG.** 1A, lanes 2-3), but under reducing conditions the same samples did not contain any bands of comparable intensity (other than the expected light and heavy chains), indicating that the 75 kDa band does not include any components other than antibody heavy and light chains (or other species having the same electrophoretic mobility). Third, disappearance of the 75 kDa band from the reduced samples also indicates that its constituents are linked by at least one disulfide bond. Fourth, SEC analysis had demonstrated co-elution of the 75 kDa species with the full antibody, strongly suggesting that the 75 kDa species can non-covalently self-associate to form a full antibody (or another complex of the same apparent hydrodynamic radius). Finally, the apparent molecular weight of about 75 kDa (determined by reference to electrophoresis standards), taken together with the observation (from denaturing gels) that this complex was only made up of full-length antibody chains, was consistent with complex containing only one heavy chain and only one light chain, but was inconsistent with other complexes.

### Methods

Mass spectrometry was used to detect the relative abundance of heavy chains lacking the inter-heavy chain disulfide bonds (normally found at amino acids 220 and 223) in different samples. Two-hundred and fifty micrograms of each sample was added into an Eppendorf tube. An appropriate amount (-450 µL) of denaturing buffer (6 M Guanidine-HCl, 1 mM EDTA, 0.25 M Tris, pH 7.5) was added to the tube to obtain a final volume of 500 µL and a sample concentration of 0.5 mg/mL. Twelve and a half microliters of 2 M Iodoacetamide was added into each sample to alkylate any free cysteine. The samples were vortexed then incubated at room temperature, in the dark, for 30 ± 5 minutes. The samples were then desalted using NAP-5 columns pre-equilibrated with digestion buffer (0.1 M Tris-HCl, pH 7.5). Each sample solution was added to separate (pre-equilibrated) columns and allowed to enter the column bed. One milliliter of digestion buffer was added to each column and the eluent was collected into Eppendorf tubes. The samples were divided into equal aliquots containing approximately 50 µg of material (five 200 µL aliquots). The alkylated and desalted aliquots were stored at -20 °C until needed. One aliquot of each sample (alkylated, and desalted) was used for each digestion. Trypsin solution at 0.5 mg/mL was added to each sample aliquot at a 1:25 w:w ratio of trypsin:protein (4 µL). All trypsin tubes were incubated at 37 ± 2 °C for 4 hours. After incubation, the enzymatic digestion was quenched by adding 1 µL of Trifluoroacetic acid to each tube. The samples were then divided into two equal portions, reduced and non-reduced. Half the samples were reduced in the presence of 1 M DTT for 1 hour at 37 ± 2 °C. The contents of both reduced and nonreduced samples were transferred to HPLC vials and placed in the autosampler for analysis.

MS and MS/MS data was collected on a Micromass Q-TOF Ultima mass spectrometer using electrospray ionization (ESI) in positive ion mode. Data was acquired from m/z 200-1950 in MS mode. Prior to analysis, the mass spectrometer was calibrated using a 5th order fit on fragment ions of [Glu¹]-Fibrinopeptide covering a range from m/z 175 to 1285. The injections volumes were adjusted to achieve an on-column load of approximately 20 pmoles of protein.

### Results

Based on the observations discussed above, Applicants hypothesized that the 75 kDa band was a "half antibody" species containing one heavy antibody chain and one light antibody chain covalently linked to one another through disulfide bonds, and that pairs of half antibody complexes could non-covalently associate to form a complex having the same stoichiometry as a full antibody (two heavy chains and two light chains) but lacking the disulfide linkages between the two heavy chains ("unlinked heavy chains"). Based thereon, it was predicted the relative abundance of the 75 kDa band would correlate with the relative proportion of unlinked heavy chains, which was determined using mass spectrometry analysis of trypsin digested antibody samples.

The peptide fragments of interest for this study were the T17 trypsin fragment of the heavy chain (T17H) which is composed of amino acids 217-242, respectively. Amino acids 220 and 223 are responsible for disulfide bonding between antibody heavy chains. Typically, free cysteine analysis can be conducted by determining a ratio of alkylated cysteine residues to non-alkylated residues in reduced samples after a tryptic digestion. However, alkylated species were not present in either lot of material. It was hypothesized the two cysteine residues of the peptide fragment of interest, T17H, were bonding to each other, or the cysteines were protected by the antibody's quaternary structure. In either case, the cysteine residues would not be accessible for alkylation. Instead, the analytical approach was to utilize the ratio of non-reduced species to reduced species in both lots to calculate a percent difference. It was observed that the non-reduced samples had a 2 Da decrease in molecular weight, indicative of disulfide bonding. This behavior was exploited to calculate the percent free T17H. The theoretical mass of the non-reduced T17H species is 2727.41 Da (disulfide bonding between Cys220 & Cys223) and 2729.41 Da for reduced peptide.

The extracted ion chromatograms of reduced and non-reduced samples were analyzed for representative charge states. ratio of counts between the non-reduced to reduced samples calculates the free T17H species to be 2.3% in the antibody produced with a bolus addition of ethanol, and 26.1% in the sample produced without a bolus addition of ethanol. These results are presented in tabular form in Fig. 9.

Thus, the abundance of heavy chains lacking the inter-heavy chain disulfide bond was greatly increased in the sample produced without the bolus addition of ethanol, further confirming the identity of this species as containing one heavy chain and one light chain but lacking an inter-heavy chain disulfide bond. Moreover, the detection of a species 2 Da lighter than the expected mass indicated that heavy chain in the H1L1 species may contain an extra disulfide bond to itself which may interfere with the formation of the normal inter-heavy chain disulfide bond.

### EXAMPLE 5

This example demonstrates a correlation between cell viability and antibody purity. The addition of an ethanol bolus generally improved cell viability and antibody purity for Ab-A and the Ab-B antibody. Further, the Ab-C antibody, which already exhibited high purity even without the bolus addition, also exhibited higher culture viability. Taken together, these results suggest that the improvement in antibody purity resulting from a bolus addition of ethanol is at least partially attributable to increased culture viability.

### Methods

Culture viability was determined using a Cellometer (Nexcelom). Culture samples were diluted with PBS so that the final cell count was within 1 x 10⁷ to 5 x 10⁷ cells/mL. One half of the sample was then treated to heat conditions of 75°C for 10 minutes as positive control for propidium iodide (PI) staining. The untreated sample and treated sample were then mixed with PI (20 uL of sample plus 20 uL of PI). The sample was then placed in a slide cassette and viability was determined by counting the number of non-fluorescing cells then dividing by the total number of cells. Cells that are dead have taken up the propidium iodide and are fluorescing so the positive control heat killed sample should show less than 1% cells alive.

### Results

Cell viability was determined for antibody-producing cultures grown with or without a bolus addition of ethanol as described in Examples 1 and 2. As described above, purity of the Ab-A and Ab-B antibodies were greatly improved by a bolus addition of ethanol to the yeast culture (see Examples 1-2 and FIGS. 1-4). The addition of an ethanol bolus improved cell viability for these cultures as well. For the Ab-A antibody, viability improved from 91.9% to 97.2% on average (FIG. 11), while for the Ab-B antibody, viability improved from 84.8% to 95.1% on average (FIG. 12). Due to the already high purity of the Ab-C antibody produced even without the bolus addition of ethanol, the improvements in the purity of this antibody resulting from the bolus addition of ethanol were more modest (see Example 3 and FIG. 5). Consistent with the observation that high cell viability correlated with higher antibody purity, the Ab-C antibody cultures exhibited high cell viability (95.8%% on average) in the absence of a bolus addition of ethanol, which was little changed by the bolus addition of ethanol (96.8%).

Taken together, these results indicate that the improvement in antibody purity resulting from the addition of an ethanol bolus may be in part caused by (or at least correlates with) an improvement in cell viability.

### EXAMPLE 6

This example demonstrates that a similar improvement in antibody purity can be attained with varying ethanol bolus concentrations.

### Methods

The Ab-A antibody was produced as in Example 1, except that the bolus addition of ethanol was 5 g/L (0.5% w/v), 10 g/L (1% w/v), or 15 g/L (1.5% w/v). Antibodies samples were purified from the culture media at 63 and 86 hours and purified by protein A affinity, then the purity was analyzed by non-reduced SDS-PAGE as in Example 1.

### Results

Antibody purity was similarly high irrespective of the bolus concentration added (between 0.5% and 1.5% w/v), at both time points tested, 63 hours (FIG. 14A) and 86 hours (FIG, 14B). Detected levels of the H1L1 and H2L1 species were similarly low in each culture.

These results indicate that the improvement in antibody purity can be attained while varying the ethanol bolus concentration.

### EXAMPLE 7

This example demonstrates that similar improvements in antibody purity can be attained while varying the duration of the "starvation period" between the dissolved oxygen spike and the addition of the ethanol bolus to the cultures.

### Methods

The Ab-A antibody was produced as in Example 1, except that the duration of the starvation period, the time between the dissolved oxygen spike (indicating exhaustion of the carbon source in the culture) and the bolus addition of ethanol, was either 0 hours or 3 hours. Antibodies samples were purified from the culture media and purified by protein A affinity, then the purity was analyzed by non-reduced SDS-PAGE as in Example 1.

### Results

Antibody purity was similarly high irrespective of the variation of the starvation period between 0 and 3 hours (FIG. 15A, compare lanes 5 (0 hours starvation) and 6 (3 hours starvation)). Detected levels of the H1L1 and H2L1 species were similarly low in each culture.

These results indicate that the improvement in antibody purity can be attained with a varying the duration of the starvation period.

### EXAMPLE 8

This example tests the effect on antibody purity from varying the duration of the "equilibration period" between the addition of the ethanol bolus and commencement of adding the feed to the cultures.

### Methods

The Ab-B antibody was produced as in Example 2, except that the duration of the equilibration period, the time between addition of the ethanol bolus and the commencement of feeding the culture, was 0, 30, or 60 minutes. Additionally, the yeast strain from which the Ab-B antibody was produced contained three copies of the light chain gene instead of four. Antibodies samples were purified from the culture media and purified by protein A affinity, then the purity was analyzed by non-reduced SDS-PAGE as in Example 1. Viability was also assessed using the methods described in Example 5.

### Results

Antibody purity was similarly high for an equilibration period of 0 or 30 minutes (FIG. 16A, lanes 7 and 8 (0 minutes equilibration time) and lane 3 (30 minutes equilibration time). However, detected levels of the H1L1 and H2L1 species were increased in the culture with the 60 minute equilibration period (FIG. 16A, lanes 5 and 6).

Viability was also assessed for each culture at the 23 hour and 85 hour time points. For the 60 minute equilibration period, viability was between 75% and 80% at 23 hours, while at the same time point viability was approximately 88-90% for the 0 and 30 minute equilibration periods (FIG. 16B). Subsequently, at 85 hours, viability had improved but remained somewhat reduced for the 60 minute equilibration period relative to the 0 and 30 minute equilibration periods (FIG. 16C).

These results indicate that the improvement in antibody purity can be attained while varying the equilibration period at least between 0 and 30 minutes, while some loss of viability and purity may occur for an equilibration period of 60 minutes or longer (though purity may still be improved relative to a control culture without a bolus addition of ethanol).

The above description of various illustrated embodiments of the invention is not intended to be exhaustive or to limit the invention to the precise form disclosed. The teachings provided herein of the invention can be applied to other purposes, other than the examples described above.

The invention may be practiced in ways other than those particularly described in the foregoing description and examples.

These and other changes can be made to the invention in light of the above detailed description. In general, in the following claims, the terms used should not be construed to limit the invention to the specific embodiments disclosed in the specification and the claims. Accordingly, the invention is not limited by the disclosure, but instead the scope of the invention is to be determined entirely by the following claims.

Certain teachings related to humanization of rabbit-derived monoclonal antibodies and preferred sequence modifications to maintain antigen binding affinity were disclosed in International Application No. PCT/US2008/064421, corresponding to International Publication No. WO/2008/144757, entitled "Novel Rabbit Antibody Humanization Methods and Humanized Rabbit Antibodies", filed May 21, 2008.

Certain teachings related to producing antibodies or fragments thereof using mating competent yeast and corresponding methods were disclosed in U.S. Patent application no. 11/429,053, filed May 8, 2006, (U.S. Patent Application Publication No. US2006/0270045).

## Claims

1. A method of producing a multi-subunit complex, comprising:
(a) providing a culture comprising *Pichia pastoris* cells comprising genes that provide for the expression of the subunits of said multi-subunit complex;
(b) adding a bolus of ethanol to said culture, which results in an ethanol concentration in said culture of between 0.5% and 1.5% (w/v); and
(c) adding a feed comprising at least one fermentable carbon source to said culture, and culturing said culture to produce said multi-subunit complex,
wherein said multi subunit complex comprises an entire or full-length antibody comprising heavy and light chain polypeptides,
wherein said multi-subunit complex is not an anti-NGF antibody containing the polypeptide sequences SEQ ID NO: 401 and SEQ ID NO: 402.

2. The method of claim 1, wherein:
(i) the ethanol bolus enhances the formation of stable disulfide bonds relative to the same method effected in the absence of the bolus of ethanol;
(ii) the culture method decreases the relative abundance of one or more product-associated variants relative to the same method effected in the absence of the bolus of ethanol;
(iii) the culture method decreases the relative abundance of product-associated variants having a higher or lower apparent molecular weight than said desired antibody as detected by size exclusion chromatography or gel electrophoresis relative to the same method effected in the absence of the bolus of ethanol;
(iv) the culture method decreases the relative abundance of antibodies having aberrant stoichiometry relative to the same method effected in the absence of the bolus of ethanol;
(v) the culture method decreases the relative abundance of antibodies having aberrant disulfide bonds relative to the same method effected in the absence of the bolus of ethanol;
(vi) the culture method decreases the relative abundance of antibodies having reduced cysteines relative to the same method effected in the absence of the bolus of ethanol;
(vii) the culture method decreases the relative abundance of antibodies having aberrant glycosylation relative to the same method effected in the absence of the bolus of ethanol;
(viii) the culture method modulates the formation or stability of inter-heavy chain disulfide bonds;
(ix) the culture method modulates the formation or stability of disulfide bonds linking the light and heavy chains of said antibody;
(x) the culture method decreases the relative abundance of one or more of the H1L1, H2L1, and H4L4 product-associate variants; or
(xi) the culture method increases the purity of said antibody relative to said method effected in the absence of said bolus of ethanol.

3. The method of claim 1 or 2, wherein:
(i) step (b) results in a concentration of ethanol in said culture of between 0.7% and 1.5%, or between 0.8% and 1.25% (w/v);
(ii) step (b) results in a concentration of ethanol in said culture that is 0.5%, 0.6%, 0.7%, 0.8% or 0.9% (w/v);
(iii) step (b) results in a concentration of ethanol in said culture that is 1.5%, 1.4%, 1.3%, 1.2%, 1.1%, 1.0%, 0.9%, 0.8%, 0.7%, 0.6%, or 0.5% (w/v);
(iv) step (b) comprises adding ethanol to said culture, adding a carrier comprising ethanol to said culture, adding said cells to a medium or carrier comprising ethanol, or replacing part of the culture medium;
(v) the bolus of ethanol is added to the culture medium over a period of time between 1 and 20 minutes;
(vi) step (c) comprises providing oxygen to said cells;
(vii) step (c) comprises providing oxygen to said cells by agitating the culture;
(viii) step (c) comprises providing oxygen to said cells by contacting said culture with a gas mixture comprising oxygen;
(ix) step (c) comprises adding a feed comprising one or more of glucose, ethanol, citrate, sorbitol, xylose, trehalose, arabinose, galactose, fructose, melibiose, lactose, maltose, rhamnose, ribose, mannose, mannitol, and raffinose;
(x) the culture method further comprises maintaining the concentration of ethanol between an upper set point and a lower set point during step (c);
(xi) the culture method further comprises maintaining the concentration of ethanol between an upper set point and a lower set point during step (c), wherein said lower set point is 0.5%, 0.6%, 0.7%, 0.8% or 0.9% (w/v);
(xii) the culture method further comprises maintaining the concentration of ethanol between an upper set point and a lower set point during step (c) wherein said upper set point is 1.5%, 1.4%, 1.3%, 1.2%, 1.1%, 1.0%, 0.9%, 0.8%, 0.7%, or 0.6% (w/v); or
(xiii) the culture method further comprises maintaining the concentration of ethanol between an upper set point and a lower set point during step (c), wherein said lower set point is 0.5%, 0.6%, 0.7%, 0.8% or 0.9% (w/v) and said upper set point is 1.5%, 1.4%, 1.3%, 1.2%, 1.1%, 1.0%, 0.9%, 0.8%, 0.7%, or 0.6% (w/v).

4. The method of any one of claims 1 to 3, wherein:
(i) the concentration of ethanol is maintained at a set point during step (c);
(ii) the concentration of ethanol is maintained at a set point which is 0.5%, 0.6%, 0.7%, 0.8%, 0.9%, 1%, 1.1%, 1.2%, 1.3%, 1.4%, or 1.5% (w/v) during step (c);
(iii) step (c) comprises maintaining the concentration of ethanol in said culture between 0.7% and 1.5%, or between 0.8% and 1.25% (w/v);
(iv) the concentration of ethanol in the culture of step (c) is maintained by controlling production of ethanol by said cells or by addition of ethanol to said culture;
(v) the concentration of ethanol in the culture of step (c) is maintained by controlling production of ethanol by said cells or by addition of ethanol to said culture, wherein the step of controlling production of ethanol comprises controlling one or more of the concentration of glucose, availability of oxygen, intensity of agitation, gas pressure, flow rate of supplied air or other gas mixture, viscosity of the culture, culture density, concentration of oxygen in the supplied air or other gas mixture, and temperature;
(vi) the time between step (a) and step (b) is less than 72 hours, less than 48 hours, less than 24 hours, less than 12 hours, less than 9 hours, less than 6 hours, less than 5 hours, less than 4 hours, less than 3 hours, less than 90 minutes, less than 30 minutes, less than 5 minutes, or less than 1 minute;
(vii) the time between step (b) and step (c) is less than 10 hours, less than 9 hours, less than 8 hours, less than 7 hours, less than 6 hours, less than 5 hours, less than 4 hours, less than 3 hours, less than 2 hours, less than 90 minutes, less than 80 minutes, less than 70 minutes, less than 60 minutes, less than 50 minutes, less than 40 minutes, less than 30 minutes, less than 20 minutes, less than 10 minutes, less than 5 minutes, or less than 1 minute;
(viii) the culture of step (a) is produced by adding a carbon source to said culture, and culturing said culture until the carbon source is depleted;
(ix) the culture of step (a) is produced by adding a carbon source to said culture, and culturing said culture until the carbon source is depleted, wherein said carbon source comprises one or more of: glycerol, glucose, ethanol, citrate, sorbitol, xylose, trehalose, arabinose, galactose, fructose, melibiose, lactose, maltose, rhamnose, ribose, mannose, mannitol, and raffinose.
(x) the culture of step (a) is produced by adding a carbon source to said culture, and culturing said culture until the carbon source is depleted wherein the depletion of the carbon source is determined by detecting a decrease in the metabolic activity of said *Pichia pastoris* cells;
(xi) the culture of step (a) is produced by adding a carbon source to said culture, and culturing said culture until the carbon source is depleted wherein the depletion of the carbon source is determined by detecting a decrease in the metabolic activity of said *Pichia pastoris* cells, wherein said decrease in the metabolic activity of said *Pichia pastoris* cells is identified by detecting a decrease in the consumption of oxygen by said *Pichia pastoris* cells, by detecting an increase in pH in the culture, by detecting stabilization of the wet cell mass, or by detecting an increase in the concentration of ammonia in the culture; or
(xii) the culture of step (a) is produced by adding a carbon source to said culture, and culturing said culture until the carbon source is depleted wherein the depletion of the carbon source is determined by detecting a decrease in the metabolic activity of said *Pichia pastoris* cells, wherein said decrease in the metabolic activity of said *Pichia pastoris* cells is identified by detecting a decrease in the consumption of oxygen by said *Pichia pastoris* cells, by detecting an increase in pH in the culture, by detecting stabilization of the wet cell mass, or by detecting an increase in the concentration of ammonia in the culture, wherein said decrease in the consumption of oxygen by said *Pichia pastoris* cells is identified by detecting an increase in the concentration of dissolved oxygen in said culture.

5. The method of any one of claims 1-4, wherein:
(i) the *Pichia pastoris* cells comprise genes integrated in their genome that provide for expression of said antibody wherein said genes are integrated into one or more genomic loci;
(ii) the *Pichia pastoris* cells comprise genes integrated in their genome at loci selected from the group consisting of the pGAP locus, 3' AOX TT locus; PpURA5; OCHl; AOX1; HIS4; GAP; pGAP; 3' AOX TT; ARG; and the HIS4 TT locus;
(iii) the genes encoding the subunits of the antibody are expressed under control of an inducible or constitutive promoter;
(iv) the genes encoding the subunits of the antibody are expressed under control of an inducible promoter selected from the group consisting of the AOX1, CUP1, tetracycline inducible, thiamine inducible, and FLDl promoters;
(v) at least one of the genes encoding said subunits of the antibody are expressed under control of a promoter selected from the group consisting of: the CUP1, AOX1, ICL1, glyceraldehyde-3 -phosphate dehydrogenase (GAP), FLD1, ADH1, alcohol dehydrogenase II, GAL4, PHO3, PHO5, and Pyk promoters, tetracycline inducible promoters, thiamine inducible promoters, chimeric promoters derived therefrom, yeast promoters, mammalian promoters, insect promoters, plant promoters, reptile promoters, amphibian promoters, viral promoters, and avian promoters; and/or
(xi) the *Pichia pastoris* cells are diploid, tetraploid, or polyploid cells.

6. The method of any one of claims 1-5, further comprising at least one of the following:
(i) purifying said antibody from said *Pichia pastoris* cells or from the culture medium;
(ii) purifying said antibody from an intracellular component, cytoplasm, nucleoplasm, or a membrane of said *Pichia pastoris* cells;
(iii) the *Pichia pastoris* cells secrete said antibody into the culture medium;
(iv) purifying said antibody from said culture medium;
(v) the antibody comprises a monospecific or bispecific antibody;
(vi) the antibody comprises a human antibody or a humanized antibody;
(vii) the antibody comprises a humanized antibody wherein said humanized antibody is of mouse, rat, rabbit, goat, sheep, or cow origin;
(viii) the antibody comprises a humanized antibody of rabbit origin;
(ix) the antibody comprises a monovalent, bivalent, or multivalent antibody;
(x) the antibody is purified from said culture by protein A and/or protein G affinity;
(xi) at least one of the genes that provide for expression of a subunit of said antibody in at least one of said *Pichia pastoris* cells in said panel is optimized for expression in said yeast cell;
(xii) the purity of said antibody is assessed by measuring the fraction of the antibody produced by said yeast cell that is contained in antibody complexes having the expected apparent hydrodynamic radius, is contained in antibody complexes having the expected molecular weight, and / or specifically binds a target of said antibody ;
(xiii) the yield of said antibody is assessed by determining the amount of antibody produced by said yeast cell discounting any product-associated variants that are abnormally glycosylated, contained in antibody complexes other than complexes having the expected apparent hydrodynamic radius, contained in antibody complexes having the expected molecular weight, and / or that fail to specifically bind to the target of said antibody and the molecular weight of said antibody complexes is optionally determined by non-reducing SDS-PAGE;
(xiv) said method further comprises purifying said antibody;
(xv) the culture cells produce a supernatant antibody titer of at least 100 mg / L, at least 150 mg / L, at least 200 mg / L, at least 250 mg / L, at least 300 mg / L, between 100 and 300 mg / L, between 100 and 500 mg / L, between 100 and 1000 mg / L, at least 1000 mg / L, at least 1250 mg/liter, at least 1500 mg/liter, at least 1750 mg/liter, at least 2000 mg/liter, at least 10000 mg/liter, or more;
(xvi) one or more subunits of said antibody are expressed from more than one gene copy;
(xvii) the antibody is expressed from between 1-10 copies of a gene encoding the light chain of said antibody and from 1-10 copies of a gene encoding the heavy chain of said antibody, optionally integrated into the genome of said cells;
(xviii) the genes that provide for expression of said antibody are contained on an extrachromosomal element, plasmid, or artificial chromosome;
(xix) the *Pichia pastoris* cells comprise more copies of the gene that provide for the expression of the light chain of said antibody than copies of the gene that provide for expression of the heavy chain of said antibody;
(xx) the cultured cells comprise more copies of the gene that provide for the expression of the light chain of said antibody than copies of the gene that provide for expression of the heavy chain of said antibody, wherein the respective number of copies of the gene encoding the heavy chain of said antibody and the number of copies of the gene encoding the light chain of said antibody in said cells are: 2 and 2, 2 and 3, 3 and 3, 3 and 4, 3 and 5, 4 and 3, 4 and 4, 4 and 5, 4 and 6, 5 and 4, 5 and 5, 5 and 6, or 5 and 7; and
(xxi) the cultured cells comprise more copies of the gene that provide for the expression of the light chain of said antibody than copies of the gene that provide for expression of the heavy chain of said antibody, and further wherein the respective number of copies of the gene encoding the heavy chain of said antibody and the number of copies of the gene encoding the light chain of said antibody in said cells are: 2 and 1, 3 and 1, 4 and 1, 5 and 1, 6 and 1, 7 and 1, 8 and 1, 9 and 1, 10 and 1, 1 and 2, 2 and 2, 3 and 2, 4 and 2, 5 and 2, 6 and 2, 7 and 2, 8 and 2, 9 and 2, 10 and 2, 1 and 3, 2 and 3, 3 and 3, 4 and 3, 5 and 3, 6 and 3, 7 and 3, 8 and 3, 9 and 3, 10 and 3, 1 and 4, 2 and 4, 3 and 4, 4 and 4, 5 and 4, 6 and 4, 7 and 4, 8 and 4, 9 and 4, 10 and 4, 1 and 5, 2 and 5, 3 and 5, 4 and 5, 5 and 5, 6 and 5, 7 and 5, 8 and 5, 9 and 5, 10 and 5, 1 and 6, 2 and 6, 3 and 6, 4 and 6, 5 and 6, 6 and 6, 7 and 6, 8 and 6, 9 and 6, 10 and 6, 1 and 7, 2 and 7, 3 and 7, 4 and 7, 5 and 7, 6 and 7, 7 and 7, 8 and 7, 9 and 7, 10 and 7, 1 and 8, 2 and 8, 3 and 8, 4 and 8, 5 and 8, 6 and 8, 7 and 8, 8 and 8, 9 and 8, 10 and 8, 1 and 9, 2 and 9, 3 and 9, 4 and 9, 5 and 9, 6 and 9, 7 and 9, 8 and 9, 9 and 9, 10 and 9, 1 and 10, 2 and 10, 3 and 10, 4 and 10, 5 and 10, 6 and 10, 7 and 10, 8 and 10, 9 and 10, 10 and 10.

7. The method of any one of the foregoing claims, wherein:
(i) the culture of step (c) is grown in a production medium;
(ii) the culture of step (c) is grown in a production medium comprising a minimal medium;
(iii) the culture of step (c) is grown in a production medium comprising a minimal medium lacking selective agents.;
(iv) the culture of step (c) is grown in a production medium comprising a minimal medium lacking pre-formed amino acids or other complex biomolecules;
(v) the culture of step (c) is grown in a production medium that comprises a complex medium;
(vi) the culture of step (c) is grown in a production medium that comprises a complex medium that comprises one or more of yeast extract, soy peptones, and other plant peptones;
(vii) the culture of step (c) is grown to a high cell density;
(viii) the culture of step (c) is grown to a cell density which is at least 50 g/L;
(ix) the culture of step (c) is grown to a cell density which is at least 100 g/L;
(x) the culture of step (c) is grown to a cell density which is at least 300 g/L;
(xi) the culture of step (c) is grown to a cell density which is at least 400 g/L;
(xii) the culture of step (c) is grown to a cell density which is at least 500 g/L;
(xiii) the culture of step (c) is grown to a cell density which is at least 750 g/L;
(xiv) the *Pichia pastoris* cells of step (c) are cultured for at least 20 doublings and maintain high levels of expression of said antibody after said at least 20 doublings;
(xv) the *Pichia pastoris* cells of step (c) are cultured for at least 50 doublings and maintain high levels of expression of said antibody after said at least 50 doublings; or
(xvi) the *Pichia pastoris* cells of step (c) are cultured for at least 100 doublings and maintain high levels of expression of said antibody after said at least 100 doublings.

8. The method of any one of claims 1-7 wherein at least one subunit of the antibody is encoded by a gene that comprises a secretion signal wherein optionally the secretion signal comprises one or more polypeptides selected from the group consisting of: SEQ ID NOS: 414 to 437 and any combination thereof.

9. The method of any of claims 1 to 8 wherein the multi subunit complex does not contain all six CDR sequences of SEQ ID NOs:5-10, does not contain all six CDR sequences of SEQ ID NOs: 15-20, does not contain all six CDR sequences of SEQ ID NOs:25-30, does not contain all six CDR sequences of SEQ ID NOs:35-40, does not contain all six CDR sequences of SEQ ID NOs:45-50, does not contain all six CDR sequences of SEQ ID NOs:55-60, does not contain all six CDR sequences of SEQ ID NOs:65-70, does not contain all six CDR sequences of SEQ ID NOs:75-80, does not contain all six CDR sequences of SEQ ID NOs:85-90, does not contain all six CDR sequences of SEQ ID NOs:95-100, does not contain all six CDR sequences of SEQ ID NOs: 105-110, does not contain all six CDR sequences of SEQ ID NOs: 115-120, does not contain all six CDR sequences of SEQ ID NOs: 125-130, does not contain all six CDR sequences of SEQ ID NOs:135-140, does not contain all six CDR sequences of SEQ ID NOs: 145-150, does not contain all six CDR sequences of SEQ ID NOs: 155-160, does not contain all six CDR sequences of SEQ ID NOs:165-170, does not contain all six CDR sequences of SEQ ID NOs:175-180, does not contain all six CDR sequences of SEQ ID NOs:185-190, does not contain all six CDR sequences of SEQ ID NOs:195-200.

10. The method of any of claims 1 to 9, wherein the multi subunit complex is not an anti-NGF antibody.

11. The method of any of claims 1 to 8 or 10
wherein the multi subunit complex does not contain at least one, at least two, at least three, at least four, or at least five of the CDRs contained in any of the following antibodies: an antibody having the CDR sequences of SEQ ID Nos 5-10, an antibody having the CDR sequences of SEQ ID NOs:15-20, an antibody having the CDR sequences of SEQ ID NOs: 25-30, an antibody having the CDR sequences of SEQ ID NOs: 35-40, an antibody having the CDR sequences of SEQ ID NOs: 45-50, an antibody having the CDR sequences of SEQ ID NOs: 55-60, an antibody having the CDR sequences of SEQ ID NOs: 65-70, an antibody having the CDR sequences of SEQ ID NOs: 75-80, an antibody having the CDR sequences of SEQ ID NOs: 85-90, an antibody having the CDR sequences of SEQ ID NOs: 95-100, an antibody having the CDR sequences of SEQ ID NOs: 105-110, an antibody having the CDR sequences of SEQ ID NOs: 115-120, an antibody having the CDR sequences of SEQ ID NOs: 125-130, an antibody having the CDR sequences of SEQ ID NOs: 135-140, an antibody having the CDR sequences of SEQ ID NOs: 145-150, an antibody having the CDR sequences of SEQ ID NOs: 155-160, an antibody having the CDR sequences of SEQ ID NOs: 165-170, an antibody having the CDR sequences of SEQ ID NOs: 175-180, an antibody having the CDR sequences of SEQ ID NOs: 185-190, or an antibody having the CDR sequences of SEQ ID NOs: 195-200 and optionally having binding specificity for NGF.

## Patentansprüche

1. Verfahren zum Herstellen eines Multi-Subeinheitenkomplexes, umfassend:
(a) Bereitstellen einer Kultur, umfassend *Pichia pastoris* Zellen, umfassend Gene, welche die Expression der Subeinheiten des Multi-Subeinheitenkomplexes bereitstellen;
(b) Zusetzen eines Bolus aus Ethanol zu der Kultur, was zu einer Ethanolkonzentration in der Kultur von zwischen 0,5 % und 1,5 % (w/v) führt; und
(c) Zusetzen eines Eintrags, umfassend zumindest eine fermentierbare Kohlenstoffquelle zu der Kultur, und Kultivieren der Kultur, um den Multi-Subeinheitenkomplex herzustellen,
wobei der Multi-Subeinheitenkomplex einen gesamten oder Volllängen-Antikörper umfasst, umfassend schwer- und leichtkettige Polypeptide.
wobei der Multi-Subeinheitenkomplex kein Anti-NGF-Antikörper ist, der die Polypeptidsequenzen SEQ ID NO: 401 und SEQ ID NO: 402 enthält.

2. Verfahren nach Anspruch 1, wobei:
(i) der Ethanolbolus die Formulierung von stabilen Disulfidbindungen bezüglich desselben Verfahrens, das in Abwesenheit des Ethanolbolus bewirkt wird, verbessert;
(ii) das Kultivierungsverfahren die relative Häufigkeit eines oder mehrerer produktbezogener Varianten bezüglich desselben Verfahrens, das in der Abwesenheit des Ethanolbolus bewirkt wird, reduziert;
(iii) das Kultivierungsverfahren die relative Häufigkeit von produktbezogenen Varianten, die ein höheres oder niedrigeres offensichtliches Molekulargewicht aufweisen als der gewünschte Antikörper, wie durch Größenausschlusschromatographie oder Gelelektrophorese nachgewiesen, bezüglich desselben Verfahrens, das in der Abwesenheit des Ethanolbolus bewirkt wird, reduziert;
(iv) das Kultivierungsverfahren die relative Häufigkeit von Antikörpern, die eine abweichende Stöchiometrie bezüglich desselben Verfahrens, das in der Abwesenheit des Ethanolbolus bewirkt wird, reduziert;
(v) das Kultivierungsverfahren die relative Häufigkeit von Antikörpern, die abweichende Disulfidbindungen bezüglich desselben Verfahrens, das in der Abwesenheit des Ethanolbolus bewirkt wird, reduziert;
(vi) das Kultivierungsverfahren die relative Häufigkeit von Antikörpern, die reduzierte Cysteine bezüglich desselben Verfahrens, das in der Abwesenheit des Ethanolbolus bewirkt wird, reduziert;
(vii) das Kultivierungsverfahren die relative Häufigkeit von Antikörpern, die eine abweichende Glycosylierung bezüglich desselben Verfahrens, das in der Abwesenheit des Ethanolbolus bewirkt wird, reduziert;
(viii) das Kultivierungsverfahren die Ausbildung oder Stabilität von inter-schweren Kettendisulfidbindungen moduliert;
(ix) das Kultivierungsverfahren die Ausbildung oder Stabilität von Disulfidbindungen moduliert, die leichte und schwere Ketten des Antikörpers verketten;
(x) das Kultivierungsverfahren die relative Häufigkeit von einem oder mehreren der H1L1, H2L1 und H4L4 produktbezogenen Varianten reduziert; oder
(xi) das Kultivierungsverfahren die Reinheit des Antikörpers bezüglich des Verfahrens, das in der Abwesenheit des Ethanolbolus bewirkt wird, erhöht.

3. Verfahren nach Anspruch 1 oder 2, wobei:
(i) Schritt (b) zu einer Konzentration von Ethanol in der Kultur von zwischen 0,7 % und 1,5 % oder zwischen 0,8 % und 1,25 % (w/v) führt;
(ii) Schritt (b) zu einer Konzentration von Ethanol in der Kultur führt, die 0,5 %, 0,6 %, 0,7 %, 0,8 % oder 0,9 % (w/v) beträgt;
(iii) Schritt (b) zu einer Konzentration von Ethanol in der Kultur führt, die 1,5 %, 1,4 %, 1,3 %, 1,2 %, 1,1 %, 1,0 %, 0,9 %, 0,8 %, 0,7 %, 0,6 % oder 0,5 % (w/v) beträgt;
(iv) Schritt (b) das Zusetzen von Ethanol zu der Kultur, das Zusetzen eines Trägers, umfassend Ethanol, zu der Kultur, das Zusetzen der Zellen zu einem Medium oder Träger, das/der Ethanol umfasst, oder das Ersetzen eines Teils des Kulturmediums umfasst;
(v) das Bolus von Ethanol zu dem Kulturmedium über einem Zeitraum von zwischen 1 und 20 Minuten zugesetzt wird;
(vi) Schritt (c) das Bereitstellen von Sauerstoff an die Zellen umfasst;
(vii) Schritt (c) das Bereitstellen von Sauerstoff an die Zellen durch Rühren der Kultur umfasst;
(viii) Schritt (c) das Bereitstellen von Sauerstoff an die Zellen durch Kontaktieren der Kultur mit einer Gasmischung, umfassend Sauerstoff, umfasst;
(ix) Schritt (c) das Zusetzen eines Eintrags, umfassend ein oder mehrere von Glucose, Ethanol, Citrat, Sorbitol, Xylose, Trehalose, Arabinose, Galaktose, Fructose, Melibiose, Lactose, Maltose, Rhamnose, Ribose, Mannose, Mannitol und Raffinose, umfasst;
(x) das Kultivierungsverfahren weiter das Aufrechterhalten der Konzentration von Ethanol zwischen einem oberen Sollwert und einem unteren Sollwert während Schritt (c) umfasst;
(xi) das Kultivierungsverfahren weiter das Aufrechterhalten der Konzentration von Ethanol zwischen einem oberen Sollwert und einem unteren Sollwert während Schritt (c) umfasst, wobei der untere Sollwert 0,5 %, 0,6 %, 0,7 %, 0,8 % oder 0,9 % (w/v) beträgt;
(xii) das Kultivierungsverfahren weiter das Aufrechterhalten der Konzentration von Ethanol zwischen einem oberen Sollwert und einem unteren Sollwert während Schritt (c) umfasst, wobei der obere Sollwert 1,5 %, 1,4 %, 1,3 %, 1,2 %, 1,1 %, 1,0 %, 0,9 %, 0,8 %, 0,7 % oder 0,6 % (w/v) beträgt; oder
(xiii) das Kultivierungsverfahren weiter das Aufrechterhalten der Konzentration von Ethanol zwischen einem oberen Sollwert und einem unteren Sollwert während Schritt (c) umfasst, wobei der untere Sollwert 0,5 %, 0,6 %, 0,7 %, 0,8 % oder 0,9 % (w/v) beträgt und der obere Sollwert 1,5 %, 1,4 %, 1,3 %, 1,2 %, 1,1 %, 1,0 %, 0,9 %, 0,8 %, 0,7 % oder 0,6 % (w/v) beträgt.

4. Verfahren nach einem der Ansprüche 1 bis 3, wobei:
(i) die Konzentration von Ethanol an einem Sollwert während Schritt (c) aufrechterhalten wird;
(ii) die Konzentration von Ethanol an einem Sollwert aufrechterhalten wird, der 0,5 %, 0,6 %, 0,7 %, 0,8 %, 0,9 %, 1 %, 1,1 %, 1,2 %, 1,3 %, 1,4 % oder 1,5 % (w/v) während Schritt (c) beträgt;
(iii) Schritt (c) das Aufrechterhalten der Konzentration von Ethanol in der Kultur zwischen 0,7 % und 1,5 % oder zwischen 0,8 % und 1,25 % (w/v) umfasst;
(iv) die Konzentration von Ethanol in der Kultur von Schritt (c) durch Steuern der Herstellung von Ethanol durch die Zellen oder durch einen Zusatz von Ethanol zu der Kultur aufrechterhalten wird;
(v) die Konzentration von Ethanol in der Kultur von Schritt (c) durch Steuern der Herstellung von Ethanol durch die Zellen oder durch einen Zusatz von Ethanol zu der Kultur aufrechterhalten wird, wobei der Schritt des Steuerns der Herstellung von Ethanol das Steuern von einer oder mehreren von der Konzentration von Glucose, der Verfügbarkeit von Sauerstoff, der Intensität des Rührens, des Gasdrucks, der Flussrate von zugeführter Luft oder einer anderen Gasmischung, der Viskosität der Kultur, der Kulturdichte, der Konzentration von Sauerstoff in der zugeführten Luft oder der anderen Gasmischung und der Temperatur umfasst;
(vi) der Zeitraum zwischen Schritt (a) und Schritt (b) weniger als 72 Stunden, weniger als 48 Stunden, weniger als 24 Stunden, weniger als 12 Stunden, weniger als 9 Stunden, weniger als 6 Stunden, weniger als 5 Stunden, weniger als 4 Stunden, weniger als 3 Stunden, weniger als 90 Minuten, weniger als 30 Minuten, weniger als 5 Minuten oder weniger als 1 Minute beträgt;
(vii) der Zeitraum zwischen Schritt (b) und Schritt (c) weniger als 10 Stunden, weniger als 9 Stunden, weniger als 8 Stunden, weniger als 7 Stunden, weniger als 6 Stunden, weniger als 5 Stunden, weniger als 4 Stunden, weniger als 3 Stunden, weniger als 2 Stunden, weniger als 90 Minuten, weniger als 80 Minuten, weniger als 70 Minuten, weniger als 60 Minuten, weniger als 50 Minuten, weniger als 40 Minuten, weniger als 30 Minuten, weniger als 20 Minuten, weniger als 10 Minuten, weniger als 5 Minuten oder weniger als 1 Minute beträgt;
(viii) die Kultur von Schritt (a) durch den Zusatz einer Kohlenstoffquelle zu der Kultur und das Kultivieren der Kultur, bis die Kohlenstoffquelle ausgeschöpft ist, hergestellt wird;
(ix) die Kultur von Schritt (a) durch den Zusatz einer Kohlenstoffquelle zu dieser Kultur und das Kultivieren der Kultur, bis die Kohlenstoffquelle ausgeschöpft ist, hergestellt wird, wobei die Kohlenstoffquelle eine oder mehrere von Folgendem umfasst: Glycerol, Glucose, Ethanol, Citrat, Sorbitol, Xylose, Trehalose, Arabinose, Galaktose, Fructose, Melibiose, Lactose, Maltose, Rhamnose, Ribose, Mannose, Mannitol und Raffinose;
(x) die Kultur von Schritt (a) durch den Zusatz einer Kohlenstoffquelle zu der Kultur und das Kultivieren der Kultur, bis die Kohlenstoffquelle ausgeschöpft ist, hergestellt wird, wobei das Ausschöpfen der Kohlenstoffquelle durch Nachweisen einer Reduktion in der Stoffwechselaktivität der *Pichia pastoris* Zellen bestimmt wird;
(xi) die Kultur von Schritt (a) durch den Zusatz einer Kohlenstoffquelle zu der Kultur und das Kultivieren der Kultur, bis die Kohlenstoffquelle ausgeschöpft ist, hergestellt wird, wobei das Ausschöpfen der Kohlenstoffquelle durch den Nachweis einer Reduktion der Stoffwechselaktivität der *Pichia pastoris* Zellen festgestellt wird, wobei die Reduktion der Stoffwechselaktivität der *Pichia pastoris* Zellen durch den Nachweis einer Reduktion des Sauerstoffverbrauchs der *Pichia pastoris* Zellen, durch den Nachweis einer Erhöhung des pH-Werts in der Kultur, durch den Nachweis einer Stabilisierung der Nasszellenmasse oder durch den Nachweis einer Erhöhung der Konzentration von Ammoniak in der Kultur, identifiziert wird; oder
(xii) die Kultur von Schritt (a) durch den Zusatz einer Kohlenstoffquelle zu der Kultur und das Kultivieren der Kultur, bis die Kohlenstoffquelle ausgeschöpft ist, hergestellt wird, wobei das Ausschöpfen der Kohlenstoffquelle durch den Nachweis einer Reduktion der Stoffwechselaktivität der *Pichia pastoris* Zellen bestimmt wird, wobei die Reduktion der Stoffwechselaktivität der *Pichia pastoris* Zellen durch den Nachweis einer Reduktion des Sauerstoffverbrauchs der *Pichia pastoris* Zellen, durch den Nachweis einer Erhöhung des pH-Werts in der Kultur, durch den Nachweis einer Stabilisierung der Nasszellenmasse oder durch den Nachweis einer Erhöhung der Konzentration von Ammoniak in der Kultur identifiziert wird, wobei die Erhöhung des Sauerstoffverbrauchs der *Pichia pastoris* Zellen durch den Nachweis einer Erhöhung der Konzentration von aufgelöstem Sauerstoff in der Kultur identifiziert wird.

5. Verfahren nach einem der Ansprüche 1-4, wobei:
(i) die *Pichia pastoris* Zellen Gene umfassen, die in deren Genom integriert sind, die eine Expression des Antikörpers bereitstellen, wobei die Gene in einem oder mehreren Genomen loci integriert sind;
(ii) die *Pichia pastoris* Zellen Gene umfassen, die in deren Genom an Loci integriert sind, die ausgewählt sind aus der Gruppe bestehend aus dem pGAP-Locus, 3' AOXTT-Locus; PpURA5; OCH1; AOX1; HIS4; GAP; pGAP; 3' AOXTT; ARG und dem HIS4TT-Locus;
(iii) die Gene, die für die Subeinheiten des Antikörpers kodieren, unter Steuerung eines induzierbaren oder konstitutiven Promotors exprimiert werden;
(iv) die Gene, die für die Subeinheiten des Antikörpers kodieren, unter Steuerung eines induzierbaren Promotors exprimiert werden, der ausgewählt ist aus der Gruppe bestehend aus den AOX1-, CUP1-, Tetracyclin-induzierbaren, Thiamin-induzierbaren und FLD1-Promotoren;
(v) zumindest eines der Gene, das für die Subeinheiten des Antikörpers kodiert, unter Steuerung eines Promotors exprimiert wird, der ausgewählt ist aus der Gruppe bestehend aus: den CUP1-, AOX1-, ICL1-, Glyceraldehyd-3-phosphatdehydrogenase (GAP), FLD1-, ADH1-, Alkoholdehydrogenase II-, GAL4-, PHO3-, PHO5- und Pyk-Promotoren, Tetracylin-induzierbaren Promotoren, Thiamin-induzierbaren Promotoren, davon abgeleiteten chimären Promotoren, Hefe-Promotoren, Säuger-Promotoren, Insekten-Promotoren, Pflanzen-Promotoren, Reptil-Promotoren, Amphibien-Promotoren, viralen Promotoren und Vogel-Promotoren; und/oder
(vi) die *Pichia pastoris* Zellen Diploid-, Tetraploid- oder Polyploidzellen sind.

6. Verfahren nach einem der Ansprüche 1-5, weiter umfassend zumindest eines der Folgenden:
(i) Reinigen des Antikörpers von den *Pichia pastoris* Zellen oder von dem Kulturmedium;
(ii) Reinigen des Antikörpers von einer intrazellulären Komponente, Zytoplasma, Nukleoplasma oder einer Membran dieser *Pichia pastoris* Zellen;
(iii) wobei die *Pichia pastoris* Zellen den Antikörper in das Kulturmedium sekretieren;
(iv) Reinigen des Antikörpers von dem Kulturmedium;
(v) wobei der Antikörper einen monospezifischen oder bispezifischen Antikörper umfasst;
(vi) wobei der Antikörper einen humanen Antikörper oder einen humanisierten Antikörper umfasst;
(vii) wobei der Antikörper einen humanisierten Antikörper umfasst, wobei der humanisierte Antikörper einen Ursprung von Mäusen, Ratten, Hasen, Ziegen, Schafen oder Kühen aufweist;
(viii) wobei der Antikörper einen humanisierten Antikörper umfasst, der einen Ursprung von Hasen aufweist;
(ix) wobei der Antikörper einen monovalenten, bivalenten oder multivalenten Antikörper umfasst;
(x) wobei der Antikörper durch eine Protein A- und/oder Protein G-Affinität von der Kultur gereinigt wird;
(xi) wobei zumindest eines der Gene, das eine Expression einer Subeinheit des Antikörpers in zumindest einer der *Pichia pastoris* Zellen in dem Panel bereitstellt, für eine Expression in der Hefezelle optimiert ist;
(xii) wobei die Reinheit des Antikörpers durch Messen der Fraktion des Antikörpers bewertet wird, der durch die Hefezelle produziert wird, welche in den Antikörperkomplexen mit dem erwartungsgemäßen, offensichtlich hydrodynamischen Radius enthalten ist, welche in Antikörperkomplexen mit dem erwarteten Molekulargewicht enthalten ist, und/oder sich spezifisch an ein Ziel des Antikörpers bindet;
(xiii) wobei die Ausbeute des Antikörpers durch Bestimmen der Menge des durch die Hefezelle produzierten Antikörpers bewertet ist, unter Abzug beliebiger mit dem Produkt assoziierten Varianten, die abweichend glykosyliert sind, die in den Antikörperkomplexen mit dem erwartungsgemäßen, offensichtlich hydrodynamischen Radius enthalten sind, in Antikörperkomplexen mit dem erwarteten Molekulargewicht enthalten sind, und/oder sich nicht spezifisch an das Ziel des Antikörpers binden und wobei das Molekulargewicht der Antikörperkomplexe optional durch nichtreduzierende SDS-PAGE bestimmt wird;
(xiv) wobei das Verfahren weiter das Reinigen des Antikörpers umfasst;
(xv) wobei die Kultivierungszellen einen Überstands-Antikörpertiter von zumindest 100 mg/l, zumindest 150 mg/l, zumindest 200 mg/l, zumindest 250 mg/l, zumindest 300 mg/l, zwischen 100 und 300 mg/l, zwischen 100 und 500 mg/l, zwischen 100 und 1000 mg/l, zumindest 1000 mg/l, zumindest 1250 mg/Liter, zumindest 1500 mg/Liter, zumindest 1750 mg/Liter, zumindest 2000 mg/Liter, zumindest 10000 mg/Liter oder mehr produzieren;
(xvi) wobei eine oder mehrere Subeinheiten des Antikörpers aus mehr als einer Genkopie exprimiert werden;
(xvii) wobei der Antikörper aus zwischen 1-10 Kopien eines Gens exprimiert wird, das für die leichte Kette des Antikörpers codiert, und von 1-10 Kopien eines Gens, das für die schwere Kette des Antikörpers kodiert, das optional in das Genom der Zellen integriert ist;
(xviii) wobei die Gene, die eine Expression des Antikörpers bereitstellen, an einem extrachromosomalen Element, Plasmid oder künstlichen Chromosom enthalten sind;
(xix) wobei die *Pichia pastoris* Zellen mehr Kopien des Gens umfassen, welche die Expression der leichten Kette des Antikörpers bereitstellen, als Kopien der Gene, die eine Expression der schweren Kette des Antikörpers bereitstellen;
(xx) wobei die kultivierten Zellen mehr Kopien des Gens umfassen, welches die Expression der leichten Kette des Antikörpers bereitstellen, als Kopien der Gene, die eine Expression der schweren Kette des Antikörpers bereitstellen, wobei die entsprechende Anzahl von Kopien des Gens, die für die schwere Kette des Antikörpers kodieren, und die Anzahl der Kopien des Gens, die für die leichte Kette des Antikörpers in den Zellen kodieren, folgende sind: 2 und 2, 2 und 3, 3 und 3, 3 und 4, 3 und 5, 4 und 3, 4 und 4, 4 und 5, 4 und 6, 5 und 4, 5 und 5, 5 und 6 oder 5 und 7; und
(xxi) wobei die kultivierten Zellen mehr Kopien des Gens umfassen, welches die Expression der leichten Kette des Antikörpers bereitstellen, als Kopien der Gene, die eine Expression der schweren Kette des Antikörpers bereitstellen, und weiter wobei die entsprechende Anzahl von Kopien des Gens, die für die schwere Kette des Antikörpers kodieren, und die Anzahl der Kopien des Gens, die für die leichte Kette des Antikörpers in den Zellen kodieren, folgende sind: 2 und 1, 3 und 1, 4 und 1, 5 und 1, 6 und 1, 7 und 1, 8 und 1, 9 und 1, 10 und 1, 1 und 2, 2 und 2, 3 und 2, 4 und 2, 5 und 2, 6 und 2, 7 und 2, 8 und 2, 9 und 2, 10 und 2, 1 und 3, 2 und 3, 3 und 3, 4 und 3, 5 und 3, 6 und 3, 7 und 3, 8 und 3, 9 und 3, 10 und 3, 1 und 4, 2 und 4, 3 und 4, 4 und 4, 5 und 4, 6 und 4, 7 und 4, 8 und 4, 9 und 4, 10 und 4, 1 und 5, 2 und 5, 3 und 5, 4 und 5, 5 und 5, 6 und 5, 7 und 5, 8 und 5, 9 und 5, 10 und 5, 1 und 6, 2 und 6, 3 und 6, 4 und 6, 5 und 6, 6 und 6, 7 und 6, 8 und 6, 9 und 6, 10 und 6, 1 und 7, 2 und 7, 3 und 7, 4 und 7, 5 und 7, 6 und 7, 7 und 7, 8 und 7, 9 und 7, 10 und 7, 1 und 8, 2 und 8, 3 und 8, 4 und 8, 5 und 8, 6 und 8, 7 und 8, 8 und 8, 9 und 8, 10 und 8, 1 und 9, 2 und 9, 3 und 9, 4 und 9, 5 und 9, 6 und 9, 7 und 9, 8 und 9, 9 und 9, 10 und 9, 1 und 10, 2 und 10, 3 und 10, 4 und 10, 5 und 10, 6 und 10, 7 und 10, 8 und 10, 9 und 10, 10 und 10.

7. Verfahren nach einem der vorstehenden Ansprüche, wobei:
(i) die Kultur von Schritt (c) in einem Produktionsmedium kultiviert wird;
(ii) die Kultur von Schritt (c) in einem Produktionsmedium kultiviert wird, das ein minimales Medium umfasst;
(iii) die Kultur von Schritt (c) in einem Produktionsmedium kultiviert wird, das ein minimales Medium umfasst, dem selektive Mittel fehlen;
(iv) die Kultur von Schritt (c) in einem Produktionsmedium kultiviert wird, das ein minimales Medium umfasst, dem vorgeformte Aminosäuren oder andere komplexe Biomoleküle fehlen;
(v) die Kultur von Schritt (c) in einem Produktionsmedium kultiviert wird, das ein komplexes Medium umfasst;
(vi) die Kultur von Schritt (c) in einem Produktionsmedium kultiviert wird, das ein komplexes Medium umfasst, das eines oder mehrere von Hefeextrakt, Sojapeptonen und anderen Pflanzenpeptonen umfasst;
(vii) die Kultur von Schritt (c) bis zu einer hohen Zelldichte kultiviert wird;
(viii) die Kultur von Schritt (c) bis zu einer Zelldichte kultiviert wird, die zumindest 50 g/l beträgt;
(ix) die Kultur von Schritt (c) bis zu einer Zelldichte kultiviert wird, die zumindest 100 g/l beträgt;
(x) die Kultur von Schritt (c) bis zu einer Zelldichte kultiviert wird, die zumindest 300 g/l beträgt;
(xi) die Kultur von Schritt (c) bis zu einer Zelldichte kultiviert wird, die zumindest 400 g/l beträgt;
(xii) die Kultur von Schritt (c) bis zu einer Zelldichte kultiviert wird, die zumindest 500 g/l beträgt;
(xiii) die Kultur von Schritt (c) bis zu einer Zelldichte kultiviert wird, die zumindest 750 g/l beträgt;
(xiv) die *Pichia pastoris* Zellen von Schritt (c) für zumindest 20 Verdopplungen kultiviert werden und hohe Expressionswerte des Antikörpers nach den zumindest 20 Verdopplungen aufrechterhalten;
(xv) die *Pichia pastoris* Zellen von Schritt (c) für zumindest 50 Verdopplungen kultiviert werden und hohe Expressionswerte des Antikörpers nach den zumindest 50 Verdopplungen aufrechterhalten; oder
(xvi) die *Pichia pastoris* Zellen von Schritt (c) für zumindest 100 Verdopplungen kultiviert werden und hohe Expressionswerte des Antikörpers nach den zumindest 100 Verdopplungen aufrechterhalten.

8. Verfahren nach einem der Ansprüche 1-7, wobei zumindest eine Subeinheit des Antikörpers durch ein Gen kodiert wird, das ein Sekretionssignal umfasst, wobei das Sekretionssignal optional ein oder mehrere Polypeptide umfasst, die ausgewählt sind aus der Gruppe bestehend aus: SEQ ID NO: 414 bis 437 und jeder beliebigen Kombination davon.

9. Verfahren nach einem der Ansprüche 1 bis 8, wobei der Multi-Subeinheitenkomplex nicht alle sechs CDR-Sequenzen von SEQ ID NO: 5-10 enthält, nicht alle sechs CDR-Sequenzen von SEQ ID NO: 15-20 enthält, nicht alle sechs CDR-Sequenzen von SEQ ID NO: 25-30 enthält, nicht alle sechs CDR-Sequenzen von SEQ ID NO: 35-40 enthält, nicht alle sechs CDR-Sequenzen von SEQ ID NO: 45-50 enthält, nicht alle sechs CDR-Sequenzen von SEQ ID NO: 55-60 enthält, nicht alle sechs CDR-Sequenzen von SEQ ID NO: 65-70 enthält, nicht alle sechs CDR-Sequenzen von SEQ ID NO: 75-80 enthält, nicht alle sechs CDR-Sequenzen von SEQ ID NO: 85-90 enthält, nicht alle sechs CDR-Sequenzen von SEQ ID NO: 95-100 enthält, nicht alle sechs CDR-Sequenzen von SEQ ID NO: 105-110 enthält, nicht alle sechs CDR-Sequenzen von SEQ ID NO: 115-120 enthält, nicht alle sechs CDR-Sequenzen von SEQ ID NO: 125-130 enthält, nicht alle sechs CDR-Sequenzen von SEQ ID NO: 135-140 enthält, nicht alle sechs CDR-Sequenzen von SEQ ID NO: 145-150 enthält, nicht alle sechs CDR-Sequenzen von SEQ ID NO: 155-160 enthält, nicht alle sechs CDR-Sequenzen von SEQ ID NO: 165-170 enthält, nicht alle sechs CDR-Sequenzen von SEQ ID NO: 175-180 enthält, nicht alle sechs CDR-Sequenzen von SEQ ID NO: 185-190 enthält, nicht alle sechs CDR-Sequenzen von SEQ ID NO: 195-200 enthält.

10. Verfahren nach einem der Ansprüche 1 bis 9, wobei der Multi-Subeinheitenkomplex kein Anti-NGF-Antikörper ist.

11. Verfahren nach einem der Ansprüche 1 bis 8 oder 10, wobei der Multi-Subeinheitenkomplex nicht zumindest eine, zumindest zwei, zumindest drei, zumindest vier oder zumindest fünf der CDR enthält, die in einem beliebigen der folgenden Antikörper enthalten sind: einem Antikörper, der die CDR-Sequenzen von SEQ ID NO: 5-10 aufweist, einem Antikörper, der die CDR-Sequenzen von SEQ ID NO: 15-20 aufweist, einem Antikörper, der die CDR-Sequenzen von SEQ ID NO: 25-30 aufweist, einem Antikörper, der die CDR-Sequenzen von SEQ ID NO: 35-40 aufweist, einem Antikörper, der die CDR-Sequenzen von SEQ ID NO: 45-50 aufweist, einem Antikörper, der die CDR-Sequenzen von SEQ ID NO: 55-60 aufweist, einem Antikörper, der die CDR-Sequenzen von SEQ ID NO: 65-70 aufweist, einem Antikörper, der die CDR-Sequenzen von SEQ ID NO: 75-80 aufweist, einem Antikörper, der die CDR-Sequenzen von SEQ ID NO: 85-90 aufweist, einem Antikörper, der die CDR-Sequenzen von SEQ ID NO: 95-100 aufweist, einem Antikörper, der die CDR-Sequenzen von SEQ ID NO: 105-110 aufweist, einem Antikörper, der die CDR-Sequenzen von SEQ ID NO: 115-120 aufweist, einem Antikörper, der die CDR-Sequenzen von SEQ ID NO: 125-130 aufweist, einem Antikörper, der die CDR-Sequenzen von SEQ ID NO: 135-140 aufweist, einem Antikörper, der die CDR-Sequenzen von SEQ ID NO: 145-150 aufweist, einem Antikörper, der die CDR-Sequenzen von SEQ ID NO: 155-160 aufweist, einem Antikörper, der die CDR-Sequenzen von SEQ ID NO: 165-170 aufweist, einem Antikörper, der die CDR-Sequenzen von SEQ ID NO: 175-180 aufweist, einem Antikörper, der die CDR-Sequenzen von SEQ ID NO: 185-190 aufweist oder einem Antikörper, der die CDR-Sequenzen von SEQ ID NO: 195-200 aufweist und optional eine Bindungsspezifität für NGF aufweist.

## Revendications

1. Procédé de production d'un complexe à sous-unités multiples, comprenant les étapes consistant à :
(a) fournir une culture comprenant des cellules de *Pichia pastoris* comprenant des gènes qui prévoient l'expression des sous-unités dudit complexe à sous-unités multiples ;
(b) ajouter un bolus d'éthanol à ladite culture, ce qui a pour résultat une concentration en éthanol dans ladite culture comprise entre 0,5 % et 1,5 % (p/v) ; et
(c) ajouter une charge comprenant au moins une source de carbone fermentescible à ladite culture, et cultiver ladite culture pour produire ledit complexe à sous-unités multiples,
dans lequel ledit complexe à sous-unités multiples comprend un anticorps entier ou de longueur totale comprenant des polypeptides à chaîne lourde et légère,
dans lequel ledit complexe à sous-unités multiples n'est pas un anticorps anti-NGF contenant les séquences polypeptidiques SEQ ID NO : 401 et SEQ ID NO : 402.

2. Procédé selon la revendication 1, dans lequel :
(i) le bolus d'éthanol favorise la formation de liaisons disulfure stables par rapport au même procédé effectué en l'absence du bolus d'éthanol ;
(ii) le procédé de culture diminue l'abondance relative d'un ou plusieurs variants associés à un produit par rapport au même procédé effectué en l'absence du bolus d'éthanol ;
(iii) le procédé de culture diminue l'abondance relative de variants associés à un produit présentant un poids moléculaire apparent supérieur ou inférieur à celui dudit anticorps désiré tel que détecté par chromatographie d'exclusion de taille ou électrophorèse en gel par rapport au même procédé effectué en l'absence du bolus d'éthanol ;
(iv) le procédé de culture diminue l'abondance relative d'anticorps présentant une stœchiométrie aberrante par rapport au même procédé effectué en l'absence du bolus d'éthanol ;
(v) le procédé de culture diminue l'abondance relative d'anticorps présentant des liaisons disulfure aberrantes par rapport au même procédé effectué en l'absence du bolus d'éthanol ;
(vi) le procédé de culture diminue l'abondance relative d'anticorps présentant des cystéines réduites par rapport au même procédé effectué en l'absence du bolus d'éthanol ;
(vii) le procédé de culture diminue l'abondance relative d'anticorps présentant une glycosylation aberrante par rapport au même procédé effectué en l'absence du bolus d'éthanol ;
(viii) le procédé de culture module la formation ou la stabilité de liaisons disulfure entre des chaînes lourdes ;
(ix) le procédé de culture module la formation ou la stabilité de liaisons disulfure liant les chaînes légère et lourde dudit anticorps ;
(x) le procédé de culture diminue l'abondance relative d'un ou plusieurs des variants associés à un produit H1L1, H2L1 et H4L4 ; ou
(xi) le procédé de culture augmente la pureté dudit anticorps par rapport audit procédé effectué en l'absence dudit bolus d'éthanol.

3. Procédé selon la revendication 1 ou 2, dans lequel :
(i) l'étape (b) a pour résultat une concentration d'éthanol dans ladite culture comprise entre 0,7 % et 1,5 %, ou entre 0,8 % et 1,25 % (p/v) ;
(ii) l'étape (b) a pour résultat une concentration d'éthanol dans ladite culture qui est de 0,5 %, 0,6 %, 0,7 %, 0,8 % ou 0,9 % (p/v) ;
(iii) l'étape (b) a pour résultat une concentration d'éthanol dans ladite culture qui est de 1,5 %, 1,4 %, 1,3 %, 1,2 %, 1,1 %, 1,0 %, 0,9 %, 0,8 %, 0,7%, 0,6 % ou 0,5 % (p/v) ;
(iv) l'étape (b) comprend un ajout d'éthanol à ladite culture, un ajout d'un support comprenant de l'éthanol à ladite culture, un ajout desdites cellules à un milieu ou support comprenant de l'éthanol, ou un remplacement d'une partie du milieu de culture ;
(v) le bolus d'éthanol est ajouté au milieu de culture pendant une période de temps comprise entre 1 et 20 minutes ;
(vi) l'étape (c) comprend un apport d'oxygène auxdites cellules ;
(vii) l'étape (c) comprend un apport d'oxygène auxdites cellules par agitation de la culture ;
(viii) l'étape (c) comprend un apport d'oxygène auxdites cellules par mise en contact de ladite culture avec un mélange gazeux comprenant de l'oxygène ;
(ix) l'étape (c) comprend un ajout d'une charge comprenant un ou plusieurs parmi le glucose, l'éthanol, le citrate, le sorbitol, le xylose, le tréhalose, l'arabinose, le galactose, le fructose, le mélibiose, le lactose, le maltose, le rhamnose, le ribose, le mannose, le mannitol et le raffinose ;
(x) le procédé de culture comprend en outre un maintien de la concentration d'éthanol entre un point de consigne supérieur et un point de consigne inférieur pendant l'étape (c) ;
(xi) le procédé de culture comprend en outre un maintien de la concentration d'éthanol entre un point de consigne supérieur et un point de consigne inférieur pendant l'étape (c), dans lequel ledit point de consigne inférieur est de 0,5 %, 0,6 %, 0,7 %, 0,8 % ou 0,9 % (p/v) ;
(xii) le procédé de culture comprend en outre un maintien de la concentration d'éthanol entre un point de consigne supérieur et un point de consigne inférieur pendant l'étape (c), dans lequel ledit point de consigne supérieur est de 1,5 %, 1,4 %, 1,3 %, 1,2 %, 1,1 %, 1,0 %, 0,9 %, 0,8 %, 0,7 % ou 0,6 % (p/v) ; ou
(xiii) le procédé de culture comprend en outre un maintien de la concentration d'éthanol entre un point de consigne supérieur et un point de consigne inférieur pendant l'étape (c), dans lequel ledit point de consigne inférieur est de 0,5 %, 0,6 %, 0,7 %, 0,8 % ou 0,9 % (p/v) et ledit point de consigne supérieur est de 1,5 %, 1,4 %, 1,3 %, 1,2 %, 1,1 %, 1,0 %, 0,9 %, 0,8 %, 0,7 % ou 0,6 % (p/v).

4. Procédé selon l'une quelconque des revendications 1 à 3, dans lequel
(i) la concentration d'éthanol est maintenue à un point de consigne pendant l'étape (c) ;
(ii) la concentration d'éthanol est maintenue à un point de consigne qui est de 0,5 %, 0,6 %, 0,7 %, 0,8 %, 0,9 %, 1 %, 1,1 %, 1,2 %, 1,3 %, 1,4 % ou 1,5 % (p/v) pendant l'étape (c) ;
(iii) l'étape (c) comprend un maintien de la concentration d'éthanol dans ladite culture entre 0,7 % et 1,5 %, ou entre 0,8 % et 1,25 % (p/v) ;
(iv) la concentration d'éthanol dans la culture de l'étape (c) est maintenue en commandant une production d'éthanol par lesdites cellules ou en ajoutant de l'éthanol à ladite culture ;
(v) la concentration d'éthanol dans la culture de l'étape (c) est maintenue en commandant une production d'éthanol par lesdites cellules ou en ajoutant de l'éthanol à ladite culture, dans lequel l'étape de commande de production d'éthanol comprend une commande d'une ou plusieurs parmi la concentration de glucose, la disponibilité d'oxygène, l'intensité d'agitation, la pression de gaz, le débit d'écoulement d'air ou d'un autre mélange de gaz apporté, la viscosité de la culture, la densité de culture, la concentration d'oxygène dans l'air ou l'autre mélange de gaz apporté, et la température ;
(vi) le temps entre l'étape (a) et l'étape (b) est inférieur à 72 heures, inférieur à 48 heures, inférieur à 24 heures, inférieur à 12 heures, inférieur à 9 heures, inférieur à 6 heures, inférieur à 5 heures, inférieur à 4 heures, inférieur à 3 heures, inférieur à 90 minutes, inférieur à 30 minutes, inférieur à 5 minutes ou inférieur à 1 minute ;
(vii) le temps entre l'étape (b) et l'étape (c) est inférieur à 10 heures, inférieur à 9 heures, inférieur à 8 heures, inférieur à 7 heures, inférieur à 6 heures, inférieur à 5 heures, inférieur à 4 heures, inférieur à 3 heures, inférieur à 2 heures, inférieur à 90 minutes, inférieur à 80 minutes, inférieur à 70 minutes, inférieur à 60 minutes, inférieur à 50 minutes, inférieur à 40 minutes, inférieur à 30 minutes, inférieur à 20 minutes, inférieur à 10 minutes, inférieur à 5 minutes ou inférieur à 1 minute ;
(viii) la culture de l'étape (a) est produite en ajoutant une source de carbone à ladite culture, et en cultivant ladite culture jusqu'à ce que la source de carbone soit épuisée ;
(ix) la culture de l'étape (a) est produite en ajoutant une source de carbone à ladite culture, et en cultivant ladite culture jusqu'à ce que la source de carbone soit épuisée, dans lequel ladite source de carbone comprend un ou plusieurs parmi : le glycérol, le glucose, l'éthanol, le citrate, le sorbitol, le xylose, le tréhalose, l'arabinose, le galactose, le fructose, le mélibiose, le lactose, le maltose, le rhamnose, le ribose, le mannose, le mannitol et le raffinose.
(x) la culture de l'étape (a) est produite en ajoutant une source de carbone à ladite culture, et en cultivant ladite culture jusqu'à ce que la source de carbone soit épuisée, dans lequel l'épuisement de la source de carbone est déterminé en détectant une diminution de l'activité métabolique desdites cellules de *Pichia pastoris ;*
(xi) la culture de l'étape (a) est produite en ajoutant une source de carbone à ladite culture, et en cultivant ladite culture jusqu'à ce que la source de carbone soit épuisée, dans lequel l'épuisement de la source de carbone est déterminé en détectant une diminution de l'activité métabolique desdites cellules de *Pichia pastoris,* dans lequel ladite diminution de l'activité métabolique desdites cellules de *Pichia pastoris* est identifiée en détectant une diminution de la consommation d'oxygène par lesdites cellules de *Pichia pastoris,* en détectant une augmentation de pH dans la culture, en détectant une stabilisation de la masse cellulaire humide, ou en détectant une augmentation de la concentration d'ammoniac dans la culture ; ou
(xii) la culture de l'étape (a) est produite en ajoutant une source de carbone à ladite culture, et en cultivant ladite culture jusqu'à ce que la source de carbone soit épuisée, dans lequel l'épuisement de la source de carbone est déterminé en détectant une diminution de l'activité métabolique desdites cellules de *Pichia pastoris,* dans lequel ladite diminution de l'activité métabolique desdites cellules de *Pichia pastoris* est identifiée en détectant une diminution de la consommation d'oxygène par lesdites cellules de *Pichia pastoris,* en détectant une augmentation de pH dans la culture, en détectant une stabilisation de la masse cellulaire humide, ou en détectant une augmentation de la concentration d'ammoniac dans la culture, dans lequel ladite diminution de la consommation d'oxygène par lesdites cellules de *Pichia pastoris* est identifiée en détectant une augmentation de la concentration d'oxygène dissous dans ladite culture.

5. Procédé selon l'une quelconque des revendications 1-4, dans lequel :
(i) les cellules de *Pichia pastoris* comprennent des gènes intégrés dans leur génome qui prévoient une expression dudit anticorps, dans lequel lesdits gènes sont intégrés dans un ou plusieurs loci génomiques ;
(ii) les cellules de *Pichia pastoris* comprennent des gènes intégrés dans leur génome à des loci choisis dans le groupe consistant en le locus pGAP, le locus 3' AOX TT ; PpURAS ; OCH1 ; AOX1 ; HIS4; GAP ; pGAP ; 3' AOX TT ; ARG ; et le locus HIS4 TT ;
(iii) les gènes codant pour les sous-unités de l'anticorps sont exprimés sous commande d'un promoteur inductible ou constitutif ;
(iv) les gènes codant pour les sous-unités de l'anticorps sont exprimés sous commande d'un promoteur inductible choisi dans le groupe consistant en les promoteurs AOX1, CUP1, inductible par tétracycline, inductible par thiamine et FLD1 ;
(v) au moins un des gènes codant pour lesdites sous-unités de l'anticorps est exprimé sous la commande d'un promoteur choisi dans le groupe consistant en : les promoteurs CUP1, AOX1, ICL1, glycéraldéhyde-3-phosphate déshydrogénase (GAP), FLD1, ADH1, alcool déshydrogénase II, GAL4, PHO3, PHO5 et Pyk, des promoteurs inductibles par tétracycline, des promoteurs inductibles par thiamine, des promoteurs chimériques dérivés de ceux-ci, des promoteurs de levure, des promoteurs de mammifère, des promoteurs d'insecte, des promoteurs de plante, des promoteurs de reptile, des promoteurs d'amphibien, des promoteurs de virus et des promoteurs d'oiseau ; et/ou
(xi) les cellules de *Pichia pastoris* sont des cellules diploïdes, tétraploïdes ou polyploïdes.

6. Procédé selon l'une quelconque des revendications 1-5, comprenant au moins une des étapes consistant à :
(i) purifier ledit anticorps à partir desdites cellules de *Pichia pastoris* ou à partir du milieu de culture ;
(ii) purifier ledit anticorps à partir d'un composant intracellulaire, d'un cytoplasme, d'un nucléoplasme ou d'une membrane desdites cellules de *Pichia pastoris ;*
(iii) les cellules de *Pichia pastoris* sécrètent ledit anticorps dans le milieu de culture ;
(iv) purifier ledit anticorps à partir dudit milieu de culture ;
(v) l'anticorps comprend un anticorps monospécifique ou bispécifique ;
(vi) l'anticorps comprend un anticorps humain ou un anticorps humanisé ;
(vii) l'anticorps comprend un anticorps humanisé, dans lequel ledit anticorps humanisé a pour origine une souris, un rat, un lapin, une chèvre, un mouton ou une vache ;
(viii) l'anticorps comprend un anticorps humanisé ayant pour origine un lapin ;
(ix) l'anticorps comprend un anticorps monovalent, bivalent ou multivalent ;
(x) l'anticorps est purifié à partir de ladite culture par affinité de protéine A et/ou de protéine G ;
(xi) au moins l'un des gènes qui prévoient l'expression d'une sous-unité dudit anticorps dans au moins l'une desdites cellules de *Pichia pastoris* dans ledit groupe est optimisé pour une expression dans ladite cellule de levure ;
(xii) la pureté dudit anticorps est évaluée en mesurant la fraction de l'anticorps produite par ladite cellule de levure qui est contenue dans des complexes d'anticorps présentant le rayon hydrodynamique apparent attendu, est contenue dans des complexes d'anticorps présentant le poids moléculaire attendu, et/ou se lie spécifiquement à une cible dudit anticorps ;
(xiii) le rendement dudit anticorps est évalué en déterminant la quantité d'anticorps produite par ladite cellule de levure en retranchant de quelconques variants associés à un produit qui sont anormalement glycosylés, contenue dans des complexes d'anticorps autres que des complexes présentant le rayon hydrodynamique apparent attendu, contenue dans des complexes d'anticorps présentant le poids moléculaire attendu, et/ou qui échouent à se lier spécifiquement à la cible dudit anticorps et le poids moléculaire desdits complexes d'anticorps est facultativement déterminé par SDS-PAGE non réducteur ;
(xiv) ledit procédé comprend en outre une purification dudit anticorps ;
(xv) les cellules de culture produisent un titre d'anticorps surnageant d'au moins 100 mg/L, d'au moins 150 mg/L, d'au moins 200 mg/L, d'au moins 250 mg/L, d'au moins 300 mg/L, d'entre 100 et 300 mg/L, d'entre 100 et 500 mg/L, d'entre 100 et 1000 mg/L, d'au moins 1000 mg/L, d'au moins 1 250 mg/litre, d'au moins 1500 mg/litre, d'au moins 1 750 mg/litre, d'au moins 2 000 mg/litre, d'au moins 10 000 mg/litre, ou plus ;
(xvi) une ou plusieurs sous-unités dudit anticorps sont exprimées à partir de plus d'une copie de gène ;
(xvii) l'anticorps est exprimé à partir de 1-10 copies d'un gène codant pour la chaîne légère dudit anticorps et à partir de 1-10 copies d'un gène codant pour la chaîne lourde dudit anticorps, facultativement intégrés dans le génome desdites cellules ;
(xviii) les gènes qui prévoient une expression dudit anticorps sont contenus sur un élément extrachromosomique, un plasmide ou un chromosome artificiel ;
(xix) les cellules de *Pichia pastoris* comprennent plus de copies du gène qui prévoient l'expression de la chaîne légère dudit anticorps que de copies du gène qui prévoient une expression de la chaîne lourde dudit anticorps ;
(xx) les cellules cultivées comprennent plus de copies du gène qui prévoient l'expression de la chaîne légère dudit anticorps que de copies du gène qui prévoient une expression de la chaîne lourde dudit anticorps, dans lequel le nombre respectif de copies du gène codant pour la chaîne lourde dudit anticorps et le nombre de copies du gène codant pour la chaîne légère dudit anticorps dans lesdites cellules sont : 2 et 2, 2 et 3, 3 et 3, 3 et 4, 3 et 5, 4 et 3, 4 et 4, 4 et 5, 4 et 6, 5 et 4, 5 et 5, 5 et 6, ou 5 et 7 ; et
(xxi) les cellules cultivées comprennent plus de copies du gène qui prévoient l'expression de la chaîne légère dudit anticorps que de copies du gène qui prévoient une expression de la chaîne lourde dudit anticorps, et en outre dans lequel le nombre respectif de copies du gène codant pour la chaîne lourde dudit anticorps et le nombre de copies du gène codant pour la chaîne légère dudit anticorps dans lesdites cellules sont : 2 et 1, 3 et 1, 4 et 1,5 et 1, 6 et 1, 7 et 1, 8 et 1, 9 et 1, 10 et 1, 1 et 2, 2 et 2, 3 et 2, 4 et 2, 5 et 2, 6 et 2, 7 et 2, 8 et 2, 9 et 2, 10 et 2, 1 et 3, 2 et 3, 3 et 3, 4 et 3, 5 et 3, 6 et 3, 7 et 3, 8 et 3, 9 et 3, 10 et 3, 1 et 4, 2 et 4, 3 et 4, 4 et 4, 5 et 4, 6 et 4, 7 et 4, 8 et 4, 9 et 4, 10 et 4, 1 et 5, 2 et 5, 3 et 5, 4 et 5, 5 et 5, 6 et 5, 7 et 5, 8 et 5, 9 et 5, 10 et 5, 1 et 6, 2 et 6, 3 et 6, 4 et 6, 5 et 6, 6 et 6, 7 et 6, 8 et 6, 9 et 6, 10 et 6, 1 et 7, 2 et 7, 3 et 7, 4 et 7, 5 et 7, 6 et 7, 7 et 7, 8 et 7, 9 et 7, 10 et 7, 1 et 8, 2 et 8, 3 et 8, 4 et 8, 5 et 8, 6 et 8, 7 et 8, 8 et 8, 9 et 8, 10 et 8, 1 et 9, 2 et 9, 3 et 9, 4 et 9, 5 et 9, 6 et 9, 7 et 9, 8 et 9, 9 et 9, 10 et 9, 1 et 10, 2 et 10, 3 et 10, 4 et 10, 5 et 10, 6 et 10, 7 et 10, 8 et 10, 9 et 10, 10 et 10.

7. Procédé selon l'une quelconque des revendications précédentes, dans lequel :
(i) la culture de l'étape (c) est mise à croître dans un milieu de production ;
(ii) la culture de l'étape (c) est mise à croître dans un milieu de production comprenant un milieu minimal ;
(iii) la culture de l'étape (c) est mise à croître dans un milieu de production comprenant un milieu minimal manquant d'agents sélectifs ;
(iv) la culture de l'étape (c) est mise à croître dans un milieu de production comprenant un milieu minimal manquant d'acides aminés préformés ou d'autres biomolécules complexes ;
(v) la culture de l'étape (c) est mise à croître dans un milieu de production qui comprend un milieu complexe ;
(vi) la culture de l'étape (c) est mise à croître dans un milieu de production qui comprend un milieu complexe qui comprend un ou plusieurs parmi un extrait de levure, des peptones de soja et d'autres peptones de plante ;
(vii) la culture de l'étape (c) est mise à croître à une densité cellulaire élevée ;
(viii) la culture de l'étape (c) est mise à croître à une densité cellulaire qui est d'au moins 50 g/L ;
(ix) la culture de l'étape (c) est mise à croître à une densité cellulaire qui est d'au moins 100 g/L ;
(x) la culture de l'étape (c) est mise à croître à une densité cellulaire qui est d'au moins 300 g/L ;
(xi) la culture de l'étape (c) est mise à croître à une densité cellulaire qui est d'au moins 400 g/L ;
(xii) la culture de l'étape (c) est mise à croître à une densité cellulaire qui est d'au moins 500 g/L ;
(xiii) la culture de l'étape (c) est mise à croître à une densité cellulaire qui est d'au moins 750 g/L ;
(xiv) les cellules de *Pichia pastoris* de l'étape (c) sont cultivées pendant au moins 20 doublements et maintiennent des niveaux élevés d'expression dudit anticorps après lesdits au moins 20 doublements ;
(xv) les cellules de *Pichia pastoris* de l'étape (c) sont cultivées pendant au moins 50 doublements et maintiennent des niveaux élevés d'expression dudit anticorps après lesdits au moins 50 doublements ; ou
(xvi) les cellules de *Pichia pastoris* de l'étape (c) sont cultivées pendant au moins 100 doublements et maintiennent des niveaux élevés d'expression dudit anticorps après lesdits au moins 100 doublements.

8. Procédé selon l'une quelconque des revendications 1-7, dans lequel au moins une sous-unité de l'anticorps est codée par un gène qui comprend un signal de sécrétion, dans lequel facultativement le signal de sécrétion comprend un ou plusieurs polypeptides choisis dans le groupe consistant en : SEQ ID NOS : 414 à 437 et toute combinaison de celles-ci.

9. Procédé selon l'une quelconque des revendications 1 à 8, dans lequel le complexe à sous-unités multiples ne contient pas la totalité des six séquences CDR des SEQ ID NO : 5-10, ne contient pas la totalité des six séquences CDR des SEQ ID NO : 15-20, ne contient pas la totalité des six séquences CDR des SEQ ID NO : 25-30, ne contient pas la totalité des six séquences CDR des SEQ ID NO : 35-40, ne contient pas la totalité des six séquences CDR des SEQ ID NO : 45-50, ne contient pas la totalité des six séquences CDR des SEQ ID NO : 55-60, ne contient pas la totalité des six séquences CDR des SEQ ID NO : 65-70, ne contient pas la totalité des six séquences CDR des SEQ ID NO : 75-80, ne contient pas la totalité des six séquences CDR des SEQ ID NO : 85-90, ne contient pas la totalité des six séquences CDR des SEQ ID NO : 95-100, ne contient pas la totalité des six séquences CDR des SEQ ID NO : 105-110, ne contient pas la totalité des six séquences CDR des SEQ ID NO : 115-120, ne contient pas la totalité des six séquences CDR des SEQ ID NO : 125-130, ne contient pas la totalité des six séquences CDR des SEQ ID NO : 135-140, ne contient pas la totalité des six séquences CDR des SEQ ID NO : 145-150, ne contient pas la totalité des six séquences CDR des SEQ ID NO : 155-160, ne contient pas la totalité des six séquences CDR des SEQ ID NO : 165-170, ne contient pas la totalité des six séquences CDR des SEQ ID NO : 175-180, ne contient pas la totalité des six séquences CDR des SEQ ID NO : 185-190, ne contient pas la totalité des six séquences CDR des SEQ ID NO : 195-200.

10. Procédé selon l'une quelconque des revendications 1 à 9, dans lequel le complexe à sous-unités multiples n'est pas un anticorps anti-NGF.

11. Procédé selon l'une quelconque des revendications 1 à 8 ou 10, dans lequel le complexe à sous-unités multiples ne contient pas au moins une, au moins deux, au moins trois, au moins quatre, ou au moins cinq des CDR contenues dans l'un quelconque des anticorps suivants : un anticorps présentant les séquences CDR des SEQ ID NO 5-10, un anticorps présentant les séquences CDR des SEQ ID NO : 15-20, un anticorps présentant les séquences CDR des SEQ ID NO : 25-30, un anticorps présentant les séquences CDR des SEQ ID NO : 35-40, un anticorps présentant les séquences CDR des SEQ ID NO : 45-50, un anticorps présentant les séquences CDR des SEQ ID NO : 55-60, un anticorps présentant les séquences CDR des SEQ ID NO : 65-70, un anticorps présentant les séquences CDR des SEQ ID NO : 75-80, un anticorps présentant les séquences CDR des SEQ ID NO : 85-90, un anticorps présentant les séquences CDR des SEQ ID NO : 95-100, un anticorps présentant les séquences CDR des SEQ ID NO : 105-110, un anticorps présentant les séquences CDR des SEQ ID NO : 115-120, un anticorps présentant les séquences CDR des SEQ ID NO : 125-130, un anticorps présentant les séquences CDR des SEQ ID NO : 135-140, un anticorps présentant les séquences CDR des SEQ ID NO : 145-150, un anticorps présentant les séquences CDR des SEQ ID NO : 155-160, un anticorps présentant les séquences CDR des SEQ ID NO : 165-170, un anticorps présentant les séquences CDR des SEQ ID NO : 175-180, un anticorps présentant les séquences CDR des SEQ ID NO : 185-190, ou un anticorps présentant les séquences CDR des SEQ ID NO : 195-200 et présentant facultativement une spécificité de liaison pour NGF.
